# EUROPEAN PATENT APPLICATION

(11) **EP 4 810 489 A1**
(43) Date of publication of application: **23.09.2026**
(21) Application number: 24890796.6
(22) Date of filing: 15.11.2024
(51) Int. Cl.: C07D 401/14, C07D 207/08, C07D 211/34, C07D 405/14, C07D 401/06, C07D 403/10, C07D 409/10, C07D 411/12, A61K 31/4025, A61K 31/4709, A61P 9/00, A61P 3/06

(54) **SUBSTITUTED 2-(PYRROLIDIN-3-YL)ACETIC ACID DERIVATIVE, PREPARATION METHOD THEREFOR AND USE THEREOF**

(30) Priority: 17.11.2023 CN 202311536343; 07.02.2024 CN 202410174573; 21.06.2024 CN 202410811526; 06.09.2024 CN 202411250214
(71) Applicant: Innovstone Therapeutics Limited, Shanghai 201318 (CN)
(72) Inventor: SONG, Yunlong, Shanghai 201318 (CN); XU, Wenqing, Shanghai 201318 (CN); YAO, Yang, Shanghai 201318 (CN); KOU, Hongyan, Shanghai 201318 (CN); HUANG, Linfeng, Shanghai 201318 (CN); CHEN, Zhiyuan, Shanghai 201318 (CN); LU, Kai, Shanghai 201318 (CN); WANG, Disha, Shanghai 201318 (CN); ZHANG, Xiaolin, Shanghai 201318 (CN); LIU, Yongmei, Shanghai 201318 (CN)
(74) Representative: AstraZeneca Intellectual Property
(86) International application number: PCT/CN2024/132222
(87) International publication number: WO 2025/103442

(57) **Abstract**

The invention provides a class of compounds represented by Formula (I), and pharmaceutically acceptable salts thereof. The compounds of the invention have strong inhibitory activity against Lp(a) assembly and can be used to treat cardiovascular diseases (CVDs).

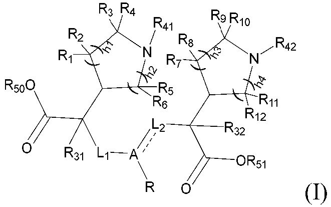

## Description

### Cross Reference to Related Applications

This application is based on and claims priority to Chinese Patent Application Nos. 202311536343.5 filed on November 17, 2023; 202410174573.X filed February 7, 2024; 202410811526.1 filed on June 21, 2024; and 202411250214.4 filed on September 6, 2024, the entire contents of all which are hereby incorporated by reference.

### Technical Field

The invention relates to the field of pharmaceutical technology, and in particular, to compounds as Lp(a) inhibitors, particularly a substituted 2-(pyrrolidine-3-yl)acetic acid derivative and its preparation method and usage.

### Background Technology

Lipoprotein(a) (Lp(a)) is a lipoprotein particle synthesized in the liver, consisting of cholesterol-rich low-density lipoprotein (LDL-C)-like particles attached to apolipoprotein(a). Lp(a) levels are primarily genetically determined, vary widely across populations, and are largely unaffected by lifestyle interventions.

Lp(a) is associated with an increased risk of coronary artery diseases, ischemic stroke, aortic stenosis, heart failure, atrial fibrillation, and peripheral artery diseases. Elevated Lp(a) levels (≥30 mg/dL) are present in approximately 20% of people. The increased risk of cardiovascular diseases (CVDs) associated with Lp(a) is mainly attributed to the dual prothrombotic effects of Apo(a) [Apo(a) structure is similar to plasminogen], as well as the atherogenic effects of Apo B(apolipoprotein B) and the pro-inflammatory effects of oxidized phospholipids (OxPL). Lp(a) is not only a causative factor for atherosclerotic cardiovascular disease(ASCVD) but also for calcific aortic valve disease. Elevated Lp(a) plasma levels are an independent risk factor for CVDs.

There are few approved treatment options for patients with elevated Lp(a) concentration. Plasmapheresis can be used to filter the blood to remove LDL and Lp(a); however, the effect is temporary and usually needs to be repeated every two weeks, and patient compliance is not very good. To date, there are no approved drug therapies for lowering Lp(a) levels. Therefore, there is a great need to provide patients with CVDs with usable compounds and treatment options to lowering plasma Lp(a) levels.

### Content of the Invention

The purpose of the invention is to provide a class of novel compounds for lowering plasma Lp(a) levels, a method for preparing the compounds, and their usage in the treatment of diseases mediated by Lp(a). The novel compounds are biochemically effective and physiologically active. Firstly, the invention provides a compound represented by the following Formula (I), or a pharmaceutically acceptable salt thereof:

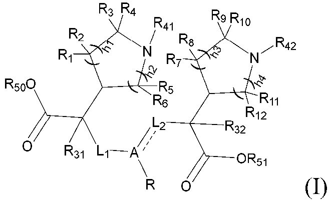

In the formula, - - - is a single bond or a double bond;
A is CR_{X}, P, P(O), N, C₆₋₁₂ aryl, 5-14 membered heteroaryl, C₃₋₈carbocyclyl or 5-18 membered heterocyclyl; wherein the aryl, heteroaryl, carbocyclyl and heterocyclyl are optionally substituted by one or more substituents independently selected from deuterium, halogen, -CN, C₁₋₃alkyl, and C₁₋₃alkoxy;
when - - - is a single bond, R_{X} is H, -OH, or C₁₋₃alkoxy;
when - - - is a double bond, R_{X} does not exist;
L₁ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, and 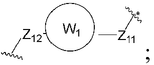 wherein the C₁-₃alkylene is optionally substituted by one or more substituents independently selected from deuterium, halogen, oxo, -CN, -OH, - NH₂, and C₁-₃alkyl; wherein the aryl and heteroaryl are optionally substituted by one or more substituents independently selected from deuterium, halogen, -CN, and C₁₋₃alkoxy; * is the connecting end of L₁ and A;
L₂ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, and 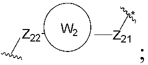 wherein the C₁-₃alkylene is optionally substituted by one or more substituents independently selected from deuterium, halogen, oxo, -CN, -OH, - NH₂, and C₁-₃alkyl; wherein the aryl and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀aryl, -C₁-₃alkylene-5-12 membered heteroaryl, C₁₋₆alkyl, and 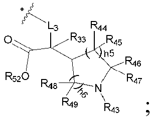 wherein the C₁-₃alkylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, C₁₋₃alkoxy, and C₁₋₃aminoalkyl; wherein the C₁₋₆alkyl is optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-C₆₋₁₀aryl-C₁₋₃alkylene-, -C₁-₃alkylene-5-12 membered heteroaryl-5-12 membered heteroaryl-C₁-₃alkylene-, and 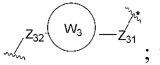 wherein the alkylene, alkenylene, and alkynylene are optionally substituted by one or more substituents independently selected from deuterium, halogen, oxo, -CN, -OH, -NH₂, and C₁₋₃alkyl; wherein the aryl and heteroaryl are optionally substituted by one or more substituents independently selected from deuterium, halogen, -CN, C₁₋₃alkyl, and C₁₋₃alkoxy; * is the connecting end of L₃ and A;
each of rings W₁, W₂, and W₃ is independently selected from C₆₋₁₂aryl, 5-12 membered heteroaryl, C₆₋₁₄cycloalkyl, and 5-12 membered heterocyclyl; wherein the aryl, heteroaryl, cycloalkyl, and heterocyclyl are optionally substituted by one or more substituents independently selected from deuterium, halogen, -CN, -OH, -NH₂, -CHO, C₁₋₃alkyl, C₁₋₃alkoxy, C₁-₃haloalkyl, C₁₋₃haloalkoxy, -N(C₁-₃alkyl)₂, -NH(C₁-₃alkyl), and -C(O)C₁-₃alkyl;
each of Z₁₁, Z₁₂, Z₂₁, Z₂₂, Z₃₁, and Z₃₂ is independently selected from a bond, -O-, -S-, -NH-, -Se-, -C₁₋₄alkylene-, -C₁₋₈oxaalkylene-, -C₁₋₄thiaalkylene-, -C₁₋₄azaalkylene-, and -C₁₋₄selenaalkylene-; wherein the alkylene, oxaalkylene, thiaalkylene, azaalkylene, and selenaalkylene are optionally substituted by one or more substituents independently selected from Z₄₁; Z₄₁ is independently selected from deuterium, halogen, oxo, thio, -CN, -OH, -NH₂, C₁₋₃alkyl, C₁-₃haloalkyl, C₁₋₃alkoxy, and C₁₋₃haloalkoxy; or any two Z₄₁ together with the atom to which they are attached form C₃₋₆cycloalkyl, 3-6 membered heterocyclyl, phenyl, or 5-6 membered heteroaryl;
each of R₃₁, R₃₂, and R₃₃ is independently selected from hydrogen, deuterium, halogen, C₁₋₆alkyl, C₁-₆haloalkyl, C₁-₆deuteroalkyl, C₁-₆alkoxy, C₁-₆haloalkoxy, and C₁-₆deuteroalkoxy;
each of h1, h2, h3, h4, h5, and h6 is independently 0, 1, or 2;
each of R₁, R₂, R₃, R₄, R₅, R₆, R₇, R₈, R₉, R₁₀, R₁₁, R₁₂, R₄₁, R₄₂, R₄₃, R₄₄, R₄₅, R₄₆, R₄₇, R₄₈, and R₄₉ is independently selected from H, deuterium, -CN, -OH, -NH₂, halogen, C₁₋₆alkyl, C₁-₆haloalkyl, C₁₋₆alkoxy, C₁-₆haloalkoxy, -N(C₁-₃alkyl)₂, and -NH(C₁-₃alkyl);
each of R₅₀, R₅₁, R₅₂ is independently selected from H, deuterium, C₁₋₆alkyl, C₂₋₆alkenyl, C₁₋₆alkoxy, -C₁₋₃alkylene-O-C₁₋₆alkyl, -C₁₋₃alkylene-O-C(O)-C₁₋₆alkyl, the alkyl, alkenyl, alkoxy, and alkylene are optionally substituted by one or more substituents independently selected from deuterium, halogen, -OH, -NH₂, -CN, oxo, C₁-₃alkyl, and C₁₋₃alkoxy;
provided that when A is N, and R₁, R₂, R₃, R₄, R₅, R₆, R₇, R₈, R₉, R₁₀, R₁₁, R₁₂, R₄₁, R₄₂, R₄₃, R₄₄, R₄₅, R₄₆, R₄₇, R₄₈, and R₄₉ are all H, L₁, L₂, and L₃ are not 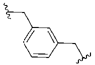 at the same time;
provided that when A is N, R is 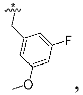 and R₁, R₂, R₃, R₄, R₅, R₆, R₇, R₈, R₉, R₁₀, R₁₁, R₁₂, R₄₁, and R₄₂ are all H, L₁ and L₂ are not 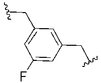 at the same time, * is the connecting end of R and A; provided that the compound is not 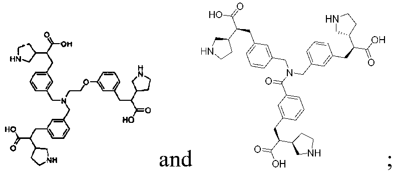
the heteroatom in the heterocyclyl or heteroaryl is independently selected from O, N, or S, and the number of heteroatoms is 1, 2, 3, or 4.

In an embodiment for the compound represented by the above formula (I), R is a group containing L₃; L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-.

In some embodiments, a compound represented by the following Formula (I-1-P1), or a pharmaceutically acceptable salt thereof is provided: 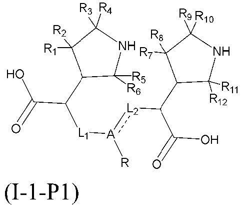
wherein, - - - is a single bond or a double bond;
A is CR_{X}, P, P(O) or N;
when - - - is a single bond; R_{X} is H, -OH, or C₁₋₃alkoxy;
when - - - is a double bond, R_{X} does not exist;
L₁ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃ alkylene-5-12 membered heteroaryl-5-12 membered heteroaryl-C₁-₃alkylene-, and 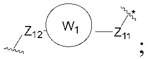 wherein the C₁-₃alkylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, and C₁₋₃alkoxy; * is the connecting end of L₁ and A;
L₂ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, and 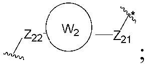 wherein the C₁-₃alkylene is optionally substituted by one or more substituents independently selected from deuterium, halogen, oxo, -CN, -OH, - NH₂, and C₁-₃alkyl; wherein the aryl and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀aryl, -C₁-₃alkylene-5-12 membered heteroaryl, C₁₋₆alkyl, and 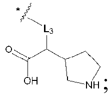 wherein the C₁-₃alkylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, C₁₋₃alkoxy, and C₁₋₃aminoalkyl; wherein the C₁₋₆alkyl is optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁₋₃alkylene-, -C₁-₃alkylene-5-12 membered heteroaryl-C₆₋₁₀aryl-C₁₋₃alkylene-, -C₁-₃alkylene-5-12 membered heteroaryl-5-12 membered heteroaryl-C₁-₃alkylene-, and 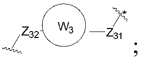 wherein the alkylene, alkenylene, and alkynylene are optionally substituted by one or more substituents independently selected from deuterium, halogen, oxo, -CN, -OH, -NH₂, and C₁₋₃alkyl; wherein the aryl and heteroaryl are optionally substituted by one or more substituents independently selected from deuterium, halogen, -CN, C₁₋₃alkyl, and C₁₋₃alkoxy; * is the connecting end of L₃ and A;
each of rings W₁, W₂, and W₃ is independently selected from C₆₋₁₂aryl, 5-12 membered heteroaryl, C₆₋₁₄cycloalkyl, and 5-12 membered heterocyclyl; wherein the aryl, heteroaryl, cycloalkyl, and heterocyclyl are optionally substituted with one or more substituents independently selected from deuterium, halogen, -CN, C₁₋₃alkyl, and C₁₋₃alkoxy;
each of Z₁₁, Z₁₂, Z₂₁, Z₂₂, Z₃₁, and Z₃₂ is independently selected from a bond, -C₁₋₄alkylene-, and - C₁₋₄oxaalkylene-; wherein the alkylene and oxaalkylene are optionally substituted with one or more substituents independently selected from deuterium, halogen, oxo, -CN, -OH, -NH₂, C₁₋₃alkyl, and C₁₋₃alkoxy;
each of R₁, R₂, R₃, R₄, R₅, R₆, R₇, R₈, R₉, R₁₀, R₁₁, and R₁₂ is independently selected from H and deuterium;
provided that when A is N, L₁, L₂, and L₃ are not 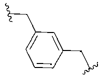 at the same time;
provided that when A is N and R is 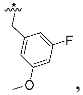 L₁ and L₂ are not 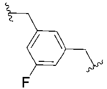 at the same time, and * is the end connecting of R and A;
the heteroatom in the heterocyclyl or heteroaryl is independently selected from O, N, or S, and the number of heteroatoms is 1, 2, or 3.

In an embodiment for the compound represented by the above formula (I-1-P1), R is a group containing L₃; L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

In some embodiments, a compound represented by the following Formula (I-1-P2), or a pharmaceutically acceptable salt thereof is provided: 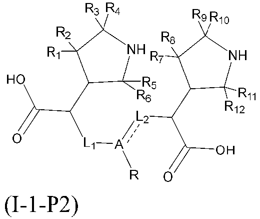
wherein, - - - is a single bond or a double bond;
A is CR_{X}, P, P(O) or N;
when - - - is a single bond, R_{X} is H, -OH, C₁₋₃alkoxy;
when - - - is a double bond, R_{X} does not exist;
L₁ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, 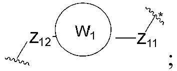 wherein the C₁-₃alkylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, and C₁₋₃alkoxy; * is the connecting end of L₁ and A;
L₂ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, 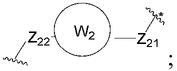 wherein the C₁-₃alkylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, and C₁₋₃alkoxy; * is the connecting end of L₂ and A;
R is selected from H, -C₁-₃alkylene-C₆₋₁₀aryl, -C₁-₃alkylene-5-12 membered heteroaryl, C₁₋₆alkyl, 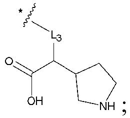 wherein the C₁-₃alkylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, C₁-₃alkoxy, and C₁₋₃aminoalkyl; wherein the C₁₋₆alkyl is optionally substituted by one or more substituents independently selected from deuterium, halogen, oxo, carboxyl, -CN, -OH, -NH₂, C₁-₃alkyl, and C₁₋₃alkoxy; * is the connecting end of R and A;
L3 is selected from -C₁₋₆alkylene-, -C₂₋₆alkenylene-, -C₂₋₆alkynylene-, -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁₋₃alkylene-, -C₁-₃alkylene-5-12 membered heteroaryl-C₆₋₁₀aryl-C₁₋₃alkylene-, -C₁-₃alkylene-5-12 membered heteroaryl-5-12 membered heteroaryl-C₁-₃alkylene-, 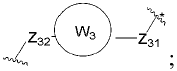 the alkylene, alkenylene, and alkynylene are optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, C₁-₃alkyl, and C₁₋₃alkoxy; * is the connecting end of L₃ and A;
each of rings W₁, W₂, and W₃ is independently selected from C₆₋₁₂aryl, 5-12 membered heteroaryl, C₆₋₁₄cycloalkyl, 5-12 membered heterocyclyl; wherein the aryl, heteroaryl, cycloalkyl, and heterocyclyl are optionally substituted with one or more substituents independently selected from deuterium, halogen, -CN, C₁-₃alkyl, and C1-3alkoxy;
each of Z₁₁, Z₁₂, Z₂₁, Z₂₂, Z₃₁, and Z₃₂ is independently selected from a bond, -C₁₋₄alkylene-, -C₁₋₄oxaalkylene-, -C₁₋₄thiaalkylene-, -C₁₋₄azaalkylene-; wherein the alkylene, oxaalkylene, thiaalkylene, and azaalkylene are optionally substituted by one or more substituents independently selected from deuterium, halogen, oxo, -CN, -OH, -NH₂, C₁-₃alkyl, and C1-3alkoxy;
each of R₁, R₂, R₃, R₄, R₅, R₆, R₇, R₈, R₉, R₁₀, R₁₁, and R₁₂ is independently selected from H and deuterium;
provided that when A is N, L₁, L₂, and L₃ are not at the same time 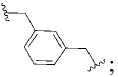
provided that when A is N, R is 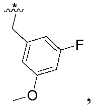 L₁ and L₂ are not 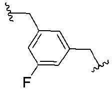 at the same time, * is the connecting end of R and A;
the heteroatom in the heterocyclyl or heteroaryl is independently selected from O, N or S, and the number of heteroatoms is 1, 2, or 3.

In an embodiment for the compound represented by the above formula (I-1-P2), R is a group containing L₃; L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

In some embodiments, a compound represented by the following Formula (I-1-P3), or a pharmaceutically acceptable salt thereof is provided: 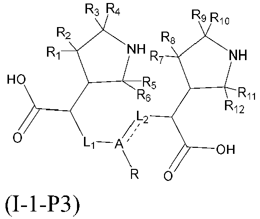
wherein, - - - is a single bond or a double bond;
A is CR_{X}, P, P(O), N, C₆₋₁₂ aryl, 5-14 membered heteroaryl, and 5-18 membered heterocyclyl;
wherein the aryl, heteroaryl, and heterocyclyl are optionally substituted by one or more substituents independently selected from deuterium, halogen, -CN, C₁₋₃alkyl, and C₁₋₃alkoxy;
when - - - is a single bond; R_{X} is H, -OH, or C₁₋₃alkoxy;
when - - - is a double bond, R_{X} does not exist;
L₁ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃ alkylene-5-12 membered heteroaryl-5-12 membered heteroaryl-C₁-₃alkylene-, and 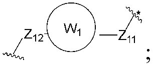 wherein the C₁-₃alkylene is optionally substituted by one or more substituents independently selected from deuterium, halogen, oxo, -CN, -OH, - NH₂, and C₁-₃alkyl; wherein the aryl and heteroaryl are optionally substituted by one or more substituents independently selected from deuterium, halogen, -CN, and C₁₋₃alkoxy; * is the connecting end of L₁ and A;
L₂ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, and 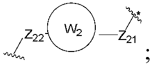 wherein the C₁-₃alkylene is optionally substituted by one or more substituents independently selected from deuterium, halogen, oxo, -CN, -OH, - NH₂, and C₁-₃alkyl; wherein the aryl and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀aryl, -C₁-₃alkylene-5-12 membered heteroaryl, C₁₋₆alkyl, and 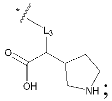 wherein the C₁₋₃alkylene is optionally substituted by one or more substituents independently selected from deuterium, halogen, oxo, -CN, -OH, -NH₂, and C₁₋₃alkyl; wherein the aryl and heteroaryl are optionally substituted by one or more substituents independently selected from deuterium, halogen, -CN, C₁₋₃alkoxy, and C₁₋₃aminoalkyl; wherein the C₁₋₆alkyl is optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁₋₃alkylene-, -C₁-₃alkylene-5-12 membered heteroaryl-C₆₋₁₀aryl-C₁₋₃alkylene-, -C₁-₃alkylene-5-12 membered heteroaryl-5-12 membered heteroaryl-C₁-₃alkylene-, and 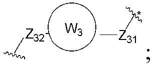 wherein the alkylene, alkenylene, and alkynylene are optionally substituted by one or more substituents independently selected from deuterium, halogen, oxo, -CN, -OH, -NH₂, and C₁₋₃alkyl; wherein the aryl and heteroaryl are optionally substituted by one or more substituents independently selected from deuterium, halogen, -CN, C₁₋₃alkyl, and C₁₋₃alkoxy; * is the connecting end of L₃ and A;
each of rings W₁, W₂, and W₃ is independently selected from C₆₋₁₂aryl, 5-12 membered heteroaryl, C₆₋₁₄cycloalkyl, and 5-12 membered heterocyclyl; wherein the aryl, heteroaryl, cycloalkyl, and heterocyclyl are optionally substituted with one or more substituents independently selected from deuterium, halogen, -CN, C₁₋₃alkyl, and C₁₋₃alkoxy;
each of Z₁₁, Z₁₂, Z₂₁, Z₂₂, Z₃₁, and Z₃₂ is independently selected from a bond, -C₁₋₄alkylene-, -C₁₋₄oxaalkylene-, -C₁₋₄thiaalkylene-, and -C₁₋₄azaalkylene-; wherein the alkylene, oxaalkylene, thiaalkylene, and azaalkylene are optionally substituted by one or more substituents independently selected from deuterium, halogen, oxo, -CN, -OH, -NH₂, C₁₋₃alkyl, C₁₋₃haloalkyl, C₁₋₃alkoxy, and C₁₋₃haloalkoxy;
each of R₁, R₂, R₃, R₄, R₅, R₆, R₇, R₈, R₉, R₁₀, R₁₁, and R₁₂ is independently selected from H and deuterium;
provided that when A is N, and R₁, R₂, R₃, R₄, R₅, R₆, R₇, R₈, R₉, R₁₀, R₁₁, and R₁₂ are all H, L₁, L₂, and L₃ are not 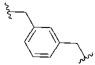 at the same time;
provided that when A is N, R is 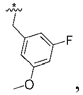 and R₁, R₂, R₃, R₄, R₈, R₆, R₇, R₈, R₉, R₁₀, R₁₁, and R₁₂ are all H, L₁ and L₂ are not 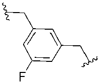 at the same time, and * is the connecting end of R and A;
provided that it is not 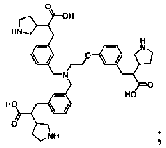
the heteroatom in the heterocyclyl or heteroaryl is independently selected from O, N, or S, and the number of heteroatoms is 1, 2, 3, or 4.

In an embodiment for the compound represented by the above formula (I-1-P3), R is a group containing L₃; L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

In some embodiments, a compound represented by the following Formula (I-1-P4), or a pharmaceutically acceptable salt thereof is provided: 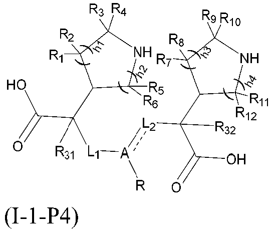
wherein, - - - is a single bond or a double bond;
Ais CR_{X}, P, P(O), N, C₆₋₁₂aryl, 5-14membered heteroaryl, or 5-18membered heterocyclyl; the aryl, heteroaryl, and heterocyclyl are optionally substituted by one or more substituents independently selected from deuterium, halogen, -CN, C₁-₃alkyl, and C₁₋₃alkoxy;
when - - - is a single bond, RX is H, -OH, or C₁₋₃alkoxy;
when - - - is a double bond, R_{X} does not exist;
L₁ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, and 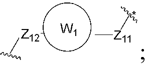 the C₁₋₃alkylene is optionally substituted by one or more substituents independently selected from deuterium, halogen, oxo, -CN, -OH, -NH₂, and C₁-₃alkyl; the aryl and heteroaryl are optionally substituted by one or more substituents independently selected from deuterium, halogen, -CN, and C₁₋₃alkoxy; * is the connecting end of L₁ and A;
L₂ is selected from -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁-₃alkylene-, -C₁-₃alkylene-5-12 membered heteroaryl-5-12 membered heteroaryl-C₁-₃alkylene-, and 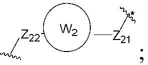 the C₁₋₃alkylene is optionally substituted by one or more substituents independently selected from deuterium, halogen, oxo, -CN, -OH, -NH₂, and C₁-₃alkyl; the aryl and heteroaryl are optionally substituted by one or more substituents independently selected from deuterium, halogen, -CN, and C₁₋₃alkoxy; * is the connecting end of L₂ and A;
R is selected from H, -C₁-₃alkylene-C₆₋₁₀aryl, -C₁-₃alkylene-5-12 membered heteroaryl, C₁₋₆alkyl,and 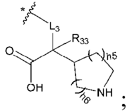 the C₁₋₃alkylene is optionally substituted by one or more substituents independently selected from deuterium, halogen, oxo, -CN, -OH, -NH₂, and C₁-₃alkyl; the aryl and heteroaryl are optionally substituted by one or more substituents independently selected from deuterium, halogen, -CN, C₁-₃alkoxy, and C₁₋₃aminoalkyl; the C₁₋₆alkyl isoptionally substituted by one or more substituents independently selected from deuterium, halogen, oxo, carboxyl, -CN, - OH, -NH₂, C₁-₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁-₃alkylene-C₆₋₁₀aryl-C₆₋₁₀aryl-C₁-₃alkylene-, -C₁-₃alkylene-C₆₋₁₀aryl-5-12 membered heteroaryl-C₁₋₃alkylene-, -C₁-₃alkylene-5-12 membered heteroaryl-C₆₋₁₀aryl-C₁₋₃alkylene-, -C₁-₃alkylene-5-12 membered heteroaryl-5-12 membered heteroaryl-C₁-₃alkylene-, and 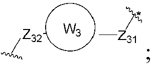 the alkylene, alkenylene, and alkynylene are optionally substituted by one or more substituents independently selected from deuterium, halogen, oxo, -CN, -OH, -NH₂, and C₁-₃alkyl; the aryl and heteroaryl are optionally substituted by one or more substituents independently selected from deuterium, halogen, -CN, C₁-₃alkyl, and C₁₋₃alkoxy; * is the connecting end of L₃ and A;
each of rings W₁, W₂, and W₃ is independently selected from C₆₋₁₂ aryl, 5-12 membered heteroaryl, C₆₋₁₄cycloalkyl, and 5-12 membered heterocyclyl; the aryl, heteroaryl, cycloalkyl, and heterocyclyl are optionally substituted by one or more substituents independently selected from deuterium, halogen, -CN, -OH, -NH₂, -CHO, C₁₋₃alkyl, C₁₋₃alkoxy, C₁₋₃haloalkyl, C₁₋₃haloalkoxy, -N(C₁₋₃alkyl)₂, -NH(C₁₋₃alkyl), and -C(O)C₁₋₃alkyl;
each of Z₁₁, Z₁₂, Z₂₁, Z₂₂, Z₃₁, and Z₃₂ is independently selected from a bond, -O-, -S-, -NH-, -Se-, -C₁₋₄alkylene-, -C₁₋₈oxaalkylene-, -C₁₋₄thiaalkylene-, -C₁₋₄azaalkylene-, and -C₁₋₄selenaalkylene-; the alkylene, oxaalkylene, thiaalkylene, azaalkylene, and selenaalkylene are optionally substituted by one or more substituents independently selected from Z₄₁; Z₄₁ is independently selected from deuterium, halogen, oxo, thio, -CN, -OH, -NH₂, C₁₋₃alkyl, C₁₋₃haloalkyl, C₁₋₃alkoxy, and C₁₋₃haloalkoxy; or any two Z₄₁ together with the atom to which they are attached form C₃₋₆cycloalkyl, 3-6 membered heterocyclyl, phenyl, or 5-6 membered heteroaryl;
each of R₃₁, R₃₂, and R₃₃ is independently selected from hydrogen, deuterium, halogen, C₁₋₆alkyl, C₁₋₆haloalkyl, C₁₋₆deuteroalkyl, C₁₋₆alkoxy, C₁-₆haloalkoxy, and C₁₋₆deuteroalkoxy;
each of h1, h2, h3, h4, h5, and h6 is independently 0, 1, or 2;
each of R₁, R₂, R₃, R₄, R₅, R₆, R₇, R₈, R₉, R₁₀, R₁₁, and R₁₂ is independently selected from H, and deuterium;
provided that when A is N, and R₁, R₂, R₃, R₄, R₅, R₆, R₇, R₈, R₉, R₁₀, R₁₁, and R₁₂ are all H, L₁, L₂, and L₃ are not 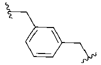 at the same time;
provided that when A is N, R is 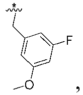 and R₁, R₂, R₃, R₄, R₅, R₆, R₇, R₈, R₉, R₁₀, R₁₁, and R₁₂ are all H, L₁ and L₂ are not 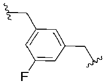 at the same time, * is the connecting end of R and A;
provided that the compound is not 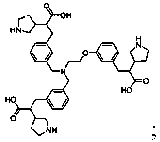
the heteroatom in the heterocyclyl and heteroaryl is independently selected from O, N and S, and the heteroatom number is 1, 2, 3, or 4;

In an embodiment for the compound represented by the above formula (I-1-P4), R is a group containing L₃; L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

In some embodiments, a compound represented by the following Formula (I-2), or a pharmaceutically acceptable salt thereof is provided: 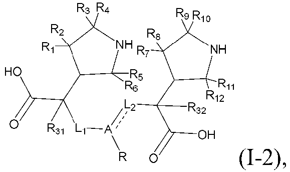 wherein, A, - - - , L₁, L₂, R, R₁, R₂, R₃, R₄, R₅, R₆, R₇, R₈, R₉, R₁₀, R₁₁, R₁₂, R₃₁, and R₃₂ are defined as in the compound of Formula (I), Formula (I-1-P1), Formula (I-1-P2), Formula (I-1-P3), or Formula (I-1-P4).

In an embodiment for the compound represented by the above formula (I-2), R is a group containing L₃; L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

In some embodiments, a compound represented by the following Formula (I-3), or a pharmaceutically acceptable salt thereof is provided:

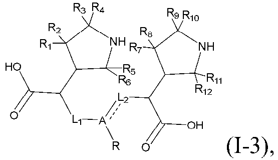

In the formula, A, - - -, L₁, L₂, R, R₁, R₂, R₃, R₄, R₅, R₆, R₇, R₈, R₉, R₁₀, R₁₁, and R₁₂ are defined as in the compound of Formula (I), Formula (I-1-P1), Formula (I-1-P2), Formula (I-1-P3), or Formula (I-1-P4).

In one embodiment of the invention, A is CR_{X}, P, P(O), N, phenyl, 5-6 membered heterocyclic group, or 12 membered heterocyclic group; wherein the heteroatom in the heterocyclic group is N, and the number of heteroatoms is 1, 2, 3 or 4.

In an embodiment for the compound represented by the above formula (I-3), R is a group containing L₃; L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

In one embodiment of the invention, A is CR_{X}, P, P(O), N, phenyl, piperazinyl, 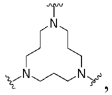 or

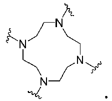

In one embodiment of the invention, A is CR_{X}, P, P(O), N, 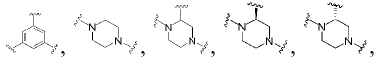 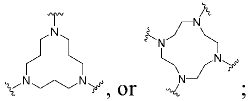 preferably A is N,

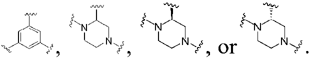

In one embodiment of the invention, A is CR_{X}, P, P(O), or N.

In one embodiment of the invention, A is N.

In one embodiment of the invention, A is P(O);

In one embodiment of the invention, A is selected from CR_{X}; R_{X} is H, -OH, or methoxy.

In one embodiment of the invention, A is selected from C-OH and C-OCH₃.

In one embodiment of the invention, A is a 6-12 membered heterocyclic group.

In one embodiment of the invention, A is a 6 membered heterocyclic group; preferably, A is piperazinyl; preferably

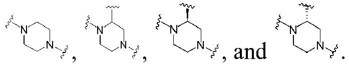

In one embodiment of the invention, A is

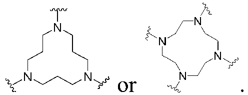

In one embodiment of the invention, A is phenyl; preferably

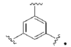

In one embodiment of the invention, R_{X} is H, -OH, or methoxy.

In one embodiment of the invention, L₁ is selected from -methylene-C₆₋₁₀aryl-C₆₋₁₀ aryl-methylene-, -methylene-5-12 membered heteroaryl-5-12 membered heteroaryl-methylene-, and 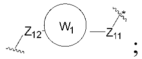 wherein the methylene is optionally substituted by one or more substituents independently selected from deuterium, halogen, oxo, -CN, -OH, -NH₂, and C₁₋₃alkyl; wherein the aryl and heteroaryl are optionally substituted by one or more substituents independently selected from deuterium, halogen, -CN, and C₁₋₃alkoxy; * is the connecting end of L₁ and A.

In one embodiment of the invention, L₁ is selected from -C₁₋₃alkylene-phenyl-phenyl-C₁₋₃alkylene-, -C₁₋₃alkylene-5-6 membered heteroaryl-5-6 membered heteroaryl-C₁₋₃alkylene-, and 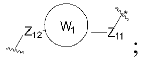 wherein the C₁₋₃alkylene is optionally substituted by one or more substituents independently selected from deuterium, halogen, oxo, -CN, -OH, -NH₂, and C₁₋₃alkyl; wherein the phenyl and heteroaryl are optionally substituted by one or more substituents independently selected from halogen, -CN, and C₁₋₃alkoxy; * is the connecting end of L₁ and N.

In one embodiment of the invention, L₁ is selected from -methylene-phenyl-phenyl-methylene-, - ethylene-phenyl-phenyl-ethylene-, -methylene-5-6 membered heteroaryl-5-6 membered heteroaryl-methylene-, -ethylene-5-6 membered heteroaryl-5-6 membered heteroaryl-ethylene-, and 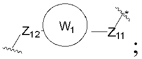 wherein the methylene and ethylene are optionally substituted by one or more substituents independently selected from deuterium, halogen, oxo, -CN, -OH, -NH₂, and C₁-₃alkyl; wherein the phenyl and heteroaryl are optionally substituted by one or more substituents independently selected from halogen, -CN, and C₁₋₃alkoxy; * is the connecting end of L₁ and A.

In one embodiment of the invention, L₁ is selected from -methylene-phenyl-phenyl-methylene-, - methylene-6-membered heteroaryl-6-membered heteroaryl-methylene-, and 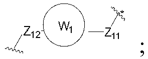 wherein the methylene is optionally substituted by one or more substituents independently selected from deuterium, halogen, oxo, -CN, -OH, -NH₂, and C₁-₃alkyl; wherein the phenyl and heteroaryl are optionally substituted by one or more substituents independently selected from deuterium, halogen, -CN, and C₁₋₃alkoxy; * is the connecting end of L₁ and A.

In one embodiment of the invention, L₁ is selected from -methylene-phenyl-phenyl-methylene-, - methylene-pyridyl-pyridyl-methylene-, and 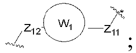 * is the connecting end of L₁ and A.

In one embodiment of the invention, L₁ is selected from 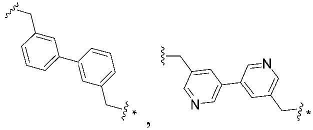 and 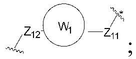 * is the connecting end of L₁ and A.

In one embodiment of the invention, L₁ is 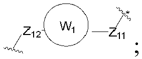 * is the connecting end of L₁ and A.

In one embodiment of the invention, L₂ is selected from -methylene-C₆₋₁₀aryl-C₆₋₁₀aryl-methylene-, -methylene-5-12 membered heteroaryl-5-12 membered heteroaryl-methylene-, and 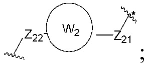 wherein the methylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, and C₁₋₃alkoxy; * is the connecting end of L₂ and A.

In one embodiment of the invention, L₂ is selected from -C₁-₃alkylene-phenyl-phenyl-C₁-₃alkylene-, -C₁-₃alkylene-5-6 membered heteroaryl-5-6 membered heteroaryl-C₁-₃alkylene-, and 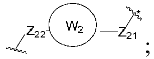 wherein the C₁-₃alkylene is optionally substituted by one or more substituents independently selected from deuterium, halogen, oxo, -CN, -OH, -NH₂, and C₁-₃alkyl; wherein the phenyl and heteroaryl are optionally substituted by one or more substituents independently selected from halogen, -CN, and C₁₋₃alkoxy; * is the connecting end of L₂ and N.

In one embodiment of the invention, L₂ is selected from -methylene-phenyl-phenyl-methylene-, - ethylene-phenyl-phenyl-ethylene-, -methylene-5-6 membered heteroaryl-5-6 membered heteroaryl-methylene-, -ethylene-5-6 membered heteroaryl-5-6 membered heteroaryl-ethylene-, and 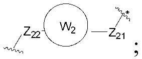 wherein the methylene and ethylene are optionally substituted by one or more substituents independently selected from deuterium, halogen, oxo, -CN, -OH, -NH₂, and C₁-₃alkyl; wherein the phenyl and heteroaryl are optionally substituted by one or more substituents independently selected from halogen, -CN, and C₁₋₃alkoxy; * is the connecting end of L₂ and A.

In one embodiment of the invention, L₂ is selected from -methylene-phenyl-phenyl-methylene-, - methylene-6-membered heteroaryl-6-membered heteroaryl-methylene-, and 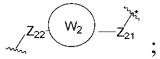 wherein the methylene is optionally substituted by one or more substituents independently selected from deuterium, halogen, oxo, -CN, -OH, -NH₂, and C₁-₃alkyl; wherein the phenyl and heteroaryl are optionally substituted by one or more substituents independently selected from deuterium, halogen, -CN, and C₁₋₃alkoxy; * is the connecting end of L₂ and A.

In one embodiment of the invention, L₂ is selected from -methylene-phenyl-phenyl-methylene-, - methylene-pyridyl-pyridyl-methylene-, and 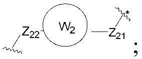 * is the connecting end of L₂ and A.

In one embodiment of the invention, L₂ is selected from 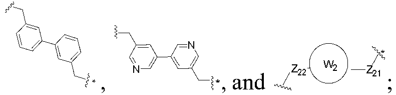 * is the connecting end of L₂ and A.

In one embodiment of the invention, L₂ is selected from 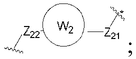 * is the connecting end of L₂ and A.

In one embodiment of the invention, R is selected from H, -C₁-₃alkylene-C₆₋₁₀aryl, -C₁-₃alkylene-5-12 membered heteroaryl, C₁₋₆alkyl, and 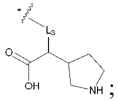 wherein the C₁-₃alkylene is optionally substituted by one or more substituents independently selected from deuterium, halogen, oxo, - CN, -OH, -NH₂, and C₁-₃alkyl; wherein the aryl and heteroaryl are optionally substituted by one or more substituents independently selected from deuterium, halogen, -CN, C₁-₃alkoxy, and C₁-₃aminoalkyl; wherein the C₁₋₆alkyl is optionally substituted by one or more substituents independently selected from deuterium, halogen, oxo, carboxyl, -CN, -OH, -NH₂, C₁-₃alkyl, and C₁₋₃alkoxy; * is the connecting end of R and A.

In one embodiment of the invention, R is selected from H, -C₁-₃alkylene-C₆₋₁₀aryl, -C₁-₃alkylene-5-10 membered heteroaryl, C₁₋₆alkyl, and 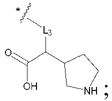 wherein the C₁-₃alkylene is optionally substituted by one or more substituents independently selected from deuterium, halogen, oxo, - CN, -OH, and -NH₂; wherein the aryl and heteroaryl are optionally substituted by one or more substituents independently selected from deuterium, halogen, -CN, methoxy, and C₁-₃aminoalkyl; and wherein the C₁₋₆alkyl is optionally substituted by one or more substituents independently selected from deuterium, halogen, oxo, carboxyl, -CN, -OH, -NH₂, C₁-₃alkyl, and C₁₋₃alkoxy; * is the connecting end of R and A.

In one embodiment of the invention, R is selected from H, -methylene-phenyl, -ethylene-phenyl, -methylene-5-6 membered heteroaryl, -ethylene-5-6 membered heteroaryl, C₁₋₆alkyl, and 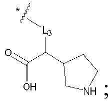 wherein the methylene and ethylene are optionally substituted by one or more substituents independently selected from halogen, oxo, -CN, -OH, and -NH₂; wherein the phenyl and heteroaryl are optionally substituted by one or more substituents independently selected from halogen, -CN, methoxy, and 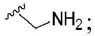 wherein the C₁₋₆alkyl is optionally substituted by one or more substituents independently selected from halogen, oxo, carboxyl, -CN, -OH, and -NH₂; * is the connecting end of R and A.

In one embodiment of the invention, R is selected from H, -methylene-phenyl, -ethylene-phenyl, -methylene-pyridinyl, -ethylene-pyridinyl, methyl, ethyl, n-propyl, isopropyl, n-butyl, n-pentyl, n-hexyl, and 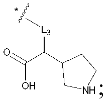 wherein the phenyl and pyridinyl are optionally substituted by one or more substituents independently selected from halogen, methoxy, and 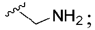 wherein the methyl, ethyl, n-propyl, isopropyl, n-butyl, n-pentyl, and n-hexyl are optionally substituted by one or more substituents independently selected from carboxyl and -NH₂; * is the connecting end of R and A. In one embodiment of the invention, R is selected from H, -C₁-₃alkylene-phenyl, -C₁-₃alkylene-5-6 membered heteroaryl, and C₁₋₆alkyl; wherein the C₁-₃alkylene is optionally substituted by one or more substituents independently selected from halogen, oxo, -CN, -OH, -NH₂, and C₁-₃alkyl; wherein the phenyl and heteroaryl are optionally substituted by one or more substituents independently selected from halogen, -CN, C₁-₃alkoxy, and C₁-₃aminoalkyl; wherein the C₁₋₆alkyl is optionally substituted by one or more substituents independently selected from halogen, carboxyl, -NH₂, methyl, and methoxy.

In one embodiment of the invention, R is selected from H, 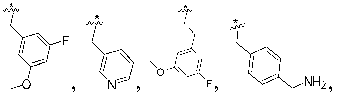 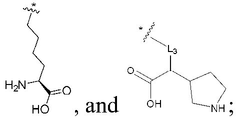 * is the connecting end of R and A.

In one embodiment of the invention, R is selected from H, 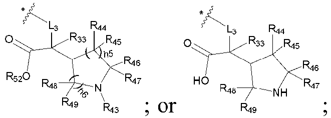 or 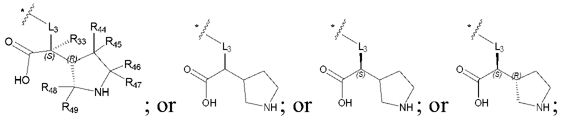 wherein, * is the connecting end with A.

In one embodiment of the invention, L₃ is selected from -C₁₋₆alkylene-, -C₂₋₆alkynylene-, - methylene-C₆₋₁₀aryl-C₆₋₁₀aryl-methylene-, -methylene-5-12 membered heteroaryl-5-12 membered heteroaryl-methylene-, and 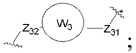 wherein the alkylene, methylene, and alkynylene are optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, C₁-₃alkyl, and C₁₋₃alkoxy; * is the connecting end of L₃ and A.

In one embodiment of the invention, L₃ is selected from -C₁₋₆alkylene-, -C₂₋₆alkynylene-, - methylene-phenyl-phenyl-methylene-, -ethylene-phenyl-phenyl-ethylene-, -methylene-5-6 membered heteroaryl-5-6 membered heteroaryl-methylene-, -ethylene-5-6 membered heteroaryl-5-6 membered heteroaryl-ethylene-, and 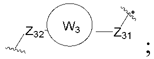 wherein the alkylene, methylene, ethylene, and alkynylene are optionally substituted by one or more substituents independently selected from halogen, oxo, -CN, -OH, -NH₂, methyl, and ethyl; wherein the phenyl and heteroaryl are optionally substituted by one or more substituents independently selected from halogen, -CN, methyl, ethyl, methoxy, and ethoxy; * is the connecting end of L₃ and A.

In one embodiment of the invention, L₃ is selected from -C₁₋₆alkylene-, -C₂₋₆alkynylene-, - methylene-phenyl-phenyl-methylene-, -methylene-6-membered heteroaryl-6-membered heteroaryl-methylene-, and 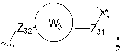 wherein the alkylene and methylene are optionally substituted by one or more substituents independently selected from F, Cl, Br, oxo, -CN, -OH, and -NH₂; wherein the phenyl and heteroaryl are optionally substituted by one or more substituents independently selected from F, Cl, Br, -CN, methyl, ethyl, methoxy, and ethoxy; * is the connecting end of L₃ and A.

In one embodiment of the invention, L₃ is selected from methylene, ethylene, n-propylidene, isopropylidene, n-butylene, n-pentylene, n-hexylene, n-propynylene, n-butynylene, n-pentynylene, n-hexynylidene, -methylene-phenyl-phenyl-methylene-, -methylene-pyridyl-pyridyl-methylene-, and 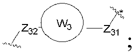 * is the connecting end of L₃ and A.

In one embodiment of the invention, L₃ is selected from 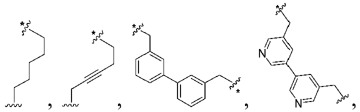 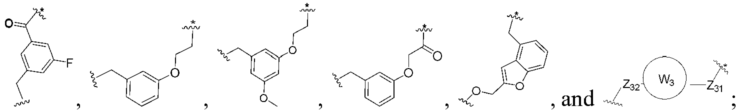 * is the connecting end of L₃ and A.

In one embodiment of the invention, L₃ is selected from 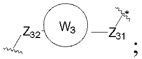 * is the connecting end of L₃ and A.

In one embodiment of the invention, each of rings W₁, W₂, and W₃ is independently selected from C₆₋₁₂aryl, 5-12-membered heteroaryl, and C₆₋₁₄cycloalkyl; wherein the aryl, heteroaryl, and cycloalkyl are optionally substituted by one or more substituents independently selected from deuterium, halogen, -CN, C₁₋₃alkyl, and C₁₋₃alkoxy.

In one embodiment of the invention, each of rings W₁, W₂, and W₃ is independently selected from C₆₋₁₀aryl, 5-10-membered heteroaryl, and C₆₋₁₀cycloalkyl; wherein the aryl, heteroaryl, and cycloalkyl are optionally substituted by one or more substituents independently selected from F, Cl, Br, -CN, methyl, ethyl, methoxy, and ethoxy.

In one embodiment of the invention, each of rings W₁, W₂, and W₃ is independently selected from phenyl, naphthyl, 8-10-membered bicyclic heteroaryl, C₈₋₁₀bicyclic cycloalkyl, and 5-6-membered monocyclic heteroaryl; wherein the phenyl, naphthyl, heteroaryl, and cycloalkyl are optionally substituted by one or more substituents independently selected from halogen, -CN, C₁₋₃alkyl, and C₁₋₃alkoxy.

In one embodiment of the invention, each of rings W₁, W₂, and W₃ is independently selected from phenyl, naphthyl, 5-membered/5-membered fused heteroaryl, 5-membered/6-membered fused heteroaryl, 6-membered/5-membered fused heteroaryl, 6-membered/6-membered fused heteroaryl, 4-membered/6-membered spirocycloalkyl, 6-membered/4-membered spirocycloalkyl, 5-membered/5-membered spirocycloalkyl, 5-membered/6-membered spirocycloalkyl, 6-membered/5-membered spirocycloalkyl, 6-membered/6-membered spirocycloalkyl, and 5-6-membered monocyclic heteroaryl; wherein the phenyl, naphthyl, fused heteroaryl, spirocycloalkyl, and monocyclic heteroaryl are optionally substituted by one or more substituents independently selected from F, Cl, Br, -CN, methyl, ethyl, methoxy, and ethoxy.

In one embodiment of the invention, each of rings W₁, W₂, and W₃ is independently selected from phenyl, naphthyl, 5-membered/5-membered fused heteroaryl, 5-membered/6-membered fused heteroaryl, 6-membered/5-membered fused heteroaryl, 6-membered/6-membered fused heteroaryl, 4-membered/6-membered spirocycloalkyl, 6-membered/4-membered spirocycloalkyl, and 5-6-membered monocyclic heteroaryl; wherein the phenyl, naphthyl, fused heteroaryl, spirocycloalkyl, and monocyclic heteroaryl are optionally substituted by one or more substituents independently selected from F, Cl, Br, methyl, ethyl, methoxy, and ethoxy.

In one embodiment of the invention, each of rings W₁, W₂, and W₃ is independently selected from phenyl and 5-6 membered monocyclic heteroaryl; wherein the phenyl and heteroaryl are optionally substituted by one or more substituents independently selected from deuterium, halogen, -CN, - OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl; wherein the heteroatom in the heteroaryl is O, N or S, and the number of heteroatoms is 1 or 2.

In one embodiment of the invention, each of ring W₁, W₂, and W₃ is independently selected from the following optionally substituted groups: 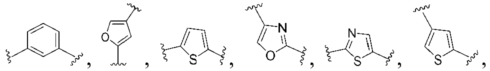 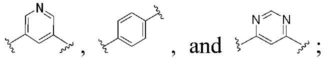 wherein "optionally substituted" refers to unsubstituted or substituted by one or more substituents independently selected from deuterium, halogen, -CN, - OH, -NH₂, -CHO, C₁-₃alkyl, C₁₋₃alkoxy, -N(C₁-₃alkyl)₂, -NH(C₁-₃alkyl), and -C(O)C₁-₃alkyl.

In one embodiment of the invention, each of rings W₁, W₂, and W₃ is independently selected from the following optionally substituted groups: 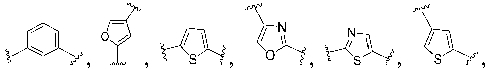 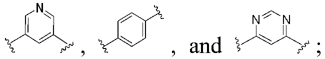 wherein "optionally substituted" refers to unsubstituted or substituted by one or more substituents independently selected from deuterium, F, Cl, Br, -CN, - OH, -NH₂, -CHO, methyl, ethyl, methoxy, ethoxy, -N(CH₃)₂, -NH(CH₃), and -C(O)CH₃.

In one embodiment of the invention, each of rings W₁, W₂, and W₃ is independently selected from phenyl and thienyl; wherein the phenyl and thienyl are optionally substituted by one or more substituents independently selected from deuterium, F, Cl, Br, -CN, -OH, -NH₂, -CHO, methyl, ethyl, methoxy, ethoxy, -N(CH₃)₂, -NH(CH₃), and -C(O)CH₃.

In one embodiment of the invention, each of rings W₁, W₂, and W₃ is independently selected from the following optionally substituted groups: 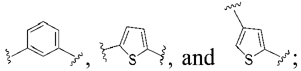 wherein "optionally substituted" refers to unsubstituted or substituted by one or more substituents independently selected from deuterium, F, Cl, Br, -CN, -OH, -NH₂, -CHO, methyl, ethyl, methoxy, ethoxy, - N(CH₃)₂, -NH(CH₃), and -C(O)CH₃.

In one embodiment of the invention, each of rings W₁, W₂, and W₃ is independently selected from phenyl; wherein the phenyl is optionally substituted by one or more substituents independently selected from F, Cl, Br, methyl, ethyl, methoxy, and ethoxy.

In one embodiment of the invention, each of W₁, W₂, and W₃ is independently selected from

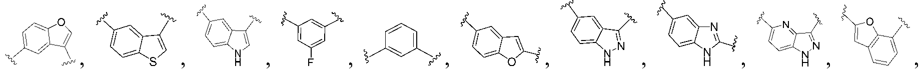

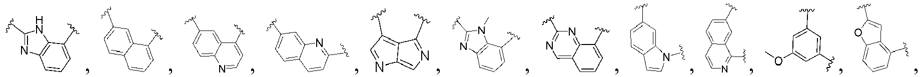

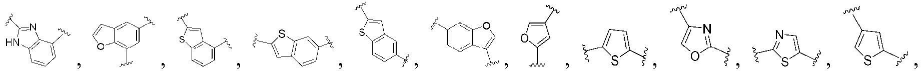

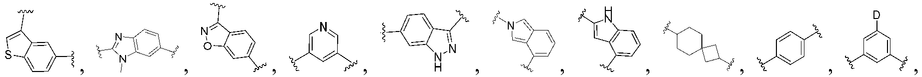

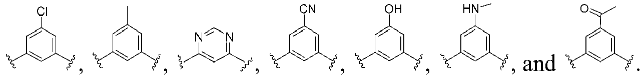

In one embodiment of the invention, each of Z₁₁, Z₁₂, Z₂₁, Z₂₂, Z₃₁, and Z₃₂ is independently selected from a bond, -O-, -S-, -NH-, -Se-, -C₁₋₄alkylene-, -C₁₋₆oxaalkylene-, -C₁₋₄thiaalkylene-, - C₁₋₄azaalkylene-, and -C₁₋₄selenaalkylene-; wherein the alkylene, oxaalkylene, thiaalkylene, azaalkylene, and selenaalkylene are optionally substituted by one or more substituents selected from Z₄₁; Z₄₁ is independently selected from deuterium, halogen (e.g., F, Cl), oxo, thio, -CN, -OH, -NH₂, C₁-₃alkyl, C₁-₃haloalkyl, C₁-₃alkoxy, and C₁-₃haloalkoxy; or any two Z₄₁ together with the atom to which they are attached form C₃₋₆cycloalkyl, and 3-6 membered heterocyclyl (e.g., oxetanyl); wherein the heteroatom in the heterocyclyl is O, N or S, and the number of heteroatoms is 1 or 2.

In one embodiment of the invention, each of Z₁₁, Z₁₂, Z₂₁, Z₂₂, Z₃₁, and Z₃₂ is independently selected from a bond, -C₁₋₃alkylene-, -C₁₋₃oxaalkylene-, -C₁₋₃thiaalkylene-, and -C₁₋₃azaalkylene-; wherein the alkylene, oxaalkylene, thiaalkylene, and azaalkylene are optionally substituted by one or more substituents independently selected from deuterium, halogen, oxo, -CN, -OH, -NH₂, methyl, and methoxy; preferably, wherein the alkylene, oxaalkylene, thiaalkylene, and azaalkylene are optionally substituted by one or more substituents independently selected from deuterium and oxo.

In one embodiment of the invention, each of Z₁₁, Z₁₂, Z₂₁, Z₂₂, Z₃₁, and Z₃₂ is independently selected from -C₁₋₂alkylene-, -C₁₋₂oxaalkylene-, -C₁₋₂thiaalkylene-, and -C₁₋₂azaalkylene-; wherein the alkylene, oxaalkylene, thiaalkylene, and azaalkylene are optionally substituted by one or more substituents independently selected from deuterium and oxo.

In one embodiment of the invention, each of Z₁₁, Z₁₂, Z₂₁, Z₂₂, Z₃₁, and Z₃₂ is independently selected from a bond, -C₁₋₃alkylene-, and -C₁₋₃oxaalkylene-; wherein the alkylene and oxaalkylene are optionally substituted by one or more substituents independently selected from deuterium, halogen, oxo, -CN, -OH, -NH₂, methyl, and methoxy; preferably, the alkylene and oxaalkylene are optionally substituted by one or more substituents independently selected from deuterium and oxo. In one embodiment of the invention, each of Z₁₁, Z₁₂, Z₂₁, Z₂₂, Z₃₁, and Z₃₂ is independently selected from -C₁₋₂alkylene-, -C₁₋₃oxaalkylene-, and -C₁₋₃azaalkylene-; wherein the alkylene, oxaalkylene, and azaalkylene are optionally substituted by one or more substituents independently selected from oxo.

In one embodiment of the invention, when Z₁₁, Z₂₁, and Z₃₁ are present in the compound at the same time, in a combination of the definitions of Z₁₁, Z₂₁, and Z₃₁, at least one heteroatom or heteroatom group is present, and the heteroatom or heteroatom group is selected from -O-, -S-, - Se-, -NH-, -CO-, and -C(S)-;.

In one embodiment of the invention, when Z₁₁, Z₂₁, and Z₃₁ are present in the compound at the same time, in a combination of the definitions of Z₁₁, Z₂₁, and Z₃₁, at least one heteroatom or heteroatom group is present, and the heteroatom or heteroatom group is selected from -CO- or - C(S)-.

In one embodiment of the invention, when Z₁₁, Z₂₁, and Z₃₁ are present in the compound at the same time, in a combination of the definitions of Z₁₁, Z₂₁, and Z₃₁, at least one group selected from 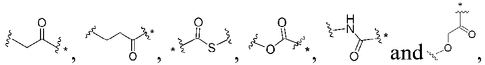 preferably 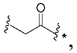 is present; * represents the connecting end with A.

In one embodiment of the invention, when Z₁₁, Z₂₁, and Z₃₁ are present in the compound at the same time, in a combination of the definitions of Z₁₁, Z₂₁, and Z₃₁, at least one heteroatom or heteroatom group is present, and the heteroatom or heteroatom group is selected from -O-, -NH-, and -CO-.

In one embodiment of the invention, when Z₁₁, Z₂₁, and Z₃₁ are present in the compound at the same time, in a combination of the definitions of Z₁₁, Z₂₁, and Z₃₁, 1-4 heteroatoms or heteroatom groups are present, the heteroatom or heteroatom group is selected from -O-, -S-, -Se-, -NH-, -CO-, and -C(S)-, and the number of heteroatoms or heteroatom groups is specifically 1, 2, 3, or 4; preferably 1-3 heteroatoms or heteroatom groups are present, and the number thereof is specifically 1, 2, or 3; or preferably 2-4 heteroatoms or heteroatom groups are present, and the number thereof is 2, 3, or 4; further preferably 2 or 3 heteroatoms or heteroatom groups are present; the heteroatom or heteroatom group is preferably -O-, -S-, -NH-, or -CO-; the heteroatom or heteroatom group is more preferably -O-, or -CO-; preferably, the heteroatom or heteroatom group at least comprises -CO- or -C(S)-.

In one embodiment of the invention, Z₂₁ contains a heteroatom or heteroatom group selected from -O-, -S-, -Se-, and -NH-; and Z₃₁ contains a heteroatom or heteroatom group selected from -CO-and -C(S)-; and the total number of the heteroatoms or heteroatom groups in the combination of Z₂₁ and Z₃₁ is 1-4;

In one embodiment of the invention, when Z₁₁, Z₂₁, and Z₃₁ are present in the compound at the same time, in a combination of the definitions of Z₁₁, Z₂₁, and Z₃₁, 1-4, preferably 1-3 and more preferably 2-3 heteroatoms or heteroatom groups are present, and the heteroatom or heteroatom group is selected from -O-, -NH-, and -CO-; preferably, the heteroatom or heteroatom group at least comprises -CO-.

In one embodiment of the invention, Z₁₁, Z₂₁ and Z₃₁ together contain 1-4 heteroatoms or heteroatom groups, wherein the heteroatoms or heteroatom groups are selected from -O-, -NH-, - CO-, and the number of heteroatoms or heteroatom groups is 1, 2, 3, and 4.

In one embodiment of the invention, Z₁₁, Z₂₁ and Z₃₁ together contain 1-3 heteroatoms or heteroatom groups, wherein the heteroatoms or heteroatom groups are selected from -O-, -NH-, and -CO-, specifically 1, 2, and 3.

In one embodiment of the invention, Z₁₁, Z₂₁ and Z₃₁ together contain 2-3 heteroatoms or heteroatom groups, wherein the heteroatoms or heteroatom groups are selected from -O-, -NH-, and -CO-, and the number of heteroatoms or heteroatom groups is 2 and 3; preferably, the heteroatoms or heteroatom groups contain at least -CO-.

In one embodiment of the invention, each of Z₁₁, Z₁₂, Z₂₁, Z₂₂, Z₃₁,and Z₃₂ is independently selected from -CD₂-, -C₁₋₄alkylene-, -OC₁₋₃alkylene-, -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₂alkylene-O-C₁₋₃alkylene-, -NH-C₁₋₃alkylene-, -S-C₁₋₃alkylene-, -C₁₋₃alkylene-NH-C(O)-, -C₁₋₃alkylene-O-C(O)-, -NH-C(O)-, -O-C(O)-, -S-C(O)-, -C₁₋₃alkylene-C(S)-, -Se-C₁₋₃alkylene-, 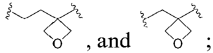 wherein the alkylene, methylene, and ethylene are optionally substituted by one or more substituents independently selected from deuterium, F, and Cl.

In one embodiment of the invention, each of Z₁₁, Z₁₂, Z₂₁, Z₂₂, Z₃₁, and Z₃₂ is independently selected from -CD₂-, -C₁₋₃alkylene-, -OC₁₋₃alkylene-, -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₃alkylene-C(O)-C₁₋₃alkylene-, -C₁₋₃alkylene-O-C₁₋₃alkylene-, -NH-C₁₋₃alkylene-, -S-C₁₋₃alkylene-, -C₁₋₃alkylene-NH-C(O)-, -C₁₋₃alkylene-O-C(O)-, -NH-C(O)-, -O-C(O)-, and -S-C(O)-.

In one embodiment of the invention, each of Z₁₁, Z₁₂, Z₂₁, Z₂₂, Z₃₁, and Z₃₂ is independently selected from a bond, -CD₂-, -C₁₋₃alkylene-, -OC₁₋₃alkylene-, -C(O)-, -C₁₋₃alkylene-C(O)-, -OC₁₋₃alkylene-C(O)-, -NH-C₁₋₃alkylene-C(O)-, -S-C₁₋₃alkylene-C(O)-, and -O-C₁₋₃alkylene-C(O)-C₁₋₃alkylene-.

In a preferred embodiment of the invention, each of Z₁₁, Z₁₂, Z₂₁, Z₂₂, Z₃₁, and Z₃₂ is independently selected from -C₁₋₃alkylene-, -OC₁₋₃alkylene-, -C₁₋₃alkylene-C(O)-, -OC₁₋₃alkylene-C(O)-, -NH-C(O)-, and -O-C(O)-.

In one embodiment of the invention, each of Z₁₁, Z₁₂, Z₂₁, Z₂₂, Z₃₁, and Z₃₂ is independently selected from -CD₂-, -C₁₋₂alkylene-, -OC₁₋₃alkylene-, -C(O)-, -C₁₋₂alkylene-C(O)-, -OC₁₋₂alkylene-C(O)-, -NH-C₁₋₂alkylene-C(O)-, -S-C₁₋₂alkylene-C(O)-, -O-C₁₋₂alkylene-C(O)-C₁₋₂alkylene-, -C₁₋₂alkylene-O-C₁₋₂alkylene-, -NH-C₁₋₂alkylene-, -S-C₁₋₂alkylene-, -C₁₋₂alkylene-NH-C(O)-, -C₁₋₂alkylene-O-C(O)-, -NH-C(O)-, -O-C(O)-, and -S-C(O)-.

In one embodiment of the invention, each of Z₁₁, Z₁₂, Z₂₁, Z₂₂, Z₃₁, and Z₃₂ is independently selected from a bond, -CD₂-, -C₁₋₂alkylene-, -OC₁₋₂alkylene-, -C(O)-, -C₁₋₂alkylene-C(O)-, -OC₁₋₂alkylene-C(O)-, -NH-C₁₋₂alkylene-C(O)-, -S-C₁₋₂alkylene-C(O)-, and -O-C₁₋₂alkylene-C(O)-C₁₋₂alkylene-.

In one embodiment of the invention, each of Z₁₁, Z₁₂, Z₂₁, Z₂₂, Z₃₁, and Z₃₂ is independently selected from a bond, methylene, ethylene, -CD₂-, -O-, -S-, -NH-,

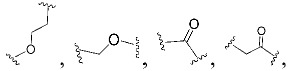

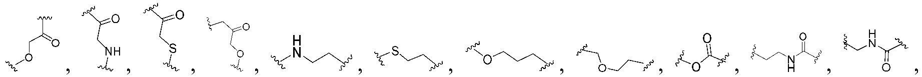

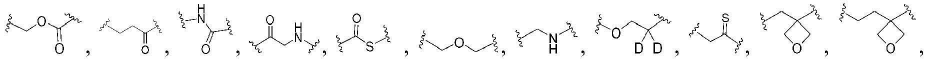

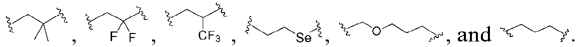

In one embodiment of the invention, each of Z₁₁, Z₁₂, Z₂₁, Z₂₂, Z₃₁, and Z₃₂ is independently selected from methylene, ethylene,

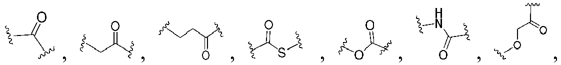

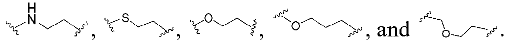

In one embodiment of the invention, each of Z₁₁, Z₁₂, Z₂₁, Z₂₂, Z₃₁, and Z₃₂ is independently selected from methylene, ethylene, 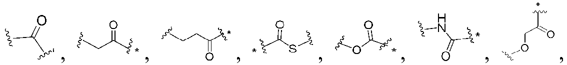 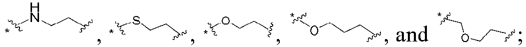 * represents the connecting end with A.

In one embodiment of the invention, each of Z₁₁, Z₁₂, Z₂₁, Z₂₂, Z₃₁, and Z₃₂ is independently selected from methylene, ethylene, -CD₂-, 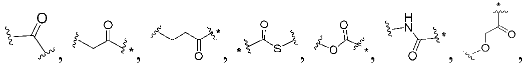 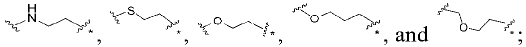 * represents the connecting end with A.

In one embodiment of the invention, each of Z₁₂, Z₂₂ and Z₃₂ is independently selected from methylene, and -CD₂, and Z₁₁ is selected from methylene, ethylene, -CD₂-, 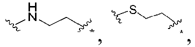 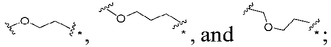 and Z₂₁ is selected from methylene, ethylene, -CD₂-, 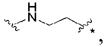 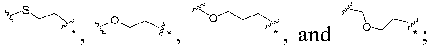 and Z₃₁ is selected from 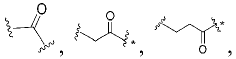 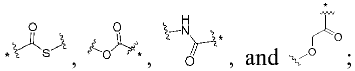 further preferably Z₃₁ is selected from 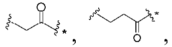 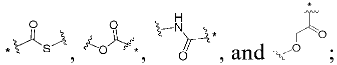 * represents the connecting end with A.

In one embodiment of the invention, each of Z₁₂, Z₂₂ and Z₃₂ is independently selected from methylene, -CD₂, and Z₁₁ is selected from methylene, ethylene, -CD₂-, and 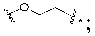 **and** Z₂₁ is selected from methylene, ethylene, -CD₂-, 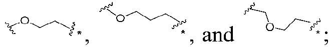 and Z₃₁ is selected from 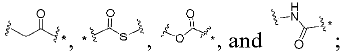 * represents the connecting end with A.

In one embodiment of the invention, each of Z₁₁, Z₁₂, Z₂₁, Z₂₂, Z₃₁, and Z₃₂ is independently selected from methylene, ethylene,

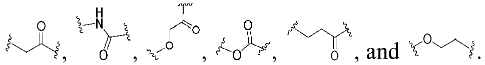

In one embodiment of the invention, each of Z₁₁, Z₁₂, Z₂₁, and Z₂₂ is independently selected from -CD₂- and methylene, preferably methylene.

In one embodiment of the invention, each of Z₁₂, Z₂₂, and Z₃₂ is independently selected from - CD₂-, methylene and ethylene.

In one embodiment of the invention, Z₁₂, Z₂₂, and Z₃₂ are all -CD₂- or methylene, preferably methylene.

In one embodiment of the invention, Z₁₁ is selected from -CD₂-, -C₁₋₃alkylene- and -OC₁₋₃alkylene-, and preferably -C₁₋₃alkylene-; preferably, it is selected from -CD₂-, methylene, ethylene, methyleneoxy, and ethyleneoxy, and more preferably methylene.

In one embodiment of the invention, Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; preferably -OC₁₋₃alkylene-, - NH-C₁₋₂alkylene-, -S-C₁₋₂alkylene-, and -C₁₋₂alkylene-O-C₁₋₃alkylene-.

In one embodiment of the invention, Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-; it is preferably selected from -C(O)-, -C₁₋₂alkylene-C(O)-, -OC₁₋₂alkylene-C(O)-, -NH-C(O)-, -O-C(O)-, and -S-C(O)-, preferably -C₁₋₂alkylene-C(O)-, -OC₁₋₂alkylene-C(O)-, -NH-C(O)-, -O-C(O)-, and -S-C(O)-; and preferably -CH₂C(O)-.

In one embodiment of the invention, Z₁₁ is independently selected from -OC₁₋₃alkyl-, -NH-C₁₋₃alkyl-, -S-C₁₋₃alkyl-, -Se-C₁₋₃alkyl-, and -C₁₋₃alkyl-O-C₁₋₃alkyl-; and Z₂₁ is independently selected from -OC₁₋₃alkyl-, -NH-C₁₋₃alkyl-, -S-C₁₋₃alkyl-, -Se-C₁₋₃alkyl-, and -C₁₋₃alkyl-O-C₁₋₃alkyl-.

In one embodiment of the invention, Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; and Z₃₁ is selected from - C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkyl-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-.

In one embodiment of the invention, Z₁₁ is selected from -CD₂, -C₁₋₃alkylene-, and -OC₁₋₃alkylene-; and Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; and Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-.

In one embodiment of the invention, Z₁₁ is selected from -CD₂-, -C₁₋₃alkylene- and -OC₁₋₃alkylene-; and Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; and Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-.

In one embodiment of the invention, Z₁₁ is selected from -C₁₋₃alkylene- and -OC₁₋₃alkylene-; and Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; and Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-.

In one embodiment of the invention, Z₁₁ is selected from -CD₂, -C₁₋₃alkylene-, and -OC₁₋₃alkylene-*; and Z₂₁ is selected from -OC₁₋₃alkylene-*, -NH-C₁₋₃alkylene-*, -S-C₁₋₃alkylene-*, -Se-C₁₋₃alkylene-*, and -C₁₋₂alkylene-O-C₁₋₃alkylene-*; and Z₃₁ is selected from -C(O)-, -C₁₋₃alkylene-C(O)-*, -OC₁₋₃alkylene-C(O)-*, -NH-C₁₋₃alkylene-C(O)-*, -S-C₁₋₃alkylene-C(O)-*, -O-C₁₋₂alkylene-C(O)-C₁₋₃alkylene-*, -C₁₋₃alkylene-NH-C(O)-*, -C₁₋₃alkylene-O-C(O)-*, -NH-C(O)-*, -O-C(O)-*, -S-C(O)-*, and -C₁₋₃alkylene-C(S)-*; * represents the connecting end with A.

In one embodiment of the invention, Z₁₁ is selected from -CD₂-, -C₁₋₃ alkylene- and -OC₁₋₃alkylene-*; and Z₂₁ is selected from -OC₁₋₃alkylene-*, -NH-C₁₋₃alkylene-*, -S-C₁₋₃alkylene-*, - Se-C₁₋₃alkylene-*, and -C₁₋₂alkylene-O-C₁₋₃alkylene-*; and Z₃₁ is selected from -C(O)-, -C₁₋₃alkylene-C(O)-*, -OC₁₋₃alkylene-C(O)-*, -NH-C₁₋₃alkylene-C(O)-*, -S-C₁₋₃alkylene-C(O)-*, -O-C₁₋₂alkylene-C(O)-C₁₋₃alkylene-*, -C₁₋₃alkylene-NH-C(O)-*, -C₁₋₃alkylene-O-C(O)-*, -NH-C(O)- *, -O-C(O)-*, -S-C(O)-*, and -C₁₋₃alkylene-C(S)-*; * represents the connecting end with A. In one embodiment of the invention, Z₁₁ is selected from -C₁₋₃ alkylene- and -OC₁₋₃alkylene-*; and Z₂₁ is selected from -OC₁₋₃alkylene-*, -NH-C₁₋₃alkylene-*, -S-C₁₋₃alkylene-*, -Se-C₁₋₃alkylene-*, and -C₁₋₂alkylene-O-C₁₋₃alkylene-*; and Z₃₁ is selected from -C(O)-, -C₁₋₃alkylene-C(O)-*, -OC₁₋₃alkylene-C(O)-*, -NH-C₁₋₃alkylene-C(O)-*, -S-C₁₋₃alkylene-C(O)-*, -O-C₁₋₂alkylene-C(O)-C₁₋₃alkylene-*, -C₁₋₃alkylene-NH-C(O)-*, -C₁₋₃alkylene-O-C(O)-*, -NH-C(O)- *, -O-C(O)-*, -S-C(O)-*, and -C₁₋₃alkylene-C(S)- *; * represents the connecting end with A.

In one embodiment of the invention, Z₁₁ is selected from -CD₂-, -C₁₋₂alkylene-, and -OC₁₋₂alkylene-*; and Z₂₁ is selected from -OC₁₋₂alkylene-*, -NH-C₁₋₂alkylene-*, -S-C₁₋₂alkylene-*, - Se-C₁₋₂alkylene-*, and -CH₂-O-C₁₋₂alkylene-*; and Z₃₁ is selected from -C(O)-, -C₁₋₂alkylene-C(O)-*, -OC₁₋₂alkylene-C(O)-*, -NH-C₁₋₂alkylene-C(O)-*, -S-C₁₋₂alkylene-C(O)-*, -C₁₋₂alkylene-NH-C(O)-*, -C₁₋₂alkylene-O-C(O)-*, -NH-C(O)-*, -O-C(O)-*, -S-C(O)-*, and -C₁₋₂alkylene-C(S)-*; further preferably, Z₃₁ is selected from -C₁₋₂alkylene-C(O)-*, -OC₁₋₂alkylene-C(O)-*, -NH-C₁₋₂alkylene-C(O)-*, -S-C₁₋₂alkylene-C(O)-*, -C₁₋₂alkylene-NH-C(O)-*, -C_{1 2}alkylene-O-C(O)-*, -CH₂NH-C(O)-*, -NH-C(O)-*, -O-C(O)-*, -S-C(O)-*, and -C₁₋₂alkylene-C(S)-*; further preferably, Z₃₁ is selected from -C₁₋₂alkylene-C(O)-*, -OCH₂-C(O)-*, -CH₂-O-C(O)-*, -NH-C(O)-*, -O-C(O)-*, and -S-C(O)-*; further preferably, Z₃₁ is selected from - CH₂C(O)-*; * represents the connecting end with A.

In one embodiment of the invention, each of L₁ and L₂ is independently selected from 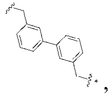 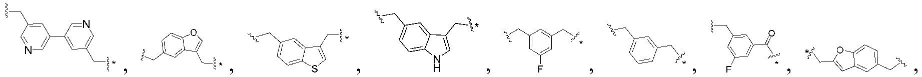 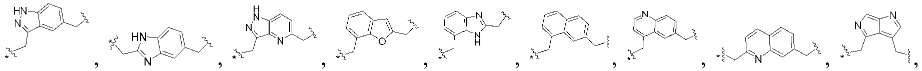 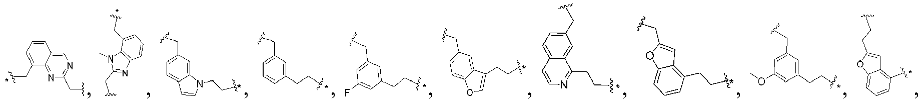 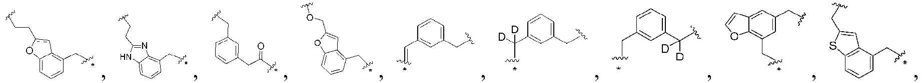 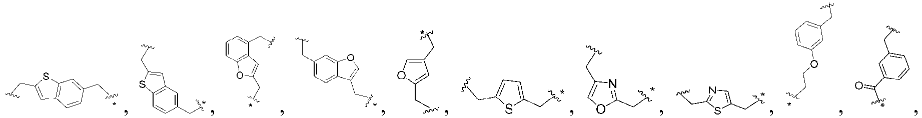 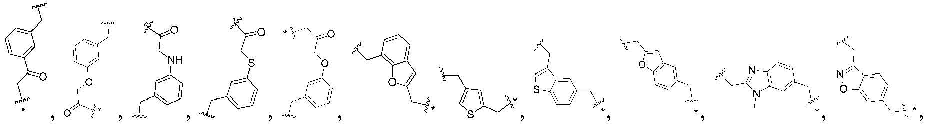 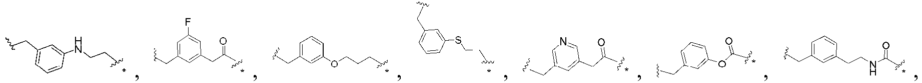 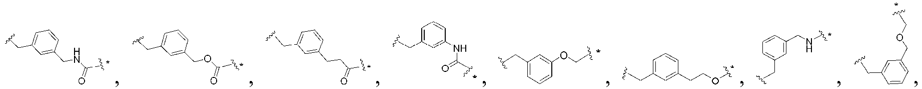 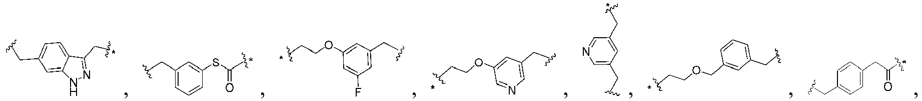 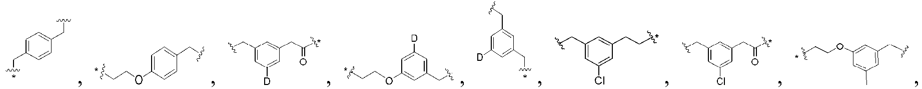 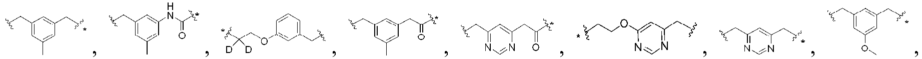 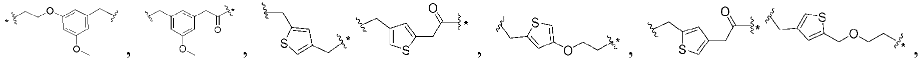 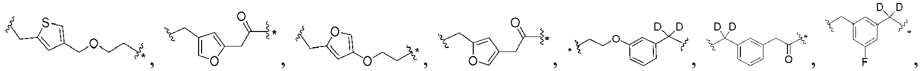 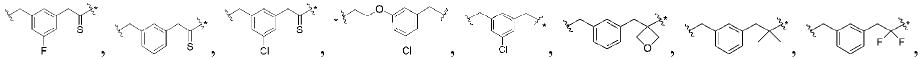 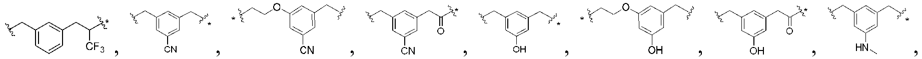 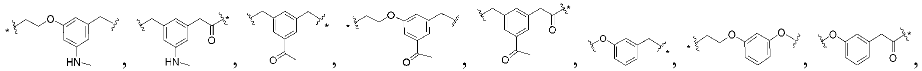 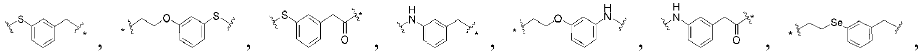 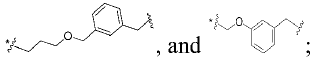 * represents the connecting end with A.

In one embodiment of the invention, each of L₁ and L₂ is independently selected from 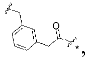 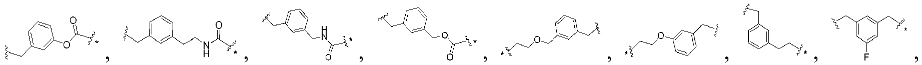 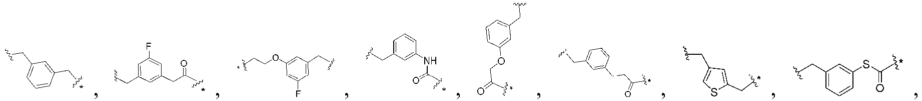 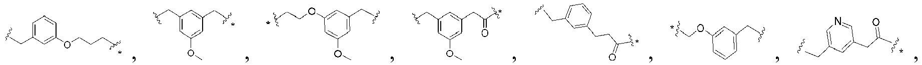 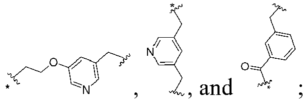 * represents the connecting end with N (i.e. A).

In one embodiment of the invention, L₃ is selected from 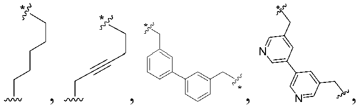 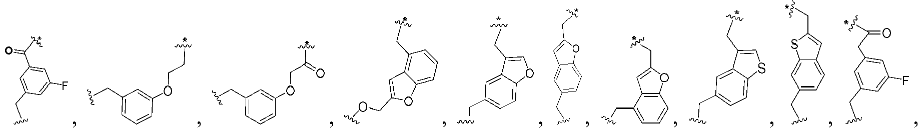 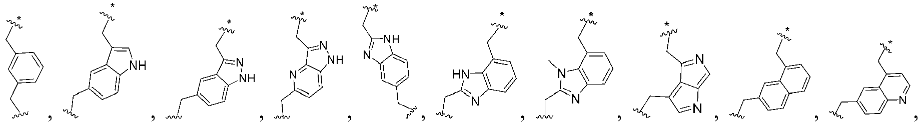 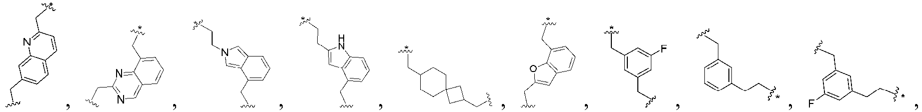 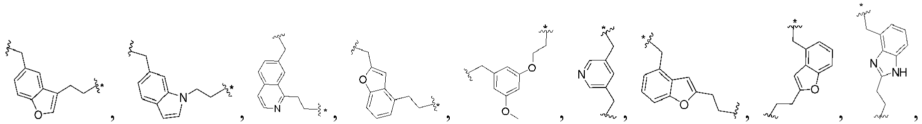 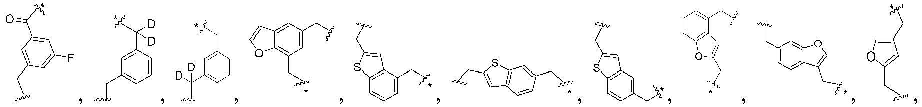 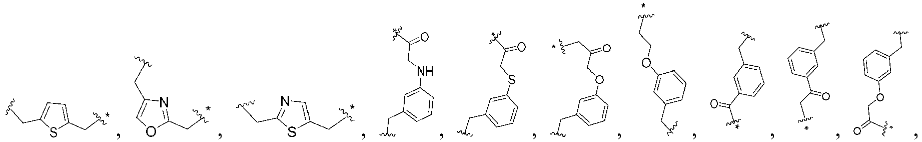 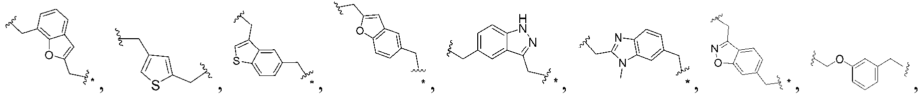 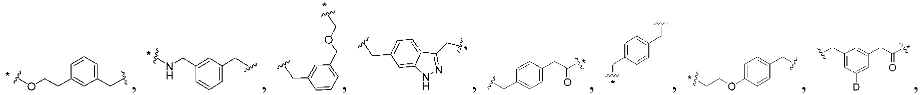 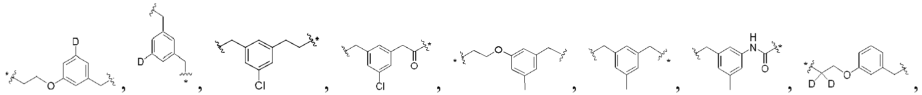 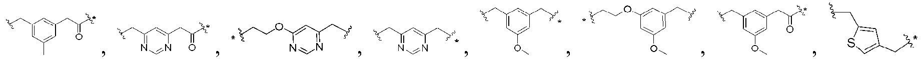 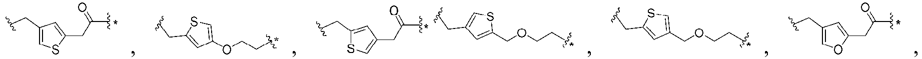 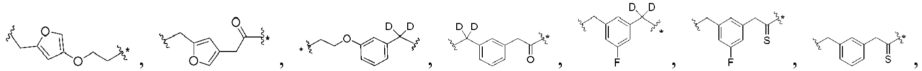 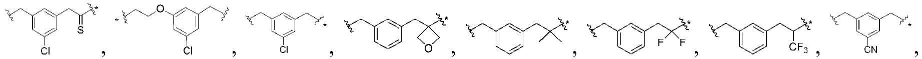 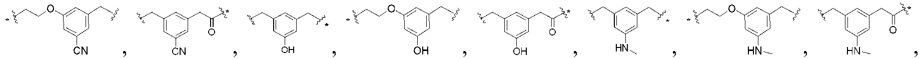 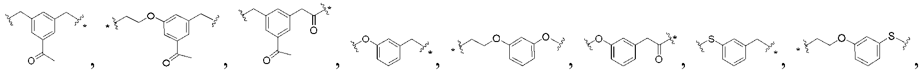 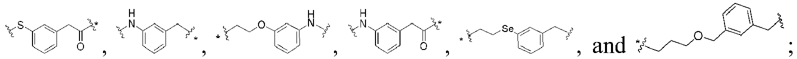 * represents the connecting end with A.

In one embodiment of the invention, each of L₁, L₂, and L₃ is independently selected from 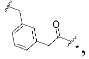 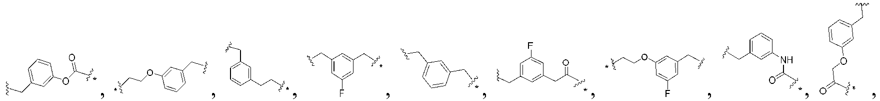 and 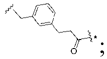 * represents the connecting end with N(i.e. A).

In one embodiment of the invention, R is selected from H, 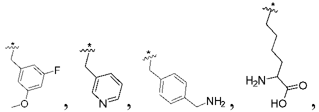 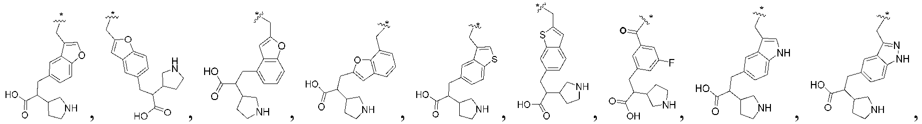 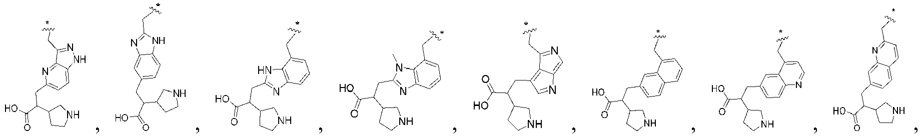 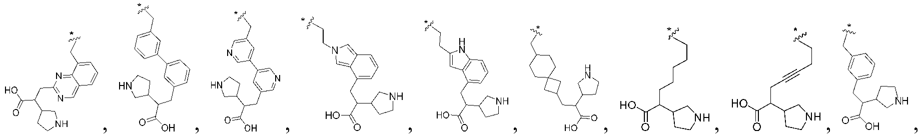 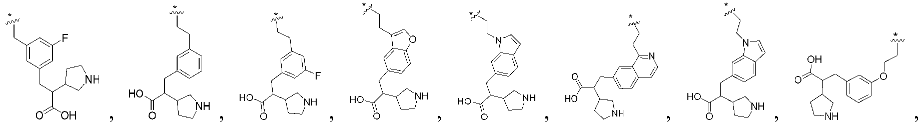 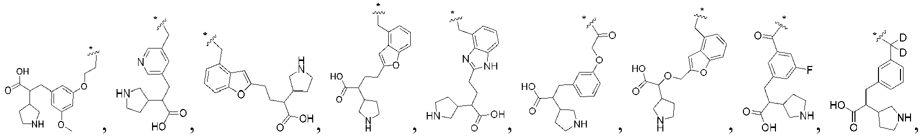 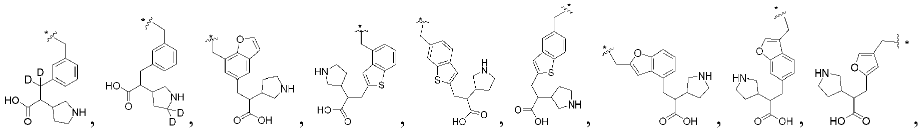 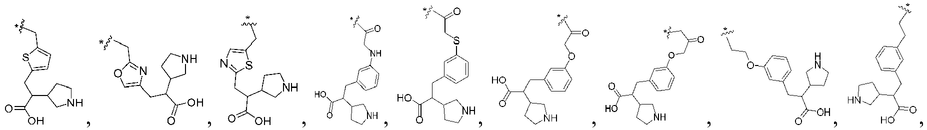 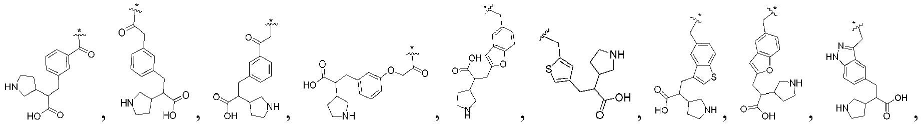 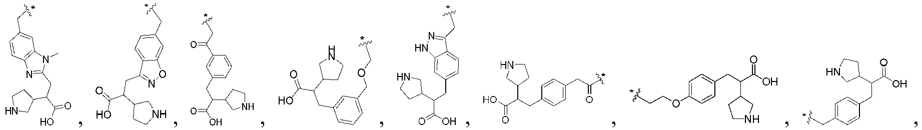 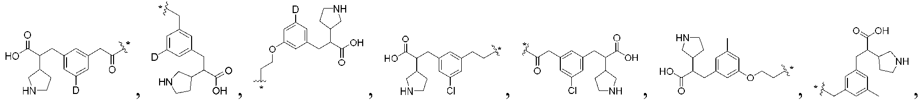 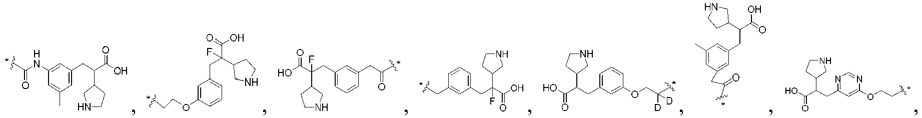 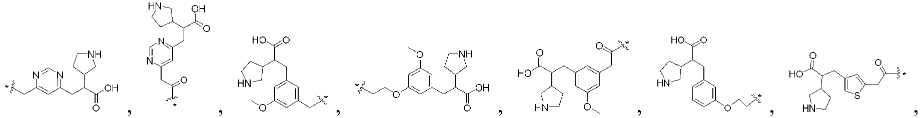 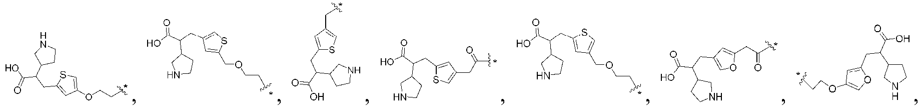 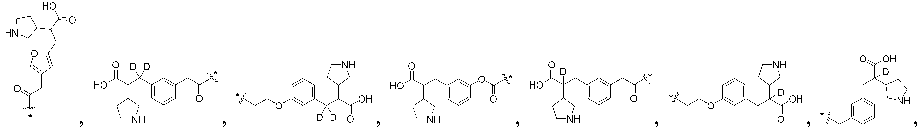 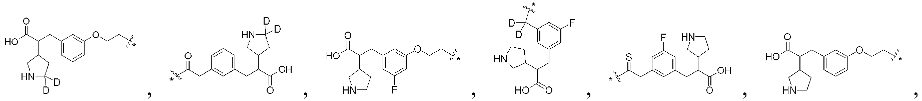 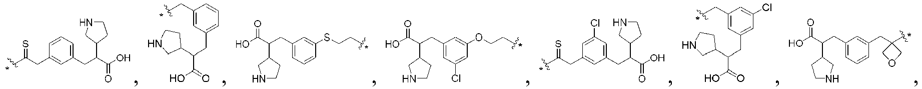 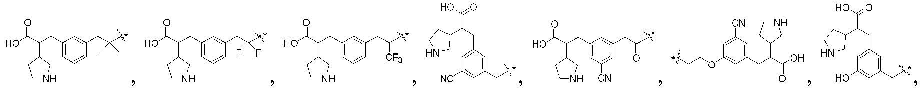 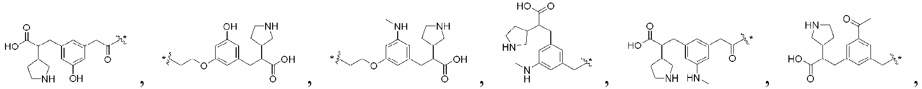 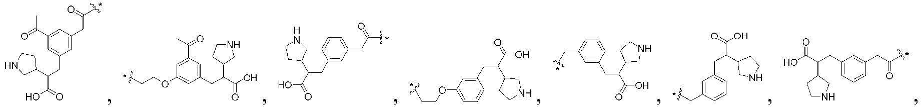 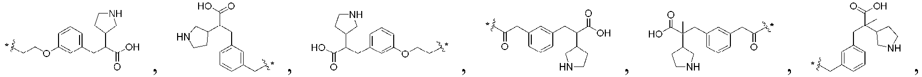 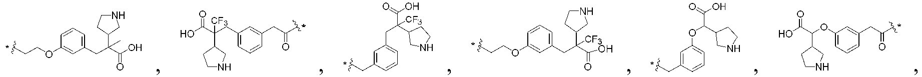 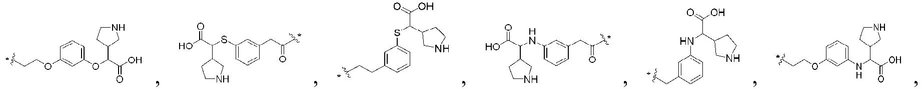 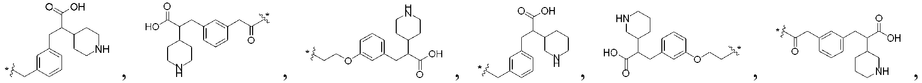 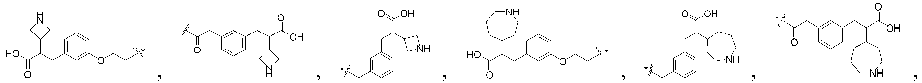 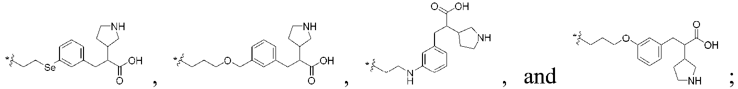 * represents the connecting end with A.

In one embodiment of the invention, R is selected from H, 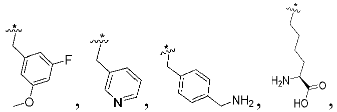 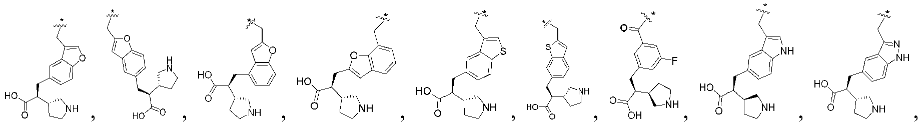 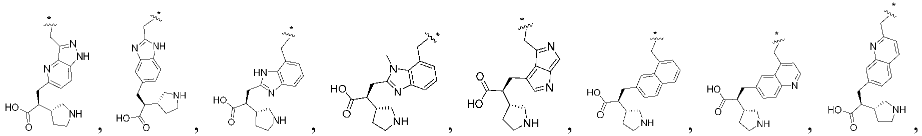 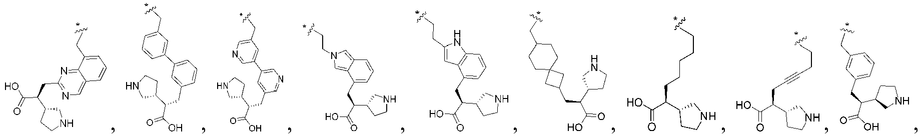 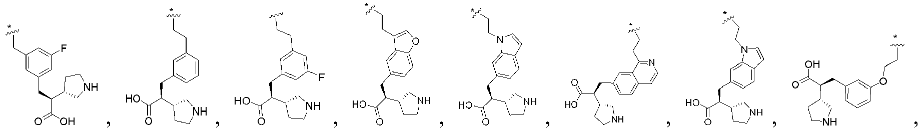 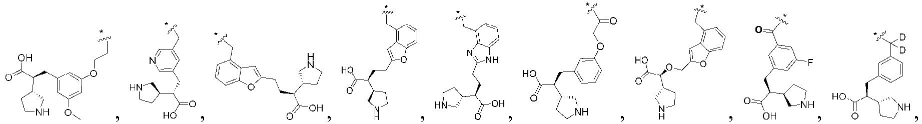 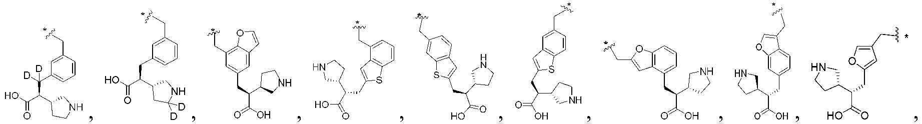 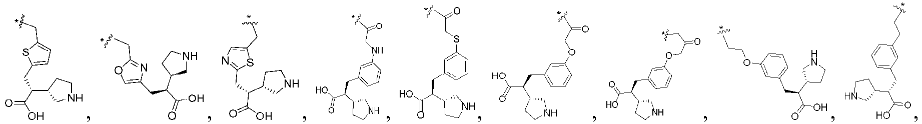 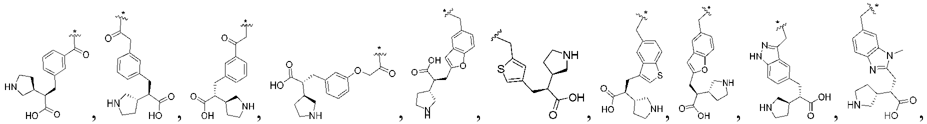 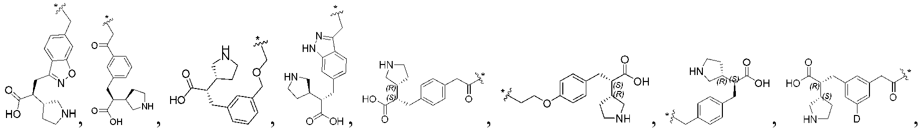 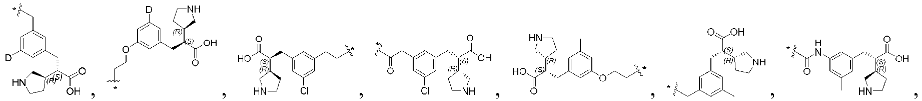 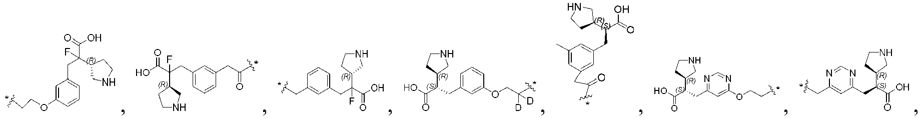 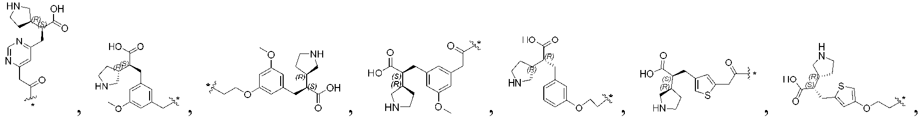 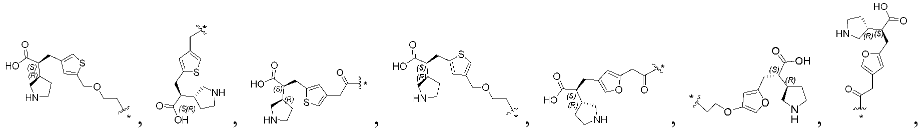 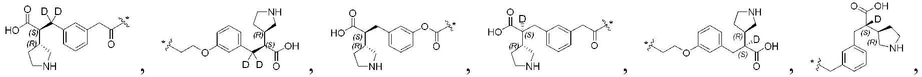 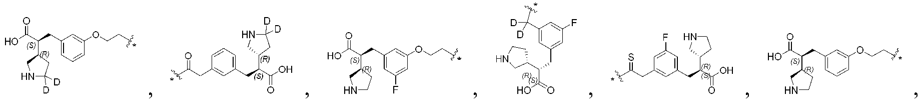 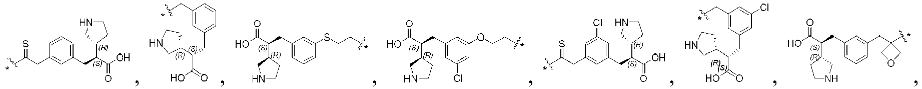 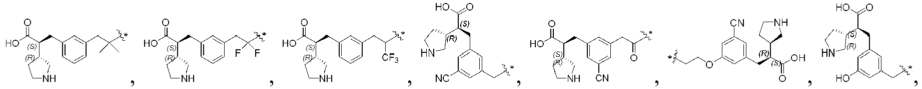 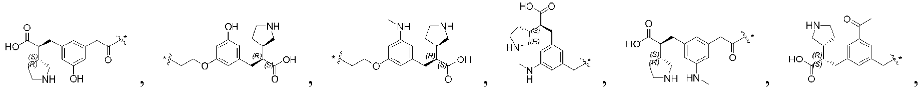 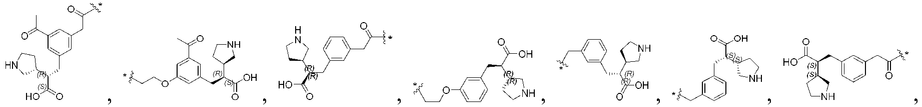 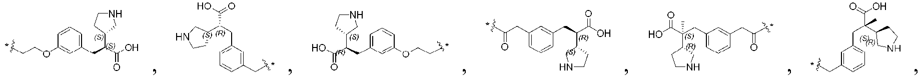 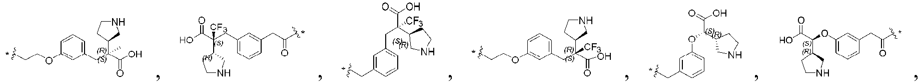 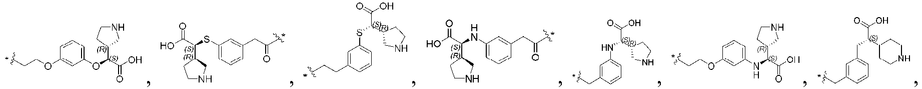 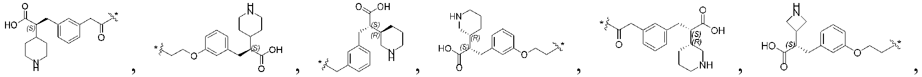 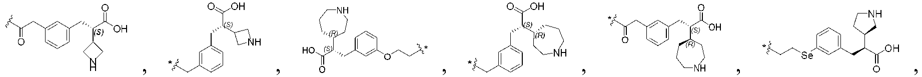 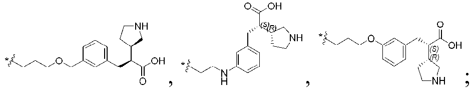 * represents the connecting end with A.

Firstly, the invention provides a compound represented by the following Formula (I'-1), Formula (I'-2), or Formula (I'-3) or a pharmaceutically acceptable salt thereof: 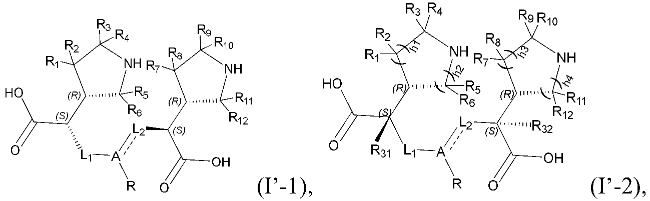 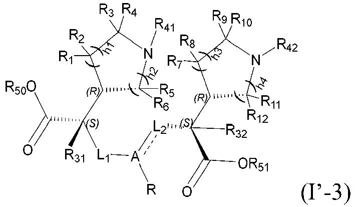 wherein A, L₁, L₂, R, R₁, R₂, R₃, R₄, R₅, R₆, R₇, R₈, R₉, R₁₀, R₁₁, R₁₂, R₃₁, R₃₂, R₄₁, R₄₂, R₅₀, R₅₁, h1, h2, h3, and h4 are defined as in the compound of Formula (I), Formula (I-1-P1), Formula (I-1-P2), Formula (I-1-P3), or Formula (I-1-P4).

In an embodiment for the compound represented by the above Formula (I'-1), (I'-2), or (I'-3), R is a group containing L₃; and one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, - C(O)-, -CH₂C(O)-, -NHC(O)-, -OC(O)-, -CH₂CH₂O-, -OCH₂C(O)-, -CH₂OC(O)-, - CH₂CH₂CH₂O-, and -S-C(O)-. The invention also provides a compound represented by the following Formula (II), or a pharmaceutically acceptable salt thereof: 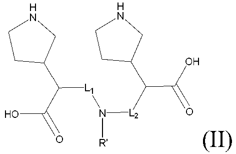 wherein, L₁ and L₂ are defined as in the compound of Formula (I), Formula (I-1-P1), Formula (I-1-P2), Formula (I-1-P3), or Formula (I-1-P4);

R' is selected from H, -C₁-₃alkylene-C₆₋₁₀aryl, -C₁-₃alkylene-5-12 membered heteroaryl, and C₁₋₆alkyl; wherein the C₁-₃alkylene is optionally substituted by one or more substituents independently selected from deuterium, halogen, oxo, -CN, -OH, -NH₂, and C₁-₃alkyl; wherein the aryl and heteroaryl are optionally substituted by one or more substituents independently selected from deuterium, halogen, -CN, C₁-₃alkoxy, and C₁-₃aminoalkyl; wherein the C₁₋₆alkyl is optionally substituted by one or more substituents independently selected from deuterium, halogen, oxo, carboxyl, -CN, -OH, -NH₂, C₁-₃alkyl, and C₁₋₃alkoxy.

In one embodiment of the invention, R' is selected from H, -C₁₋₃alkylene-C₆₋₁₀aryl, -C₁₋₃alkylene-5-10 membered heteroaryl, and C₁₋₆alkyl; wherein the C₁₋₃alkylene is optionally substituted by one or more substituents independently selected from deuterium, halogen, oxo, -CN, -OH, and -NH₂; wherein the aryl and heteroaryl are optionally substituted by one or more substituents independently selected from deuterium, halogen, -CN, methoxy, and C₁₋₃aminoalkyl; and wherein the C₁₋₆alkyl is optionally substituted by one or more substituents independently selected from deuterium, halogen, oxo, carboxyl, -CN, -OH, -NH₂, C₁₋₃alkyl, and C₁₋₃alkoxy;
L₁ is 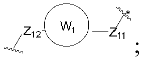 * represents the connecting end with N;
L₂ is 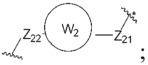 * represents the connecting end with N;
each of rings W₁ and W₂ is independently selected from C₆₋₁₂aryl and 5-12-membered heteroaryl; wherein the aryl and heteroaryl are optionally substituted by one or more substituents independently selected from deuterium, halogen, -CN, C₁₋₃alkyl, and C₁₋₃alkoxy;
each of Z₁₁, Z₁₂, Z₂₁, and Z₂₂ is independently selected from -C₁₋₃alkylene- and -C₁₋₃oxaalkylene-; wherein the alkylene and oxaalkylene are optionally substituted by one or more substituents independently selected from deuterium, halogen, oxo, -CN, -OH, -NH₂, methyl, and methoxy.

In one embodiment of the invention, R' is selected from H, -C₁-₃alkylene-phenyl, -methylene-5-6 membered heteroaryl, and C₁₋₆alkyl; wherein the methylene and C₁-₃alkylene are optionally substituted by one or more substituents independently selected from halogen, oxo, -CN, -OH, - NH₂, and C₁-₃alkyl; wherein the phenyl and heteroaryl are optionally substituted by one or more substituents independently selected from halogen, -CN, C₁-₃alkoxy, and C₁-₃aminoalkyl; wherein the C₁₋₆alkyl is optionally substituted by one or more substituents independently selected from halogen, oxo, carboxyl, -CN, -OH, -NH₂, C₁-₃alkyl, and C₁₋₃alkoxy.

In one embodiment of the invention, R' is selected from H, -methylene-phenyl, -ethylene-phenyl, -methylene-pyridinyl, C₁₋₆alkyl; wherein the phenyl and pyridinyl are optionally substituted by one or more substituents independently selected from halogen, -CN, methoxy, and 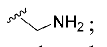 wherein the C₁₋₆alkyl is optionally substituted by one or more substituents independently selected from carboxyl and -NH₂.

In one embodiment of the invention, R' is selected from H, 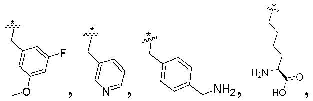 and

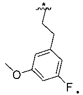

In one embodiment of the invention, each of rings W₁ and W₂ is independently selected from phenyl and 5-12-membered heteroaryl; wherein the phenyl and heteroaryl are optionally substituted by one or more substituents independently selected from halogen and -CN.

In one embodiment of the invention, each of rings W₁ and W₂ is independently selected from phenyl and 8-10-membered bicyclic heteroaryl; wherein the phenyl and bicyclic heteroaryl are optionally substituted by one or more substituents independently selected from halogen and -CN; wherein the heteroatoms in the bicyclic heteroaryl are independently selected from O, N or S, and the number of heteroatoms is 1 or 2.

In one embodiment of the invention, each of rings W₁ and W₂ is independently selected from

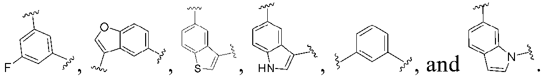

In one embodiment of the invention, each of Z₁₁, Z₁₂, Z₂₁, and Z₂₂ is independently selected from -C₁₋₃alkylene- and -C₁₋₃oxaalkylene-; wherein the alkylene and oxaalkylene are optionally substituted by one or more substituents independently selected from deuterium and oxo.

In one embodiment of the invention, each of Z₁₁, Z₁₂, Z₂₁, and Z₂₂ is independently selected from methylene, ethylene,

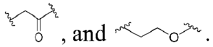

In one embodiment of the invention, each of L₁ and L₂ is independently selected from 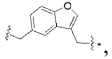 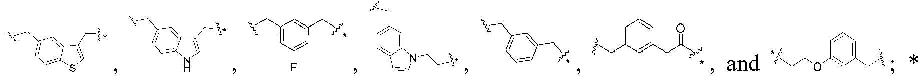 represents the connecting end with N.

The invention also provides a compound represented by the following Formula (II-A), (II-B), (II-C), (II-A'), (II-B'), or (II-C'), or a pharmaceutically acceptable salt thereof: 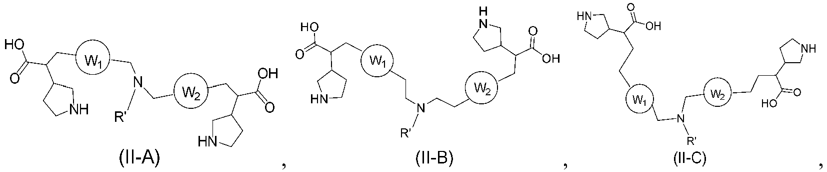 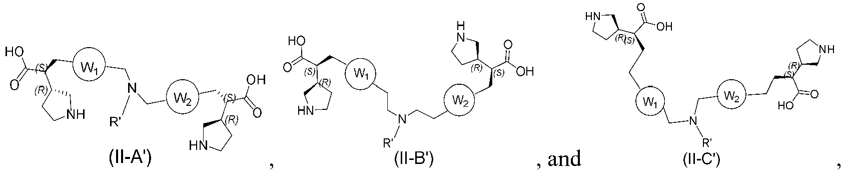 wherein, W₁, W₂, and R' are defined as in the compound of Formula (I), Formula (I-1-P 1), Formula (I-1-P2), Formula (I-1-P3), Formula (I-1-P4) or Formula (II).

The invention also provides a compound represented by the following Formula (III), or a pharmaceutically acceptable salt thereof: 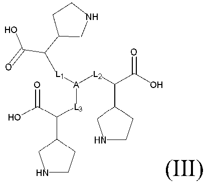 wherein, A, L₁, L₂, and L₃ are defined as in the compound of Formula (I), Formula (I-1-P1), Formula (I-1-P2), Formula (I-1-P3), or Formula (I-1-P4).

In an embodiment for the compound represented by the above formula (III), L₁, L₂ and L₃ are groups containing Z₁₁, Z₂₁ and Z₃₁ respectively, and one and only one of Z₁₁, Z₂₁ and Z₃₁ is a group containing a double bond; the double bond is, for example, a double bond between C and O in - C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, - CH₂CH₂CH₂O-, and -S-C(O)-.

The invention also provides a compound represented by the following Formula (III-1), or a pharmaceutically acceptable salt thereof: 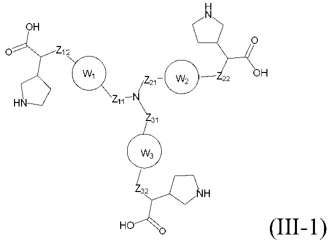 wherein, W₁, W₂, W₃, Z₁₁, Z₁₂, Z₂₁, Z₂₂, Z₃₁, and Z₃₂ are defined as in the compound of Formula (I), Formula (I-1-P1), Formula (I-1-P2), Formula (I-1-P3), Formula (I-1-P4) or Formula (III).

In one embodiment of the invention, each of Z₁₂, Z₂₂, and Z₃₂ are independently selected from methylene and ethylene;
each of Z₁₁, Z₂₁, and Z₃₁ is independently selected from a bond, -C₁₋₄alkylene-, -C₁₋₆oxaalkylene-(e.g., -C₁₋₄oxaalkylene-), -C₁₋₄thiaalkylene-, -C₁₋₄azaalkylene-, and -C₁₋₄selenaalkylene-; wherein the alkylene, oxaalkylene, thiaalkylene, azaalkylene, and selenaalkylene are optionally substituted by one or more substituents selected from Z₄₁; Z₄₁ is independently selected from deuterium, halogen (e.g., F and Cl), oxo, thio, -CN, -OH, -NH₂, C₁-₃alkyl, C₁₋₃haloalkyl, C₁₋₃alkoxy, and C₁₋₃haloalkoxy; or any two Z₄₁ together with the atoms to which they are attached form C₃₋₆cycloalkyl or 3-6-membered heterocyclyl (e.g., oxetanyl); wherein the heteroatom in the heterocyclyl is O, N, or S, and the number of the heteroatom is 1;
each of rings W₁, W₂, and W₃ is independently selected from phenyl and 5-6 membered monocyclic heteroaryl; wherein the phenyl and heteroaryl are optionally substituted by one or more substituents independently selected from deuterium, halogen, -CN, -OH, -NH₂, -CHO, C₁-₃alkyl, C₁₋₃alkoxy, -N(C₁-₃alkyl)₂, -NH(C₁₋₃alkyl), and -C(O)C₁-₃alkyl; wherein the heteroatom in the heteroaryl is O, N, or S, and the number of heteroatoms is 1 or 2.

In one embodiment of the invention, Z₁₂, Z₂₂, and Z₃₂ are all methylene.

In one embodiment of the invention, when Z₁₁, Z₂₁, and Z₃₁ are present in the compound at the same time, in a combination of the definitions of Z₁₁, Z₂₁, and Z₃₁ 2 or 3 heteroatoms or heteroatom groups are present, and the heteroatom or heteroatom group is selected from -O-, -S-, -NH-, and - CO-. In one embodiment of the invention, each of rings W₁, W₂, and W₃ is independently selected from phenyl and thienyl; the phenyl and thienyl are optionally substituted by one or more substituents independently selected from deuterium, halogen, -CN, -OH, -NH₂, -CHO, C₁-₃alkyl, C₁₋₃alkoxy, -N(C₁-₃alkyl)₂, -NH(C₁₋₃alkyl), and -C(O)C₁₋₃alkyl.

In one embodiment of the invention, each of rings W₁, W₂, and W₃ is independently selected from 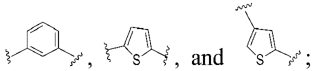 the above groups are optionally substituted by one or more substituents independently selected from deuterium, F, Cl, Br, methyl, ethyl, methoxy, and ethoxy. In an embodiment for the compound represented by the above formula (III-1), one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-.

The invention also provides a compound represented by the following Formula (III-2), or a pharmaceutically acceptable salt thereof: 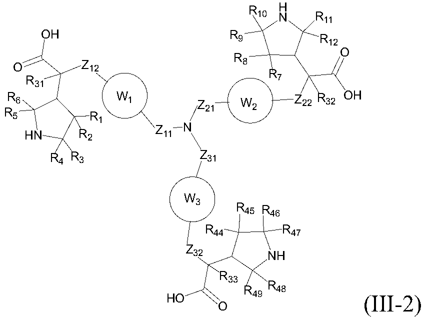 wherein, W₁, W₂, W₃, Z₁₁, Z₁₂, Z₂₁, Z₂₂, Z₃₁, Z₃₂, R₁, R₂, R₃, R₄, R₅, R₆, R₇, R₈, R₉, R₁₀, R₁₁, R₁₂, R₄₄, R₄₅, R₄₆, R₄₇, R₄₈, R₄₉, R₃₁, R₃₂, and R₃₃ are defined as in the compound of Formula (I), Formula (I-1-P1), Formula (I-1-P2), Formula (I-1-P3), Formula (I-1-P4), Formula (III) or Formula (III-1).

In one embodiment of the invention, the heteroatom or heteroatom group in Z₃₁ comprises -CO-or -C(S)-.

In one embodiment of the invention, R₁, R₂, R₃, R₄, R₅, R₆, R₇, R₈, R₉, R₁₀, R₁₁, R₁₂, R₄₄, R₄₅, R₄₆, R₄₇, R₄₈, R₄₉, R₃₁, R₃₂, and R₃₃ are selected from hydrogen or deuterium.

In an embodiment for the compound represented by the above formula (III-2), one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-.

The invention also provides a compound represented by the following Formula (III-1-1) or Formula (III-2-1), or a pharmaceutically acceptable salt thereof: 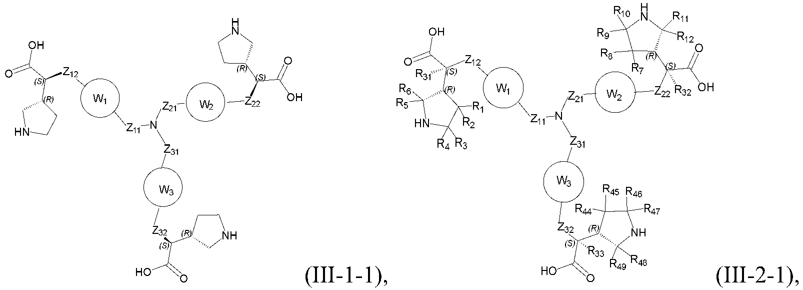 wherein, W₁, W₂, W₃, Z₁₁, Z₁₂, Z₂₁, Z₂₂, Z₃₁, Z₃₂, R₁, R₂, R₃, R₄, R₅, R₆, R₇, R₈, R₉, R₁₀, R₁₁, R₁₂, R₄₄, R₄₅, R₄₆, R₄₇, R₄₈, R₄₉, R₃₁, R₃₂, and R₃₃ are defined as in the compound of Formula (I), Formula (I-1-P1), Formula (I-1-P2), Formula (I-1-P3), Formula (I-1-P4), Formula (III), Formula (III-1) or Formula (III-2).

The invention also provides a compound represented by the following Formula (III-A), or a pharmaceutically acceptable salt thereof: 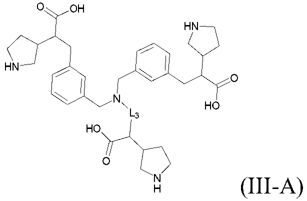 wherein, L₃ is defined as in the compound of Formula (I), Formula (I-1-P1), Formula (I-1-P2), Formula (I-1-P3), Formula (I-1-P4), Formula (III) or Formula (III-1).

In one embodiment of the invention, L₃ is selected from -C₁₋₆alkylene-, -C₂₋₆alkynylene-, - methylene-phenyl-phenyl-methylene-, -methylene-6-membered heteroaryl-6-membered heteroaryl-methylene- (e.g., -methylene-pyridinyl-pyridinyl-methylene-), and 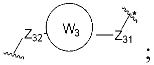 wherein the alkylene, methylene, and alkynylene are optionally substituted by one or more substituents independently selected from halogen, oxo, -CN, -OH, and -NH₂; wherein the aryl and heteroaryl are optionally substituted by one or more substituents independently selected from halogen, -CN, C₁-₃alkyl, and C₁₋₃alkoxy; * represents the connecting end with N.

In one embodiment of the invention, the ring W₃ is selected from phenyl, naphthyl, 8-10-membered bicyclic heteroaryl, and C₈₋₁₀bicycloalkyl; wherein the phenyl, naphthyl, heteroaryl, and cycloalkyl are optionally substituted by one or more substituents independently selected from halogen, -CN, C₁-₃alkyl, and C₁₋₃alkoxy.

In one embodiment of the invention, the ring W₃ is selected from phenyl, naphthyl, 5-membered/5-membered fused heteroaryl, 5-membered/6-membered fused heteroaryl, 6-membered/5-membered fused heteroaryl, 6-membered/6-membered fused heteroaryl, 4-membered/6-membered spirocycloalkyl, and 6-membered/4-membered spirocycloalkyl; wherein the phenyl, naphthyl, fused heteroaryl, and spirocycloalkyl are optionally substituted by one or more substituents independently selected from F, Cl, Br, methyl, ethyl, methoxy, and ethoxy.

In one embodiment of the invention, W₃ is selected from

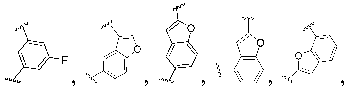

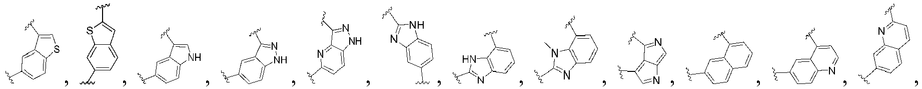

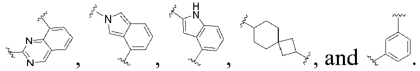

In one embodiment of the invention, each of Z₃₁ and Z₃₂ is independently selected from -C₁₋₃alkylene-, -C₁₋₃oxaalkylene-, -C₁₋₃thiaalkylene-, and -C₁₋₃azaalkylene-; preferably, each of Z₃₁ and Z₃₂ is independently selected from -C₁₋₂alkylene-, -C₁₋₂oxaalkylene-, -C₁₋₂thiaalkylene-, and - C₁₋₂azaalkylene-; wherein the alkylene, oxaalkylene, thiaalkylene, and azaalkylene are optionally substituted by one or more substituents independently selected from deuterium and oxo.

In one embodiment of the invention, each of Z₃₁ and Z₃₂ is independently selected from -CD₂-, - C₁₋₂alkylene-, -OC₁₋₂alkylene-, -C(O)-, -C₁₋₂alkylene-C(O)-, -OC₁₋₂alkylene-C(O)-, -NH-C₁₋₂alkylene-C(O)-, -S-C₁₋₂alkylene-C(O)-, and -O-C₁₋₂alkylene-C(O)-C₁₋₂alkylene-.

In one embodiment of the invention, each of Z₃₁ and Z₃₂ is independently selected from -C₁₋₃alkylene- and -C₁₋₃oxaalkylene-; wherein the alkylene and oxaalkylene are optionally substituted by one or more substituents independently selected from deuterium, halogen, oxo, -CN, -OH, - NH₂, methyl, and methoxy.

In one embodiment of the invention, each of Z₃₁ and Z₃₂ is independently selected from -C₁₋₃alkylene-; wherein the alkylene is optionally substituted by one or more substituents independently selected from deuterium and oxo.

In one embodiment of the invention, each of Z₃₁ and Z₃₂ is independently selected from methylene, ethylene, -CD₂-,

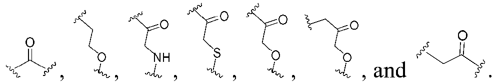

In one embodiment of the invention, L₃ is selected from 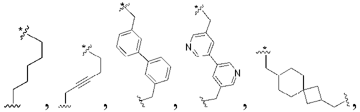 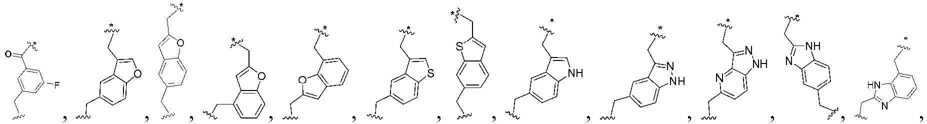 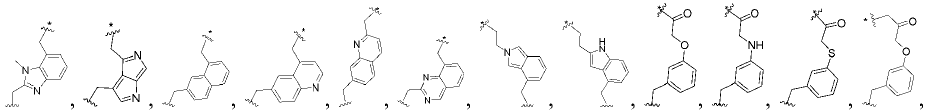 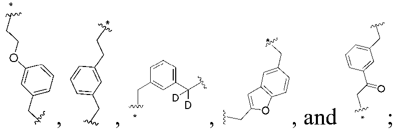 * represents the connecting end with N.

The invention also provides a compound represented by the following Formula (III-A'), or a pharmaceutically acceptable salt thereof: 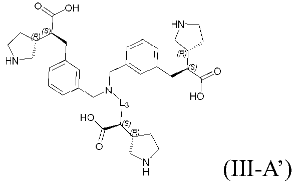 wherein, L₃ is defined as in the compound of Formula (I), Formula (I-1-P1), Formula (I-1-P2), Formula (I-1-P3), Formula (I-1-P4), Formula (III), Formula (III-1) or Formula (III-A).

The invention also provides a compound represented by the following Formula (III-B), or a pharmaceutically acceptable salt thereof: 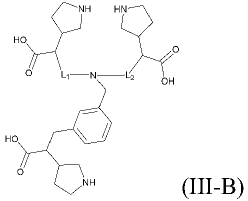 wherein, L₁ and L₂ are defined as in the compound of Formula (I), Formula (I-1-P1), Formula (I-1-P2), Formula (I-1-P3), Formula (I-1-P4), Formula (III) or Formula (III-1).

In one embodiment of the invention, L₁ is 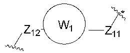 and * represents the connecting end with N.

In one embodiment of the invention, L₂ is 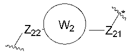 and * represents the connecting end with N.

In one embodiment of the invention, each of rings W₁ and W₂ is independently selected from C₆₋₁₂aryl and 5-12-membered heteroaryl; wherein the aryl and heteroaryl are optionally substituted by one or more substituents independently selected from deuterium, halogen, -CN, C₁₋₃alkyl, and C₁₋₃alkoxy.

In one embodiment of the invention, each of rings W₁ and W₂ is independently selected from phenyl, C₈₋₁₂aryl, and 7-12-membered heteroaryl; wherein the phenyl, aryl, and heteroaryl are optionally substituted by one or more substituents independently selected from halogen, -CN, methyl, and methoxy.

In one embodiment of the invention, each of rings W₁ and W₂ is independently selected from phenyl, naphthyl, and 8-10-membered bicyclic heteroaryl; wherein the phenyl, naphthyl, and heteroaryl are optionally substituted by one or more substituents independently selected from halogen, -CN, C₁₋₃alkyl, and C₁₋₃alkoxy.

In one embodiment of the invention, each of rings W₁ and W₂ is independently selected from phenyl, naphthyl, 5-membered/5-membered fused heteroaryl, 5-membered/6-membered fused heteroaryl, 6-membered/5-membered fused heteroaryl, and 6-membered/6-membered fused heteroaryl; wherein the phenyl, naphthyl, and fused heteroaryl are optionally substituted by one or more substituents independently selected from F, Cl, Br, -CN, methyl, ethyl, methoxy, and ethoxy. In one embodiment of the invention, each of rings W₁ and W₂ is independently selected from

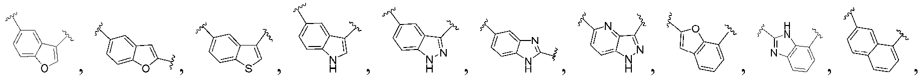

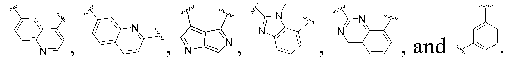

In one embodiment of the invention, each of Z₁₁, Z₁₂, Z₂₁, and Z₂₂ is independently selected from -C₁₋₃alkylene-, -C₁₋₃oxaalkylene-, -C₁₋₃thiaalkylene-, and -C₁₋₃azaalkylene-; wherein the alkylene, oxaalkylene, thiaalkylene, and azaalkylene are optionally substituted by one or more substituents independently selected from deuterium, halogen, oxo, -CN, -OH, -NH₂, methyl, and methoxy.

In one embodiment of the invention, each of Z₁₁, Z₁₂, Z₂₁, and Z₂₂ is independently selected from -C₁₋₃alkylene- and -C₁₋₃oxaalkylene-; wherein the alkylene and oxaalkylene are optionally substituted by one or more substituents independently selected from deuterium, halogen, oxo, - CN, -OH, -NH₂, methyl, and methoxy.

In one embodiment of the invention, each of Z₁₁, Z₁₂, Z₂₁, and Z₂₂ is independently selected from -C₁₋₂alkylene-, -C₁₋₂oxaalkylene-, -C₁₋₂thiaalkylene-, and -C₁₋₂azaalkylene-; wherein the alkylene, oxaalkylene, thiaalkylene, and azaalkylene are optionally substituted by one or more substituents independently selected from deuterium, halogen, oxo, -CN, -OH, -NH₂, methyl, and methoxy.

In one embodiment of the invention, each of Z₁₁, Z₁₂, Z₂₁, and Z₂₂ is independently selected from methylene.

In a preferred embodiment of the invention, each of Z₁₁, Z₁₂, Z₂₁, and Z₂₂ is independently selected from methylene,

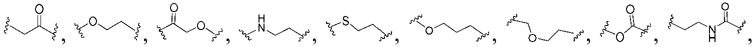

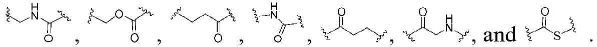

For example, L₁ and L₂ are groups containing Z₁₁ and Z₂₁ respectively, one and only one of Z₁₁ and Z₂₁ is a group containing a double bond; the double bond is, for example, a double bond between C and O in -C(O)-, or a double bond between C and S in -C(S)-; preferably, the double bond is for example a double bond between C and O in -C(O)-; further preferably, neither Z₁₁ nor Z₂₁ is methylene, or a group not containing the double bond of Z₁₁ and Z₂₁ contains -O-, -NH-, - S-, or -Se-, preferably, -O-; still further preferably, Z₁₁ and Z₂₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, - OC(O)-, -CH₂CH₂O-, -OCH₂C(O)-, -CH₂OC(O)-, -CH₂CH₂CH₂O-, and -S-C(O)-.

In one embodiment of the invention, each of L₁ and L₂ is independently selected from 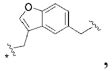 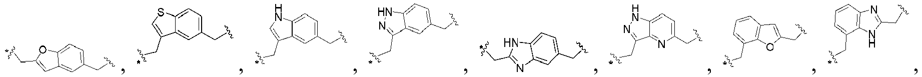 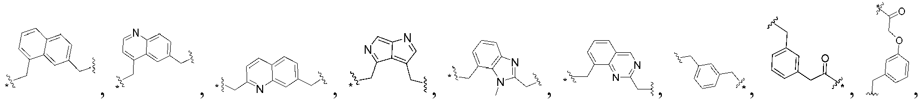 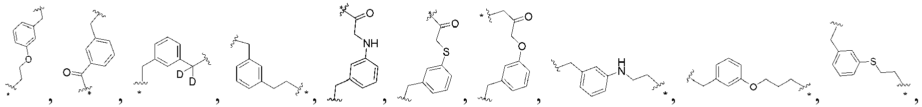 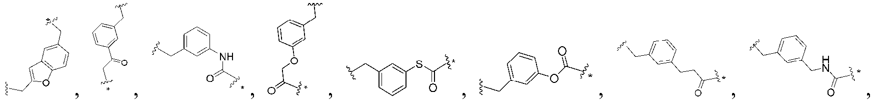 and 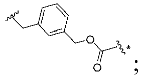 * represents the connecting end with N.

The invention also provides a compound represented by the following Formula (III-B') or Formula (III-B'-1), or a pharmaceutically acceptable salt thereof: 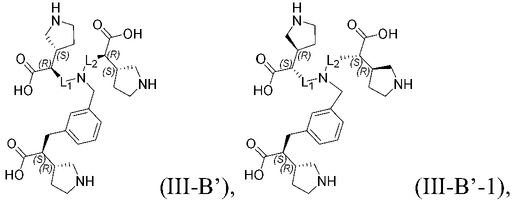 wherein, L₁ and L₂ are defined as in the compound of Formula (I), Formula (I-1-P1), Formula (I-1-P2), Formula (I-1-P3), Formula (I-1-P4), Formula (III), Formula (III-1) or Formula (III-B).

For example, L₁ and L₂ are groups containing Z₁₁ and Z₂₁ respectively, one and only one of Z₁₁ and Z₂₁ is a group containing a double bond; the double bond is, for example, a double bond between C and O in -C(O)-, or a double bond between C and S in -C(S)-; further preferably, neither Z₁₁ nor Z₂₁ is methylene, or a group not containing the double bond of Z₁₁ and Z₂₁ contains -O-, - NH-, -S-, or -Se-, preferably, -O-; still further preferably, Z₁₁ and Z₂₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, - OC(O)-, -CH₂CH₂O-, -OCH₂C(O)-, -CH₂OC(O)-, -CH₂CH₂CH₂O-, and -S-C(O)-.

The invention also provides a compound represented by the following Formula (III-C), or a pharmaceutically acceptable salt thereof: 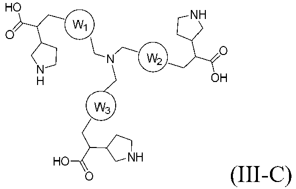 wherein, W₁, W₂, and W₃ are defined as in compound of Formula (I), Formula (I-1-P1), Formula (I-1-P2), Formula (I-1-P3), Formula (I-1-P4), Formula (III) or Formula (III-1).

In one embodiment of the invention, each of rings W₁, W₂ and W₃ is independently selected from phenyl, naphthyl, 8-10-membered bicyclic heteroaryl, and 5-6-membered monocyclic heteroaryl; wherein the phenyl, naphthyl, and heteroaryl are optionally substituted by one or more substituents independently selected from halogen, C₁-₃alkyl, and C₁₋₃alkoxy.

In one embodiment of the invention, each of rings W₁, W₂, and W₃ is independently selected from

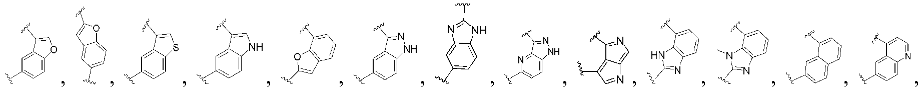

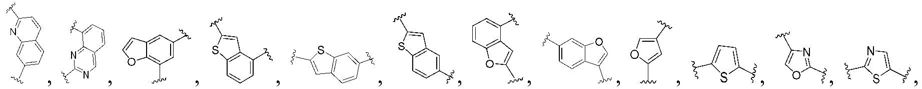

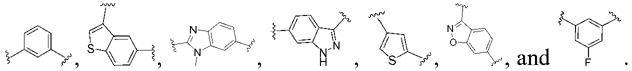

The invention also provides a compound represented by the following Formula (III-C'), or a pharmaceutically acceptable salt thereof: 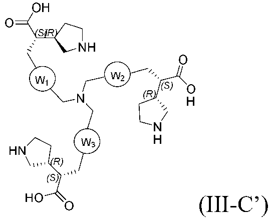 wherein, W₁, W₂, and W₃ are defined as in the compound of Formula (I), Formula (I-1-P1), Formula (I-1-P2), Formula (I-1-P3), Formula (I-1-P4), Formula (III), Formula (III-1) or Formula (III-C).

The invention also provides a compound represented by the following Formula (III-D), or a pharmaceutically acceptable salt thereof: 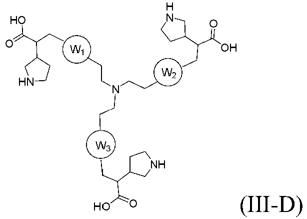 wherein, W₁, W₂, and W₃ are defined as in compound of Formula (I), Formula (I-1-P1), Formula (I-1-P2), Formula (I-1-P3), Formula (I-1-P4), Formula (III) or Formula (III-1).

In one embodiment of the invention, each of rings W₁, W₂, and W₃ is independently selected from phenyl, naphthyl, and 8-10-membered bicyclic heteroaryl groups; wherein the phenyl, naphthyl, and heteroaryl are optionally substituted by one or more substituents independently selected from halogen, C₁-₃alkyl, and C₁₋₃alkoxy.

In one embodiment of the invention, each of rings W₁, W₂, and W₃ is independently selected from phenyl and 9-10-membered bicyclic heteroaryl; wherein the heteroatoms in the heteroaryl are independently selected from oxygen or N, and the number of heteroatoms is 1 or 2; wherein the phenyl and heteroaryl are optionally substituted by one or more substituents independently selected from F, Cl, methyl, and methoxy.

In one embodiment of the invention, each of rings W₁, W₂, and W₃ is independently selected from

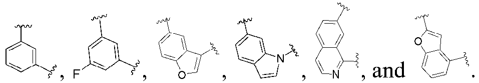

The invention also provides a compound represented by the following Formula (III-D'), or a pharmaceutically acceptable salt thereof: 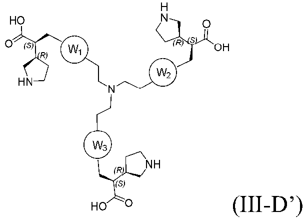 wherein, W₁, W₂, and W₃ are defined as in the compound of Formula (I), Formula (I-1-P1), Formula (I-1-P2), Formula (I-1-P3), Formula (I-1-P4), Formula (III), Formula (III-1) or Formula (III-D).

The invention also provides a compound represented by the following Formula (III-E), or a pharmaceutically acceptable salt thereof: 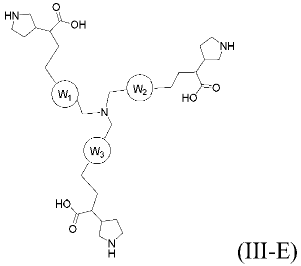 wherein, W₁, W₂, and W₃ are defined as in the compound of Formula (I), Formula (I-1-P1), Formula (I-1-P2), Formula (I-1-P3), Formula (I-1-P4), Formula (III) or Formula (III-1).

In one embodiment of the invention, each of rings W₁, W₂, and W₃ is independently selected from phenyl, naphthyl, and 8-10-membered bicyclic heteroaryl groups; wherein the phenyl, naphthyl, and heteroaryl are optionally substituted by one or more substituents independently selected from halogen, C₁-₃alkyl, and C₁₋₃alkoxy.

In one embodiment of the invention, each of rings W₁, W₂, and W₃ is independently selected from 9-membered bicyclic heteroaryl; wherein the heteroatoms in the heteroaryl are independently selected from O or N, and the number of heteroatoms is 1 or 2.

In one embodiment of the invention, each of rings W₁, W₂, and W₃ is independently selected from

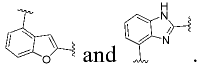

The invention also provides a compound represented by the following Formula (III-E'), or a pharmaceutically acceptable salt thereof: 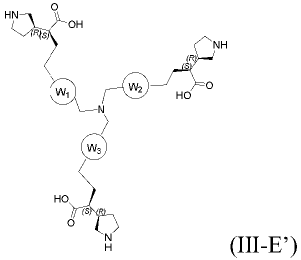 wherein, W₁, W₂, and W₃ are defined as in the compound of Formula (I), Formula (I-1-P1), Formula (I-1-P2), Formula (I-1-P3), Formula (I-1-P4), Formula (III), Formula (III-1) or Formula (III-E).

The invention also provides a compound represented by the following Formula (IV), or a pharmaceutically acceptable salt thereof: 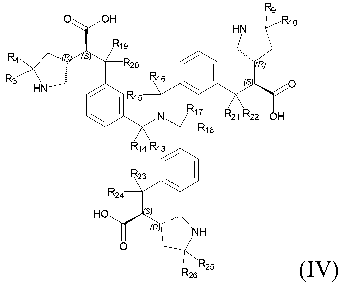 wherein, each of R₃, R₄, R₉, R₁₀, R₁₃, R₁₄, R₁₅, R₁₆, R₁₇, R₁₈, R₁₉, R₂₀, R₂₁, R₂₂, R₂₃, R₂₄, R₂₅, and R₂₆ is independently selected from H and deuterium, and at least one of R₃, R₄, R₉, R₁₀, and R₁₃-R₂₆ is deuterium.

The invention also provides a compound represented by the following Formula (A), or a pharmaceutically acceptable salt thereof: 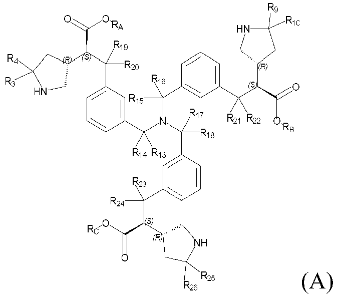
wherein, each of R₃, R₄, R₉, R₁₀, R₁₃, R₁₄, R₁₅, R₁₆, R₁₇, R₁₈, R₁₉, R₂₀, R₂₁, R₂₂, R₂₃, R₂₄, R₂₅, and R₂₆ is independently selected from H and deuterium, and at least one of R₃, R₄, R₉, R₁₀, and R₁₃-R₂₆ is deuterium;
each of R_{A}, R_{B}, and R_{C} are independently selected from hydrogen, C₁₋₆alkyl, C₂₋₆alkenyl, C₁₋₆alkoxy, -C₁₋₃alkylene-O-C₁₋₆alkyl, and -C₁₋₃alkylene-O-C(O)-C₁₋₆alkyl, wherein the alkyl, alkenyl, alkoxy, and alkylene are optionally substituted by one or more substituents independently selected from deuterium, halogen, -OH, -NH₂, -CN, oxo, C₁₋₃alkyl, and C₁₋₃alkoxy.

In one embodiment of the invention, each of R_{A}, R_{B}, and Rc is independently selected from C₁₋₆alkyl, C₁₋₆alkoxy, -C₁₋₂alkylene-O-C₁₋₄alkyl, -C₁₋₂alkylene-O-C(O)-C₁₋₄alkyl, wherein the alkyl, C₁₋₆alkoxy, and alkylene are optionally substituted by one or more substituents independently selected from deuterium, F, Cl, Br, -OH, -NH₂, -CN, oxo, methyl, ethyl, n-propyl, isopropyl, methoxy, ethoxy, n-propoxy, and isopropoxy.

In one embodiment of the invention, each of R_{A}, R_{B}, and R_{C} is independently selected from C₁₋₃alkyl, -methylene-O-C₁₋₄alkyl, and -methylene-O-C(O)-C₁₋₃alkyl, wherein the methylene and alkyl are optionally substituted by one or more substituents independently selected from methyl, ethyl, n-propyl, and isopropyl.

In one embodiment of the invention,each of R_{A}, R_{B}, and Rc is independently selected from methyl, ethyl, and -CH₂-OC(O)-CH(CH₃)₂.

The invention also provides a compound represented by the following Formula (B), Formula (B-1), Formula (C), Formula (C-1), Formula (D), or Formula (D-1), or a pharmaceutically acceptable salt thereof: 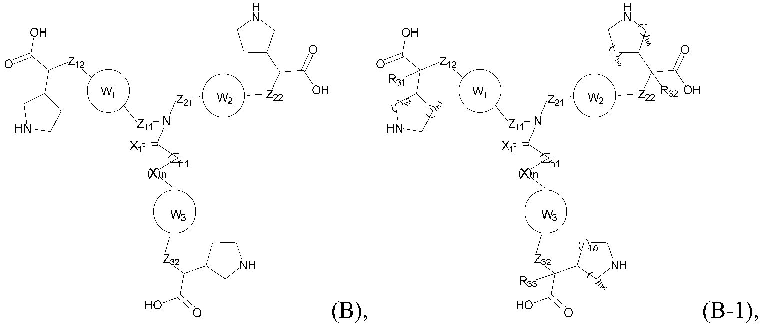 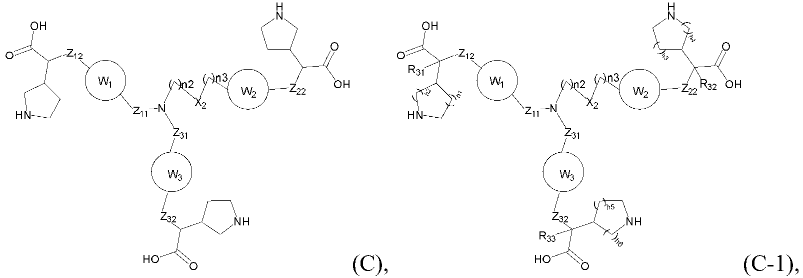 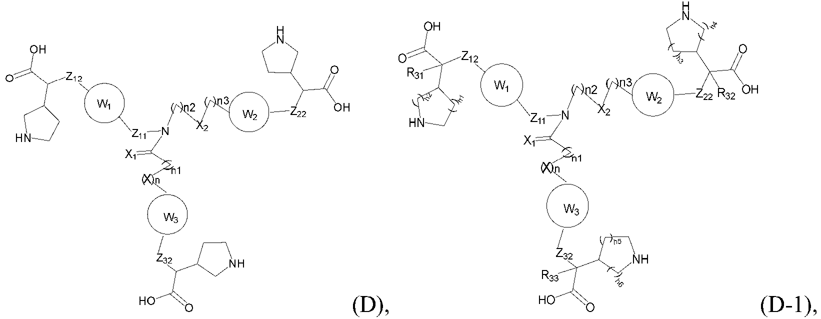
wherein, Z₁₁, Z₂₁, Z₃₁, R₃₁, R₃₂, R₃₃, h1, h2, h3, h4, h5, and h6 are respectively defined as in the compound of Formula (I), Formula (I-1-P1), Formula (I-1-P2), Formula (I-1-P3), Formula (I-1-P4), Formula (III), Formula (III-1) or Formula (III-B); each of X and X₂ is independently selected from O, CH₂, NH, and S, and Se, X₁ is independently selected from O and S, each of n, n1, and n3 are independently 0 or 1, and n2 is independently 0, 1, 2, or 3; each of Z₁₂, Z₂₂, and Z₃₂ is independently selected from methylene, ethylene, and -CD₂-, or selected from methylene and ethylene;
each of rings W₁, W₂, and W₃ is independently selected from phenyl and 5-6 membered monocyclic heteroaryl; wherein the phenyl and heteroaryl are optionally substituted by one or more substituents independently selected from deuterium, halogen, -CN, -OH, -NH₂, -CHO, C₁-₃alkyl, C₁₋₃alkoxy, -N(C₁-₃alkyl)₂, -NH(C₁-₃alkyl), and -C(O)C₁-₃alkyl; wherein the heteroatom in the heteroaryl is O, N, or S, and the number of heteroatoms is 1 or 2.

In one embodiment of the invention, each of Z₁₂, Z₂₂ and Z₃₂ is independently selected from methylene, and -CD₂-.

In one embodiment of the invention, Z₁₁ is -CD₂-.

In one embodiment of the invention, Z₁₁ is independently selected from -C₁₋₃alkylene-, -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; preferably, Z₁₁ is independently selected from -C₁₋₃alkylene- and -OC₁₋₃alkylene-; preferably, Z₁₁ is independently selected from methylene, ethylene, methylene oxide, and ethylene oxide; preferably, Z₁₁ is independently selected from methylene, and -CD₂-; preferably, Z₁₁ is independently selected from methylene.

In one embodiment of the invention, Z₂₁ is -CD₂-.

In one embodiment of the invention, Z₂₁ is independently selected from -C₁₋₃alkylene-, -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; it is preferably selected from -OC₁₋₃alkylene-, -NH-C₁₋₂alkylene-, -S-C₁₋₂alkylene-, and -C₁₋₂alkylene-O-C₁₋₃alkylene-.

In one embodiment of the invention, Z₃₁ is independently selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-; it is preferably selected from -C(O)-, -C₁₋₂alkylene-C(O)-, -OC₁₋₂alkylene-C(O)-, -NH-C(O)-, -O-C(O)-, and -S-C(O)-.

In one embodiment of the invention, Z₁₂, Z₂₂, and Z₃₂ are all methylene.

In one embodiment of the invention, X₂ is selected from O, NH, and S; preferably, X₂ is O.

In one embodiment of the invention, n2 is 0 or 1, n3 is 0, and X₂ is CH₂.

In one embodiment of the invention, n2 is 2 or 3, n3 is 0, X₂ is O, NH, or S; or n2 is 1 or 2, n3 is 1, X₂ is O, NH, or S; further preferably, n2 is 2 or 3, n3 is 0, X₂ is O; or n2 is 2, n3 is 1, X₂ is O. In one embodiment of the invention, n2 is 2, n3 is 0, and X₂ is O, NH, or S; preferably, n2 is 2, n3 is 0, and X₂ is O.

In one embodiment of the invention, the sum of n2 and n3 is not more than 3 (specifically 0, 1, 2, and 3); preferably the sum of n2 and n3 is not more than 2 (specifically 0, 1, and 2); preferably the sum of n2 and n3 is 1 or 2 (i.e., n2 is 2 and n3 is 0; or n2 is 1 and n3 is 1; or n2 is 0 and n3 is 2). In one embodiment of the invention, X₁ is O.

In one embodiment of the invention, X is CH₂.

In one embodiment of the invention, X is selected from O, CH₂, NH, and S, n is 1, and n1 is 0; or X is O or CH₂, n is 1, and n1 is 1.

In one embodiment of the invention, X₁ is O, X is selected from O, CH₂, NH, and S, n is 1, n1 is 0; or X₁ is O, X is O or CH₂, n is 1, n1 is 1; further preferably, X₁ is O, X is selected from O, and CH₂, n is 1, n1 is 0; or X₁ is O, X is O or CH₂, n is 1, n1 is 1; further preferably, X₁ is O, X is selected from CH₂, n is 1, n1 is 0.

In one embodiment of the invention, the sum of n and n1 is 0, 1, or 2; preferably the sum of n and n1 is 1 or 2 (i.e., n is 1 and n1 is 0; or n is 0 and n1 is 1; or n is 1 and n1 is 1; or n is 0 and n1 is 2; or n is 2 and n1 is 0); preferably the sum of n and n1 is 1 (i.e., n is 1 and n1 is 0; or n is 0 and n1 is 1).

For example, in any of the above-mentioned Formula (B) and Formula (B-1), each of Z₁₁ and Z₂₁ is a group not containing a double bond; the double bond is, for example, a double bond between C and O in -C(O)-, or a double bond between C and S in -C(S)-; further preferably, Z₁₁ and Z₂₁ are identical or different, and at least one of Z₁₁ and Z₂₁ is a group containing -O-, -NH-, -S-, or -Se-, preferably, a group containing -O-, e.g. *-CH₂O-, *-CH₂CH₂O-, * is the connecting end with N; still further preferably, Z₁₁, Z₂₁ and *-C(X₁)-(CH₂)ₙ₁-(X)ₙ- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

For example, in any of the above-mentioned Formula (B) and Formula (B-1), each of Z₁₂, Z₂₂ and Z₃₂ is independently selected from methylene and -CD₂-;
each of Z₁₁ and Z₂₁ is independently selected from -CD₂-, -C₁₋₃alkylene- (preferably -C₁₋₂alkylene-), -OC₁₋₃alkylene-* (preferably -OC₁₋₂alkylene-*), -SC₁₋₃alkylene-*, -NHC₁₋₃alkylene-*, -Se-C₁₋₃alkylene-*, and -CH₂-O-C₁₋₂alkylene-*, * is the connecting end with N;
X₁ is O or S, X is selected from O, CH₂, NH, and S, n is 1, n1 is 0; or X₁ is O or S, X is O or CH₂, n is 1, n1 is 1; or X₁ is O or S, n is 0, n1 is 0.

For example, in any of the above-mentioned Formula (B) and Formula (B-1), each of Z₁₂, Z₂₂ and Z₃₂ is independently selected from methylene, and -CD₂-;
Z₁₁ is independently selected from -CD₂-, -CH₂-, -CH₂CH₂-, -OCH₂-*, and -OCH₂CH₂-*, * is the connecting end with N;
Z₂₁ is independently selected from -OCH₂CH₂-*, -CH₂OCH₂CH₂-*, and -OCH₂CH₂CH₂-*, * is the connecting end with N;
X₁ is O, X is selected from O, CH2, NH, and S, n is 1, n1 is 0; or X₁ is O, X is O or CH₂, n is 1, n1 is 1.

For example, in any of the above-mentioned Formula (B) and Formula (B-1), each of Z₁₂, Z₂₂ and Z₃₂ is independently selected from methylene, and -CD₂-; each of Z₁₁ and Z₂₁ is independently selected from -CD₂-, -C₁₋₃alkylene- (preferably -C₁₋₂alkylene-), -OC₁₋₃alkylene-* (preferably - OC₁₋₂alkylene-*), -SC₁₋₃alkylene-*, -NHC₁₋₃alkylene-*, -Se-C₁₋₃alkylene-*, and -CH₂-O-C₁₋₂alkylene-*, * is the connecting end with N, X₁ is O or S, X is selected from O, CH₂, NH, and S, the sum of n and n1 is 0, 1, or 2; further preferably, Z₂₁ is independently selected from -OCH₂CH₂-*, -CH₂OCH₂CH₂-*, and -OCH₂CH₂CH₂-*, * is the connecting end with N; further preferably, X₁ is O, X is selected from O, CH₂, NH, and S, the sum of n and n1 is 1, or 2.

For example, in any of the above-mentioned Formula (C) and Formula (C-1), one and only one of Z₁₁ and Z₃₁ is a group containing a double bond; the double bond is, for example, a double bond between C and O in -C(O)-, or a double bond between C and S in -C(S)-; further preferably, the group not containing a double bond of Z₁₁ and Z₃₁ is identical to or different from *-(CH₂)ₙ₂-X₂-(CH₂)ₙ₃- (* represents the connecting end with N); still further preferably, Z₁₁, -(CH₂)ₙ₂-X₂-(CH₂)ₙ₃- and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, - CH₂CH₂CH₂O-, and -S-C(O)-.

For example, in any of the above-mentioned Formula (C) and Formula (C-1), each of Z₁₂, Z₂₂ and Z₃₂ is independently selected from methylene, and -CD₂-;
Z₁₁ is independently selected from -CD₂-, -C₁₋₃alkylene- (preferably -C₁₋₂alkylene-), -OC₁₋₃alkylene-* (preferably -OC₁₋₂alkylene-*), -SC₁₋₂alkylene-*, -NHC₁₋₂alkylene-*, -Se-C₁₋₂alkylene-*, and -CH₂-O-C₁₋₂alkylene-*, * is the connecting end with N;
Z₃₁ is independently selected from -C(O)-, -C₁₋₃alkylene-C(O)-*, -OC₁₋₂alkylene-C(O)-*, - NHCH₂-C(O)-*, -SCH₂-C(O)-*, -CH₂-NHC(O)-*, -C₁₋₂alkylene-O-C(O)-*, -NH-C(O)-*, -O-C(O)-*, -S-C(O)-*, and -C₁₋₂alkylene-C(S)-*, * is the connecting end with N;
n2 is 2 or 3, n3 is 0, X₂ is O, NH, or S; or n2 is 1 or 2, n3 is 1, X₂ is O, NH, or S; or n2 is 0 or 1, n3 is 0, X₂ is CH₂.

For example, in any of the above-mentioned Formula (C) and Formula (C-1), each of Z₁₂, Z₂₂ and Z₃₂ is independently selected from methylene, and -CD₂-;
Z₁₁ is independently selected from -CD₂-, -CH₂-, -CH₂CH₂-, -OCH₂-*, and -OCH₂CH₂-*, * is the connecting end with N;
Z₃₁ is independently selected from -C(O)-, -CH₂CH₂C(O)-*, -CH₂C(O)-*, -OCH₂C(O)-*, - NHC(O)-*, -OC(O)-*, and -SC(O)-*, * is the connecting end with N;
n2 is 2 or 3, n3 is 0, X₂ is O; or n2 is 1 or 2, n3 is 1, X₂ is O; or n2 is 0 or 1, n3 is 0, X₂ is CH₂.

For example, in any of the above-mentioned Formula (C) and Formula (C-1), each of Z₁₂, Z₂₂ and Z₃₂ is independently selected from methylene, and -CD₂-; Z₁₁ is independently selected from - CD₂-, -C₁₋₃alkylene- (preferably -C₁₋₂alkylene-), -OC₁₋₃alkylene-* (preferably -OC₁₋₂alkylene-*), -SC₁₋₂alkylene-*, -NHC₁₋₂alkylene-*, -Se-C₁₋₂alkylene-*, and -CH₂-O-C₁₋₂alkylene-*, Z₃₁ is independently selected from -C(O)-, -C₁₋₃alkylene-C(O)-*, -OC₁₋₂alkylene-C(O)-*, -NHCH₂-C(O)-*, -SCH₂-C(O)-*, -CH₂-NHC(O)-*, -C₁₋₂alkylene-O-C(O)-*, -NH-C(O)-*, -O-C(O)-*, -S-C(O)-*, and -C₁₋₂alkylene-C(S)-*, * is the connecting end with N; further preferably, Z₁₁ is independently selected from -CD₂-, -CH₂-, -CH₂CH₂-, and -OCH₂CH₂-*, * is the connecting end with N; further preferably, Z₃₁ is independently selected from -CH₂CH₂C(O)-*, -CH₂C(O)-*, - OCH₂C(O)-*, -NHC(O)-*, -OC(O)-*, and -SC(O)-*, * is the connecting end with N; further preferably, X₂ is O, NH, S, or Se, the sum of n2 and n3 is 1, 2, or 3; further preferably, X₂ is O, the sum of n2 and n3 is 2, or 3.

For example, in any of the above-mentioned Formula (D) and Formula (D-1), Z₁₁ is a group not containing a double bond; the double bond is, for example, a double bond between C and O in - C(O)-, or a double bond between C and S in -C(S)-; further preferably, Z₁₁ is identical to or different from *-(CH₂)ₙ₂-X₂-(CH₂)ₙ₃- (* represents the connecting end with N); still further preferably, Z₁₁, -C(X₁)-(CH₂)ₙ₁-(X)ₙ- and -(CH₂)ₙ₂-X₂-(CH₂)ₙ₃- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-.

For example, in any of the above-mentioned Formula (D) and Formula (D-1), each of Z₁₂, Z₂₂ and Z₃₂ is independently selected from methylene, and -CD₂-;
Z₁₁ is independently selected from -CD₂-, -C₁₋₂alkylene-, -OC₁₋₂alkylene-*, -SC₁₋₂alkylene-*, - NHC₁₋₂alkylene-*, -Se-C₁₋₂alkylene-*, and -CH₂-O-C₁₋₂alkylene-*, * is the connecting end with N;
n2 is 2 or 3, n3 is 0, X₂ is O, NH, or S; or n2 is 1 or 2, n3 is 1, X₂ is O, NH, or S; or n2 is 0 or 1, n3 is 0, X₂ is CH₂;
X₁ is O or S, X is selected from O, CH₂, NH, and S, n is 1, n1 is 0; or X₁ is O or S, X is O or CH₂, n is 1, n1 is 1.

For example, in any of the above-mentioned Formula (D) and Formula (D-1), each of Z₁₂, Z₂₂ and Z₃₂ is independently selected from methylene, and -CD₂-;
Z₁₁ is independently selected from -CD₂-, -CH₂-, -CH₂CH₂-, -OCH₂-*, and -OCH₂CH₂-*, * is the connecting end with N;
n2 is 2 or 3, n3 is 0, X₂ is O; or n2 is 1 or 2, n3 is 1, X₂ is O; or n2 is 0 or 1, n3 is 0, X₂ is CH₂;
X₁ is O, X is selected from O, CH₂, NH, and S, n is 1, n1 is 0; or X₁ is O, X is O or CH₂, n is 1, n1 is 1.

For example, in any of the above-mentioned Formula (D) and Formula (D-1), each of Z₁₂, Z₂₂ and Z₃₂ is independently selected from methylene, and -CD₂-;
Z₁₁ is independently selected from -CD₂-, -CH₂-, -CH₂CH₂-, and -OCH₂CH₂-*, * is the connecting end with N;
n2 is 2 or 3, n3 is 0, X₂ is O; or n2 is 2, n3 is 1, X₂ is O;
X₁ is O, X is selected from O, CH₂, NH, and S, n is 1, n1 is 0.

For example, in any of the above-mentioned Formula (D) and Formula (D-1), each of Z₁₂, Z₂₂ and Z₃₂ is independently selected from methylene, and -CD₂-; Z₁₁ is independently selected from - CD₂-, -C₁₋₂alkylene-, -OC₁₋₂alkylene-*, -SC₁₋₂alkylene-*, -NHC₁₋₂alkylene-*, -Se-C₁₋₂alkylene-*, and -CH₂-O-C₁₋₂alkylene-*, * is the connecting end with N; X₂ is O, NH, S, or Se, the sum of n2 and n3 is 1, 2, or 3; X₁ is O or S, X is selected from O, CH₂, NH, and S, the sum of n and n1 is 0, 1, or 2; further preferably, Z₁₁ is independently selected from -CD₂-, -CH₂-, -CH₂CH₂-, and - OCH₂CH₂-*, * is the connecting end with N; further preferably, X₂ is O, the sum of n2 and n3 is 2, or 3; X₁ is O, X is selected from O, CH₂, NH, and S, the sum of n and n1 is 1, or 2.

In one embodiment of the invention, each of ring W₁, W₂, and W₃ is independently selected from the following optionally substituted groups: 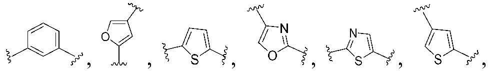 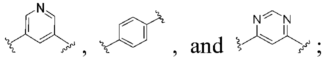 wherein "optionally substituted" refers to unsubstituted or substituted by one or more substituents independently selected from deuterium, halogen, -CN, - OH, -NH₂, -CHO, C₁-₃alkyl, C₁₋₃alkoxy, -N(C₁-₃alkyl)₂, -NH(C₁-₃alkyl), and -C(O)C₁-₃alkyl.

In one embodiment of the invention, each of rings W₁, W₂, W₃ is independently selected from the following groups optionally substituted: 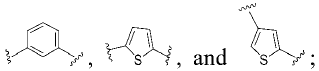 wherein "optionally substituted" refers to unsubstituted or substituted by one or more substituents independently selected from deuterium, F, Cl, Br, methyl, ethyl, methoxy, and ethoxy.

The invention also provides a compound represented by the following Formula (B-2), Formula (B-3), Formula (C-2), Formula (C-3), Formula (D-2), and Formula (D-3), or a pharmaceutically acceptable salt thereof: 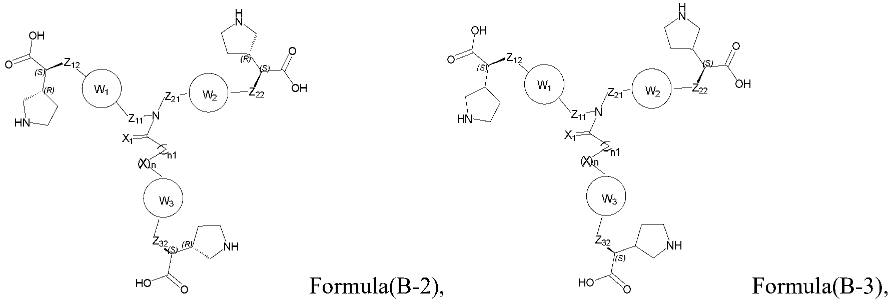 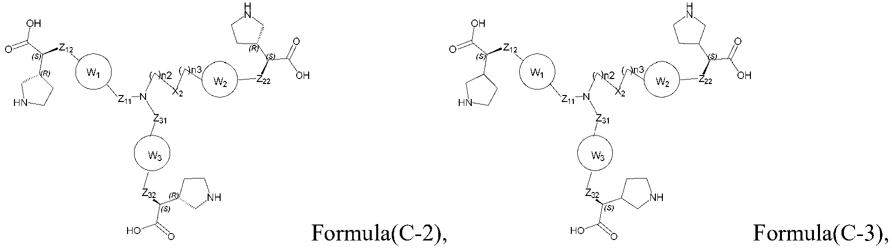 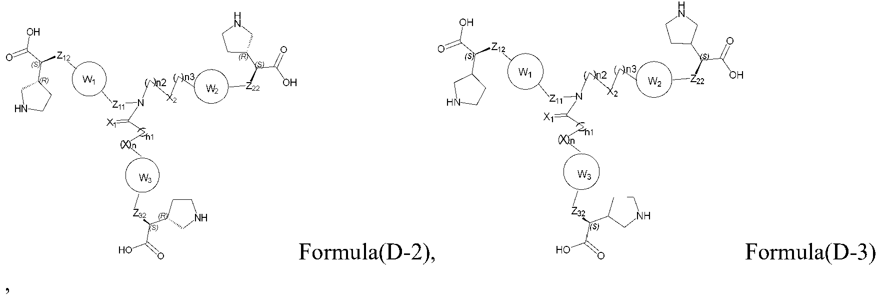 wherein, Z₁₁, Z₂₁, Z₁₂, Z₂₂, Z₃₂, X, X₁, n, n1, and rings W₁, W₂, and W₃ in Formula (B-2) and Formula (B-3) are defined as in the compound of Formula (B); Z₁₁, Z₃₁, Z₁₂, Z₂₂, Z₃₂, X₂, n2, n3, and rings W₁, W₂, and W₃ in Formula (C-2) and Formula (C-3) are defined as in the compound of Formula (C); and Z₁₁, Z₁₂, Z₂₂, Z₃₂, X, X₁, X₂, n, n1, n2, n3, and rings W₁, W₂, and W₃ in Formula (D-2) and Formula (D-3) are defined as in the compound of Formula (D).

For example, in any of the above-mentioned Formula (B-2) and Formula (B-3), each of Z₁₁ and Z₂₁ is a group not containing a double bond; the double bond is, for example, a double bond between C and O in -C(O)-, or a double bond between C and S in -C(S)-; further preferably, Z₁₁ and Z₂₁ are identical or different, and at least one of Z₁₁ and Z₂₁ is a group containing -O-, -NH-, - S-, or -Se-, preferably, a group containing -O-, e.g. *-CH₂O-, *-CH₂CH₂O-, * is the connecting end with N; still further preferably, Z₁₁, Z₂₁ and *-C(X₁)-(CH₂)ₙ₁-(X)ₙ- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, - OC(O)-, -CH₂CH₂O-, -OCH₂C(O)-, -CH₂OC(O)-, -CH₂CH₂CH₂O-, and -S-C(O)-.

For example, in any of the above-mentioned Formula (C-2) and Formula (C-3), one and only one of Z₁₁ and Z₃₁ is a group containing a double bond; the double bond is, for example, a double bond between C and O in -C(O)-, or a double bond between C and S in -C(S)-; further preferably, the group not containing a double bond of Z₁₁ and Z₃₁ is identical to or different from *-(CH₂)ₙ₂-X₂-(CH₂)ₙ₃- (* represents the connecting end with N); still further preferably, Z₁₁, -(CH₂)ₙ₂-X₂-(CH₂)ₙ₃- and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, - CH₂CH₂CH₂O-, and -S-C(O)-.

For example, in any of the above-mentioned Formula (D-2) and Formula (D-3), Z₁₁ is a group not containing a double bond; the double bond is, for example, a double bond between C and O in - C(O)-, or a double bond between C and S in -C(S)-; further preferably, Z₁₁ is identical to or different from *-(CH₂)ₙ₂-X₂-(CH₂)ₙ₃- (* represents the connecting end with N); still further preferably, Z₁₁, -C(X₁)-(CH₂)ₙ₁-(X)ₙ₋ and -(CH₂)ₙ₂-X₂-(CH₂)ₙ₃- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-.

The invention also provides a compound represented by the following Formula (E), Formula (E-1) and Formula (F), or a pharmaceutically acceptable salt thereof: 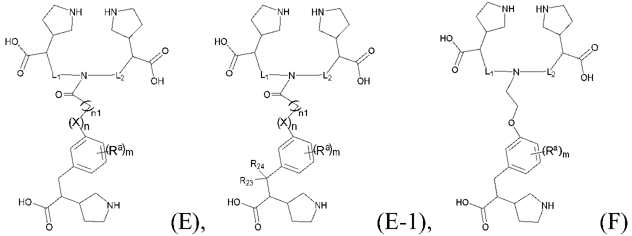 wherein, L₁ and L₂ are defined as in the compound of Formula (I), Formula (I-1), Formula (III), Formula (III-1), Formula (III-B), Formula (B), Formula (C) or Formula (D), X is O, CH₂ or NH, R^{a} is deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl, n1 is 0 or 1, m is 0 or 1, and n is 0 or 1; R₂₃ and R₂₄ are each independently selected from H, and deuterium.

For example, in any of the above-mentioned Formula (E) and Formula (E-1), L₁ and L₂ are groups containing Z₁₁ and Z₂₁ respectively, and each of Z₁₁ and Z₂₁ is a group not containing a double bond; the double bond is, for example, a double bond between C and O in -C(O)-, or a double bond between C and S in -C(S)-; further preferably, Z₁₁ and Z₂₁ are identical or different; still further preferably, Z₁₁, Z₂₁ and -C(O)-(CH₂)ₙ₁-(X)ₙ- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, - CH₂CH₂O-, -OCH₂C(O)-, -CH₂OC(O)-, -CH₂CH₂CH₂O-, and -S-C(O)-.

For example, in the above-mentioned Formula (F), L₁ and L₂ are groups containing Z₁₁ and Z₂₁ respectively, and
one and only one of Z₁₁ and Z₂₁ is a group containing a double bond; the double bond is, for example, a double bond between C and O in -C(O)-, or a double bond between C and S in -C(S)-; further preferably, the group not containing a double bond of Z₁₁ and Z₂₁ is identical to or different from *-(CH₂)₂-O- (*represents the connecting end with N); still further preferably, Z₁₁ and Z₂₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, - C(O)-, -CH₂C(O)-, -NHC(O)-, -OC(O)-, -CH₂CH₂O-, -OCH₂C(O)-, -CH₂OC(O)-, - CH₂CH₂CH₂O-, and -S-C(O)-.

In one embodiment of the invention, R^{a} is F, Cl, methyl, ethyl, methoxy and ethoxy; preferably R^{a} is F.

In one embodiment of the invention, m is 1.

In one embodiment of the invention, X is CH₂.

In one embodiment of the invention, X is selected from O, CH₂, NH, and S, n is 1, and n1 is 0; or X is O or CH₂, n is 1, and n1 is 1.

In one embodiment of the invention, the sum of n and n1 is 0, 1, or 2; preferably the sum of n and n1 is 1 or 2 (i.e., n is 1 and n1 is 0; or n is 0 and n1 is 1; or n is 1 and n1 is 1; or n is 0 and n1 is 2; or n is 2 and n1 is 0); preferably the sum of n and n1 is 1 (i.e., n is 1 and n1 is 0; or n is 0 and n1 is 1).

For example, in any of the above-mentioned Formula (E), Formula (E-1), and Formula (F), R^{a} is F, Cl, methyl, ethyl, methoxy, or ethoxy, m is 1, X is selected from O, CH₂, NH, and S, the sum of n and n1 is 0, 1, or 2.
further preferably, R^{a} is F, or methoxy, m is 1, X is selected from O, CH₂, NH, and S, the sum of n and n1 is 1, or 2.

In one embodiment of the invention, L₁ is 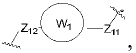 L₂ is 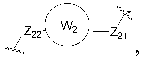 and * represents the connecting end with N; each of rings W₁ and W₂ is independently selected from 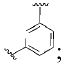 the phenyl is optionally substituted by one or more substituents independently selected from halogen and C₁₋₃alkoxy (preferably F and methoxy).

In one embodiment of the invention, each of Z₁₁, Z₁₂, Z₂₁ and Z₂₂ is independently selected from - C₁₋₂alkylene-, -OC₁₋₃alkyl-, -NH-C₁₋₂alkylene-, -S-C₁₋₂alkylene-, -C₁₋₂alkylene-O-C₁₋₂alkylene-, - C₁₋₂alkylene-C(O)-, -OC₁₋₂alkylene-C(O)-, -C₁₋₂alkylene-NH-C(O)-, -C₁₋₂alkylene-O-C(O)-, - NH-C(O)-, -NH- C₁₋₂alkyl-C(O)-, -O-C(O)-, and -S-C(O)-.

In one embodiment of the invention, each of Z₁₁, Z₁₂, Z₂₁, and Z₂₂ is independently selected from methylene, ethylene, 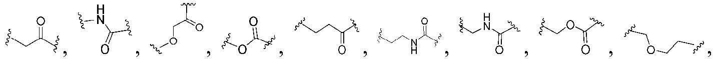 and

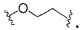

In one embodiment of the invention, each of L₁ and L₂ is independently selected from 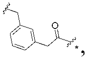 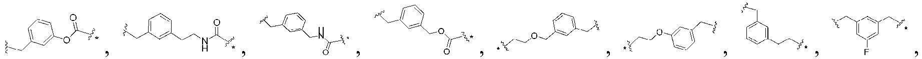 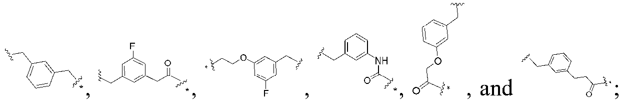 * indicates the connecting end with N.

The invention also provides a compound represented by the following Formula (E-2), Formula (E-3), Formula (E-4),Formula (F-1), and Formula (F-2), or a pharmaceutically acceptable salt thereof: 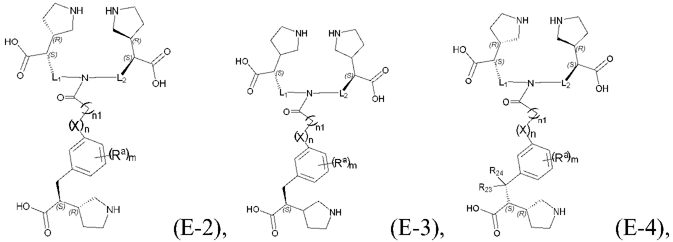 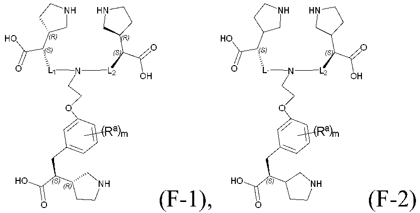 wherein, L₁, L₂, X, m, n, n1, and R^{a} in Formula (E-2), Formula (E-3) and Formula (E-4) are defined as in Formula (E) and Formula (E-1); R₂₃ and R₂₄ in Formula (E-4) are defined as in Formula (E-1); and L₁ and L₂ in Formula (F-1) and Formula (F-2) are defined as in Formula (F).

For example, in any of the above-mentioned Formula (E-2), Formula (E-3), and Formula (E-4), L₁ and L₂ are groups containing Z₁₁ and Z₂₁ respectively, and each of Z₁₁ and Z₂₁ is a group not containing a double bond; the double bond is, for example, a double bond between C and O in - C(O)-, or a double bond between C and S in -C(S)-; further preferably, Z₁₁ and Z₂₁ are identical or different; still further preferably, Z₁₁, Z₂₁ and -C(O)-(CH₂)ₙ₁-(X)ₙ- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, - OC(O)-, -CH₂CH₂O-, -OCH₂C(O)-, -CH₂OC(O)-, -CH₂CH₂CH₂O-, and -S-C(O)-.

For example, in any of the above-mentioned Formula (F-1) and Formula (F-2), L₁ and L₂ are groups containing Z₁₁ and Z₂₁ respectively, and one and only one of Z₁₁ and Z₂₁ is a group containing a double bond; the double bond is, for example, a double bond between C and O in - C(O)-, or a double bond between C and S in -C(S)-; further preferably, the group not containing a double bond of Z₁₁ and Z₂₁ is identical to or different from *-(CH₂)₂-O- (*represents the connecting end with N); still further preferably, Z₁₁ and Z₂₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-.

The invention also provides a compound represented by the following Formula (G), or a pharmaceutically acceptable salt thereof: 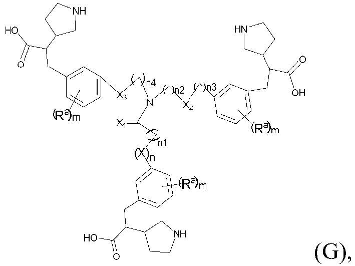 wherein, X, X₁, X₂, n, n1, n2, and n3 are defined as in the compounds of Formula (I), Formula (I-1-P1), Formula (I-1-P2), Formula (I-1-P3), Formula (I-1-P4), Formula (III), Formula (III-1), Formula (III-B), Formula (B), Formula (C), or Formula (D), m is independently 0 or 1, n4 is 0, 1, 2, or 3, X₃ is selected from O, S, CH₂, and NH; or X₃ is O, S, CH₂, NH, or CD₂, R^{a} is selected from deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl.

In one embodiment of the invention, R^{a} is F, Cl, methyl, ethyl, methoxy, ethoxy; preferably F and methoxy.

In one embodiment of the invention, m is 1.

In one embodiment of the invention, n4 is 0, 1, or 2, preferably 0 or 1 and more preferably 0.

In one embodiment of the invention, X₃ is O or CH₂; or X₃ is O, CH₂, or CD₂.

In one embodiment of the invention, X₃ is O and n4 is 2; or X₃ is CH₂ and n4 is 0 or 1; or X₃ is CH₂, or CD₂, n4 is 0 or 1; further preferably, X₃ is CH₂, or CD₂, n4 is 0.

For example, in the above-mentioned Formula (G), R^{a} is F, Cl, methyl, ethyl, methoxy, or ethoxy;
m is 1; X₂ is O, NH, S, or Se, the sum of n2 and n3 is 1, 2, or 3; X₁ is O or S, X is selected from O, CH₂, NH, and S, the sum of n and n1 is 0, 1, or 2;
X₃ is O, n4 is 2; or X₃ is CH₂, or CD₂, n4 is 0 or 1.
further preferably, R^{a} is F, Cl, or methoxy; m is 1; X₂ is O, the sum of n2 and n3 is 2, or 3; X₁ is O, X is selected from O, CH₂, NH, and S, the sum of n and n1 is 1, or 2;
X₃ is O, n4 is 2; or X₃ is CH₂, or CD₂, n4 is 0 or 1.

For example, in the above-mentioned Formula (G), R^{a} is F, Cl, methyl, ethyl, methoxy, or ethoxy;
m is 1;
X₃ is O, n4 is 2; or X₃ is CH₂, or CD₂, n4 is 0 or 1;
n2 is 2 or 3, n3 is 0, X₂ is O, NH, S; or n2 is 1 or 2, n3 is 1, X₂ is O, NH, S; or n2 is 0 or 1, n3 is 0, X₂ is CH₂;
X₁ is O or S, X is selected from O, CH₂, NH, and S, n is 1, n1 is 0; or X₁ is O or S, X is O or CH₂, n is 1, n1 is 1.

For example, in the above-mentioned Formula (G), R^{a} is F, Cl, or methoxy; m is 1;
X₃ is O, n4 is 2; or X₃ is CH₂, or CD₂, n4 is 0 or 1;
n2 is 2 or 3, n3 is 0, X₂ is O; or n2 is 1 or 2, n3 is 1, X₂ is O; or n2 is 0 or 1, n3 is 0, X₂ is CH₂;
X₁ is O, X is selected from O, CH₂, NH, and S, n is 1, n1 is 0; or X₁ is O, X is O or CH₂, n is 1, n1 is 1.

The invention also provides a compound represented by the following Formula (G-1) and Formula (G-2), or a pharmaceutically acceptable salt thereof: 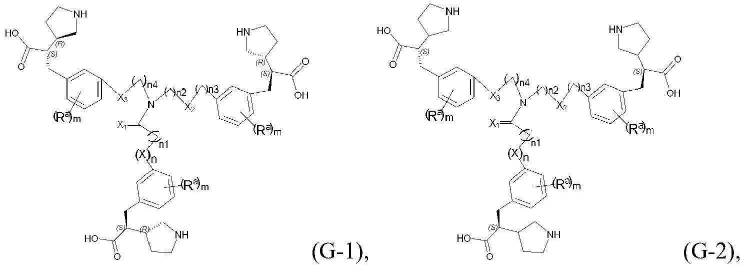 wherein, X, X₁, X₂, X₃, n, n1, n2, n3, n4, m, and R^{a} are defined as in the compound of Formula (G).

The invention also provides a compound represented by the following Formula (G-3) or Formula (G-4), or a pharmaceutically acceptable salt thereof: 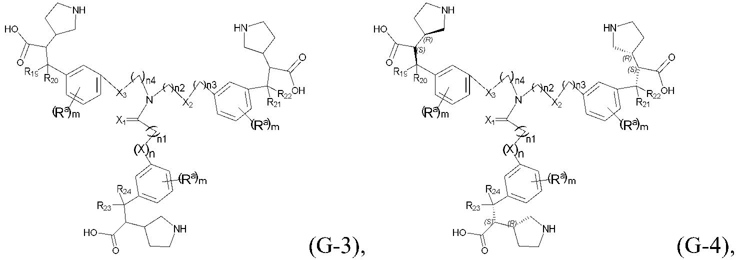 wherein, X, X₁, X₂, X₃, n, n1, n2, n3, n4, m, and R^{a} are defined as in Formula (G), and R₁₉, R₂₀, R₂₁, R₂₂, R₂₃, and R₂₄ are each independently selected from hydrogen and deuterium.

For example, in any of the above-mentioned Formula (G), Formula (G-1), Formula (G-2), Formula (G-3), and Formula (G-4), *-(CH₂)ₙ₄-X₃-, *-(CH₂)ₙ₂-X₂-(CH₂)ₙ₃-, and *-C(X₁)-(CH₂)ₙ₁-(X)ₙ- (* represents the connecting end with N) are identical to or different from each other, and they each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, - CH₂C(O)-, -NHC(O)-, -OC(O)-, -CH₂CH₂O-, -OCH₂C(O)-, -CH₂OC(O)-, -CH₂CH₂CH₂O-, and - S-C(O)-.

The invention also provides a compound represented by the following Formula (H), or a pharmaceutically acceptable salt thereof: 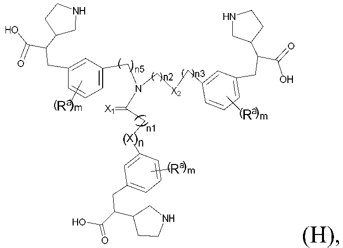 wherein, X, X₁, X₂, n, n1, n2, and n3 are defined as in the compound of Formula (I), Formula (I-1-P1), Formula (I-1-P2), Formula (I-1-P3), Formula (I-1-P4), Formula (III), Formula (III-1), Formula (III-B), Formula (B), Formula (C), Formula (D), or Formula (G), m is independently 0 or 1, n5 is 1, 2, 3 or 4, and R^{a} is selected from deuterium, halogen, -CN, -OH, -NH₂, -CHO, C₁-₃alkyl, C₁₋₃alkoxy, -N(C₁-₃alkyl)₂, -NH(C₁-₃alkyl), and -C(O)C₁-₃ alkyl.

In one embodiment of the invention, R^{a} is F, Cl, methyl, ethyl, methoxy, or ethoxy; preferably R^{a} is F or methoxy.

In one embodiment of the invention, m is 1.

In one embodiment of the invention, n5 is 1, 2, or 3, preferably 1 or 2 and more preferably 1.

The invention also provides a compound represented by the following Formula (H-1) and Formula (H-2), or a pharmaceutically acceptable salt thereof: 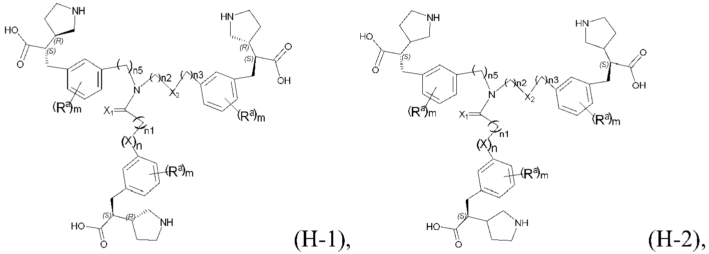 wherein, X, X₁, X₂, n, n1, n2, n3, n5, m, and R^{a} are defined as in the compound of Formula (H).

The invention also provides a compound represented by the following Formula (H-3) and Formula (H-4), or a pharmaceutically acceptable salt thereof: 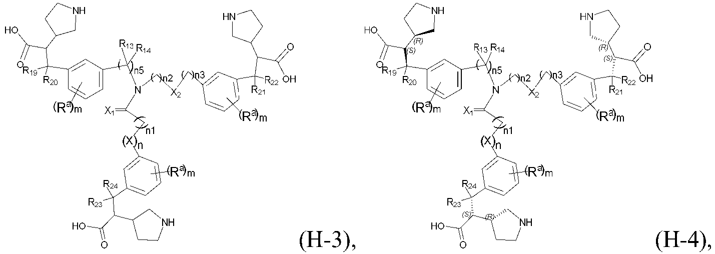 wherein, X, X₁, X₂, n, n1, n2, n3, n5, m, and R^{a} are defined as in Formula (H), and R₁₃, R₁₄, R₁₉, R₂₀, R₂₁, R₂₂, R₂₃, and R₂₄ are each independently selected from hydrogen and deuterium.

The invention also provides a compound represented by Formula (D-c), Formula (D-f), Formula (D-g), or Formula (D-h) or a pharmaceutically acceptable salt thereof: 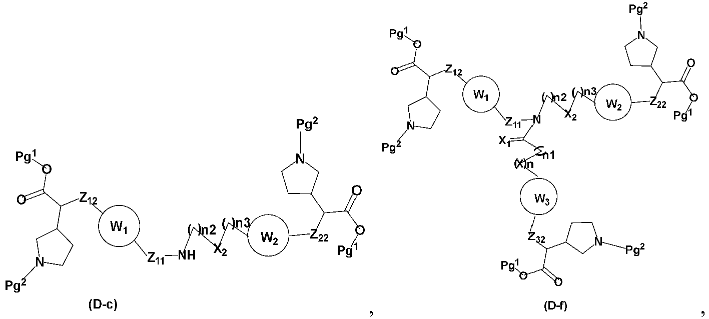 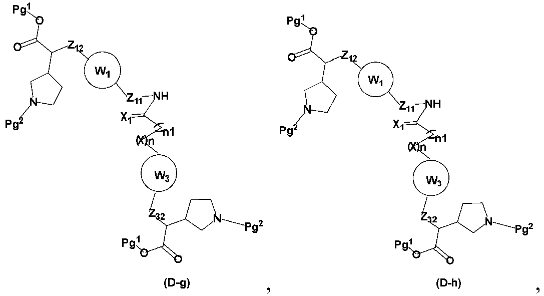 wherein, Z₁₁, Z₁₂, Z₂₂, Z₃₂, X, X₁, X₂, n, n1, n2, n3, and rings W₁, W₂, and W₃ are defined as in the compound of Formula (I), Formula (I-1-P1), Formula (I-1-P2), Formula (I-1-P3), Formula (I-1-P4), or Formula (D), each of Pg¹ and Pg² is independently a protecting group. Preferably, Pg¹ is independently selected from tert-butyl, methyl, and benzyl, Pg² is independently selected from tert-butoxycarbonyl (Boc), 9-fluorenylmethoxycarbonyl (FMOC), and andbenzyloxycarbonyl (Cbz), preferably Boc.

Based on the common knowledge in the field, the above specific technical features can be combined arbitrarily to obtain various preferred embodiments of the invention.

In one embodiment of the invention, the compounds of the invention or a pharmaceutically acceptable salt thereof are selected from:

| Co mp. No. | Structure | Co mp. No. | Structure |
|---|---|---|---|
| 1-a | 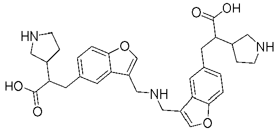 3,3'-((azanediylbis(methylene))bis(benzofuran-3,5-diyl))bis(2-(pyrrolidin-3-yl)propanoic acid) | 2-a | 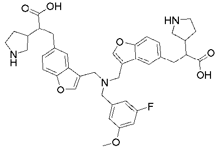 3,3'-((((3-fluoro-5-methoxybenzyl)azanediyl)bis(methylene))bis( benzofuran-3,5-diyl))bis(2-(pyrrolidin-3-yl)propanoic acid) |
| 3-a | 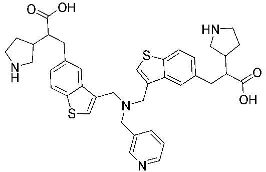 3,3'-((((pyridin-3-ylmethyl)azanediyl)bis(methylene))bis(ben zo[b]thiophene-3,5-diyl))bis(2-(pyrrolidin-3-yl)propanoic acid) | 4-a | 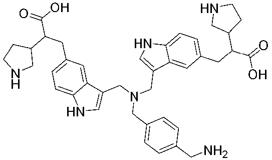 3,3'-((((4-(aminomethyl)benzyl)azanediyl)bis(methylen e))bis(1H-indole-3,5-diyl))bis(2-(pyrrolidin-3-yl)propanoic acid) |
| 5-a | 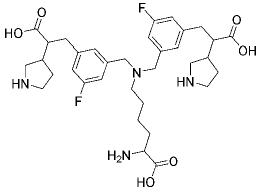 3,3'-((((5-amino-5-carboxypentyl)azanediyl)bis(methylene))bi s(5-fluoro-3,1-phenylene))bis(2-(pyrrolidin-3-yl)propanoic acid) | 6-a | 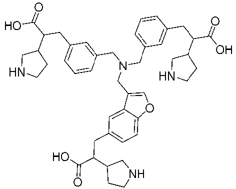 3,3'-(((((5-(2-carboxy-2-(pyrrolidin-3-yl)ethyl)benzofuran-3-yl)methyl)azanediyl)bis(methylene))bis(3,1-phenylene))bis(2-(pyrrolidin-3-yl)propanoic acid) |
| 7-a | 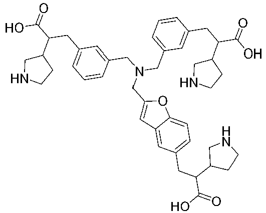 3,3'-(((((5-(2-carboxy-2-(pyrrolidin-3-yl)ethyl)benzofuran-2-yl)methyl)azanediyl)bis(methylene))bis(3, 1-phenylene))bis(2-(pyrrolidin-3-yl)propanoic acid) | 8-a | 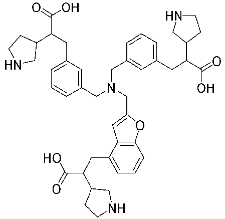 3,3'-(((((4-(2-carboxy-2-(pyrrolidin-3-yl)ethyl)benzofuran-2-yl)methyl)azanediyl)bis(methylene))bis(3,1-phenylene))bis(2-(pyrrolidin-3-yl)propanoic acid) |
| 9-a | 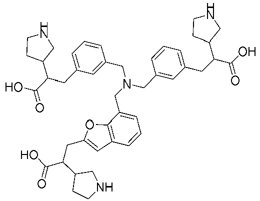 3,3'-(((((2-(2-carboxy-2-(pyrrolidin-3-yl)ethyl)benzofuran-7-yl)methyl)azanediyl)bis(methylene))bis(3, 1-phenylene))bis(2-(pyrrolidin-3-yl)propanoic acid) | 10-a | 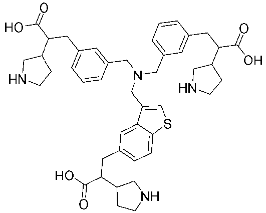 3,3'-(((((5-(2-carboxy-2-(pyrrolidin-3-yl)ethyl)benzo[b]thiophen-3-yl)methyl)azanediyl)bis(methylene))bis(3,1-phenylene))bis(2-(pyrrolidin-3-yl)propanoic acid) |
| 11-a | 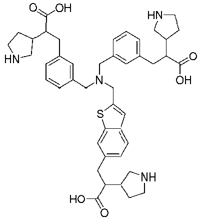 3,3'-(((((6-(2-carboxy-2-(pyrrolidin-3-yl)ethyl)benzo[b]thiophen-2-yl)methyl)azanediyl)bis(methylene))bis(3, 1-phenylene))bis(2-(pyrrolidin-3-yl)propanoic acid) | 12-a | 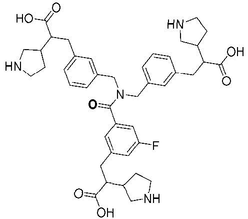 3,3'-((((3-(2-carboxy-2-(pyrrolidin-3-yl)ethyl)-5-fluorobenzoyl)azanediyl)bis(methylene))bis(3 ,1-phenylene))bis(2-(pyrrolidin-3-yl)propanoic acid) |
| 13-a | 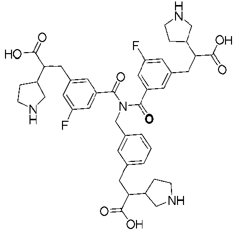 3,3'-((((3-(2-carboxy-2-(pyrrolidin-3-yl)ethyl)benzyl)azanediyl)bis(carbonyl))bis (5-fluoro-3,1-phenylene))bis(2-(pyrrolidin-3-yl)propanoic acid) | 14-a | 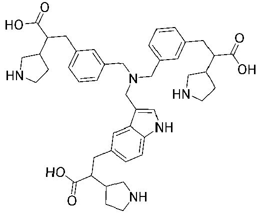 3,3'-(((((5-(2-carboxy-2-(pyrrolidin-3-yl)ethyl)-1H-indol-3-yl)methyl)azanediyl)bis(methylene))bis(3,1-phenylene))bis(2-(pyrrolidin-3-yl)propanoic acid) |
| 15-a | 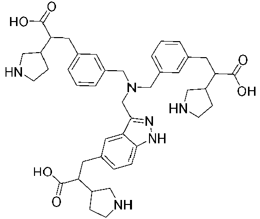 3,3'-(((((5-(2-carboxy-2-(pyrrolidin-3-yl)ethyl)-1H-indazol-3-yl)methyl)azanediyl)bis(methylene))bis(3, 1-phenylene))bis(2-(pyrrolidin-3-yl)propanoic acid) | 16-a | 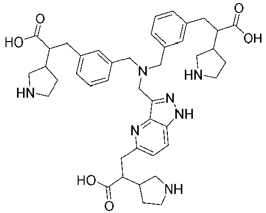 3,3'-(((((5-(2-carboxy-2-(pyrrolidin-3-yl)ethyl)-1H-pyrazolo[4,3-b]pyridin-3-yl)methyl)azanediyl)bis(methylene))bis(3,1-phenylene))bis(2-(pyrrolidin-3-yl)propanoic acid) |
| 17-a | 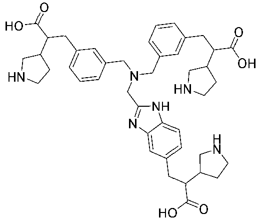 3,3'-(((((5-(2-carboxy-2-(pyrrolidin-3-yl)ethyl)-1H-benzo[d]imidazol-2-yl)methyl)azanediyl)bis(methylene))bis(3, 1-phenylene))bis(2-(pyrrolidin-3-yl)propanoic acid) | 18-a | 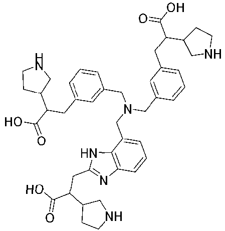 3,3'-(((((2-(2-carboxy-2-(pyrrolidin-3-yl)ethyl)-1H-benzo[d]imidazol-7-yl)methyl)azanediyl)bis(methylene))bis(3,1-phenylene))bis(2-(pyrrolidin-3-yl)propanoic acid) |
| 19-a | 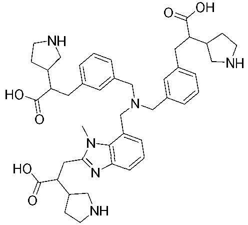 3,3'-(((((2-(2-carboxy-2-(pyrrolidin-3-yl)ethyl)-1-methyl-1H-benzo[d]imidazol-7-yl)methyl)azanediyl)bis(methylene))bis(3, 1-phenylene))bis(2-(pyrrolidin-3-yl)propanoic acid) | 20-a | 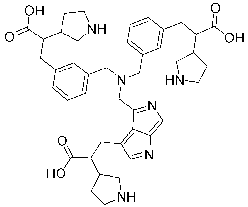 3,3'-(((((3-(2-carboxy-2-(pyrrolidin-3-yl)ethyl)pyrrolo[3,4-b]pyrrol-4-yl)methyl)azanediyl)bis(methylene))bis(3,1-phenylene))bis(2-(pyrrolidin-3-yl)propanoic acid) |
| 21-a | 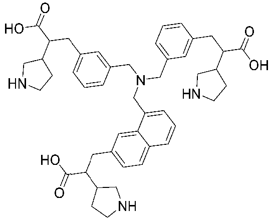 3,3'-(((((7-(2-carboxy-2-(pyrrolidin-3-yl)ethyl)naphthalen-1-yl)methyl)azanediyl)bis(methylene))bis(3, 1-phenylene))bis(2-(pyrrolidin-3-yl)propanoic acid) | 22-a | 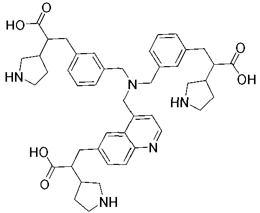 3,3'-(((((6-(2-carboxy-2-(pyrrolidin-3-yl)ethyl)quinolin-4-yl)methyl)azanediyl)bis(methylene))bis(3,1-phenylene))bis(2-(pyrrolidin-3-yl)propanoic acid) |
| 23-a | 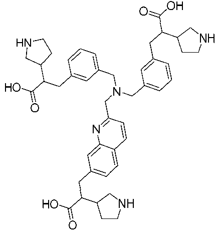 3,3'-(((((7-(2-carboxy-2-(pyrrolidin-3-yl)ethyl)quinolin-2-yl)methyl)azanediyl)bis(methylene))bis(3, 1-phenylene))bis(2-(pyrrolidin-3-yl)propanoic acid) | 24-a | 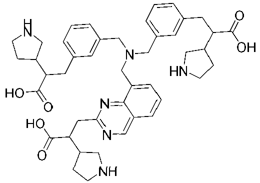 3,3'-(((((2-(2-carboxy-2-(pyrrolidin-3-yl)ethyl)quinazolin-8-yl)methyl)azanediyl)bis(methylene))bis(3,1-phenylene))bis(2-(pyrrolidin-3-yl)propanoic acid) |
| 25-a | 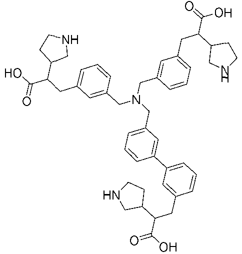 3,3'-(((((3'-(2-carboxy-2-(pyrrolidin-3-yl)ethyl)-[1,1'-biphenyl]-3-yl)methyl)azanediyl)bis(methylene))bis(3, 1-phenylene))bis(2-(pyrrolidin-3-yl)propanoic acid) | 26-a | 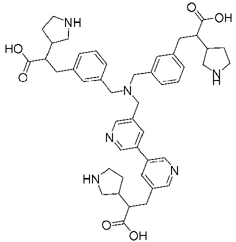 3,3'-(((((5'-(2-carboxy-2-(pyrrolidin-3-yl)ethyl)-[3,3'-bipyridin]-5-yl)methyl)azanediyl)bis(methylene))bis(3,1-phenylene))bis(2-(pyrrolidin-3-yl)propanoic acid) |
| 27-a | 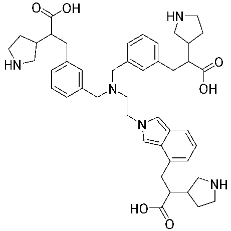 3,3'-((((2-(4-(2-carboxy-2-(pyrrolidin-3-yl)ethyl)-2H-isoindol-2-yl)ethyl)azanediyl)bis(methylene))bis(3,1-phenylene))bis(2-(pyrrolidin-3-yl)propanoic acid) | 28-a | 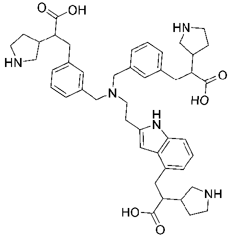 3,3'-((((2-(4-(2-carboxy-2-(pyrrolidin-3-yl)ethyl)-1H-indol-2-yl)ethyl)azanediyl)bis(methylene))bis(3,1-phenylene))bis(2-(pyrrolidin-3-yl)propanoic acid) |
| 29-a | 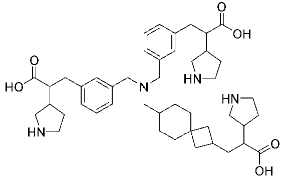 3,3'-(((((2-(2-carboxy-2-(pyrrolidin-3-yl)ethyl)spiro[3.5]nonan-7-yl)methyl)azanediyl)bis(methylene))bis(3, 1-phenylene))bis(2-(pyrrolidin-3-yl)propanoic acid) | 30-a | 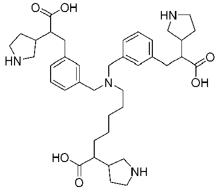 3,3'-((((6-carboxy-6-(pyrrolidin-3-yl)hexyl)azanediyl)bis(methylene))bis(3,1-phenylene))bis(2-(pyrrolidin-3-yl)propanoic acid) |
| 31-a | 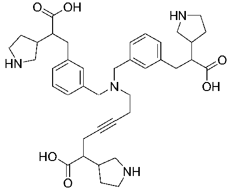 3,3'-((((6-carboxy-6-(pyrrolidin-3-yl)hex-3-yn-1-yl)azanediyl)bis(methylene))bis(3,1-phenylene))bis(2-(pyrrolidin-3-yl)propanoic acid) | 32-a | 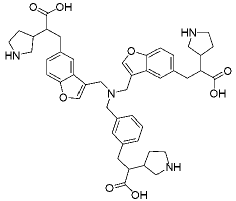 3,3'-((((3-(2-carboxy-2-(pyrrolidin-3-yl)ethyl)benzyl)azanediyl)bis(methylene))bis( benzofuran-3,5-diyl))bis(2-(pyrrolidin-3-yl)propanoic acid) |
| 33-a | 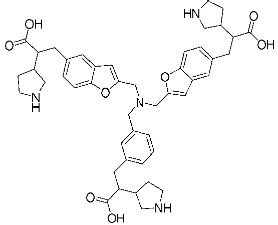 3,3'-((((3-(2-carboxy-2-(pyrrolidin-3-yl)ethyl)benzyl)azanediyl)bis(methylene))b is(benzofuran-2,5-diyl))bis(2-(pyrrolidin-3-yl)propanoic acid) | 34-a | 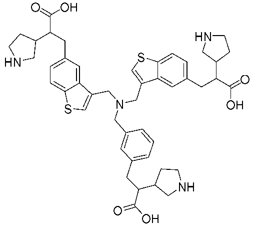 3,3'-((((3-(2-carboxy-2-(pyrrolidin-3-yl)ethyl)benzyl)azanediyl)bis(methylene))bis( benzo[b]thiophene-3,5-diyl))bis(2-(pyrrolidin-3-yl)propanoic acid) |
| 35-a | 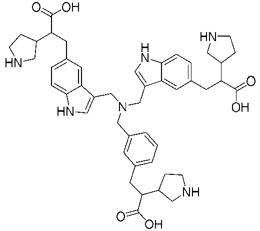 3,3'-((((3-(2-carboxy-2-(pyrrolidin-3-yl)ethyl)benzyl)azanediyl)bis(methylene))b is(1H-indole-3,5-diyl))bis(2-(pyrrolidin-3-yl)propanoic acid) | 36-a | 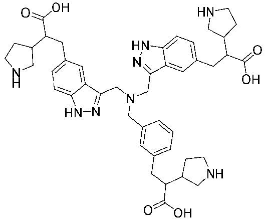 3,3'-((((3-(2-carboxy-2-(pyrrolidin-3-yl)ethyl)benzyl)azanediyl)bis(methylene))bis( 1H-indazole-3,5-diyl))bis(2-(pyrrolidin-3-yl)propanoic acid) |
| 37-a | 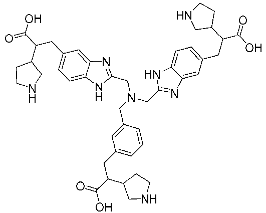 3,3'-((((3-(2-carboxy-2-(pyrrolidin-3-yl)ethyl)benzyl)azanediyl)bis(methylene))b is(1H-benzo[d]imidazole-2,5-diyl))bis(2-(pyrrolidin-3-yl)propanoic acid) | 38-a | 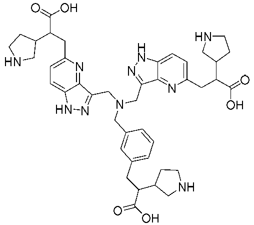 3,3'-((((3-(2-carboxy-2-(pyrrolidin-3-yl)ethyl)benzyl)azanediyl)bis(methylene))bis( 1H-pyrazolo[4,3-b]pyridine-3,5-diyl))bis(2-(pyrrolidin-3-yl)propanoic acid) |
| 39-a | 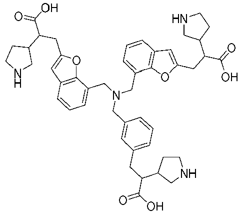 3,3'-((((3-(2-carboxy-2-(pyrrolidin-3-yl)ethyl)benzyl)azanediyl)bis(methylene))b is(benzofuran-7,2-diyl))bis(2-(pyrrolidin-3-yl)propanoic acid) | 40-a | 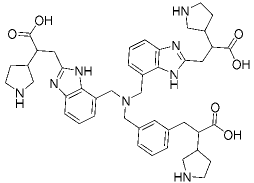 3,3'-((((3-(2-carboxy-2-(pyrrolidin-3-yl)ethyl)benzyl)azanediyl)bis(methylene))bis( 1H-benzo[d]imidazole-7,2-diyl))bis(2-(pyrrolidin-3-yl)propanoic acid) |
| 41-a | 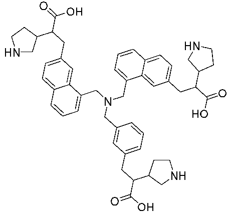 3,3'-((((3-(2-carboxy-2-(pyrrolidin-3-yl)ethyl)benzyl)azanediyl)bis(methylene))b is(naphthalene-8,2-diyl))bis(2-(pyrrolidin-3-yl)propanoic acid) | 42-a | 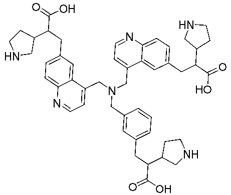 3,3'-((((3-(2-carboxy-2-(pyrrolidin-3-yl)ethyl)benzyl)azanediyl)bis(methylene))bis( quinoline-4,6-diyl))bis(2-(pyrrolidin-3-yl)propanoic acid) |
| 43-a | 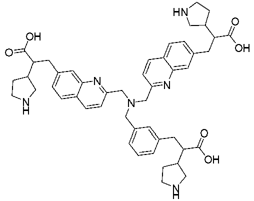 3,3'-((((3-(2-carboxy-2-(pyrrolidin-3-yl)ethyl)benzyl)azanediyl)bis(methylene))b is(quinoline-2,7-diyl))bis(2-(pyrrolidin-3-yl)propanoic acid) | 44-a | 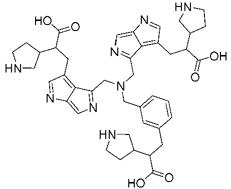 3,3'-((((3-(2-carboxy-2-(pyrrolidin-3-yl)ethyl)benzyl)azanediyl)bis(methylene))bis( pyrrolo[3,4-b]pyrrole-4,3-diyl))bis(2-(pyrrolidin-3-yl)propanoic acid) |
| 45-a | 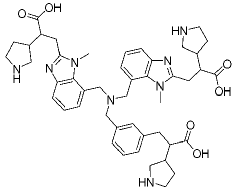 3,3'-((((3-(2-carboxy-2-(pyrrolidin-3-yl)ethyl)benzyl)azanediyl)bis(methylene))b is(1-methyl-1H-benzo[d]imidazole-7,2-diyl))bis(2-(pyrrolidin-3-yl)propanoic acid) | 46-a | 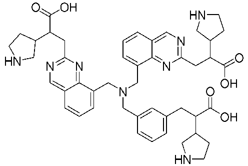 3,3'-((((3-(2-carboxy-2-(pyrrolidin-3-yl)ethyl)benzyl)azanediyl)bis(methylene))bis( quinazoline-8,2-diyl))bis(2-(pyrrolidin-3-yl)propanoic acid) |
| 47-a | 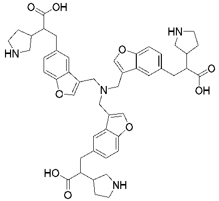 3,3',3"-((nitrilotris(methylene))tris(benzofuran-3,5-diyl))tris(2-(pyrrolidin-3-yl)propanoic acid) | 48-a | 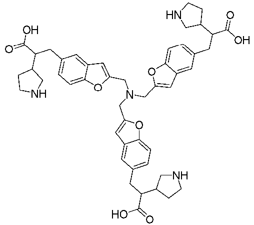 3,3',3"-((nitrilotris(methylene))tris(benzofuran-2,5-diyl))tris(2-(pyrrolidin-3-yl)propanoic acid) |
| 49-a | 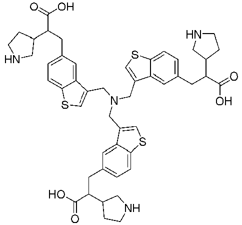 3,3',3"-((nitrilotris(methylene))tris(benzo[b]thioph ene-3,5-diyl))tris(2-(pyrrolidin-3-yl)propanoic acid) | 50-a | 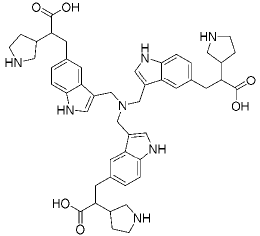 3,3',3"-((nitrilotris(methylene))tris(1H-indole-3,5-diyl))tris(2-(pyrrolidin-3-yl)propanoic acid) |
| 51-a | 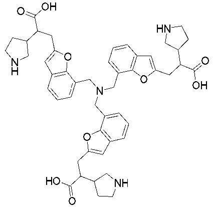 3,3',3"-((nitrilotris(methylene))tris(benzofuran-7,2-diyl))tris(2-(pyrrolidin-3-yl)propanoic acid) | 52-a | 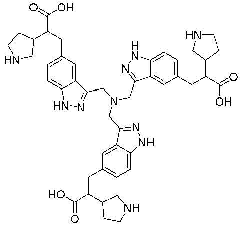 3,3',3"-((nitrilotris(methylene))tris(1H-indazole-3,5-diyl))tris(2-(pyrrolidin-3-yl)propanoic acid) |
| 53-a | 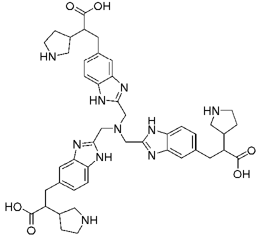 3,3',3"-((nitrilotris(methylene))tris(1H-benzo[d]imidazole-2,5-diyl))tris(2-(pyrrolidin-3-yl)propanoic acid) | 54-a | 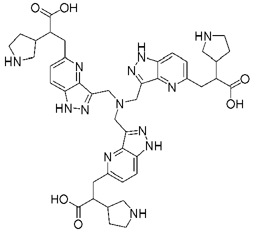 3,3',3"-((nitrilotris(methylene))tris(1H-pyrazolo[4,3-b]pyridine-3,5-diyl))tris(2-(pyrrolidin-3-yl)propanoic acid) |
| 55-a | 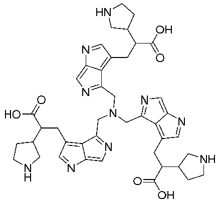 3,3',3"-((nitrilotris(methylene))tris(pyrrolo[3,4-b]pyrrole-4,3-diyl))tris(2-(pyrrolidin-3-yl)propanoic acid) | 56-a | 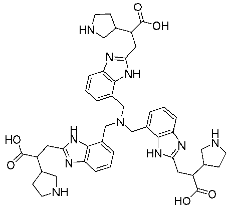 3,3',3"-((nitrilotris(methylene))tris(1H-benzo[d]imidazole-7,2-diyl))tris(2-(pyrrolidin-3-yl)propanoic acid) |
| 57-a | 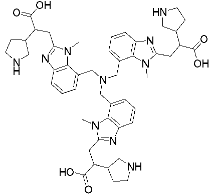 3,3',3 "-((nitrilotris(methylene))tris(1-methyl-1H-benzo[d]imidazole-7,2-diyl))tris(2-(pyrrolidin-3-yl)propanoic acid) | 58-a | 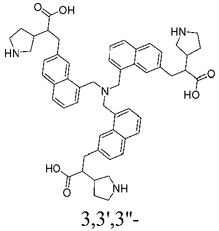 ((nitrilotris(methylene))tris(naphthalene-8,2-diyl))tris(2-(pyrrolidin-3-yl)propanoic acid) |
| 59-a | 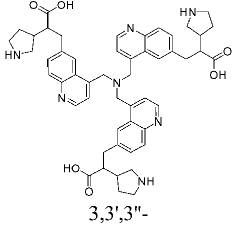 ((nitrilotris(methylene))tris(quinoline-4,6-diyl))tris(2-(pyrrolidin-3-yl)propanoic acid) | 60-a | 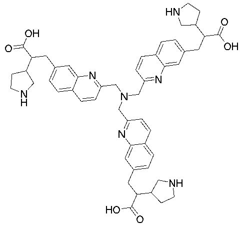 3,3',3 "-((nitrilotris(methylene))tris(quinoline-2,7-diyl))tris(2-(pyrrolidin-3-yl)propanoic acid) |
| 61-a | 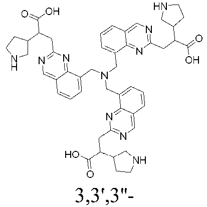 ((nitrilotris(methylene))tris(quinazoline-8,2-diyl))tris(2-(pyrrolidin-3-yl)propanoic acid) | 62-a | 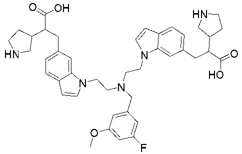 3,3'-((((3-fluoro-5-methoxybenzyl)azanediyl)bis(ethane-2,1-diyl))bis(1H-indole-1,6-diyl))bis(2-(pyrrolidin-3-yl)propanoic acid) |
| 63-a | 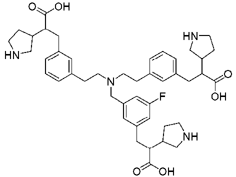 3,3'-((((3-(2-carboxy-2-(pyrrolidin-3-yl)ethyl)-5-fluorobenzyl)azanediyl)bis(ethane-2,1-diyl))bis(3,1-phenylene))bis(2-(pyrrolidin-3-yl)propanoic acid) | 64-a | 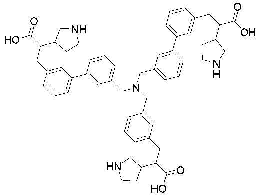 3,3'-((((3-(2-carboxy-2-(pyrrolidin-3-yl)ethyl)benzyl)azanediyl)bis(methylene))bis( [1,1'-biphenyl]-3',3-diyl))bis(2-(pyrrolidin-3-yl)propanoic acid) |
| 65-a | 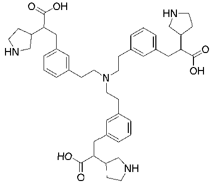 3,3',3"-((nitrilotris(ethane-2,1-diyl))tris(benzene-3,1-diyl))tris(2-(pyrrolidin-3-yl)propanoic acid) | 66-a | 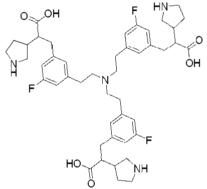 3,3',3"-((nitrilotris(ethane-2,1-diyl))tris(5-fluorobenzene-3,1-diyl))tris(2-(pyrrolidin-3-yl)propanoic acid) |
| 67-a | 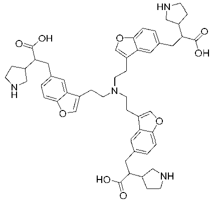 3,3',3"-((nitrilotris(ethane-2,1-diyl))tris(benzofuran-3,5-diyl))tris(2-(pyrrolidin-3-yl)propanoic acid) | 68-a | 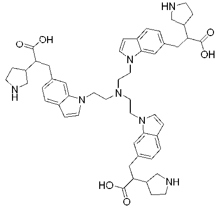 3,3',3"-((nitrilotris(ethane-2,1-diyl))tris(1H-indole-1,6-diyl))tris(2-(pyrrolidin-3-yl)propanoic acid) |
| 69-a | 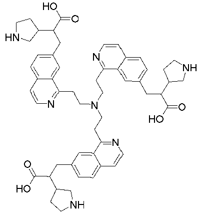 3,3',3"-((nitrilotris(ethane-2,1-diyl))tris(isoquinoline-1,7-diyl))tris(2-(pyrrolidin-3-yl)propanoic acid) | 70-a | 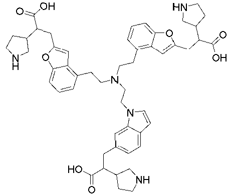 3,3'-((((2-(6-(2-carboxy-2-(pyrrolidin-3-yl)ethyl)-1H-indol-1-yl)ethyl)azanediyl)bis(ethane-2,1-diyl))bis(benzofuran-4,2-diyl))bis(2-(pyrrolidin-3-yl)propanoic acid) |
| 71-a | 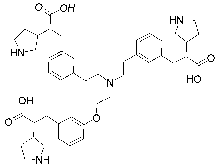 3,3'-((((2-(3-(2-carboxy-2-(pyrrolidin-3-yl)ethyl)phenoxy)ethyl)azanediyl)bis(ethan e-2,1-diyl))bis(3,1-phenylene))bis(2-(pyrrolidin-3-yl)propanoic acid) | 72-a | 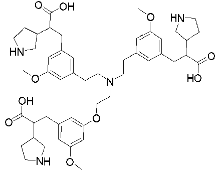 3,3'-((((2-(3-(2-carboxy-2-(pyrrolidin-3-yl)ethyl)-5-methoxyphenoxy)ethyl)azanediyl)bis(ethane-2,1-diyl))bis(5-methoxy-3,1-phenylene))bis(2-(pyrrolidin-3-yl)propanoic acid) |
| 73-a | 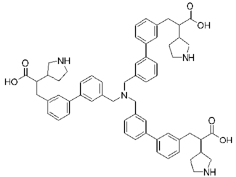 3,3',3"-((nitrilotris(methylene))tris([1,1'-biphenyl]-3',3-diyl))tris(2-(pyrrolidin-3-yl)propanoic acid) | 74-a | 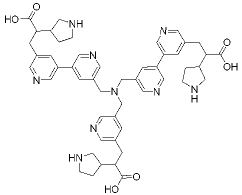 3,3'-(((((5-(2-carboxy-2-(pyrrolidin-3-yl)ethyl)pyridin-3-yl)methyl)azanediyl)bis(methylene))bis([3,3'-bipyridine]-5',5-diyl))bis(2-(pyrrolidin-3-yl)propanoic acid) |
| 75-a | 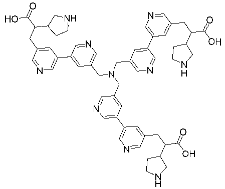 3,3',3 "-((nitrilotris(methylene))tris([3,3'-bipyridine]-5',5-diyl))tris(2-(pyrrolidin-3-yl)propanoic acid) | 76-a | 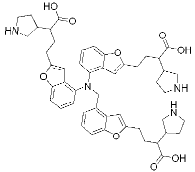 4,4'-((((2-(3-carboxy-3-(pyrrolidin-3-yl)propyl)benzofuran-4-yl)methyl)azanediyl)bis(benzofuran-4,2-diyl))bis(2-(pyrrolidin-3-yl)butanoic acid) |
| 77-a | 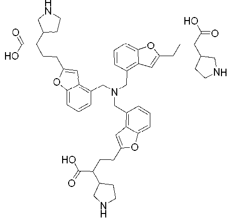 4,4',4"-((nitrilotris(methylene))tris(benzofuran-4,2-diyl))tris(2-(pyrrolidin-3-yl)butanoic acid) | 78-a | 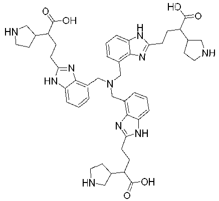 4,4',4"-((nitrilotris(methylene))tris(1H-benzo[d]imidazole-4,2-diyl))tris(2-(pyrrolidin-3-yl)butanoic acid) |
| 79-a | 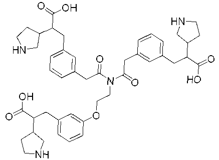 3,3'-((((2-(3-(2-carboxy-2-(pyrrolidin-3-yl)ethyl)phenoxy)ethyl)azanediyl)bis(2-oxoethane-2,1-diyl))bis(3,1-phenylene))bis(2-(pyrrolidin-3-yl)propanoic acid) | 80-a | 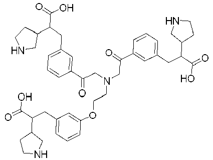 3,3'-((2,2'-((2-(3-(2-carboxy-2-(pyrrolidin-3-yl)ethyl)phenoxy)ethyl)azanediyl)bis(acetyl)) bis(3,1-phenylene))bis(2-(pyrrolidin-3-yl)propanoic acid) |
| 81-a | 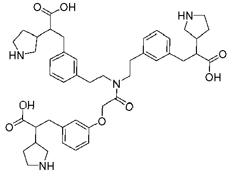 3,3'-((((2-(3-(2-carboxy-2-(pyrrolidin-3-yl)ethyl)phenoxy)acetyl)azanediyl)bis(etha ne-2,1-diyl))bis(3,1-phenylene))bis(2-(pyrrolidin-3-yl)propanoic acid) | 82-a | 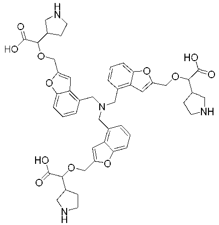 2,2',2"-((((nitrilotris(methylene))tris(benzofuran-4,2-diyl))tris(methylene))tris(oxy))tris(2-(pyrrolidin-3-yl)acetic acid) |
| 85-a | 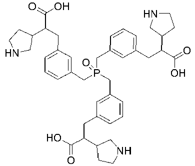 3,3',3"-(((oxo-15-phosphanetriyl)tris(methylene))tris(benzen e-3,1-diyl))tris(2-(pyrrolidin-3-yl)propanoic acid) | 86-a | 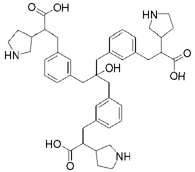 3-[3-(3-{3-[2-carboxy-2-(tetrahydro-1*H-*pyrrol-3-yl)ethyljphenyl} -2-(13-[2-carboxy-2-(tetrahydro-1*H*-pyrrol-3-yl)ethyl]phenyl}methyl)-2-hydroxypropyl)phenyl]-2-(tetrahydro-1*H-*pyrrol-3-yl)propanoic acid |
| 87-a | 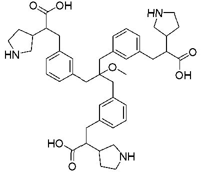 3-[3-(3-{3-[2-carboxy-2-(tetrahydro-1*H-*pyrrol-3-yl)ethyl]phenyl}-2-({3-[2-carboxy-2-(tetrahydro-1*H*-pyrrol-3-yl)ethyl]phenyl} methyl)-2-methoxypropyl)phenyl]-2-(tetrahydro-1*H-*pyrrol-3-yl)propanoic acid | 88-a | 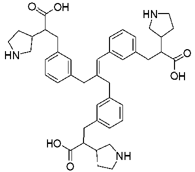 3-[3-(3- {3-[2-carboxy-2-(tetrahydro-1*H-*pyrrol-3-yl)ethyl]phenyl}-2-({3-[2-carboxy-2-(tetrahydro-1*H*-pyrrol-3-yl)ethyl]phenyl}methyl)prop-2-enyl)phenyl]-2-(tetrahydro-1*H*-pyrrol-3-yl)propanoic acid |
| 89-a | 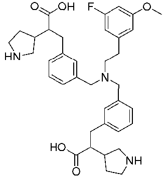 3,3'-((((3-fluoro-5-methoxyphenethyl)azanediyl)bis(methylen e))bis(3,1-phenylene))bis(2-(pyrrolidin-3-yl)propanoic acid) | 90-a | 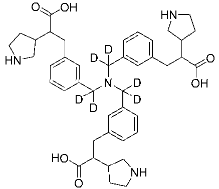 3,3',3" -((nitrilotris(methylene-d2))tris(benzene-3,1-diyl))tris(2-(pyrrolidin-3-yl)propanoic acid) |
| 91-a | 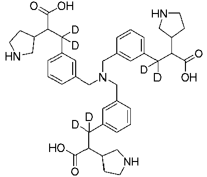 3,3',3"-((nitrilotris(methylene))tris(benzene-3,1-diyl))tris(2-(pyrrolidin-3-yl)propanoic-3,3-d2 acid) | 92-a | 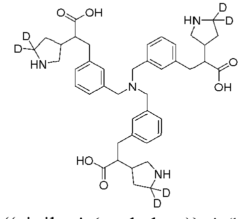 3,3',3"-((nitrilotris(methylene))tris(benzene-3,1-diyl))tris(2-(pyrrolidin-3-yl-5,5-d2)propanoic acid) |
| 93-a | 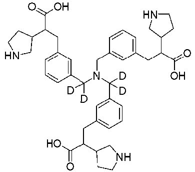 3,3'-((((3-(2-carboxy-2-(pyrrolidin-3-yl)ethyl)benzyl)azanediyl)bis(methylene-d2))bis(3,1-phenylene))bis(2-(pyrrolidin-3-yl)propanoic acid) | 94-a | 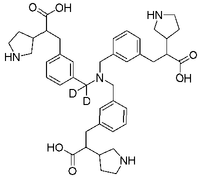 3,3'-(((((3-(2-carboxy-2-(pyrrolidin-3-yl)ethyl)phenyl)methyl-d2)azanediyl)bis(methylene))bis(3,1-phenylene))bis(2-(pyrrolidin-3-yl)propanoic acid) |
| 95-a | 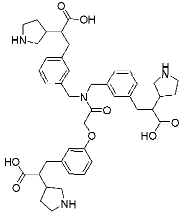 3,3'-((((2-(3-(2-carboxy-2-(pyrrolidin-3-yl)ethyl)phenoxy)acetyl)azanediyl)bis(met hylene))bis(3,1-phenylene))bis(2-(pyrrolidin-3-yl)propanoic acid) | 96-a | 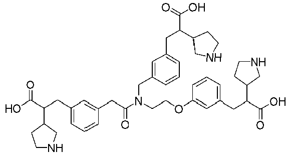 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-oxoethyl)phenyl)-2-(pyrrolidin-3-yl)propanoic acid |
| 97-a | 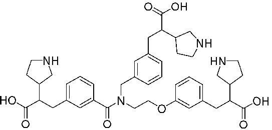 3-(3-((3-(2-carboxy-2-(pyrrolidin-3-yl)ethyl)-N-(2-(3-(2-carboxy-2-(pyrrolidin-3-yl)ethyl)phenoxy)ethyl)benzamido)methyl) phenyl)-2-(pyrrolidin-3-yl)propanoic acid | 98-a | 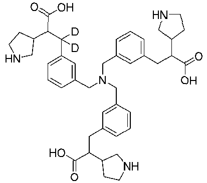 3,3'-((((3-(2-carboxy-2-(pyrrolidin-3-yl)ethyl-1,1-d2)benzyl)azanediyl)bis(methylene))bis(3,1-phenylene))bis(2-(pyrrolidin-3-yl)propanoic acid) |
| 99-a | 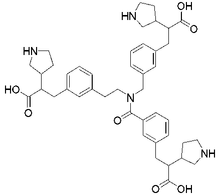 3-(3-(2-(3-(2-carboxy-2-(pyrrolidin-3-yl)ethyl)-N-(3-(2-carboxy-2-(pyrrolidin-3-yl)ethyl)benzyl)benzamido)ethyl)phenyl)-2-(pyrrolidin-3-yl)propanoic acid | 100-a | 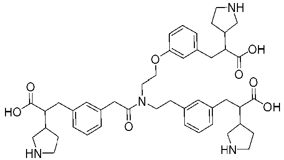 3-(3-(2-((3-(2-carboxy-2-(pyrrolidin-3-yl)ethyl)phenethyl)(2-(3-(2-carboxy-2-(pyrrolidin-3-yl)ethyl)phenoxy)ethyl)amino)-2-oxoethyl)phenyl)-2-(pyrrolidin-3-yl)propanoic acid |
| 101-a | 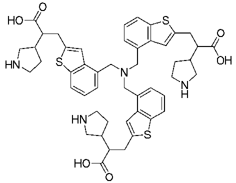 3,3',3"-((nitrilotris(methylene))tris(benzo[b]thioph ene-4,2-diyl))tris(2-(pyrrolidin-3-yl)propanoic acid) | 102-a | 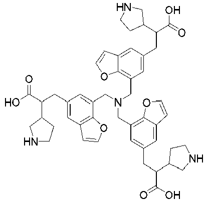 3,3',3"-((nitrilotris(methylene))tris(benzofuran-7,5-diyl))tris(2-(pyrrolidin-3-yl)propanoic acid) |
| 103-a | 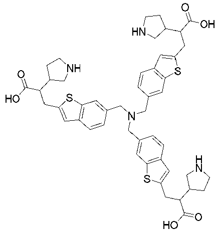 3,3',3"-((nitrilotris(methylene))tris(benzo[b]thioph ene-6,2-diyl))tris(2-(pyrrolidin-3-yl)propanoic acid) | 104-a | 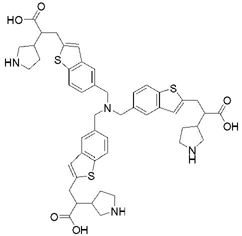 3,3',3"-((nitrilotris(methylene))tris(benzo[b]thiophen e-5,2-diyl))tris(2-(pyrrolidin-3-yl)propanoic acid) |
| 105-a | 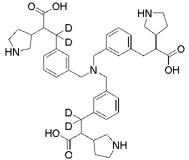 3,3'-((((3-(2-carboxy-2-(pyrrolidin-3-yl)ethyl)benzyl)azanediyl)bis(methylene))b is(3,1-phenylene))bis(2-(pyrrolidin-3-yl)propanoic-3,3-d2 acid) | 106-a | 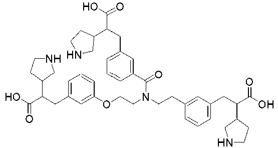 3-(3-(2-(3-(2-carboxy-2-(pyrrolidin-3-yl)ethyl)-N-(2-(3-(2-carboxy-2-(pyrrolidin-3-yl)ethyl)phenoxy)ethyl)benzamido)ethyl)phen yl)-2-(pyrrolidin-3-yl)propanoic acid |
| 107-a | 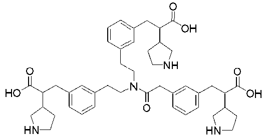 3,3'-((((2-(3-(2-carboxy-2-(pyrrolidin-3-yl)ethyl)phenyl)acetyl)azanediyl)bis(ethan e-2,1-diyl))bis(3,1-phenylene))bis(2-(pyrrolidin-3-yl)propanoic acid) | 108-a | 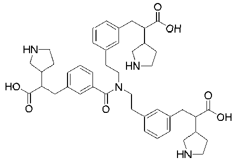 3,3'-((((3-(2-carboxy-2-(pyrrolidin-3-yl)ethyl)benzoyl)azanediyl)bis(ethane-2,1-diyl))bis(3,1-phenylene))bis(2-(pyrrolidin-3-yl)propanoic acid) |
| 109-a | 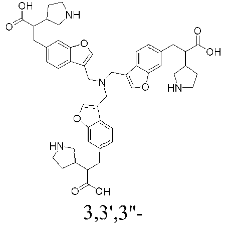 ((nitrilotris(methylene))tris(benzofuran-3,6-diyl))tris(2-(pyrrolidin-3-yl)propanoic acid) | 110-a | 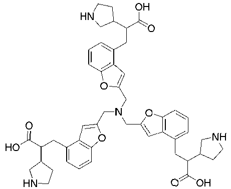 3,3',3"-((nitrilotris(methylene))tris(benzofuran-2,4-diyl))tris(2-(pyrrolidin-3-yl)propanoic acid) |
| 111-a | 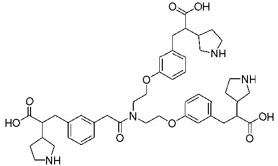 3,3'-(((((2-(3-(2-carboxy-2-(pyrrolidin-3-yl)ethyl)phenyl)acetyl)azanediyl)bis(ethan e-2,1-diyl))bis(oxy))bis(3,1-phenylene))bis(2-(pyrrolidin-3-yl)propanoic acid) | 112-a | 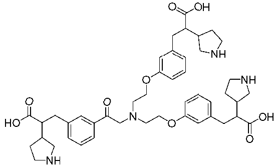 3,3'-(((((2-(3-(2-carboxy-2-(pyrrolidin-3-yl)ethyl)phenyl)-2-oxoethyl)azanediyl)bis(ethane-2,1-diyl))bis(oxy))bis(3,1-phenylene))bis(2-(pyrrolidin-3-yl)propanoic acid) |
| 113-a | 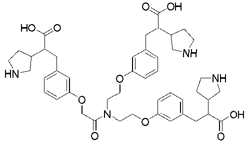 3,3'-(((((2-(3-(2-carboxy-2-(pyrrolidin-3-yl)ethyl)phenoxy)acetyl)azanediyl)bis(etha ne-2,1-diyl))bis(oxy))bis(3,1-phenylene))bis(2-(pyrrolidin-3-yl)propanoic acid) | 114-a | 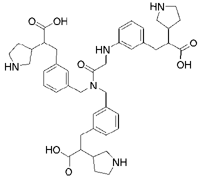 3,3'-(((((3-(2-carboxy-2-(pyrrolidin-3-yl)ethyl)phenyl)glycyl)azanediyl)bis(methyle ne))bis(3,1-phenylene))bis(2-(pyrrolidin-3-yl)propanoic acid) |
| 115-a | 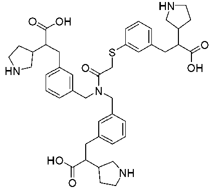 3,3'-((((2-((3-(2-carboxy-2-(pyrrolidin-3-yl)ethyl)phenyl)thio)acetyl)azanediyl)bis( methylene))bis(3,1-phenylene))bis(2-(pyrrolidin-3-yl)propanoic acid) | 116-a | 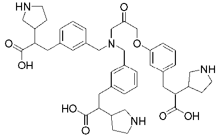 3,3'-((((3-(3-(2-carboxy-2-(pyrrolidin-3-yl)ethyl)phenoxy)-2-oxopropyl)azanediyl)bis(methylene))bis(3,1-phenylene))bis(2-(pyrrolidin-3-yl)propanoic acid) |
| 117-a | 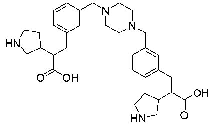 3,3'-((piperazine-1,4-diylbis(methylene))bis(3,1-phenylene))bis(2-(pyrrolidin-3-yl)propanoic acid) | 118-a | 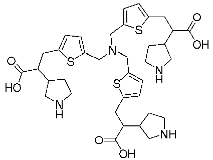 3,3',3"-((nitrilotris(methylene))tris(thiophene-5,2-diyl))tris(2-(pyrrolidin-3-yl)propanoic acid) |
| 119-a | 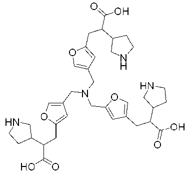 3,3'-(((((4-(2-carboxy-2-(pyrrolidin-3-yl)ethyl)furan-2-yl)methyl)azanediyl)bis(methylene))bis(fur an-4,2-diyl))bis(2-(pyrrolidin-3-yl)propanoic acid) | 120-a | 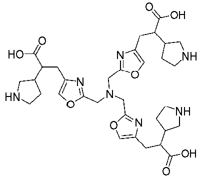 3,3',3 "-((nitrilotris(methylene))tris(oxazole-2,4-diyl))tris(2-(pyrrolidin-3-yl)propanoic acid) |
| 121-a | 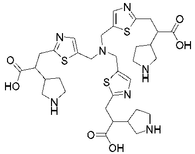 3,3',3"-((nitrilotris(methylene))tris(thiazole-5,2-diyl))tris(2-(pyrrolidin-3-yl)propanoic acid) | 122-a | 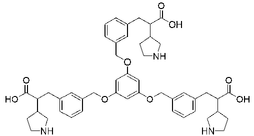 3,3',3 "-(((benzene-1,3,5-triyltris(oxy))tris(methylene))tris(benzene-3,1-diyl))tris(2-(pyrrolidin-3-yl)propanoic acid) |
| 123-a | 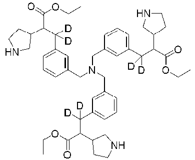 triethyl 3,3',3"-((nitrilotris(methylene))tris(benzene-3,1-diyl))tris(2-(pyrrolidin-3-yl)propanoate-3',3'-d2) | 124-a | 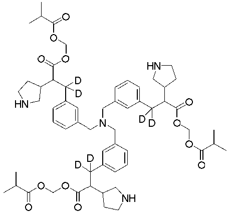 tris((isobutyryloxy)methyl) 3,3',3"-((nitrilotris(methylene))tris(benzene-3,1-diyl))tris(2-(pyrrolidin-3-yl)propanoate-3',3'-d2) |
| 126-a | 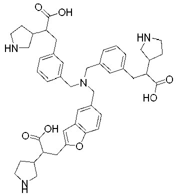 3,3'-(((((2-(2-carboxy-2-(pyrrolidin-3-yl)ethyl)benzofuran-5-yl)methyl)azanediyl)bis(methylene))bis(3, 1-phenylene))bis(2-(pyrrolidin-3-yl)propanoic acid) | 127-a | 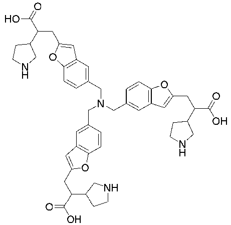 3,3',3"-((nitrilotris(methylene))tris(benzofuran-5,2-diyl))tris(2-(pyrrolidin-3-yl)propanoic acid) |
| 128-a | 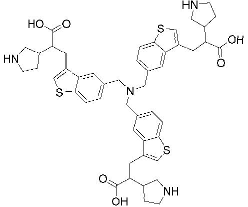 3,3',3"-((nitrilotris(methylene))tris(benzo[b]thioph ene-5,3-diyl))tris(2-(pyrrolidin-3-yl)propanoic acid) | 129-a | 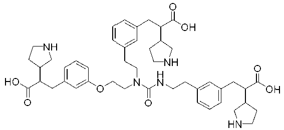 3-(3-(2-(1,3-bis(3-(2-carboxy-2-(pyrrolidin-3-yl)ethyl)phenethyl)ureido)ethoxy)phenyl)-2-(pyrrolidin-3-yl)propanoic acid |
| 130-a | 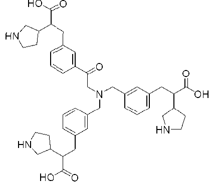 3,3'-((((2-(3-(2-carboxy-2-(pyrrolidin-3-yl)ethyl)phenyl)-2-oxoethyl)azanediyl)bis(methylene))bis(3,1-phenylene))bis(2-(pyrrolidin-3-yl)propanoic acid) | 131-a | 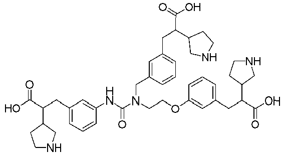 3-(3-(3-(3-(2-carboxy-2-(pyrrolidin-3-yl)ethyl)benzyl)-3-(2-(3-(2-carboxy-2-(pyrrolidin-3-yl)ethyl)phenoxy)ethyl)ureido)phenyl)-2-(pyrrolidin-3-yl)propanoic acid |
| 132-a | 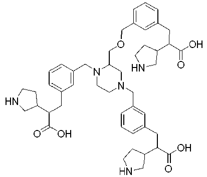 3,3'-(((2-(((3-(2-carboxy-2-(pyrrolidin-3-yl)ethyl)benzyl)oxy)methyl)piperazine-1,4-diyl)bis(methylene))bis(3,1-phenylene))bis(2-(pyrrolidin-3-yl)propanoic acid) | 133-a | 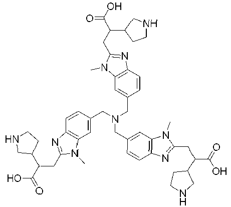 3,3',3"-((nitrilotris(methylene))tris(1-methyl-1H-benzo[d]imidazole-6,2-diyl))tris(2-(pyrrolidin-3-yl)propanoic acid) |
| 134-a | 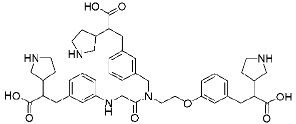 3-(3-((2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-oxoethyl)amino)phenyl)-2-(pyrrolidin-3-yl)propanoic acid | 135-a | 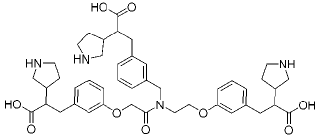 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-oxoethoxy)phenyl)-2-(pyrrolidin-3-yl)propanoic acid |
| 136-a | 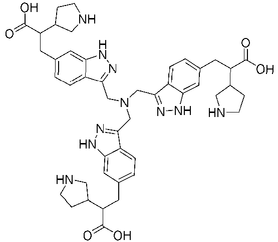 3,3',3"-((nitrilotris(methylene))tris(1H-indazole-3,6-diyl))tris(2-(pyrrolidin-3-yl)propanoic acid) | 137-a | 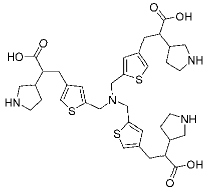 3,3',3"-((nitrilotris(methylene))tris(thiophene-5,3-diyl))tris(2-(pyrrolidin-3-yl)propanoic acid) |
| 138-a | 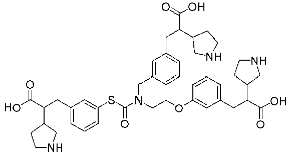 3-(3-(2-((3-(2-carboxy-2-(pyrrolidin-3-yl)ethyl)benzyl)(((3-(2-carboxy-2-(pyrrolidin-3-yl)ethyl)phenyl)thio)carbonyl)amino)ethox y)phenyl)-2-(pyrrolidin-3-yl)propanoic acid | 139-a | 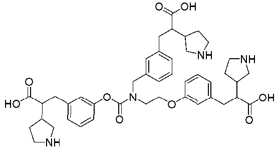 3-(3-(2-((3-(2-carboxy-2-(pyrrolidin-3-yl)ethyl)benzyl)((3-(2-carboxy-2-(pyrrolidin-3-yl)ethyl)phenoxy)carbonyl)amino)ethoxy)phe nyl)-2-(pyrrolidin-3-yl)propanoic acid |
| 140-a | 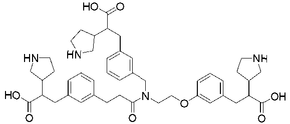 3-(3-(3-((3-(2-carboxy-2-(pyrrolidin-3-yl)ethyl)benzyl)(2-(3-(2-carboxy-2-(pyrrolidin-3-yl)ethyl)phenoxy)ethyl)amino)-3-oxopropyl)phenyl)-2-(pyrrolidin-3-yl)propanoic acid | 141-a | 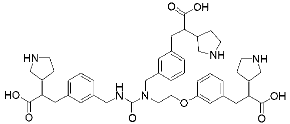 3-(3-(2-(1,3-bis(3-(2-carboxy-2-(pyrrolidin-3-yl)ethyl)benzyl)ureido)ethoxy)phenyl)-2-(pyrrolidin-3-yl)propanoic acid |
| 142-a | 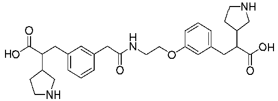 3-(3-(2-((2-(3-(2-carboxy-2-(pyrrolidin-3-yl)ethyl)phenoxy)ethyl)amino)-2-oxoethyl)phenyl)-2-(pyrrolidin-3-yl)propanoic acid | 143-a | 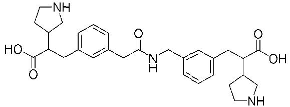 3-(3-(2-((3-(2-carboxy-2-(pyrrolidin-3-yl)ethyl)benzyl)amino)-2-oxoethyl)phenyl)-2-(pyrrolidin-3-yl)propanoic acid |
| 144-a | 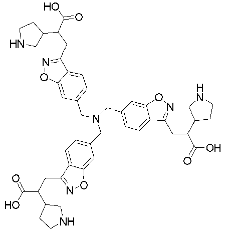 3,3',3"-((nitrilotris(methylene))tris(benzo[d]isoxaz ole-6,3-diyl))tris(2-(pyrrolidin-3-yl)propanoic acid) | 145 | 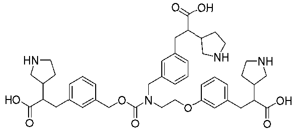 3-(3-(2-((3-(2-carboxy-2-(pyrrolidin-3-yl)ethyl)benzyl)(((3-(2-carboxy-2-(pyrrolidin-3-yl)ethyl)benzyl)oxy)carbonyl)amino)ethoxy)p henyl)-2-(pyrrolidin-3-yl)propanoic acid |
| 146-a | 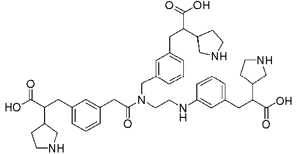 3-(3-(2-((3-(2-carboxy-2-(pyrrolidin-3-yl)ethyl)benzyl)(2-((3-(2-carboxy-2-(pyrrolidin-3-yl)ethyl)phenyl)amino)ethyl)amino)-2-oxoethyl)phenyl)-2-(pyrrolidin-3-yl)propanoic acid | 147-a | 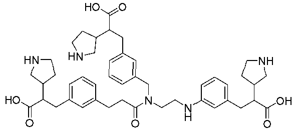 3-(3-(3-((3-(2-carboxy-2-(pyrrolidin-3-yl)ethyl)benzyl)(2-((3-(2-carboxy-2-(pyrrolidin-3-yl)ethyl)phenyl)amino)ethyl)amino)-3-oxopropyl)phenyl)-2-(pyrrolidin-3-yl)propanoic acid |
| 148-a | 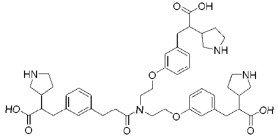 3,3'-(((((3-(3-(2-carboxy-2-(pyrrolidin-3-yl)ethyl)phenyl)propanoyl)azanediyl)bis(et hane-2,1-diyl))bis(oxy))bis(3,1-phenylene))bis(2-(pyrrolidin-3-yl)propanoic acid) | 149-a | 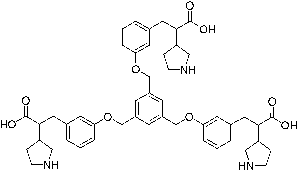 3,3',3 "-(((benzene-1,3,5-triyltris(methylene))tris(oxy))tris(benzene-3,1-diyl))tris(2-(pyrrolidin-3-yl)propanoic acid) |
| 150-a | 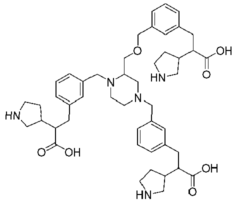 3,3'-(((2-(((3-(2-carboxy-2-(pyrrolidin-3-yl)ethyl)benzyl)oxy)methyl)piperazine-1,4-diyl)bis(methylene))bis(3,1-phenylene))bis(2-(pyrrolidin-3-yl)propanoic acid) | 152-a | 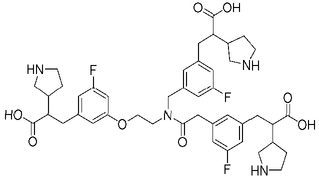 3-(3-(2-((3-(2-carboxy-2-(pyrrolidin-3-yl)ethyl)-5-fluorobenzyl)(2-(3-(2-carboxy-2-(pyrrolidin-3-yl)ethyl)-5-fluorophenoxy)ethyl)amino)-2-oxoethyl)-5-fluorophenyl)-2-(pyrrolidin-3-yl)propanoic acid |
| 153-a | 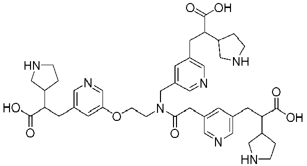 3-(5-(2-(2-(5-(2-carboxy-2-(pyrrolidin-3-yl)ethyl)pyridin-3-yl)-N-((5-(2-carboxy-2-(pyrrolidin-3-yl)ethyl)pyridin-3-yl)methyl)acetamido)ethoxy)pyridin-3-yl)-2-(pyrrolidin-3-yl)propanoic acid | 154-a | 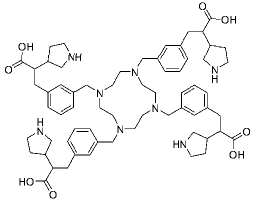 3,3',3 ",3 "'-(((1,4,7, 10-tetraazacyclododecane-1,4,7,10-tetrayl)tetrakis(methylene))tetrakis(benzene-3,1-diyl))tetrakis(2-(pyrrolidin-3-yl)propanoic acid) |
| *155-*a | 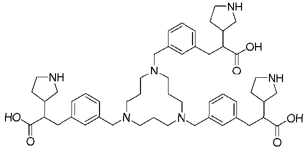 3,3',3"-(((1,5,9-triazacyclododecane-1,5,9-triyl)tris(methylene))tris(benzene-3,1-diyl))tris(2-(pyrrolidin-3-yl)propanoic acid) | 156-a | 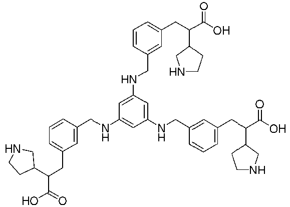 3,3',3 "-(((benzene-1,3,5-triyltris(azanediyl))tris(methylene))tris(benze ne-3,1-diyl))tris(2-(pyrrolidin-3-yl)propanoic acid) |
| 157-a | 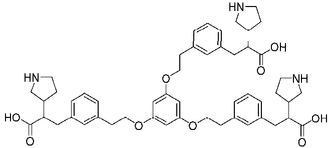 3,3',3 "-(((benzene-1,3,5-triyltris(oxy))tris(ethane-2,1-diyl))tris(benzene-3,1-diyl))tris(2-(pyrrolidin-3-yl)propanoic acid) | 158-a | 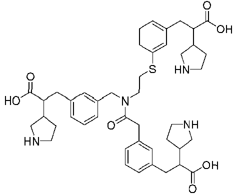 3-(3-(2-((3-(2-carboxy-2-(pyrrolidin-3-yl)ethyl)benzyl)(2-((3-(2-carboxy-2-(pyrrolidin-3-yl)ethyl)phenyl)thio)ethyl)amino)-2-oxoethyl)phenyl)-2-(pyrrolidin-3-yl)propanoic acid |
| 159-a | 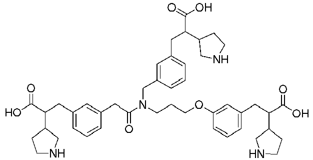 3-(3-(2-((3-(2-carboxy-2-(pyrrolidin-3-yl)ethyl)benzyl)(3-(3-(2-carboxy-2-(pyrrolidin-3-yl)ethyl)phenoxy)propyl)amino)-2-oxoethyl)phenyl)-2-(pyrrolidin-3-yl)propanoic acid | 160-a | 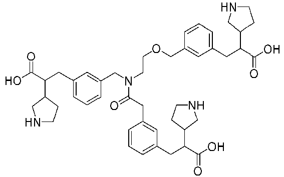 3-(3-(2-((3-(2-carboxy-2-(pyrrolidin-3-yl)ethyl)benzyl)(2-((3-(2-carboxy-2-(pyrrolidin-3-yl)ethyl)benzyl)oxy)ethyl)amino)-2-oxoethyl)phenyl)-2-(pyrrolidin-3-yl)propanoic acid |
| 161-a | 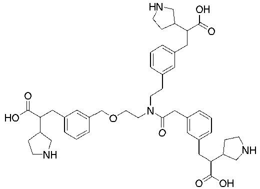 3-(3-(2-((2-((3-(2-carboxy-2-(pyrrolidin-3-yl)ethyl)benzyl)oxy)ethyl)(3-(2-carboxy-2-(pyrrolidin-3-yl)ethyl)phenethyl)amino)-2-oxoethyl)phenyl)-2-(pyrrolidin-3-yl)propanoic acid | 162-a | 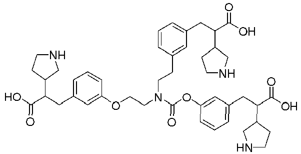 3-(3-(2-((3-(2-carboxy-2-(pyrrolidin-3-yl)ethyl)phenethyl)((3-(2-carboxy-2-(pyrrolidin-3-yl)ethyl)phenoxy)carbonyl)amino)ethoxy)phe nyl)-2-(pyrrolidin-3-yl)propanoic acid |
| 163-a | 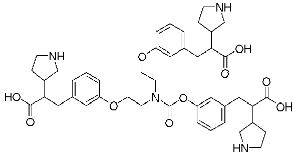 3,3'-((((((3-(2-carboxy-2-(pyrrolidin-3-yl)ethyl)phenoxy)carbonyl)azanediyl)bis(et hane-2,1-diyl))bis(oxy))bis(3,1-phenylene))bis(2-(pyrrolidin-3-yl)propanoic acid) | 164-a | 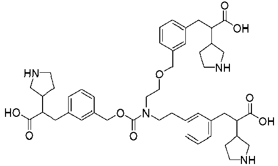 3-(3-(2-((((3-(2-carboxy-2-(pyrrolidin-3-yl)ethyl)benzyl)oxy)carbonyl)(2-((3-(2-carboxy-2-(pyrrolidin-3-yl)ethyl)benzyl)oxy)ethyl)amino)ethyl)phenyl )-2-(pyrrolidin-3-yl)propanoic acid |
| 165-a | 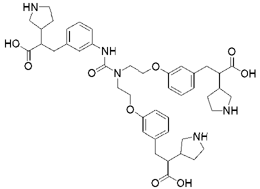 3,3'-((((((3-(2-carboxy-2-(pyrrolidin-3-yl)ethyl)phenyl)carbamoyl)azanediyl)bis(et hane-2,1-diyl))bis(oxy))bis(3,1-phenylene))bis(2-(pyrrolidin-3-yl)propanoic acid) | 166-a | 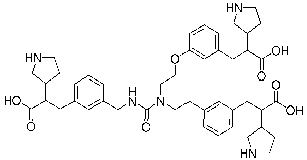 3-(3-(2-(3-(3-(2-carboxy-2-(pyrrolidin-3-yl)ethyl)benzyl)-1-(2-(3-(2-carboxy-2-(pyrrolidin-3-yl)ethyl)phenoxy)ethyl)ureido)ethyl)phenyl)-2-(pyrrolidin-3-yl)propanoic acid |
| 167-a | 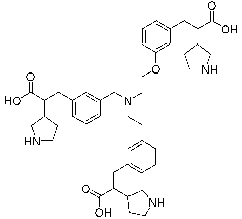 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)ethyl)phenyl )-2-(pyrrolidin-3-yl)propanoic acid | 168-a | 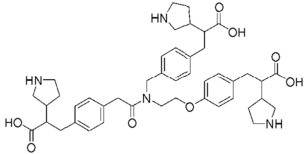 3-(4-(2-((4-(2-carboxy-2-(pyrrolidin-3-yl)ethyl)benzyl)(2-(4-(2-carboxy-2-(pyrrolidin-3-yl)ethyl)phenoxy)ethyl)amino)-2-oxoethyl)phenyl)-2-(pyrrolidin-3-yl)propanoic acid |
| 169-a | 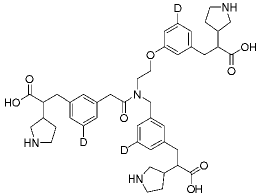 3-(3-{[(2-{5-[2-carboxy-2-(tetrahydro-1*H-*pyrrol-3-yl)ethyl]-3-deuteriophenyl}acetyl)[2-({3-[2-carboxy-2-(tetrahydro-1*H*-pyrrol-3-yl)ethyl]-5-deuteriophenyl}oxy)ethyl]amino]methyl}-5-deuteriophenyl)-2-(tetrahydro-1*H*-pyrrol-3-yl)propanoic acid | 170-a | 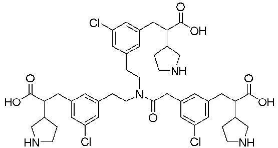 3,3'-((((2-(3-(2-carboxy-2-(pyrrolidin-3-yl)ethyl)-5-chlorophenyl)acetyl)azanediyl)bis(ethane-2,1-diyl))bis(5-chloro-3,1-phenylene))bis(2-(pyrrolidin-3-yl)propanoic acid) |
| 171-a | 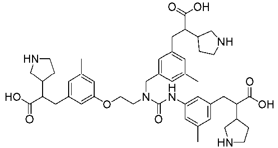 3-(3-(3-(3-(2-carboxy-2-(pyrrolidin-3-yl)ethyl)-5-methylbenzyl)-3-(2-(3-(2-carboxy-2-(pyrrolidin-3-yl)ethyl)-5-methylphenoxy)ethyl)ureido)-5-methylphenyl)-2-(pyrrolidin-3-yl)propanoic acid | 172-a | 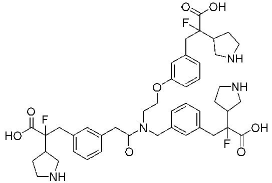 3-(3-(2-((3-(2-carboxy-2-fluoro-2-(pyrrolidin-3-yl)ethyl)benzyl)(2-(3-(2-carboxy-2-fluoro-2-(pyrrolidin-3-yl)ethyl)phenoxy)ethyl)amino)-2-oxoethyl)phenyl)-2-fluoro-2-(pyrrolidin-3-yl)propanoic acid |
| 173-a | 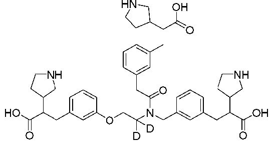 3-[3-({2-[(2-{3-[2-carboxy-2-(tetrahydro-1*H*-pyrrol-3-yl)ethyl]phenyl}acetyl)({3-[2-carboxy-2-(tetrahydro-1*H*-pyrrol-3-yl)ethyl]phenyl} methyl)amino]-2,2-dideuterioethyl}oxy)phenyl]-2-(tetrahydro-1*H*-pyrrol-3-yl)propanoic acid | 174-a | 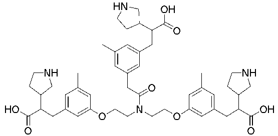 3,3'-(((((2-(3-(2-carboxy-2-(pyrrolidin-3-yl)ethyl)-5-methylphenyl)acetyl)azanediyl)bis(ethane-2,1-diyl))bis(oxy))bis(5-methyl-3,1-phenylene))bis(2-(pyrrolidin-3-yl)propanoic acid) |
| 175-a | 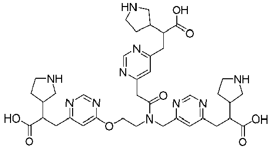 3-(6-(2-(2-(6-(2-carboxy-2-(pyrrolidin-3-yl)ethyl)pyrimidin-4-yl)-N-((6-(2-carboxy-2-(pyrrolidin-3-yl)ethyl)pyrimidin-4-yl)methyl)acetamido)ethoxy)pyrimidin-4-yl)-2-(pyrrolidin-3-yl)propanoic acid | 176-a | 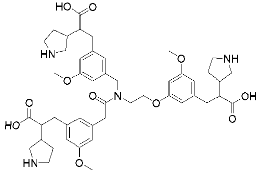 3-(3-(2-((3-(2-carboxy-2-(pyrrolidin-3-yl)ethyl)-5-methoxybenzyl)(2-(3-(2-carboxy-2-(pyrrolidin-3-yl)ethyl)-5-methoxyphenoxy)ethyl)amino)-2-oxoethyl)-5-methoxyphenyl)-2-(pyrrolidin-3-yl)propanoic acid |
| 177-a | 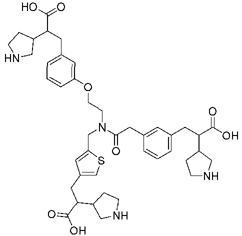 3-(3-(2-((2-(3-(2-carboxy-2-(pyrrolidin-3-yl)ethyl)phenoxy)ethyl)((4-(2-carboxy-2-(pyrrolidin-3-yl)ethyl)thiophen-2-yl)methyl)amino)-2-oxoethyl)phenyl)-2-(pyrrolidin-3-yl)propanoic acid | 178-a | 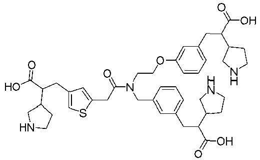 3-(5-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-oxoethyl)thiophen-3-yl)-2-(pyrrolidin-3-yl)propanoic acid |
| 179-a | 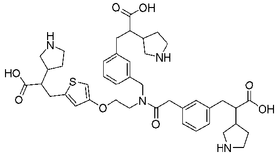 3-(3-(2-((3-(2-carboxy-2-(pyrrolidin-3-yl)ethyl)benzyl)(2-((5-(2-carboxy-2-(pyrrolidin-3-yl)ethyl)thiophen-3-yl)oxy)ethyl)amino)-2-oxoethyl)phenyl)-2-(pyrrolidin-3-yl)propanoic acid | 180-a | 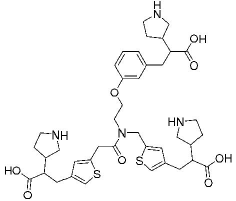 3-(5-(2-((2-(3-(2-carboxy-2-(pyrrolidin-3-yl)ethyl)phenoxy)ethyl)((4-(2-carboxy-2-(pyrrolidin-3-yl)ethyl)thiophen-2-yl)methyl)amino)-2-oxoethyl)thiophen-3-yl)-2-(pyrrolidin-3-yl)propanoic acid |
| 181-a | 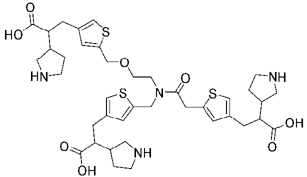 3-(5-((2-(2-(4-(2-carboxy-2-(pyrrolidin-3-yl)ethyl)thiophen-2-yl)-N-((4-(2-carboxy-2-(pyrrolidin-3-yl)ethyl)thiophen-2-yl)methyl)acetamido)ethoxy)methyl)thioph en-3-yl)-2-(pyrrolidin-3-yl)propanoic acid | 182-a | 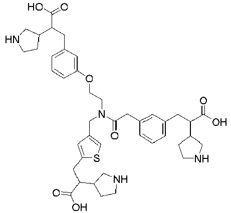 3-(3-(2-((2-(3-(2-carboxy-2-(pyrrolidin-3-yl)ethyl)phenoxy)ethyl)((5-(2-carboxy-2-(pyrrolidin-3-yl)ethyl)thiophen-3-yl)methyl)amino)-2-oxoethyl)phenyl)-2-(pyrrolidin-3-yl)propanoic acid |
| 183-a | 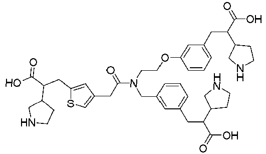 3-(4-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-oxoethyl)thiophen-2-yl)-2-(pyrrolidin-3-yl)propanoic acid | 184-a | 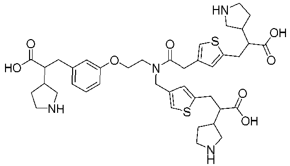 3-(4-(2-((2-(3-(2-carboxy-2-(pyrrolidin-3-yl)ethyl)phenoxy)ethyl)((5-(2-carboxy-2-(pyrrolidin-3-yl)ethyl)thiophen-3-yl)methyl)amino)-2-oxoethyl)thiophen-2-yl)-2-(pyrrolidin-3-yl)propanoic acid |
| 185-a | 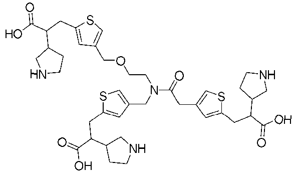 3-(4-((2-(2-(5-(2-carboxy-2-(pyrrolidin-3-yl)ethyl)thiophen-3-yl)-N-((5-(2-carboxy-2-(pyrrolidin-3-yl)ethyl)thiophen-3-yl)methyl)acetamido)ethoxy)methyl)thioph en-2-yl)-2-(pyrrolidin-3-yl)propanoic acid | 186-a | 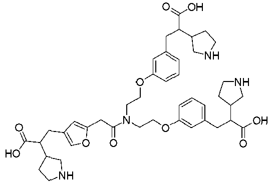 3,3'-(((((2-(4-(2-carboxy-2-(pyrrolidin-3-yl)ethyl)furan-2-yl)acetyl)azanediyl)bis(ethane-2,1-diyl))bis(oxy))bis(3,1-phenylene))bis(2-(pyrrolidin-3-yl)propanoic acid) |
| 187-a | 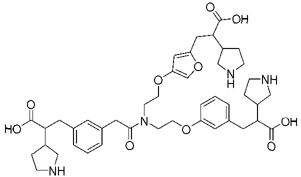 3-(3-(2-((2-((5-(2-carboxy-2-(pyrrolidin-3-yl)ethyl)furan-3-yl)oxy)ethyl)(2-(3-(2-carboxy-2-(pyrrolidin-3-yl)ethyl)phenoxy)ethyl)amino)-2-oxoethyl)phenyl)-2-(pyrrolidin-3-yl)propanoic acid | 188-a | 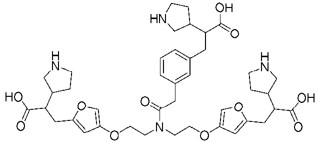 3,3'-(((((2-(3-(2-carboxy-2-(pyrrolidin-3-yl)ethyl)phenyl)acetyl)azanediyl)bis(ethane-2,1-diyl))bis(oxy))bis(furan-4,2-diyl))bis(2-(pyrrolidin-3-yl)propanoic acid) |
| 189-a | 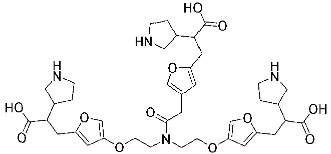 3,3'-(((((2-(5-(2-carboxy-2-(pyrrolidin-3-yl)ethyl)furan-3-yl)acetyl)azanediyl)bis(ethane-2,1-diyl))bis(oxy))bis(furan-4,2-diyl))bis(2-(pyrrolidin-3-yl)propanoic acid) | 190-a | 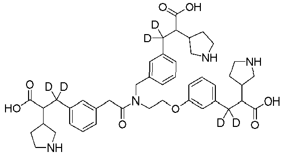 3-(3-(2-((3-(2-carboxy-2-(pyrrolidin-3-yl)ethyl-1,1-d2)benzyl)(2-(3-(2-carboxy-2-(pyrrolidin-3-yl)ethyl-1,1-d2)phenoxy)ethyl)amino)-2-oxoethyl)phenyl)-2-(pyrrolidin-3-yl)propanoic-3,3-d2 acid |
| 191-a | 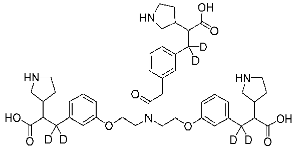 3,3'-(((((2-(3-(2-carboxy-2-(pyrrolidin-3-yl)ethyl-1,1-d2)phenyl)acetyl)azanediyl)bis(ethane-2,1-diyl))bis(oxy))bis(3,1-phenylene))bis(2-(pyrrolidin-3-yl)propanoic-3,3-d2 acid) | 192-a | 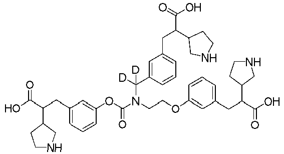 3-(3-(2-(((3-(2-carboxy-2-(pyrrolidin-3-yl)ethyl)phenoxy)carbonyl)((3-(2-carboxy-2-(pyrrolidin-3-yl)ethyl)phenyl)methyl-d2)amino)ethoxy)phenyl)-2-(pyrrolidin-3-yl)propanoic acid |
| 193-a | 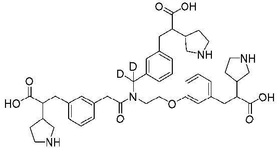 3-(3-(2-((2-(3-(2-carboxy-2-(pyrrolidin-3-yl)ethyl)phenoxy)ethyl)((3-(2-carboxy-2-(pyrrolidin-3-yl)ethyl)phenyl)methyl-d2)amino)-2-oxoethyl)phenyl)-2-(pyrrolidin-3-yl)propanoic acid | 194-a | 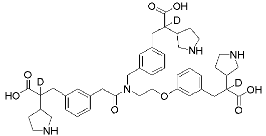 3-(3-(2-((3-(2-carboxy-2-(pyrrolidin-3-yl)ethyl-2-d)benzyl)(2-(3-(2-carboxy-2-(pyrrolidin-3-yl)ethyl-2-d)phenoxy)ethyl)amino)-2-oxoethyl)phenyl)-2-(pyrrolidin-3-yl)propanoic-2-d acid |
| 195-a | 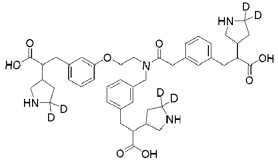 3-(3-(2-((3-(2-carboxy-2-(pyrrolidin-3-yl-5,5-d2)ethyl)benzyl)(2-(3-(2-carboxy-2-(pyrrolidin-3-yl-5,5-d2)ethyl)phenoxy)ethyl)amino)-2-oxoethyl)phenyl)-2-(pyrrolidin-3-yl-5,5-d2)propanoic acid | 196-a | 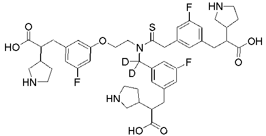 3-(3-(2-((2-(3-(2-carboxy-2-(pyrrolidin-3-ylethyl)-5-fluorophenoxy)ethyl)((3-(2-carboxy-2-(pyrrolidin-3-yl)ethyl)-5-fluorophenyl)methyl-d2)amino)-2-thioxoethyl)-5-fluorophenyl)-2-(pyrrolidin-3-yl)propanoic acid |
| 197-a | 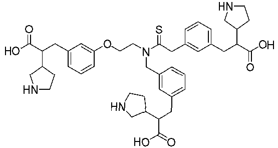 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-thioxoethyl)phenyl)-2-(pyrrolidin-3-yl)propanoic acid | 198-a | 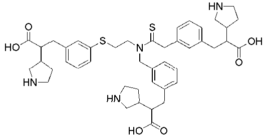 3-(3-(2-((3-(2-carboxy-2-(pyrrolidin-3-yl)ethyl)benzyl)(2-((3-(2-carboxy-2-(pyrrolidin-3-yl)ethyl)phenyl)thio)ethyl)amino)-2-thioxoethyl)phenyl)-2-(pyrrolidin-3-yl)propanoic acid |
| 199-a | 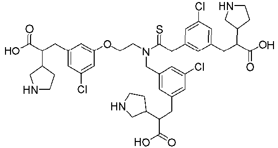 3-(3-(2-((3-(2-carboxy-2-(pyrrolidin-3-yl)ethyl)-5-chlorobenzyl)(2-(3-(2-carboxy-2-(pyrrolidin-3-yl)ethyl)-5-chlorophenoxy)ethyl)amino)-2-thioxoethyl)-5-chlorophenyl)-2-(pyrrolidin-3-yl)propanoic acid | 200-a | 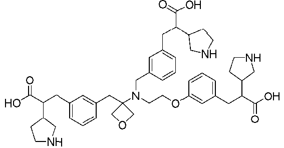 3-(3-((3-((3-(2-carboxy-2-(pyrrolidin-3-yl)ethyl)benzyl)(2-(3-(2-carboxy-2-(pyrrolidin-3-yl)ethyl)phenoxy)ethyl)amino)oxetan-3-yl)methyl)phenyl)-2-(pyrrolidin-3-yl)propanoic acid |
| 201-a | 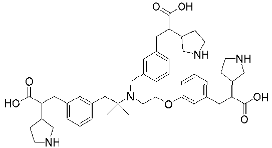 3-(3-(2-((3-(2-carboxy-2-(pyrrolidin-3-yl)ethyl)benzyl)(1-(3-(2-carboxy-2-(pyrrolidin-3-yl)ethyl)phenyl)-2-methylpropan-2-yl)amino)ethoxy)phenyl)-2-(pyrrolidin-3-yl)propanoic acid | 202-a | 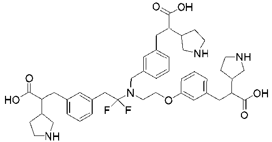 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2,2-difluoroethyl)phenyl)-2-(pyrrolidin-3-yl)propanoic acid |
| 203-a | 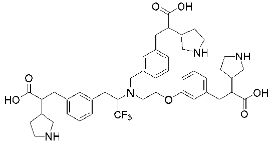 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-3,3,3-trifluoropropyl)phenyl)-2-(pyrrolidin-3-yl)propanoic acid | 204-a | 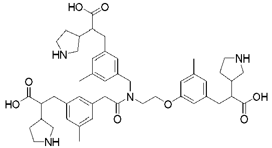 3-(3-(2-((3-(2-carboxy-2-(pyrrolidin-3-yl)ethyl)-5-methylbenzyl)(2-(3-(2-carboxy-2-(pyrrolidin-3-yl)ethyl)-5-methylphenoxy)ethyl)amino)-2-oxoethyl)-5-methylphenyl)-2-(pyrrolidin-3-yl)propanoic acid |
| 205-a | 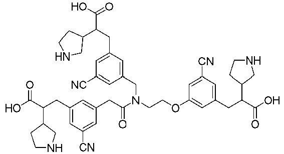 3-(3-(2-((3-(2-carboxy-2-(pyrrolidin-3-yl)ethyl)-5-cyanobenzyl)(2-(3-(2-carboxy-2-(pyrrolidin-3-yl)ethyl)-5-cyanophenoxy)ethyl)amino)-2-oxoethyl)-5-cyanophenyl)-2-(pyrrolidin-3-yl)propanoic acid | 206-a | 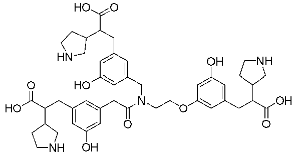 3-(3-(2-((3-(2-carboxy-2-(pyrrolidin-3-yl)ethyl)-5-hydroxybenzyl)(2-(3-(2-carboxy-2-(pyrrolidin-3-yl)ethyl)-5-hydroxyphenoxy)ethyl)amino)-2-oxoethyl)-5-hydroxyphenyl)-2-(pyrrolidin-3-yl)propanoic acid |
| 207-a | 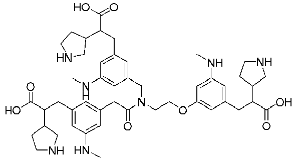 3-(3-(2-((3-(2-carboxy-2-(pyrrolidin-3-yl)ethyl)-5-(methylamino)benzyl)(2-(3-(2-carboxy-2-(pyrrolidin-3-yl)ethyl)-5-(methylamino)phenoxy)ethyl)amino)-2-oxoethyl)-5-(methylamino)phenyl)-2-(pyrrolidin-3-yl)propanoic acid | 208-a | 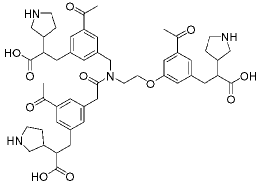 3-(3-acetyl-5-((N-(2-(3-acetyl-5-(2-carboxy-2-(pyrrolidin-3-yl)ethyl)phenoxy)ethyl)-2-(3-acetyl-5-(2-carboxy-2-(pyrrolidin-3-yl)ethyl)phenyl)acetamido)methyl)phenyl)-2-(pyrrolidin-3-yl)propanoic acid |
| 212-a | 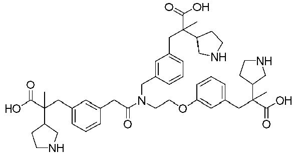 3-(3-(2-((3-(2-carboxy-2-(pyrrolidin-3-yl)propyl)benzyl)(2-(3-(2-carboxy-2-(pyrrolidin-3-yl)propyl)phenoxy)ethyl)amino)-2-oxoethyl)phenyl)-2-methyl-2-(pyrrolidin-3-yl)propanoic acid | 213-a | 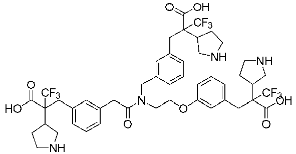 2-(3-(2-((3-(2-carboxy-3,3,3-trifluoro-2-(pyrrolidin-3-yl)propyl)benzyl)(2-(3-(2-carboxy-3,3,3-trifluoro-2-(pyrrolidin-3-yl)propyl)phenoxy)ethyl)amino)-2-oxoethyl)benzyl)-3,3,3-trifluoro-2-(pyrrolidin-3-yl)propanoic acid |
| 214-a | 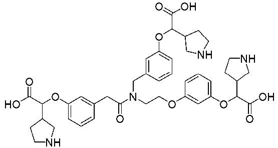 2-(3-(2-((3-(carboxy(pyrrolidin-3-yl)methoxy)benzyl)(2-(3-(carboxy(pyrrolidin-3-yl)methoxy)phenoxy)ethyl)amino)-2-oxoethyl)phenoxy)-2-(pyrrolidin-3-yl)acetic acid | 215-a | 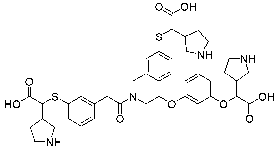 2-((3-(2-((2-(3-(carboxy(pyrrolidin-3-yl)methoxy)phenoxy)ethyl)(3-((carboxy(pyrrolidin-3-yl)methyl)thio)benzyl)amino)-2-oxoethyl)phenyl)thio)-2-(pyrrolidin-3-yl)acetic acid |
| 216-a | 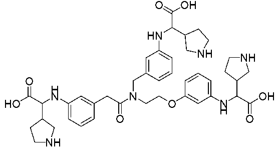 2-((3-(2-((3-((carboxy(pyrrolidin-3-yl)methyl)amino)benzyl)(2-(3-((carboxy(pyrrolidin-3-yl)methyl)amino)phenoxy)ethyl)amino)-2-oxoethyl)phenyl)amino)-2-(pyrrolidin-3-yl)acetic acid | 217-a | 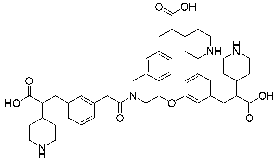 3-(3-(2-((3-(2-carboxy-2-(piperidin-4-yl)ethyl)benzyl)(2-(3-(2-carboxy-2-(piperidin-4-yl)ethyl)phenoxy)ethyl)amino)-2-oxoethyl)phenyl)-2-(piperidin-4-yl)propanoic acid |
| 218-a | 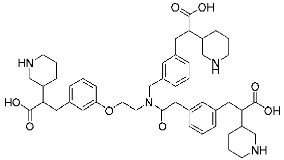 3-(3-(2-((3-(2-carboxy-2-(piperidin-3-yl)ethyl)benzyl)(2-(3-(2-carboxy-2-(piperidin-3-yl)ethyl)phenoxy)ethyl)amino)-2-oxoethyl)phenyl)-2-(piperidin-3-yl)propanoic acid | 219-a | 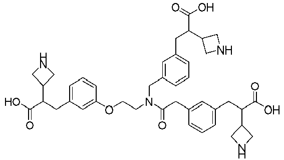 2-(azetidin-3-yl)-3-(3-(2-((3-(2-(azetidin-3-yl)-2-carboxyethyl)benzyl)(2-(3-(2-(azetidin-3-yl)-2-carboxyethyl)phenoxy)ethyl)amino)-2-oxoethyl)phenyl)propanoic acid |
| 220-a | 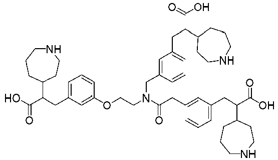 2-(azepan-4-yl)-3-(3-(2-((3-(2-(azepan-4-yl)-2-carboxyethyl)benzyl)(2-(3-(2-(azepan-4-yl)-2-carboxyethyl)phenoxy)ethyl)amino)-2-oxoethyl)phenyl)propanoic acid | 221-a | 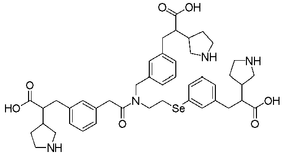 3-(3-(2-((3-(2-carboxy-2-(pyrrolidin-3-yl)ethyl)benzyl)(2-((3-(2-carboxy-2-(pyrrolidin-3-yl)ethyl)phenyl)selanyl)ethyl)amino)-2-oxoethyl)phenyl)-2-(pyrrolidin-3-yl)propanoic acid |
| 222-a | 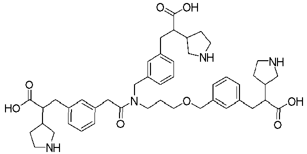 3-(3-(2-((3-(2-carboxy-2-(pyrrolidin-3-yl)ethyl)benzyl)(3-((3-(2-carboxy-2-(pyrrolidin-3-yl)ethyl)benzyl)oxy)propyl)amino)-2-oxoethyl)phenyl)-2-(pyrrolidin-3-yl)propanoic acid | 223-a | 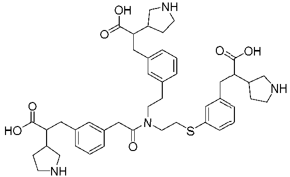 3-(3-(2-((3-(2-carboxy-2-(pyrrolidin-3-yl)ethyl)phenethyl)(2-((3-(2-carboxy-2-(pyrrolidin-3-yl)ethyl)phenyl)thio)ethyl)amino)-2-oxoethyl)phenyl)-2-(pyrrolidin-3-yl)propanoic acid |
| 224-a | 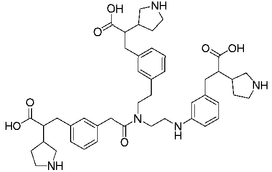 3-(3-(2-((3-(2-carboxy-2-(pyrrolidin-3-yl)ethyl)phenethyl)(2-((3-(2-carboxy-2-(pyrrolidin-3-yl)ethyl)phenyl)amino)ethyl)amino)-2-oxoethyl)phenyl)-2-(pyrrolidin-3-yl)propanoic acid | 225-a | 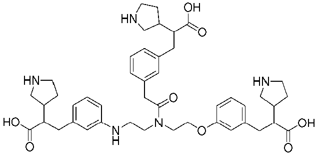 3-(3-(2-((2-(3-(2-carboxy-2-(pyrrolidin-3-yl)ethyl)phenoxy)ethyl)(2-((3-(2-carboxy-2-(pyrrolidin-3-yl)ethyl)phenyl)amino)ethyl)amino)-2-oxoethyl)phenyl)-2-(pyrrolidin-3-yl)propanoic acid |
| 226-a | 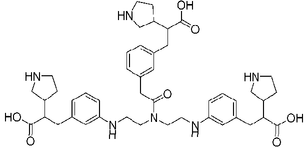 3,3'-(((((2-(3-(2-carboxy-2-(pyrrolidin-3-yl)ethyl)phenyl)acetyl)azanediyl)bis(ethan e-2,1-diyl))bis(azanediyl))bis(3,1-phenylene))bis(2-(pyrrolidin-3-yl)propanoic acid) | 227-a | 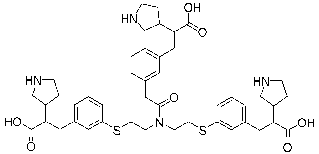 3,3'-(((((2-(3-(2-carboxy-2-(pyrrolidin-3-yl)ethyl)phenyl)acetyl)azanediyl)bis(ethane-2,1-diyl))bis(sulfanediyl))bis(3,1-phenylene))bis(2-(pyrrolidin-3-yl)propanoic acid) |
| 228-a | 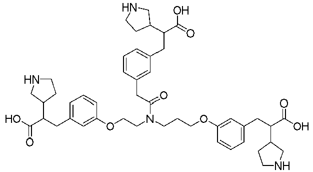 3-(3-(2-((2-(3-(2-carboxy-2-(pyrrolidin-3-yl)ethyl)phenoxy)ethyl)(3-(3-(2-carboxy-2-(pyrrolidin-3-yl)ethyl)phenoxy)propyl)amino)-2-oxoethyl)phenyl)-2-(pyrrolidin-3-yl)propanoic acid | 96-b | 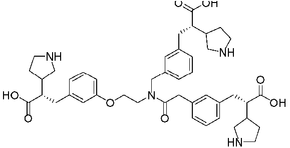 (S)-3-(3-(2-((3-((S)-2-carboxy-2-(pyrrolidin-3-yl)ethyl)benzyl)(2-(3-((S)-2-carboxy-2-(pyrrolidin-3-yl)ethyl)phenoxy)ethyl)amino)-2-oxoethyl)phenyl)-2-(pyrrolidin-3-yl)propanoic acid |

In one embodiment of the invention, the compounds of the invention or a pharmaceutically acceptable salt thereof are selected from:

| Comp. No. | Structure | Comp. No. | Structure |
|---|---|---|---|
| 1 | 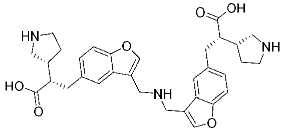 | 2 | 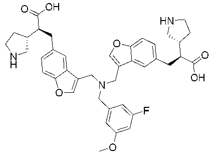 |
| 3 | 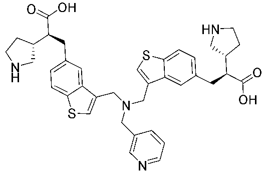 | 4 | 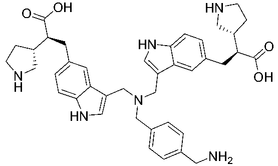 |
| 5 | 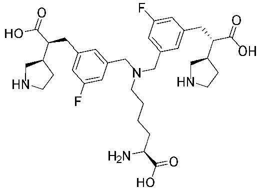 | 6 | 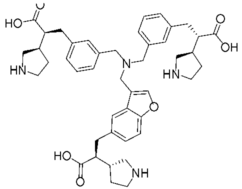 |
| 7 | 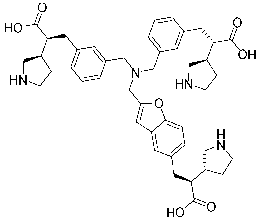 | 8 | 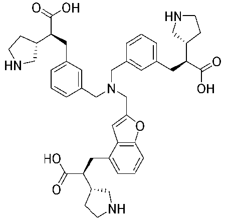 |
| 9 | 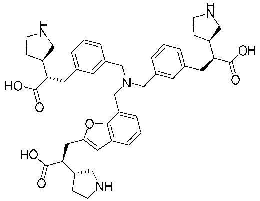 | 10 | 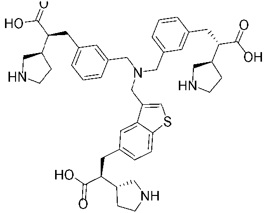 |
| 11 | 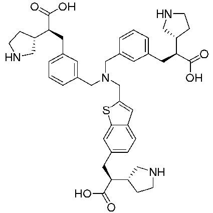 | 12 | 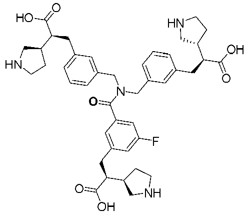 |
| 13 | 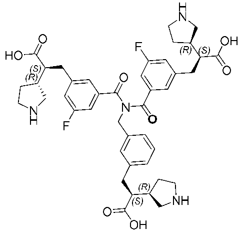 | 14 | 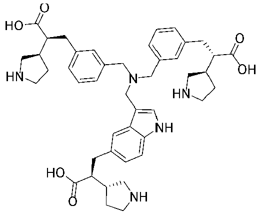 |
| 15 | 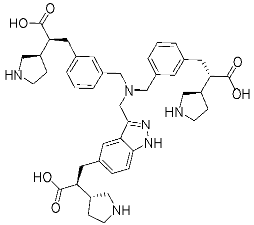 | 16 | 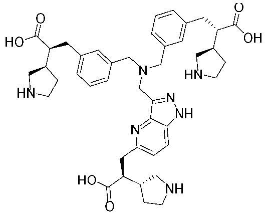 |
| 17 | 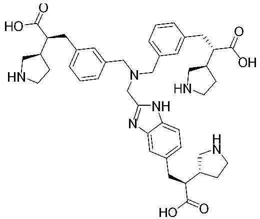 | 18 | 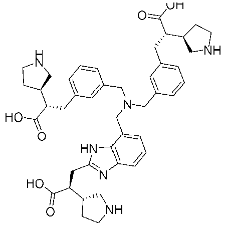 |
| 19 | 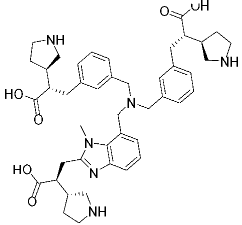 | 20 | 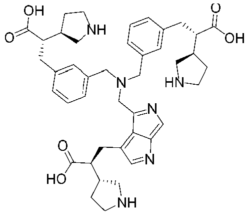 |
| 21 | 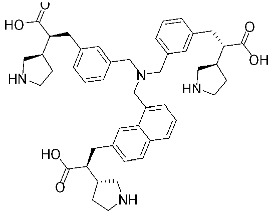 | 22 | 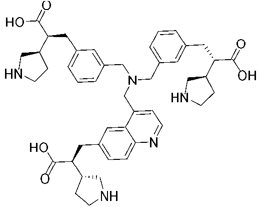 |
| 23 | 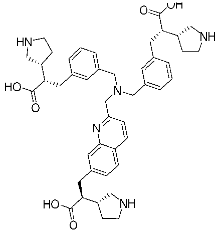 | 24 | 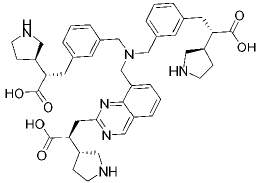 |
| 25 | 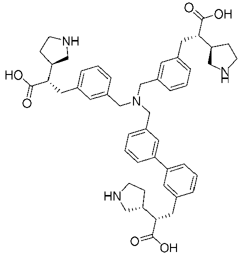 | 26 | 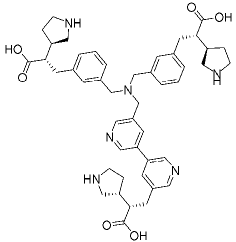 |
| 27 | 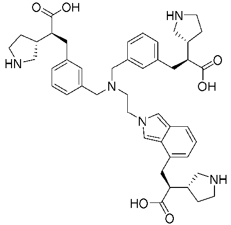 | 28 | 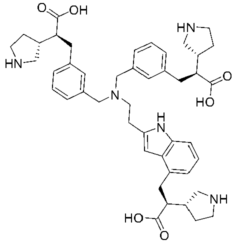 |
| 29 | 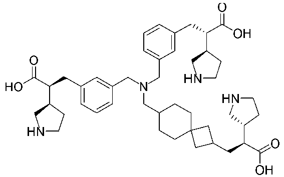 | 30 | 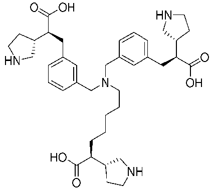 |
| 31 | 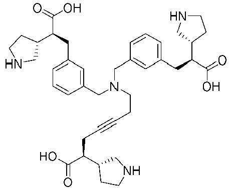 | 32 | 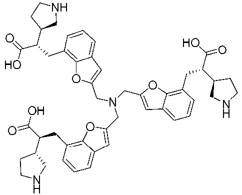 |
| 33 | 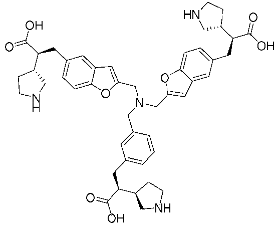 | 34 | 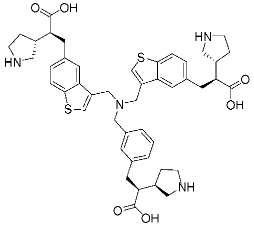 |
| 35 | 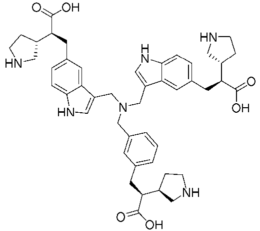 | 36 | 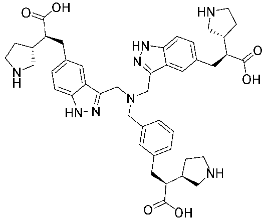 |
| 37 | 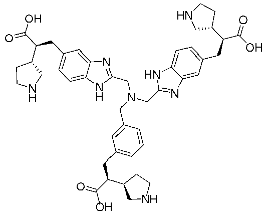 | 38 | 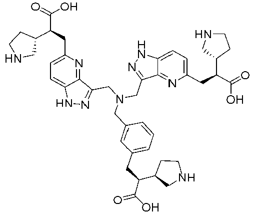 |
| 39 | 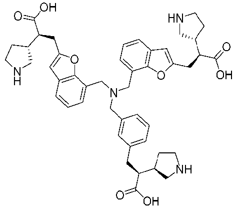 | 40 | 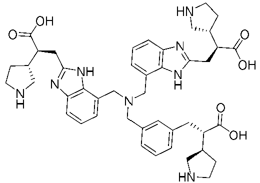 |
| 41 | 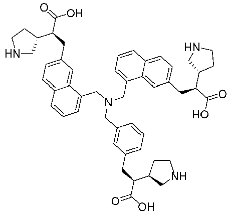 | 42 | 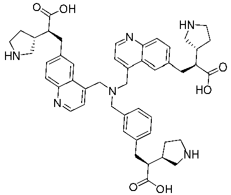 |
| 43 | 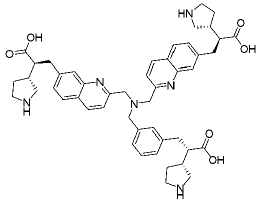 | 44 | 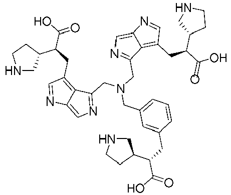 |
| 45 | 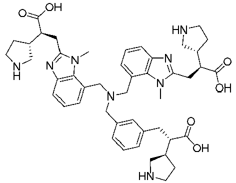 | 46 | 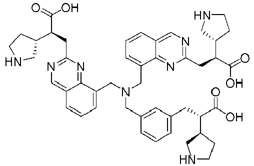 |
| 47 | 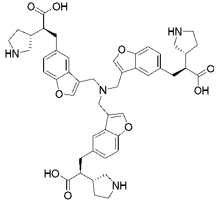 | 48 | 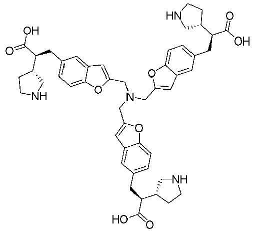 |
| 49 | 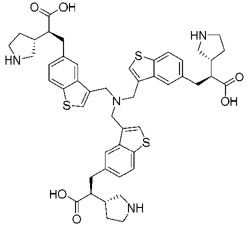 | 50 | 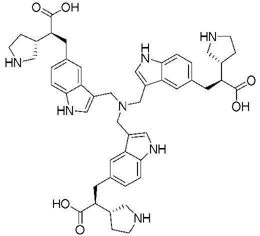 |
| 51 | 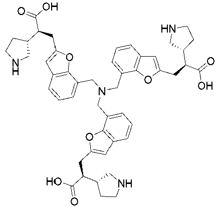 | 52 | 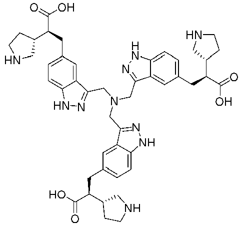 |
| 53 | 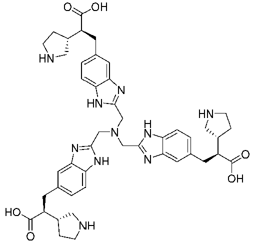 | 54 | 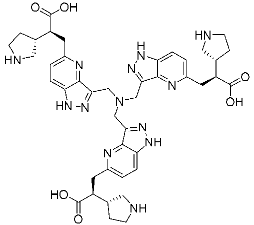 |
| 55 | 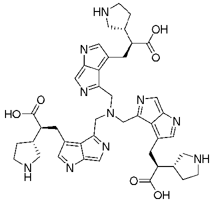 | 56 | 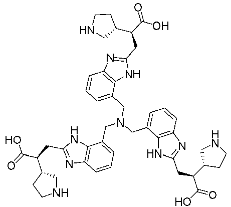 |
| 57 | 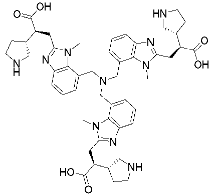 | 58 | 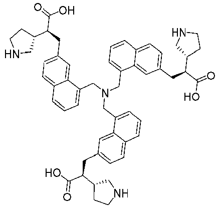 |
| 59 | 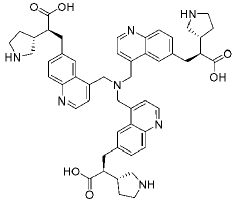 | 60 | 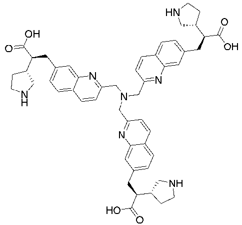 |
| 61 | 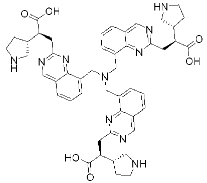 | 62 | 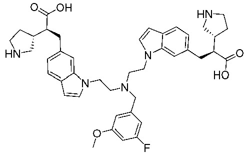 |
| 63 | 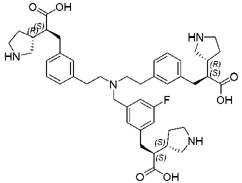 | 64 | 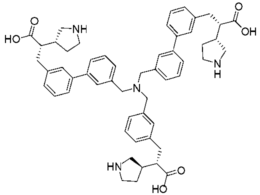 |
| 65 | 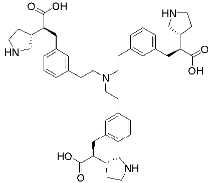 | 66 | 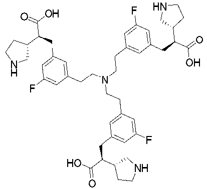 |
| 67 | 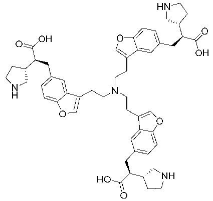 | 68 | 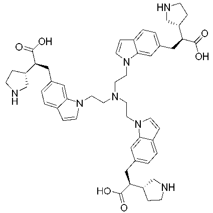 |
| 69 | 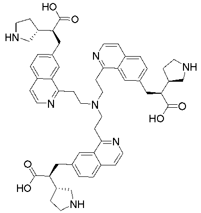 | 70 | 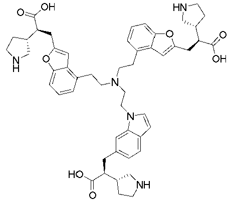 |
| 71 | 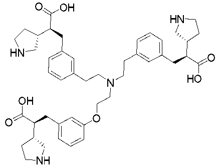 | 72 | 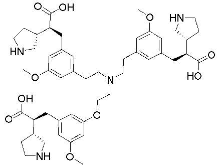 |
| 73 | 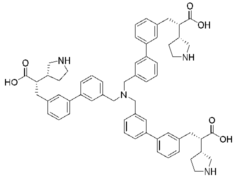 | 74 | 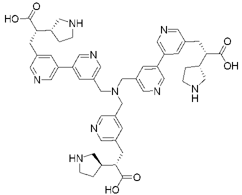 |
| 75 | 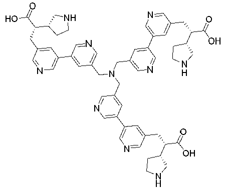 | 76 | 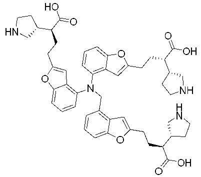 |
| 77 | 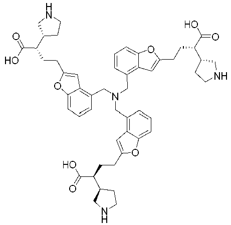 | 78 | 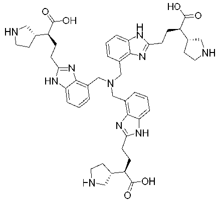 |
| 79 | 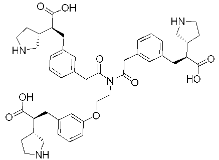 | 80 | 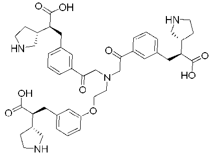 |
| 81 | 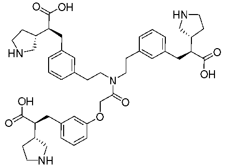 | 82 | 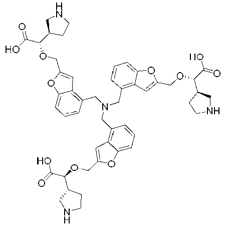 |
| 83 | 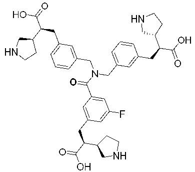 | 84 | 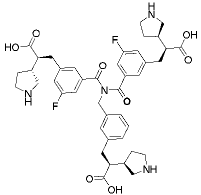 |
| 85 | 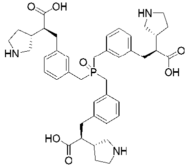 | 86 | 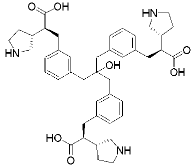 |
| 87 | 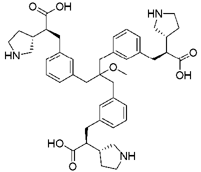 | 88 | 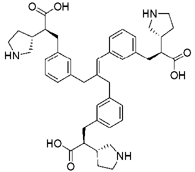 |
| 89 | 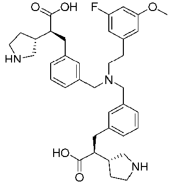 | 90 | 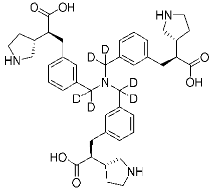 |
| 91 | 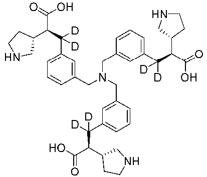 | 92 | 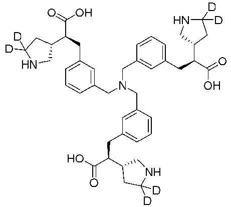 |
| 93 | 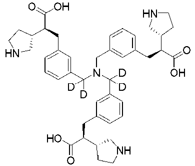 | 94 | 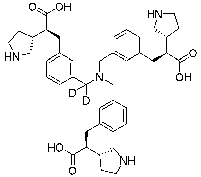 |
| 95 | 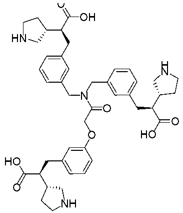 | 96 | 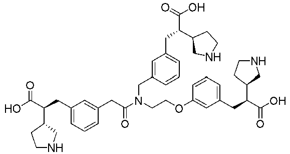 |
| 97 | 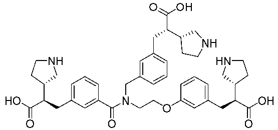 | 98 | 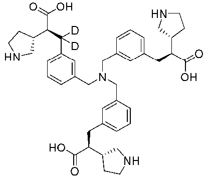 |
| 99 | 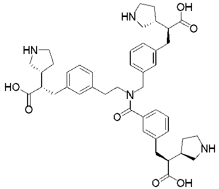 | 100 | 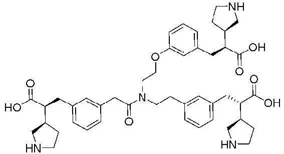 |
| 101 | 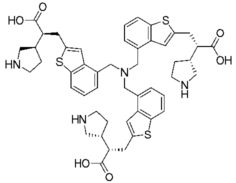 | 102 | 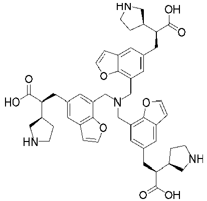 |
| 103 | 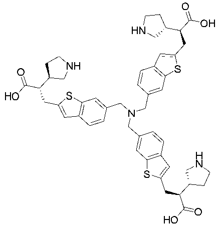 | 104 | 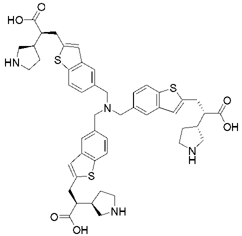 |
| 105 | 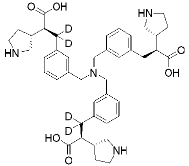 | 106 | 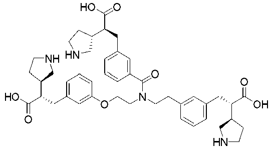 |
| 107 | 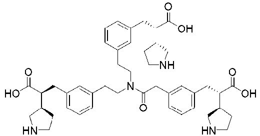 | 108 | 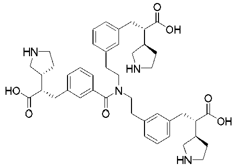 |
| 109 | 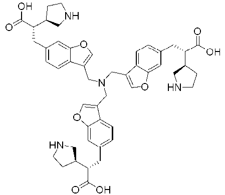 | 110 | 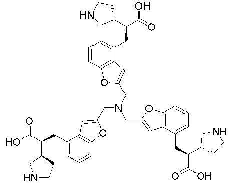 |
| 111 | 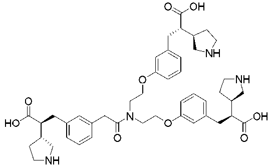 | 112 | 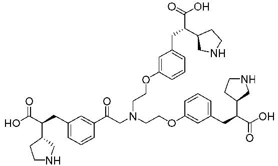 |
| 113 | 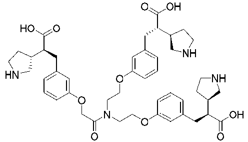 | 114 | 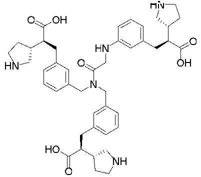 |
| 115 | 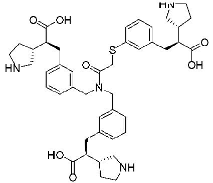 | 116 | 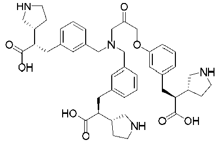 |
| 117 | 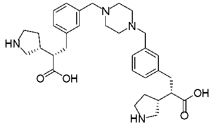 | 118 | 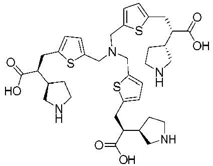 |
| 119 | 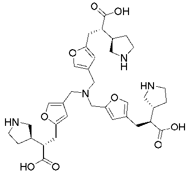 | 120 | 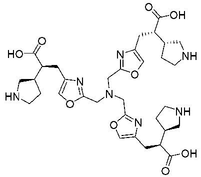 |
| 121 | 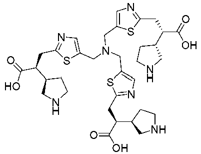 | 122 | 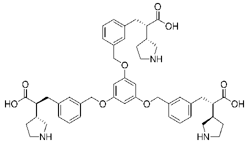 |
| 123 | 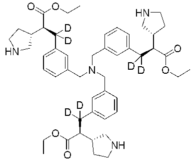 | 124 | 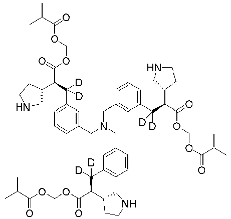 |
| 125 | 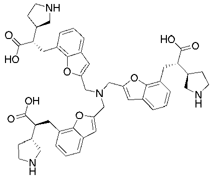 | 126 | 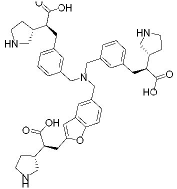 |
| 127 | 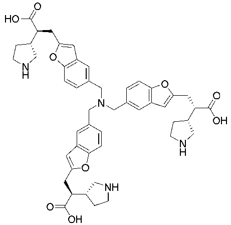 | 128 | 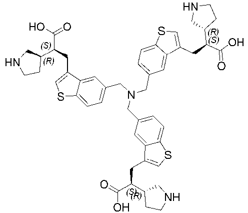 |
| 129 | 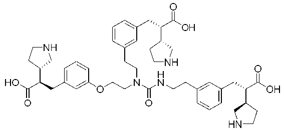 | 130 | 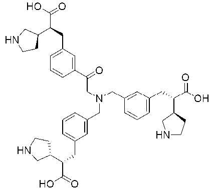 |
| 131 | 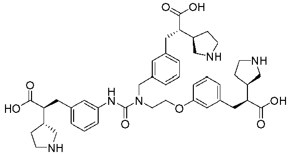 | 132 | 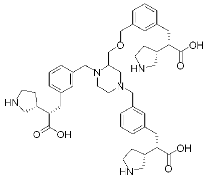 |
| 133 | 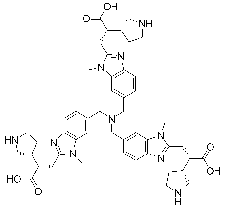 | 134 | 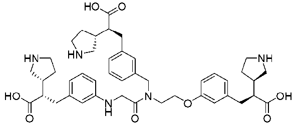 |
| 135 | 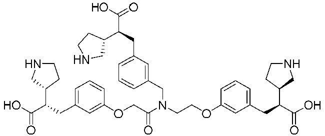 | 136 | 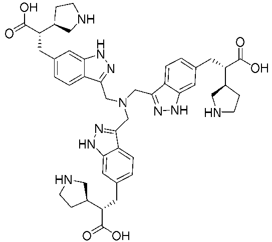 |
| 137 | 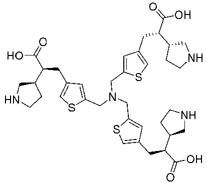 | 138 | 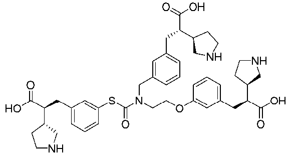 |
| 139 | 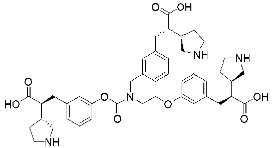 | 140 | 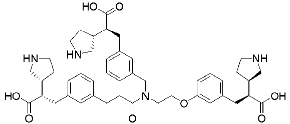 |
| 141 | 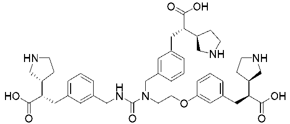 | 142 | 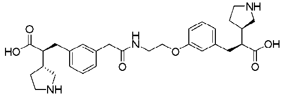 |
| 143 | 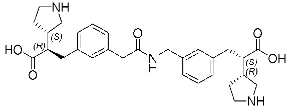 | 144 | 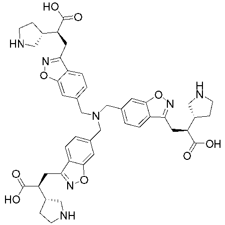 |
| 145 | 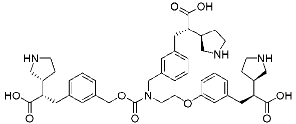 | 146 | 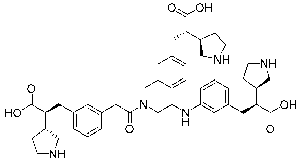 |
| 147 | 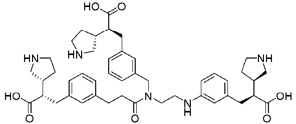 | 148 | 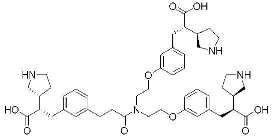 |
| 149 | 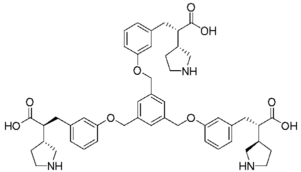 | 150 | 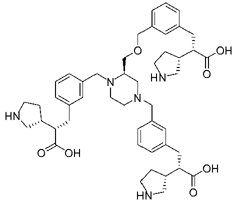 |
| 151 | 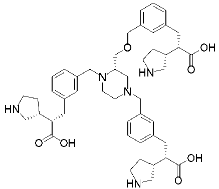 | 152 | 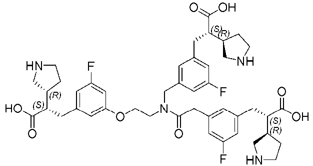 |
| 153 | 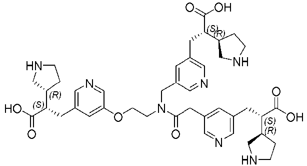 | 154 | 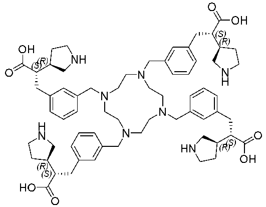 |
| 155 | 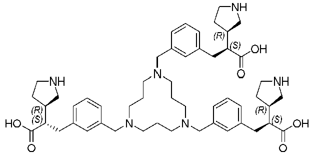 | 156 | 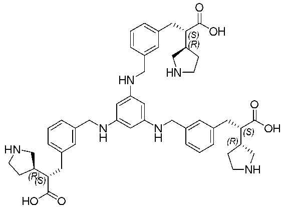 |
| 157 | 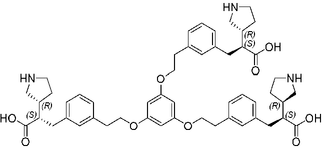 | 158 | 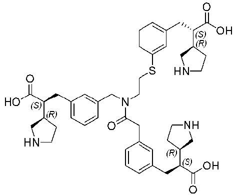 |
| 159 | 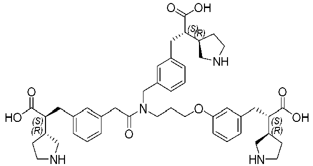 | 160 | 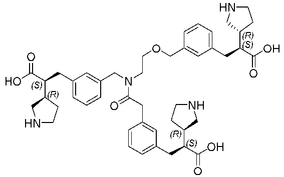 |
| 161 | 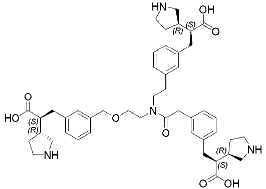 | 162 | 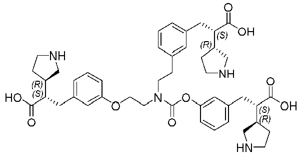 |
| 163 | 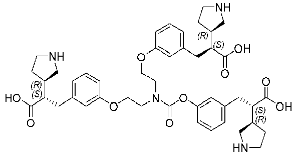 | 164 | 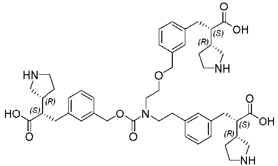 |
| 165 | 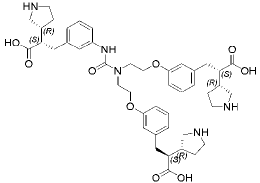 | 166 | 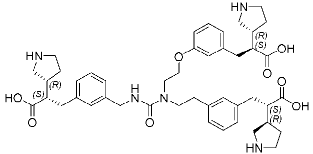 |
| 167 | 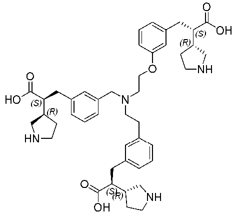 | 168 | 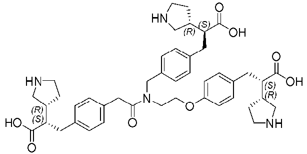 |
| 169 | 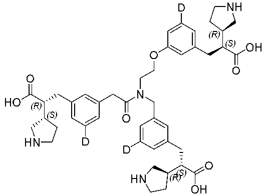 | 170 | 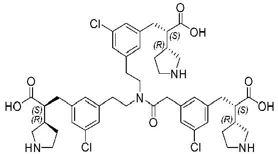 |
| 171 | 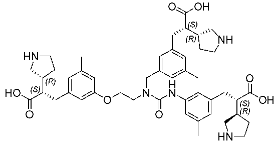 | 172 | 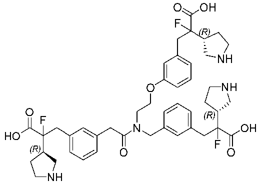 |
| 173 | 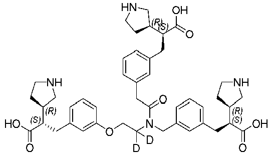 | 174 | 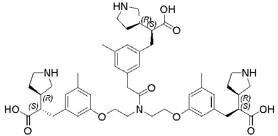 |
| 175 | 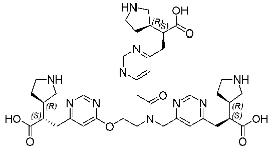 | 176 | 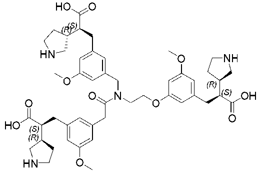 |
| 177 | 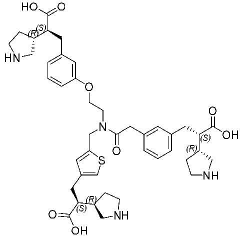 | 178 | 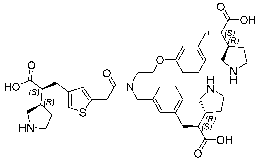 |
| 179 | 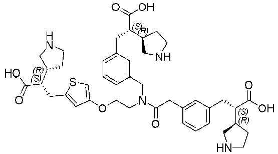 | 180 | 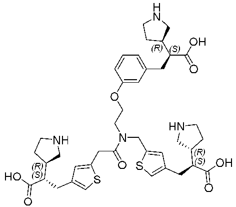 |
| 181 | 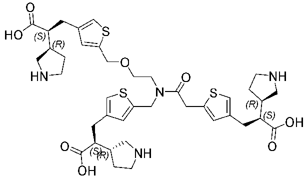 | 182 | 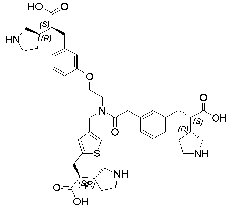 |
| 183 | 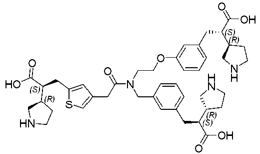 | 184 | 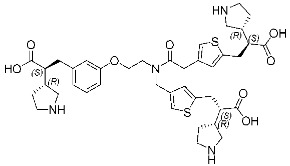 |
| 185 | 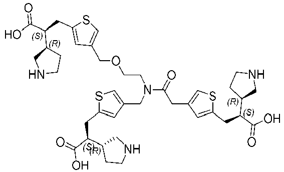 | 186 | 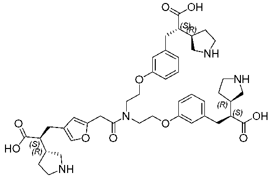 |
| 187 | 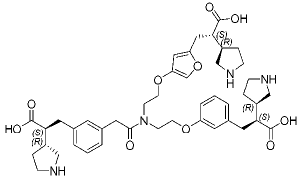 | 188 | 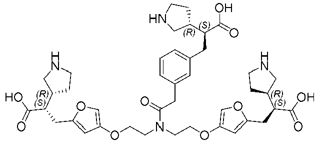 |
| 189 | 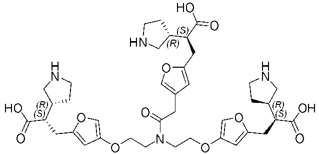 | 190 | 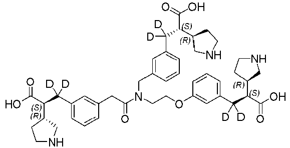 |
| 191 | 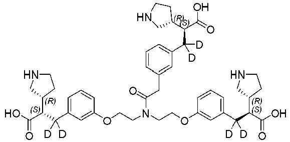 | 192 | 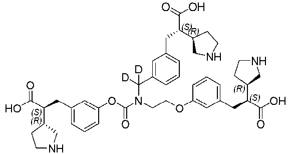 |
| 193 | 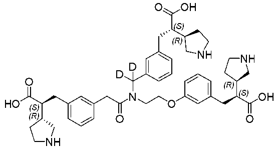 | 194 | 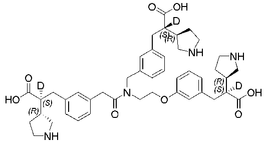 |
| 195 | 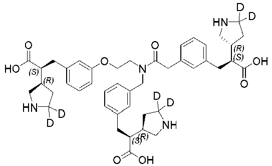 | 196 | 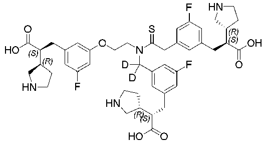 |
| 197 | 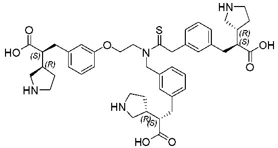 | 198 | 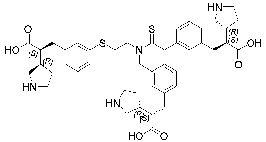 |
| 199 | 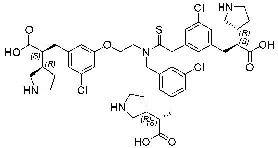 | 200 | 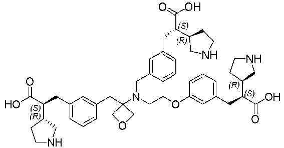 |
| 201 | 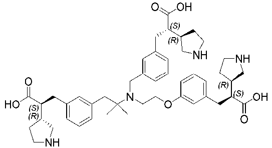 | 202 | 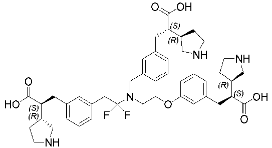 |
| 203 | 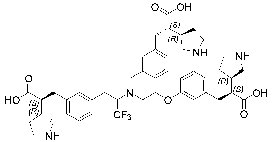 | 204 | 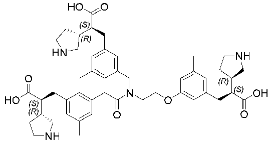 |
| 205 | 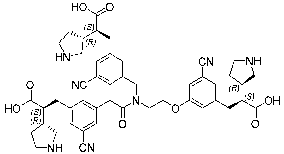 | 206 | 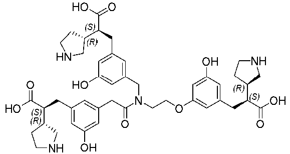 |
| 207 | 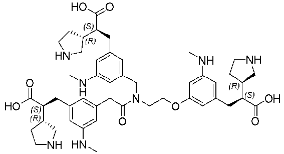 | 208 | 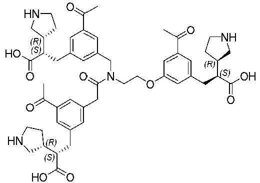 |
| 209 | 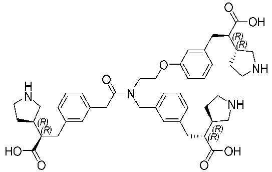 | 210 | 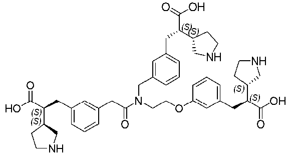 |
| 211 | 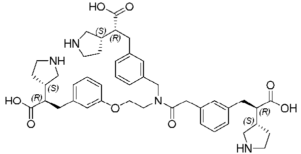 | 212 | 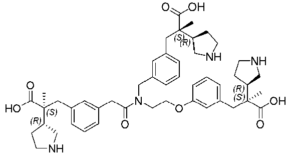 |
| 213 | 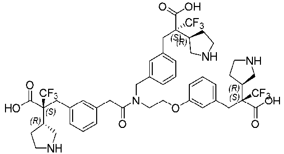 | 214 | 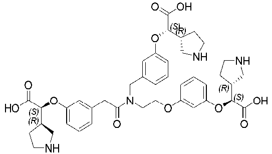 |
| 215 | 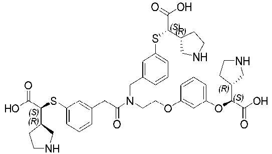 | 216 | 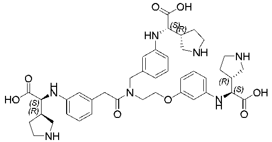 |
| 217 | 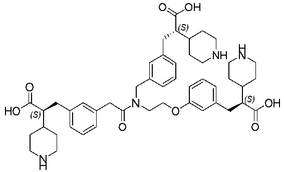 | 218 | 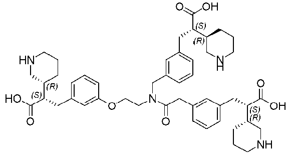 |
| 219 | 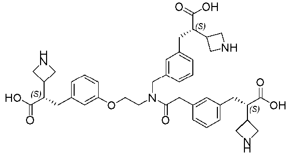 | 220 | 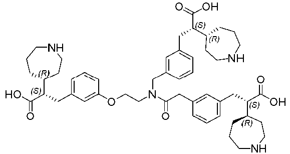 |
| 221 | 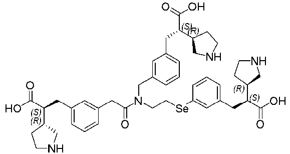 | 222 | 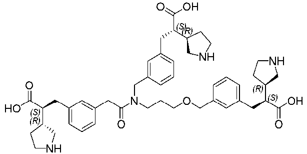 |
| 223 | 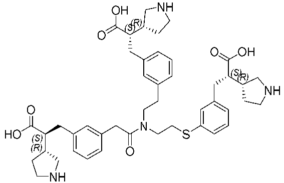 | 224 | 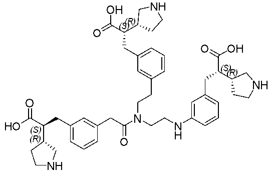 |
| 225 | 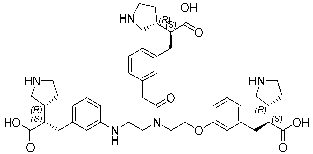 | 226 | 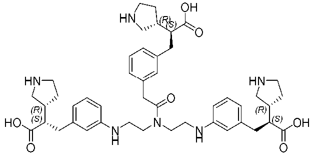 |
| 227 | 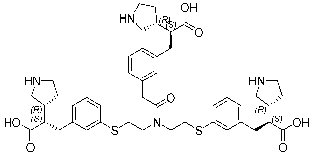 | 228 | 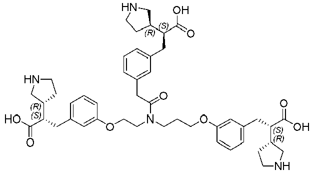 |

In one embodiment of the invention, the compound is 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-oxoethyl)phenyl)-2-(pyrrolidin-3-yl)propanoic acid, or a pharmaceutically acceptable salt thereof:

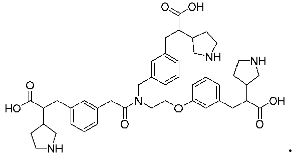

In one embodiment of the invention, the compound is 3-(3-(2-((3-(2-carboxy-2-(pyrrolidin-3-yl)ethyl)benzyl)(2-(3-(2-carboxy-2-(pyrrolidin-3-yl)ethyl)phenoxy)ethyl)amino)-2-oxoethyl)phenyl)-2-(pyrrolidin-3-yl)propanoic acid, or a pharmaceutically acceptable salt thereof, in a stereoisomeric form:

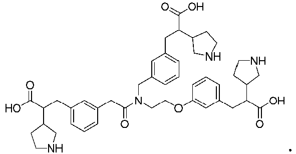

In one embodiment of the invention, the compounds is (S)-3-(3-(2-((3-((S)-2-carboxy-2-(pyrrolidin-3-yl)ethyl)benzyl)(2-(3-((S)-2-carboxy-2-(pyrrolidin-3-yl)ethyl)phenoxy)ethyl)amino)-2-oxoethyl)phenyl)-2-(pyrrolidin-3-yl)propanoic acid, or a pharmaceutically acceptable salt thereof:

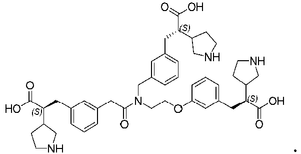

In one embodiment of the invention, the compounds is (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid, or a pharmaceutically acceptable salt thereof:

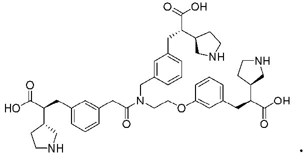

In one embodiment of the invention, the compounds is (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid, or a deuterated derivative form thereof, or a pharmaceutically acceptable salt thereof:

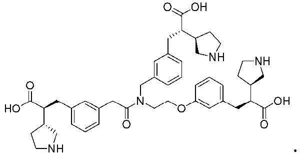

In one embodiment of the invention, the compounds is (2S,2'S)-3,3'-(((((2-(3-((S)-2-carboxy-2-((R)-pyrrolidin-3-yl)ethyl)phenyl)acetyl)azanediyl)bis(ethane-2,1-diyl))bis(oxy))bis(3,1-phenylene))bis(2-((R)-pyrrolidin-3-yl)propanoic acid), or a deuterated derivative form thereof, or a pharmaceutically acceptable salt thereof:

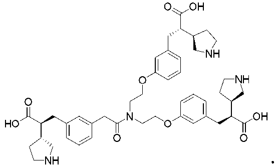

In one embodiment of the invention, the compounds is (S)-3-(3-(2-((3-((S)-2-carboxy-2-((R)-pyrrolidin-3-yl)ethyl)benzyl)((3-((S)-2-carboxy-2-((R)-pyrrolidin-3-yl)ethyl)phenoxy)carbonyl)amino)ethoxy)phenyl)-2-((R)-pyrrolidin-3-yl)propanoic acid, or a deuterated derivative form thereof, or a pharmaceutically acceptable salt thereof:

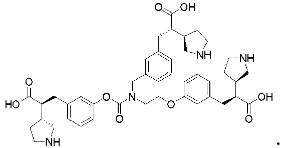

In one embodiment of the invention, the compounds of the invention or a pharmaceutically acceptable salt thereof are selected from: 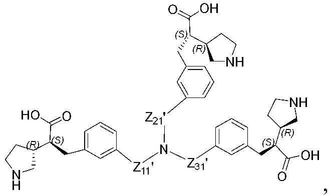 wherein * represents the connecting end with N;

| Compound No. | Z₃₁' | Z₁₁' | Z₂₁' |
|---|---|---|---|
| 229 | C(O) | CH₂ | CH₂CH₂CH₂ |
| 230 | C(O) | CH₂ | *CH₂CH₂S |
| 231 | C(O) | CH₂ | *CH₂CH₂NH |
| 232 | C(O) | CH₂ | *CH₂CH₂CH₂O |
| 233 | C(O) | CH₂ | *CH₂CH₂C(O) |
| 234 | C(O) | CH₂ | *CH₂CH₂C(S) |
| 235 | C(O) | CH₂ | *CH₂CH₂C(O)O |
| 236 | C(O) | CH₂ | *CH₂CH₂C(O)NH |
| 237 | C(O) | CH₂ | *CH₂CH₂C(O)NCH₂ |
| 238 | C(O) | CH₂ | *CH₂CH₂NHC(O) |
| 239 | C(O) | CH₂CH₂ | CH₂CH₂CH₂ |
| 240 | C(O) | CH₂CH₂ | *CH₂CH₂S |
| 241 | C(O) | CH₂CH₂ | *CH₂CH₂CH₂O |
| 242 | C(O) | *CH₂CH₂O | *CH₂CH₂O |
| 243 | C(O) | *CH₂CH₂O | *CH₂CH₂S |
| 244 | C(O) | *CH₂CH₂O | *CH₂CH₂NH |
| 245 | C(O) | *CH₂CH₂O | *CH₂CH₂C(O) |
| 246 | C(O) | *CH₂CH₂O | *CH₂CH₂C(O)NH |
| 247 | *C(O)CH₂ | CH₂ | CH₂ |
| 248 | *C(O)CH₂ | CH₂ | CH₂CH₂ |
| 249 | *C(O)CH₂ | CH₂CH₂ | CH₂CH₂CH₂ |
| 250 | *C(O)CH₂ | *CH₂CH₂O | CH₂CH₂CH₂ |
| 251 | *C(O)CH₂ | *CH₂CH₂O | *CH₂CH₂S |
| 252 | *C(O)CH₂CH₂ | *CH₂CH₂O | *CH₂CH₂CH₂S |
| 253 | *C(O)CH₂CH₂ | *CH₂CH₂O | *CH₂CH₂C(O) |
| 254 | *C(O)CH₂CH₂ | *CH₂CH₂O | CH₂CH₂ |
| 255 | *C(O)CH₂CH₂ | CH₂ | *CH₂CH₂CH₂O |
| 256 | *C(O)CH₂CH₂ | CH₂ | *CH₂CH₂C(O) |
| 257 | *C(O)CH₂CH₂ | CH₂ | *CH₂CH₂C(O)O |
| 258 | *C(O)CH₂CH₂ | CH₂ | *CH₂CH₂C(O)NH |
| 259 | *CH₂C(O) | CH₂ | CH₂CH₂ |
| 260 | *CH₂C(O) | CH₂ | *CH₂CH₂O |
| 261 | *CH₂C(O) | CH₂ | *CH₂CH₂NH |
| 262 | *CH₂CH₂C(O) | CH₂ | CH₂ |
| 263 | *CH₂CH₂C(O) | CH₂ | CH₂CH₂ |
| 264 | *CH₂CH₂C(O) | CH₂ | *CH₂CH₂O |
| 265 | *CH₂CH₂C(O) | CH₂ | *CH₂CH₂S |
| 266 | *CH₂CH₂O | *CH₂CH₂O | *CH₂CH₂O |
| 267 | *CH₂CH₂O | *CH₂CH₂O | *CH₂CH₂C(O) |
| 268 | *CH₂CH₂O | *CH₂CH₂O | *CH₂CH₂C(O)NH |
| 269 | *CH₂CH₂NH | CH₂ | *CH₂CH₂C(O)NCH₂ |
| 270 | *CH₂CH₂NH | CH₂ | *CH₂CH₂NHC(O) |
| 271 | *CH₂CH₂NH | *CH₂CH₂NH | *CH₂CH₂O |
| 272 | *CH₂CH₂NH | *CH₂CH₂NCH₂ | *CH₂CH₂C(O) |
| 273 | *CH₂CH₂NH | *CH₂CH₂O | *CH₂CH₂C(O)NH |
| 274 | *C(O)OCH₂ | CH₂ | *CH₂CH₂NH |
| 275 | *C(O)OCH₂ | CH₂ | *CH₂CH₂S |
| 276 | *C(O)OCH₂ | CH₂CH₂ | *CH₂CH₂O |
| 277 | *C(O)OCH₂ | CH₂CH₂ | *CH₂CH₂S |
| 278 | *C(O)OCH₂ | *CH₂CH₂NH | *CH₂CH₂O |
| 279 | *C(O)OCH₂ | *CH₂CH₂NH | *CH₂CH₂CH₂O |
| 280 | *C(O)OCH₂ | CH₂ | CH₂ |
| 281 | *C(O)OCH₂ | CH₂CH₂ | CH₂ |
| 282 | *C(O)NHCH₂ | CH₂ | *CH₂CH₂NH |
| 283 | *C(O)NHCH₂ | CH₂CH₂ | *CH₂CH₂NH |
| 284 | *C(O)NHCH₂ | *CH₂CH₂NH | *CH₂CH₂O |
| 285 | *C(O)NH | CH₂ | *CH₂CH₂S |
| 286 | *C(O)NH | CH₂CH₂ | *CH₂CH₂NH |
| 287 | *C(O)NH | CH₂CH₂ | *CH₂CH₂O |
| 288 | *C(O)NH | CH₂CH₂ | *CH₂CH₂S |
| 289 | *C(O)NH | *CH₂CH₂NH | *CH₂CH₂O |
| 290 | *C(O)O | CH₂ | *CH₂CH₂S |
| 291 | *C(O)O | CH₂CH₂ | *CH₂CH₂S |
| 292 | *C(O)CH₂O | CH₂ | *CH₂CH₂NH |
| 293 | *C(O)CH₂O | CH₂CH₂ | *CH₂CH₂C(O)NH |
| 294 | *C(O)CH₂O | CH₂CH₂ | *CH₂CH₂O |
| 295 | *C(O)CH₂O | CH₂CH₂ | *CH₂CH₂NH |
| 296 | *C(O)CH₂NH | *CH₂CH₂O | *CH₂CH₂S |
| 297 | *C(O)CH₂NH | *CH₂CH₂O | *CH₂CH₂O |
| 298 | *C(O)CH₂NH | CH₂CH₂ | *CH₂CH₂NH |
| 299 | CH₂ | *CH₂CH₂O | *CH₂CH₂O |

The invention also provides methods for preparing compounds represented by Formula(I), Formula (I-1-P1), Formula (I-1-P2), Formula (I-1-P3), Formula (I-1-P4), Formula (I-2), Formula (I-3), Formula (I'-1), (I'-2), (I'-3), Formula (II), Formula (II-A), Formula (II-A'), Formula (II-B), Formula (II-B'), Formula (II-C), Formula (II-C'), Formula (III), Formula (III-1), Formula (III-2), Formula (III-1-1), Formula (III-2-1), Formula (III-A), Formula (III-A'), Formula (III-B), Formula (III-B'), Formula (III-B'-1), Formula (III-C), Formula (III-C'), Formula (III-D), Formula (III-D'), Formula (III-E), Formula (III-E'), Formula (IV), Formula (A), Formula (B), Formula (B-1), Formula (C), Formula (C-1), Formula (D), Formula (D-1), Formula (B-2), Formula (B-3), Formula (C-2), Formula (C-3), Formula (D-2), Formula (D-3), Formula (E), Formula (F), Formula (E-1), Formula (E-2), Formula (E-3), Formula (E-4), Formula (F-1), Formula (F-2), Formula (G), Formula (G-1), Formula (G-2), Formula (G-3), Formula (G-4), Formula (H), Formula (H-1), Formula (H-2), Formula (H-3), Formula (H-4), Formula (D-c), Formula (D-f),Formula (D-g),and Formula (D-h), or pharmaceutically acceptable salts thereof.

The compounds of the general formulas mentioned can be prepared by a variety of methods, including but not limited to the following methods: 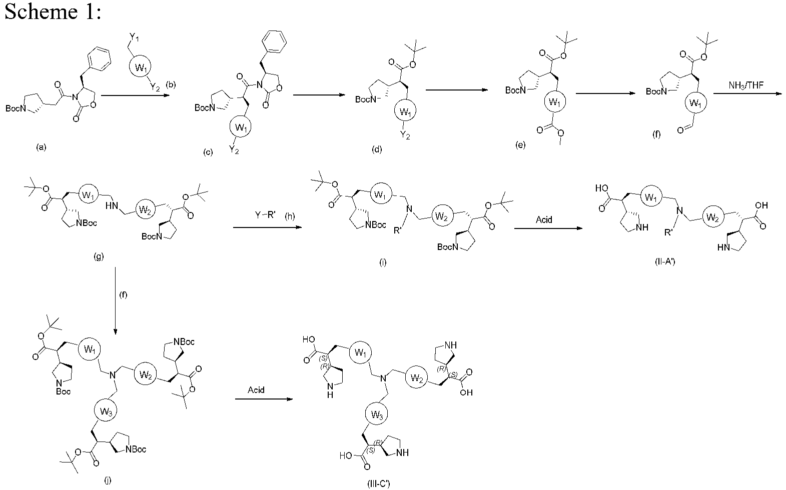 wherein, each of Y₁ and Y₂ is independently halogen(i.e. F, Cl, Br), preferably Br; Y is independently halogen(i.e. F, Cl, Br) or oxo (The "oxo" refers to the =O of -C(O)H), preferably Br or oxo (The "oxo" refers to the =O of -C(O)H); R' is defined as in the compounds of Formula (II), W₁, W₂, and W₃ are defined as in the compounds of Formula (I), Formula (I-1-P1), Formula (I-1-P2), Formula (I-1-P3), Formula (I-1-P4) and Formula (I-1);

Under the action of a base, Compound (a) and Compound (b) are reacted at room temperature for 16 h to obtain Compound (c), Compound (c) is hydrolyzed under alkaline conditions of hydrogen peroxide, and then esterified to obtain Intermediate (d), which is subjected to carbonyl insertion under atmospheric pressure to obtain Intermediate (e). DIBAL-H reduction gives Intermediate (f), and Intermediate (f) is subjected to reductive amination in a tetrahydrofuran solution of ammonia to obtain Intermediate (g). The intermediate is further subjected to reductive amination or substitution reaction to obtain Intermediate (i), and deprotection under acidic conditions gives the compound of Formula (II-A'); Intermediate (g) and Intermediate (f) undergo a substitution reaction or reductive amination to give Intermediate (j) which is deprotected under acidic conditions to give a compound of Formula (III-C'). 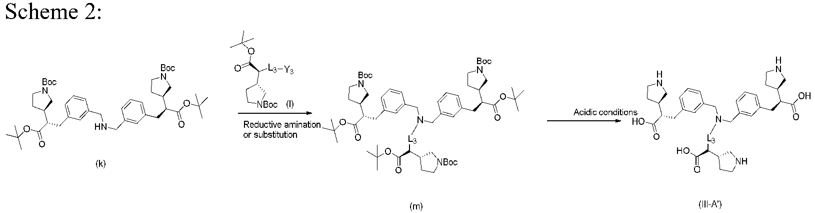 Y₃ is independently halogen (i.e. F, Cl, Br) or oxo (The "oxo" refers to the =O of -C(O)H), preferably Br or oxo (The "oxo" refers to the =O of -C(O)H), L₃ is defined as in the compounds of Formula (I) Formula (I-1-P1), Formula (I-1-P2), Formula (I-1-P3), Formula (I-1-P4) and Formula (I-1);
Compound (k) is synthesized according to the conditions of patent CN114008021A, Compound (m) is obtained from Compound (k) and Compound (l) by reductive amination or substitution reaction, and then deprotected under acidic conditions to obtain a compound of Formula (III-A'). 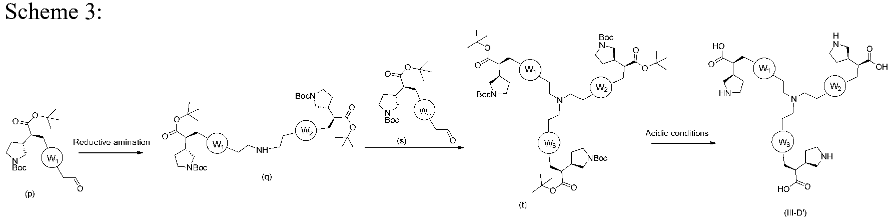 wherein, W₁, W₂, W₃ are defined as in the compounds of Formula (I) Formula (I-1-P1), Formula (I-1-P2), Formula (I-1-P3), Formula (I-1-P4) and Formula (I-1);
In the tetrahydrofuran solution of ammonia, Compound (p) is obtained by reductive amination of Compound (q); Compound (q) and Compound (s) are further subjected to reductive amination to obtain Intermediate (t); Intermediate (t) is deprotected under acidic conditions to obtain a compound of Formula (III-D'). 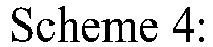 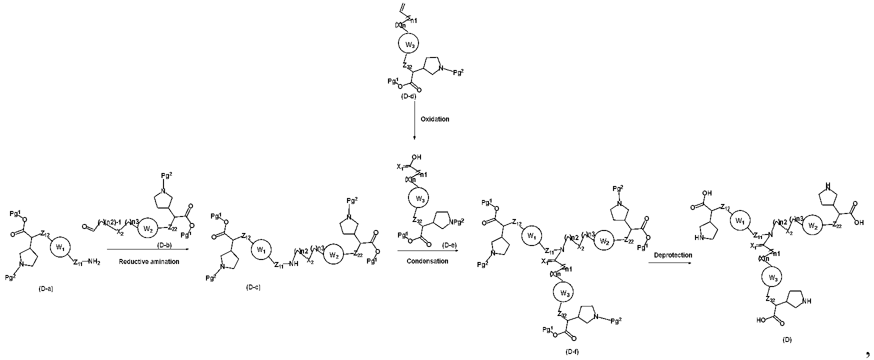 wherein, Z₁₁, Z₁₂, Z₂₂, Z₃₂, X, X₁, X₂, n, n1, n2, n3, and rings W₁, W₂, and W₃ are defined as in compounds of Formula (I), Formula (I-1-P1), Formula (I-1-P2), Formula (I-1-P3), Formula (I-1-P4), and Formula (D), each of Pg¹ and Pg² is independently a protecting group. Preferably, Pg¹ is independently selected from tert-butyl, methyl, and benzyl, and Pg² is independently selected from tert-butoxycarbonyl (Boc), 9-fluorenylmethoxycarbonyl (FMOC), andbenzyloxycarbonyl (Cbz), preferably Boc.

The invention also provides a pharmaceutical composition comprising the compounds of the invention, or pharmaceutically acceptable salts thereof.

The invention also provides a pharmaceutical composition comprising the compounds of the invention, or pharmaceutically acceptable salts thereof, and pharmaceutically acceptable excipients thereof.

In the above-mentioned pharmaceutical composition, the compound of the present invention, or a pharmaceutically acceptable salt thereof accounts for 0.1-99.9wt% of the pharmaceutical composition, for example, 0.5wt%, 1wt%, 2wt%, 3wt%, 4wt%, 5wt%, 6wt%, 7wt%, 8wt%, 9wt%, 10wt%, 20wt%, 30wt%, 40wt%, 50wt%, 60wt%, 70wt%, 80wt%, 90wt%, 91wt%, 92wt%, 93wt%, 94wt%, 95wt%, 96wt%, 97wt%, 98wt%, 99wt%, 99.5wt%, or a range formed by the combination of any two of the above-mentioned point values, and the balance is a pharmaceutically acceptable excipient.

In some embodiments of the invention, the pharmaceutical composition is used for an inhibitor of Lp(a) assembly.

In some embodiments of the invention, the pharmaceutical composition is used for a medicament for treating cardiovascular diseases (CVD).

The compounds shown in the invention, or pharmaceutically acceptable salts thereof may be carried out in pure form or in the form of a suitable pharmaceutical composition by providing any acceptable mode of administration of a medicament for similar use. The pharmaceutical composition of the invention may be prepared by combining the compounds of the invention with suitable pharmaceutically acceptable excipients. The pharmaceutical composition of the invention may be formulated as solid, semi-solid, liquid or gaseous formulations. In general, the above pharmaceutical composition may be prepared by conventional preparative methods using vehicles conventional in the field of formulations.

The invention also provides the use of the compounds of the invention, or pharmaceutically acceptable salts thereof, in the manufacture of a medicament for preventing and/or treating cardiovascular diseases (CVD).

In one embodiment of the invention, it provides the use of the compounds of the invention, or pharmaceutically acceptable salts thereof, in the manufacture of a medicament for preventing and/or treating diseases or conditions associated with elevated blood plasma Lp(a) levels.

In one embodiment of the invention, diseases or conditions associated with elevated blood plasma Lp(a) levels are cardiovascular diseases (CVD), including, but not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

The term "elevated blood plasma Lp(a) level" refers to a blood plasma level equal to or above a normal level, and for human beings, "elevated blood plasma level of Lp(a)" refers to a blood plasma level equal to or above about 30 mg/dL.

In one embodiment of the present invention, for human beings, the term "elevated blood plasma Lp(a) level" refers to blood plasma levels ≥ about 50 mg/dL. The compounds provided herein can be used in therapy to reduce blood plasma levels of Lp(a).

The invention also provides the use of the compounds of the invention, or pharmaceutically acceptable salts thereof, in the manufacture of a medicament for preventing and/or treating of diseases mediated by Lp(a).

Moreover, the invention provides the use, wherein diseases mediated by Lp(a) are cardiovascular diseases (CVD).

Furthermore, the invention provides the use, wherein said cardiovascular diseases (CVD) are selected from atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

The invention also provides a method for preventing and/or treating cardiovascular diseases, comprising administering to a patient a therapeutically effective amount of a compound of the invention, or a pharmaceutically acceptable salt of the compound or a pharmaceutical composition of the invention.

In one embodiment of the invention, it provides a method of preventing and/or treating diseases or conditions associated with elevated blood plasma Lp(a) levels, comprising administering to a patient a therapeutically effective amount of the compounds of the invention, or pharmaceutically acceptable salts thereof or pharmaceutical composition of the invention. In one embodiment of the invention, diseases or conditions associated with elevated blood plasma Lp(a) levels are cardiovascular diseases (CVD), including, but not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

The invention also provides a method for preventing and/or treating diseases mediated by Lp(a), comprising administering to a patient a therapeutically effective amount of the compounds of the invention, or pharmaceutically acceptable salts thereof or pharmaceutical composition of the invention; preferably, diseases mediated by Lp(a) are cardiovascular diseases (CVD), including, but not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

Further, the invention provides a use or method, wherein the compounds of the invention, or pharmaceutically acceptable salts thereof may be administered in combination with another compound for treating or preventing diseases mediated by Lp(a) such as cardiovascular diseases (CVD).

The invention also provides a compound of the invention, or a pharmaceutically acceptable salt thereof or a pharmaceutical composition of the invention for preventing and/or treating cardiovascular diseases.

The invention also provides a compound of the invention, or a pharmaceutically acceptable salt thereof or a pharmaceutical composition of the invention for use in medicament.

The invention provides a compound of the invention, or a pharmaceutically acceptable salt thereof or a pharmaceutical composition of the invention for use in preventing and/or treating cardiovascular disease. The invention provides a compound of the invention, or a pharmaceutically acceptable salt thereof or a pharmaceutical composition of the invention for use in preventing and/or treating diseases or conditions associated with elevated blood plasma Lp(a) levels. In one embodiment of the invention, the diseases or conditions associated with elevated blood plasma Lp(a) levels are cardiovascular diseases (CVD), including, but not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

The invention also provides a compound of the invention, or a pharmaceutically acceptable salt thereof or a pharmaceutical composition of the invention for use in preventing and/or treating diseases mediated by Lp(a); preferably, the diseases mediated by Lp(a) are cardiovascular diseases (CVD); further preferably, the cardiovascular diseases (CVD) include, but are not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

In one embodiment of the invention, it provides a compound of the invention, or a mixture thereof, or a pharmaceutically acceptable salt thereof or a pharmaceutical composition of the invention, for preventing and/or treating diseases or conditions associated with elevated blood plasma Lp(a) levels. In one embodiment of the invention, the diseases or conditions associated with elevated blood plasma Lp(a) levels are cardiovascular diseases (CVD), including, but not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

In a further aspect, this application provides a compound of the invention, or a pharmaceutically acceptable salt thereof or a pharmaceutical composition of the invention for the preventing and/or treating diseases mediated by Lp(a); preferably, the diseases mediated by Lp(a) are cardiovascular diseases (CVD); further preferably, the cardiovascular diseases (CVD) are selected from atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

The use of the compounds of the invention, or pharmaceutically acceptable salts thereof in the manufacture of a medicament for preventing and/or treating cardiovascular diseases in combination with another compound for preventing and/or treating cardiovascular diseases.

In one embodiment of the invention, cardiovascular diseases are diseases or conditions associated with elevated blood plasma Lp(a) levels.

In one embodiment of the invention, the diseases or conditions associated with elevated blood plasma Lp(a) levels are cardiovascular diseases (CVD), including, but not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

The compounds of the invention, or pharmaceutically acceptable salts thereof for use in preventing and/or treating cardiovascular diseases in combination with other compounds. Moreover, the another compound is for preventing and/or treating cardiovascular diseases. Moreover, the cardiovascular diseases are diseases or conditions associated with elevated blood plasma Lp(a) levels. Moreover, the disease or condition associated with elevated blood plasma Lp(a) levels is including, but are not limited to atherosclerotic cardiovascular disease (ASCVD), coronary artery stenosis, coronary artery-related diseases, aortic valve stenosis, aortic valve stenosis-related diseases, heart failure, heart failure-related diseases, atrial fibrillation, and atrial fibrillation-related diseases; said ASCVD includes peripheral vascular disease, peripheral artery-related diseases, coronary heart disease, ischemic stroke, and ischemic stroke-related diseases.

A compound of the invention or a pharmaceutically acceptable salt thereof can be co-applied with a therapeutic method. In certain embodiments, the therapeutic method may be, but is not limited to, Lp(a) plasmapheresis. The drug or therapeutic method may be co-applied or simultaneously applied. The drug or therapeutic method may be applied sequentially or subsequently.

The invention also provides a pharmaceutical composition, comprising a compound of the invention, or a pharmaceutically acceptable salt thereof, and another compound for treating or preventing diseases mediated by Lp(a); preferably, diseases mediated by Lp(a) are for example, cardiovascular diseases (CVD).

When the compounds of the invention or pharmaceutically acceptable salts thereof are administered in combination with an additional therapeutic agent for treating a disease such as a CVD, the compounds of the invention, or pharmaceutically acceptable salts thereof may provide a more effective therapeutic effect for cardiovascular-related diseases.

### Definition

The term "optional", "arbitrary", "optionally" or "arbitrarily" and the like means that the subsequently described event or circumstance may but does not necessarily occur, and that the description includes instances where the event or circumstance mentioned occurs and instances where it does not occur.

The "more" in "optionally substituted with one or more substituents independently selected from..." refers to 1, 2, 3, 4, 5, 6, 7, 8, 9 or 10; preferably 1, 2, 3 or 4; more preferably 1 or 2.

When "about" is used to describe molecular weight, the word "about" indicates rounding.

Unless otherwise specified, the term "alkyl" refers to a monovalent saturated aliphatic hydrocarbon group, a linear or branched group comprising from 1 to 20 carbon atoms, preferably from 1 to 10 carbon atoms (i.e., C₁₋₁₀alkyl), further preferably from 1 to 8 carbon atoms (C₁₋₈alkyl), and more preferably from 1 to 6 carbon atoms (i.e., C₁₋₆alkyl). For example, "C₁₋₆alkyl" means that the group is an alkyl group and the number of carbon atoms on the carbon chain is between 1 to 6 (specifically 1, 2, 3, 4, 5 or 6). The examples include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, and n-octyl.

Unless otherwise specified, the term "alkylene" refers to a monovalent saturated aliphatic hydrocarbon group, including straight or branched groups containing 1-20 carbon atoms, preferably containing 1-10 carbon atoms (i.e., C₁₋₁₀alkylene), more preferably containing 1-8 carbon atoms (C₁₋₈alkylene), and even more preferably containing 1-6 carbon atoms (i.e., C₁₋₆alkylene). For example, "C₁₋₆alkylene" refers to an alkylene group, and the number of carbon atoms on the carbon chain is between 1-6 (specifically 1, 2, 3, 4, 5, or 6). Examples include, but are not limited to, methylene, ethylene, n-propylene, n-pentylene, n-hexylene, and 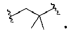 .

Unless otherwise specified, the term "alkenyl" refers to a straight or branched chain unsaturated aliphatic hydrocarbon group composed of carbon atoms and hydrogen atoms and with at least one double bond. Alkenyl may contain 2-20 carbon atoms, preferably 2-10 carbon atoms (i.e., C₂₋₁₀alkenyl), more preferably 2-8 carbon atoms (C₂₋₈alkenyl), and even more preferably 2-6 carbon atoms (i.e., C₂₋₆alkenyl), 2-5 carbon atoms (i.e., C₂₋₅alkenyl), 2-4 carbon atoms (i.e., C₂₋₄alkenyl), 2-3 carbon atoms (i.e., C₂₋₃alkenyl), and 2 carbon atoms (i.e., C₂alkenyl). For example, "C₂₋₆alkenyl" refers to an alkenyl group, and the number of carbon atoms on the carbon chain is between 2-6 (specifically 2, 3, 4, 5, or 6). Non-limiting examples of alkenyl groups include, but are not limited to, vinyl, 1-propenyl, 2-propenyl, 1-butenyl, isobutenyl, and 1,3-butadienyl.

Unless otherwise specified, the term "alkynyl" refers to a straight or branched chain unsaturated aliphatic hydrocarbon group with at least one triple bond composed of carbon atoms and hydrogen atoms. Alkynyl may contain 2-20 carbon atoms, preferably contains 2-10 carbon atoms (i.e., C₂₋₁₀alkynyl), more preferably contains 2-8 carbon atoms (C₂₋₈alkynyl), even more preferably contains 2-6 carbon atoms (i.e., C₂₋₆alkynyl), 2-5 carbon atoms (i.e., C₂₋₅alkynyl), 2-4 carbon atoms (i.e., C₂₋₄alkynyl), 2-3 carbon atoms (i.e., C₂₋₃alkynyl), and 2 carbon atoms (i.e., C₂alkynyl). For example, "C₂₋₆alkynyl" refers to an alkynyl group, and the number of carbon atoms on the carbon chain is between 2-6 (specifically 2, 3, 4, 5, or 6). Non-limiting examples of alkynyl groups include, but are not limited to, ethynyl, 1-propynyl, 2-propynyl, and 1-butynyl.

Unless otherwise specified, the term "cycloalkyl" refers to a monocyclic, bicyclic or polycyclic cyclic saturated aliphatic hydrocarbon group with a specified number of carbon atoms, preferably containing 3-14 carbon atoms (i.e., C₃₋₁₄cycloalkyl), preferably containing 4-14 carbon atoms (i.e., C₄₋₁₄cycloalkyl), preferably containing 5-14 carbon atoms (i.e., C₅₋₁₄cycloalkyl), more preferably containing 6-14 carbon atoms (C₆₋₁₄cycloalkyl), and further preferably 6-12 carbon atoms (C₆₋₁₂cycloalkyl). The examples include, but are not limited to, cyclohexyl and spiro[3.5]nonyl.

Unless otherwise specified, the term "oxaalkyl" refers to an alkyl residue in which one or more carbons (and their associated hydrogens) are replaced by oxygen, such as "alkoxy", "alkoxyalkyl". For example, C₃₀oxaalkylene includes -OC₁₋₃alkoxy, -CH₂OCH₂CH₃, -CH₂CH₂OCH₃, etc. The examples include methoxy, ethoxy, propoxy and methoxypropyl. The term oxaalkyl has as its ordinary meaning as understood in the field [See Nomenclature of Organic Compounds: Principles and Practice, published by the American Chemical Society, 196, but not limited to 127(a)], that is, it refers to compounds in which the oxygen is bonded to its adjacent atom by a single bond (forming an ether bond); it does not refer to double-bond oxygen found in carbonyl groups. The terms "thiaalkyl" and "selenaalkyl" are similar to "oxaalkyl". The term "azaalkyl" refers to an alkyl group containing atomic groups "NH" or "-N(C₁₋₃alkyl)-", e.g., C₃azaalkylene includes - NHCH₂CH₂CH₃, -CH₂NHCH₂CH₃, -CH₂CH₂NHCH₃, -CH₂CH₂N(CH₃)CH₃, etc.

"Alkoxy" means -O-alkyl, defined hereinabove, that is, comprising 1-20 carbon atoms, preferably 1-10 carbon atoms, more preferably 1-8 carbon atoms, and even more preferably 1-6 carbon atoms (specifically 1, 2, 3, 4, 5 or 6). The examples include, but are not limited to, methoxy, ethoxy, propoxy, isopropoxy, butoxy, 1-methylpropoxy, 2-methylpropoxy, tert-butoxy, pentoxy, 1-methylbutoxy, 2-methylbutoxy, 3-methylbutoxy, 1,1-dimethylpropoxy, 1,2-dimethylpropoxy, 2,2-dimethylpropoxy, and 1-ethylpropoxy.

Unless otherwise specified, the term "halogen" or "halo/halogenated" refers to F, Cl, Br, orI.

Unless otherwise specified, the term "heterocyclic/heterocyclyl" refers to a saturated or partially unsaturated monocyclic, bicyclic or polycyclic hydrocarbon substituent that is nonaromatic and contains from 3 to 20 ring atoms, of which 1, 2, 3 or more ring atoms are selected from N, O or S, and the remaining ring atoms are C. Preferably it includes 3-12 ring atoms, further preferably 3-10 ring atoms, or 3-8 ring atoms, or 3-6 ring atoms, or 4-6 ring atoms, or 5-6 ring atoms. Heteroatoms are preferably 1-4, more preferably 1-3 (that is, 1, 2 or 3). The examples of monocyclic heterocyclics include pyrrolidinyl, imidazolidinyl, tetrahydrofuranyl, dihydropyrrolidinyl, piperidinyl, piperazinyl, pyranyl, etc. Bicyclic or polycyclic heterocyclic groups include heterocyclic groups of spiro, fused, and bridged rings.

Unless otherwise specified, the term "aryl" means an aromatic carbon ring system containing 6-16 carbon atoms, or 6-14 carbon atoms, or 6-12 carbon atoms, or a monocyclic, bicyclic and tricyclic ring of 6-10 carbon atoms, preferably 6-10 carbon atoms, and the term "aryl" may be used interchangeably with the term "aromatic ring". The examples of aryl groups may include, but are not limited to, phenyl, naphthyl, anthryl, phenanthryl, or pyrenyl.

Unless otherwise specified, the term "heteroaryl" means an aromatic monocyclic, bicyclic or polycyclic ring system containing 5-16 membered structures, or 5-14 membered structures, 5-12 membered structures, 5-10 membered structures, 5-8 membered structures, wherein 1, 2, 3 or more ring atoms are heteroatoms and the remaining atoms are carbon, the heteroatoms are independently selected from O, N or S, and the number of heteroatoms is preferably 1, 2 or 3. Polycyclic heteroaryl is fused heteroaryl. The examples of heteroaryl groups may include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazinyl, triazinyl, phthalazinyl, quinolinyl, isoquinolinyl, pteridinyl, purinyl, indolyl, isoindolyl, indazolyl, benzofuranyl, benzothiophenyl, benzoylpyridinyl, benzopyrimidinyl, quinoxalinyl, benzimidazoyl, benzophthalazinyl, pyrrolo[2,3-b]pyridinyl, imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridinyl.

Unless otherwise specified, the term "fused heteroaryl" refers to an aromatic ring system formed by two or more cyclic structures sharing two adjacent atoms with each other, each ring in the fused heteroaryl being an unsaturated aromatic ring, which may contain 5-20 ring atoms, preferably 6-14 ring atoms, more preferably 7-10 ring atoms, containing 1-4 cyclic heteroatoms, preferably 1-3 (that is, 1, 2 or 3) ring heteroatoms, and the heteroatoms are independently selected from N, O and S. Fused heteroaryl groups include bicyclic, tricyclic, tetracyclic or polycyclic fused heteroaryl, preferably bicyclic, tricyclic or tetracyclic fused heteroaryl, more preferably bicyclic or tricyclic fused heteroaryl. The examples of fused heteroaryl groups include (but are not limited to) 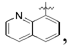 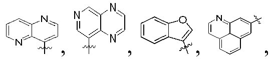 etc.

Unless otherwise specified, the term "pharmaceutically acceptable salt (s)" refers to salts that are, within the scope of sound medical judgment, suitable for use in contact with the tissues of mammals, particularly humans, irritation, allergic response, etc., and are commensurate with a reasonable benefit/risk ratio, such as medically acceptable salts of amines, carboxylic acids, and other types of compounds, which are well known in the field. The salt may be prepared *in situ* during the final isolation and purification of the compounds of the invention or by reacting the free base or free acid with a suitable reagent alone.

The compounds of the present invention and pharmaceutically acceptable salts also include "stereoisomers" thereof. Unless otherwise specified, the term "stereoisomer" refers to a compound with the same chemical structure but with different spatially arranged atoms or groups. Stereoisomers include enantiomers, diastereomers, conformers (rotamers), geometric isomers (cis/trans-isomerism), atropisomers, etc. Any obtained mixtures of stereoisomers can be separated into pure or substantially pure geometric isomers, enantiomers, and diastereomers, for example, by chromatography and/or fractional crystallization. Compounds and salts described in this specification may include one or more chiral (i.e., asymmetric) centers.To the extent a structure or chemical name in this specification does not indicate the chirality, the structure or name is intended to encompass any single stereoisomer (i.e., any single chiral isomer) corresponding to that structure or name, as well as any mixture of stereoisomers (e.g., a racemate). In some embodiments, a single stereoisomer is obtained by isolating it from a mixture of isomers (e.g., a racemate) using, for example, chiral chromatographic separation. In other embodiments, a single stereoisomer is obtained through direct synthesis from, for example, a chiral starting material.

A particular enantiomer of a compound described herein may be more active than other enantiomers of the same compound. In one embodiment, the compound, or a pharmaceutically acceptable salt thereof, is a single enantiomer being in an enantiomeric excess (%ee) of ≥ 90%, ≥ 95%, ≥ 96%, ≥ 97%, ≥ 98%, or ≥ 99%. In one aspect, the single enantiomer is present in an enantiomeric excess (%ee) of ≥ 99%.

A particular diastereoisomer of a compound described herein may be more active than other diastereoisomers of the same compound. In one embodiment, the compound, or a pharmaceutically acceptable salt thereof, is a single diastereoisomer being in a diastereomeric excess (%de) of ≥ 90% ≥ 95%, ≥ 96%, ≥ 97%, ≥ 98%, or ≥ 99%. In one aspect, the single diastereomer is present in an diastereomeric excess (%de) of ≥ 99%.

The compounds of the invention and pharmaceutically acceptable salts thereof also include their "Tautomers". Compounds and salts described in this specification may exist in various tautomeric forms. "Tautomers" are structural isomers that exist in equilibrium resulting from the migration of a hydrogen atom. Unless otherwise specified, the term "tautomer" refers to structural isomers with different energies that can be interconverted by low energy barriers. If tautomerism is possible (e.g. in solution), the chemical equilibrium of the tautomer can be achieved. For example, proton tautomers (also known as prototropic tautomers) include interconversion via proton migration such as keto-enol isomerization and imine-enamine isomerization. Valence tautomers include interconversions by the reorganization of some of the bonding electrons.

The compounds of the invention and pharmaceutically acceptable salts thereof also include their "isotopic derivatives". Unless otherwise specified, the term "isotopic derivative" refers to a compound of the invention that may exist in an isotopically labelled or enriched form containing one or more atoms with an atomic mass or mass number different from the atomic mass or mass number of the atom which is present in the greatest abundance in nature. Isotopes can be radioactive or non-radioactive isotopes. Isotopes that are typically used as isotopic labels include isotopes of hydrogen, ²H and ³H; isotopes of carbon: ¹³C and ¹⁴C; isotopes of chlorine: ³⁵Cl and ³⁷Cl; isotopes of fluorine: ¹⁸F; isotopes of iodine: ¹²³I and ¹²⁵I; isotopes of nitrogen: ¹³N and ¹⁵N; isotopes of oxygen: ¹⁵O, ¹⁷O and ¹⁸O and isotopes of sulfur ³⁵S. These isotopically labeled compounds can be used to study the distribution of pharmaceutical molecules in tissues. Especially ³H and ¹³C, are more widely used because they are easy to label and easy to detect. Substitution of certain heavy isotopes, such as heavy hydrogen (²H), enhances metabolic stability, prolongs half-life, and thus provides therapeutic advantages for dose reduction. Isotopically labeled compounds generally begin with labeled starting materials and are synthesized using known synthetic techniques like non-isotopically labeled compounds. All compounds disclosed herein, including genernal formula compounds and specific compounds, where an atom is replaced by one or more of its isotopes (for example a compound of Formula (I) where one or more carbon atoms is an ¹¹C or ¹³C carbon isotope, or where one or more hydrogen atoms is a ²H or ³H isotope), are encompassed herein.

Unless otherwise specified, the compounds of the invention and pharmaceutically acceptable salts thereof also include their "solvates". The term "solvate" refers to the physical association of a compound of the invention with one or more solvent molecules, whether organic or inorganic. This physical association includes hydrogen bonds. In some cases, for example, when one or more solvent molecules are incorporated into the crystal lattice of a crystalline solid, the solvate will be able to be separated. Solvent molecules in solvates may be present in regular and/or disordered order. Solvates may contain stoichiometric or non-stoichiometric solvent molecules. "Solvate" covers the solution phase and separable solvates. The examples of solvates include, but are not limited to, hydrates, ethanolates, methanolates, and isopropanolates. Solvation methods are well known in the field. The term "hydrate" refers to a substance in which a water molecule binds to a cation or anion in a compound by a coordination bond or a covalent bond, or refers to a substance in which the water ion does not bind directly to a cation or anion but is present in a certain proportion at a defined position in the solid lattice.

Unless otherwise specified, the compounds of the invention and pharmaceutically acceptable salts thereof also include their "prodrugs". The term "prodrug" refers to a drug that is converted *in vivo* to a parent drug. Prodrugs are usually useful as they can improve certain determined and undesirable physical or biological properties. Physical properties are usually relevant solubility (excessive or insufficient lipid or water solubility) or stability, while problematic biological characteristics include rapid metabolism or poor bioavailability, which may themselves be related to physicochemical properties, for example, they can be bioavailable through oral administration, whereas the parent compound cannot. The solubility of the prodrug in the pharmaceutical composition is also improved compared to the parent drug. An example of, but not limited to, a prodrug of the invention may be any compound of the invention administered as an ester ("prodrug") to facilitate transmission across the cell membrane, where water solubility is detrimental to mobility, but once it enters the intracellular water solubility is beneficial, which is subsequently metabolically hydrolyzed to a carboxylic acid, the active entity. Another example of a prodrug may be a short peptide (polyamino acid) bound to an acid moiety in which the peptide is metabolized to show an active moiety.

The term "oxo" refers to the replacement of two H at the same substitution position by one O to form a double bond.

The term "thio" refers to the replacement of two H at the same substitution position by one S to form a double bond.

The term "therapeutically effective amount" refers to the amount of a compound or combination of compounds that provides the intended clinical or therapeutic benefit in the subject being treated. The therapeutically effective amount will depend on the general condition of the subject being treated (e.g., weight, age and sex), the severity of the disease, the specific compound being administered, the dosing regimen, the use of concomitant medications and other factors, and can be determined by the prescribing physician in accordance with routine practice. For example, the pharmaceutical composition can be administered at a dose appropriate for the disease to be treated (or prevented), such as administering about 0.5 µg to about 50 mg of at least one compound/kg of subject body weight, preferably about 10 µg to about 100 mg/kg of body weight/day.

The term "pharmaceutically acceptable excipient" refers to a carrier that does not cause significant irritation to an organism and does not eliminate the biological activity and properties of the administered compound. Any commonly used pharmaceutically acceptable carrier may be used, the selection of which depends on factors such as the specific mode of administration, the effect of the carrier on solubility and stability, and the nature of the dosage form and is within the ordinary skills of those skilled in the art. Examples of the pharmaceutically acceptable excipient include, but are not limited to, diluents, excipients, fillers, binders, wetting agents, disintegrants, lubricants, colorants, fragrances, absorption promoters, surfactants, adsorption carriers, etc. which are conventional in the pharmaceutical field.

Unless otherwise specified, the term "treatment" encompasses any treatment of a patient's disease, disorder, and condition, including: (a) inhibiting a disease, disorder, or condition, i.e., arresting its development; or (b) relief of symptoms of a disease, condition and condition, that is, leading to resolution of a disease or symptoms; or (c) ameliorating or eliminating a disease, disorder and condition or one or more symptoms associated with the disease.

Abbreviations used in the preparation examples, examples, and elsewhere herein are:
THF: tetrahydrofuran; Dioxane: 1,4-dioxane; FA: formic acid; PE: petroleum ether; EA: ethyl acetate; DCM: dichloromethane; TFA: trifluoroacetic acid; XPhos Pd G2: Chloro(2-dicyclohexylphosphino-2',4',6'-tri-isopropyl-1,1'-biphenyl)[2-(2'-amino-1,1'-biphenyl)] palladium(II).

The beneficial effects of the invention are one or more of the following:
The invention designs a class of compounds with novel structures, providing a new direction for the development of drugs that reduce plasma Lp(a) levels. *In vitro* activity studies showed that the compound of the invention had strong inhibitory effects on Lp(a) assembly; the compound of the invention had good pharmacokinetic properties; safety evaluation experiments showed that the compound of the invention had excellent safety; *in vivo* pharmacodynamic experiments in mice showed that the compound of the invention significantly reduced serum Lp(a) levels and therefore could be used as a promising compound for the treatment of diseases mediated by Lp(a). In addition, the invention investigated a specific synthesis method, which is simple in process, convenient in operation, and is advantageous for large-scale industrial production and application.

### Detailed description of the invention

The invention is further elaborated below in conjunction with specific examples. It should be understood that these examples are intended to illustrate the invention only and not to limit the scope of the invention. Experimental methods without specific conditions are indicated in the following examples, usually under conventional conditions or as recommended by the manufacturer. All professional and scientific terms used herein have the same meaning as those familiar to professionals in the field, unless otherwise defined. In addition, any method and material similar to or equal to what has been described can be applied to the method of the invention. The preferred implementation methods and materials shown in the text are for demonstration purposes only.

The structures of the compounds of the invention are determined by nuclear magnetic resonance (NMR) or/and liquid chromatography-mass spectrometry (LC-MS) or/and high-performance liquid chromatography (HPLC). NMR was determined using Bruker 400MHz or/and Varian 400MHz; LC-MS was performed using Agilent, 1260 Infinity II-6120/6125MSD; HPLC was performed using Waters UPCC (CA-352).

The starting materials used in the examples of the invention are either known and commercially available or can be synthesized by methods known in the field or by analogy therewith.

The invention provides the methods of preparing the compounds. The compounds can be prepared by the following steps.

### Synthesis of tert-butyl (R)-3-((S)-1-(tert-butoxy)-3-(3-formylphenyl)-1-oxopropan-2-yl)pyrrolidine-1-carboxylate (Intermediate 1-5)

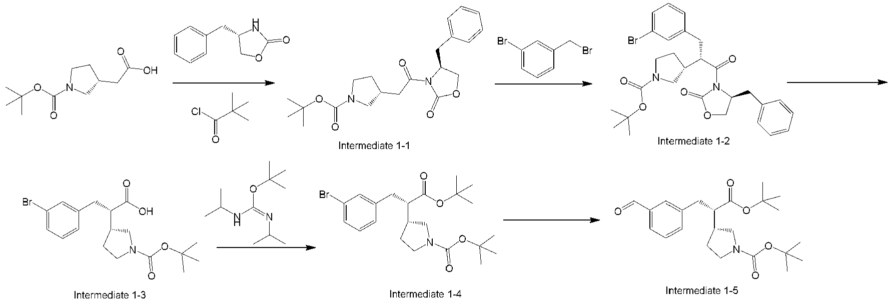

### Step 1: Synthesis of tert-butyl (R)-3-(2-((S)-4-benzyl-2-oxooxazolidin-3-yl)-2-oxoethyl)pyrrolidine-1-carboxylate (Intermediate 1-1)

Triethylamine (11.03g, 109.04mmol, 2.5eq) was added to a solution of (R)-2-(1-(tert-butoxycarbonyl)pyrrolidin-3-yl)acetic acid (10g, 43.62mmol, 1eq) in tetrahydrofuran (150mL) maintained at 10°C. After 5 min, pivaloyl chloride (6.57g, 54.52mmol, 1.25eq) was added. After 15 min, lithium chloride (2.31g, 54.52mmol, 1.25eq) and (S)-4-benzyloxazolidin-2-one (7.73g, 43.62mmol, 1eq) in tetrahydrofuran (50mL) were added. The mixture was warmed to room temperature and stirred for 24h. After 24h 1N HCl aqueous solution (50mL) was added and the organic phase was separated from the aqueous phase. The organic phase was washed with 1N aqueous sodium hydroxide (50mL) and saturated aqueous NaCl solution (50mL), dried over anhydrous sodium sulfate, filtered and concentrated in vacuo, and purified by rapid chromatography (silica gel, petroleum ether:ethyl acetate=5:1, v/v) to give the target compound (15g). LCMS(ESI)[M+H]⁺=332.7. ¹HNMR(400MHz, CDCl₃) δ 7.38-7.27 (m, 3H), 7.23-7.17 (m, 2H), 4.68 (ddd, *J*=10.8, 7.2, 3.2Hz, 1H), 4.26-4.16 (m, 2H), 3.68 (dd, *J*=10.8, 7.2Hz, 1H), 3.48 (ddd, *J*=11.6, 8.4, 3.6Hz, 1H), 3.37-3.23 (m, 2H), 3.12-2.94 (m, 3H), 2.78 (dd, *J*=13.2, 9.6Hz, 1H), 2.67 (dt, *J*=14.8, 7.2Hz, 1H), 2.17-2.06 (m, 1H), 1.63-1.55 (m, 1H), 1.46 (d, *J*=4.4Hz, 9H).

### Step 2: Synthesis of tert-butyl (R)-3-((S)-1-((S)-4-benzyl-2-oxooxazolidin-3-yl)-3-(3-bromophenyl)-1-oxopropan-2-yl)pyrrolidine-1-carboxylate (Intermediate 1-2)

Under nitrogen protection, a solution of lithium bis(trimethylsilyl)amide (1M in THF, 30.1mL, 30.1mmol, 1eq) was added dropwise to a solution of *tert*-butyl (R)-3-(2-((S)-4-benzyl-2-oxooxazolidin-3-yl)-2-oxoethyl)pyrrolidine-1-carboxylate (12g, 30.89mmol, 1eq) in tetrahydrofuran (100mL) at 0°C. The mixture was stirred at 0°C for 30 min, then a solution of 1-bromo-3-(bromomethyl)benzene (8.49g, 33.98mmol, 1.1eq) in tetrahydrofuran (20mL) was added slowly, and the reaction mixture was slowly warmed to room temperature and stirred overnight. The reaction mixture was cooled using an ice-water bath, a saturated aqueous solution of ammonium chloride was added, water was added to the mixture and extracted with ethyl acetate, and then the organic layer was washed with saturated aqueous sodium chloride. The organic matter was dried over anhydrous sodium sulfate, filtered, and concentrated in vacuo to give the target compound (17g). LCMS(ESI)[M-*tert*-butyl+H]⁺=501.1.

### Step 3: Synthesis of (S)-3-(3-bromophenyl)-2-((R)-1-(tert-butoxycarbonyl)pyrrolidin-3-yl)propanoic acid (Intermediate 1-3)

A solution of hydrogen peroxide (0.88 M in water, 3.4mL, 32.3mmol, 1.5eq) was added in one portion to a solution of *tert*-butyl (R)-3-((S)-1-((S)-4-benzyl-2-oxooxazolidin-3-yl)-3-(3-bromophenyl)-1-oxopropan-2-yl)pyrrolidine-1-carboxylate (12g, 21.53mmol, 1eq) in tetrahydrofuran (120mL), cooled with an ice/water bath, followed by adding a solution of lithium hydroxide monohydrate (0.77g, 32.3mmol, 1.5eq) in water (10mL), and the reaction temperature was raised to room temperature and stirred for 2.5h. The reaction mixture was cooled to 0°C and a solution of sodium bisulfite (4.1g) in water (20mL) was added, followed by adding a solution of sodium hydroxide in water (5N) to adjust the pH of the reaction mixture to >12. Then water and methyl *tert*-butyl ether were added to separate the layers and the aqueous layer was extracted with methyl *tert*-butyl ether. The combined organics were extracted with water, and then this aqueous extract was added to the main aqueous solution, the aqueous solution was stirred with methyl *tert-*butyl ether (100mL) and the mixture was cooled to 0°C, hydrochloric acid (5N) was added to adjust the pH of the solution to 3; the layers were separated and the organic layer was washed with a mixture of water and saturated aqueous NaCl solution; the organic matter was dried over anhydrous sodium sulfate, filtered, and concentrated to give the target compound (5.5g). LCMS(ESI)[M-*tert*-butyl+H]⁺=341.6.

### Step 4: Synthesis of tert-butyl (R)-3-((S)-3-(3-bromophenyl)-1-(tert-butoxy)-1-oxopropan-2-yl)pyrrolidine-1-carboxylate (Intermediate 1-4)

(S)-3-(3-bromophenyl)-2-((R)-1-(tert-butoxycarbonyl)pyrrolidin-3-yl)propanoic acid (5.5g, 13.81mmol, 1eq) was dissolved in 2-methyltetrahydrofuran (80mL). Under nitrogen protection, *tert*-butyl N,N'-diisopropylcarbamimidate (13.83g, 69.05mmol, 5eq) was added, and the mixture was heated to 65°C and stirred for 16h. The insoluble materials were filtered off, the filter cake was washed with methyl *tert*-butyl ether. The filtrate was concentrated and purified by rapid chromatography (silica gel, petroleum ether:ethyl acetate=10:1) to give the target compound (5g). LCMS(ESI)[M-2×*tert*-butyl+H]⁺=343.6.

### Step 5: Synthesis of tert-butyl (R)-3-((S)-1-(tert-butoxy)-3-(3-formylphenyl)-1-oxopropan-2-yl)pyrrolidine-1-carboxylate (Intermediate 1-5)

A solution of *tert*-butyl (R)-3-((S)-3-(3-bromophenyl)-1-(tert-butoxy)-1-oxopropan-2-yl)pyrrolidine-1-carboxylate (3g, 6.6mmol, 1eq) in toluene (25mL) was added to a pressure vessel, followed by adding palladium acetate (148.22mg, 0.66mmol, 0.1eq), n-butyldi(1-adamantyl)phosphine (355.07mg, 0.99mmol, 0.15eq) and N,N,N',N'-tetramethylethylenediamine (1.15g, 9.9mmol, 1.5eq); the mixture was stirred at 100°C for 16h under 2 MPa syngas (CO/H₂ 1:1) atmosphere. The insoluble materials were filtered off through a celite pad, the filter cake was washed with ethyl acetate, and the filtrate was concentrated. Purification by rapid chromatography (silica gel, petroleum ether:ethyl acetate=5: 1) was performed to give the target compound (3g). LCMS(ESI)[M-Boc]⁺=303.8. ¹HNMR(400MHz, CDCl₃) δ 9.99 (s, 1H), 7.72 (dd, *J*=8.6, 4.5Hz, 2H), 7.44 (dd, *J*=8.5, 4.4Hz, 2H), 3.66 (dd, *J*=10.3, 7.7Hz, 1H), 3.50 (t, *J*=8.6Hz, 1H), 3.26 (td, *J*=10.4, 6.9Hz, 1H), 3.06-2.83 (m, 3H), 2.56-2.48 (m, 1H), 2.40 (dd, *J*=16.5, 7.1Hz, 1H), 2.01-1.90 (m, 1H), 1.68 (dt, *J*=22.3, 10.2Hz, 1H), 1.47 (s, 9H), 1.26 (s, 5H).

### Synthesis of di-tert-butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (Intermediate 1-6)

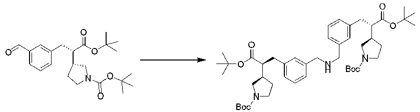

*Tert*-butyl (R)-3-((S)-1-(tert-butoxy)-3-(3-formylphenyl)-1-oxopropan-2-yl)pyrrolidine-1-carboxylate (1.6g, 3.97mmol, 1eq) was dissolved in tetrahydrofuran (10mL), ammonia methanol solution (0.62mL, 4.36mmol, 1.1eq, 7 N) was added, and the mixture was stirred at room temperature for 0.5h. Then sodium cyanoborohydride (0.49g, 7.9.3mmol, 2eq) was added and the mixture was stirred at room temperature for 16h. Liquid chromatography-mass spectrometry (LCMS) showed that the reaction was completed, and the reaction solution was directly concentrated and purified by rapid chromatography (silica gel, dichloromethane:methanol=30:1) to give the target compound (400mg). LCMS(ESI)[M+H]⁺=793.3.

### Preparation of Intermediate 2

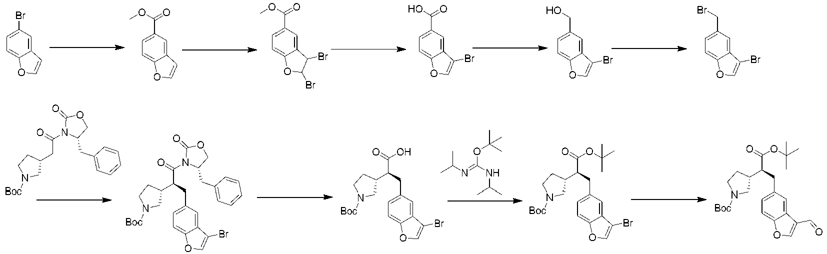

### Step 1: Synthesis of methyl benzofuran-5-carboxylate

In an autoclave, 5-bromobenzofuran (20.00g, 101.51mmol, 1eq), palladium acetate (2.28g, 10.15mmol, 0.1eq) and triethylamine (2.05g, 203.02mmol, 2eq) were added to a mixed solvent of dimethyl sulfoxide (100mL) and methanol (100mL), CO gas was introduced until the pressure reached 20 atmospheres, the oil bath was heated to 80°C, and the reaction was carried out for 18h. Water was added into the reaction. The mixture was extracted with ethyl acetate, the organic phases were combined, washed with saturated sodium chloride solution, dried over anhydrous sodium sulfate, filtered and concentrated, and separated through a normal phase separation column (petroleum ether:ethyl acetate=10:1) to give the target compound (6.00g). ¹HNMR (400MHz, CD₃OD) δ 8.35 (d, J=1.6Hz, 1H), 8.01 (dd, J=8.8, 1.6Hz, 1H), 7.87 (d, J=2.4Hz, 1H), 7.58 (d, J=8.8Hz, 1H), 7.00-6.89 (m, 1H), 3.94 (s, 3H).

### Step 2: Synthesis of methyl 2,3-dibromo-2,3-dihydrobenzofuran-5-carboxylate

Methyl benzofuran-5-carboxylate (6.00g, 34.06mmol, 1eq) was added in anhydrous dichloromethane (60mL) and then bromine (6.53g, 40.87mmol, 1.2eq) was added slowly dropwise at 0°C under nitrogen protection for 2h. The reaction mixture was added slowly dropwise to a solution of sodium sulfite, extracted with dichloromethane, washed with saturated brine, dried over anhydrous sodium sulfate and concentrated to give the crude target compound (11.44g, crude). The crude compound was used directly in the next step without further purification. ¹HNMR(400MHz, CD₃OD) δ 8.14 (s, 1H), 8.00 (dd, J=8.4, 1.6Hz, 1H), 7.15 (s, 1H), 7.06 (d, J=8.4Hz, 1H), 5.97 (s, 1H), 3.82 (d, J=3.6Hz, 3H).

### Step 3: Synthesis of 3-bromobenzofuran-5-carboxylic acid

Methyl 2,3-dibromo-2,3-dihydrobenzofuran-5-carboxylate (11.00g, 32.74mmol, 1eq) was dissolved in ethanol, an ethanol solution of potassium hydroxide (2.0eq, 15%) was added, and the reaction was carried out at 80°C for 2h. The pH of the reaction mixture was adjusted to 2-3 with hydrochloric acid (2M) solution. The mixture was extracted with ethyl acetate, washed with saturated brine, dried over anhydrous sodium sulfate, and concentrated to give the target compound (10.00g, crude). The crude compound was used directly in the next step without further purification. Step 4: Synthesis of (3-bromobenzofuran-5-yl)methanol

3-bromobenzofuran-5-carboxylic acid (10.00g, 41.49mmol, 1.0eq) was dissolved in tetrahydrofuran, and a solution of borane/tetrahydrofuran (860mg, 62.23mmol, 1.5eq) was added at 0°C and the reaction was carried out at 0°C for 2h. Methanol was added dropwise at low temperature to quench the reaction, and the mixture was concentrated to give the target compound (10.00g, crude). The crude compound was used directly in the next step without further purification. LCMS(ESI)[M-OH]⁺=209.2.

### Step 5: Synthesis of 3-bromo-5-(bromomethyl)benzofuran

(3-bromobenzofuran-5-yl)methanol (10.00g, 37.44mmol, 1eq) was dissolved in dichloromethane (100mL), and phosphorus tribromide (11.15g, 41.18mmol, 1.1eq) was slowly added dropwise at 0°C, and the reaction was carried out at 0°C for 2h. The reaction mixture was slowly poured into water, extracted with dichloromethane, washed with saturated brine, dried over anhydrous sodium sulfate, and filtered and concentrated to give the crude target compound (10.10g). The crude compound was used directly in the next step without further purification.

### Step 6: Synthesis of tert-butyl (R)-3-((S)-1-((S)-4-benzyl-2-oxooxazolidin-3-yl)-3-(3-bromobenzofuran-5-yl)-1-oxopropan-2-yl)pyrrolidine-1-carboxylate

*Tert*-butyl (R)-3-(2-((S)-4-benzyl-2-oxooxazolidin-3-yl)-2-oxoethyl)pyrrolidine-1-carboxylate (10.25g, 26.38mmol, 1eq) was dissolved in tetrahydrofuran (100mL), and lithium bis(trimethylsilyl)amide (5.30g, 31.65mmol, 1.2eq) was slowly added dropwise at -78°C. The reaction was carried out for 1h. 3-bromo-5-(bromomethyl)benzofuran (9.00g, 26.38mmol, 1eq) was dissolved in tetrahydrofuran (30mL) and slowly added dropwise to the reaction mixture. The reaction mixture was slowly warmed to room temperature and stirred for 12h. The reaction mixture was added to saturated ammonium chloride, extracted with ethyl acetate, and the organic phases were combined. The mixture was washed with saturated sodium chloride, dried over sodium sulfate, filtered, and concentrated to give the target compound (12.00g). The crude compound was used directly in the next step without further purification. LCMS(ESI)[M+Na]⁺=619.0.

### Step 7: Synthesis of (S)-3-(3-bromobenzofuran-5-yl)-2-((R)-1-(tert-butoxycarbonyl)pyrrolidin-3-yl)propanoic acid

*Tert*-butyl (R)-3-((S)-1-((S)-4-benzyl-2-oxooxazolidin-3-yl)-3-(3-bromobenzofuran-5-yl)-1-oxopropan-2-yl)pyrrolidine-1-carboxylate (11.00g, 18.41mmol, 1eq) was dissolved in tetrahydrofuran (300mL), lithium hydroxide (440mg, 18.41mmol, 1eq) was dissolved in water (100mL) and hydrogen peroxide (11mL). The above were added to the reaction mixture at 0°C, and the reaction was carried out at room temperature for 2h. The reaction was quenched with sodium sulfite solution, extracted with ethyl acetate, washed with saturated sodium chloride, dried over sodium sulfate, filtered, and concentrated to give the target compound (5.50g). The crude compound was used directly in the next step without further purification. LCMS(ESI)[M-Boc+H]⁺=339.0.

### Step 8: Synthesis of tert-butyl (R)-3-((S)-3-(3-bromobenzofuran-5-yl)-1-(tert-butoxy)-1-oxopropan-2-yl)pyrrolidine-1-carboxylate

(S)-3-(3-bromobenzofuran-5-yl)-2-((R)-1-(tert-butoxycarbonyl)pyrrolidin-3-yl)propanoic acid (5.50g, 12.55mmol, 1.0eq) and *tert*-butyl N,N'-diisopropylcarbamimidate (7.54g, 37.64mmol, 3.0eq) were dissolved in 2-methyltetrahydrofuran (50mL) and heated to 80°C for 3h. The reaction mixture was extracted with ethyl acetate and water, the organic phase was washed with saturated brine, dried over sodium sulfate, concentrated, mixed, and purified by column chromatography to give the target compound (2.50g). LCMS(ESI)[M+Na]⁺=516.2, 518.2.

### Step 9: Synthesis of tert-butyl (R)-3-((S)-1-(tert-butoxy)-3-(3-formylbenzofuran-5-yl)-1-oxopropan-2-yl)pyrrolidine-1-carboxylate

In a high-pressure reaction vessel, *tert*-butyl (R)-3-((S)-3-(3-bromobenzofuran-5-yl)-1-(tert-butoxy)-1-oxopropan-2-yl)pyrrolidine-1-carboxylate (1.5g, 3.03mmol, 1.0eq), palladium acetate (68mg, 0.30mmol, 0.1eq), [2-(dimethylamino)ethyl]dimethylamine (704mg, 6.06mmol, 2.0eq), and bis(adamantan-1-yl)(butyl)phosphine (217mg, 0.61mmol, 0.2eq) were added in toluene (15mL), and H₂/CO (v/v=1/1) was introduced until the pressure reached 1.3 MPa. The reaction was carried out at 100°C for 18h. The reaction mixture was extracted with ethyl acetate and water, washed with saturated sodium chloride solution, dried over sodium sulfate, and purified by normal phase separation (silica gel, petroleum ether:ethyl acetate=6:1, v/v) to give the target compound (700mg). ¹HNMR(400MHz, CDCl₃) δ 10.09 (s, 1H), 8.18 (d, *J*=4.1Hz, 1H), 7.94 (d, *J*=6.0Hz, 1H), 7.38 (d, *J*=8.5Hz, 1H), 7.15 (t, *J*=7.2Hz, 1H), 3.71-3.34 (m, 2H), 3.23-3.13 (m, 1H), 3.02-2.77 (m, 3H), 2.45 (d, *J*=8.6Hz, 1H), 2.39-2.28 (m, 1H), 1.92-1.84 (m, 1H), 1.57 (d, *J*=13.9Hz, 1H), 1.40 (s, 9H), 1.21 (s, 9H).

### Tert-butyl (R)-3-((S)-1-(tert-butoxy)-3-(3-fluoro-5-formylphenyl)-1-oxopropan-2-yl)pyrrolidine-1-carboxylate (Intermediate 3-5)

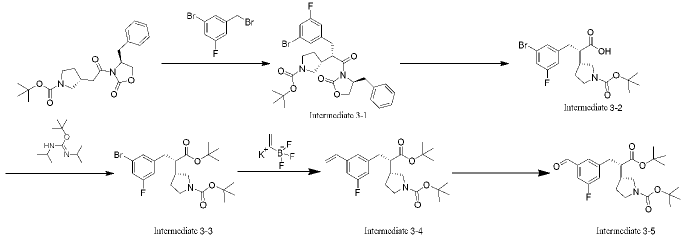

### Synthesis of tert-butyl (R)-3-((S)-1-((S)-4-benzyl-2-oxooxazolidin-3-yl)-3-(3-bromo-5-fluorophenyl)-1-oxopropan-2-yl)pyrrolidine-1-carboxylate (Intermediate 3-1)

The target product (21.9g) was synthesized from 1-bromo-3-(bromomethyl)-5-fluorobenzene as the starting material by referring to the method of Intermediate 1-2. LCMS(ESI)[M-*tert-*butyl+H]⁺=521.2.

### Synthesis of (S)-3-(3-bromo-5-fluorophenyl)-2-((R)-1-(tert-butoxycarbonyl)pyrrolidin-3-yl)propanoic acid (Intermediate 3-2)

The target product (8.8g) was synthesized by referring to the method of Intermediate 1-3. LCMS(ESI)[M -*tert*-butyl+H]⁺=359.9.

### Synthesis of tert-butyl (R)-3-((S)-3-(3-bromo-5-fluorophenyl)-1-(tert-butoxy)-1-oxopropan-2-yl)pyrrolidine-1-carboxylate (Intermediate 3-3)

The target product (6.1g) was synthesized by referring to the method of Intermediate 1-4. LCMS(ESI)[M -2×*tert*-butyl+H]⁺=359.9.

### Synthesis of tert-butyl (R)-3-((S)-1-(tert-butoxy)-3-(3-fluoro-5-vinylphenyl)-1-oxopropan-2-yl)pyrrolidine-1-carboxylate (Intermediate 3-4)

In a three-necked flask, *tert*-butyl (R)-3-((S)-3-(3-bromo-5-fluorophenyl)-1-(tert-butoxy)-1-oxopropan-2-yl)pyrrolidine-1-carboxylate (5.6g) was dissolved in 1,4-dioxane (45mL). Potassium vinyltrifluoroborate (8g), [1,1'-bis(diphenylphosphino)ferrocene]palladium(II) dichloride dichloromethane complex (922mg), and triethylamine (3.6g) were added in turn. Water (15mL) was added. The mixture was heated to 75°C under nitrogen protection and stirred for 5h. LCMS showed that the reaction was completed. The insoluble materials were filtered off through diatomaceous earth, the filter cake was washed with an appropriate amount of ethyl acetate, and the filtrate was concentrated. The filtrate was separated and purified by rapid chromatography (silica gel, PE:EA=10:1, v/v) to give the target compound (4.4g). LCMS(ESI)[M-2×*tert-*butyl+H]⁺=308.3.

### Synthesis of tert-butyl (R)-3-((S)-1-(tert-butoxy)-3-(3-fluoro-5-formylphenyl)-1-oxopropan-2-yl)pyrrolidine-1-carboxylate (Intermediate 3-5)

In a three-necked flask, *tert*-butyl (R)-3-((S)-1-(tert-butoxy)-3-(3-fluoro-5-vinylphenyl)-1-oxopropan-2-yl)pyrrolidine-1-carboxylate (4.4g, 10.53mmol, 1eq) was dissolved in tetrahydrofuran (44mL) and water (12mL). The mixture was under nitrogen protection and then potassium osmate (388mg, 1.05mmol, 0.1eq) and sodium periodate (6.76g, 31.59mmol, 3eq) were added in turn under ice bath. The reaction mixture was warmed to room temperature and stirred for 3h. The insoluble materials were filtered off, the filtrate was poured into water, and then extracted with ethyl acetate. The organic phases were combined, washed with saturated NaCl aqueous solution, dried over anhydrous sodium sulfate, concentrated under reduced pressure, and purified by rapid chromatography (silica gel, PE:EA=10:1, v/v) to give the target compound (2.7g). LCMS(ESI)[M-2×*tert*-butyl+H]⁺=310.0. ¹HNMR(400MHz, DMSO-*d₆*) δ 9.97 (d, J=1.5Hz, 1H), 7.63 (s, 1H), 7.46 (d, J=9.8Hz, 1H), 3.52 (q, J=10.5Hz, 1H), 3.42-3.34 (m, 1H), 3.24-3.09 (m, 1H), 3.02 (t, J=10.0Hz, 1H), 2.83 (q, J=13.9Hz, 2H), 2.59 (d, J=8.9Hz, 1H), 2.30 (s, 1H), 1.91-1.76 (m, 1H), 1.68-1.49 (m, 1H), 1.40 (s, 9H), 1.21 (s, 9H).

### Synthesis of tert-butyl (R)-3-((S)-1-(tert-butoxy)-3-(5-formylpyridin-3-yl)-1-oxopropan-2-yl)pyrrolidine-1-carboxylate (Intermediate 4-1)

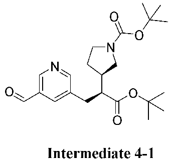

The target compound (320mg) was synthesized from 3-bromo-5-(bromomethyl)pyridine instead of 1-bromo-3-(bromomethyl)benzene by referring to the method of Intermediate 1-5. LCMS(ESI)[M+H]⁺=405.2; ¹HNMR(400MHz, DMSO-*d₆*) δ 10.11 (s, 1H), 8.95 (d, *J*=1.8Hz, 1H), 8.71 (d, *J*=1.9Hz, 1H), 8.09 (s, 1H), 3.59-3.47 (m, 2H), 3.21-3.12 (m, 1H), 3.05 (t, *J*=10.0Hz, 1H), 2.96-2.88 (m, 1H), 2.87-2.79 (m, 1H), 2.65-2.56 (m, 1H), 2.39-2.27 (m, 1H), 1.90-1.81 (m, 1H), 1.64-1.54 (m, 1H), 1.40 (s, 9H), 1.19 (s, 9H).

### Example 1 (Compound 5)

### Preparation of (25,2'S)-3,3'-(((((S)-5-amino-5-carboxypentyl)azanediyl)bis(methylene))bis(5-fluoro-3,1-phenylene))bis(2-((R)-pyrrolidin-3-yl)propanoic acid)

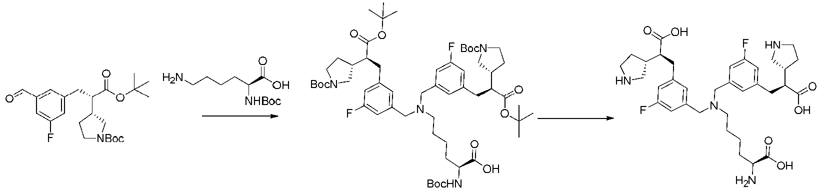

### Step 1: Synthesis of N⁶,N⁶-bis(3-((S)-3-(tert-butoxy)-2-((R)-1-(tert-butoxycarbonyl)pyrrolidin-3-yl)-3-oxopropyl)-5-fluorobenzyl)-N2-(tert-butoxycarbonyl)-L-lysine

*Tert*-butyl (R)-3-((S)-1-(tert-butoxy)-3-(3-fluoro-5-formylphenyl)-1-oxopropan-2-yl)pyrrolidine-1-carboxylate (376mg, 0.893mmol, 2.2eq) and (tert-butoxycarbonyl)-L-lysine (100mg, 0.406mmol, 1.0eq) were dissolved in tetrahydrofuran (2mL), and sodium cyanoborohydride (64mg, 1.02mmol, 2.5eq) and a drop of acetic acid were added. The reaction mixture was stirred at room temperature overnight. A saturated ammonium chloride solution and dichloromethane were added. The organic solvent was evaporated under reduced pressure and the residue was purified by rapid chromatography (Silica gel, DCM:MeOH=15:1, volume ratio) to obtain the target compound (89mg). LCMS(ESI)[M+H]⁺=1057.9.

### Step 2: Synthesis of (2S,2'S)-3,3'-(((((S)-5-amino-5-carboxypentyl)azanediyl)bis(methylene))bis(5-fluoro-3,1-phenylene))bis(2-((R)-pyrrolidin-3-yl)propanoic acid)

N⁶,N⁶-bis(3-((S)-3-(tert-butoxy)-2-((R)-1-(tert-butoxycarbonyl)pyrrolidin-3-yl)-3-oxopropyl)-5-fluorobenzyl)-N2-(tert-butoxycarbonyl)-L-lysine (89mg, 0.084mmol, 1.0eq) was dissolved in 1,4-dioxane (0.2mL), and a solution of 4 M HCl (1,4-dioxane) (0.2mL) was added. The reaction mixture was stirred at room temperature overnight. LCMS monitoring showed that the reaction was complete. The solvent was evaporated under reduced pressure, and the residue was purified by preparative-HPLC (C₁₈, 10mmol/L NH₄HCO₃ in water/acetonitrile) to obtain the target compound (9.8mg). LCMS(ESI)[M+H]⁺=645.4; ¹HNMR(400MHz, D₂O) δ 6.97-6.86 (m, 6H), 3.78 (s, 4H), 3.64-3.57 (m, 1H), 3.51-3.42 (m, 2H), 3.40-3.31 (m, 2H), 3.23-3.13 (m, 2H), 2.97-2.87 (m, 2H), 2.77-2.68 (m, 4H), 2.66-2.53 (m, 2H), 2.45-2.33 (m, 4H), 2.10-2.00 (m, 2H), 1.81-1.53 (m, 6H), 1.32-1.17 (m, 2H).

### Example 2 (Compound 6)

### Preparation of (2S,2'S)-3,3'-(((((5-((S)-2-carboxy-2-((R)-pyrrolidin-3-yl)ethyl)benzofuran-3-yl)methyl)azanediyl)bis(methylene))bis(3,1-phenylene))bis(2-((R)-pyrrolidin-3-yl)propanoic acid)

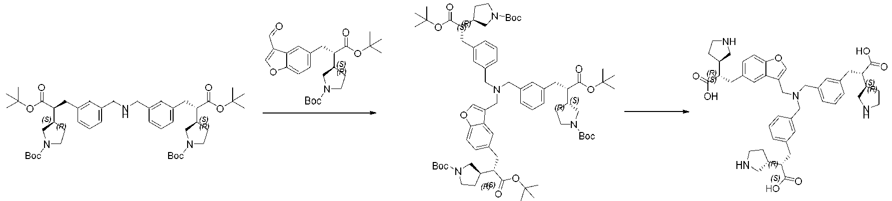

### Step 1: Synthesis of di-tert-butyl 3,3'-((2S,2'S)-(((((5-((S)-3-(tert-butoxy)-2-((R)-1-(tert-butoxycarbonyl)pyrrolidin-3-yl)-3-oxopropyl)benzofuran-3-yl)methyl)azanediyl)bis(methylene))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (50mg, 0.063mmol, 1.0eq) and *tert*-butyl (R)-3-((S)-1-(tert-butoxy)-3-(3-formylbenzofuran-5-yl)-1-oxopropan-2-yl)pyrrolidine-1-carboxylate (33mg, 0.076mmol, 1.2eq) were dissolved in tetrahydrofuran (0.2mL), and sodium cyanoborohydride (6mg, 0.095mmol, 1.5eq) and one drop of acetic acid were added. The reaction mixture was stirred at room temperature overnight. A saturated ammonium chloride solution and dichloromethane were added. The organic phase was concentrated under reduced pressure and purified by rapid chromatography (Silica gel, DCM:MeOH=15:1, volume ratio) to obtain the target compound (45mg).

### Step 2: Synthesis of (2S,2'S)-3,3'-(((((5-((S)-2-carboxy-2-((R)-pyrrolidin-3-yl)ethyl)benzofuran-3-yl)methyl)azanediyl)bis(methylene))bis(3,1-phenylene))bis(2-((R)-pyrrolidin-3-yl)propanoic acid)

Di-*tert*-butyl 3,3'-((2S,2'S)-(((((5-((S)-3-(tert-butoxy)-2-((R)-1-(tert-butoxycarbonyl)pyrrolidin-3-yl)-3-oxopropyl)benzofuran-3-yl)methyl)azanediyl)bis(methylene))bis(3,1-phenylene))bis(3-(tert-butoxy)-3-oxopropane-1,2-diyl))(3R,3'R)-bis(pyrrolidine-1-carboxylate) (45mg, 0.037mmol, 1.0eq) was dissolved in 1,4-dioxane (0.2mL), 4 M HCl (1,4-dioxane) solution (0.2mL) was added, and the reaction mixture was stirred at room temperature overnight. The solvent was evaporated under reduced pressure, and the residue was purified by preparative-HPLC (C₁₃, water/acetonitrile solution of 10mmol/L NH₄HCO₃) to obtain the target compound (13mg). LCMS(ESI)[M+H]⁺=751.5, ¹HNMR(400MHz, D₂O) δ 7.45 (s, 1H), 7.30 (d, *J*=8.4Hz, 1H), 7.18 (t, *J*=7.5Hz, 2H), 7.06 (d, *J*=9.1Hz, 4H), 7.01 (d, *J*=11.7Hz, 4H), 3.53-3.44 (m, 3H), 3.32-3.18 (m, 7H), 3.12-3.05 (m, 3H), 2.83-2.67 (m, 7H), 2.65-2.54 (m, 3H), 2.38-2.27 (m, 7H), 2.03-1.96 (m, 3H), 1.68-1.60 (m, 3H).

### Example 3 (Compound 12)

### Preparation of (2S,2'S)-3,3'-((((3-((S)-2-carboxy-2-((R)-pyrrolidin-3-yl)ethyl)-5-fluorobenzoyl)azanediyl)bis(methylene))bis(3,1-phenylene))bis(2-((R)-pyrrolidin-3-yl)propanoic acid)

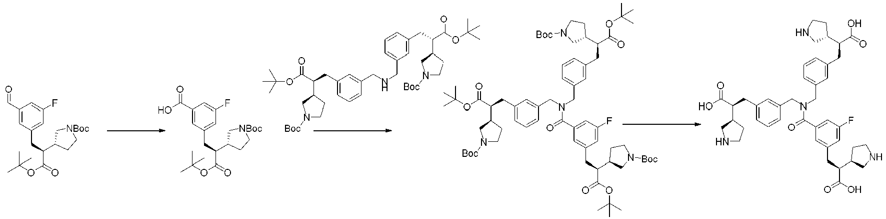

### Step 1: Synthesis of 3-((S)-3-(tert-butoxy)-2-((R)-1-(tert-butoxycarbonyl)pyrrolidin-3-yl)-3-oxopropyl)-5-fluorobenzoic acid

*Tert*-butyl (R)-3-((S)-1-(tert-butoxy)-3-(3-fluoro-5-formylphenyl)-1-oxopropan-2-yl)pyrrolidine-1-carboxylate (50mg), sodium hydroxide (8mg) and potassium permanganate (30mg) were dissolved in acetonitrile (0.5mL) and water (0.5mL). The reaction mixture was stirred at 60°C overnight. Water and dichloromethane were added. The organic phase was concentrated under reduced pressure and purified by rapid chromatography (Silica gel, DCM:MeOH=30:1, volume ratio) to obtain the target compound (15mg). LCMS(ESI)[M-Boc+H]⁺=338.49.

### Step 2: Synthesis of di-tert-butyl 3,3'-((2S,2'S)-((((3-((S)-3-(tert-butoxy)-2-((R)-1-(tert-butoxycarbonyl)pyrrolidin-3-yl)-3-oxopropyl)-5-fluorobenzoyl)azanediyl)bis(methylene))bis(3,1-phenylene))bis(3-(tert-butoxy)-3-oxopropane-1,2-diyl))(3R,3'R)-bis(pyrrolidine-1-carboxylate)

3-((S)-3-(tert-butoxy)-2-((R)-1-(tert-butoxycarbonyl)pyrrolidin-3-yl)-3-oxopropyl)-5-fluorobenzoic acid (15mg) was dissolved in N,N-dimethylformamide (0.3mL), and di-tert-butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (27mg), O-(7-azabenzotriazol-1-yl)-N,N,N',N'-tetramethyluronium hexafluorophosphate (15mg), and N,N-diisopropylethylamine (14mg) were added. The reaction mixture was stirred at room temperature overnight. Water and ethyl acetate were added. The organic phase was concentrated under reduced pressure and purified by rapid chromatography (Silica gel, DCM:MeOH=30:1, volume ratio) to obtain the target compound (20mg). LCMS(ESI)[M-Boc+H]⁺=1111.92.

### Step 3: Synthesis of (2S,2'S)-3,3'-((((3-((S)-2-carboxy-2-((R)-pyrrolidin-3-yl)ethyl)-5-fluorobenzoyl)azanediyl)bis(methylene))bis(3,1-phenylene))bis(2-((R)-pyrrolidin-3-yl)propanoic acid)

Di-*tert*-butyl 3,3'-((2S,2'S)-((((3-((S)-3-(tert-butoxy)-2-((R)-1-(tert-butoxycarbonyl)pyrrolidin-3-yl)-3-oxopropyl)-5-fluorobenzoyl)azanediyl)bis(methylene))bis(3,1-phenylene))bis(3-(tert-butoxy)-3-oxopropane-1,2-diyl))(3R,3'R)-bis(pyrrolidine-1-carboxylate) (115mg) was dissolved in 1,4-dioxane (0.2mL), 4 M HCl (1,4-dioxane) solution (0.2mL) was added, and the reaction mixture was stirred at room temperature overnight. The solvent was evaporated under reduced pressure, and purified by preparative-HPLC (C_{18,} 10mmol/L NH₄HCO₃ in water/acetonitrile) to obtain the target compound (39.9mg). LCMS(ESI)[M+H]⁺=743.65; ¹HNMR(400MHz, D₂O) δ 7.30-7.18 (m, 2H), 7.16-6.93 (m, 7H), 6.92-6.81 (m, 2H), 4.60 (s, 2H), 4.41 (s, 2H), 3.42-3.23 (m, 6H), 3.20-3.06 (m, 3H), 2.88-2.60 (m, 9H), 2.45-2.24 (m, 6H), 2.11-1.93 (m, 3H), 1.75-1.53 (m, 3H).

### Example 4 (Compound 47)

### Preparation of (2S,2'S,2"S)-3,3',3"-((nitrilotris(methylene))tris(benzofuran-3,5-diyl))tris(2-((R)-pyrrolidin-3-yl)propanoic acid)

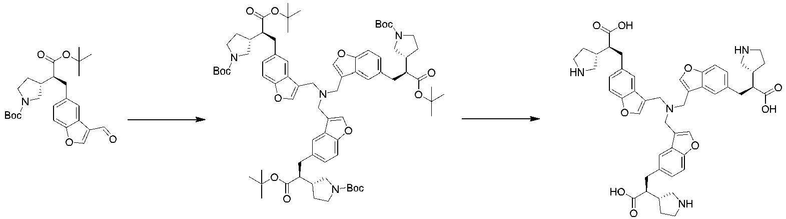

Step 1: Synthesis of tri-*tert*-butyl 3,3',3"-((2S,2'S,2"S)-((nitrilotris(methylene))tris(benzofuran-3,5-diyl))tris(3-(tert-butoxy)-3-oxopropane-1,2-diyl))(3R,3'R,3"R)-tris(pyrrolidine-1-carboxylate) *Tert*-butyl (R)-3-((S)-1-(tert-butoxy)-3-(3-formylbenzofuran-5-yl)-1-oxopropan-2-yl)pyrrolidine-1-carboxylate (600mg, 1.38mmol, 1eq) was dissolved in isopropanol (6mL), ammonia methanol solution (1.0eq, 7.0M) and sodium triacetoxyborohydride (436mg, 2.10mmol, 1.5eq) were added, and the reaction was carried out at 25°C for 16h. The reaction mixture was concentrated and purified by silica gel column chromatography (Silica gel, petroleum ether/ethyl acetate=1/1, volume ratio) to obtain the target compound (30mg). LCMS(ESI)[M+H]⁺=1322.6.

### Step 2: Synthesis of (2S,2'S,2"S)-3,3',3"-((nitrilotris(methylene))tris(benzofuran-3,5-diyl))tris(2-((R)-pyrrolidin-3-yl)propanoic acid)

Tri-*tert*-butyl 3,3',3"-((2S,2'S,2"S)-((nitrilotris(methylene))tris(benzofuran-3,5-diyl))tris(3-(tert-butoxy)-3-oxopropane-1,2-diyl))(3R,3'R,3"R)-tris(pyrrolidine-1-carboxylate) (35mg, 0.03mmol, 1eq) was dissolved in hydrogen chloride dioxane solution (1mL, 4M) and the mixture was stirred at room temperature for 1h. The reaction mixture was concentrated, water was added, and the mixture was lyophilized to obtain the target compound (15.88mg). LCMS(ESI)[M+H]⁺=831.4; ¹HNMR(400MHz, CD₃OD) δ 8.10 (s, 3H), 7.56-7.47 (m, 6H), 7.27 (d, *J*=8.4Hz, 3H), 4.64 (s, 6H), 3.52-3.35 (m, 6H), 3.26-3.19 (m, 3H), 3.08-2.93 (m, 12H), 2.52 (d, *J*=7.6Hz, 3H), 2.16 (d,*J*=7.2Hz, 3H), 1.91-1.73 (m, 3H).

### Example 5 (Compound 89)

### Preparation of (2S,2'S)-3,3'-((((3-fluoro-5-methoxyphenethyl)azanediyl)bis(methylene))bis(3,1-phenylene))bis(2-((R)-pyrrolidin-3-yl)propanoic acid)

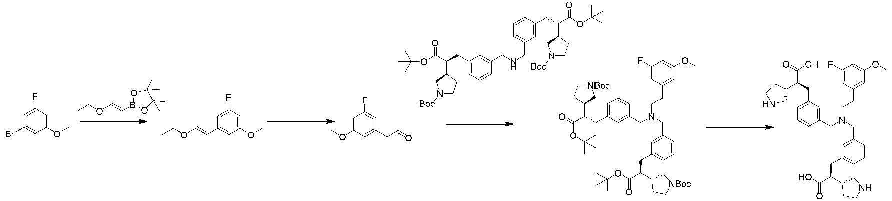

### Step 1: Synthesis of (E)-1-(2-ethoxyvinyl)-3-fluoro-5-methoxybenzene

Under nitrogen, in a three-necked flask, [1,1'-bis(diphenylphosphino)ferrocene]palladium(II) dichloride dichloromethane complex (1.14g), cesium carbonate (29.32g), 1,4-dioxane (180mL), and water (30mL) were added. 1-bromo-3-fluoro-5-methoxybenzene (6.15g) and (E)-2-(2-ethoxyvinyl)-4,4,5,5-tetramethyl-1,3,2-dioxaborolane (6.54g) were added in turn; the reaction mixture was heated to 100°C and reacted for 3h. After the reaction was completed, the reaction mixture was quenched with saturated saline solution and extracted with ethyl acetate. After combining the organic phases, the organic phase was washed with saturated saline solution, dried over anhydrous sodium sulfate, concentrated, and separated by column chromatography (Silica gel, petroleum ether:ethyl acetate=3: 1, volume ratio) to obtain the target compound (3.60g).

### Step 2: Synthesis of 2-(3-fluoro-5-methoxyphenyl)acetaldehyde

(E)-1-(2-ethoxyvinyl)-3-fluoro-5-methoxybenzene (500mg) was dissolved in HCl/dioxane (5mL) and the mixture was stirred at room temperature for 16h. LCMS showed that the reaction was completed. The reaction mixture was concentrated directly and purified by rapid chromatography (Silica gel, petroleum ether:ethyl acetate=10:1, volume ratio) to obtain the target compound (150mg). LCMS(ESI)[M+H]⁺=169.07.

### Step 3: Synthesis of di-tert-butyl 3,3'-((2S,2'S)-((((3-fluoro-5-methoxyphenethyl)azanediyl)bis(methylene))bis(3,1-phenylene))bis(3-(tert-butoxy)-3-oxopropane-1,2-diyl))(3R,3'R)-bis(pyrrolidine-1-carboxylate)

2-(3-fluoro-5-methoxyphenyl)acetaldehyde (47mg) was dissolved in methanol (5mL), and di-*tert-*butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (200mg) and two drops of acetic acid were added at room temperature. The mixture was stirred at room temperature for 16h. The reaction mixture was directly concentrated and purified by rapid chromatography (Silica gel, petroleum ether:ethyl acetate=5:1, volume ratio) to obtain the target compound (120mg). LCMS(ESI)[M+H]⁺=944.89.

### Step 4: Synthesis of (2S,2'S)-3,3'-((((3-fluoro-5-methoxyphenethyl)azanediyl)bis(methylene))bis(3,1-phenylene))bis(2-((R)-pyrrolidin-3-yl)propanoic acid)

### Di-tert-butyl 3,3'-((2S,2'S)-((((3-fluoro-5-methoxyphenethyl)azanediyl)bis(methylene))bis(3,1-phenylene))bis(3-(tert-butoxy)-3-oxopropane-1,2-diyl))(3R,3'R)-bis(pyrrolidine-1-carboxylate)

(120mg) was dissolved in HCl/dioxane (5mL) and the mixture was stirred at room temperature for 16h. The reaction mixture was concentrated directly and separated and purified by preparative-HPLC (C_{18,} 0.1%FA in water/acetonitrile) to obtain the target compound (47.46mg). LCMS(ESI)[M+H]⁺=632.4.

### Example 6 (Compound 62)

### Preparation of (25,2'S)-3,3'-((((3-fluoro-5-methoxybenzyl)azanediyl)bis(ethane-2,1-diyl))bis(1H-indole-1,6-diyl))bis(2-((R)-pyrrolidin-3-yl)propanoic acid)

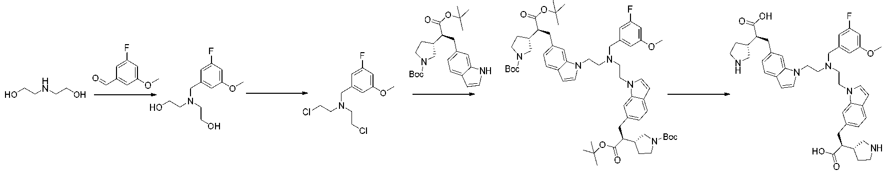

### Step 1: Synthesis of 2,2'-((3-fluoro-5-methoxybenzyl)azanediyl)bis(ethan-1-ol)

2,2'-azanediylbis(ethan-1-ol) (2.00g) was added in dichloromethane (30mL), and 3-fluoro-5-methoxybenzaldehyde (4.00g) and sodium triacetoxyborohydride (12.00g) were added at 25°C. The reaction was carried out at 25°C for 2h, and the reaction was monitored by LCMS. The reaction mixture was extracted with dichloromethane and water. After the organic phases were concentrated under reduced pressure, the residue was purified (by reversed-phase column chromatography:acetonitrile/water=10%) to obtain the target compound (4.00g). LCMS(ESI)[M+H]⁺=244.2.

### Step 2: Synthesis of 2-chloro-N-(2-chloroethyl)-N-(3-fluoro-5-methoxybenzyl)ethan-1-amine

2,2'-((3-fluoro-5-methoxybenzyl)azanediyl)bis(ethan-1-ol) (1.50g) was added to thionyl chloride (15mL) and the reaction was carried out at 70°C overnight. Compounds were detected by LCMS. The thionyl chloride was concentrated under reduced pressure. The residue was triturated with ethyl acetate. The mixture was extracted with aqueous ammonium chloride solution without further purification to obtain the target compound (1.8g, crude product) which was used in the next step without further purification.

### Step 3: Synthesis of di-tert-butyl 3,3'-((2S,2'S)-((((3-fluoro-5-methoxybenzyl)azanediyl)bis(ethane-2,1-diyl))bis(1H-indole-1,6-diyl))bis(3-(tert-butoxy)-3-oxopropane-1,2-diyl))(3R,3'R)-bis(pyrrolidine-1-carboxylate)

*Tert*-butyl (R)-3-((S)-1-(tert-butoxy)-3-(1H-indol-6-yl)-1-oxopropan-2-yl)pyrrolidine-1-carboxylate (0.5g) was added in N,N-dimethylformamide (5mL) and 2-chloro-N-(2-chloroethyl)-N-(3-fluoro-5-methoxybenzyl)ethan-1-amine (338mg) and cesium carbonate (1.2g) were added at room temperature. The reaction mixture was stirred at 100°C under nitrogen overnight. After the reaction mixture was detected by LCMS, saturated saline solution was added. The reaction mixture was extracted with ethyl acetate and dried over anhydrous sodium sulfate. After the organic phases were concentrated under reduced pressure, the residue was washed by silica gel column chromatography (pure ethyl acetate) to obtain the target compound (190mg). LCMS(ESI)[M+H]⁺=1036.6.

### Step 4: Synthesis of (2S,2'S)-3,3'-((((3-fluoro-5-methoxybenzyl)azanediyl)bis(ethane-2,1-diyl))bis(1H-indole-1,6-diyl))bis(2-((R)-pyrrolidin-3-yl)propanoic acid)

Di-*tert*-butyl 3,3'-((2S,2'S)-((((3-fluoro-5-methoxybenzyl)azanediyl)bis(ethane-2,1-diyl))bis(1H-indole-1,6-diyl))bis(3-(tert-butoxy)-3-oxopropane-1,2-diyl))(3R,3'R)-bis(pyrrolidine-1-carboxylate) (140mg) was added in TFA (2mL) and the mixture was stirred at 25°C for 0.5h. LCMS detected the desired product. The reaction mixture was concentrated under reduced pressure. The residue was dissolved in acetonitrile and purified by preparative-HPLC (FA) to obtain the target compound (13.6mg). LCMS(ESI)[M+H]⁺=725.04; ¹HNMR(400MHz, MeOD-d4) δ 8.42 (s, 1H), 7.42 (d, *J*=8.0Hz, 2H), 7.11 (d, *J*=3.2Hz, 2H), 6.91 (d, *J*=7.2Hz, 2H), 6.87-6.84 (m, 2H), 6.45-6.43 (m, 3H), 6.29 (t, *J*=2.8Hz, 2H), 4.07 (d, *J*=6.2Hz, 4H), 3.63 (d, *J*=3.2Hz, 3H), 3.59 (s, 2H), 3.21-3.18 (m, 4H), 3.13-3.03 (m, 6H), 2.99-2.92 (m, 2H), 2.87 (s, 2H), 2.78-2.66 (m, 2H), 2.60-2.54 (m, 2H), 2.38-2.30 (m, 2H), 2.01 (d, *J*=24.0Hz, 2H), 1.85-1.76 (m, 1H), 1.56 (s, 1H).

### Example 7 (Compound 95)

### Preparation of (2S,2'S)-3,3'-((((2-(3-((S)-2-carboxy-2-((R)-pyrrolidin-3-yl)ethyl)phenoxy)acetyl)azanediyl)bis(methylene))bis(3,1-phenylene))bis(2-((R)-pyrrolidin-3-yl)propanoic acid)

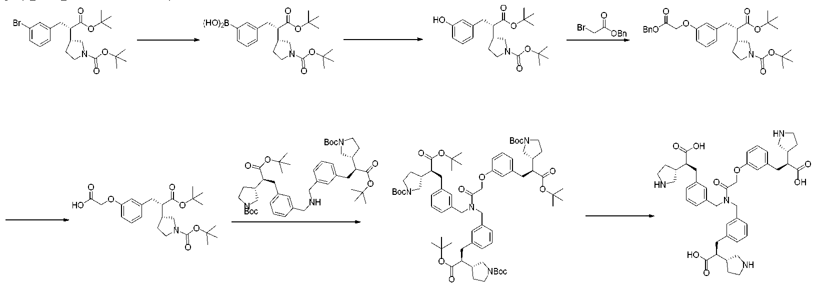

### Step 1: Synthesis of (3-((S)-3-(tert-butoxy)-2-((R)-1-(tert-butoxycarbonyl)pyrrolidin-3-yl)-3-oxopropyl)phenyl)boronic acid

*Tert*-butyl (R)-3-((S)-3-(3-bromophenyl)-1-(tert-butoxy)-1-oxopropan-2-yl)pyrrolidine-1-carboxylate (1.9g, 4.18mmol, 1eq), (dihydroxyboranyl)boronic acid (562.29mg, 6.27mmol, 1.5eq), potassium acetate (1230.7mg, 12.54mmol, 3eq), 2-(dicyclohexylphosphino)-2',4',6'-tri-i-propyl-1,1'-biphenyl (199.27mg, 0.42mmol, 0.1eq) and X-Phos Pd G₂ (164mg, 0.21mmol, 0.05eq) were added to EtOH (20mL). The reaction mixture was purged with nitrogen three times, and reacted at 90°C for 16h; The product was detected by LCMS, ethyl acetate (20mL) was added to the reaction system. The mixture was filtered through diatomaceous earth, and the filtrate was evaporated under reduced pressure to obtain the target compound (2.0g). The crude product was not further purified and directly used for the next step. LCMS[M-2×*tert*-butyl+H]⁺=308.2.

### Step 2: Synthesis of tert-butyl (R)-3-((S)-1-(tert-butoxy)-3-(3-hydroxyphenyl)-1-oxopropan-2-yl)pyrrolidine-1-carboxylate

(3-((S)-3-(tert-butoxy)-2-((R)-1-(tert-butoxycarbonyl)pyrrolidin-3-yl)-3-oxopropyl)phenyl)boronic acid (2000mg, 4.77mmol, 1eq) was dissolved in THF (20mL), and hydrogen peroxide (8mL, 235.2mmol, 49.31eq) wad added, and the reaction mixture was stirred at 20°C for 16h. LCMS showed the desired product. A saturated solution of sodium bisulfite (20mL) and ethyl acetate (20mL) were added, and the mixture was extracted. The organic phase was dried over sodium sulfate, concentrated, and purified by rapid chromatography (Silica gel, PE:EtOAc=4:1, v/v) to obtain the target compound (800mg). LCMS(ESI)[M+H]⁺=392.2.

### Step 3: Synthesis of tert-butyl (R)-3-((S)-3-(3-(2-(benzyloxy)-2-oxoethoxy)phenyl)-1-(tert-butoxy)-1-oxopropan-2-yl)pyrrolidine-1-carboxylate

*Tert*-butyl (R)-3-((S)-1-(tert-butoxy)-3-(3-hydroxyphenyl)-1-oxopropan-2-yl)pyrrolidine-1-carboxylate (2.0g, 5.11mmol, 1eq), benzyl 2-bromoacetate (2.34g, 10.22mmol, 2eq) and potassium carbonate (2.12g, 15.33mmol, 3eq) were added to acetonitrile (50mL), and the mixture was reacted at 90°C for 16h; LCMS showed the desired product. Rapid chromatography (Silica gel, PE:EtOAc=5:1, volume ratio) was used to obtain the target compound (2.5g). LCMS(ESI)[M+Na]⁺=561.9.

### Step 4: Synthesis of 2-(3-((S)-3-(tert-butoxy)-2-((R)-1-(tert-butoxycarbonyl)pyrrolidin-3-yl)-3-oxopropyl)phenoxy)acetic acid

*Tert*-butyl (R)-3-((S)-3-(3-(2-(benzyloxy)-2-oxoethoxy)phenyl)-1-(tert-butoxy)-1-oxopropan-2-yl)pyrrolidine-1-carboxylate (2.5g, 4.63mmol, 1eq) was dissolved in methanol (50mL), followed by adding Pd/C (300mg, 10%), and the mixture was purged with hydrogen for three times. The reaction mixture was stirred at room temperature for 16h. LCMS showed the reaction was completed. The mixture was filtered, and the filtrate was concentrated to obtain the target compound (1.5g). LCMS(ESI)[M+Na]⁺=472.2.

### Step 5: Synthesis of di-tert-butyl 3,3'-((2S,2'S)-((((2-(3-((S)-3-(tert-butoxy)-2-((R)-1-(tert-butoxycarbonyl)pyrrolidin-3-yl)-3-oxopropyl)phenoxy)acetyl)azanediyl)bis(methylene))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (88.1mg, 0.11mmol, 1eq), 2-(3-((S)-3-(tert-butoxy)-2-((R)-1-(tert-butoxycarbonyl)pyrrolidin-3-yl)-3-oxopropyl)phenoxy)acetic acid (50mg, 0.11mmol, 1eq), O-(7-azabenzotriazol-1-yl)-N,N,N',N'-tetramethyluronium hexafluorophosphate (41.83mg, 0.11mmol, 1eq) and N,N-diisopropylethylamine (43.12mg, 0.33mmol, 3eq) were dissolved in N,N-dimethylformamide (3mL); the mixture was stirred at room temperature for 16h, and LCMS showed that the reaction was completed; the reaction solvent was evaporated, and the residue was purified by thin-layer silica gel plate (DCM:MeOH=20: 1, volume ratio) to obtain the target compound (100mg). LCMS(ESI)[M+Na]⁺=1245.8.

### Step 6: Synthesis of (2S,2'S)-3,3'-((((2-(3-((S)-2-carboxy-2-((R)-pyrrolidin-3-yl)ethyl)phenoxy)acetyl)azanediyl)bis(methylene))bis(3,1-phenylene))bis(2-((R)-pyrrolidin-3-yl)propanoic acid)

Di-*tert*-butyl 3,3'-((2S,2'S)-((((2-(3-((S)-3-(tert-butoxy)-2-((R)-1-(tert-butoxycarbonyl)pyrrolidin-3-yl)-3-oxopropyl)phenoxy)acetyl)azanediyl)bis(methylene))bis(3,1-phenylene))bis(3-(tert-butoxy)-3-oxopropane-1,2-diyl))(3R,3'R)-bis(pyrrolidine-1-carboxylate) (100mg, 0.08mmol, 1eq) was added to hydrochloric acid-dioxane (2mL, 4.0M) and reacted at room temperature for 6h. The reaction mixture was filtered and the filter cake was washed with dioxane to obtain the target compound (46mg). LCMS(ESI)[M+H]⁺=755.2. ¹HNMR(400MHz, CD₃OD) δ 7.34-7.20 (m, 5H), 7.18-7.10 (m, 4H), 6.92-6.85 (m, 2H), 6.7-6.75 (m, 1H), 4.92 (s, 2H), 4.61 (s, 4H), 3.57 -3.40 (m, 6H), 3.34-3.21 (m, 3H), 2.99-2.81 (m, 11H), 2.78-2.72 (m, 3H), 2.56-2.49 (m, 3H), 2.29-2.15 (m, 3H), 1.87-1.81 (m, 3H).

### Example 8 (Compound 96)

### Preparation of (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid

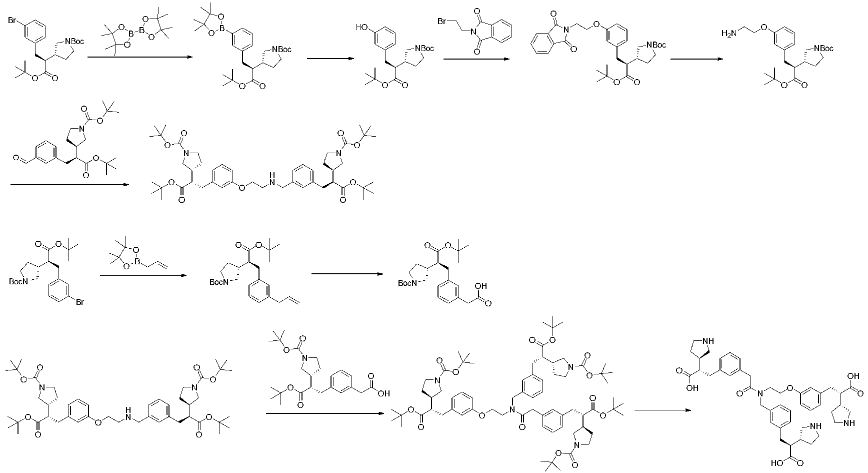

### Step 1: Preparation of tert-butyl (R)-3-((S)-1-(tert-butoxy)-1-oxo-3-(3-(4,4,5,5-tetramethyl-1,3,2-dioxaborolan-2-yl)phenyl)propan-2-yl)pyrrolidine-1-carboxylate

*Tert*-butyl (R)-3-((S)-3-(3-bromophenyl)-1-(tert-butoxy)-1-oxopropan-2-yl)pyrrolidine-1-carboxylate (1.5g, 3.30mmol, 1.0eq) was dissolved in 1,4-dioxane (20mL) in a single-necked flask, followed by adding [1,1'-bis(diphenylphosphino)ferrocene]palladium(II) dichloride dichloromethane complex (270.0mg, 0.33mmol, 0.1eq), bis(pinacolato)diboron (1.26g, 4.95mmol, 1.5eq) and potassium acetate (972mg, 9.9mmol, 3eq); the mixture was stirred at 90°C for 16h under nitrogen. LCMS detection showed that the reaction was completed, and the reaction mixture was directly concentrated, separated and purified by rapid chromatography (Silica gel, PE:EtOAc=2:1) to obtain the target compound (1.2g). LCMS(ESI)[M-Boc+H]⁺=402.2.

### Step 2: Preparation of tert-butyl (R)-3-((S)-1-(tert-butoxy)-3-(3-hydroxyphenyl)-1-oxopropan-2-yl)pyrrolidine-1-carboxylate

In a single-neck bottle, the product from the first step (1.2g, 2.39mmol, 1.0eq) was dissolved in tetrahydrofuran (20mL). Subsequently hydrogen peroxide (0.5mL) was added, and the mixture was stirred at room temperature for 16h. LCMS detection showed that the reaction was completed, and the reaction mixture was directly concentrated, separated and purified by rapid chromatography (Silica gel, PE:EtOAc=1:1) to obtain the target compound (800.0mg). LCMS(ESI)[M-2×*tert*-butyl+H]⁺=280.0.

### Step 3: Preparation of tert-butyl (R)-3-((S)-1-(tert-butoxy)-3-(3-(2-(1,3-dioxoisoindolin-2-yl)ethoxy)phenyl)-1-oxopropan-2-yl)pyrrolidine-1-carboxylate

In a single-neck bottle, the product from the second step (500.0mg, 1.28mmol, 1.0eq) was dissolved in N,N-dimethylformamide (5mL), followed by adding 2-(2-bromoethyl)isoindoline-1,3-dione (647.7mg, 2.56mmol, 2eq), potassium carbonate (530.7mg, 3.83mmol, 3eq). The mixture was stirred at 90°C for 2h. LCMS detection showed that the reaction was completed, and the reaction mixture was directly concentrated, separated and purified by rapid chromatography (Silica gel, PE:EtOAc=1:1) to obtain the target compound (280mg). LCMS(ESI)[M+H]⁺=565.3.

### Step 4: Preparation of tert-butyl (R)-3-((S)-3-(3-(2-aminoethoxy)phenyl)-1-(tert-butoxy)-1-oxopropan-2-yl)pyrrolidine-1-carboxylate

In a single-neck bottle, the product from the third step (280.0mg, 0.5mmol, 1.0eq) was dissolved in ethanol (10mL), followed by adding hydrazine hydrate (124mg, 2.50mmol, 5.0eq). The mixture was reacted at 90°C for 16h. LCMS detection showed that the reaction was completed, and the reaction mixture was directly concentrated, separated and purified by rapid chromatography (Silica gel, PE:EtOAc=1:5) to obtain the target compound (160mg). LCMS(ESI)[M+H]⁺=435.3.

### Step 5: Preparation of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

In a single-neck bottle, the product from the fourth step (160mg, 0.37mmol, 1.0eq) was dissolved in methanol (10mL). Subsequently, *tert*-butyl (R)-3-((S)-1-(tert-butoxy)-3-(3-formylphenyl)-1-oxopropan-2-yl)pyrrolidine-1-carboxylate (178.1mg, 0.44mmol, 1.2eq) was added, followed by addition of sodium cyanoborohydride (23.2mg, 0.37mmol, 1.0eq). The mixture was stirred at room temperature for 16h. LCMS detection showed that the reaction was completed, and the reaction mixture was directly concentrated, separated and purified by rapid chromatography (Silica gel, PE:EtOAc=1:5) to obtain the target compound (233mg). LCMS(ESI)[M+H]⁺=822.6.

### Step 6: Preparation of tert-butyl (R)-3-((S)-3-(3-allylphenyl)-1-(tert-butoxy)-1-oxopropan-2-yl)pyrrolidine-1-carboxylate

*Tert*-butyl (R)-3-((S)-3-(3-bromophenyl)-1-(tert-butoxy)-1-oxopropan-2-yl)pyrrolidine-1-carboxylate (7.0g, 15.4mmol, 1.0eq) was dissolved in 1,4-dioxane (20mL) and water (4mL) in a single-necked flask, followed by adding 2-allyl-4,4,5,5-tetramethyl-1,3,2-dioxaborolane (5.2g, 30.8mmol, 2eq), potassium phosphate (9.8g, 46.2mmol, 3eq) and [1,1'-bis(diphenylphosphino)ferrocene]palladium(II) dichloride dichloromethane complex (1.25g, 1.54mmol, 0.1eq). The mixture was degassed with nitrogen for 3 times and stirred at 90°C for 16h. LCMS detection showed that the reaction was completed, and the reaction mixture was directly concentrated, separated and purified by rapid chromatography (Silica gel, PE:EtOAc=2:1) to obtain the target compound (5.0g). LCMS[M-2×*tert*-butyl+H]⁺=304.1.

### Step 7: Preparation of 2-(3-((S)-3-(tert-butoxy)-2-((R)-1-(tert-butoxycarbonyl)pyrrolidin-3-yl)-3-oxopropyl)phenyl)acetic acid

In a single-neck bottle, the product from the sixth step (6.0g, 14.4mmol, 1.0eq) was dissolved in 1,4-dioxane (100mL), followed by adding sodium periodate (12.3g, 57.75mmol, 4eq) and potassium osmate dihydrate (532.0mg, 1.44mmol, 0.1eq). The mixture was stirred at room temperature for 16h. LCMS detection showed that the reaction was completed, and the reaction mixture was directly concentrated, separated and purified by rapid chromatography (Silica gel, PE:EtOAc=1:1) to obtain the target compound (500mg). LCMS(ESI)[M+H]⁺=434.2.

### Step 8: Preparation of tert-butyl (R)-3-((S)-1-(tert-butoxy)-3-(3-(N-(2-(3-((S)-3-(tert-butoxy)-2-((R)-1-(tert-butoxycarbonyl)pyrrolidin-3-yl)-3-oxopropyl)phenoxy)ethyl)-2-(3-((S)-3-(tert-butoxy)-2-((R)-1-(tert-butoxycarbonyl)pyrrolidin-3-yl)-3-oxopropyl)phenyl)acetamido)methyl)phenyl)-1-oxopropan-2-yl)pyrrolidine-1-carboxylate

In a single-neck bottle, the product from the fifth step (70mg, 0.0852mmol, 1.0eq) was dissolved in DMF (5mL), followed by adding the product obtained in the seventh step (44.3mg, 0.102mmol, 1.2eq), O-(7-azabenzotriazol-1-yl)-N,N,N',N'-tetramethyluronium hexafluorophosphate (64.7mg, 0.17mmol, 2.0eq) and *N,N*-diisopropylethylamine (33.0mg, 0.25mmol, 3.0eq). The mixture was stirred at room temperature for 16h, and LCMS showed the reaction was complete. The reaction mixture was directly concentrated, separated and purified by rapid chromatography (Silica gel, PE:EtOAc=1:8) to obtain the target compound (70mg). LCMS(ESI)[M+H]⁺=1237.9.

### Step 9: Preparation of (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid

In a single-neck bottle, the product from the eighth step (70.0mg, 0.056mmol) was dissolved in hydrochloric acid-dioxane (2mL), and stirred at room temperature for 16h. LCMS showed the reaction was completed. The reaction mixture was directly concentrated, separated and purified by preparative chromatography to obtain the target compound (27.2mg). LCMS(ESI)[M+H]⁺=769.4. ¹HNMR(400MHz, D₂O) δ 7.20 (dq, *J*=30.0, 7.5Hz, 3H), 7.11-6.86 (m, 6H), 6.81 (s, 1H), 6.69-6.59 (m, 2H), 4.59 (d, *J*=4.6Hz, 2H), 4.10 (t, *J*=5.0Hz, 1H), 4.00 (d, *J*=5.2Hz, 1H), 3.88 (s, 1H), 3.79 (d, *J*=5.2Hz, 1H), 3.69 (d, *J*=5.2Hz, 2H), 3.29 (dtt, *J*=13.1, 9.3, 4.6Hz, 6H), 3.12 (dd, *J*=11.9, 7.9Hz, 3H), 2.87-2.58 (m, 8H), 2.54-2.48 (m, 1H), 2.41-2.23 (m, 6H), 2.01 (s, 3H), 1.63 (dt, *J*=22.4, 11.3Hz, 3H).

### Example 9 (Compound 8)

### Preparation of 3,3'-(((((4-(2-carboxy-2-((R)-pyrrolidin-3-yl)ethyl)benzofuran-2-yl)methyl)azanediyl)bis(methylene))bis(3,1-phenylene))bis(2-((R)-pyrrolidin-3-yl)propanoic acid)

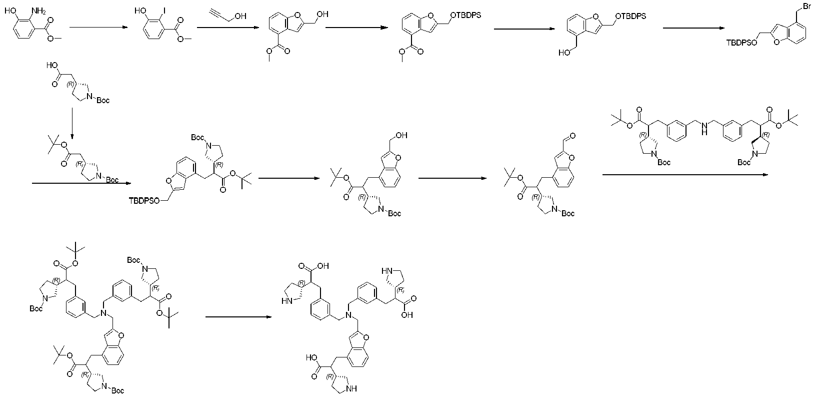

### Step 1: Synthesis of methyl 3-hydroxy-2-iodobenzoate

Methyl 2-amino-3-hydroxybenzoate (25g, 149.65mmol, 1eq) was dissolved in an aqueous solution of sulfuric acid (1000mL, 1.0M) and cooled to 0°C. An aqueous solution (100mL) of sodium nitrite (11.25g, 163.0mmol, 1.08eq) was added and the mixture was stirred at 25°C for 20 min. An aqueous solution (100mL) of potassium iodide (111.7g, 672.9mmol, 4.5eq) was added and the mixture was stirred at 70°C for 1.5h. LCMS detection showed that the reaction was completed. Water and ethyl acetate was added to the reaction mixture, and the mixture was extracted. The organic phase was washed with saturated sodium chloride, dried over anhydrous sodium sulfate, filtered, and concentrated to obtain a crude product. The crude product was separated and purified by rapid chromatography (Silica gel, petroleum ether:ethyl acetate=5:1) to obtain the target compound (20.0g). LCMS(ESI)[M+H]⁺=278.9.

### Step 2: Synthesis of methyl 2-(hydroxymethyl)benzofuran-4-carboxylate

The product obtained in the first step (20.0g, 71.94mmol, 1.0eq) was dissolved in N,N-dimethylformamide (200mL), followed by adding cuprous iodide (1.37g, 7.19mmol, 0.1eq), bis(triphenylphosphine)palladium(II) dichloride (5g, 7.19mmol, 0.1eq), triethylamine (21.86g, 214.55mmol, 3.0eq), and propargyl alcohol (6.1g, 108.81mmol, 1.5eq). The reaction mixture was stirred at 75°C for 16h under nitrogen. LCMS detection showed that the reaction was completed. Water and ethyl acetate were added to the reaction mixture and the mixture was extracted. The organic phase was washed with saturated saline, dried over anhydrous sodium sulfate, filtered, and concentrated to obtain a crude product. The crude product was separated and purified by rapid chromatography (Silica gel, petroleum ether:ethyl acetate=10:1) to obtain the target compound (11g). LCMS(ESI)[M+H]⁺=207.4.

### Step 3: Synthesis of methyl 2-(((tert-butyldiphenylsilyl)oxy)methyl)benzofuran-4-carboxylate

The product obtained in the second step (11.0g, 53.38mmol, 1.0eq) was dissolved in dichloromethane (80mL), followed by adding imidazole (4.5g, 66.09mmol, 1.5eq) and *tert-*butyl(chloro)diphenylsilane (13.0g, 47.30mmol, 0.8eq); the mixture was stirred at 25°C for 16h. LCMS detection showed that the reaction was completed. The reaction mixture was filtered, and the filtrate was directly evaporated under reduced pressure. The residue was separated and purified by rapid chromatography (Silica gel, petroleum ether:ethyl acetate=10:1) to obtain the target compound (12.2g). LCMS(ESI)[M+Na]⁺=467.4.

### Step 4: Synthesis of (2-(((tert-butyldiphenylsilyl)oxy)methyl)benzofuran-4-yl)methanol

The product obtained in the third step (12.2g, 27.47mmol, 1eq) was dissolved in tetrahydrofuran (100mL), lithium borohydride (1.79g, 82.41mmol, 3.0eq) was added at 0°C, and the mixture was stirred at 25°C for 16h. LCMS detection showed that the reaction was completed. Water and ethyl acetate were added, and the mixture was extracted. The organic phase was washed with saturated saline, dried over anhydrous sodium sulfate, filtered, concentrated and mixed. The residue was separated and purified by rapid chromatography (Silica gel, petroleum ether:ethyl acetate=2:1) to obtain the target compound (7.64g). LCMS(ESI)[M+Na]⁺=439.4.

### Step 5: Synthesis of ((4-(bromomethyl)benzofuran-2-yl)methoxy)(tert-butyl)diphenylsilane

The product of the fourth step (2.0g, 4.81mmol, 1.0eq) was added to dichloromethane (20mL), phosphorus tribromide (1.95g, 7.21mmol, 1.5eq) was added at 0°C, and the mixture was stirred for 2h. TLC detection showed that the reaction was completed. Saturated ammonium chloride aqueous solution was added to quench the reaction, and the reaction mixture was extracted with ethyl acetate. The organic phase was dried over anhydrous sodium sulfate, filtered and concentrated to obtain a crude product. The crude product was separated and purified by rapid chromatography (Silica gel, petroleum ether:ethyl acetate=100:1) to obtain the target compound (1.42g).

### Step 6: Synthesis of tert-butyl (R)-3-(2-(tert-butoxy)-2-oxoethyl)pyrrolidine-1-carboxylate

(R)-2-(1-(tert-butoxycarbonyl)pyrrolidin-3-yl)acetic acid (25.0g, 109.11mmol, 1.0eq) was dissolved in dichloromethane (200mL), N,N'-dicyclohexylcarbodiimide (22.5g, 109.11mmol, 1.0eq), tert-butanol (24.25mL, 327.33mmol, 3.0eq) and 4-dimethylaminopyridine (13.3g, 109.11mmol, 1.0eq) were added at 25°C, and the mixture was stirred for 16h after the addition. LCMS detection showed that the reaction was completed. Water and ethyl acetate were added, and the mixture was extracted. The organic phase was dried over anhydrous sodium sulfate, filtered, and concentrated to obtain a crude product. The crude product was separated and purified by rapid chromatography (Silica gel, petroleum ether:ethyl acetate=10:1) to obtain the target compound (24.0g).

### Step 7: Synthesis of tert-butyl (3R)-3-(1-(tert-butoxy)-3-(2-(((tert-butyldiphenylsilyl)oxy)methyl)benzofuran-4-yl)-1-oxopropan-2-yl)pyrrolidine-1-carboxylate

The product obtained in the sixth step (905mg) was dissolved in tetrahydrofuran (10mL), and lithium bis(trimethylsilyl)amide (4.8mL) was added slowly dropwise at 0°C, and the mixture was stirred at 0°C for 30min; the product from the fifth step (1.42g) was dissolved in tetrahydrofuran (10mL) and slowly added dropwise to the previous reaction mixture; the mixture was stirred at 25°C for 3h. LCMS showed that the reaction was completed. Saturated ammonium chloride aqueous solution was added to quench the reaction, and the reaction mixture was extracted with ethyl acetate. The organic phase was dried over anhydrous sodium sulfate, filtered and concentrated, separated and purified by rapid chromatography (Silica gel, petroleum ether:ethyl acetate=3:1) to obtain the target compound (1.67g). LCMS(ESI)[M+H]⁺=684.2.

### Step 8: Synthesis of tert-butyl (3R)-3-(1-(tert-butoxy)-3-(2-(hydroxymethyl)benzofuran-4-yl)-1-oxopropan-2-yl)pyrrolidine-1-carboxylate

*Tert*-butyl (3R)-3-(1-(tert-butoxy)-3-(2-(((*tert*-butyldiphenylsilyl)oxy)methyl)benzofuran-4-yl)-1-oxopropan-2-yl)pyrrolidine-1-carboxylate (1.67g, 2.44mmol, 1.0eq) was dissolved in tetrahydrofuran (10mL), and tetra-n-butylammonium fluoride (3.18g, 12.2mmol, 5.0eq) was added at 25°C. The mixture was stirred at room temperature for 2h. LCMS showed the reaction was completed. The mixture was filtered, and the organic phase was evaporated under reduced pressure to obtain the target compound (1.8g). LCMS(ESI)[M+Na]⁺=468.4.

### Step 9: Synthesis of tert-butyl (3R)-3-(1-(tert-butoxy)-3-(2-formylbenzofuran-4-yl)-1-oxopropan-2-yl)pyrrolidine-1-carboxylate

The product obtained in the eighth step (1.8g, 4.04mmol, 1.0eq) was dissolved in dichloromethane (10mL), and manganese dioxide (1.75g, 20.2mmol, 5.0eq) was added at 25°C; the reaction mixture was stirred at 60°C for 2h. LCMS showed the reaction was completed. After filtration, the organic phase was concentrated, separated and purified by rapid chromatography (Silica gel, petroleum ether:ethyl acetate=5:1) to obtain the target compound (894g). LCMS(ESI)[M+Na]⁺=476.4.

### Step 10: Synthesis of di-tert-butyl 3,3'-((((((4-(3-(tert-butoxy)-2-((R)-1-(tert-butoxycarbonyl)pyrrolidin-3-yl)-3-oxopropyl)benzofuran-2-yl)methyl)azanediyl)bis(methylene))bis(3,1-phenylene))bis(3-(tert-butoxy)-3-oxopropane-1,2-diyl))(3R,3'R)-bis(pyrrolidine-1-carboxylate)

The product from the ninth step (100mg, 0.23mmol, 1.0eq) was dissolved in methanol (5mL), di-*tert*-butyl 3,3'-(((azanediylbis(methylene))bis(3,1-phenylene))bis(3-(tert-butoxy)-3-oxopropane-1,2-diyl))(3R,3'R)-bis(pyrrolidine-1-carboxylate) (197.8mg, 0.25mmol, 1.1eq) was added and stirred at 25°C for 30min. Sodium triacetoxyborohydride (146.2mg, 0.69mmol, 3.0eq) was added and the reaction was continued for 2h. LCMS detection showed that the reaction was completed. Water and ethyl acetate were added, and the mixture was extracted. The organic phase was dried over anhydrous sodium sulfate, filtered, and concentrated to obtain a crude product, which was purified by silica gel column chromatography (petroleum ether:ethyl acetate=1:1) to obtain the target compound (65mg). LCMS(ESI)[M+H]⁺=1220.1.

### Step 11: Synthesis of 3,3'-(((((4-(2-carboxy-2-((R)-pyrrolidin-3-yl)ethyl)benzofuran-2-yl)methyl)azanediyl)bis(methylene))bis(3,1-phenylene))bis(2-((R)-pyrrolidin-3-yl)propanoic acid)

The product from the tenth step (65mg) was dissolved in hydrochloric acid/1,4-dioxane (1mL) and stirred at 25°C for 2h. LCMS detection showed the reaction was completed. After concentration, the crude product was obtained. The target compound (4mg) was obtained by Prep-HPLC. LCMS(ESI)[M+H]⁺=751.5; ¹HNMR(400MHz, MeOD-*d4*)δ 7.36-7.30 (m, 3H), 7.26-7.21 (m, 4H), 7.18-7.09 (m, 4H), 6.85(s, 1H), 3.81- 3.71 (m, 2H), 3.67-3.60 (m, 4H), 3.26-2.96 (m, 14H), 2.81-2.64 (m, 5H), 2.51-2.42 (m, 2H), 2.42-2.30 (m, 3H), 2.10-2.01 (m, 3H), 1.84-1.57 (m, 3H).

### Example 10 (Compound 11)

### Preparation of (2S,2'S)-3,3'-(((((6-((S)-2-carboxy-2-((R)-pyrrolidin-3-yl)ethyl)benzo[b]thiophen-2-yl)methyl)azanediyl)bis(methylene))bis(3,1-phenylene))bis(2-((R)-pyrrolidin-3-yl)propanoic acid)

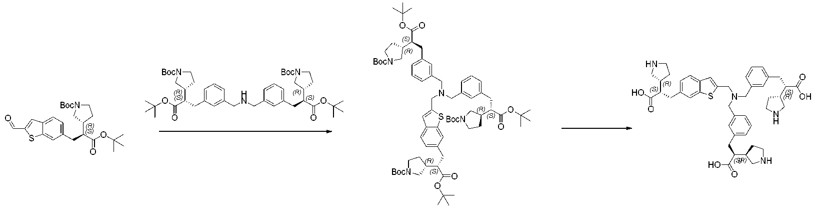

### Step 1: Synthesis of di-tert-butyl 3,3'-((2S,2'S)-(((((6-((S)-3-(tert-butoxy)-2-((R)-1-(tert-butoxycarbonyl)pyrrolidin-3-yl)-3-oxopropyl)benzo[b]thiophen-2-yl)methyl)azanediyl)bis(methylene))bis(3,1-phenylene))bis(3-(tert-butoxy)-3-oxopropane-1,2-diyl))(3R,3'R)-bis(pyrrolidine-1-carboxylate)

*Tert*-butyl (R)-3-((S)-1-(tert-butoxy)-3-(2-formylbenzo[b]thiophen-6-yl)-1-oxopropan-2-yl)pyrrolidine-1-carboxylate (86.7mg, 0.189mmol, 1.5eq) was dissolved in methanol (5mL), di-*tert*-butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (100mg, 0.126mmol, 1.0eq) and sodium cyanoborohydride (15.84mg, 0.252mmol, 2.0eq) were added at room temperature. The reaction mixture was stirred at room temperature for 36h. LCMS showed that the reaction was completed. The reaction mixture was directly concentrated, separated and purified by rapid chromatography (Silica gel, petroleum ether:ethyl acetate=1:8) to obtain the target compound (90g). LCMS(ESI)[M+H]⁺=1235.8.

### Step 2: Synthesis of (2S,2'S)-3,3'-(((((6-((S)-2-carboxy-2-((R)-pyrrolidin-3-yl)ethyl)benzo[b]thiophen-2-yl)methyl)azanediyl)bis(methylene))bis(3,1-phenylene))bis(2-((R)-pyrrolidin-3-yl)propanoic acid)

Di-*tert*-butyl 3,3'-((2S,2'S)-(((((6-((S)-3-(tert-butoxy)-2-((R)-1-(tert-butoxycarbonyl)pyrrolidin-3-yl)-3-oxopropyl)benzo[b]thiophen-2-yl)methyl)azanediyl)bis(methylene))bis(3,1-phenylene))bis(3-(tert-butoxy)-3-oxopropane-1,2-diyl))(3R,3'R)-bis(pyrrolidine-1-carboxylate) (90mg, 0.07mmol) was dissolved in hydrochloric acid/1,4-dioxane (2mL) and stirred at room temperature for 16h. LCMS showed the reaction was completed. The reaction mixture was directly concentrated, separated and purified by preparative chromatography to obtain the target compound (32.96mg). LCMS(ESI)[M+H]⁺=767.4; ¹HNMR(400MHz, D₂O) δ 7.78 (d, *J*=8.2Hz, 1H), 7.70 (s, 1H), 7.43 (s, 1H), 7.30 (dd, *J*=18.7, 7.5Hz, 5H), 7.17 (d, *J*=7.6Hz, 2H), 7.12 (s, 2H), 4.59 (s, 2H), 4.35 (s, 4H), 3.56 (ddd, *J*=16.4, 11.8, 7.9Hz, 3H), 3.43-3.34 (m, 3H), 3.27-3.16 (m, 3H), 3.08-2.90 (m, 5H), 2.86-2.69 (m, 5H), 2.64 (td, *J*=9.6, 5.0Hz, 2H), 2.51 (dq, *J=*17.4, 8.7Hz, 3H), 2.18-2.04 (m, 3H), 1.72 (ddd, *J*=18.5, 13.0, 9.2Hz, 3H).

### Example 11 (Compound 49)

### Preparation of (2S,2'S,2"S)-3,3',3"-((nitrilotris(methylene))tris(benzo[b]thiophene-3,5-diyl))tris(2-((R)-pyrrolidin-3-yl)propanoic acid)

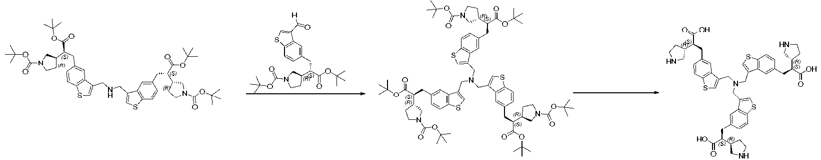

### Step 1: Synthesis of tri-tert-butyl 3,3',3"-((2S,2'S,2"S)-((nitrilotris(methylene))tris(benzo[b]thiophene-3,5-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((azanediylbis(methylene))bis(benzo[b]thiophene-3,5-diyl))bis(3-(tert-butoxy)-3-oxopropane-1,2-diyl))(3R,3'R)-bis(pyrrolidine-1-carboxylate) (40mg, 0.04mmol, 1.0eq) in tetrahydrofuran (5mL), followed by adding *tert*-butyl (R)-3-((S)-1-(tert-butoxy)-3-(3-formylbenzo[b]thiophen-5-yl)-1-oxopropan-2-yl)pyrrolidine-1-carboxylate (22.36mg, 0.05mmol, 1.1eq) and sodium cyanoborohydride (2.74mg, 0.04mmol, 1.0eq). The mixture was stirred at room temperature for 16h. LCMS showed that the reaction was completed. The reaction mixture was directly concentrated, separated and purified by rapid chromatography (Silica gel, petroleum ether:ethyl acetate=1:8) to obtain the target compound (40mg). LCMS(ESI)[M+H]⁺=1347.1.

### Step 2: Synthesis of (2S,2'S,2"S)-3,3',3"-((nitrilotris(methylene))tris(benzo[b]thiophene-3,5-diyl))tris(2-((R)-pyrrolidin-3-yl)propanoic acid)

Tri-*tert*-butyl 3,3',3"-((2S,2'S,2"S)-((nitrilotris(methylene))tris(benzo[b]thiophene-3,5-diyl))tris(3-(tert-butoxy)-3-oxopropane-1,2-diyl))(3R,3'R,3"R)-tris(pyrrolidine-1-carboxylate) (40mg, 0.03mmol) was dissolved in hydrochloric acid/1,4-dioxane (2mL) and stirred at room temperature for 16h. LCMS showed the reaction was completed. The reaction mixture was directly concentrated, separated and purified by preparative chromatography to obtain the target compound (22.36mg). LCMS(ESI)[M+H]⁺=879.0; ¹HNMR(400MHz, D₂O) δ 8.08-8.00 (m, 3H), 7.92 (d, *J*=8.3Hz, 3H), 7.23 (dd, *J*=8.4, 1.5Hz, 3H), 6.47 (s, 3H), 4.86 (d, *J*=13.7Hz, 3H), 4.75 (s, 3H), 3.37 (ddd, *J*=12.0, 10.0, 5.6Hz, 6H), 3.21-3.13 (m, 3H), 2.91 (t, *J=*11.0Hz, 3H), 2.53 (ddd, *J*=44.9, 11.3, 5.5Hz, 6H), 2.42-2.24 (m, 6H), 2.05 (dtd, *J*=13.6, 7.0, 3.3Hz, 3H), 1.65 (dq, *J*=13.2, 9.7Hz, 3H).

### Example 12 (Compound 65)

### Preparation of (2S,2'S,2"S)-3,3',3"-((nitrilotris(ethane-2,1-diyl))tris(benzene-3,1-diyl))tris(2-((R)-pyrrolidin-3-yl)propanoic acid)

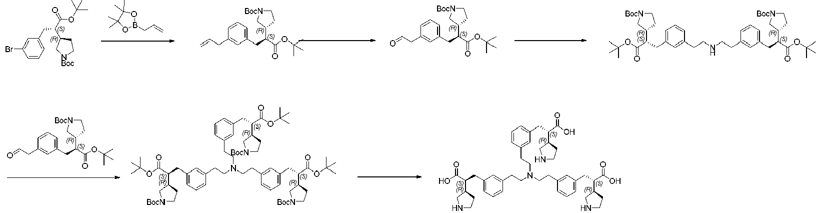

### Step 1: Synthesis of tert-butyl (R)-3-((S)-3-(3-allylphenyl)-1-(tert-butoxy)-1-oxopropan-2-yl)pyrrolidine-1-carboxylate

*Tert*-butyl (R)-3-((S)-3-(3-bromophenyl)-1-(tert-butoxy)-1-oxopropan-2-yl)pyrrolidine-1-carboxylate (1500mg, 3.3mmol, 1.0eq) was dissolved in 1,4-dioxane (20mL), followed by adding 2-allyl-4,4,5,5-tetramethyl-1,3,2-dioxaborolane (1.11g, 6.6mmol, 2eq) and potassium phosphate (2.1g, 9.9mmol, 3eq), and then water (4mL); the mixture was stirred at 90°C for 16h under nitrogen. LCMS showed that the reaction was completed. The reaction mixture was directly concentrated, separated and purified by rapid chromatography (Silica gel, petroleum ether:ethyl acetate=2:1) to obtain the target compound (900mg). LCMS(ESI)[M+H]⁺=416.3.

### Step 2: Synthesis of tert-butyl (R)-3-((S)-1-(tert-butoxy)-1-oxo-3-(3-(2-oxoethyl)phenyl)propan-2-yl)pyrrolidine-1-carboxylate

*Tert*-butyl (R)-3-((S)-3-(3-allylphenyl)-1-(tert-butoxy)-1-oxopropan-2-yl)pyrrolidine-1-carboxylate (900mg, 2.17mmol, 1.0eq) was dissolved in 1,4-dioxane (20mL), and sodium periodate (1.85g, 8.66mmol, 4eq) and potassium osmate dihydrate (80mg, 0.22mmol, 0.1eq) were added, which were stirred at room temperature for 16h. LCMS showed that the reaction was completed. The reaction mixture was directly concentrated, separated and purified by rapid chromatography (Silica gel, petroleum ether:ethyl acetate=1:1) to obtain the target compound (600mg). LCMS(ESI)[M+H]⁺=418.3.

### Step 3: Synthesis of di-tert-butyl 3,3'-((2S,2'S)-((azanediylbis(ethane-2,1-diyl))bis(3,1-phenylene))bis(3-(tert-butoxy)-3-oxopropane-1,2-diyl))(3R,3'R)-bis(pyrrolidine-1-carboxylate)

*Tert*-butyl (R)-3-((S)-1-(tert-butoxy)-1-oxo-3-(3-(2-oxoethyl)phenyl)propan-2-yl)pyrrolidine-1-carboxylate (400mg, 0.96mmol, 1.0eq) was dissolved in methanol (10mL), ammonia/methanol (16.31mg, 0.96mmol, 1.0eq), two drops of acetic acid and sodium cyanoborohydride (71mg, 1.15mmol, 1.2eq) were added, and the mixture was stirred at room temperature for 16h. LCMS showed that the reaction was completed. The reaction mixture was directly concentrated, separated and purified by rapid chromatography (Silica gel, petroleum ether:ethyl acetate=1:8) to obtain the target compound (100mg). LCMS(ESI)[M+H]⁺=820.6.

### Step 4: Synthesis of tri-tert-butyl 3,3',3"-((2S,2'S,2"S)-((nitrilotris(ethane-2,1-diyl))tris(benzene-3,1-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((azanediylbis(ethane-2,1-diyl))bis(3,1-phenylene))bis(3-(tert-butoxy)-3-oxopropane-1,2-diyl))(3R,3'R)-bis(pyrrolidine-1-carboxylate) (100mg, 0.12mmol, 1.0eq) was dissolved in methanol (5mL), followed by adding *tert*-butyl (R)-3-((S)-1-(tert-butoxy)-1-oxo-3-(3-(2-oxoethyl)phenyl)propan-2-yl)pyrrolidine-1-carboxylate (51mg, 0.12mmol, 1.0eq), two drops of acetic acid, and sodium cyanoborohydride (7.5mg, 0.12mmol, 1.0eq). The mixture was stirred at room temperature for 16h. LCMS showed that the reaction was completed. The reaction mixture was directly concentrated, separated and purified by rapid chromatography (Silica gel, petroleum ether:ethyl acetate=1:8) to obtain the target compound (80mg). LCMS(ESI)[M+H]⁺=1221.9.

### Step 5: Synthesis of (2S,2'S,2"S)-3,3',3"-((nitrilotris(ethane-2,1-diyl))tris(benzene-3,1-diyl))tris(2-((R)-pyrrolidin-3-yl)propanoic acid)

Tri-*tert*-butyl 3,3',3"-((2S,2'S,2"S)-((nitrilotris(ethane-2,1-diyl))tris(benzene-3,1-diyl))tris(3-(tert-butoxy)-3-oxopropane-1,2-diyl))(3R,3'R,3"R)-tris(pyrrolidine-1-carboxylate) (80mg) was dissolved in hydrochloric acid/1,4-dioxane (2mL) and stirred at room temperature for 16h. LCMS showed the reaction was completed. The reaction mixture was directly concentrated, separated and purified by preparative chromatography to obtain the target compound (9.7mg). LCMS(ESI)[M+H]⁺=753.7; ¹HNMR(400MHz, D₂O) δ 7.21 (t, J=7.5Hz, 3H), 7.09-6.99 (m, 9H), 3.40-3.30 (m, 6H), 3.14 (ddd, J=11.7, 9.9, 7.3Hz, 4H), 2.96 (d, J=7.5Hz, 4H), 2.85 (dd, J=11.7, 9.2Hz, 4H), 2.78 (d, J=9.1Hz, 7H), 2.72-2.64 (m, 5H), 2.39 (dq, J=14.9, 9.1, 6.8Hz, 6H), 2.04 (td, J=6.5, 3.6Hz, 3H), 1.71-1.63 (m, 3H).

### Example 13 (Compound 67)

### Preparation of (2S,2'S,2"S)-3,3',3"-((nitrilotris(ethane-2,1-diyl))tris(benzofuran-3,5-diyl))tris(2-((R)-pyrrolidin-3-yl)propanoic acid)

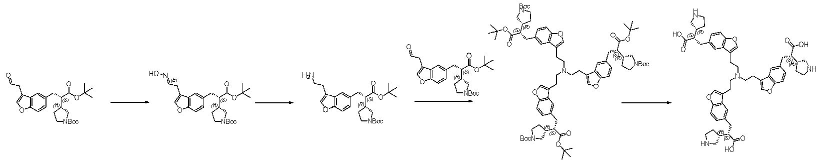

### Step 1: Synthesis of tert-butyl (R)-3-((S)-1-(tert-butoxy)-3-(3-((E)-2-(hydroxyimino)ethyl)benzofuran-5-yl)-1-oxopropan-2-yl)pyrrolidine-1-carboxylate

*Tert*-butyl (R)-3-((S)-1-(tert-butoxy)-1-oxo-3-(3-(2-oxoethyl)benzofuran-5-yl)propan-2-yl)pyrrolidine-1-carboxylate (120mg, 0.26mmol, 1.0eq) was dissolved in ethanol (10mL), and hydroxylamine hydrochloride (37mg, 0.52mmol, 2.0eq) was added; the mixture was stirred at room temperature for 16h; LCMS showed the reaction was completed. The reaction mixture was filtered, and the filtrate was concentrated, separated and purified by rapid chromatography (Silica gel, petroleum ether:ethyl acetate=1:8) to obtain the target compound (100mg). LCMS(ESI)[M+H]⁺=473.2.

### Step 2: Synthesis of tert-butyl (R)-3-((S)-3-(3-(2-aminoethyl)benzofuran-5-yl)-1-(tert-butoxy)-1-oxopropan-2-yl)pyrrolidine-1-carboxylate

*Tert*-butyl (R)-3-((S)-1-(tert-butoxy)-3-(3-((E)-2-(hydroxyimino)ethyl)benzofuran-5-yl)-1-oxopropan-2-yl)pyrrolidine-1-carboxylate (100mg, 0.21mmol, 1.0eq) was dissolved in methanol (10mL), and Pd/C (10mg) was added; the mixture was stirred at room temperature for 16h under a hydrogen. LCMS showed the reaction was completed. The reaction mixture was filtered and evaporated under reduced pressure. The residue was separated and purified by rapid chromatography (Silica gel, petroleum ether:ethyl acetate=1:8) to obtain the target compound (70mg). LCMS(ESI)[M+H]⁺=459.2.

### Step 3: Synthesis of tri-tert-butyl 3,3',3"-((2S,2'S,2"S)-((nitrilotris(ethane-2,1-diyl))tris(benzofuran-3,5-diyl))tris(3-(tert-butoxy)-3-oxopropane-1,2-diyl))(3R,3'R,3"R)-tris(pyrrolidine-1-carboxylate)

*Tert*-butyl (R)-3-((S)-3-(3-(2-aminoethyl)benzofuran-5-yl)-1-(tert-butoxy)-1-oxopropan-2-yl)pyrrolidine-1-carboxylate (100mg, 0.22mmol, 1.0eq) was dissolved in methanol (10mL), followed by adding *tert*-butyl (R)-3-((S)-1-(tert-butoxy)-1-oxo-3-(3-(2-oxoethyl)benzofuran-5-yl)propan-2-yl)pyrrolidine-1-carboxylate (200mg, 0.44mmol, 2.0eq) and sodium cyanoborohydride (27mg, 0.44mmol, 2.0eq); the mixture was stirred at room temperature for 16h. LCMS showed the reaction was completed. The reaction mixture was concentrated after cooling and separated and purified by rapid chromatography (Silica gel, petroleum ether: ethyl acetate=1:1) to obtain the target compound (100mg).

### Step 4: Synthesis of (2S,2'S,2"S)-3,3',3"-((nitrilotris(ethane-2,1-diyl))tris(benzofuran-3,5-diyl))tris(2-((R)-pyrrolidin-3-yl)propanoic acid)

Tri-*tert*-butyl 3,3',3"-((2S,2'S,2"S)-((nitrilotris(ethane-2,1-diyl))tris(benzofuran-3,5-diyl))tris(3-(tert-butoxy)-3-oxopropane-1,2-diyl))(3R,3'R,3"R)-tris(pyrrolidine-1-carboxylate) (100mg, 0.07mmol) was dissolved in hydrochloric acid/1,4-dioxane (4mL) and stirred at room temperature for 16h. LCMS showed the reaction was completed. The reaction mixture was directly concentrated, separated and purified by preparative chromatography to obtain the target compound (6.58mg). LCMS(ESI)[M+H]⁺=873.2; ¹HNMR(400MHz, D₂O) δ 7.26-7.14 (m, 6H), 6.94 (s, 6H), 3.17 (d, *J*=33.2Hz, 4H), 2.95 (d, *J*=26.8Hz, 5H), 2.61 (d, *J*=51.5Hz, 22H), 2.41-2.10 (m, 7H), 1.73 (dd, *J*=101.8, 31.7Hz, 3H), 1.08 (s, 1H).

### Example 14 (Compound 71)

### Preparation of (2S,2'S)-3,3'-((((2-(3-((S)-2-carboxy-2-((R)-pyrrolidin-3-yl)ethyl)phenoxy)ethyl)azanediyl)bis(ethane-2,1-diyl))bis(3,1-phenylene))bis(2-((R)-pyrrolidin-3-yl)propanoic acid)

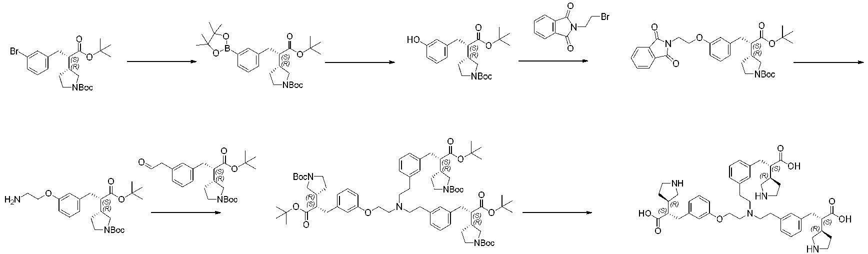

### Step 1: Synthesis of tert-butyl (R)-3-((S)-1-(tert-butoxy)-1-oxo-3-(3-(4,4,5,5-tetramethyl-1,3,2-dioxaborolan-2-yl)phenyl)propan-2-yl)pyrrolidine-1-carboxylate

*Tert*-butyl (R)-3-((S)-3-(3-bromophenyl)-1-(tert-butoxy)-1-oxopropan-2-yl)pyrrolidine-1-carboxylate (1.5g) was dissolved in 1,4-dioxane (20mL), followed by adding bis(pinacolato)diboron (1.75g), [1,1'-bis(diphenylphosphino)ferrocene]palladium(II) dichloride (241.4mg) and potassium acetate (972mg), and water (4mL); the mixture was stirred at 90°C for 16h under nitrogen. LCMS showed that the reaction was completed. The reaction mixture was directly concentrated, separated and purified by rapid chromatography (Silica gel, petroleum ether:ethyl acetate=2:1) to obtain the target compound (1.2g). LCMS(ESI)[M+H-Boc]⁺=402.2.

### Step 2: Synthesis of tert-butyl (R)-3-((S)-1-(tert-butoxy)-3-(3-hydroxyphenyl)-1-oxopropan-2-yl)pyrrolidine-1-carboxylate

*Tert*-butyl (R)-3-((S)-1-(tert-butoxy)-1-oxo-3-(3-(4,4,5,5-tetramethyl-1,3,2-dioxaborolan-2-yl)phenyl)propan-2-yl)pyrrolidine-1-carboxylate (1200mg) was dissolved in tetrahydrofuran (20mL), followed by adding hydrogen peroxide (0.5mL). The mixture was stirred at room temperature for 16h. LCMS showed that the reaction was completed. The reaction mixture was directly concentrated, separated and purified by rapid chromatography (Silica gel, petroleum ether:ethyl acetate=1:1) to obtain the target compound (800mg). LCMS(ESI)[M-2×tert-butyl]⁺=280.

### Step 3: Synthesis of tert-butyl (R)-3-((S)-1-(tert-butoxy)-3-(3-(2-(1,3-dioxoisoindolin-2-yl)ethoxy)phenyl)-1-oxopropan-2-yl)pyrrolidine-1-carboxylate

*Tert*-butyl (R)-3-((S)-1-(tert-butoxy)-3-(3-hydroxyphenyl)-1-oxopropan-2-yl)pyrrolidine-1-carboxylate (500mg) was dissolved in N,N-dimethylformamide (5mL), followed by adding 2-(2-bromoethyl)isoindoline-1,3-dione (649mg) and potassium carbonate (530mg). The mixture was stirred at 90°C for 2h. LCMS showed that the reaction was completed. The reaction mixture was directly concentrated, separated and purified by rapid chromatography (Silica gel, petroleum ether:ethyl acetate=1:1) to obtain the target compound (280mg). LCMS(ESI)[M+H]⁺=565.3.

### Step 4: Synthesis of tert-butyl (R)-3-((S)-3-(3-(2-aminoethoxy)phenyl)-1-(tert-butoxy)-1-oxopropan-2-yl)pyrrolidine-1-carboxylate

*Tert*-butyl (R)-3-((S)-1-(tert-butoxy)-3-(3-(2-(1,3-dioxoisoindolin-2-yl)ethoxy)phenyl)-1-oxopropan-2-yl)pyrrolidine-1-carboxylate (280mg) was dissolved in ethanol (10mL) and hydrazine hydrate (124mg) was added. The mixture was stirred at 90°C for 16h. LCMS showed that the reaction was completed. The reaction mixture was directly concentrated, separated and purified by rapid chromatography (Silica gel, petroleum ether:ethyl acetate=1:5) to obtain the target compound (160mg). LCMS(ESI)[M+H]⁺=435.3.

### Step 5: Synthesis of di-tert-butyl 3,3'-((2S,2'S)-((((2-(3-((S)-3-(tert-butoxy)-2-((R)-1-(tert-butoxycarbonyl)pyrrolidin-3-yl)-3-oxopropyl)phenoxy)ethyl)azanediyl)bis(ethane-2,1-diyl))bis(3,1-phenylene))bis(3-(tert-butoxy)-3-oxopropane-1,2-diyl))(3R,3'R)-bis(pyrrolidine-1-carboxylate)

*Tert*-butyl (R)-3-((S)-3-(3-(2-aminoethoxy)phenyl)-1-(tert-butoxy)-1-oxopropan-2-yl)pyrrolidine-1-carboxylate (80mg) was dissolved in methanol (10mL), followed by adding *tert-*butyl (R)-3-((S)-1-(tert-butoxy)-1-oxo-3-(3-(2-oxoethyl)phenyl)propan-2-yl)pyrrolidine-1-carboxylate (154mg) and sodium cyanoborohydride (23mg). The mixture was stirred at room temperature for 16h. LCMS showed that the reaction was completed. The reaction mixture was directly concentrated, separated and purified by rapid chromatography (Silica gel, petroleum ether:ethyl acetate=1:8) to obtain the target compound (100mg). LCMS(ESI)[M+H]⁺=1237.9.

### Step 6: Synthesis of (2S,2'S)-3,3'-((((2-(3-((S)-2-carboxy-2-((R)-pyrrolidin-3-yl)ethyl)phenoxy)ethyl)azanediyl)bis(ethane-2,1-diyl))bis(3,1-phenylene))bis(2-((R)-pyrrolidin-3-yl)propanoic acid)

Di-*tert*-butyl 3,3'-((2S,2'S)-((((2-(3-((S)-3-(tert-butoxy)-2-((R)-1-(tert-butoxycarbonyl)pyrrolidin-3-yl)-3-oxopropyl)phenoxy)ethyl)azanediyl)bis(ethane-2,1-diyl))bis(3,1-phenylene))bis(3-(tert-butoxy)-3-oxopropane-1,2-diyl))(3R,3'R)-bis(pyrrolidine-1-carboxylate) (100mg) was dissolved in hydrochloric acid/1,4-dioxane (2mL) and stirred at room temperature for 16h. LCMS showed the reaction was completed. The reaction mixture was directly concentrated, separated and purified by preparative chromatography to obtain the target compound (27.9mg). LCMS(ESI)[M+H]⁺=769.5; ¹HNMR(400MHz, D₂O) δ 7.24-7.09 (m, 4H), 7.00 (q, J=8.0Hz, 5H), 6.82 (d, J=7.4Hz, 1H), 6.73 (d, J=8.3Hz, 2H), 4.17-4.06 (m, 2H), 3.39-3.28 (m, 6H), 3.17-3.03 (m, 5H), 2.90 (d, J=9.4Hz, 3H), 2.86-2.58 (m, 14H), 2.37 (td, J=17.0, 15.4, 6.6Hz, 6H), 2.02 (s, 3H), 1.66 (dd, J=14.2, 6.5Hz, 3H).

### Example 15 (Compound 80)

### Preparation of (2S,2'S)-3,3'-((2,2'-((2-(3-((S)-2-carboxy-2-((R)-pyrrolidin-3-yl)ethyl)phenoxy)ethyl)azanediyl)bis(acetyl))bis(3,1-phenylene))bis(2-((R)-pyrrolidin-3-yl)propanoic acid)

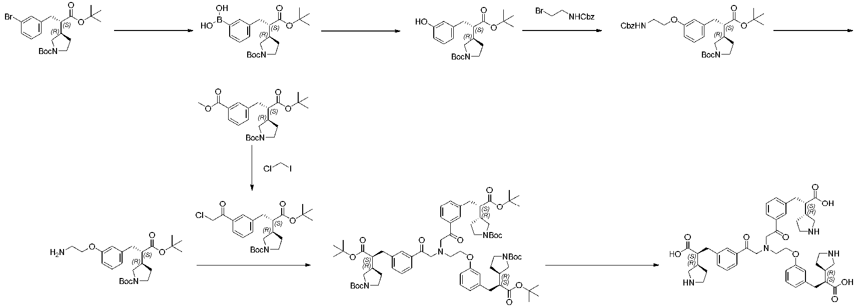

### Step 1: Synthesis of (3-((S)-3-(tert-butoxy)-2-((R)-1-(tert-butoxycarbonyl)pyrrolidin-3-yl)-3-oxopropyl)phenyl)boronic acid

*Tert*-butyl (R)-3-((S)-3-(3-bromophenyl)-1-(tert-butoxy)-1-oxopropan-2-yl)pyrrolidine-1-carboxylate (1900mg, 4.18mmol, 1eq), (dihydroxyboranyl)boronic acid (562.29mg, 6.27mmol, 1.5eq), potassium acetate (1230.7mg, 12.54mmol, 3eq), dicyclohexyl[2',4',6'-tri(propan-2-yl)-[1,1'-biphenyl]-2-yl]phosphine (199.27mg, 0.42mmol, 0.1eq) and XPhos Pd G2 (164mg, 0.21mmol, 0.05eq) were added in ethanol (20mL). The reaction mixture was purged with nitrogen three times and stirred at 90°C for 16h. LCMS showed the reaction was completed. After the addition of ethyl acetate, the mixture was filtered through diatomaceous earth. The filtrate was evaporated under reduced pressure to obtain the target compound (2000mg), which was used in the next step directly.

### Step 2: Synthesis of tert-butyl (R)-3-((S)-1-(tert-butoxy)-3-(3-hydroxyphenyl)-1-oxopropan-2-yl)pyrrolidine-1-carboxylate

(3-((S)-3-(tert-butoxy)-2-((R)-1-(tert-butoxycarbonyl)pyrrolidin-3-yl)-3-oxopropyl)phenyl)boronic acid (2000mg, 4.77mmol, 1eq) was dissolved in tetrahydrofuran (30mL) and hydrogen peroxide (8mL) was added. The mixture was stirred at 20°C for 16h; LCMS detection showed that the reaction was completed, saturated sodium bisulfite solution and ethyl acetate were added, and the mixture was extracted. The organic phase was dried over anhydrous sodium sulfate and filtered. The filtrate was concentrated to obtain a crude product. The crude product was purified by column chromatography (petroleum ether:ethyl acetate=4:1) to obtain the target compound (800mg). LCMS(ESI)[M+H]⁺=392.2.

### Step 3: Synthesis of tert-butyl (R)-3-((S)-3-(3-(2-(((benzyloxy)carbonyl)amino)ethoxy)phenyl)-1-(tert-butoxy)-1-oxopropan-2-yl)pyrrolidine-1-carboxylate

*Tert*-butyl (R)-3-((S)-1-(tert-butoxy)-3-(3-hydroxyphenyl)-1-oxopropan-2-yl)pyrrolidine-1-carboxylate (2g, 5.11mmol, 1eq), benzyl (2-bromoethyl)carbamate (2.64g, 10.22mmol, 2eq) and potassium carbonate (2.12g, 15.33mmol, 3eq) were added in acetonitrile (50mL) and stirred at 90°C for 16h; LCMS showed the reaction was completed. The reaction mixture was directly purified by column chromatography (petroleum ether:ethyl acetate=7:1) to obtain the target compound (3g). LCMS(ESI)[M+H]⁺=569.0.

### Step 4: Synthesis of tert-butyl (R)-3-((S)-3-(3-(2-aminoethoxy)phenyl)-1-(tert-butoxy)-1-oxopropan-2-yl)pyrrolidine-1-carboxylate

*Tert*-butyl (R)-3-((S)-3-(3-(2-(((benzyloxy)carbonyl)amino)ethoxy)phenyl)-1-(tert-butoxy)-1-oxopropan-2-yl)pyrrolidine-1-carboxylate (3g, 5.27mmol, 1eq) was dissolved in methanol (50mL), palladium (300mg, 10%) was added, and the mixture was exchanged with hydrogen three times; the mixture was stirred at 25°C for 16h. LCMS detection showed that the reaction was completed. The reaction mixture was filtered, and the filtrate was concentrated to obtain the target compound (2g). LCMS(ESI)[M+H]⁺=435.4

### Step 5: Synthesis of tert-butyl (R)-3-((S)-1-(tert-butoxy)-3-(3-(2-chloroacetyl)phenyl)-1-oxopropan-2-yl)pyrrolidine-1-carboxylate

*Tert*-butyl (R)-3-((S)-1-(tert-butoxy)-3-(3-(methoxycarbonyl)phenyl)-1-oxopropan-2-yl)pyrrolidine-1-carboxylate (700mg, 1.614mmol, 1eq) was dissolved in anhydrous tetrahydrofuran (10mL) and cooled to -78°C. Lithium diisopropylamide (1.0M, 2.42mL, 2.42mmol, 1.5eq) was added to the mixture and stirred at -78°C for 1h. Chloroiodomethane (854.03mg, 4.84mmol, 3eq) was added to the mixture and stirred at -78°C for another 2h. LCMS detection showed that the reaction was completed. Saturated ammonium chloride aqueous solution and ethyl acetate were added to the reaction mixture and the reaction mixture was extracted. The organic phase was dried over anhydrous sodium sulfate, filtered and concentrated to obtain a crude product. The crude product was purified by column chromatography (petroleum ether:ethyl acetate=4:1) to obtain the target compound (100mg). LCMS(ESI)[M+H-Boc]⁺=352.4.

### Step 6: Synthesis of di-tert-butyl 3,3'-((2S,2'S)-((2,2'-((2-(3-((S)-3-(tert-butoxy)-2-((R)-1-(tert-butoxycarbonyl)pyrrolidin-3-yl)-3-oxopropyl)phenoxy)ethyl)azanediyl)bis(acetyl))bis(3,1-phenylene))bis(3-(tert-butoxy)-3-oxopropane-1,2-diyl))(3R,3'R)-bis(pyrrolidine-1-carboxylate)

*Tert*-butyl (R)-3-((S)-3-(3-(2-aminoethoxy)phenyl)-1-(tert-butoxy)-1-oxopropan-2-yl)pyrrolidine-1-carboxylate (192.2mg, 0.44mmol, 2eq) and *tert*-butyl (R)-3-((S)-1-(tert-butoxy)-3-(3-(2-chloroacetyl)phenyl)-1-oxopropan-2-yl)pyrrolidine-1-carboxylate (100mg, 0.22mmol, 1eq) were dissolved in acetonitrile (3mL), anhydrous potassium carbonate (91.1mg, 0.66mmol, 3eq) was added, and the mixture was stirred at 60°C for 2h; LCMS detection showed that the reaction was completed. The reaction mixture was directly concentrated, and purified by silica gel column chromatography (petroleum ether:ethyl acetate=1:1) to obtain the target compound (50mg). LCMS(ESI)[M+H]⁺=1265.3.

### Step 7: Synthesis of (2S,2'S)-3,3'-((2,2'-((2-(3-((S)-2-carboxy-2-((R)-pyrrolidin-3-yl)ethyl)phenoxy)ethyl)azanediyl)bis(acetyl))bis(3,1-phenylene))bis(2-((R)-pyrrolidin-3-yl)propanoic acid)

Di-*tert*-butyl 3,3'-((2S,2'S)-((2,2'-((2-(3-((S)-3-(tert-butoxy)-2-((R)-1-(tert-butoxycarbonyl)pyrrolidin-3-yl)-3-oxopropyl)phenoxy)ethyl)azanediyl)bis(acetyl))bis(3,1-phenylene))bis(3-(tert-butoxy)-3-oxopropane-1,2-diyl))(3R,3'R)-bis(pyrrolidine-1-carboxylate) (50mg, 0.04mmol, 1eq) was added in hydrochloric acid/1,4-dioxane (2mL, 4.0M) at 0°C, the mixture was stirred at 25°C for 6h; LCMS showed the reaction was completed. The reaction mixture was concentrated to obtain a crude product, which was purified by Prep-HPLC to obtain the target compound (6mg). LCMS(ESI)[M-H]⁻=795.6; ¹HNMR(400MHz, DMSO) δ7.90 (s, 2H), 7.81 (d, J=8.0Hz, 2H), 7.63 (d, J=7.6Hz, 2H), 7.53 (d, J=8.0Hz, 2H), 7.10 (t, J=8.0Hz, 1H), 6.76 (d, J=7.6Hz, 1H), 6.53-6.45 (m, 2H), 5.34 (s, 3H), 4.46 (s, 2H), 3.92 (s, 2H), 3.34-2.69 (m, 25H), 2.41-2.32 (m, 3H), 2.03-1.95 (m, 3H), 1.69-1.62 (m, 3H).

### Example 16 (Compound 81)

### Preparation of (2S,2'S)-3,3'-((((2-(3-((S)-2-carboxy-2-((R)-pyrrolidin-3-yl)ethyl)phenoxy)acetyl)azanediyl)bis(ethane-2,1-diyl))bis(3,1-phenylene))bis(2-((R)-pyrrolidin-3-yl)propanoic acid)

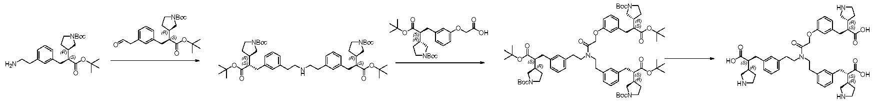

### Step 1: Synthesis of di-tert-butyl 3,3'-((2S,2'S)-((azanediylbis(ethane-2,1-diyl))bis(3,1-phenylene))bis(3-(tert-butoxy)-3-oxopropane-1,2-diyl))(3R,3'R)-bis(pyrrolidine-1-carboxylate)) Tert-butyl (R)-3-((S)-3-(3-(2-aminoethyl)phenyl)-1-(tert-butoxy)-1-oxopropan-2-yl)pyrrolidine-1-carboxylate (100mg) was dissolved in methanol (10mL), followed by adding tert-butyl (R)-3-((S)-1-(tert-butoxy)-1-oxo-3-(3-(2-oxoethyl)phenyl)propan-2-yl)pyrrolidine-1-carboxylate

(100mg) and sodium cyanoborohydride (15mg). The mixture was stirred at room temperature for 16h. LCMS showed the reaction was completed. The reaction mixture was concentrated after cooling and separated and purified by rapid chromatography (Silica gel, petroleum ether:ethyl acetate=1:1) to obtain the target compound (100mg). LCMS(ESI)[M+H]⁺=820.6.

### Step 2: Synthesis of di-tert-butyl 3,3'-((2S,2'S)-((((2-(3-((S)-3-(tert-butoxy)-2-((R)-1-(tert-butoxycarbonyl)pyrrolidin-3-yl)-3-oxopropyl)phenoxy)acetyl)azanediyl)bis(ethane-2,1-diyl))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-((azanediylbis(ethane-2,1-diyl))bis(3,1-phenylene))bis(3-(tert-butoxy)-3-oxopropane-1,2-diyl))(3R,3'R)-bis(pyrrolidine-1-carboxylate) (10mL) was added in 2-(3-((S)-3-(tert-butoxy)-2-((R)-1-(tert-butoxycarbonyl)pyrrolidin-3-yl)-3-oxopropyl)phenoxy)acetic acid (82mg), O-(7-azabenzotriazol-1-yl)-N,N,N',N'-tetramethyluronium hexafluorophosphate (93mg) and N,N-diisopropylethylamine (47mg); The mixture was stirred at room temperature for 16h. LCMS showed the reaction was completed. The reaction mixture was diluted with water and extracted with ethyl acetate. The organic phase was dried, filtered, and concentrated to obtain a crude product, which was separated and purified by rapid chromatography (Silica gel, petroleum ether:ethyl acetate=1:8) to obtain the target compound (70mg). LCMS(ESI)[M+H-Boc]⁺=1152.0.

### Step 3: Synthesis of (2S,2'S)-3,3'-((((2-(3-((S)-2-carboxy-2-((R)-pyrrolidin-3-yl)ethyl)phenoxy)acetyl)azanediyl)bis(ethane-2,1-diyl))bis(3,1-phenylene))bis(2-((R)-pyrrolidin-3-yl)propanoic acid)

Di-*tert*-butyl 3,3'-((2S,2'S)-((((2-(3-((S)-3-(tert-butoxy)-2-((R)-1-(tert-butoxycarbonyl)pyrrolidin-3-yl)-3-oxopropyl)phenoxy)acetyl)azanediyl)bis(ethane-2,1-diyl))bis(3,1-phenylene))bis(3-(tert-butoxy)-3-oxopropane-1,2-diyl))(3R,3'R)-bis(pyrrolidine-1-carboxylate)) (70mg) was dissolved in hydrochloric acid/1,4-dioxane (4mL) and stirred at room temperature for 16h. LCMS showed the reaction was completed. The reaction mixture was directly concentrated, separated and purified by preparative chromatography to obtain the target compound (13.41mg). LCMS(ESI)[M+H]⁺=783.1; ¹HNMR(400MHz, D₂O) δ 7.21 (td, *J*=7.7, 2.0Hz, 2H), 7.14-6.99 (m, 7H), 6.80 (d, *J*=7.6Hz, 1H), 6.57 (t, *J*=2.1Hz, 1H), 6.08 (dd, *J*=8.2, 2.6Hz, 1H), 4.14-4.04 (m, 2H), 3.63-3.55 (m, 2H), 3.41-3.26 (m, 7H), 3.14 (dddd, *J*=25.2, 23.0, 11.2, 4.0Hz, 4H), 2.87-2.63 (m, 12H), 2.60-2.54 (m, 1H), 2.43-2.25 (m, 6H), 2.10-1.98 (m, 3H), 1.66 (td, *J*=12.8, 11.1, 7.5Hz, 3H).

### Example 17 (Compound 90)

### Preparation of (2S,2'S,2"S)-3,3',3"-((nitrilotris(methylene-d₂))tris(benzene-3,1-diyl))tris(2-((R)-pyrrolidin-3-yl)propanoic acid)

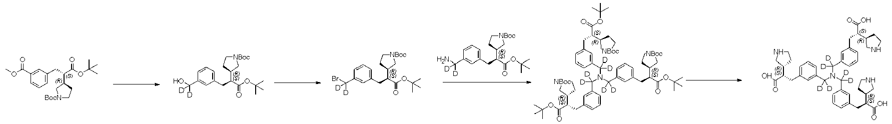

### Step 1: Synthesis of tert-butyl (R)-3-((S)-1-(tert-butoxy)-3-(3-(hydroxymethyl-d₂)phenyl)-1-oxopropan-2-yl)pyrrolidine-1-carboxylate

*Tert-butyl* (R)-3-((S)-1-(tert-butoxy)-3-(3-(methoxycarbonyl)phenyl)-1-oxopropan-2-yl)pyrrolidine-1-carboxylate (1.2g, 2.77mmol, 1eq) was dissolved in a mixture of tetrahydrofuran (14.4mL) and deuterated methanol (4.2mL) and deuterated sodium borohydride (695.19mg, 16.61mmol, 6eq) was added batchwise under nitrogen. The reaction mixture was stirred at 70°C for 3h. LCMS showed the reaction was completed. The reaction mixture was cooled to room temperature, followed by addition of deuterium oxide (5mL). The reaction mixture was stirred for 10 min. Water and ethyl acetate were added to the reaction mixture and the mixture was extracted. The organic phase was dried over anhydrous sodium sulfate, filtered, and concentrated to obtain a crude product. The residue was purified by silica gel column chromatography (petroleum ether/ethyl acetate=2:1) to obtain the target compound (600mg). LCMS(ESI)[M+Na]⁺=430.2.

### Step 2: Synthesis of tert-butyl (R)-3-((S)-3-(3-(bromomethyl-d₂)phenyl)-1-(tert-butoxy)-1-oxopropan-2-yl)pyrrolidine-1-carboxylate

*Tert*-butyl (*R*)-3-((S)-1-(tert-butoxy)-3-(3-(hydroxymethyl-*d₂*)phenyl)-1-oxopropan-2-yl)pyrrolidine-1-carboxylate (600mg, 1.47mmol, 1eq) was dissolved in dichloromethane (5mL) and cooled to 0°C. Triphenylphosphine (1158.47mg, 4.42mmol, 3eq) and N-bromosuccinimide (786.09mg, 4.42mmol, 3eq) were added. The mixture was stirred at room temperature for 2h. LCMS detection showed that the reaction was completed. Water and dichloromethane were added, and the reaction mixture was extracted. The organic phase was dried over anhydrous sodium sulfate, filtered and concentrated to obtain a crude product. The crude product was purified by silica gel column chromatography (petroleum ether/ethyl acetate=3:1) to obtain the target compound (400mg). LCMS(ESI)[M+Na]⁺=492.7.

### Step 3: Synthesis of tri-tert-butyl 3,3',3"-((2S,2'S,2"S)-((nitrilotris(methylene-d₂))tris(benzene-3,1-diyl))tris(3-(tert-butoxy)-3-oxopropane-1,2-diyl))(3R,3'R,3"R)-tris(pyrrolidine-1-carboxylate)

*Tert-butyl* (R)-3-((S)-3-(3-(aminomethyl-*d₂*)phenyl)-1-(tert-butoxy)-1-oxopropan-2-yl)pyrrolidine-1-carboxylate (30mg, 0.07mmol, 1eq), *tert-butyl* (R)-3-((S)-3-(3-(bromomethyl-d₂)phenyl)-1-(tert-butoxy)-1-oxopropan-2-yl)pyrrolidine-1-carboxylate (69.43mg, 0.15mmol, 2eq) and potassium carbonate (30.6mg, 0.22mmol, 3eq) were added in acetonitrile (1mL), heated to 60°C and stirred for 4h. LCMS detection showed that the reaction was completed and the reaction mixture was filtered. The filtrate was directly purified by silica gel column chromatography (petroleum ether/ethyl acetate=3:1) to obtain the target compound (55mg). LCMS(ESI)[M+H]⁺=1185.8.

### Step 4: Synthesis of (2S,2'S,2"S)-3,3',3"-((nitrilotris(methylene-d₂))tris(benzene-3,1-diyl))tris(2-((R)-pyrrolidin-3-yl)propanoic acid)

Tri-tert-butyl 3,3',3"-((2S,2'S,2"S)-((nitrilotris(methylene-*d₂*))tris(benzene-3,1-diyl))tris(3-(tert-butoxy)-3-oxopropane-1,2-diyl))(3R,3'R,3"R)-tris(pyrrolidine-1-carboxylate) (50mg, 0.04mmol, 1eq) was added in hydrochloric acid/1,4-dioxane (3.0mL, 4M) and stirred at 25°C for 2h. LCMS detection showed that the reaction was completed and the reaction mixture was filtered. The filter cake was lyophilized with acetonitrile and water to obtain the target compound (25.84mg). LCMS(ESI)[M-H]⁻=715.5; ¹HNMR(400MHz, D₂O) δ 7.38-7.27 (m, 6H), 7.1-7.11 (m, 6H), 3.61-3.51 (m, 3H), 3.38-3.33 (m, 3H), 3.28-3.16 (m, 3H), 3.07-2.98 (m, 3H), 2.91-2.87 (m, 3H), 2.84-2.74 (m, 3H), 2.68-2.58 (m, 3H), 2.53-2.47 (m, 3H), 2.15-2.09 (m, 3H), 1.78-1.65 (m, 3H).

### Example 18 (Compound 91)

### Preparation of (2S,2'S,2"S)-3,3',3"-((nitrilotris(methylene))tris(benzene-3,1-diyl))tris(2-((R)-pyrrolidin-3-yl)propanoic-3,3-d2 acid)

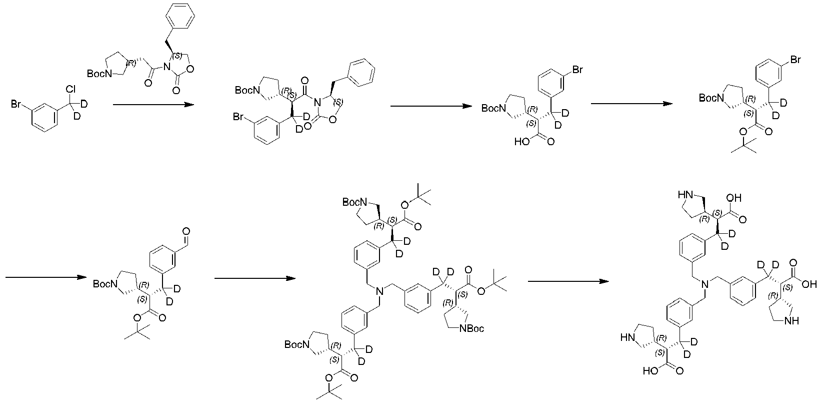

### Step 1: Synthesis of tert-butyl (R)-3-((S)-1-((S)-4-benzyl-2-oxooxazolidin-3-yl)-3-(3-bromophenyl)-1-oxopropan-2-yl-3,3-d₂)pyrrolidine-1-carboxylate

Under ice bath and nitrogen, a solution of lithium bis(trimethylsilyl)amide (7.08mL, 7.08mmol, 1.1eq, 1.0M) was added dropwise to a solution of *tert*-butyl (R)-3-(2-((S)-4-benzyl-2-oxooxazolidin-3-yl)-2-oxoethyl)pyrrolidine-1-carboxylate (2.5g, 6.44mmol, 1eq) in tetrahydrofuran (30mL). The mixture was stirred at 0°C for 30min; a solution of 1-bromo-3-(chloromethyl-*d₂*)benzene (1.47g, 7.08mmol, 1.1eq) in tetrahydrofuran (2mL) was slowly added dropwise, and after the addition, the reaction temperature was slowly raised to room temperature and the mixture was stirred for 16h. LCMS showed the reaction was completed, the reaction mixture was cooled with an ice-water bath, saturated aqueous ammonium chloride solution and water were added, and the mixture was extracted with ethyl acetate. The organic phase was washed with saturated aqueous sodium chloride solution, dried over anhydrous sodium sulfate, and filtered. The filtrate was concentrated to obtain a crude product. The crude product was purified by column chromatography to obtain the target compound (3.1g). LCMS(ESI)[M+H-*tert*-butyl]⁺=503.2.

### Step 2: Synthesis of (S)-3-(3-bromophenyl)-2-((R)-1-(tert-butoxycarbonyl)pyrrolidin-3-yl)propanoic-3,3-d2 acid

A solution of hydrogen peroxide (275mg, 8.1mmol, 1.5eq) was added to a solution of *tert*-butyl (R)-3-((S)-1-((S)-4-benzyl-2-oxooxazolidin-3-yl)-3-(3-bromophenyl)-1-oxopropan-2-yl-3,3-*d₂*)pyrrolidine-1-carboxylate (3g, 5.28mmol, 1eq) in tetrahydrofuran (120mL) and cooled with an ice bath, followed by adding a solution (10mL) of lithium hydroxide (192mg, 8.1mmol, 1.5eq). The reaction mixture was stirred at 25°C for 2.5h. LCMS showed the reaction was completed, the reaction mixture was cooled to 0°C, and a solution (5mL) of sodium bisulfite (1g) and sodium hydroxide solution (5N) were added to adjust the pH of the reaction mixture to >12. The mixture was extracted with water and methyl *tert*-butyl ether, and the organic phase was discarded. The aqueous phase was acidified to pH3 with hydrochloric acid solution (5N) and extracted with methyl *tert*-butyl ether. The organic phase was washed with saturated sodium chloride solution, dried over anhydrous sodium sulfate, filtered, and concentrated to obtain the target compound (1.8g). LCMS(ESI)[M+H-Boc]⁺=300.20.

### Step 3: Synthesis of tert-butyl (R)-3-((S)-3-(3-bromophenyl)-1-(tert-butoxy)-1-oxopropan-2-yl-3,3-d₂)pyrrolidine-1-carboxylate

(S)-3-(3-bromophenyl)-2-((R)-1-(tert-butoxycarbonyl)pyrrolidin-3-yl)propanoic-3,3-d2 acid (1.8g, 4.5mmol, 1eq) was dissolved in 2-methyltetrahydrofuran (50mL), *tert*-butyl N,N'-diisopropylcarbamimidate (4.5g, 22.5mmol, 5eq) was added, and the mixture was heated to 65°C and stirred for 16h under nitrogen, LCMS showed the reaction was completed, the insoluble matter was filtered, the filter cake was washed with methyl *tert*-butyl ether, and the filtrate was concentrated to obtain a crude product. The crude product was separated and purified by rapid chromatography (Silica gel, petroleum ether:ethyl acetate=10:1) to obtain the target compound (0.6g). LCMS(ESI)[M+H-2×*tert*-butyl]⁺=344.16.

### Step 4: Synthesis of tert-butyl (R)-3-((S)-1-(tert-butoxy)-3-(3-formylphenyl)-1-oxopropan-2-yl-3,3-d₂)pyrrolidine-1-carboxylate

*Tert*-butyl (R)-3-((S)-3-(3-bromophenyl)-1-(tert-butoxy)-1-oxopropan-2-yl-3,3-*d₂*)pyrrolidine-1-carboxylate (0.6g, 1.31mmol, 1eq) was dissolved in N,N-dimethylformamide (4mL), followed by adding palladium acetate (59mg, 0.26mmol, 0.2eq), 1,4-bis(diphenylphosphino)butane (112mg, 0.26mmol, 0.2eq), N-formylsaccharin (691mg, 3.296mmol, 2.5eq), sodium carbonate (418mg, 3.94mmol, 3eq) and triethylsilane (305mg, 2.63mmol, 2eq); under nitrogen, the mixture was heated to 75°C and stirred for 16h. The LCMS showed the reaction was completed. The insoluble matter was filtered, the filter cake was washed with methyl *tert*-butyl ether. The filtrate was concentrated, separated and purified by rapid chromatography (Silica gel, petroleum ether:ethyl acetate=10:1) to obtain the target compound (165mg). LCMS(ESI)[M+H-Boc]⁺=306.35.

### Step 5: Synthesis of tri-tert-butyl 3,3',3"-((2S,2'S,2"S)-((nitrilotris(methylene))tris(benzene-3,1-diyl))tris(3-(tert-butoxy)-3-oxopropane-1,2-diyl-1,1-d₂))(3R,3'R,3"R)-tris(pyrrolidine-1-carboxylate)

*Tert*-butyl (R)-3-((S)-1-(tert-butoxy)-3-(3-formylphenyl)-1-oxopropan-2-yl-3,3-*d₂*)pyrrolidine-1-carboxylate (160mg, 0.39mmol, 1eq) was dissolved in tetrahydrofuran (5mL), to which ammonia methanol (0.018mL, 0.13mmol, 0.33eq, 7.0M), sodium cyanoborohydride (61mg, 0.97mmol, 2.5eq) and one drop of acetic acid were added. The reaction mixture was stirred at room temperature overnight. LCMS showed the reaction was completed. The reaction was quenched with saturated ammonium chloride solution, extracted with ethyl acetate and water. The organic phase was dried over anhydrous sodium sulfate, filtered and concentrated to obtain a crude product. The crude product was purified by column chromatography to obtain the product (40mg). LCMS(ESI)[M+H]⁺=1186.02.

### Step 6: Synthesis of (2S,2'S,2"S)-3,3',3"-((nitrilotris(methylene))tris(benzene-3,1-diyl))tris(2-((R)-pyrrolidin-3-yl)propanoic-3,3-d2 acid)

Tri-*tert*-butyl 3,3',3"-((2S,2'S,2"S)-((nitrilotris(methylene))tris(benzene-3,1-diyl))tris(3-(tert-butoxy)-3-oxopropane-1,2-diyl-1,1-*d₂*))(3R,3'R,3"R)-tris(pyrrolidine-1-carboxylate) (30mg, 0.03mmol, 1eq) was dissolved in 1,4-dioxane (1mL), and hydrochloric acid/1,4-dioxane (1mL, 4.0M) was added; the mixture was stirred at room temperature overnight, and the reaction was monitored by LCMS until completion. The solvent was evaporated under reduced pressure, and the residue was separated and purified by Prep-HPLC (C₁₈, 10mmol/L NH₄HCO₃ in water, MeCN) to obtain the target compound (4.5mg). LCMS(ESI)[M+H]⁺=717.63; ¹HNMR(400MHz, D₂O) δ 8.37 (s, 2H), 7.40-7.25 (m, 6H), 7.24-7.09 (m, 6H), 4.19 (s, 6H), 3.56-3.43 (m, 3H), 3.40-3.29 (m, 3H), 3.26-3.13 (m, 3H), 3.00-2.87 (m, 3H), 2.49-2.30 (m, 6H), 2.15-1.97 (m, 3H), 1.75-1.59 (m, 3H).

### Example 19 (Compound 93)

### Preparation of (2S,2'S)-3,3'-((((3-((S)-2-carboxy-2-((R)-pyrrolidin-3-yl)ethyl)benzyl)azanediyl)bis(methylene-d₂))bis(3,1-phenylene))bis(2-((R)-pyrrolidin-3-yl)propanoic acid)

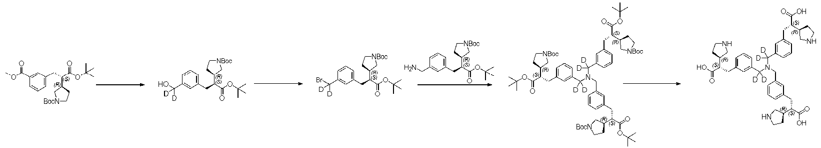

### Step 1: Synthesis of tert-butyl (R)-3-((S)-1-(tert-butoxy)-3-(3-(hydroxymethyl-d₂)phenyl)-1-oxopropan-2-yl)pyrrolidine-1-carboxylate

*Tert*-butyl (R)-3-((S)-1-(tert-butoxy)-3-(3-(methoxycarbonyl)phenyl)-1-oxopropan-2-yl)pyrrolidine-1-carboxylate (1.2g, 2.77mmol, 1eq) was dissolved in a mixture of tetrahydrofuran (14.4mL) and deuterated methanol (4.2mL) and deuterated sodium borohydride (695.19mg, 16.61mmol, 6eq) was added batchwise under nitrogen; the reaction mixture was stirred at 70°C for 3h. LCMS showed the reaction was completed. The reaction mixture was cooled to room temperature, followed by addition of deuterium oxide (5mL). The reaction mixture was stirred for 10 min. Water and ethyl acetate were added to the reaction mixture and the mixture was extracted. The organic phase was dried over anhydrous sodium sulfate, filtered, and concentrated to obtain a crude product. The crude product was purified by column chromatography (petroleum ether:ethyl acetate=2:1) to obtain the target compound (600mg). LCMS(ESI)[M+Na]⁺=430.2.

### Step 2: Synthesis of tert-butyl (R)-3-((S)-3-(3-(bromomethyl-d₂)phenyl)-1-(tert-butoxy)-1-oxopropan-2-yl)pyrrolidine-1-carboxylate

*Tert*-butyl (R)-3-((S)-1-(tert-butoxy)-3-(3-(hydroxymethyl-*d₂*)phenyl)-1-oxopropan-2-yl)pyrrolidine-1-carboxylate (600mg, 1.47mmol, 1eq) was dissolved in dichloromethane (5mL) and cooled to 0°C. Triphenylphosphine (1158.47mg, 4.42mmol, 3eq) and N-bromosuccinimide (786.09mg, 4.42mmol, 3eq) were added under ice bath. The mixture was continued to be stirred at room temperature for 2h; LCMS detection showed that the reaction was completed. Water and dichloromethane were added, and the reaction mixture was extracted. The organic phase was dried over anhydrous sodium sulfate, filtered and concentrated to obtain a crude product. The crude product was purified by column chromatography (petroleum ether:ethyl acetate=3:1) to obtain the target compound (400mg). LCMS(ESI)[M+Na]⁺=491.7.

### Step 3: Synthesis of di-tert-butyl 3,3'-((2S,2'S)-((((3-((S)-3-(tert-butoxy)-2-((R)-1-(tert-butoxycarbonyl)pyrrolidin-3-yl)-3-oxopropyl)benzyl)azanediyl)bis(methylene-d₂))bis(3,1-phenylene))bis(3-(tert-butoxy)-3-oxopropane-1,2-diyl))(3R,3'R)-bis(pyrrolidine- 1 -carboxylate)

*Tert*-butyl (R)-3-((S)-3-(3-(aminomethyl)phenyl)-1-(tert-butoxy)-1-oxopropan-2-yl)pyrrolidine-1-carboxylate (40mg, 0.1mmol, 1eq), *tert*-butyl (R)-3-((S)-3-(3-(bromomethyl-*d₂*)phenyl)-1-(tert-butoxy)-1-oxopropan-2-yl)pyrrolidine-1-carboxylate (93.03mg, 0.2mmol, 2eq) and potassium carbonate (41mg, 0.3mmol, 3eq) were added in acetonitrile (1mL) and stirred at 60°C for 4h. LCMS detection showed that the reaction was completed and the reaction mixture was filtered to obtain a crude product. The crude product was purified by silica gel column chromatography (petroleum ether:ethyl acetate=3:1) to obtain the target compound (60mg). LCMS(ESI)[M+Na]⁺=1205.6.

### Step 4: Synthesis of (2S,2'S)-3,3'-((((3-((S)-2-carboxy-2-((R)-pyrrolidin-3-yl)ethyl)benzyl)azanediyl)bis(methylene-d₂))bis(3,1-phenylene))bis(2-((R)-pyrrolidin-3-yl)propanoic acid)

Di-*tert*-butyl 3,3'-((2S,2'S)-((((3-((S)-3-(tert-butoxy)-2-((R)-1-(tert-butoxycarbonyl)pyrrolidin-3-yl)-3-oxopropyl)benzyl)azanediyl)bis(methylene-*d₂*))bis(3,1-phenylene))bis(3-(tert-butoxy)-3-oxopropane-1,2-diyl))(3R,3'R)-bis(pyrrolidine-1-carboxylate) (70mg, 0.06mmol, 1eq) was added to hydrochloric acid/1,4-dioxane (3mL, 4.0M). The mixture was stirred at 25°C for 2h. LCMS showed the reaction was completed. The reaction mixture was filtered and lyophilized with acetonitrile and water to obtain the target compound (41.67mg). LCMS(ESI)[M+H]⁺=715.2. ¹HNMR(400MHz, D₂O) δ 7.41-7.26(m, 6H), 7.15-7.11(m, 6H), 4.25-421(m, 2H), 3.59-353(m, 3H), 3.39-3.34(m, 3H), 3.27-3.17(m, 3H), 3.08-2.98(m, 3H), 2.92-2.87(m, 3H), 2.85-2.64(m, 6H), 2.53-2.48(m, 3H), 2.21-2.02(m,3H), 1.78-1.63(m, 3H).

### Example 20 (Compound 94)

### Preparation of (2S,2'S)-3,3'-(((((3-((S)-2-carboxy-2-((R)-pyrrolidin-3-yl)ethyl)phenyl)methyl-d₂)azanediyl)bis(methylene))bis(3,1-phenylene))bis(2-((R)-pyrrolidin-3-yl)propanoic acid)

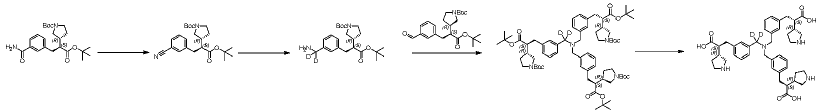

### Step 1: Synthesis of tert-butyl (R)-3-((S)-1-(tert-butoxy)-3-(3-cyanophenyl)-1-oxopropan-2-yl)pyrrolidine-1-carboxylate

Oxalyl chloride (365mg) was added in acetonitrile (10mL), the temperature of the reaction mixture was reduced to 0°C under nitrogen and N, N-dimethylformamide (262mg) was added. After stirring for 40min, a solution of *tert*-butyl (R)-3-((S)-1-(tert-butoxy)-3-(3-carbamoylphenyl)-1-oxopropan-2-yl)pyrrolidine-1-carboxylate (1.0g) in N,N-dimethylformamide (10mL) was added and the mixture was stirred for 3h. TLC showed the reaction was completed. Triethylamine (1mL) was added and the solvent was evaporated under reduced pressure to obtain a crude product. The crude product was purified by column chromatography (petroleum ether:ethyl acetate=2:1) to obtain the target compound (500mg). LCMS(ESI)[M+Na]⁺=423.2.

### Step 2: Synthesis of tert-butyl (R)-3-((S)-3-(3-(aminomethyl-d₂)phenyl)-1-(tert-butoxy)-1-oxopropan-2-yl)pyrrolidine-1-carboxylate

*Tert*-butyl (R)-3-((S)-1-(tert-butoxy)-3-(3-cyanophenyl)-1-oxopropan-2-yl)pyrrolidine-1-carboxylate (190mg) was dissolved in deuterated methanol (2mL), cooled to 0°C, and anhydrous nickel chloride (30.74mg) and deuterated sodium borohydride (107.68mg) were added; under nitrogen, the temperature was raised to 15°C, and the mixture was stirred for 16h; LCMS detection showed the reaction was completed. Deuterium oxide (1mL) was added to quench the reaction, and the mixture was stirred for ten min and extracted with ethyl acetate and water. The organic phase was evaporated under reduced pressure to obtain a crude product. The crude product was purified by column chromatography (dichloromethane:methanol=10:1) to obtain the target product (90mg). LCMS(ESI)[M+H]⁺=407.2.

### Step 3: Synthesis of di-tert-butyl 3,3'-((2S,2'S)-(((((3-((S)-3-(tert-butoxy)-2-((R)-1-(tert-butoxycarbonyl)pyrrolidin-3-yl)-3-oxopropyl)phenyl)methyl-d₂)azanediyl)bis(methylene))bis(3,1-phenylene))bis(3-(tert-butoxy)-3-oxopropane-1,2-diyl))(3R,3'R)-bis(pyrrolidine-1-carboxylate)

*Tert*-butyl (R)-3-((S)-3-(3-(aminomethyl-*d₂*)phenyl)-1-(tert-butoxy)-1-oxopropan-2-yl)pyrrolidine-1-carboxylate (90mg) and *tert*-butyl (R)-3-((S)-1-(tert-butoxy)-3-(3-formylphenyl)-1-oxopropan-2-yl)pyrrolidine-1-carboxylate (223.31mg) were dissolved in isopropanol (3mL), sodium triacetoxyborohydride (232.03mg) was added, and the mixture was stirred at 15°C for 16h; LCMS detection showed that the reaction was completed. The reaction mixture was extracted with water and ethyl acetate, and the organic phase was dried over anhydrous sodium sulfate, filtered and concentrated to obtain a crude product. The crude product was purified by column chromatography (petroleum ether:ethyl acetate=2:1) to obtain the target compound (150mg). LCMS(ESI)[M+Na]⁺=1203.6.

### Step 4: Synthesis of (2S,2'S)-3,3'-(((((3-((S)-2-carboxy-2-((R)-pyrrolidin-3-yl)ethyl)phenyl)methyl-d₂)azanediyl)bis(methylene))bis(3,1-phenylene))bis(2-((R)-pyrrolidin-3-yl)propanoic acid)

Di-*tert*-butyl 3,3'-((2S,2'S)-(((((3-((S)-3-(tert-butoxy)-2-((R)-1-(tert-butoxycarbonyl)pyrrolidin-3-yl)-3-oxopropyl)phenyl)methyl-*d*₂)azanediyl)bis(methylene))bis(3,1-phenylene))bis(3-(tert-butoxy)-3-oxopropane-1,2-diyl))(3R,3'R)-bis(pyrrolidine-1-carboxylate) (150mg) was added to hydrochloric acid/1,4-dioxane (5mL, 4.0M). The mixture was stirred at 25°C for 3h. The reaction mixture was filtered and lyophilized with acetonitrile and water to obtain the target compound (74mg). LCMS(ESI)[M-H]⁺=711.42; ¹HNMR(400MHz, MeOD) δ 7.54 (s, 3H), 7.45-7.34 (m, 9H), 4.37-4.25 (m, 4H), 3.61-3.53 (m, 3H), 3.50-3.42 (m, 3H), 3.32-3.26 (m, 3H), 3.22-3.13 (m, 3H), 3.10-2.98 (m, 6H), 2.95-2.84 (m, 3H), 2.67-2.58 (m, 3H), 2.26-2.17 (m, 3H), 1.94-1.79 (m, 3H).

### Example 21 (Compound 97)

### Preparation of (S)-3-(3-((3-((S)-2-carboxy-2-((R)-pyrrolidin-3-yl)ethyl)-N-(2-(3-((S)-2-carboxy-2-((R)-pyrrolidin-3-yl)ethyl)phenoxy)ethyl)benzamido)methyl)phenyl)-2-((R)-pyrrolidin-3-yl)propanoic acid

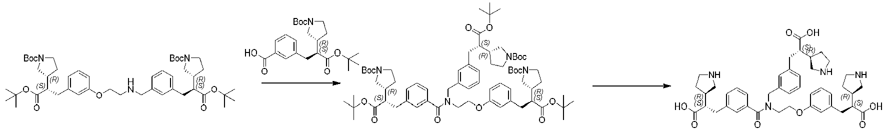

### Step 1: tert-butyl (R)-3-((S)-1-(tert-butoxy)-3-(3-((3-((S)-3-(tert-butoxy)-2-((R)-1-(tert-butoxycarbonyl)pyrrolidin-3-yl)-3-oxopropyl)-N-(2-(3-((S)-3-(tert-butoxy)-2-((R)-1-(tert-butoxycarbonyl)pyrrolidin-3-yl)-3-oxopropyl)phenoxy)ethyl)benzamido)methyl)phenyl)-1-oxopropan-2-yl)pyrrolidine-1-carboxylate

*Tert*-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (70mg) was dissolved in N,N-dimethylformamide (5mL), and 3-((S)-3-(tert-butoxy)-2-((R)-1-(tert-butoxycarbonyl)pyrrolidin-3-yl)-3-oxopropyl)benzoic acid (43mg), O-(7-azabenzotriazol-1-yl)-N,N,N',N'-tetramethyluronium hexafluorophosphate (32mg) and N,N-diisopropylethylamine (17mg) were added. The mixture was stirred at room temperature for 16h. LCMS showed the reaction was completed. The reaction mixture was directly concentrated, separated and purified by rapid chromatography (Silica gel, petroleum ether:ethyl acetate=1:8) to obtain the target compound (70mg). LCMS(ESI)[M+H]⁺=1223.9.

### Step 2: Synthesis of (S)-3-(3-((3-((S)-2-carboxy-2-((R)-pyrrolidin-3-yl)ethyl)-N-(2-(3-((S)-2-carboxy-2-((R)-pyrrolidin-3-yl)ethyl)phenoxy)ethyl)benzamido)methyl)phenyl)-2-((R)-pyrrolidin-3-yl)propanoic acid

*Tert*-butyl (R)-3-((S)-1-(tert-butoxy)-3-(3-((3-((S)-3-(tert-butoxy)-2-((R)-1-(tert-butoxycarbonyl)pyrrolidin-3-yl)-3-oxopropyl)-N-(2-(3-((S)-3-(tert-butoxy)-2-((R)-1-(tert-butoxycarbonyl)pyrrolidin-3-yl)-3-oxopropyl)phenoxy)ethyl)benzamido)methyl)phenyl)-1-oxopropan-2-yl)pyrrolidine-1-carboxylate (70mg) was dissolved in hydrochloric acid/1,4-dioxane (2mL) and stirred at room temperature for 16h. LCMS showed the reaction was completed. The reaction mixture was directly concentrated, separated and purified by preparative chromatography to obtain the target compound (15.41mg). LCMS(ESI)[M+H]⁺=755.4; ¹HNMR(400MHz, D₂O) δ 7.32-7.15 (m, 4H), 7.14-6.97 (m, 3H), 6.94-6.74 (m, 3H), 6.71-6.49 (m, 2H), 4.53 (d, *J*=14.7Hz, 2H), 4.26 (d, *J*=5.5Hz, 1H), 4.06 (d, *J*=15.0Hz, 1H), 3.94 (s, 1H), 3.82-3.67 (m, 2H), 3.32 (dt, *J*=23.6, 8.8Hz, 5H), 3.13 (dt, *J*=18.8, 9.5Hz, 3H), 2.73 (dtd, *J*=41.0, 21.0, 9.8Hz, 9H), 2.34 (t, *J*=11.9Hz, 6H), 2.02 (d, *J*=9.2Hz, 3H), 1.72-1.55 (m, 3H).

### Example 22 (Compound 98)

### Preparation of (2S,2'S)-3,3'-((((3-((S)-2-carboxy-2-((R)-pyrrolidin-3-yl)ethyl-1,1-d₂)benzyl)azanediyl)bis(methylene))bis(3,1-phenylene))bis(2-((R)-pyrrolidin-3-yl)propanoic acid)

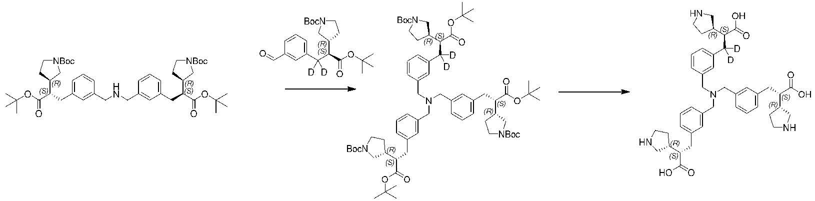

### Step 1: Synthesis of di-tert-butyl 3,3'-((2S,2'S)-((((3-((S)-3-(tert-butoxy)-2-((R)-1-(tert-butoxycarbonyl)pyrrolidin-3-yl)-3-oxopropyl-1,1-d₂)benzyl)azanediyl)bis(methylene))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (117mg, 0.15mmol, 1eq), *tert*-butyl (R)-3-((S)-1-(tert-butoxy)-3-(3-formylphenyl)-1-oxopropan-2-yl-3,3-*d₂*)pyrrolidine-1-carboxylate (60mg, 0.15mmol, 1eq) and a drop of acetic acid were dissolved in tetrahydrofuran (2mL) and stirred at room temperature for 0.5h. Sodium cyanoborohydride (18mg, 0.30mmol, 2eq) was added; the mixture was stirred at room temperature for 16h. LCMS detection showed that the reaction was completed. The reaction mixture was concentrated, and ethyl acetate was added. The mixture was washed with saturated sodium bicarbonate solution and saturated saline solution successively, and separated. The organic phase was dried over anhydrous sodium sulfate, filtered, and concentrated to obtain a crude product. The crude product was separated and purified by rapid chromatography (Silica gel, dichloromethane:methanol=40:1) to obtain the target compound (120mg). LCMS(ESI)[M+H]⁺=1181.99.

### Step 2: Synthesis of (2S,2'S)-3,3'-((((3-((S)-2-carboxy-2-((R)-pyrrolidin-3-yl)ethyl-1,1-d₂)benzyl)azanediyl)bis(methylene))bis(3,1-phenylene))bis(2-((R)-pyrrolidin-3-yl)propanoic acid)

Di-*tert*-butyl 3,3'-((2S,2'S)-((((3-((S)-3-(tert-butoxy)-2-((R)-1-(tert-butoxycarbonyl)pyrrolidin-3-yl)-3-oxopropyl-1,1-*d₂*)benzyl)azanediyl)bis(methylene))bis(3,1-phenylene))bis(3-(tert-butoxy)-3-oxopropane-1,2-diyl))(3R,3'R)-bis(pyrrolidine-1-carboxylate) (110mg, 0.09mmol, 1eq) was dissolved in 1,4-dioxane (2mL), hydrochloric acid/1,4-dioxane (2mL, 4.0M) was added, and the mixture was stirred at room temperature overnight. The solvent was evaporated under reduced pressure, and the residue was separated and purified by Prep-HPLC (C_{18,} 10mmol/L NH₄HCO₃ in water, MeCN) to obtain the target compound (33mg). LCMS(ESI)[M+H]⁺=713.62; ¹HNMR(400MHz, D₂O) δ 7.27 (t, *J*=7.5Hz, 3H), 7.23-6.89 (m, 9H), 3.59 (s, 6H), 3.40-3.28 (m, 6H), 3.17-3.09 (m, 3H), 2.88-2.69 (m, 7H), 2.47-2.32 (m, 6H), 2.10-1.97 (m, 3H), 1.72-1.58 (m, 3H).

### Example 23 (Compound 99)

### Preparation of (S)-3-(3-(2-(3-((S)-2-carboxy-2-((R)-pyrrolidin-3-yl)ethyl)-N-(3-((S)-2-carboxy-2-((R)-pyrrolidin-3-yl)ethyl)benzyl)benzamido)ethyl)phenyl)-2-((R)-pyrrolidin-3-yl)propanoic acid

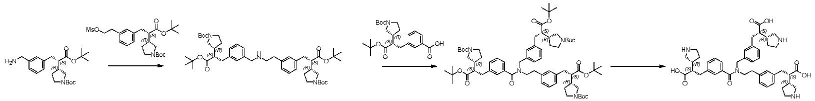

### Step 1: Synthesis of tert-butyl (R)-3-((S)-1-(tert-butoxy)-3-(3-(((3-((S)-3-(tert-butoxy)-2-((R)-1-(tert-butoxycarbonyl)pyrrolidin-3-yl)-3-oxopropyl)phenethyl)amino)methyl)phenyl)-1-oxopropan-2-yl)pyrrolidine-1-carboxylate

*Tert*-butyl (R)-3-((S)-3-(3-(aminomethyl)phenyl)-1-(tert-butoxy)-1-oxopropan-2-yl)pyrrolidine-1-carboxylate (100mg) was dissolved in acetonitrile (10mL), *tert*-butyl (R)-3-((S)-1-(tert-butoxy)-3-(3-(2-((methylsulfonyl)oxy)ethyl)phenyl)-1-oxopropan-2-yl)pyrrolidine-1-carboxylate (122mg) was added, and the mixture was stirred at 75°C overnight; LCMS showed the reaction was completed. The solvent was evaporated under reduced pressure, and the residue was purified by column chromatography to obtain the target compound (70mg). LCMS(ESI)[M+H]⁺=806.79.

### Step 2: Synthesis of tert-butyl (R)-3-((S)-1-(tert-butoxy)-3-(3-((3-((S)-3-(tert-butoxy)-2-((R)-1-(tert-butoxycarbonyl)pyrrolidin-3-yl)-3-oxopropyl)-N-(3-((S)-3-(tert-butoxy)-2-((R)-1-(tert-butoxycarbonyl)pyrrolidin-3-yl)-3-oxopropyl)phenethyl)benzamido)methyl)phenyl)-1-oxopropan-2-yl)pyrrolidine-1-carboxylate

*Tert*-butyl (R)-3-((S)-1-(tert-butoxy)-3-(3-(((3-((S)-3-(tert-butoxy)-2-((R)-1-(tert-butoxycarbonyl)pyrrolidin-3-yl)-3-oxopropyl)phenethyl)amino)methyl)phenyl)-1-oxopropan-2-yl)pyrrolidine-1-carboxylate (70mg) and 3-((S)-3-(tert-butoxy)-2-((R)-1-(tert-butoxycarbonyl)pyrrolidin-3-yl)-3-oxopropyl)benzoic acid (44mg) were dissolved in *N,N-*dimethylformamide (0.5mL), O-(7-azabenzotriazol-1-yl)-N,N,N',N'-tetramethyluronium hexafluorophosphate (50mg) and triethylamine (34mg) were added, and the reaction mixture was stirred at room temperature for 16h. TLC monitoring showed that the reaction was completed, ethyl acetate and water were added, and the mixture was extracted and separated. The organic phase was dried over anhydrous sodium sulfate, filtered, concentrated, and purified by column chromatography to obtain the target compound (75mg). LCMS(ESI)[M+H-Boc]⁺=1107.97.

### Step 3: Synthesis of (S)-3-(3-(2-(3-((S)-2-carboxy-2-((R)-pyrrolidin-3-yl)ethyl)-N-(3-((S)-2-carboxy-2-((R)-pyrrolidin-3-yl)ethyl)benzyl)benzamido)ethyl)phenyl)-2-((R)-pyrrolidin-3-yl)propanoic acid

*Tert*-butyl (R)-3-((S)-1-(tert-butoxy)-3-(3-((3-((S)-3-(tert-butoxy)-2-((R)-1-(tert-butoxycarbonyl)pyrrolidin-3-yl)-3-oxopropyl)-N-(3-((S)-3-(tert-butoxy)-2-((R)-1-(tert-butoxycarbonyl)pyrrolidin-3-yl)-3-oxopropyl)phenethyl)benzamido)methyl)phenyl)-1-oxopropan-2-yl)pyrrolidine-1-carboxylate (75mg) was dissolved in 1,4-dioxane (2mL), and hydrochloric acid/1,4-dioxane (4.0M, 2mL) was added; the mixture was stirred at room temperature for 16h; The solvent was evaporated under reduced pressure, and the residue was separated and purified by Prep-HPLC (C_{18,} 10mmol/L NH₄HCO₃ in water, MeCN) to obtain the target compound (17mg). LCMS(ESI)[M+H]⁺=739.4; ¹HNMR(400MHz, D₂O) δ 7.33-6.83 (m, 10H), 6.82-6.59 (m, 2H), 3.70-3.07 (m, 12H), 3.02-2.50 (m, 12H), 2.46-2.28 (m, 6H), 2.10-1.99 (m, 3H), 1.74-1.59 (m, 3H).

### Example 24 (Compound 100)

### Preparation of (S)-3-(3-(2-((3-((S)-2-carboxy-2-((R)-pyrrolidin-3-yl)ethyl)phenethyl)(2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid

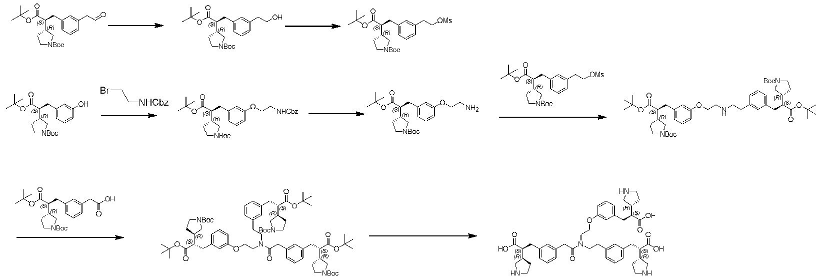

### Step 1: Synthesis of tert-butyl (R)-3-((S)-1-(tert-butoxy)-3-(3-(2-hydroxyethyl)phenyl)-1-oxopropan-2-yl)pyrrolidine-1-carboxylate

*Tert*-butyl (R)-3-((S)-1-(tert-butoxy)-1-oxo-3-(3-(2-oxoethyl)phenyl)propan-2-yl)pyrrolidine-1-carboxylate (300mg) was dissolved in ethanol (10mL), and sodium borohydride (55mg) was added; the mixture was stirred at room temperature for 16h. LCMS showed the reaction was completed. The reaction mixture was concentrated directly and purified by column chromatography to obtain the target compound (280mg). LCMS(ESI)[M+H]⁺=420.3.

### Step 2: Synthesis of tert-butyl (R)-3-((S)-1-(tert-butoxy)-3-(3-(2-((methylsulfonyl)oxy)ethyl)phenyl)-1-oxopropan-2-yl)pyrrolidine-1-carboxylate

*Tert*-butyl (R)-3-((S)-1-(tert-butoxy)-3-(3-(2-hydroxyethyl)phenyl)-1-oxopropan-2-yl)pyrrolidine-1-carboxylate (280mg) was dissolved in dichloromethane (6mL), methanesulfonic anhydride (116mg) and triethylamine (135mg) were added; the mixture was stirred at room temperature for 16h. LCMS showed the reaction was completed. The crude product was separated and purified by rapid chromatography (Silica gel, petroleum ether:ethyl acetate=10:1) to obtain the target compound (300mg). LCMS(ESI)[M+H]⁺=498.2.

### Step 3: Synthesis of tert-butyl (R)-3-((S)-3-(3-(2-(((benzyloxy)carbonyl)amino)ethoxy)phenyl)-1-(tert-butoxy)-1-oxopropan-2-yl)pyrrolidine-1-carboxylate

*Tert*-butyl (R)-3-((S)-1-(tert-butoxy)-3-(3-hydroxyphenyl)-1-oxopropan-2-yl)pyrrolidine-1-carboxylate (2000mg) was dissolved in N,N-dimethylformamide (20mL), benzyl (2-bromoethyl)carbamate (3956mg) and potassium carbonate (3530mg) were added. The mixture was stirred at 90°C for 16h under nitrogen. LCMS showed the reaction was completed. Water was added to the reaction mixture, it was extracted with ethyl acetate, and the organic phase was dried over anhydrous sodium sulfate, filtered, and concentrated to obtain a crude product. The product was separated and purified by rapid chromatography (Silica gel, petroleum ether: ethyl acetate=2:1) to obtain the target compound (1500mg). LCMS(ESI)[M+H]⁺=569.3.

### Step 4: Synthesis of tert-butyl (R)-3-((S)-3-(3-(2-aminoethoxy)phenyl)-1-(tert-butoxy)-1-oxopropan-2-yl)pyrrolidine-1-carboxylate

*Tert*-butyl (R)-3-((S)-3-(3-(2-(((benzyloxy)carbonyl)amino)ethoxy)phenyl)-1-(tert-butoxy)-1-oxopropan-2-yl)pyrrolidine-1-carboxylate (1500mg) was dissolved in methanol (10mL), and Pd/C (150mg) was added; the mixture was stirred at room temperature for 16h under a hydrogen (hydrogen balloon). LCMS showed that the reaction was completed. The reaction mixture was directly concentrated, separated and purified by rapid chromatography (Silica gel, petroleum ether:ethyl acetate=1:1) to obtain the target compound (1100mg). LCMS(ESI)[M+H]⁺=435.3.

### Step 5: Synthesis of tert-butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)ethyl)phenyl)-1-oxopropan-2-yl)pyrrolidine-1-carboxylate

*Tert-*butyl (R)-3-((S)-3-(3-(2-aminoethoxy)phenyl)-1-(tert-butoxy)-1-oxopropan-2-yl)pyrrolidine-1-carboxylate (175mg) was dissolved in acetonitrile (5mL), followed by adding *tert-*butyl (R)-3-((S)-1-(tert-butoxy)-3-(3-(2-((methylsulfonyl)oxy)ethyl)phenyl)-1-oxopropan-2-yl)pyrrolidine-1-carboxylate (200mg) and potassium carbonate (156mg). The reaction mixture was stirred at 80°C for 16h. LCMS showed the reaction was completed. The reaction mixture was concentrated after cooling and separated and purified by rapid chromatography (silica gel, petroleum ether:ethyl acetate=1:1) to give the target compound (280mg). LCMS(ESI)[M+H]⁺=836.6.

### Step 6: Synthesis of tert-butyl (R)-3-((S)-1-(tert-butoxy)-3-(3-(2-((3-((S)-3-(tert-butoxy)-2-((R)-1-(tert-butoxycarbonyl)pyrrolidin-3-yl)-3-oxopropyl)phenethyl)(2-(3-((S)-3-(tert-butoxy)-2-((R)-1-(tert-butoxycarbonyl)pyrrolidin-3-yl)-3-oxopropyl)phenoxy)ethyl)amino)-2-oxoethyl)phenyl)-1-oxopropan-2-yl)pyrrolidine-1-carboxylate

*Tert-*butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)ethyl)phenyl)-1-oxopropan-2-yl)pyrrolidine-1-carboxylate (140mg) was dissolved in N,N-dimethylformamide (10mL), followed by adding 2-(3-((S)-3-(tert-butoxy)-2-((R)-1-(tert-butoxycarbonyl)pyrrolidin-3-yl)-3-oxopropyl)phenyl)acetic acid (80mg), O-(7-azabenzotriazol-1-yl)-N,N,N',N'-tetramethyluronium hexafluorophosphate (64mg), and *N,N*-diisopropylethylamine (32mg). The reaction mixture was stirred at room temperature for 16h. LCMS showed the reaction was completed. Water and ethyl acetate were added to the reaction mixture. The mixture was extracted, and the organic phase was dried over anhydrous sodium sulfate, filtered, and concentrated. The product was separated and purified by rapid chromatography (silica gel, petroleum ether:ethyl acetate=1:8) to give the target compound (70mg). LCMS(ESI)[M+H]⁺=1251.8.

### Step 7: Synthesis of (S)-3-(3-(2-((3-((S)-2-carboxy-2-((R)-pyrrolidin-3-yl)ethyl)phenethyl)(2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid

*Tert-*butyl (R)-3-((S)-1-(tert-butoxy)-3-(3-(2-((3-((S)-3-(tert-butoxy)-2-((R)-1-(tert-butoxycarbonyl)pyrrolidin-3-yl)-3-oxopropyl)phenethyl)(2-(3-((S)-3-(tert-butoxy)-2-((R)-1-(tert-butoxycarbonyl)pyrrolidin-3-yl)-3-oxopropyl)phenoxy)ethyl)amino)-2-oxoethyl)phenyl)-1-oxopropan-2-yl)pyrrolidine-1-carboxylate (70mg) was dissolved in hydrochloric acid/1,4-dioxane (4.0M, 4mL) and stirred at room temperature for 16h. LCMS showed the reaction was completed. The reaction mixture was concentrated directly, separated and purified by preparative chromatography to give the target compound (19.18mg). LCMS(ESI)[M+H]⁺=783.4; ¹HNMR(400MHz, D₂O) δ 7.20-7.08 (m, 2H), 7.07-6.98 (m, 2H), 6.96-6.81 (m, 4H), 6.74 (d, *J*=7.6Hz, 1H), 6.64 (dt, *J*=6.7, 2.8Hz, 2H), 6.61-6.54 (m, 1H), 4.49 (s, 2H), 4.08 (t, *J*=5.2Hz, 1H), 3.79 (d, *J*=5.6Hz, 1H), 3.63-3.50 (m, 3H), 3.43 (d, *J*=5.3Hz, 1H), 3.30-3.18 (m, 5H), 3.14-2.97 (m, 5H), 2.75-2.49 (m, 10H), 2.33-2.16 (m, 6H), 1.94 (qq, *J*=6.6, 3.8Hz, 3H), 1.65-1.47 (m, 3H).

### Example 25 (Compound 101)

### Preparation of (2S,2'S,2"S)-3,3',3"-((nitrilotris(methylene))tris(benzo[b]thiophene-4,2-diyl))tris(2-((R)-pyrrolidin-3-yl)propanoic acid)

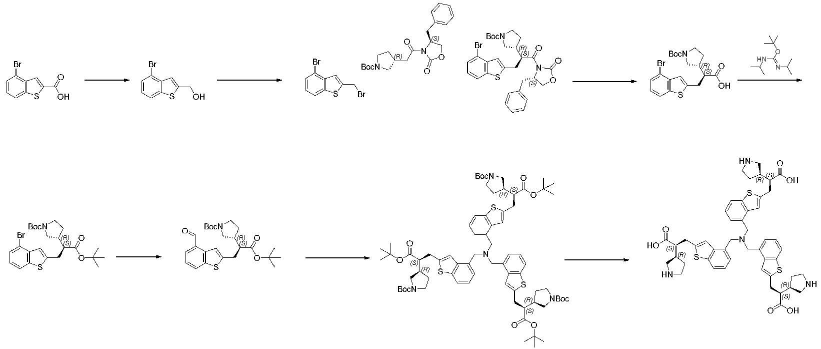

### Step 1: Synthesis of (4-bromobenzo[b]thiophen-2-yl)methanol

4-bromobenzo[b]thiophene-2-carboxylic acid (10g, 38.9mmol, 1.0eq) was dissolved in THF (100mL), followed by the slow addition of a solution of borane/THF (58.3mL, 110mmol, 1.5eq, 1.0M) at 0°C. The mixture was heated to 60°C under nitrogen protection and stirred for 3h. LCMS showed the reaction was completed. It was quenched with methanol, the reaction mixture was filtered, water was added to the filtrate, it was extracted with ethyl acetate, the organic phases were combined, dried over anhydrous sodium sulfate, filtered, and concentrated to give the crude product, and the target compound (4.0g) was obtained by column chromatography (petroleum ether:ethyl acetate=5: 1). LCMS(ESI)[M+H]⁺=242.6.

### Step 2: Synthesis of 4-bromo-2-(bromomethyl)benzo[b]thiophene

(4-bromobenzo[b]thiophen-2-yl)methanol (4.0g, 16.45mmol, 1eq) and hydrogen bromide (24mL, 40%) were added to dichloromethane (32mL). Under nitrogen protection, it was heated to 46°C and stirred for 18h. LCMS showed the reaction was completed. Water and dichloromethane were added to the reaction mixture which was extracted and separated. The organic phase was washed with saturated saline solution, dried over anhydrous sodium sulfate, filtered and concentrated to give the crude product, which was subjected to column chromatography (petroleum ether=100%) to give the target compound (3.7g).

### Step 3: Synthesis of tert-butyl (R)-3-((S)-1-((S)-4-benzyl-2-oxooxazolidin-3-yl)-3-(4-bromobenzo[b]thiophen-2-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate (7.16g, 18.43mmol, 1.2eq) was dissolved in tetrahydrofuran (70mL), lithium bis(trimethylsilyl)amide (8.3mL, 23.04mmol, 1.5eq) was added at 0°C and stirred for 0.5h, and 4-bromo-2-(bromomethyl)benzo[b]thiophene (4.7g, 15.36mmol, 1.0eq) was added and stirred for 2h. LCMS showed the reaction was completed. The reaction mixture was poured into ice water and extracted with ethyl acetate. The organic phase was washed with saturated saline solution, dried over anhydrous sodium sulfate, filtered, and concentrated to give the crude product, which was separated and purified by rapid chromatography (silica gel, petroleum ether:ethyl acetate=5:1) to give the target product (3.7g). LCMS(ESI)[M+H-Boc]⁺=513.4.

### Step 4: Synthesis of (S)-3-(4-bromobenzo[b]thiophen-2-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(4-bromobenzo[b]thiophen-2-yl)-1-oxopropan-2-yl)pyrrolidine-1-carboxylate (8.5g, 13.85mmol, 1.0eq) was added in tetrahydrofuran (90mL), followed by adding the aqueous solution of hydrogen peroxide (22.4mL, 30%) and lithium hydroxide (664mg, 27.7mmol, 2.0eq) at 0°C. The reaction mixture was warmed to room temperature and incubated for 2h. LCMS showed the reaction was completed. It was quenched with sodium sulfate decahydrate. The pH was adjusted to 5-6 with dilute hydrochloric acid, and the mixture was extracted with ethyl acetate. The organic phase was washed with saturated saline solution, dried over anhydrous sodium sulfate, filtered, and concentrated to give the crude product, which was separated and purified by rapid chromatography (silica gel, petroleum ether:ethyl acetate=3:1) to give the target compound (6.0g). LCMS(ESI)[M+H-*tert-*butyl]⁺=398.3

### Step 5: Synthesis of tert-butyl (R)-3-((S)-3-(4-bromobenzo[b]thiophen-2-yl)-1-(tert-butoxy)-1-oxopropan-2-yl)pyrrolidine-1-carboxylate

(S)-3-(4-bromobenzo[b]thiophen-2-yl)-2-((R)-1-(tert-butoxycarbonyl)pyrrolidin-3-yl)propanoic acid (6g, 13.2mmol, 1.0eq) was added in 2-methyltetrahydrofuran (70mL), followed by adding *tert-*butyl N,N'-diisopropylcarbamimidate (10.58g, 52.82mmol, 4.0eq). The mixture was stirred at 70°C for 3h under nitrogen protection. LCMS showed the reaction was completed. The reaction mixture was added with water and extracted with ethyl acetate. The organic phase was washed with saturated sodium carbonate solution and saturated sodium chloride solution, dried over anhydrous sodium sulfate, filtered, and concentrated to give the crude product, which was separated and purified by rapid chromatography (silica gel, petroleum ether:ethyl acetate=9:1) to give the target compound (5.3g). LCMS(ESI) [2M+Na]⁺=1041.2.

### Step 6: Synthesis of tert-butyl (R)-3-((S)-1-(tert-butoxy)-3-(4-formylbenzo[b]thiophen-2-yl)-1-oxopropan-2-yl)pyrrolidine-1-carboxylate

*Tert-*butyl (R)-3-((S)-3-(4-bromobenzo[b]thiophen-2-yl)-1-(tert-butoxy)-1-oxopropan-2-yl)pyrrolidine-1-carboxylate(2.2g, 4.31mmol, 1.0eq) was dissolved in toluene (25mL), followed by adding palladium acetate (193.53mg, 0.862mmol, 0.2eq), N,N,N',N'-tetramethylethylenediamine (901.52mg, 7.758mmol, 1.8eq), and n-butyldi(1-adamantyl)phosphine (309.06mg, 0.862mmol, 0.2eq). The reaction mixture was charged with hydrogen and carbon monoxide (1:1=1.6 MPa:1.6 MPa), heated to 110°C, and stirred for 48h. LCMS showed the reaction was completed. the reaction mixture was concentrated directly and separated and purified by rapid chromatography (silica gel, petroleum ether:ethyl acetate=5:1) to give the target compound (1.3g). LCMS(ESI)[M+H-Boc]⁺=360.2

### Step 7: Synthesis of tri-tert-butyl 3,3',3"-((2S,2'S,2"S)-((nitrilotris(methylene))tris(benzo[b]thiophene-4,2-diyl))tris(3-(tert-butoxy)-3-oxopropane-1,2-diyl))(3R,3'R,3"R)-tris(pyrrolidine-1-carboxylate)

*Tert-*butyl (R)-3-((S)-1-(tert-butoxy)-3-(4-formylbenzo[b]thiophen-2-yl)-1-oxopropan-2-yl)pyrrolidine-1-carboxylate (300mg) was dissolved in methanol (5mL), followed by adding acetic acid (3 drops), ammonia methanol solution (3.3mg), and sodium cyanoborohydride (241mg). LCMS showed the reaction was completed. The reaction mixture was concentrated directly and separated and purified by rapid chromatography (silica gel, petroleum ether:ethyl acetate=1:1) to give the target compound (100mg). LCMS(ESI)[M+H]⁺=1347.9.

### Step 8: Synthesis of (2S,2'S,2"S)-3,3',3"-((nitrilotris(methylene))tris(benzo[b]thiophene-4,2-diyl))tris(2-((R)-pyrrolidin-3-yl)propanoic acid)

Tri-*tert*-butyl 3,3',3"-((2S,2'S,2"S)-((nitrilotris(methylene)tris(benzo[b]thiophene-4,2-diyl))tris(3-(tert-butoxy)-3-oxopropane-1,2-diyl))(3R,3'R,3"R)-tris(pyrrolidine-1-carboxylate) (90mg) was added in the solution of hydrochloric acid/1,4-dioxane (4mL, 4M) and stirred at room temperature, and the mixture was stirred for 6h. After filtration, acetonitrile and water were added, and the mixture was lyophilized to give the target compound (31mg). LCMS(ESI)[M+H]⁺=879.2; ¹HNMR(400MHz, D₂O) δ 8.06-8.04 (m, 3H), 7.59-7.57 (m, 3H), 7.47-7.43 (m, 3H), 7.39-7.21 (m, 3H), 5.29(s, 3H),4.92-4.91(m, 3H), 4.75-4.72(m, 3H), 3.51-3.46 (m, 3H), 3.43-3.37 (m, 3H), 3.27-3.17 (m, 12H), 2.98-2.93 (m, 3H), 2.72-2.70 (m, 3H), 2.49-2.41(m, 6H), 2.12-2.07 (m, 3H), 1.76-1.70 (m, 3H).

### Example 26 (Compound 102)

### Preparation of (2S,2'S,2"S)-3,3',3"-((nitrilotris(methylene))tris(benzofuran-7,5-diyl))tris(2-((R)-pyrrolidin-3-yl)propanoic acid)

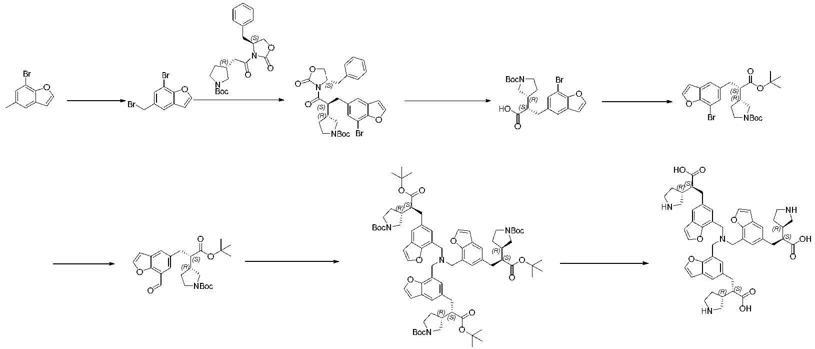

### Step 1: Synthesis of 7-bromo-5-(bromomethyl)benzofuran

7-bromo-5-methylbenzofuran (10g, 47.3mmol, 1.0eq) was dissolved in carbon tetrachloride (100mL), followed by adding benzoyl peroxide (1.15g, 4.73mmol, 0.1eq) and N-bromosuccinimide (16.8g, 94.6mmol, 2.0eq). The reaction was maintained at 25°C for 16h. LCMS showed the reaction was completed. Water was added to the reaction mixture, and the mixture was extracted with ethyl acetate. The organic phase was washed with saturated sodium chloride solution, dried over anhydrous sodium sulfate, and filtered, and the filtrate was concentrated. The mixture was separated and purified by rapid chromatography (silica gel, petroleum ether:ethyl acetate=20:1) to give the title compound (11.5g).

### Step 2: Synthesis of tert-butyl (R)-3-((S)-1-((S)-4-benzyl-2-oxooxazolidin-3-yl)-3-(7-bromobenzofuran-5-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate (11.5g, 29.6mmol, 1.0eq) was dissolved in tetrahydrofuran (100mL) and cooled to -78°C with dry ice-ethanol bath, followed by adding lithium bis(trimethylsilyl)amide (40mL, 1.0M, 1.35eq). The reaction mixture was stirred at -78°C for 30min. 7-bromo-5-(bromomethyl)benzofuran (8.6g, 29.6mmol, 1.0eq) was dissolved in tetrahydrofuran (20mL), and the solution was slowly added dropwise to the above reaction mixture. The reaction mixture was stirred at 25°C for 16h. LCMS showed the reaction was completed. The reaction mixture was added with saturated ammonium chloride aqueous solution (50mL) and extracted with ethyl acetate (100mL×3). The organic phase was washed with saturated saline solution, dried over anhydrous sodium sulfate, filtered. The filtrate was rotary-evaporated to dryness and separated and purified by rapid chromatography (silica gel, petroleum ether:ethyl acetate=5:1) to give the target compound (7.5g). LCMS(ESI)[M+Na]⁺=619.2.

### Step 3: Synthesis of (S)-3-(7-bromobenzofuran-5-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(7-bromobenzofuran-5-yl)-1-oxopropan-2-yl)pyrrolidine-1-carboxylate (7.5g, 12.6mmol, 1.0eq) was dissolved in tetrahydrofuran (80mL) and cooled to 0°C in an ice-water bath, followed by adding hydrogen peroxide (8mL). Lithium hydroxide (603mg, 25.2mmol, 2.0eq) was dissolved in water (15mL), and the solution was slowly added dropwise to the above reaction mixture. After the addition was completed, the solution was allowed to gradually return to 25°C and stirred for 16h. LCMS showed the reaction was completed. The mixture was filtered and concentrated to give the target compound (5.6g). LCMS(ESI)[M-*tert*-butyl]⁺=382.2.

### Step 4: Synthesis of tert-butyl (R)-3-((S)-3-(7-bromobenzofuran-5-yl)-1-(tert-butoxy)-1-oxopropan-2-yl)pyrrolidine-1-carboxylate

(S)-3-(7-bromobenzofuran-5-yl)-2-((R)-1-(tert-butoxycarbonyl)pyrrolidin-3-yl)propanoic acid (5.6g, 12.8mmol, 1.0eq) was dissolved in 2-methyltetrahydrofuran (50mL), followed by adding *tert-*butyl N,N'-diisopropylcarbamimidate (7.7g, 38.4mmol, 3.0eq). The reaction mixture was stirred at 65°C for 16h. LCMS showed the reaction was completed. The mixture was filtered and concentrated to give a crude product, which was separated and purified by rapid chromatography (silica gel, petroleum ether:ethyl acetate=2:1) to give the target compound (3.85g). LCMS(ESI)[M+Na]⁺=516.3.

### Step 5: Synthesis of tert-butyl (R)-3-((S)-1-(tert-butoxy)-3-(7-formylbenzofuran-5-yl)-1-oxopropan-2-yl)pyrrolidine-1-carboxylate

*Tert-*butyl (R)-3-((S)-3-(7-bromobenzofuran-5-yl)-1-(tert-butoxy)-1-oxopropan-2-yl)pyrrolidine-1-carboxylate (2.0g, 4.06mmol, 1.0eq) was dissolved in toluene (20mL), followed by adding palladium acetate (91.6mg, 0.406mmol, 0.1eq), N,N,N',N'-tetramethylethylenediamine (849.4mg, 7.31mmol, 1.8eq), and n-butyldi(1-adamantyl)phosphine (294.1mg, 0.81mmol, 0.2eq). The mixture was charged with carbon monoxide to 1.2 MPa and then hydrogen to 2.4 MPa, heated to 110°C, and stirred for 48h. TLC showed the reaction was completed. The reaction mixture was filtered, concentrated, separated, and purified by rapid chromatography (silica gel, petroleum ether:ethyl acetate=5:1) to give the target compound (1.3g). LCMS(ESI)[M+Na]⁺=466.4.

### Step 6: Synthesis of tri-tert-butyl 3,3',3"-((2S,2'S,2"S)-((nitrilotris(methylene))tris(benzofuran-7,5-diyl))tris(3-(tert-butoxy)-3-oxopropane-1,2-diyl))(3R,3'R,3"R)-tris(pyrrolidine-1-carboxylate)

*Tert-*butyl (R)-3-((S)-1-(tert-butoxy)-3-(7-formylbenzofuran-5-yl)-1-oxopropan-2-yl)pyrrolidine-1-carboxylate (200mg, 0.451mmol, 1.0eq) was dissolved in methanol (2mL), followed by adding ammonia methanol solution (0.1mL, 7.0M) and sodium cyanoborohydride (84mg, 1.354mmol, 3.0eq) at 25°C. The mixture was stirred at 25 for 16h. LCMS showed the reaction was completed. Water and ethyl acetate was added. The mixture was extracted and separated. The organic phase was dried over anhydrous sodium sulfate and filtered. The filtrate was concentrated to give the crude product, which was purified by chromatography (petroleum ether:ethyl acetate=2:1) to give the target compound (110mg). LCMS(ESI)[M+H]⁺=1299.2.

### Step 7: Synthesis of (2S,2'S,2"S)-3,3',3"-((nitrilotris(methylene))tris(benzofuran-7,5-diyl))tris(2-((R)-pyrrolidin-3-yl)propanoic acid)

Tri-*tert*-butyl 3,3',3"-((2S,2'S,2"S)-((nitrilotris(methylene))tris(benzofuran-7,5-diyl))tris(3-(tert-butoxy)-3-oxopropane-1,2-diyl))(3R,3'R,3"R)-tris(pyrrolidine-1-carboxylate) (110mg) was dissolved in hydrochloric acid/1,4-dioxane (5mL) and stirred at 25°C for 3h. LCMS showed the reaction was completed. The reaction mixture was filtered to give the target compound (35.98mg). LCMS(ESI)[M+Na]⁺=853.3; ¹HNMR(EN1298-248-P):(400MHz, D₂O) *δ* 7.48-7.52 (m, 6H), 7.0 (s, 3H), 6.79 (d, *J*=4.8Hz, 3H), 4.77 (s, 6H), 3.56-3.50 (m, 3H),3.36-3.25 (m, 3H), 3.15-3.22 (m, 3H), 2.97-3.03 (m, 3H), 2.79-2.92 (m, 6H), 2.67-2.54 (m, 3H), 2.50-2.38 (m, 3H), 2.11-2.05 (m, 3H), 1.68-1.63 (m, 3H).

### Example 27 (Compound 103)

### Preparation of (2S,2'S,2"S)-3,3',3"-((nitrilotris(methylene))tris(benzo[b]thiophene-6,2-diyl))tris(2-((R)-pyrrolidin-3-yl)propanoic acid)

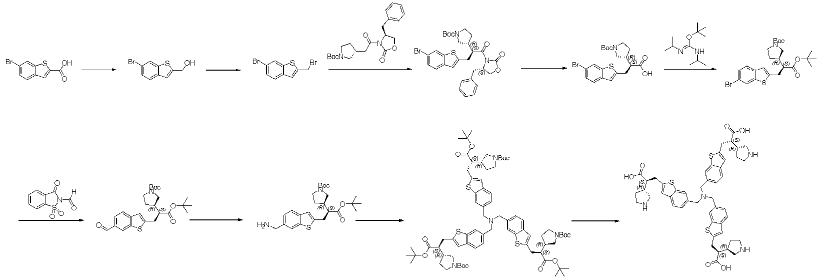

### Step 1: Synthesis of (6-bromobenzo[b]thiophen-2-yl)methanol

6-bromobenzo[b]thiophene-2-carboxylic acid (5g, 19.45mmol) was dissolved in tetrahydrofuran (40mL), followed by adding a solution of borane/tetrahydrofuran (58.34mL, 3.0eq, 3.0 M) at room temperature. The mixture was stirred at room temperature for 16h. TLC showed the reaction of starting material was completed. The reaction mixture was added with a saturated solution of sodium bicarbonate slowly under ice-water bath. The mixture was extracted and separated. The organic phase was dried over anhydrous sodium sulfate, filtered and concentrated to give the crude product, which was separated and purified by rapid chromatography (silica gel, petroleum ether:ethyl acetate=5:1) to give the target compound (4.0g). ¹HNMR( 400MHz, DMSO-*d*₆) δ 8.23-8.18 (m, 1H), 7.72 (d, *J*=8.5Hz, 1H), 7.48 (dd,*J*=8.5, 1.9Hz, 1H), 7.27 (q, *J*=1.1Hz, 1H), 5.70 (s, 1H), 4.73 (s, 2H).

### Step 2: Synthesis of 6-bromo-2-(bromomethyl)benzo[b]thiophene

(6-bromobenzo[b]thiophen-2-yl)methanol (4g, 16.45mmol) was dissolved in dichloromethane (20mL), followed by adding phosphorus tribromide (4454mg, 16.45mmol) at room temperature. The mixture was stirred at room temperature for 16h. TLC showed the reaction of starting material was completed. The reaction mixture was added with a saturated solution of sodium bicarbonate slowly under ice-water bath. The mixture was extracted. The organic phase was dried over anhydrous sodium sulfate, filtered and concentrated to give the crude product, which was separated and purified by rapid chromatography (silica gel, petroleum ether:ethyl acetate=10:1) to give the target compound (3.8g). ¹HNMR(400MHz, DMSO-*d*₆) δ 8.28- 8.23(m, 1H), 7.78 (d, *J*=8.5Hz, 1H), 7.57-7.50 (m, 2H), 5.09 (d, *J*=0.7Hz, 2H).

### Step 3: Synthesis of tert-butyl (R)-3-((S)-1-((S)-4-benzyl-2-oxooxazolidin-3-yl)-3-(6-bromobenzo[b]thiophen-2-yl)-1-oxopropan-2-yl)pyrrolidine-1-carboxylate

A solution of lithium bis(trimethylsilyl)amide (7.45mL, 7.45mmol, 1.2eq, 1.0 M in THF) was added dropwise to the solution of *tert-*butyl (R)-3-(2-((S)-4-benzyl-2-oxooxazolidin-3-yl)-2-oxoethyl)pyrrolidine-1-carboxylate (2.653g, 6.83mmol, 1.1eq) in tetrahydrofuran (20mL) under ice-bath and nitrogen protection. The mixture was stirred at 0°C for 30min. Then a solution of 6-bromo-2-(bromomethyl)benzo[b]thiophene (1.9g, 6.21mmol, 1.0eq) in tetrahydrofuran (10mL) was added slowly. The reaction temperature was increased slowly to room temperature and stirred for 16h. The reaction mixture was cooled with an ice-water bath. Saturated ammonium chloride aqueous solution was added. The mixture was added with water and extracted with ethyl acetate. The organic phase was washed with saturated aqueous sodium chloride, dried over anhydrous sodium sulfate, filtered, and concentrated to give the target compound (2g). LCMS(ESI)[M+H-*tert*-butyl]=557.07.

### Step 4: Synthesis of (S)-3-(6-bromobenzo[b]thiophen-2-yl)-2-((R)-1-(tert-butoxycarbonyl)pyrrolidin-3-yl)propanoic acid

A solution of hydrogen peroxide (333mg, 9.78mmol, 3.0eq) was added once to the solution of *tert-*butyl (R)-3-((S)-1-((S)-4-benzyl-2-oxooxazolidin-3-yl)-3-(6-bromobenzo[b]thiophen-2-yl)-1-oxopropan-2-yl)pyrrolidine-1-carboxylate (2g, 3.26mmol, 1.0eq) in tetrahydrofuran (20mL) and cooled with an ice/water bath, followed by adding a solution of lithium hydroxide monohydrate (274mg, 6.52mmol, 2.0eq). The reaction temperature was increased to room temperature, and the mixture was stirred for 16h. LCMS showed the reaction was completed. The reaction mixture was cooled to 0°C. The sodium hydrogen sulfite (300mg) aqueous solution (10mL) and sodium hydroxide (5N) aqueous solution were added. The pH of the reaction mixture was adjusted to >12. Water (20mL) and methyl *tert-*butyl ether (50mL) were added. The mixture was extracted and separated. The organic phase was discarded; the mixture was cooled to 0°C, the pH of the mixture was adjusted to 3 by the addition of hydrochloric acid (5N), it was extracted with methyl *tert-*butyl ether, and the organic phase was washed with saturated aqueous sodium chloride, dried over anhydrous sodium sulfate, filtered, and concentrated to give the target compound (1.2g). LCMS(ESI)[M+H-*tert*-butyl]⁺=397.8.

### Step 5: Synthesis of tert-butyl (R)-3-((S)-3-(6-bromobenzo[b]thiophen-2-yl)-1-(tert-butoxy)-1-oxopropan-2-yl)pyrrolidine-1-carboxylate

(S)-3-(6-bromobenzo[b]thiophen-2-yl)-2-((R)-1-(tert-butoxycarbonyl)pyrrolidin-3-yl)propanoic acid (1.2g, 2.64mmol, 1eq) was dissolved in 2-methyltetrahydrofuran (20mL), followed by adding *tert-*butyl N,N'-diisopropylcarbamimidate (2.645g, 13.2mmol, 5eq). It was heated to 65°C under nitrogen protection and stirred for 16h. LCMS showed the reaction was completed. The insoluble matter was filtered. The filter cake was washed with methyl *tert-*butyl ether, the filtrate was concentrated, separated and purified by rapid chromatography (silica gel, petroleum ether:ethyl acetate=10:1) to give the target compound (1g). LCMS(ESI)[M+H-2×*tert*-butyl]⁺=397.8.

### Step 6: Synthesis of tert-butyl (R)-3-((S)-1-(tert-butoxy)-3-(6-formylbenzo[b]thiophen-2-yl)-1-oxopropan-2-yl)pyrrolidine-1-carboxylate

*Tert-*butyl (R)-3-((S)-3-(6-bromobenzo[b]thiophen-2-yl)-1-(tert-butoxy)-1-oxopropan-2-yl)pyrrolidine-1-carboxylate (900mg, 1.76mmol, 1.0eq) and N,N-dimethylformamide (10mL) were added in a microwave tube, followed by adding N-formylsaccharin (745mg, 3.53mmol, 2.0eq), palladium acetate (40mg, 0.18mmol, 0.1eq), 1,4-bis(diphenylphosphino)butane (75mg, 0.18mmol, 0.1eq), triethylsilane (410mg, 3.53mmol, 2.0eq), and sodium carbonate (561mg, 5.29mmol, 3.0eq). Nitrogen was replaced 3 times. The mixture was stirred at 70°C for 16h. LCMS showed the reaction was completed. The insoluble matter was filtered through diatomaceous earth. The filter cake was washed with ethyl acetate. The filtrate was concentrated, separated and purified by rapid chromatography (silica gel, petroleum ether:ethyl acetate=5:1) to give the target compound (400mg). LCMS(ESI)[M-*tert*-butyl-Boc]⁺=303.9.

### Step 7: Synthesis of tert-butyl (R)-3-((S)-3-(6-(aminomethyl)benzo[b]thiophen-2-yl)-1-(tert-butoxy)-1-oxopropan-2-yl)pyrrolidine-1-carboxylate

*Tert-*butyl (R)-3-((S)-1-(tert-butoxy)-3-(6-formylbenzo[b]thiophen-2-yl)-1-oxopropan-2-yl)pyrrolidine-1-carboxylate(170mg, 0.37mmol, 1.0eq) was added in ammonia methanol solution (1.5mL, 7.0M) and stirred at room temperature for 0.5h, followed by adding sodium cyanoborohydride (160mg, 2.59mmol, 7.0eq). The mixture was stirred at room temperature for 16h. LCMS showed the reaction was completed. The reaction mixture was concentrated directly, separated and purified by rapid chromatography (silica gel, dichloromethane:methanol=30:1) to give the target compound (50mg). LCMS(ESI)[M+H]⁺=461.0.

### Step 8: Synthesis of tri-tert-butyl 3,3',3"-((2S,2'S,2"S)-((nitrilotris(methylene))tris(benzo[b]thiophene-6,2-diyl))tris(3-(tert-butoxy)-3-oxopropane-1,2-diyl))(3R,3'R,3"R)-tris(pyrrolidine-1-carboxylate)

*Tert-*butyl (R)-3-((S)-3-(6-(aminomethyl)benzo[b]thiophen-2-yl)-1-(tert-butoxy)-1-oxopropan-2-yl)pyrrolidine-1-carboxylate (50mg) and *tert-*butyl (R)-3-((S)-1-(tert-butoxy)-3-(6-formylbenzo[b]thiophen-2-yl)-1-oxopropan-2-yl)pyrrolidine-1-carboxylate (100mg) were dissolved in methanol (5mL) and stirred at room temperature for 0.5h, followed by adding sodium cyanoborohydride (13mg). The mixture was stirred at room temperature for 16h. LCMS showed the reaction was completed. The reaction mixture was concentrated, diluted with ethyl acetate, washed with saturated sodium bicarbonate solution and saturated saline solution successively, dried over anhydrous sodium sulfate, and filtered. The organic phase was concentrated, separated and purified by rapid chromatography (silica gel, dichloromethane:methanol=40:1) to give the target compound (70mg). LCMS(ESI)[M+H]⁺=1347.2.

### Step 9: Synthesis of (2S,2'S,2"S)-3,3',3"-((nitrilotris(methylene))tris(benzo[b]thiophene-6,2-diyl))tris(2-((R)-pyrrolidin-3-yl)propanoic acid)

Tri-*tert*-butyl 3,3',3"-((2S,2'S,2"S)-((nitrilotris(methylene))tris(benzo[b]thiophene-6,2-diyl))tris(3-(tert-butoxy)-3-oxopropane-1,2-diyl))(3R,3'R,3"R)-tris(pyrrolidine-1-carboxylate) (70mg) was added in hydrochloric acid/1,4-dioxane (3mL) and stirred at room temperature for 16h. LCMS showed the reaction was completed. The reaction mixture was concentrated directly. The concentrate was separated and purified by Prep-HPLC to give the target compound (31.90mg). LCMS(ESI)[M+H]⁺=879.0; ¹HNMR(400MHz, D₂O) δ 7.56 (d, *J*=8.3Hz, 3H), 7.53-7.46 (m, 3H), 7.13-7.03 (m, 6H), 4.29 (s, 6H), 3.56 (dd, *J*=11.8, 7.9Hz, 3H), 3.39 (ddd, *J*=11.9, 8.5, 3.4Hz, 3H), 3.23-3.02 (m, 12H), 2.83 (dt, *J*=9.6, 4.6Hz, 3H), 2.56 (q, *J*=8.5Hz, 3H), 2.14 (dtd, *J*=13.7, 7.1, 3.4Hz, 3H), 1.80-1.70 (m, 3H).

### Example 28 (Compound 104)

### Preparation of (2S,2'S,2"S)-3,3',3"-((nitrilotris(methylene))tris(benzo[b]thiophene-5,2-diyl))tris(2-((R)-pyrrolidin-3-yl)propanoic acid)

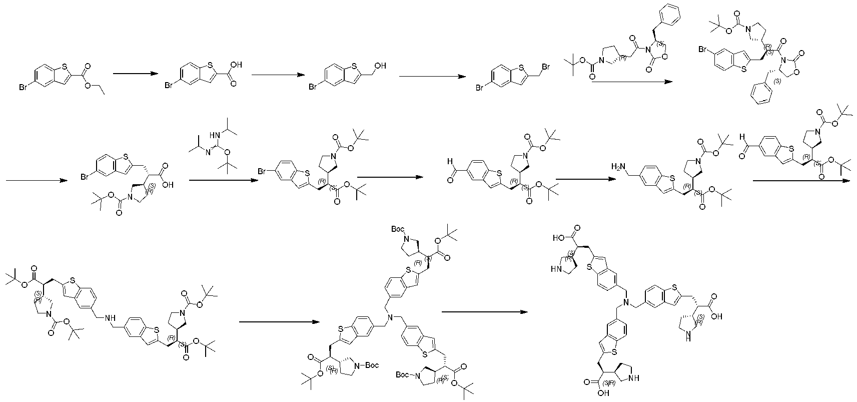

### Step 1: Synthesis of 5-bromobenzo[b]thiophene-2-carboxylic acid

Ethyl 5-bromobenzo[b]thiophene-2-carboxylate (4.5g) was dissolved in a mixed solution of THF (10mL) and water (10mL), followed by adding lithium hydroxide (2.0g). The mixture was stirred at room temperature for 3h. The pH of the reaction mixture was adjusted to 3 with dilute hydrochloric acid (1.0M) solution. The mixture was filtered to give the target compound (3.5g). ¹HNMR(400MHz, CDCl₃) δ 8.27 (d, J=1.9Hz, 1H), 8.08 (s, 1H), 8.04 (d, J=8.7Hz, 1H), 7.65 (dd, J=8.7, 2.0Hz, 1H).

### Step 2: Synthesis of (5-bromobenzo[b]thiophen-2-yl)methanol

5-bromobenzo[b]thiophene-2-carboxylic acid (200mg) was dissolved in THF (15.0mL). The reaction mixture was cooled to 0°C. A solution of BH₃ in THF (1.0 M, 2.33mL) was added dropwise. Then the reaction was stirred for 2h. Methanol was added to quench the reaction. The reaction mixture was concentrated to give the crude product. Ethyl acetate was added to the crude product and filtered. The filtrate was concentrated to give the target compound (1.6g). ¹HNMR(400MHz, CDCl₃) δ 8.01 (d, J=1.8Hz, 1H), 7.90 (d, J=8.6Hz, 1H), 7.43 (dd, J=8.5, 1.8Hz, 1H), 7.25 (s, 1H), 5.72 (t, J=5.7Hz, 1H), 4.75 (d, J=5.8Hz, 2H).

### Step 3: Synthesis of 5-bromo-2-(bromomethyl)benzo[b]thiophene

(5-bromobenzo[b]thiophen-2-yl)methanol (2.5g) was dissolved in DCM (20mL), followed by adding HBr (15mL, 48%) at room temperature. The reaction mixture was heated to 45°C and stirred for 16h. TLC showed the reaction of starting material was completed. The reaction mixture was slowly quenched with saturated sodium bicarbonate solution in an ice-water bath and extracted with ethyl acetate. The organic phase was dried over anhydrous sodium sulfate, filtered, and concentrated to give the crude product, which was separated and purified by rapid chromatography (silica gel, petroleum ether:ethyl acetate=10:1) to give the target compound (2.7g). ¹HNMR(400MHz, CDCl₃) δ 8.08 (d, J=1.9Hz, 1H), 7.93 (d, J=8.6Hz, 1H), 7.52 (dd, J=8.6, 1.9Hz, 2H), 5.10 (s, 2H).

### Step 4: Synthesis of tert-butyl (R)-3-((S)-1-((S)-4-benzyl-2-oxooxazolidin-3-yl)-3-(5-bromobenzo[b]thiophen-2-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate (609mg) was dissolved in anhydrous THF (10mL). The reaction mixture was cooled to 0°C. Lithium bis(trimethylsilyl)amide (1.83mL, 1.0M) was added dropwise at 0°C. The mixture was stirred for 30min at the same temperature. A solution of 5-bromo-2-(bromomethyl)benzo[b]thiophene (400mg) in THF (5mL) was added dropwise. The reaction mixture was warmed to room temperature and stirred for 16h. LCMS showed the reaction was completed. The reaction mixture was quenched with saturated ammonium chloride aqueous solution at 0°C in an ice-water bath, extracted with ethyl acetate, dried, filtered, and concentrated to give the crude product, which was separated and purified by rapid chromatography (silica gel, petroleum ether:ethyl acetate=2:1) to give the target compound (900mg). LCMS(ESI)[M+H-Boc]⁺=513.13.

### Step 5: Synthesis of (S)-3-(5-bromobenzo[b]thiophen-2-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(5-bromobenzo[b]thiophen-2-yl)-1-oxopropan-2-yl)pyrrolidine-1-carboxylate (200mg) was dissolved in THF (10mL). The reaction mixture was cooled to 0°C. H₂O₂/H₂O (47.5mg) was added and stirred for 15 min. The mixture was stirred for another 3h. LCMS showed the reaction was completed. The reaction mixture was quenched with the sodium bisulfite (75mg) aqueous solution slowly in an ice-water bath. The pH was adjusted to 3. The mixture was extracted with ethyl acetate, dried, filtered, and concentrated to give the target compound (148mg, crude product) which was used directly in the next step. LCMS(ESI)[M+Na]⁺=476.19

### Step 6: Synthesis of tert-butyl (R)-3-((S)-3-(5-bromobenzo[b]thiophen-2-yl)-1-(tert-butoxy)-1-oxopropan-2-yl)pyrrolidine-1-carboxylate

(S)-3-(5-bromobenzo[b]thiophen-2-yl)-2-((R)-1-(tert-butoxycarbonyl)pyrrolidin-3-yl)propanoic acid (250mg) was dissolved in 2-methyltetrahydrofuran (10mL), followed by adding *tert-*butyl N,N'-diisopropylcarbamimidate (551mg). The mixture was stirred at room temperature for 15 min. The reaction mixture was stirred at 65°C overnight. TLC showed the reaction was completed. The reaction mixture was filtered. The filter cake was washed with *tert*-butyl ether. The combined filtrates were concentrated to give the crude product, which was separate and purified by rapid chromatography (silica gel, petroleum ether:ethyl acetate=3:1) to give the target compound (140mg). LCMS(ESI)[M+H]⁺=510.12.

### Step 7: Synthesis of tert-butyl (R)-3-((S)-1-(tert-butoxy)-3-(5-formylbenzo[b]thiophen-2-yl)-1-oxopropan-2-yl)pyrrolidine-1-carboxylate

*Tert-*butyl (R)-3-((S)-3-(5-bromobenzo[b]thiophen-2-yl)-1-(tert-butoxy)-1-oxopropan-2-yl)pyrrolidine-1-carboxylate (240mg), palladium acetate (21.1mg), 1,4-bis(diphenylphosphino)butane (40mg), N-formylsaccharin (298mg), sodium carbonate (242mg), and triethylsilane (82mg) were dissolved in DMF (5mL). The reaction mixture was heated to 75°C and stirred for 16h under nitrogen protection. LCMS showed the reaction was completed. The reaction mixture was quenched with water and extracted with ethyl acetate. The organic phase was dried over anhydrous sodium sulfate, filtered, and concentrated to give the crude product, which was separated and purified by rapid chromatography (silica gel, petroleum ether: ethyl acetate=3:1) to give the target product (100mg). LCMS(ESI)[M+H]⁺=460.2.

### Step 8: Synthesis of tert-butyl (R)-3-((S)-3-(5-(aminomethyl)benzo[b]thiophen-2-yl)-1-(tert-butoxy)-1-oxopropan-2-yl)pyrrolidine-1-carboxylate

*Tert-*butyl (R)-3-((S)-1-(tert-butoxy)-3-(5-formylbenzo[b]thiophen-2-yl)-1-oxopropan-2-yl)pyrrolidine-1-carboxylate (50mg) was dissolved in methanol (2mL), followed by adding ammonia methanol (0.19mL, 7M). The mixture was stirred at room temperature for 30min. sodium cyanoborohydride (47.1mg) was added and stirred at 60°C for 1.5h under microwave irradiation. LCMS showed the reaction was completed. The reaction mixture was filtered. The filtrate was concentrated, separated and purified by reversed-phase column chromatography (C₁₈, water:acetonitrile=40%) to give the target compound (30mg). LCMS(ESI)[M+H]⁺=461.36.

### Step 9: Synthesis of di-tert-butyl 3,3'-((2S,2'S)-((azanediylbis(methylene))bis(benzo[b]thiophene-5,2-diyl))bis(3-(tert-butoxy)-3-oxopropane-1,2-diyl))(3R,3'R)-bis(pyrrolidine-1-carboxylate)

*Tert-*butyl (R)-3-((S)-3-(5-(aminomethyl)benzo[b]thiophen-2-yl)-1-(tert-butoxy)-1-oxopropan-2-yl)pyrrolidine-1-carboxylate (20mg) and *tert-*butyl (R)-3-((S)-1-(tert-butoxy)-3-(5-formylbenzo[b]thiophen-2-yl)-1-oxopropan-2-yl)pyrrolidine-1-carboxylate (20mg) were dissolved in THF (3.0mL). The mixture was stirred at room temperature for 3h under nitrogen protection. Then, sodium cyanoborohydride (5.65mg) was added to the reaction mixture to continue stirring for 13h. LCMS showed the reaction was completed. The reaction mixture was quenched with water and extracted with ethyl acetate. The organic phase was dried over anhydrous sodium sulfate, filtered, and concentrated to give the crude product, which was purified by rapid chromatography (silica gel, petroleum ether:ethyl acetate=3:1) to give the target compound (20mg). LCMS(ESI)[M+H]⁺=904.26.

### Step 10: Synthesis of tri-tert-butyl 3,3',3"-((2S,2'S,2"S)-((nitrilotris(methylene))tris(benzo[b]thiophene-5,2-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((azanediylbis(methylene))bis(benzo[b]thiophene-5,2-diyl))bis(3-(tert-butoxy)-3-oxopropane-1,2-diyl))(3R,3'R)-bis(pyrrolidine-1-carboxylate) (20mg), *tert-*butyl (R)-3-((S)-1-(tert-butoxy)-3-(5-formylbenzo[b]thiophen-2-yl)-1-oxopropan-2-yl)pyrrolidine-1-carboxylate (10mg), and one drop of acetic acid were dissolved in THF (3.0mL). The mixture was stirred at room temperature for 3h under nitrogen protection. sodium cyanoborohydride (3.0mg) was added. The mixture was stirred for another 13h. Water was added to the reaction mixture to quench the reaction. The mixture was extracted, dried, filtered, and concentrated to give the crude product, which was separated and purified by rapid chromatography (silica gel, petroleum ether:ethyl acetate=3:1) to give the target compound (20mg). LCMS(ESI)[M+H]⁺=1347.80

### Step 11: Synthesis of (2S,2'S,2"S)-3,3',3"-((nitrilotris(methylene))tris(benzo[b]thiophene-5,2-diyl))tris(2-((R)-pyrrolidin-3-yl)propanoic acid)

Tri-*tert*-butyl 3,3',3"-((2S,2'S,2"S)-((nitrilotris(methylene))tris(benzo[b]thiophene-5,2-diyl))tris(3-(tert-butoxy)-3-oxopropane-1,2-diyl))(3R,3'R,3"R)-tris(pyrrolidine-1-carboxylate) (20mg) was dissolved in dichloromethane (4mL), followed by adding trifluoroacetic acid (17.1mg). The reaction was maintained at room temperature overnight. The reaction mixture was concentrated, and the crude product was separated and purified by Prep-HPLC to give the target compound (5mg). LCMS(ESI)(M+H)⁺=879.1; ¹HNMR(400MHz, D₂O)δ7.62 (d,J=8.3Hz, 3H), 7.40 (s, 3H), 6.98 (d,J=8.2Hz, 3H), 6.92 (s, 3H),4.29 (s, 6H), 3.54-3.41 (m, 3H), 3.36-3.24 (m, 3H), 3.20 -2.90 (m, 14H), 2.75-2.62 (m, 3H), 2.51-2.38 (m, 3H), 2.11-1.98 (m, 3H), 1.73 -1.59 (m, 3H).

### Example 29 (Compound 105)

### Preparation of (2S,2'S)-3,3'-((((3-((S)-2-carboxy-2-((R)-pyrrolidin-3-yl)ethyl)benzyl)azanediyl)bis(methylene))bis(3,1-phenylene))bis(2-((R)-pyrrolidin-3-yl)propanoic-3,3-d2 acid)

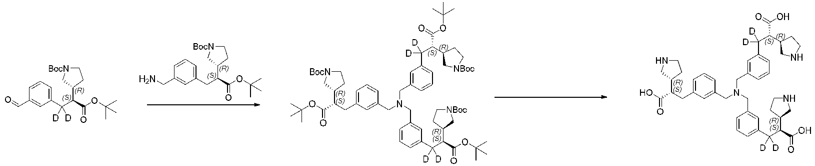

### Step 1: Synthesis of di-tert-butyl 3,3'-((2S,2'S)-((((3-((S)-3-(tert-butoxy)-2-((R)-1-(tert-butoxycarbonyl)pyrrolidin-3-yl)-3-oxopropyl)benzyl)azanediyl)bis(methylene))bis(3,1-phenylene))bis(3-(tert-butoxy)-3-oxopropane-1,2-diyl-1,1-d₂))(3R,3'R)-bis(pyrrolidine-1-carboxylate)

*Tert-*butyl (R)-3-((S)-3-(3-(aminomethyl)phenyl)-1-(tert-butoxy)-1-oxopropan-2-yl)pyrrolidine-1-carboxylate (48mg, 0.12mmol, 1eq) was added a solution of *tert-*butyl (R)-3-((S)-1-(tert-butoxy)-3-(3-formylphenyl)-1-oxopropan-2-yl-3,3-*d₂*)pyrrolidine-1-carboxylate (110mg, 0.27mmol, 2.2eq) in tetrahydrofuran (1mL), followed by the dropwise addition of one drop of acetic acid. The mixture was stirred at room temperature for 0.5h. sodium cyanoborohydride (18mg, 0.3mmol, 2.5eq) was added. The reaction mixture was stirred at room temperature overnight. TLC showed the reaction was completed. Saturated ammonium chloride solution was added to quench the reaction. The mixture was extracted and separated with ethyl acetate and water. The organic phase was dried over anhydrous sodium sulfate, filtered, and concentrated to give the crude product, which was purified by column chromatography to give the target compound. LCMS(ESI)[M+H]⁺=1184.81.

Step 2: Synthesis of (2S,2'S)-3,3'-((((3-((S)-2-carboxy-2-((R)-pyrrolidin-3-yl)ethyl)benzyl)azanediyl)bis(methylene))bis(3,1-phenylene))bis(2-((R)-pyrrolidin-3-yl)propanoic-3,3-d2 acid)Di-*tert*-butyl 3,3'-((2S,2'S)-((((3-((S)-3-(tert-butoxy)-2-((R)-1-(tert-butoxycarbonyl)pyrrolidin-3-yl)-3-oxopropyl)benzyl)azanediyl)bis(methylene))bis(3,1-phenylene))bis(3-(tert-butoxy)-3-oxopropane-1,2-diyl-1,1-*d₂*))(3R,3'R)-bis(pyrrolidine-1-carboxylate) (60mg, 0.05mmol, 1eq) was dissolved in 1,4-dioxane (2mL), followed by adding hydrochloric acid/1,4-dioxane (2mL). The mixture was stirred at room temperature overnight. LCMS showed the reaction was completed. The solvent was removed by rotary-evaporation to dryness, and the residue was separated and purified by Prep-HPLC (Cis, 10mmol/L NH₄HCO₃ in water, MeCN) to give the target compound (61mg). LCMS(ESI)[M+H]⁺=715.68; ¹HNMR(400MHz, D₂O) δ 7.29-7.21 (m, 3H), 7.20-7.05 (m, 9H), 3.72-3.41 (m, 6H), 3.37-3.23 (m, 6H), 3.18-3.04 (m, 3H), 2.89-2.64 (m, 5H), 2.44-2.27 (m, 6H), 2.13-1.94 (m, 3H), 1.72-1.57 (m, 3H).

### Example 30 (Compound 106)

### Preparation of (S)-3-(3-(2-(3-((S)-2-carboxy-2-((R)-pyrrolidin-3-yl)ethyl)-N-(2-(3-((S)-2-carboxy-2-((R)-pyrrolidin-3-yl)ethyl)phenoxy)ethyl)benzamido)ethyl)phenyl)-2-((R)-pyrrolidin-3-yl)propanoic acid

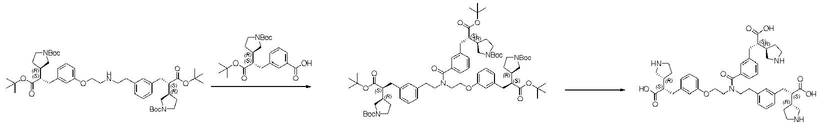

### Step 1: Synthesis of tert-butyl (R)-3-((S)-1-(tert-butoxy)-3-(3-((3-((S)-3-(tert-butoxy)-2-((R)-1-(tert-butoxycarbonyl)pyrrolidin-3-yl)-3-oxopropyl)phenethyl)(2-(3-((S)-3-(tert-butoxy)-2-((R)-1-(tert-butoxycarbonyl)pyrrolidin-3-yl)-3-oxopropyl)phenoxy)ethyl)carbamoyl)phenyl)-1-oxopropan-2-yl)pyrrolidine-1-carboxylate

*Tert-*butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)ethyl)phenyl)-1-oxopropan-2-yl)pyrrolidine-1-carboxylate (120mg, 0.14mmol, 1.0eq) was dissolved in N,N-dimethylformamide (10mL), followed by adding 3-((S)-3-(tert-butoxy)-2-((R)-1-(tert-butoxycarbonyl)pyrrolidin-3-yl)-3-oxopropyl)benzoic acid (72mg, 0.17mmol, 1.2eq), O-(7-azabenzotriazol-1-yl)-N,N,N',N'-tetramethyluronium hexafluorophosphate (109mg, 0.29mmol, 2.0eq), and *N,N*-diisopropylethylamine (56mg, 0.43mmol, 3.0eq) at room temperature. The reaction mixture was stirred at room temperature for 16h. LCMS showed the reaction was completed. The reaction mixture was diluted with water and extracted with ethyl acetate. The organic phase was dried, filtered, and concentrated to give the crude product, which was separated and purified by rapid chromatography (silica gel, petroleum ether:ethyl acetate=1:8) to give the target compound (70mg). LCMS(ESI)[M+H]⁺=1237.9.

### Step 2: Synthesis of (S)-3-(3-(2-(3-((S)-2-carboxy-2-((R)-pyrrolidin-3-yl)ethyl)-N-(2-(3-((S)-2-carboxy-2-((R)-pyrrolidin-3-yl)ethyl)phenoxy)ethyl)benzamido)ethyl)phenyl)-2-((R)-pyrrolidin-3-yl)propanoic acid

*Tert-*butyl (R)-3-((S)-1-(tert-butoxy)-3-(3-((3-((S)-3-(tert-butoxy)-2-((R)-1-(tert-butoxycarbonyl)pyrrolidin-3-yl)-3-oxopropyl)phenethyl)(2-(3-((S)-3-(tert-butoxy)-2-((R)-1-(tert-butoxycarbonyl)pyrrolidin-3-yl)-3-oxopropyl)phenoxy)ethyl)carbamoyl)phenyl)-1-oxopropan-2-yl)pyrrolidine-1-carboxylate (70mg, 0.06mmol) was dissolved in hydrochloric acid/1,4-dioxane (4mL) and stirred at room temperature for 16h. LCMS showed the reaction was completed. The reaction mixture was concentrated directly, separated and purified by preparative chromatography to give the target compound (11.7mg). LCMS(ESI)[M+H]⁺=769.5; ¹HNMR(400MHz, D₂O) δ 7.31-6.91 (m, 7H), 6.89-6.65 (m, 4H), 6.49 (d, *J*=85.2Hz, 1H), 4.35 (s, 1H), 4.12 (t, *J*=5.3Hz, 1H), 4.01 (t, *J*=5.3Hz, 1H), 3.96-3.73 (m, 2H), 3.55 (dt, *J*=29.9, 6.6Hz, 3H), 3.43-3.26 (m, 6H), 3.20-3.08 (m, 3H), 2.86-2.62 (m, 9H), 2.47-2.23 (m, 6H), 2.07 (s, 3H), 1.67 (d, *J*=12.1Hz, 3H).

### Example 31 (Compound 107)

### Preparation of (2S,2'S)-3,3'-((((2-(3-((S)-2-carboxy-2-((R)-pyrrolidin-3-yl)ethyl)phenyl)acetyl)azanediyl)bis(ethane-2,1-diyl))bis(3,1-phenylene))bis(2-((R)-pyrrolidin-3-yl)propanoic acid)

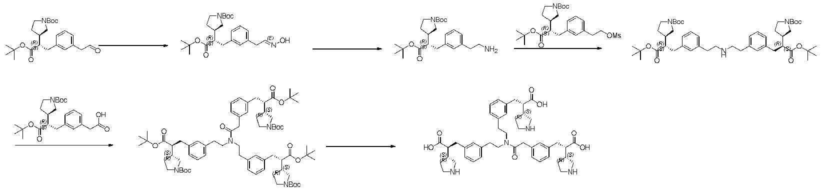

### Step 1: Synthesis of tert-butyl (R)-3-((S)-1-(tert-butoxy)-3-(3-((E)-2-(hydroxyimino)ethyl)phenyl)-1-oxopropan-2-yl)pyrrolidine-1-carboxylate

*Tert-*butyl (R)-3-((S)-1-(tert-butoxy)-1-oxo-3-(3-(2-oxoethyl)phenyl)propan-2-yl)pyrrolidine-1-carboxylate (1000mg) was dissolved in ethanol (20mL), followed by adding hydroxylamine hydrochloride (250mg). The mixture was stirred at room temperature for 16h under nitrogen protection. LCMS showed the reaction was completed. The reaction mixture was added with water, extracted with ethyl acetate, and concentrated to give the crude product, which was separated and purified by rapid chromatography (silica gel, petroleum ether:ethyl acetate=2:1) to give the target compound (900mg). LCMS(ESI)[M+H]⁺=433.3

### Step 2: Synthesis of tert-butyl (R)-3-((S)-3-(3-(2-aminoethyl)phenyl)-1-(tert-butoxy)-1-oxopropan-2-yl)pyrrolidine-1-carboxylate

The product from the first step (900mg) was dissolved in methanol (10mL), followed by adding Pd/C (150mg). The mixture was stirred at room temperature for 16h under hydrogen. LCMS showed the reaction was completed. The reaction mixture was directly concentrated to give the crude product, which was separated and purified by rapid chromatography (silica gel, petroleum ether:ethyl acetate=1:1) to give the target compound (600mg). LCMS(ESI)[M+H]⁺=419.3

### Step 3: Synthesis of di-tert-butyl 3,3'-((2S,2'S)-((azanediylbis(ethane-2,1-diyl))bis(3,1-phenylene))bis(3-(tert-butoxy)-3-oxopropane-1,2-diyl))(3R,3'R)-bis(pyrrolidine-1-carboxylate)

The product from the second step (219mg) was dissolved in acetonitrile (10mL), followed by adding *tert-*butyl (R)-3-((S)-1-(tert-butoxy)-3-(3-(2-((methylsulfonyl)oxy)ethyl)phenyl)-1-oxopropan-2-yl)pyrrolidine-1-carboxylate (200mg) and potassium carbonate (78mg). The reaction mixture was stirred at 80°C for 16h. LCMS showed the reaction was completed. The reaction mixture was concentrated to give the crude product, which was separated and purified by rapid chromatography (silica gel, petroleum ether:ethyl acetate=1:1) to give the target compound (240mg). LCMS(ESI)[M+H]⁺=820.6.

### Step 4: Synthesis of di-tert-butyl 3,3'-((2S,2'S)-((((2-(3-((S)-3-(tert-butoxy)-2-((R)-1-(tert-butoxycarbonyl)pyrrolidin-3-yl)-3-oxopropyl)phenyl)acetyl)azanediyl)bis(ethane-2,1-diyl))bis(3,1-phenylene))bis(3-(tert-butoxy)-3-oxopropane-1,2-diyl))(3R,3'R)-bis(pyrrolidine-1-carboxylate)

The product from the third step (120mg) was dissolved in N,N-dimethylformamide (10mL), followed by adding 2-(3-((S)-3-(tert-butoxy)-2-((R)-1-(tert-butoxycarbonyl)pyrrolidin-3-yl)-3-oxopropyl)phenyl)acetic acid (70mg), O-(7-azabenzotriazol-1-yl)-N,N,N',N'-tetramethyluronium hexafluorophosphate (56mg), and *N,N*-diisopropylethylamine (28mg). The reaction mixture was stirred at room temperature for 16h. LCMS showed the reaction was completed. The reaction mixture was diluted with water and extracted with ethyl acetate. The organic phase was dried over anhydrous sodium sulfate, filtered, and concentrated to give the crude product, which was separated and purified by rapid chromatography (silica gel, petroleum ether:ethyl acetate=1:8) to give the target compound (70mg). LCMS(ESI)[M+H]⁺=1235.8

### Step 5: Synthesis of (2S,2'S)-3,3'-((((2-(3-((S)-2-carboxy-2-((R)-pyrrolidin-3-yl)ethyl)phenyl)acetyl)azanediyl)bis(ethane-2,1-diyl))bis(3,1-phenylene))bis(2-((R)-pyrrolidin-3-yl)propanoic acid)

The product from the fourth step (70mg) was dissolved in hydrochloric acid/1,4-dioxane (4mL) and stirred at room temperature for 16h. LCMS showed the reaction was completed. The reaction mixture was concentrated directly, separated and purified by preparative chromatography to give the target compound (29.99mg). LCMS(ESI)[M+H]⁺=767.4; ¹HNMR(400MHz, D₂O) δ 7.27-7.14 (m, 3H), 7.12-7.02 (m, 3H), 6.99-6.91 (m, 4H), 6.75-6.68 (m, 2H), 3.49 (dt, *J*=11.1, 7.6Hz, 4H), 3.43-3.31 (m, 4H), 3.25 (t, *J*=6.4Hz, 2H), 3.21-3.05 (m, 5H), 3.00-2.92 (m, 3H), 2.86-2.73 (m, 8H), 2.65 (q, *J*=7.2, 6.7Hz, 5H), 2.50-2.37 (m, 3H), 2.10 (d, *J*=7.6Hz, 3H), 1.70 (dq, *J*=12.9, 3.5Hz, 3H).

### Example 32 (Compound 108)

### Preparation of (2S,2'S)-3,3'-((((3-((S)-2-carboxy-2-((R)-pyrrolidin-3-yl)ethyl)benzoyl)azanediyl)bis(ethane-2,1-diyl))bis(3,1-phenylene))bis(2-((R)-pyrrolidin-3-yl)propanoic acid)

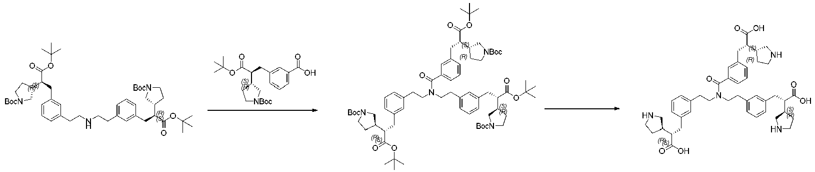

### Step 1: Synthesis of di-tert-butyl 3,3'-((2S,2'S)-((((3-((S)-3-(tert-butoxy)-2-((R)-1-(tert-butoxycarbonyl)pyrrolidin-3-yl)-3-oxopropyl)benzoyl)azanediyl)bis(ethane-2,1-diyl))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-((azanediylbis(ethane-2,1-diyl))bis(3,1-phenylene))bis(3-(tert-butoxy)-3-oxopropane-1,2-diyl))(3R,3'R)-bis(pyrrolidine-1-carboxylate) (120mg) was dissolved in N,N-dimethylformamide (10mL), followed by adding 3-((S)-3-(tert-butoxy)-2-((R)-1-(tert-butoxycarbonyl)pyrrolidin-3-yl)-3-oxopropyl)benzoic acid (74mg), O-(7-azabenzotriazol-1-yl)-N,N,N',N'-tetramethyluronium hexafluorophosphate (111mg), and *N,N*-diisopropylethylamine (57mg). The reaction mixture was stirred at room temperature for 16h. LCMS showed the reaction was completed. The reaction mixture was diluted with water and extracted with ethyl acetate. The organic phase was dried over anhydrous sodium sulfate and concentrated to give the crude product, which was separated and purified by rapid chromatography (silica gel, petroleum ether:ethyl acetate=1:8) to give the target compound (70mg). LCMS(ESI)[M+H]⁺=1221.9

### Step 2: Synthesis of (2S,2'S)-3,3'-((((3-((S)-2-carboxy-2-((R)-pyrrolidin-3-yl)ethyl)benzoyl)azanediyl)bis(ethane-2,1-diyl))bis(3,1-phenylene))bis(2-((R)-pyrrolidin-3-yl)propanoic acid)

Di-*tert*-butyl 3,3'-((2S,2'S)-((((3-((S)-3-(tert-butoxy)-2-((R)-1-(tert-butoxycarbonyl)pyrrolidin-3-yl)-3-oxopropyl)benzoyl)azanediyl)bis(ethane-2,1-diyl))bis(3,1-phenylene))bis(3-(tert-butoxy)-3-oxopropane-1,2-diyl))(3R,3'R)-bis(pyrrolidine-1-carboxylate) (70mg) was dissolved in hydrochloric acid/1,4-dioxane (4mL) and stirred at room temperature for 16h. LCMS showed the reaction was completed. The reaction mixture was concentrated directly, separated and purified by preparative chromatography to obtain the target compound (8.41mg). LCMS(ESI)[M+H]⁺=753.5; ¹HNMR(400MHz, D₂O) δ 7.26 (t, *J*=7.5Hz, 1H), 7.17-7.13 (m, 4H), 7.09 (dd, *J*=7.6, 4.0Hz, 2H), 7.04 (d, *J*=8.0Hz, 1H), 6.72-6.61 (m, 2H), 6.48 (d, *J*=3.8Hz, 1H), 6.30 (s, 1H), 3.76 (dq, *J*=20.4, 6.8Hz, 2H), 3.39-3.26 (m, 8H), 3.19-3.08 (m, 3H), 2.95 (t, *J*=6.9Hz, 2H), 2.86-2.77 (m, 4H), 2.68 (dd, *J*=13.8, 7.0Hz, 3H), 2.58 (q, *J*=5.0Hz, 3H), 2.48-2.22 (m, 7H), 2.04 (td, *J*=6.3, 3.5Hz, 3H), 1.72-1.60 (m, 3H).

### Example 33 (Compound 109)

### Preparation of (2S,2'S,2"S)-3,3',3"-((nitrilotris(methylene))tris(benzofuran-3,6-diyl))tris(2-((R)-pyrrolidin-3-yl)propanoic acid)

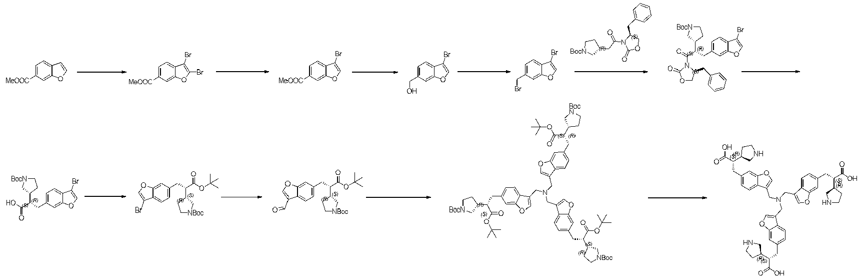

### Step 1: Synthesis of methyl 2,3-dibromobenzofuran-6-carboxylate

Methyl benzofuran-6-carboxylate (5.0g, 28.4mmol, 1eq) was dissolved in dichloromethane (50mL), followed by adding bromine (5.1g, 31.2mmol, 1.1eq). The mixture was stirred at -10°C for 1h. TLC showed the reaction was completed. A saturated solution of Na₂S₂O₃ was added. The mixture was extracted with dichloromethane. The organic phases were combined, dried over anhydrous sodium sulfate, filtered, and concentrated to give the target compound (8.0g). LCMS(ESI)[M+H]⁺=332.6.

### Step 2: Synthesis of methyl 3-bromobenzofuran-6-carboxylate

Methyl 2,3-dibromobenzofuran-6-carboxylate (8g, 23.95mmol, 1eq) was dissolved in a mixed solvent of THF (100mL) and MeOH (20mL), followed by adding cesium carbonate (15.61g, 47.91mmol, 2eq). The mixture was stirred at room temperature for 2.5h. TLC showed the reaction was completed. The mixture was filtered. The filtrate was added with water and extracted with ethyl acetate. The organic phase was dried over anhydrous sodium sulfate and filtered, and the filtrate was concentrated to give the crude product, which was purified by column chromatography (petroleum ether:ethyl acetate=10:1) and slurried to give the target compound (6.4g). LCMS(ESI)[M+H]⁺=254.9.

### Step 3: Synthesis of (3-bromobenzofuran-6-yl)methanol

Methyl 3-bromobenzofuran-6-carboxylate (6.4g, 25.09mmol, 1eq) was dissolved in THF (80mL), followed by adding lithium borohydride (2732.53mg, 125.46mmol, 5eq) under nitrogen protection. The mixture was stirred at 30°C for 6h. LCMS showed the reaction was completed. Water was added. The mixture was extracted with ethyl acetate. The organic phases were combined, washed with saturated sodium chloride, dried over anhydrous sodium sulfate, filtered and concentrated to give the crude product. The crude product was slurried by the mixed solvent (petroleum ether:ethyl acetate=10:1) to give the target compound (5.6g).

### Step 4: Synthesis of 3-bromo-6-(bromomethyl)benzofuran

(3-bromobenzofuran-6-yl)methanol (5.6g, 24.70mmol, 1eq) was dissolved in dichloromethane (100mL), followed by adding hydrobromic acid aqueous solution (20mL, 40 %); the tube was sealed and stirred at 45°C for 16h; TLC (petroleum ether 100 %) showed the reaction was completed. Saturated sodium carbonate solution (150mL) was added to neutralize. The mixture was extracted with dichloromethane. The organic phase was dried over anhydrous sodium sulfate and filtered. The filtrate was concentrated to give the target compound (5.4g).

### Step 5: Synthesis of tert-butyl (R)-3-((S)-1-((S)-4-benzyl-2-oxooxazolidin-3-yl)-3-(3-bromobenzofuran-6-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate (5.31g, 13.66mmol, 1.1eq) was dissolved in tetrahydrofuran (50mL). The mixture was cooled to 0°C in an ice bath, and lithium bis(trimethylsilyl)amide (16.14mL, 16.14mmol, 1.3eq, 1.0M) was added dropwise. Then, the mixture was stirred for 30 min, and a solution of 3-bromo-6-(bromomethyl)benzofuran (3.6g, 12.42mmol, 1.0eq) in tetrahydrofuran (20mL) was added slowly dropwise. The reaction mixture was stirred at 25°C under nitrogen protection for 16h. LCMS showed the reaction was completed. The reaction mixture was poured into saturated ammonium chloride aqueous solution and extracted with ethyl acetate. The organic phase was dried over anhydrous sodium sulfate and filtered, and the filtrate was concentrated. The residue was purified by column chromatography (dichloromethane:methanol=98:2) to give the target compound (5.4g). LCMS(ESI)[M+Na]⁺=619.3

### Step 6: Synthesis of (S)-3-(3-bromobenzofuran-6-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(3-bromobenzofuran-6-yl)-1-oxopropan-2-yl)pyrrolidine-1-carboxylate (5.3g, 8.87mmol, 1eq) was dissolved in tetrahydrofuran (50mL). The mixture was cooled to 0°C in an ice-water bath. An aqueous solution (20mL) of hydrogen peroxide (20mL, 176.4mmol, 19.89eq, 30% content) and lithium hydroxide (320mg, 13.31mmol, 1.5eq) was added. The reaction mixture was stirred under nitrogen protection for 16h. LCMS showed the reaction was completed. Saturated sodium sulfite solution was added. The mixture was extracted with ethyl acetate. The organic phase was dried over anhydrous sodium sulfate, filtered, and concentrated to give the target compound (5.0g). LCMS(ESI)[M+H-*tert-*butyl]⁺=382.10

### Step 7: Synthesis of tert-butyl (R)-3-((S)-3-(3-bromobenzofuran-6-yl)-1-(tert-butoxy)-1-oxopropan-2-yl)pyrrolidine-1-carboxylate

(S)-3-(3-bromobenzofuran-6-yl)-2-((R)-1-(tert-butoxycarbonyl)pyrrolidin-3-yl)propanoic acid (4.9g, 11.18mmol, 1eq) was dissolved in 2-methyltetrahydrofuran (50mL), followed by adding *tert-*butyl N,N'-diisopropylcarbamimidate (6.72g, 33.54mmol, 3eq). The reaction mixture was raised to 70°C and stirred for 3h under nitrogen protection. LCMS showed the reaction was completed. The reaction mixture was cooled to room temperature and filtered with celite. The filtrate was rotary-evaporated to dryness for column chromatography (petroleum ether:ethyl acetate=90:10) to give the target compound (2.1g). LCMS(ESI)[M+Na]⁺=516.0.

### Step 8: Synthesis of tert-butyl (R)-3-((S)-1-(tert-butoxy)-3-(3-formylbenzofuran-6-yl)-1-oxopropan-2-yl)pyrrolidine-1-carboxylate

*Tert-*butyl (R)-3-((S)-3-(3-bromobenzofuran-6-yl)-1-(tert-butoxy)-1-oxopropan-2-yl)pyrrolidine-1-carboxylate (1.0g, 2.03mmol, 1.0eq) was dissolved in toluene (20mL), followed by adding palladium acetate (45.9mg, 0.203mmol, 0.1eq), N,N,N',N'-tetramethylethylenediamine (424.6mg, 3.65mmol, 1.8eq), and n-butyldi(1-adamantyl)phosphine (145.6mg, 0.406mmol, 0.2eq). The reaction system was charged with carbon monoxide to 1.2 MPa and then hydrogen to 2.4 MPa. The mixture was raised to 110°C and stirred for 48h. TLC showed the reaction was completed. The mixture was filtered. The filtrate was concentrated, separated and purified by rapid chromatography (silica gel, petroleum ether:ethyl acetate=5:1) to give the target compound (570mg). LCMS(ESI)[M+Na]⁺=466.4.

### Step 9: Synthesis of tri-tert-butyl 3,3',3"-((2S,2'S,2"S)-((nitrilotris(methylene))tris(benzofuran-3,6-diyl))tris(3-(tert-butoxy)-3-oxopropane-1,2-diyl))(3R,3'R,3"R)-tris(pyrrolidine-1-carboxylate)

*Tert-*butyl (R)-3-((S)-1-(tert-butoxy)-3-(3-formylbenzofuran-6-yl)-1-oxopropan-2-yl)pyrrolidine-1-carboxylate (150mg, 0.34mmol, 1.0eq) was dissolved in methanol (2mL), followed by adding ammonia methanol solution (0.1mL, 7mol/L), sodium cyanoborohydride (62mg, 1.02mmol, 3.0eq) at 25°C, and 2 drops of acetic acid. The mixture was raised to 60°C and stirred for 3h. LCMS showed the formation of a product. Water was added. The mixture was extracted with ethyl acetate. The organic phase was dried over anhydrous sodium sulfate, filtered, concentrated, and purified by column chromatography (petroleum ether:ethyl acetate=2:1) to give the target compound (42mg). LCMS(ESI)[M+H]⁺=1300.1.

### Step 10: (2S,2'S,2"S)-3,3',3"-((nitrilotris(methylene))tris(benzofuran-3,6-diyl))tris(2-((R)-pyrrolidin-3-yl)propanoic acid)

Tri-*tert*-butyl 3,3',3"-((2S,2'S,2"S)-((nitrilotris(methylene))tris(benzofuran-3,6-diyl))tris(3-(tert-butoxy)-3-oxopropane-1,2-diyl))(3R,3'R,3"R)-tris(pyrrolidine-1-carboxylate) (42mg, 0.03mmol, 1.0eq) was dissolved in hydrochloric acid/1,4-dioxane (3mL, 4.0M) and stirred at room temperature for 3h. LCMS showed the reaction was completed. The reaction mixture was filtered to give the target compound (2mg). LCMS(ESI)[M+H]⁺=831; ¹HNMR(EN1298-249-P):(400MHz, D₂O) *δ* 7.21-7.27 (m, 6H), 7.03-6.98 (m, 3H), 6.85-6.88 (m, 3H), 4.73 (s, 6H), 3.38-3.21 (m, 6H), 3.17-3.27 (m, 4H), 2.89-2.81 (m, 9H), 2.62-2.58 (m, 3H), 2.48-2.52 (m, 3H), 2.26-2.21 (m, 2H), 1.68-1.73 (m, 3H).

### Example 34 (Compound 110)

### Preparation of 3,3',3"-((nitrilotris(methylene))tris(benzofuran-2,4-diyl))tris(2-((R)-pyrrolidin-3-yl)propanoic acid)

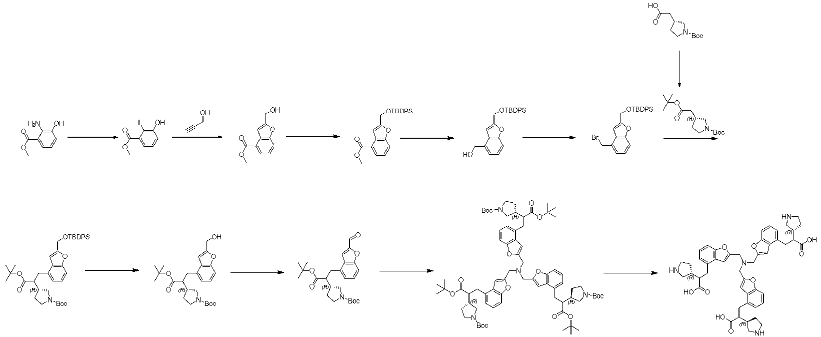

### Step 1: Synthesis of methyl 3-hydroxy-2-iodobenzoate

Methyl 2-amino-3-hydroxybenzoate (25g) was dissolved in a mixed solution of sulfuric acid (90mL) and water (910mL). The mixture was cooled to 0°C. Sodium nitrite (11.25g, formulated as a 25 mL aqueous solution) was added. Then, the reaction mixture was warmed to room temperature and stirred for 20 min. Potassium iodide (111.7g, formulated as a 100 mL aqueous solution) was added, the temperature was raised to 70°C, and the mixture was stirred for 1.5h. LCMS showed the reaction was completed. Water was added to the reaction mixture. The mixture was extracted with ethyl acetate. The organic phase was washed with saturated sodium chloride solution, dried over anhydrous sodium sulfate, filtered, and concentrated to give the crude product. The crude product was separated and purified by rapid chromatography (silica gel, petroleum ether:ethyl acetate=5:1) to give the target compound (20g). LCMS(ESI)[M+H]⁺=279.2.

### Step 2: Synthesis of methyl 2-(hydroxymethyl)benzofuran-4-carboxylate

Methyl 3-hydroxy-2-iodobenzoate (20.0g) was dissolved in N,N-dimethylformamide (200mL), followed by adding copper iodide (1.37g), bis(triphenylphosphine)palladium(II) dichloride (5g), triethylamine (21.86g), and propargyl alcohol (6.1g). The reaction mixture was heated to 75°C and stirred for 16h under nitrogen protection. LCMS showed the reaction was completed. Water and ethyl acetate were added. The mixture was extracted and separated. The organic phase was washed with saturated saline solution, dried over anhydrous sodium sulfate, filtered, and concentrated. The residue was separated and purified by rapid chromatography (silica gel, petroleum ether:ethyl acetate=10:1) to give the target compound (11.0g). LCMS(ESI)[M+H]⁺=207.4.

### Step 3: Synthesis of methyl 2-(((tert-butyldiphenylsilyl)oxy)methyl)benzofuran-4-carboxylate

Methyl 2-(hydroxymethyl)benzofuran-4-carboxylate(11.0g) was dissolved in dichloromethane (80mL), followed by adding imidazole (4.5g) and *tert*-butyl(chloro)diphenylsilane (13.0g). The reaction mixture was stirred at room temperature for 16h. LCMS showed the reaction was completed. The mixture was filtered. The filtrate was concentrated to give the crude product, which was separated and purified by rapid chromatography (silica gel, petroleum ether: ethyl acetate=10:1) to give the target compound (12.2g). LCMS(ESI)[M+Na]⁺=467.2.

### Step 4: Synthesis of (2-(((tert-butyldiphenylsilyl)oxy)methyl)benzofuran-4-yl)methanol

Methyl 2-(((*tert*-butyldiphenylsilyl)oxy)methyl)benzofuran-4-carboxylate (12.2g) was dissolved in tetrahydrofuran (100mL), followed by adding lithium borohydride (1.79g) at 0°C. Then, the reaction mixture was brought to room temperature and stirred for 16h. LCMS showed the reaction was completed. The reaction mixture was added with water and extracted with ethyl acetate. The organic phase was washed with saturated saline solution, dried over anhydrous sodium sulfate, and concentrated. The residue was separated and purified by rapid chromatography (silica gel, petroleum ether:ethyl acetate=2:1) to give the target compound (7.64g). LCMS(ESI)[M+Na]⁺=439.4.

### Step 5: Synthesis of ((4-(bromomethyl)benzofuran-2-yl)methoxy)(tert-butyl)diphenylsilane

(2-(((*tert*-butyldiphenylsilyl)oxy)methyl)benzofuran-4-yl)methanol (2.0g) was dissolved in dichloromethane (20mL), followed by adding phosphorus tribromide (1.95g) at 0°C. The reaction mixture was stirred for 2h. TLC showed the reaction was completed. The reaction was quenched with saturated ammonium chloride aqueous solution. The mixture was extracted with ethyl acetate. The organic phase was dried over anhydrous sodium sulfate, separated and purified by rapid chromatography (silica gel, petroleum ether:ethyl acetate=100:1) to give the target compound (1.42g). LCMS(ESI)[M+H]⁺=479.10.

### Step 6: Synthesis of tert-butyl (R)-3-(2-(tert-butoxy)-2-oxoethyl)pyrrolidine-1-carboxylate

(R)-2-(1-(tert-butoxycarbonyl)pyrrolidin-3-yl)acetic acid (25.0g) was dissolved in dichloromethane (200mL), followed by adding N,N'-dicyclohexylcarbodiimide (22.5g), *tert-*butyl alcohol (24.25mL), and 4-dimethylaminopyridine (13.3g) at room temperature. The mixture was stirred at room temperature for 16h. LCMS showed the reaction was completed. Water and ethyl acetate were added. The mixture was extracted and separated. The organic phase was dried over anhydrous sodium sulfate, filtered and concentrated to give the crude product, which was separated and purified by rapid chromatography (silica gel, petroleum ether:ethyl acetate=10:1) to give the target compound (24.0g). LCMS(ESI)[M+H]⁺=286.5.

### Step 7: Synthesis of tert-butyl (3R)-3-(1-(tert-butoxy)-3-(2-(((tert-butyldiphenylsilyl)oxy)methyl)benzofuran-4-yl)-1-oxopropan-2-yl)pyrrolidine-1-carboxylate

*Tert-*butyl (R)-3-(2-(tert-butoxy)-2-oxoethyl)pyrrolidine-1-carboxylate (905mg) was dissolved in tetrahydrofuran (10mL), followed by the dropwise addition of lithium bis(trimethylsilyl)amide (4.8mL, 1.0M) at 0°C. Then, the mixture was stirred at this temperature for 30min. A solution of ((4-(bromomethyl)benzofuran-2-yl)methoxy)(*tert*-butyl)diphenylsilane (1.42g) in tetrahydrofuran (10mL) was added dropwise to the reaction system. After the addition, the reaction mixture was warmed to room temperature and stirred for 3h. LCMS showed the reaction was completed. The reaction was quenched with saturated ammonium chloride aqueous solution. The mixture was extracted with ethyl acetate. The organic phase was dried over anhydrous sodium sulfate. The residue was separated and purified by rapid chromatography (silica gel, petroleum ether:ethyl acetate=3:1) to give the target compound (1.67g). LCMS(ESI)[M+H]⁺=684.2.

### Step 8: Synthesis of tert-butyl (3R)-3-(1-(tert-butoxy)-3-(2-(hydroxymethyl)benzofuran-4-yl)-1-oxopropan-2-yl)pyrrolidine-1-carboxylate

*Tert-*butyl (3R)-3-(1-(tert-butoxy)-3-(2-(((*tert*-butyldiphenylsilyl)oxy)methyl)benzofuran-4-yl)-1-oxopropan-2-yl)pyrrolidine-1-carboxylate (1.67g) was dissolved in tetrahydrofuran (10mL), followed by adding di-tetra-n-butylammonium fluoride (3.19g) at room temperature. The mixture was stirred at room temperature for 3h. LCMS showed the reaction was completed. The reaction was quenched with saturated ammonium chloride aqueous solution. The mixture was extracted with ethyl acetate and separated. The organic phase was dried over anhydrous sodium sulfate, filtered, and concentrated to give the crude product (1.8g). LCMS(ESI)[M+Na]⁺=468.4.

### Step 9: Synthesis of tert-butyl (3R)-3-(1-(tert-butoxy)-3-(2-formylbenzofuran-4-yl)-1-oxopropan-2-yl)pyrrolidine-1-carboxylate

*Tert-*butyl (3R)-3-(1-(tert-butoxy)-3-(2-(hydroxymethyl)benzofuran-4-yl)-1-oxopropan-2-yl)pyrrolidine-1-carboxylate (1.8g) was dissolved in dichloromethane (10mL), followed by adding manganese dioxide (1.76g) at room temperature. The reaction mixture was heated to 60°C and stirred for 3h. LCMS showed the reaction was completed. The reaction mixture was filtered. The filtrate was concentrated. The residue was separated and purified by rapid chromatography (silica gel, petroleum ether:ethyl acetate=5:1) to give the target compound (894mg). LCMS(ESI)[M+Na]⁺=466.4.

### Step 10: Synthesis of tri-tert-butyl 3,3',3"-(((nitrilotris(methylene))tris(benzofuran-2,4-diyl))tris(3-(tert-butoxy)-3-oxopropane-1,2-diyl))(3R,3'R,3"R)-tris(pyrrolidine-1-carboxylate)

*Tert-*butyl (3R)-3-(1-(tert-butoxy)-3-(2-formylbenzofuran-4-yl)-1-oxopropan-2-yl)pyrrolidine-1-carboxylate (200mg) was dissolved in methanol (2mL), followed by adding ammonia methanol solution (0.01mL, 7 mol/L) and sodium cyanoborohydride (84.8mg) at room temperature. Two drops of acetic acid were added, and the temperature was raised to 60°C. The mixture was stirred for 3h. The reaction was detected by LCMS until a product was formed. The product was separated and purified by silica gel plate (silica gel, petroleum ether:ethyl acetate=1:1) to give the target compound (32mg). LCMS(ESI)[M+H]⁺=1299.5.

### Step 11: Synthesis of 3,3',3"-((nitrilotris(methylene))tris(benzofuran-2,4-diyl))tris(2-((R)-pyrrolidin-3-yl)propanoic acid)

Tri-*tert*-butyl 3,3',3"-(((nitrilotris(methylene))tris(benzofuran-2,4-diyl))tris(3-(tert-butoxy)-3-oxopropane-1,2-diyl))(3R,3'R,3"R)-tris(pyrrolidine-1-carboxylate) (32mg) was dissolved in hydrochloric acid/1,4-dioxane (5mL) and stirred at room temperature for 3h. LCMS showed the reaction was completed. The reaction mixture was filtered to give the target compound (4.41mg). LC-MS:[M+H]⁺=831.5; ¹HNMR:(400MHz, D₂O) *δ* 7.21-7.26 (m, 6H), 7.03-6.98 (m, 3H), 6.86-6.82 (m, 3H), 4.73 (s, 6H), 3.49-3.42 (m, 1H),3.38-3.24 (m, 6H), 3.20-3.25 (m, 3H), 2.89-2.81 (m, 9H), 2.62-2.59 (m, 3H), 2.51-2.47 (m, 3H), 2.27-2.21 (m, 3H), 1.72-1.68 (m, 3H).

### Example 35 (Compound 111)

### Preparation of (2S,2'S)-3,3'-(((((2-(3-((S)-2-carboxy-2-((R)-pyrrolidin-3-yl)ethyl)phenyl)acetyl)azanediyl)bis(ethane-2,1-diyl))bis(oxy))bis(3,1-phenylene))bis(2-((R)-pyrrolidin-3-yl)propanoic acid)

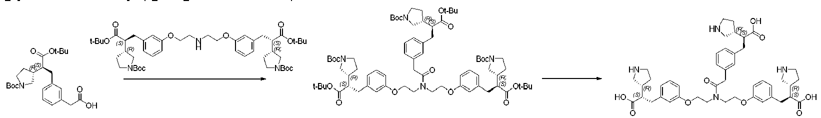

### Step 1: Synthesis of di-tert-butyl 3,3'-((2S,2'S)-(((((2-(3-((S)-3-(tert-butoxy)-2-((R)-1-(tert-butoxycarbonyl)pyrrolidin-3-yl)-3-oxopropyl)phenyl)acetyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-(((azanediylbis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate) (100mg, 0.12mmol, 1eq) was dissolved in ethyl acetate (2mL), followed by adding 2-(3-((S)-3-(tert-butoxy)-2-((R)-1-(tert-butoxycarbonyl)pyrrolidin-3-yl)-3-oxopropyl)phenyl)acetic acid (52.74mg, 0.12mmol, 1eq), 1-propylphosphonic anhydride (116.11mg, 0.18mmol, 1.5eq, 50% content), and *N,N-*diisopropylethylamine (47.16mg, 0.36mmol, 3eq). The reaction was maintained at 50°C for 16h under nitrogen protection. The reaction mixture was concentrated, separated and purified by rapid chromatography (silica gel, tetrahydrofuran:petroleum ether=1:3) to give the target compound (75mg). LCMS(ESI)[M+H-Boc]⁺=1167.5.

### Step 2: Synthesis of (2S,2'S)-3,3'-(((((2-(3-((S)-2-carboxy-2-((R)-pyrrolidin-3-yl)ethyl)phenyl)acetyl)azanediyl)bis(ethane-2,1-diyl))bis(oxy))bis(3,1-phenylene))bis(2-((R)-pyrrolidin-3-yl)propanoic acid)

The product from the first step (75mg, 0.06mmol, 1eq) was dissolved in dioxane (2mL), followed by adding hydrochloric acid/1,4-dioxane (2mL). The reaction was maintained at room temperature for 2h under nitrogen protection. The reaction mixture was concentrated, separated and purified by Prep-HPLC (0.1% NH₄HCO₃ in water, acetonitrile) to give the target compound (15.11mg). LCMS(ESI)[M+H]⁺=799.3; ¹HNMR(400MHz, D₂O) δ 7.23-6.96 (m, 6H), 6.95-6.76 (m, 4H), 6.70-6.64 (m, 1H), 6.60-6.56 (m, 1H), 4.56-4.44 (m, 2H), 4.00 (t, *J*=5.2Hz, 1H), 3.93-3.84 (m, 1H), 3.67-3.57 (m, 2H), 3.41-3.25 (m, 6H), 3.19-3.08 (m, 3H), 2.90-2.48 (m, 13H), 2.42-2.25 (m, 6H), 2.10-1.94 (m, 3H), 1.72-1.57 (m, 3H).

### Example 36 (Compound 112)

### Preparation of (2S,2'S)-3,3'-(((((2-(3-((S)-2-carboxy-2-((R)-pyrrolidin-3-yl)ethyl)phenyl)-2-oxoethyl)azanediyl)bis(ethane-2,1-diyl))bis(oxy))bis(3,1-phenylene))bis(2-((R)-pyrrolidin-3-yl)propanoic acid)

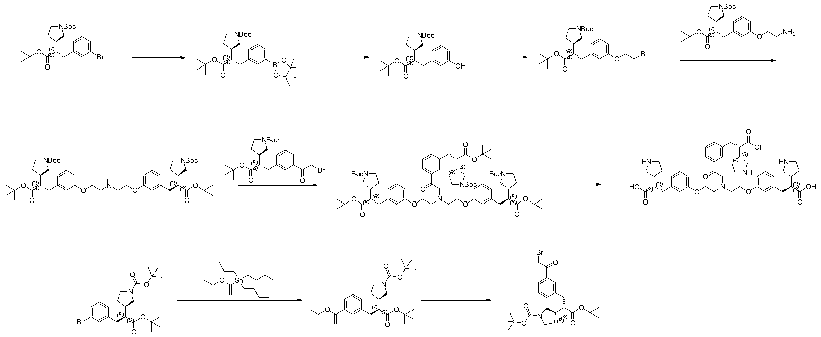

### Step 1: Synthesis of tert-butyl (R)-3-((S)-1-(tert-butoxy)-1-oxo-3-(3-(4,4,5,5-tetramethyl-1,3,2-dioxaborolan-2-yl)phenyl)propan-2-yl)pyrrolidine-1-carboxylate

*Tert-*butyl (R)-3-((S)-3-(3-bromophenyl)-1-(tert-butoxy)-1-oxopropan-2-yl)pyrrolidine-1-carboxylate (10g, 22.01mmol, 1.0eq) was dissolved in 1,4-dioxane (100mL), followed by adding bis(pinacolato)diboron (11.18g, 44.02mmol, 2eq), potassium acetate (6.48g, 66.03mmol, 3eq), and [1,1'-bis(diphenylphosphino)ferrocene]palladium(II) dichloride (1.61g, 2.2mmol, 0.1eq). The reaction mixture was heated to 90°C for 18h under nitrogen protection. After the reaction was completed, the mixture was filtered and concentrated to give the crude product, which was separated and purified by rapid chromatography (silica gel, petroleum ether:tetrahydrofuran=10:1) to give the target compound (10g). LCMS(ESI)[M+Na]⁺=524.0; ¹HNMR(400MHz, DMSO-*d*₆) δ 7.51 (s, 1H), 7.49 (d, *J*=6.9Hz, 1H), 7.33-7.24 (m, 2H), 3.56-3.48 (m, 1H), 3.37-3.34 (m, 1H), 3.14-3.13 (m, 1H), 3.00 (t, *J*=10.0Hz, 1H), 2.73 (d, *J*=10.2Hz, 2H), 2.29 (d, *J*=8.1Hz, 1H), 1.83 (d, *J*=5.7Hz, 1H), 1.67-1.50 (m, 2H), 1.28 (s, 12H), 1.23 (s, 9H), 1.16 (s, 9H).

### Step 2: Synthesis of tert-butyl (R)-3-((S)-1-(tert-butoxy)-3-(3-hydroxyphenyl)-1-oxopropan-2-yl)pyrrolidine-1-carboxylate

*Tert-*butyl (R)-3-((S)-1-(tert-butoxy)-1-oxo-3-(3-(4,4,5,5-tetramethyl-1,3,2-dioxaborolan-2-yl)phenyl)propan-2-yl)pyrrolidine-1-carboxylate (8g, 15.95mmol, 1eq) was dissolved in methanol (80mL), followed by slow addition of hydrogen peroxide (18.09.53mmol, 10eq, 30% content) in an ice bath. The mixture was stirred at room temperature for 1h. After the reaction was completed, the reaction mixture was added with water and extracted with ethyl acetate. The organic phases were combined, washed with saturated sodium chloride solution, dried over anhydrous sodium sulfate, and filtered. The filtrate was concentrated to give the crude product, which was separated and purified by rapid chromatography (silica gel, petroleum ether:tetrahydrofuran=5:1) to give the target compound (4.8g). LCMS(ESI)[M+Na]⁺=414.0.

### Step 3: Synthesis of tert-butyl (R)-3-((S)-3-(3-(2-bromoethoxy)phenyl)-1-(tert-butoxy)-1-oxopropan-2-yl)pyrrolidine-1-carboxylate

In a sealed vessel, *tert-*butyl (R)-3-((S)-1-(tert-butoxy)-3-(3-hydroxyphenyl)-1-oxopropan-2-yl)pyrrolidine-1-carboxylate (500mg, 1.28mmol, 1eq) was dissolved in acetonitrile (10mL) solution, followed by adding 1,2-dibromoethane (1.2g, 6.39mmol, 5eq) and potassium carbonate (529.54mg, 3.83mmol, 3eq). The mixture was heated to 70°C and stirred for 18h. After the reaction was completed, the reaction mixture was added with water and extracted with ethyl acetate. The organic phases were combined, washed with saturated sodium chloride solution, dried over anhydrous sodium sulfate, and filtered. The filtrate was concentrated to give the crude product, which was separated and purified by rapid chromatography (petroleum ether:ethyl acetate=5:1) to give the target compound (300mg). LCMS(ESI)[M+Na]⁺=520.1; ¹HNMR(400MHz, DMSO-*d*₆) δ 7.18 (d, *J*=7.9Hz, 1H), 6.82-6.75 (m, 3H), 4.29-4.28 (m, 2H), 3.79 (t, *J*=5.4Hz, 2H), 3.57-3.43 (m, 1H), 3.38-3.36 (m, 2H), 3.21-3.08 (m, 1H), 2.99 (t, *J*=10.0Hz, 1H), 2.70-2.68 (m, 2H), 2.33-2.21 (m, 1H), 1.90-1.78 (m, 1H), 1.64-1.51 (m, 1H), 1.40 (s, 9H), 1.24 (s, 9H).

### Step 4: Synthesis of di-tert-butyl 3,3'-((2S,2'S)-(((azanediylbis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)

In a sealed vessel, *tert-*butyl (R)-3-((S)-3-(3-(2-bromoethoxy)phenyl)-1-(tert-butoxy)-1-oxopropan-2-yl)pyrrolidine-1-carboxylate(300mg, 0.6mmol, 1eq) was dissolved in acetonitrile (5mL), *tert-*butyl (R)-3-((S)-3-(3-(2-aminoethoxy)phenyl)-1-(tert-butoxy)-1-oxopropan-2-yl)pyrrolidine-1-carboxylate (261.55mg, 0.6mmol, 1eq) and potassium carbonate (249.55mg, 1.81mmol, 3eq) were added; the mixture was heated to 90°C and the reaction was maintained for 18h under nitrogen protection. The reaction mixture was diluted with water, extracted with ethyl acetate. The organic phases were combined, washed with saturated sodium chloride solution, dried over anhydrous sodium sulfate, filtered, concentrated, and the residue was purified by rapid chromatography (ethyl acetate:petroleum ether=2:1) to give the target compound (180mg). LCMS(ESI)[M+H]⁺=852.9; ¹HNMR(400MHz, DMSO-*d*₆) δ 7.23-7.21 (m, 2H), 6.87-6.80 (m, 6H), 4.31-4.20 (m, 4H), 3.64-3.59 (m, 2H), 3.55-3.47 (m, 4H), 3.19-3.11 (m, 2H), 2.99 (d, *J*=7.0Hz, 2H), 2.72 (d, *J*=8.2Hz, 4H), 2.34-2.17 (m, 3H), 2.01 (d, *J*=8.2Hz, 1H), 1.88-1.81 (m, 2H), 1.78-1.75 (m, 1H), 1.64-1.52 (m, 3H), 1.40 (s, 18H), 1.25 (s, 18H).

### Step 5: Synthesis of tert-butyl (R)-3-((S)-1-(tert-butoxy)-3-(3-(1-ethoxyvinyl)phenyl)-1-oxopropan-2-yl)pyrrolidine-1-carboxylate

*Tert-*butyl (R)-3-((S)-3-(3-bromophenyl)-1-(tert-butoxy)-1-oxopropan-2-yl)pyrrolidine-1-carboxylate (400mg, 0.88mmol, 1eq) was dissolved in dioxane (8mL), tributyl(1-ethoxyvinyl)stannane (635.81mg, 1.76mmol, 2eq) and bis(triphenylphosphine)palladium(II) dichloride (61.79mg, 0.09mmol, 0.1eq) were added; the mixture was heated to 100°C and stirred for 1h under nitrogen. After the reaction mixture was cooled to room temperature, a saturated solution of potassium fluoride and dichloromethane were added, and then stirred the mixture for 0.5h. The mixture was extracted with dichloromethane, the organic phases were combined, dried over anhydrous sodium sulfate, filtered, and the filtrate was concentrated to give the crude product (350mg). LCMS(ESI)[M+Na]⁺=468.2.

### Step 6: Synthesis of tert-butyl (R)-3-((S)-3-(3-(2-bromoacetyl)phenyl)-1-(tert-butoxy)-1-oxopropan-2-yl)pyrrolidine-1-carboxylate

*Tert-*butyl (R)-3-((S)-1-(tert-butoxy)-3-(3-(1-ethoxyvinyl)phenyl)-1-oxopropan-2-yl)pyrrolidine-1-carboxylate(350mg, 0.79mmol, 1eq) was dissolved in tetrahydrofuran (10mL) and water (0.4mL), N-bromosuccinimide (139.8mg, 0.79mmol, 1eq) was added, and the mixture was stirred at 25°C for 30min under nitrogen. The reaction mixture was quenched with water and extracted with ethyl acetate. The organic phases were combined, dried over anhydrous sodium sulfate, filtered, and the filtrate was concentrated. The residue was purified by rapid chromatography (Silica gel, petroleum ether:dichloromethane=5:1) to give the target compound (220mg). LCMS(ESI)[M+H-Boc]⁺=396.0; ¹HNMR(400MHz, DMSO-*d*₆) δ 7.88-7.82 (m, 2H), 7.53-7.45 (m, 2H), 4.94-4.86 (m, 2H), 3.58-3.49 (m, 1H), 3.40-3.36 (m, 1H), 3.21-3.11 (m, 1H), 3.02 (t, *J*=10.0Hz, 1H), 2.87-2.79 (m, 2H), 2.60-2.54 (m, 1H), 2.34-2.26 (m, 1H), 1.89-1.82 (m, 1H), 1.61-1.60 (m, 1H), 1.41 (s, 9H), 1.21 (s, 9H).

### Step 7: Synthesis of di-tert-butyl 3,3'-((2S,2'S)-(((((2-(3-((S)-3-(tert-butoxy)-2-((R)-1-(tert-butoxycarbonyl)pyrrolidin-3-yl)-3-oxopropyl)phenyl)-2-oxoethyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-(((azanediylbis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate) (44mg) was dissolved in acetonitrile (2mL), *tert-*butyl (R)-3-((S)-3-(3-(2-bromoacetyl)phenyl)-1-(tert-butoxy)-1-oxopropan-2-yl)pyrrolidine-1-carboxylate (25.63mg) and potassium carbonate (42.82mg) were added; the mixture was heated to 60°C and stirred for 2h under nitrogen. The reaction mixture was quenched with water and extracted with ethyl acetate. The organic phases were combined, dried over anhydrous sodium sulfate, filtered, and the filtrate was concentrated. The residue was purified by TLC silica gel plate (ethyl acetate:petroleum ether=1:5) to give the target compound (40mg). LCMS(ESI)[M+H]⁺=1266.9.

### Step 8: Synthesis of (2S,2'S)-3,3'-(((((2-(3-((S)-2-carboxy-2-((R)-pyrrolidin-3-yl)ethyl)phenyl)-2-oxoethyl)azanediyl)bis(ethane-2,1-diyl))bis(oxy))bis(3,1-phenylene))bis(2-((R)-pyrrolidin-3-yl)propanoic acid)

Di-*tert*-butyl 3,3'-((2S,2'S)-(((((2-(3-((S)-3-(tert-butoxy)-2-((R)-1-(tert-butoxycarbonyl)pyrrolidin-3-yl)-3-oxopropyl)phenyl)-2-oxoethyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate) (27mg) was dissolved in 1,4-dioxane (0.5mL), and hydrochloric acid-1,4-dioxane (0.5mL, 4M) was added; the mixture was stirred at 20°C for 2h under nitrogen. After the reaction was completed, the reaction mixture was concentrated and purified by reversed-phase preparation (0.1% ammonia water-acetonitrile system) to give the target compound (16mg). LCMS(ESI)[M+H]⁺=799.3; ¹HNMR(400MHz, D₂O) δ 7.65 (d, *J*=7.4Hz, 1H), 7.56 (s, 1H), 7.49 (d, *J*=7.5Hz, 1H), 7.39 (t, *J*=7.6Hz, 1H), 7.12 (t, *J*=7.3Hz, 2H), 6.78 (d, *J*=6.8Hz, 2H), 6.59 (d, *J*=8.0Hz, 2H), 6.54 (s, 2H), 4.95 (s, 2H), 4.34-4.27 (m, 4H), 3.76-3.60 (m, 4H), 3.53-3.46 (m, 1H), 3.43-3.31 (m, 5H), 3.21-3.12 (m, 3H), 2.93-2.80 (m, 3H), 2.75-2.69 (m, 2H), 2.68-2.53 (m, 4H), 2.43-2.32 (m, 6H), 2.08-2.00 (m, 3H), 1.70-1.60 (m, 3H).

### Example 37 (Compound 113)

### Preparation of (2S,2'S)-3,3'-(((((2-(3-((S)-2-carboxy-2-((R)-pyrrolidin-3-yl)ethyl)phenoxy)acetyl)azanediyl)bis(ethane-2,1-diyl))bis(oxy))bis(3,1-phenylene))bis(2-((R)-pyrrolidin-3-yl)propanoic acid)

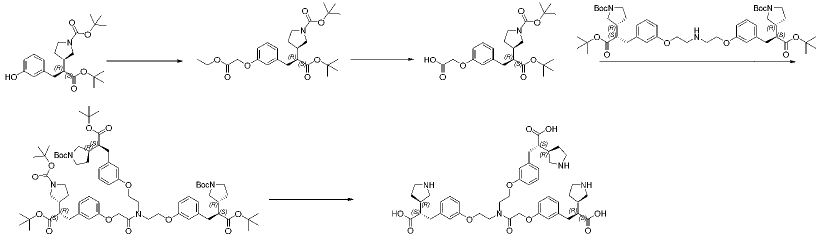

### Step 1: Synthesis of tert-butyl (R)-3-((S)-1-(tert-butoxy)-3-(3-(2-ethoxy-2-oxoethoxy)phenyl)-1-oxopropan-2-yl)pyrrolidine-1-carboxylate

*Tert-*butyl (R)-3-((S)-1-(tert-butoxy)-3-(3-hydroxyphenyl)-1-oxopropan-2-yl)pyrrolidine-1-carboxylate (400mg, 1.02mmol, 1.0eq) was dissolved in N,N-dimethylformamide (10mL), and ethyl bromoacetate (256mg, 1.54mmol, 1.5eq) and potassium carbonate (432mg, 3.06mmol, 3.0eq) were added; the mixture was stirred at room temperature for 24h, and LCMS showed the reaction was completed. The reaction mixture was concentrated to give a crude product, which was purified by rapid chromatography (Silica gel, petroleum ether:ethyl acetate=10:1) to give the target compound (300mg). LCMS(ESI)[M+H]⁺=478.0.

### Step 2: Synthesis of 2-(3-((S)-3-(tert-butoxy)-2-((R)-1-(tert-butoxycarbonyl)pyrrolidin-3-yl)-3-oxopropyl)phenoxy)acetic acid

*Tert-*butyl (R)-3-((S)-1-(tert-butoxy)-3-(3-(2-ethoxy-2-oxoethoxy)phenyl)-1-oxopropan-2-yl)pyrrolidine-1-carboxylate (300mg, 0.63mmol, 1.0eq) was dissolved in tetrahydrofuran/water (3:1, 12mL), lithium hydroxide monohydrate (27mg, 0.63mmol, 1.0eq) was added, and the mixture was stirred at room temperature for 16h. LCMS showed the reaction was completed. The crude product was purified by rapid chromatography (Silica gel, petroleum ether:ethyl acetate=10:1) to give the target compound (200mg). LCMS(ESI)[M+H-Boc]⁺=394.0.

### Step 3: Synthesis of di-tert-butyl 3,3'-((2S,2'S)-(((((2-(3-((S)-3-(tert-butoxy)-2-((R)-1-(tert-butoxycarbonyl)pyrrolidin-3-yl)-3-oxopropyl)phenoxy)acetyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)

Di-*tert*-butyl 3,3'-((2S,2'S)-(((azanediylbis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate) (100mg, 0.12mmol, 1.0eq) was dissolved in N,N-dimethylformamide (10mL). Then 2-(3-((S)-3-(tert-butoxy)-2-((R)-1-(tert-butoxycarbonyl)pyrrolidin-3-yl)-3-oxopropyl)phenoxy)acetic acid (58mg, 0.13mmol, 1.1eq), O-(7-azabenzotriazol-1-yl)-N,N,N',N'-tetramethyluronium hexafluorophosphate (89mg, 0.23mmol, 2.0eq) and *N,N*-diisopropylethylamine (46mg, 0.35mmol, 3.0eq) were added. The reaction mixture was stirred at room temperature for 6h. LCMS showed the reaction was completed. The reaction mixture was diluted with water and extracted with ethyl acetate. The organic phase was dried over anhydrous sodium sulfate, filtered and concentrated to give a crude product, which was purified by rapid chromatography (Silica gel, petroleum ether:ethyl acetate=1:8) to give the target compound (80mg). LCMS(ESI)[M+H]⁺=1183.2.

### Step 4: Synthesis of (2S,2'S)-3,3'-(((((2-(3-((S)-2-carboxy-2-((R)-pyrrolidin-3-yl)ethyl)phenoxy)acetyl)azanediyl)bis(ethane-2,1-diyl))bis(oxy))bis(3,1-phenylene))bis(2-((R)-pyrrolidin-3-yl)propanoic acid)

Di-*tert*-butyl 3,3'-((2S,2'S)-(((((2-(3-((S)-3-(tert-butoxy)-2-((R)-1-(tert-butoxycarbonyl)pyrrolidin-3-yl)-3-oxopropyl)phenoxy)acetyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate) (80mg, 0.06mmol) was dissolved in dichloromethane (4mL), trifluoroacetic acid (1mL) were added. Then the mixture was stirred at room temperature for 30h. LCMS showed the reaction was completed. The reaction mixture was concentrated directly and purified by preparative chromatography to give the target compound (48.19mg). LCMS(ESI)[M+H]⁺=815.0; ¹HNMR(400MHz, D₂O) δ 7.16 (dt, *J*=22.4, 7.9Hz, 2H), 7.03 (t, *J*=7.9Hz, 1H), 6.83-6.73 (m, 4H), 6.72-6.62 (m, 4H), 6.57 (t, *J*=2.0Hz, 1H), 4.92 (s, 2H), 4.24 (t, *J*=4.9Hz, 2H), 4.12 (t, *J*=5.0Hz, 2H), 3.76 (q, *J*=5.1, 4.2Hz, 4H), 3.45 (dt, *J*=11.7, 8.4Hz, 2H), 3.32 (dddd, *J*=23.3, 11.7, 8.6, 3.4Hz, 4H), 3.16 (dddd, *J*=19.6, 7.4, 6.0, 4.1Hz, 3H), 2.93 (td, *J*=12.4, 10.1Hz, 2H), 2.81-2.55 (m, 10H), 2.44 (p, *J*=8.2Hz, 2H), 2.30 (q, *J*=8.3Hz, 1H), 2.09 (dp, *J*=12.5, 3.8Hz, 2H), 1.99 (ddt, *J*=13.7, 7.1, 3.5Hz, 1H), 1.74-1.63 (m, 2H), 1.61-1.50 (m, 1H).

### Example 38 (Compound 117)

### Preparation of (25,2'S)-3,3'-((piperazine-1,4-diylbis(methylene))bis(3,1-phenylene))bis(2-((R)-pyrrolidin-3-yl)propanoic acid)

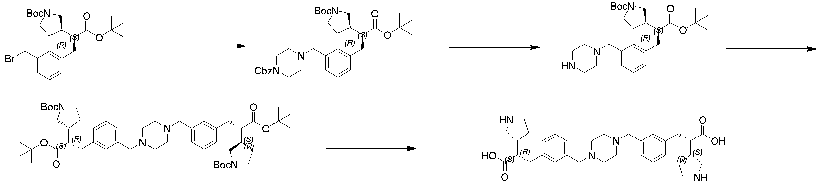

### Step 1: Synthesis of benzyl 4-(3-((S)-3-(tert-butoxy)-2-((R)-1-(tert-butoxycarbonyl)pyrrolidin-3-yl)-3-oxopropyl)benzyl)piperazine-1-carboxylate

*Tert-*butyl (R)-3-((S)-3-(3-(bromomethyl)phenyl)-1-(tert-butoxy)-1-oxopropan-2-yl)pyrrolidine-1-carboxylate (30mg) and benzyl piperazine-1-carboxylate (15.6mg) were dissolved in N,N-dimethylformamide (3mL), cesium carbonate (42mg) was added; the mixture was stirred for 3h at room temperature, quenched with water and extracted with ethyl acetate. The organic phase was dried over anhydrous sodium sulfate, filtered, concentrated to give the crude product, which was purified by rapid chromatography (Silica gel, petroleum ether:ethyl acetate=2:1) to give the target product (30mg). LCMS(ESI)[M+H]⁺=608.1.

### Step 2: Synthesis of tert-butyl (R)-3-((S)-1-(tert-butoxy)-1-oxo-3-(3-(piperazin-1-ylmethyl)phenyl)propan-2-yl)pyrrolidine-1-carboxylate

benzyl 4-(3-((S)-3-(tert-butoxy)-2-((R)-1-(tert-butoxycarbonyl)pyrrolidin-3-yl)-3-oxopropyl)benzyl)piperazine-1-carboxylate (30mg) and Pd/C (69.5mg) were dissolved in methanol (5.0mL), and the reaction mixture was replaced with hydrogen balloon three times and stirred at room temperature overnight. The reaction mixture was filtered and concentrated to give the target compound (23mg). LCMS(ESI)[M+H-*tert*-butyl]⁺=418.3.

### Step 3: Synthesis of di-tert-butyl 3,3'-((2S,2'S)-((piperazine-1,4-diylbis(methylene))bis(3,1-phenylene))bis(3-(tert-butoxy)-3-oxopropane-1,2-diyl))(3R,3'R)-bis(pyrrolidine-1-carboxylate)

*Tert-*butyl (R)-3-((S)-1-(tert-butoxy)-1-oxo-3-(3-(piperazin-1-ylmethyl)phenyl)propan-2-yl)pyrrolidine-1-carboxylate (23mg), *tert-*butyl (R)-3-((S)-3-(3-(bromomethyl)phenyl)-1-(tert-butoxy)-1-oxopropan-2-yl)pyrrolidine-1-carboxylate (26mg) and cesium carbonate (32.8mg) were dissolved in N,N-dimethylformamide (3.0mL). The mixture was stirred overnight at room temperature, quenched with water, extracted with ethyl acetate, dried over anhydrous sodium sulfate, filtered and concentrated to give a crude product, which was purified by rapid chromatography (Silica gel, petroleum ether:ethyl acetate=2:1) to give the target product (36mg). LCMS(ESI)[M+H]⁺=861.1.

### Step 4: Preparation of (2S,2'S)-3,3'-((piperazine-1,4-diylbis(methylene))bis(3,1-phenylene))bis(2-((R)-pyrrolidin-3-yl)propanoic acid)

Di-*tert*-butyl 3,3'-((2S,2'S)-((piperazine-1,4-diylbis(methylene))bis(3,1-phenylene))bis(3-(tert-butoxy)-3-oxopropane-1,2-diyl))(3R,3'R)-bis(pyrrolidine-1-carboxylate) (36mg) was dissolved in dichloromethane (4mL), and trifluoroacetic acid (47.9mg) was added. The mixture was stirred at room temperature overnight. LCMS showed the reaction was completed. The reaction mixture was concentrated to give a crude product, which was purified by Prep-HPLC to give the target product (18mg). LCMS(ESI)(M+H)⁺=549.0; ¹HNMR(400MHz, D₂O)δ7.28-7.21 (m, 2H), 7.15-7.09 (m, 6H), 3.49 (s, 4H), 3.46-3.41 (m, 2H), 3.36-3.3 (m, 3H), 3.2-3.13 (m,3H), 2.91-2.86 (m, 3H), 2.78-2.68 (m, 5H), 2.78-2.67 (m, 6H), 2.44-2.35 (m, 3H), 1.72-1.61 (m, 3H).

### Example 39 (Compound 121)

### Preparation of (2S,2'S,2"S)-3,3',3"-((nitrilotris(methylene))tris(thiazole-5,2-diyl))tris(2-((R)-pyrrolidin-3-yl)propanoic acid)

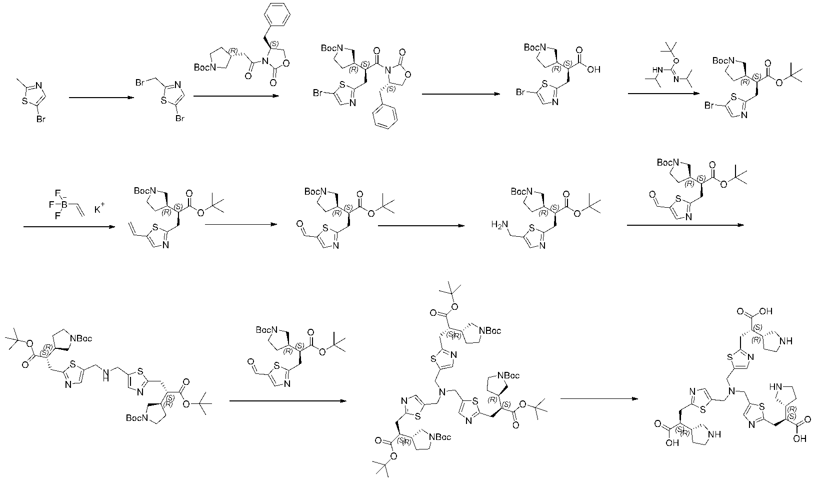

### Step 1: Synthesis of 5-bromo-2-(bromomethyl)thiazole

5-bromo-2-methylthiazole (4.5g, 25.27mmol, 1eq) was dissolved in carbon tetrachloride (90mL). Benzoyl peroxide (0.31g, 1.26mmol, 0.05eq) and N-bromosuccinimide (4.95g, 27.8mmol, 1.1eq) were added at room temperature. The reaction mixture was stirred at 80°C for 16h. LCMS showed the reaction was completed. The reaction mixture was filtered, and the filtrate was concentrated and purified by column chromatography (petroleum ether:ethyl acetate=10:1) to give the target compound (2.7g). LCMS(ESI)[M+H]⁺=257.7; ¹HNMR(400MHz, CDCl₃) δ 7.63 (s, 1H), 4.67 (s, 2H).

### Step 2: Synthesis of tert-butyl (R)-3-((S)-1-((S)-4-benzyl-2-oxooxazolidin-3-yl)-3-(5-bromothiazol-2-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate (2.42g, 6.22mmol, 0.8eq) was dissolved in anhydrous tetrahydrofuran (20mL). Cooling to 0°C in an ice-water bath, lithium bis(trimethylsilyl)amide (11.67mL, 11.67mmol, 1.5eq, 1.0M) was added to the mixture, which was stirred at 0°C for 30min. A solution of 5-bromo-2-(bromomethyl)thiazole (2g, 7.78mmol, 1eq) in tetrahydrofuran (10mL) was added slowly at this temperature. After the addition was completed, the mixture was warmed gradually to room temperature and stirred for 16h. LCMS showed the reaction was completed. A saturated solution of ammonium chloride was added, and the mixture was extracted with ethyl acetate. The organic phase was dried over anhydrous sodium sulfate, filtered, and the filtrate was rotary-evaporated to dryness. The crude product was purified by column chromatography (petroleum ether:ethyl acetate=2:1) to give the target compound (1.0g). LCMS(ESI)[M+H]⁺=564.0; ¹HNMR(400MHz, CDCl₃) δ 7.51 (s, 1H), 7.36-7.27 (m, 3H), 7.22-7.17 (m, 2H), 4.74-4.63 (m, 1H), 4.58-4.49 (m, 1H), 4.18-4.13 (m, 2H), 3.73-3.55 (m, 1H), 3.54-3.42 (m, 2H), 3.31-3.18 (m, 3H), 3.13-3.02 (m, 1H), 2.65-2.47 (m, 2H), 2.01-1.93 (m, 1H), 1.81-1.68 (m, 1H), 1.46 (s, 9H).

### Step 3: Synthesis of (S)-3-(5-bromothiazol-2-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(5-bromothiazol-2-yl)-1-oxopropan-2-yl)pyrrolidine-1-carboxylate(6g, 10.63mmol, 1eq) was dissolved in tetrahydrofuran (60mL). The mixture was cooled to 0°C in an ice-water bath, and hydrogen peroxide (7.2g, 212.58mmol, 20eq) was added; lithium hydroxide (509.13mg, 21.26mmol, 2eq) was dissolved in water (20mL) and the mixture was slowly added dropwise to the above reaction system. After the addition was completed, the mixture was warmed gradually to room temperature and stirred for 3h. LCMS showed the reaction was completed. A saturated solution of anhydrous sodium sulfite was added to quench the reaction. An aqueous solution of sodium hydroxide was added, and the mixture was extracted with ethyl acetate. The organic phase was discarded; the aqueous phase was adjusted to pH=5 with an aqueous solution of hydrochloric acid (1 N). Ethyl acetate was added, and the mixture was extracted and separated. The organic phase was dried over anhydrous sodium sulfate, filtered, and the filtrate was rotary-evaporated to dryness to give the target compound (4g). LCMS(ESI)[M+H]⁺=405.1.

### Step 4: tert-butyl (R)-3-((S)-3-(5-bromothiazol-2-yl)-1-(tert-butoxy)-1-oxopropan-2-yl)pyrrolidine-1-carboxylate

(S)-3-(5-bromothiazol-2-yl)-2-((R)-1-(tert-butoxycarbonyl)pyrrolidin-3-yl)propanoic acid(4g) was dissolved in anhydrous 2-methyltetrahydrofuran (40mL), *tert-*butyl N,N'-diisopropylcarbamimidate (4.23g) was added at room temperature, and the reaction mixture was stired at 70°C for 3h. LCMS showed the reaction was completed. The reaction mixture was filtered, and the filter cake was washed with methyl *tert*-butyl ether. The filtrate was combined and evaporated to dryness to give the crude product, which was purified by column chromatography (petroleum ether:ethyl acetate=3:1) to give the target compound (3.8g). LCMS(ESI)[M+H]⁺=461.0; ¹HNMR(400MHz, CDCl₃) δ 7.55 (s, 1H), 3.69-3.44 (m, 2H), 3.32-3.21 (m, 2H), 3.09-2.97 (m, 2H), 2.80-2.71 (m, 1H), 2.44-2.32 (m, 1H), 2.01-1.93 (m, 1H), 1.77-1.66 (m, 1H), 1.46 (s, 9H), 1.40 (s, 9H).

### Step 5: Synthesis of tert-butyl (R)-3-((S)-1-(tert-butoxy)-1-oxo-3-(5-vinylthiazol-2-yl)propan-2-yl)pyrrolidine-1-carboxylate

*Tert-*butyl (R)-3-((S)-3-(5-bromothiazol-2-yl)-1-(tert-butoxy)-1-oxopropan-2-yl)pyrrolidine-1-carboxylate (2g) was dissolved in tetrahydrofuran (30mL) and water (6mL), potassium vinyltrifluoroborate (2.76g), potassium phosphate (2.75g) and Pd(dppf)Cl₂ (317.7mg) were added at room temperature. The mixture was stirred at 80°C for 3h under nitrogen. LCMS showed the reaction was completed. The reaction mixture was diluted with water and extracted with ethyl acetate, the organic phase was dried, filtered, and the filtrate was rotary-evaporated to dryness. The crude product was purified by column chromatography (petroleum ether:ethyl acetate=3:1) to give the target compound (1.4g). LCMS(ESI)[M+H]⁺=409.2.

### Step 6: Synthesis of tert-butyl (R)-3-((S)-1-(tert-butoxy)-3-(5-formylthiazol-2-yl)-1-oxopropan-2-yl)pyrrolidine-1-carboxylate

*Tert-*butyl (R)-3-((S)-1-(tert-butoxy)-1-oxo-3-(5-vinylthiazol-2-yl)propan-2-yl)pyrrolidine-1-carboxylate (1.4g, 3.43mmol, 1eq) was dissolved in dichloromethane (30mL) and methanol (3mL). Cooling to -70°C with a dry ice-acetone bath, sufficient oxygen was introduced into the reaction solution. Oxygen was converted to ozone using an ozone generator, and the mixture was stirred at -70°C for 10 min. TLC plate showed that the starting material was basically consumed. The reaction was quenched with dimethyl sulfide, and the mixture was rotary-evaporated to dryness. The crude product was purified by column chromatography (petroleum ether:ethyl acetate=3:1) to give the target compound (900mg). LCMS(ESI)[M+H]⁺=411.4; ¹HNMR(400MHz, CDCl₃) δ 9.99 (s, 1H), 8.28 (s, 1H), 3.71-3.49 (m, 2H), 3.44-3.34 (m, 1H), 3.31-3.23 (m, 1H), 3.18-3.11 (m, 1H), 3.09-3.00 (m, 1H), 2.87-2.79 (m, 1H), 2.47-2.35 (m, 1H), 2.03-1.95 (m, 1H), 1.79-1.67 (m, 1H), 1.46 (s, 9H), 1.39 (s, 9H).

### Step 7: Synthesis of tert-butyl (R)-3-((S)-3-(5-(aminomethyl)thiazol-2-yl)-1-(tert-butoxy)-1-oxopropan-2-yl)pyrrolidine-1-carboxylate

*Tert-*butyl (R)-3-((S)-1-(tert-butoxy)-3-(5-formylthiazol-2-yl)-1-oxopropan-2-yl)pyrrolidine-1-carboxylate (300mg, 0.73mmol, 1eq) was dissolved in ammonia methanol solution (6mL, 7 M), glacial acetic acid (0.1mL) was added at room temperature. The reaction mixture was stirred at room temperature for 0.5h, and sodium cyanoborohydride (451.36mg, 7.3mmol, 10eq) was added. The reaction mixture was heated to 80°C and stirred for 1h. LCMS showed the reaction was completed.The reaction mixture was rotary-evaporated to dryness, and the crude product was purified by column chromatography (petroleum ether/ethyl acetate=1/4) to give the target compound (150mg). LCMS(ESI)[M+H]⁺=412.2.

### Step 8: Synthesis of di-tert-butyl 3,3'-((2S,2'S)-((azanediylbis(methylene))bis(thiazole-5,2-diyl))bis(3-(tert-butoxy)-3-oxopropane-1,2-diyl))(3R,3'R)-bis(pyrrolidine-1-carboxylate)

*Tert-*butyl (R)-3-((S)-3-(5-(aminomethyl)thiazol-2-yl)-1-(tert-butoxy)-1-oxopropan-2-yl)pyrrolidine-1-carboxylate (150mg, 0.36mmol, 1eq) was dissolved in isopropanol (2mL). *tert-*butyl (R)-3-((S)-1-(tert-butoxy)-3-(5-formylthiazol-2-yl)-1-oxopropan-2-yl)pyrrolidine-1-carboxylate (295.58mg, 0.72mmol, 2eq) and glacial acetic acid (0.05mL) was added at room temperature. The reaction mixture was stirred at room temperature for 2h, then sodium cyanoborohydride (225.35mg, 3.64mmol, 10eq) was added. The reaction mixture was heated to 75°C and stirred for 16h. LCMS showed the reaction was completed. The reaction mixture was rotary-evaporated to dryness, and the crude product was purified by column chromatography (petroleum ether/ethyl acetate=1/4) to give the target compound (100mg). LCMS(ESI)[M+H]⁺=806.4; ¹HNMR(400MHz, DMSO-*d*₆) δ 7.44 (s, 2H), 3.80 (s, 4H), 3.56-3.45 (m, 3H), 3.20-3.10 (m, 5H), 2.98-2.92 (m, 2H), 2.71-2.63 (m, 3H), 2.35-2.22 (m, 3H), 1.90-1.81 (m, 2H), 1.66-1.58 (m, 2H), 1.39 (s, 18H), 1.32 (s, 18H).

### Step 9: Synthesis of tri-tert-butyl 3,3',3"-((2S,2'S,2"S)-((nitrilotris(methylene))tris(thiazole-5,2-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((azanediylbis(methylene))bis(thiazole-5,2-diyl))bis(3-(tert-butoxy)-3-oxopropane-1,2-diyl))(3R,3'R)-bis(pyrrolidine-1-carboxylate) (90mg, 0.11mmol, 1eq) was dissolved in ethanol (3mL), *tert-*butyl (R)-3-((S)-1-(tert-butoxy)-3-(5-formylthiazol-2-yl)-1-oxopropan-2-yl)pyrrolidine-1-carboxylate (91.67mg, 0.22mmol, 2eq) and glacial acetic acid (0.05mL) were added at room temperature. The reaction mixture was stirred at room temperature for 8h, then sodium cyanoborohydride (69.03mg, 1.12mmol, 10eq) was added, and the reaction mixture was heated to 75°C and stirred for 16h. LCMS showed the reaction was completed. The reaction mixture was rotary-evaporated to dryness, and the crude product was purified by reversed-phase column chromatography (0.03% TFA in H₂O/ACN) to give the target compound (45mg). LCMS(ESI)[M/2+H]⁺=601.3; ¹HNMR(400MHz, DMSO-*d*₆) δ 7.52 (s, 3H), 3.57 (s, 6H), 3.52-3.49 (m, 3H), 3.38-3.32 (m, 3H), 3.21-3.12 (m, 6H), 3.10-3.03 (m, 3H), 2.98-2.91 (m, 3H), 2.72-2.65 (m, 3H), 2.37-2.27 (m, 3H), 1.92-1.84 (m, 3H), 1.66-1.57 (m, 3H), 1.39 (s, 27H), 1.30 (s, 27H).

### Step 10: Synthesis of (2S,2'S,2"S)-3,3',3"-((nitrilotris(methylene))tris(thiazole-5,2-diyl))tris(2-((R)-pyrrolidin-3-yl)propanoic acid)

Tri-*tert*-butyl 3,3',3"-((2S,2'S,2"S)-((nitrilotris(methylene))tris(thiazole-5,2-diyl))tris(3-(tert-butoxy)-3-oxopropane-1,2-diyl))(3R,3'R,3"R)-tris(pyrrolidine-1-carboxylate) (45mg, 0.04mmol, 1eq) was dissolved in 1,4-dioxane (2mL). Hydrochloric acid/1,4-dioxane (2mL, 4M) solution was added at room temperature. The mixture was stirred at room temperature for 5h. LCMS showed the reaction was completed. The reaction mixture was rotary-evaporated to dryness, and the crude product was purified by prep-HPLC (30 mmol NH₄HCO₃ in H₂O/ACN) to give the target compound (18.02mg). LCMS(ESI)[M+H]⁺=732.2; ¹HNMR(400MHz, D₂O) δ 7.44 (s, 3H), 3.79 (s, 6H), 3.44-3.32 (m, 6H), 3.22-3.13 (m, 6H), 3.08-3.00 (m, 3H), 2.95-2.87 (m, 3H), 2.63-2.55 (m, 3H), 2.48-2.36 (m, 3H), 2.13-2.03 (m, 3H), 1.77-1.66 (m, 3H).

### Example 40 (Compound 122)

### Preparation of (2S,2'S,2"S)-3,3',3"-(((benzene-1,3,5-triyltris(oxy))tris(methylene))tris(benzene-3,1-diyl))tris(2-((R)-pyrrolidin-3-yl)propanoic acid)

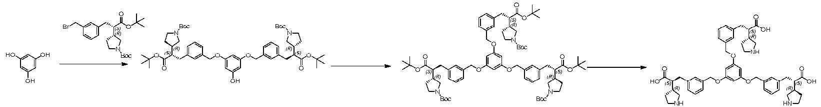

### Step 1: Synthesis of di-tert-butyl 3,3'-((2S,2'S)-((((5-hydroxy-1,3-phenylene)bis(oxy))bis(methylene))bis(3,1-phenylene))bis(3-(tert-butoxy)-3-oxopropane-1,2-diyl))(3R,3'R)-bis(pyrrolidine-1-carboxylate)

benzene-1,3,5-triol (10mg), *tert-*butyl (R)-3-((S)-3-(3-(bromomethyl)phenyl)-1-(tert-butoxy)-1-oxopropan-2-yl)pyrrolidine-1-carboxylate (74.2mg), and potassium carbonate (33mg) were dissolved in DMF (5.0mL). The reaction mixture was heated to 55°C and stirred for 48h.The reaction mixture was then quenched by the addition of water dropwise, extracted with ethyl acetate, dried over anhydrous sodium sulfate, filtered, and the filtrate was concentrated to give a crude product, which was separated and purified by rapid chromatography (Silica gel, petroleum ether:ethyl acetate=1:1) to give the target compound (20mg). LCMS(ESI)[M+H]⁺=901.1.

### Step 2: Synthesis of tri-tert-butyl 3,3',3"-((2S,2'S,2"S)-(((benzene-1,3,5-triyltris(oxy))tris(methylene))tris(benzene-3,1-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((((5-hydroxy-1,3-phenylene)bis(oxy))bis(methylene))bis(3,1-phenylene))bis(3-(tert-butoxy)-3-oxopropane-1,2-diyl))(3R,3'R)-bis(pyrrolidine-1-carboxylate) (20mg), *tert-*butyl (R)-3-((S)-3-(3-(bromomethyl)phenyl)-1-(tert-butoxy)-1-oxopropan-2-yl)pyrrolidine-1-carboxylate (11mg) and potassium carbonate (9.1mg) were dissolved in toluene (3.0mL). The mixture was heated to 120°C under nitrogen protection and the reaction was maintained for 3h under microwave irradiation. LCMS showed the reaction was completed. The reaction mixture was concentrated and separated and purified by rapid chromatography (Silica gel, petroleum ether:ethyl acetate=1:1) to give the product (18mg). LCMS(ESI)[M+H-Boc]⁺=1188.3.

### Step 3: Synthesis of (2S,2'S,2"S)-3,3',3"-(((benzene-1,3,5-triyltris(oxy))tris(methylene))tris(benzene-3,1-diyl))tris(2-((R)-pyrrolidin-3-yl)propanoic acid)

Tri-*tert*-butyl 3,3',3"-((2S,2'S,2"S)-(((benzene-1,3,5-triyltris(oxy))tris(methylene))tris(benzene-3,1-diyl))tris(3-(tert-butoxy)-3-oxopropane-1,2-diyl))(3R,3'R,3"R)-tris(pyrrolidine-1-carboxylate) (18mg) was dissolved in dichloromethane (4mL). Trifluoroacetic acid (16.0mg) was added, and the mixture was stirred at room temperature overnight. LCMS showed the reaction was completed. The reaction mixture was concentrated to give a crude product, which was separated and purified by Prep-HPLC to give the target product (2mg). LCMS(ESI)(M+H)⁺=820.2; ¹HNMR(400MHz, D₂O)δ7.31-6.98(m, 12H), 6.02 (s, 3H), 4.62 (s, 6H), 3.32-3.20 (m, 3H), 3.19-2.97 (m, 6H), 2.64 (dd,J=22.0, 11.8Hz, 6H), 2.46 (dd,J=12.3, 4.0Hz, 3H), 2.38-2.17 (m, 6H), 2.02-1.85 (m, 3H), 1.60 (td,J=18.8, 10.7Hz, 3H).

### Example 41 (Compound 123)

### Preparation of triethyl 3,3',3"-((nitrilotris(methylene))tris(benzene-3,1-diyl))(2S,2'S,2"S)-tris(2-((R)-pyrrolidin-3-yl)propanoate-3',3'-d₂)

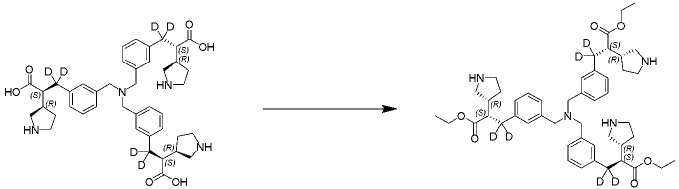

(2S,2'S,2"S)-3,3',3"-((nitrilotris(methylene))tris(benzene-3,1-diyl))tris(2-((R)-pyrrolidin-3-yl)propanoic-3,3-d2 acid) (92mg, 0.128mmol, 1eq) was dissolved in ethanol (4mL), and concentrated sulfuric acid (1mL) was added. The mixture was stirred at 75°C overnight, and LCMS showed the reaction was completed. The solvent was removed by rotary-evaporation to dryness, and the residue was separated and purified by Prep-HPLC (C₁₈, 10mmol/L NH₄HCO₃ in water, MeCN) to give the target compound (61mg). LCMS(ESI)[M+H]⁺=801.71; ¹HNMR(400MHz, D₂O) δ 7.40-7.25 (m, 6H), 7.24-7.02 (m, 6H), 4.14 (s, 6H), 3.88-3.78 (m, 6H), 3.62-3.55 (m, 3H), 3.41-3.34 (m, 3H), 3.25-3.17 (m, 3H), 3.06-2.91 (m, 6H), 2.83-2.72 (m, 6H), 2.60-2.51 (m, 3H), 2.13-2.03 (m, 3H), 1.72-1.61 (m, 3H), 0.88 (t, *J*=7.1Hz, 9H).

### Example 42 (Compound 126)

### Preparation of (2S,2'S)-3,3'-(((((2-((S)-2-carboxy-2-((R)-pyrrolidin-3-yl)ethyl)benzofuran-5-yl)methyl)azanediyl)bis(methylene))bis(3,1-phenylene))bis(2-((R)-pyrrolidin-3-yl)propanoic acid)

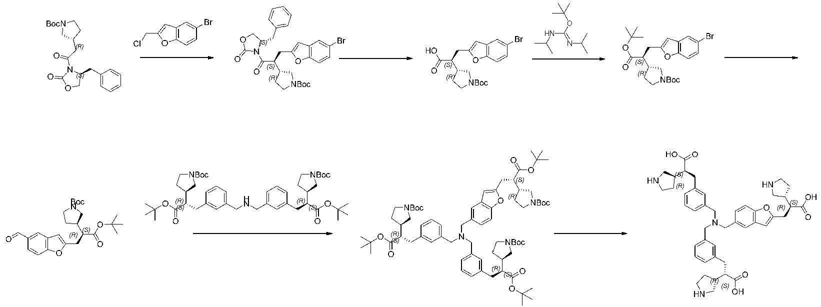

### Step 1: Synthesis of tert-butyl (R)-3-((S)-1-((S)-4-benzyl-2-oxooxazolidin-3-yl)-3-(5-bromobenzofuran-2-yl)-1-oxopropan-2-yl)pyrrolidine-1-carboxylate

Under nitrogen protection and ice bath, a solution of lithium bis(trimethylsilyl)amide (0.57mL, 0.57mmol, 1.1eq, 1.0 M in THF) was added dropwise to *tert-*butyl (R)-3-(2-((S)-4-benzyl-2-oxooxazolidin-3-yl)-2-oxoethyl)pyrrolidine-1-carboxylate (200mg, 0.51mmol, 1eq) in tetrahydrofuran (5mL). The mixture was stirred at 0°C for 30 min, and then a solution of 5-bromo-2-(chloromethyl)benzofuran (139mg, 0.57mmol, 1.1eq) in tetrahydrofuran (1mL) was added dropwise. The reaction temperature was slowly raised to room temperature and the mixture was stirred for 16h. The reaction mixture was cooled with an ice bath, and saturated aqueous ammonium chloride solution and water were added. The mixture was extracted with ethyl acetate, and the organic phase was washed with saturated aqueous sodium chloride solution, dried, filtered, and concentrated to give a crude product, which was purified by column chromatography to give the target compound (240mg). LCMS(ESI)[M+H-*tert*-butyl]⁺=541.19.

### Step 2: Synthesis of (S)-3-(5-bromobenzofuran-2-yl)-2-((R)-1-(tert-butoxycarbonyl)pyrrolidin-3-yl)propanoic acid

A solution of hydrogen peroxide (4mL, 3.51mmol, 1.5eq, 0.88 M in H₂O) was added dropwise to a solution of *tert-*butyl (R)-3-((S)-1-((S)-4-benzyl-2-oxooxazolidin-3-yl)-3-(5-bromobenzofuran-2-yl)-1-oxopropan-2-yl)pyrrolidine-1-carboxylate (1.4g, 2.34mmol, 1eq) in tetrahydrofuran (30mL), and the mixture was cooled in an ice bath. A solution of lithium hydroxide (84mg, 3.51mmol, 1.5eq) in water (1mL) was added. The reaction temperature was raised to room temperature and the mixture was stirred for 3h; LCMS showed the reaction was completed. The reaction mixture was cooled to 0°C, a solution of sodium bisulfite (1g) in water (5mL) and an aqueous solution of sodium hydroxide (5N) were added to raise the pH of the reaction mixture to >12. Water and methyl *tert-*butyl ether were added, the mixture was extracted and separated, and the organic phase was discarded. The hydrochloric acid solution (5N) was added into aqueous phase to adjust the pH to 3. The mixture was extracted with methyl *tert-*butyl ether, the organic phase was washed with water, saturated sodium chloride aqueous solution in turn, dried over anhydrous sodium sulfate, filtered and concentrated to give the target compound (0.8g). LCMS(ESI)[M+H-*tert*-butyl]⁺=382.16.

### Step 3: Synthesis of tert-butyl (R)-3-((S)-3-(5-bromobenzofuran-2-yl)-1-(tert-butoxy)-1-oxopropan-2-yl)pyrrolidine-1-carboxylate

(S)-3-(5-bromobenzofuran-2-yl)-2-((R)-1-(tert-butoxycarbonyl)pyrrolidin-3-yl)propanoic acid (870mg, 1.98mmol, 1eq) was dissolved in 2-methyltetrahydrofuran (10mL), *tert-*butyl N,N'-diisopropylcarbamimidate (1983mg, 9.9mmol, 5eq) was added, and the mixture was heated to 65°C and stirred for 16h under nitrogen protection. LCMS showed the reaction was completed. The insoluble matter was filtered, the filter cake was washed with methyl *tert-*butyl ether. The filtrate was concentrated, and separated and purified by rapid chromatography (Silica gel, petroleum ether:ethyl acetate=10:1) to give the target compound (0.8g). LCMS(ESI)[M+H-2×*tert-*butyl]⁺=382.17.

### Step 4: Synthesis of tert-butyl (R)-3-((S)-1-(tert-butoxy)-3-(5-formylbenzofuran-2-yl)-1-oxopropan-2-yl)pyrrolidine-1-carboxylate

*Tert-*butyl (R)-3-((S)-3-(5-bromobenzofuran-2-yl)-1-(tert-butoxy)-1-oxopropan-2-yl)pyrrolidine-1-carboxylate (598mg, 1.21mmol, 1eq) was dissolved in *N,N*-dimethylformamide (3mL). Palladium acetate (54mg, 0.24mmol, 0.2eq), 1,4-bis(diphenylphosphino)butane (103mg, 0.24mmol, 0.2eq), N-formylsaccharin (638mg, 3.02mmol, 2.5eq), sodium carbonate (320mg, 3.02mmol, 2.5eq) and triethylsilane (211mg, 1.81mmol, 1.5eq) were added; under nitrogen protection, the mixture was heated to 75°C and stirred for 16h. LCMS showed the reaction was completed. The insoluble matter was filtered, and the filter cake was washed with methyl *tert-*butyl ether. The filtrate was concentrated, and separated and purified by rapid column chromatography (Silica gel, PE:EA=10:1) to give the target compound (0.3g).LCMS(ESI)[M+H-Boc-*tert*-butyl]⁺=288.24.

### Step 5: Synthesis of di-tert-butyl 3,3'-((2S,2'S)-(((((2-((S)-3-(tert-butoxy)-2-((R)-1-(tert-butoxycarbonyl)pyrrolidin-3-yl)-3-oxopropyl)benzofuran-5-yl)methyl)azanediyl)bis(methylene))bis(3,1-phenylene))bis(3-(tert-butoxy)-3-oxopropane-1,2-diyl))(3R,3'R)-bis(pyrrolidine-1-carboxylate)

*Tert-*butyl (R)-3-((S)-1-(tert-butoxy)-3-(5-formylbenzofuran-2-yl)-1-oxopropan-2-yl)pyrrolidine-1-carboxylate (30mg, 0.07mmol, 1eq) and di-*tert*-butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (55mg, 0.07mmol, 1eq) were dissolved in tetrahydrofuran, sodium cyanoborohydride (8mg) and a drop of acetic acid was added. The mixture was stirred overnight at room temperature. LCMS showed the reaction was completed. The reaction was quenched with saturated ammonium chloride solution, the mixture was extracted and separated with ethyl acetate and water, and the organic phase was dried over anhydrous sodium sulfate and concentrated to give a crude product, which was purified by column chromatography to give the target compound (48mg). LCMS(ESI) [(M+H-Boc)/2]⁺=560.66.

### Step 6: Synthesis of (2S,2'S)-3,3'-(((((2-((S)-2-carboxy-2-((R)-pyrrolidin-3-yl)ethyl)benzofuran-5-yl)methyl)azanediyl)bis(methylene))bis(3,1-phenylene))bis(2-((R)-pyrrolidin-3-yl)propanoic acid)

Di-tert-butyl 3,3'-((2S,2'S)-(((((2-((S)-3-(tert-butoxy)-2-((R)-1-(tert-butoxycarbonyl)pyrrolidin-3-yl)-3-oxopropyl)benzofuran-5-yl)methyl)azanediyl)bis(methylene))bis(3,1-phenylene))bis(3-(tert-butoxy)-3-oxopropane-1,2-diyl))(3R,3'R)-bis(pyrrolidine-1-carboxylate) (35mg, 0.03mmol, 1eq) was dissolved in 1,4-dioxane (1mL), hydrochloric acid/1,4-dioxane (4 M, 1mL) was added. The mixture was stirred at room temperature overnight, and LCMS showed the reaction was completed. The solvent was removed by rotary-evaporation to dryness, and the residue was separated and purified by Prep-HPLC (C₁₈, 10mmol/L NH₄HCO₃ in water, MeCN) to give the target compound (12mg). LCMS(ESI)[M+H]⁺=751.60; ¹HNMR(400MHz, D₂O) δ 7.44 (s, 1H), 7.41-7.36 (m, 1H), 7.28-7.22 (m, 2H), 7.19-7.07 (m, 7H), 6.50 (s, 1H), 3.81-3.54 (m, 6H), 3.49-3.28 (m, 6H), 3.25-3.07 (m, 3H), 2.98-2.58 (m, 10H), 2.48-2.32 (m, 5H), 2.13-1.98 (m, 3H), 1.75-1.59 (m, 3H).

### Example 43 (Compound 127)

### Preparation of (2S,2'S,2"S)-3,3',3"-((nitrilotris(methylene))tris(benzofuran-5,3-diyl))tris(2-((R)-pyrrolidin-3-yl)propanoic acid)

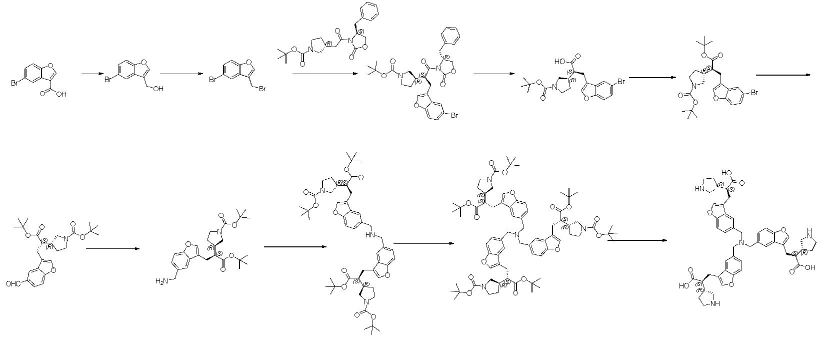

### Step 1: Synthesis of (5-bromobenzofuran-3-yl)methanol

5-bromobenzofuran-3-carboxylic acid (2g, 8.3mmol, 1eq) was dissolved in tetrahydrofuran (30mL). Under ice bath, borane/tetrahydrofuran solution (12.45mL, 12.45mmol, 1 M, 1.5eq) was added dropwise. After the addition, the mixture was slowly warmed to room temperature and stirred for 12h; TLC (petroleum ether/ethyl acetate=3/1) showed the reaction of starting material was completed. The reaction mixture was quenched with methanol, and the solvent was removed by rotary-evaporation and concentration. The residue was purified by column chromatography (ethyl acetate/petroleum ether=0-30%) to give the target compound (1.5g). ¹HNMR(400MHz, DMSO-*d*₆) δ 7.81 (d, *J*=2.1Hz, 1H), 7.53 (dt, *J*=8.7, 0.7Hz, 1H), 7.41 (dd, *J*=8.7, 2.1Hz, 1H), 6.75 (q, *J*=0.9Hz, 1H), 5.53 (t, *J*=5.9Hz, 1H), 4.57 (dd, *J*=5.9, 0.9Hz, 2H).

### Step 2: Preparation of 5-bromo-3-(bromomethyl)benzofuran

(5-bromobenzofuran-3-yl)methanol (1g, 4.4mmol, 1eq) was dissolved in tetrahydrofuran (20mL). Phosphorus tribromide (1.19g, 4.4mmol, 1eq) was added in batches under ice bath. After the addition, the mixture was warmed to room temperature and stirred for 2h; TLC (petroleum ether/ethyl acetate)=6/1 showed the reaction of starting material was completed. The reaction mixture was concentrated to remove the solvent, and the residue was purified by column chromatography (ethyl acetate/petroleum ether=0-10%) to give the target compound (1.2g). ¹HNMR(400MHz, DMSO-*d*₆) δ 7.87 (d, *J*=2.1Hz, 1H), 7.59 (d, *J*=8.8Hz, 1H), 7.49 (dd, *J*=9.1, 2.5Hz, 1H), 7.02 (s, 1H), 4.92 (s, 2H).

### Step 3: Synthesis of tert-butyl (R)-3-((S)-1-((R)-4-benzyl-2-oxooxazolidin-3-yl)-3-(5-bromobenzofuran-3-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate (2.41g, 6.21mmol, 1.2eq) was dissolved in tetrahydrofuran (25mL), lithium bis(trimethylsilyl)amide (7.24mL, 7.24mmol, 1.4eq, 1.0M) was added dropwise under ice bath. After the addition, the mixture was stirred under ice bath for 0.5h, and then a solution of 5-bromo-3-(bromomethyl)benzofuran (1.5g, 5.17mmol, 1eq) in tetrahydrofuran (15mL) was added dropwise to the reaction mixture, and the mixture was stirred at room temperature overnight. LCMS showed the reaction was completed. The reaction mixture was quenched with an ice-cold saturated ammonium chloride solution, extracted with ethyl acetate, washed with saturated brine, dried over anhydrous sodium sulfate, filtered and concentrated to give a crude product, which was purified by column chromatography (ethyl acetate/petroleum ether=0-50%) to give the target compound (2.1g).LCMS(ESI)[M+H-*tert*-butyl]⁺=541.1.

### Step 4: Synthesis of (S)-3-(5-bromobenzofuran-3-yl)-2-((R)-1-(tert-butoxycarbonyl)pyrrolidin-3-yl)propanoic acid

*Tert-*butyl (R)-3-((S)-1-((R)-4-benzyl-2-oxooxazolidin-3-yl)-3-(5-bromobenzofuran-3-yl)-1-oxopropan-2-yl)pyrrolidine-1-carboxylate (2.1g, 3.51mmol, 1eq) was dissolved in tetrahydrofuran (30mL), and hydrogen peroxide solution (239.07mg, 7.03mmol, 2eq, 30% content) was added under ice bath, and the mixture was stirred for 5min after the addition, and then an aqueous solution (3mL) of lithium hydroxide (126.26mg, 5.27mmol, 1.5eq) was added dropwise. After the addition, the mixture was slowly warmed to room temperature and stirred for 2h. LCMS showed the reaction was completed. Potassium bisulfate solution was added to quench the reaction mixture, and the mixture was extracted with ethyl acetate. The organic phase was dried over anhydrous sodium sulfate, filtered and concentrated to give the target compound (2g, crude product). LCMS(ESI)[M+H-*tert*-butyl]⁺=382.0.

### Step 5: Synthesis of tert-butyl (R)-3-((S)-3-(5-bromobenzofuran-3-yl)-1-(tert-butoxy)-1-oxopropan-2-yl)pyrrolidine-1-carboxylate

(S)-3-(5-bromobenzofuran-3-yl)-2-((R)-1-(tert-butoxycarbonyl)pyrrolidin-3-yl)propanoic acid (2g, 4.56mmol, 1eq) and *tert-*butyl N,N'-diisopropylcarbamimidate (4.57g, 22.81mmol, 5eq) were dissolved in 2-methyltetrahydrofuran (40mL), and the mixture was heated to 65°C and stirred overnight. LCMS showed the reaction of starting material was completed. The mixture was filtered, washed with 2-methyltetrahydrofuran, and the filtrate was concentrated to give a crude product, which was separated by column chromatography (ethyl acetate/petroleum ether=0-30%) to give the target compound (1.1g). LCMS(ESI)[M+H-*tert*-butylx2]⁺=382.0.

### Step 6: Synthesis of tert-butyl (R)-3-((S)-1-(tert-butoxy)-3-(5-formylbenzofuran-3-yl)-1-oxopropan-2-yl)pyrrolidine-1-carboxylate

*Tert-*butyl (R)-3-((S)-3-(5-bromobenzofuran-3-yl)-1-(tert-butoxy)-1-oxopropan-2-yl)pyrrolidine-1-carboxylate (1.1g, 2.22mmol, 1eq), 1,4-bis(diphenylphosphino)butane (189.76mg, 0.44mmol, 0.2eq), sodium carbonate (589.52mg, 5.56mmol, 2.5eq), N-formylsaccharin (1.41g, 6.67mmol, 3eq), triethylsilane (388.05mg, 1.5eq) and palladium acetate (99.9mg, 0.44mmol, 0.2eq) were added to N,N-dimethylformamide (30mL). Under nitrogen protection, the mixture was heated to 75°C and stirred for 12h. LCMS showed the reaction of starting material was completed. The reaction mixture was poured into water and extracted with ethyl acetate. The organic phase was washed with saturated brine and dried over anhydrous sodium sulfate. After filtration, the filtrate was concentrated to give a crude product, which was then separated by column chromatography (ethyl acetate/petroleum ether=0-60%) to give the target compound (700mg).LCMS(ESI)[M+H-Boc-*tert*-butyl]⁺=288.0.

### Step 7: Synthesis of tert-butyl (R)-3-((S)-3-(5-(aminomethyl)benzofuran-3-yl)-1-(tert-butoxy)-1-oxopropan-2-yl)pyrrolidine-1-carboxylate

*Tert-*butyl (R)-3-((S)-1-(tert-butoxy)-3-(5-formylbenzofuran-3-yl)-1-oxopropan-2-yl)pyrrolidine-1-carboxylate (150mg, 0.34mmol, 1eq) was added to a solution of ammonia in methanol (7 M, 3mL), and an appropriate amount of 4A molecular sieve was added. The mixture was stirred at room temperature for 40 min, and sodium cyanoborohydride (146.37mg, 2.37mmol, 7eq) was added to the reaction system. The reaction was heated at 70°C by microwave for 1h. LCMS showed the reaction of starting material was completed. The solvent was removed by rotary-evaporation, and the residue was separated by column chromatography (methanol/dichloromethane=0-10%) to give the target compound (70mg). LCMS(ESI)[M+H]⁺=445.38.

### Step 8: Synthesis of di-tert-butyl 3,3'-((2S,2'S)-((azanediylbis(methylene))bis(benzofuran-5,3-diyl))bis(3-(tert-butoxy)-3-oxopropane-1,2-diyl))(3R,3'R)-bis(pyrrolidine-1-carboxylate)

*Tert-*butyl (R)-3-((S)-3-(5-(aminomethyl)benzofuran-3-yl)-1-(tert-butoxy)-1-oxopropan-2-yl)pyrrolidine-1-carboxylate (70mg, 0.16mmol, 1eq) and *tert-*butyl (R)-3-((S)-1-(5-formylbenzofuran-3-yl)-4,4-dimethyl-3-oxopentan-2-yl)pyrrolidine-1-carboxylate (106.45mg, 0.24mmol, 1.5eq) were dissolved in tetrahydrofuran (5mL), a drop of acetic acid was added dropwise, and the mixture was stirred at room temperature for 2h. sodium cyanoborohydride (19.79mg, 0.32mmol, 2eq) was added, and the mixture was stirred for another 12h. LCMS showed the reaction of starting material was completed. The solvent was removed by rotary-evaporation, and the residue was separated by column chromatography (methanol/dichloromethane=0-10%) to give the target compound (150mg, crude). LCMS(ESI)[M+H]⁺=872.1.

### Step 9: Synthesis of tri-tert-butyl 3,3',3"-((2S,2'S,2"S)-((nitrilotris(methylene))tris(benzofuran-5,3-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((azanediylbis(methylene))bis(benzofuran-5,3-diyl))bis(3-(tert-butoxy)-3-oxopropane-1,2-diyl))(3R,3'R)-bis(pyrrolidine-1-carboxylate) (150mg, 0.17mmol, 1eq) and *tert-*butyl (R)-3-((S)-1-(5-formylbenzofuran-3-yl)-4,4-dimethyl-3-oxopentan-2-yl)pyrrolidine-1-carboxylate (114.43mg, 0.26mmol, 1.5eq) were dissolved in tetrahydrofuran (8mL). One drop of acetic acid was added dropwise, and the mixture was stirred at room temperature for 2h. sodium cyanoborohydride (21.27mg, 0.34mmol, 2eq) was added. The mixture was stirred at room temperature for 12h. LCMS showed the reaction was completed. The solvent was removed by rotary-evaporation, and the residue was separated by column chromatography (methanol/dichloromethane=0-10%) to give the target compound (150mg). LCMS(ESI)[M+H]⁺=1299.2.

### Step 10: Synthesis of (2S,2'S,2"S)-3,3',3"-((nitrilotris(methylene))tris(benzofuran-5,3-diyl))tris(2-((R)-pyrrolidin-3-yl)propanoic acid)

Tri-*tert*-butyl 3,3',3"-((2S,2'S,2"S)-((nitrilotris(methylene))tris(benzofuran-5,3-diyl))tris(3-(tert-butoxy)-3-oxopropane-1,2-diyl))(3R,3'R,3"R)-tris(pyrrolidine-1-carboxylate) (100mg, 0.08mmol, 1eq) was dissolved in dichloromethane (5mL), and trifluoroacetic acid (91.22mg, 0.8mmol, 10eq) was added dropwise. After the addition, the mixture was stirred at room temperature for 12h. LCMS showed the reaction of starting material was completed. The solvent was removed by rotary-evaporation, and the residue was dissolved in acetonitrile, filtered, and the filtrate was separated and purified by Prep-HPLC (C₁₈, 0.1%FA in water, MeCN) to give the target compound (7.02mg). LCMS(ESI)[M+H]⁺=831.1; ¹HNMR(400MHz, D₂O) δ 6.99 (s, 6H), 6.68 (s, 3H), 6.06 (s, 3H), 3.10 (s, 6H), 2.75 (d, J=71.9Hz, 11H), 2.45 (s, 4H), 2.24 (s, 5H), 1.96 (s, 3H), 1.71 (s, 4H), 1.44 (s, 3H).

### Example 44 (Compound 128)

### Preparation of (2S,2'S,2"S)-3,3',3"-((nitrilotris(methylene))tris(benzo[b]thiophene-5,3-diyl))tris(2-((R)-pyrrolidin-3-yl)propanoic acid)

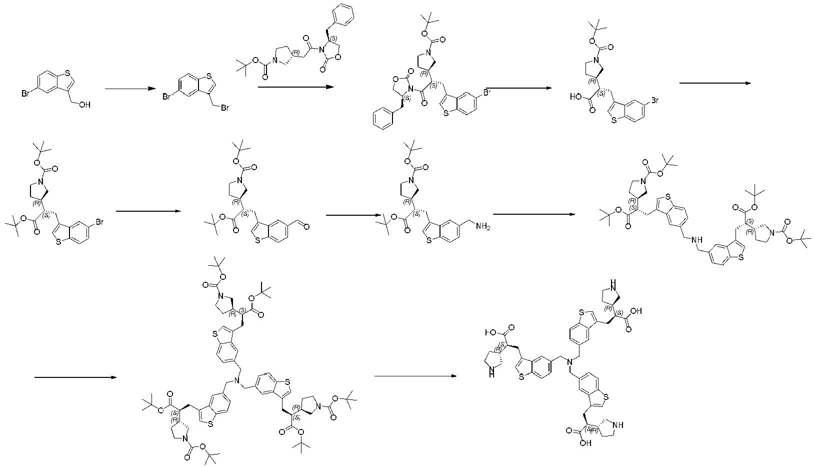

### Step 1: Synthesis of 5-bromo-3-(bromomethyl)benzo[b]thiophene

(5-bromobenzo[b]thiophen-3-yl)methanol(900mg, 3.7mmol, 1eq) was dissolved in tetrahydrofuran (10mL). Phosphorus tribromide (1.0g, 3.7mmol, 1eq) was added in batches under ice bath. After the addition, the mixture was slowly warmed to room temperature and stirred for 2h. TLC (petroleum ether/ethyl acetate)=8/1 showed the reaction of starting material was completed. The reaction mixture was concentrated to give a crude product, which was purified by column chromatography (ethyl acetate/petroleum ether=0-10%) to give the target compound (1g). ¹HNMR(400MHz, DMSO-*d*₆) δ 8.15 (d, *J*=2.0Hz, 1H), 8.04 (s, 1H), 8.02 (d, *J*=8.6Hz, 1H), 7.58 (dd, *J*=8.5, 2.0Hz, 1H), 5.04 (s, 2H).

### Step 2: Synthesis of tert-butyl (R)-3-((S)-1-((S)-4-benzyl-2-oxooxazolidin-3-yl)-3-(5-bromobenzo[b]thiophen-3-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate(1.42g, 3.65mmol, 1.2eq) was dissolved in tetrahydrofuran (25mL), lithium bis(trimethylsilyl)amide (711.93mg, 4.25mmol, 1 M, 1.4eq) was added dropwise under ice bath. After the addition, the mixture was stirred under ice bath for 30min. A solution of 5-bromo-3-(bromomethyl)benzo[b]thiophene (930mg, 3.04mmol, 1eq) in tetrahydrofuran (15mL) was slowly added to the reaction mixture. The temperature of the reaction system was slowly warmed to room temperature and the mixture was stirred for 2h; LCMS showed the reaction of starting material was completed. The reaction mixture was completely quenched with a saturated ammonium chloride ice water solution, extracted with ethyl acetate, and the organic phase was dried over anhydrous sodium sulfate, filtered, and the filtrate was concentrated to give a crude product, which was purified by column chromatography (ethyl acetate/petroleum ether=0-30%) to give the target compound (1.3g).LCMS(ESI)[M+H-*tert*-butyl]⁺=557.1

### Step 3: Synthesis of (S)-3-(5-bromobenzo[b]thiophen-3-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(5-bromobenzo[b]thiophen-3-yl)-1-oxopropan-2-yl)pyrrolidine-1-carboxylate (1.2g, 1.96mmol, 1eq) was dissolved in tetrahydrofuran (40mL), a hydrogen peroxide solution (119.73mg, 3.52mmol, 1.8eq, 30% content) was added dropwise under ice bath, followed by dropwise addition of a solution of lithium hydroxide (70.26mg, 2.93mmol, 1.5eq) in water (10mL). After the addition, the mixture was continually stirred for 2h. LCMS showed the reaction of starting material was completed. The reaction mixture was quenched with a potassium bisulfate solution, extracted with ethyl acetate, and the organic phase was dried over anhydrous sodium sulfate, filtered, and concentrated to give the target compound (1.2g, crude product).LCMS(ESI)[M+H-*tert*-butyl]⁺=398.0.

### Step 4: Synthesis of tert-butyl (R)-3-((S)-3-(5-bromobenzo[b]thiophen-3-yl)-1-(tert-butoxy)-1-oxopropan-2-yl)pyrrolidine-1-carboxylate

(S)-3-(5-bromobenzo[b]thiophen-3-yl)-2-((R)-1-(tert-butoxycarbonyl)pyrrolidin-3-yl)propanoic acid (1.2g, 2.64mmol, 1eq) was dissolved in 2-methyltetrahydrofuran (30mL), and *tert-*butyl N,N'-diisopropylcarbamimidate (2.12g, 10.56mmol, 4eq) was added; under nitrogen, the reaction mixture was heated to 65°C and stirred for 12h. LCMS showed the reaction of starting material was completed, the solvent was removed by concentration, and the residue was purified by column chromatography (ethyl acetate/petroleum ether=0-30%) to give the target compound (500mg). LCMS(ESI)[M+H-*tert*-butyl*2]⁺=398.0.

### Step 5: Synthesis of tert-butyl (R)-3-((S)-1-(tert-butoxy)-3-(5-formylbenzo[b]thiophen-3-yl)-1-oxopropan-2-yl)pyrrolidine-1-carboxylate

*Tert-*butyl (R)-3-((S)-3-(5-bromobenzo[b]thiophen-3-yl)-1-(tert-butoxy)-1-oxopropan-2-yl)pyrrolidine-1-carboxylate (500mg, 0.98mmol, 1eq), 1,4-bis(diphenylphosphino)butane (83.54mg, 0.2mmol, 0.2eq), sodium carbonate (259.54mg, 2.45mmol, 2.5eq), N-formylsaccharin (827.42mg, 3.92mmol, 4eq), triethylsilane (170.84mg, 1.47mmol, 1.5eq) and palladium acetate (43.98mg, 0.2mmol, 0.2eq) were added to N,N-dimethylformamide (15mL), under nitrogen, the mixture was heated to 75°C and stirred for 12h; LCMS showed the reaction of starting material was completed. The reaction mixture was poured into water, extracted with ethyl acetate and the organic phase was washed with saturated brine, dried over anhydrous sodium sulfate, filtered, and the filtrate was concentrated to give a crude product, which was purified by column chromatography (ethyl acetate/petroleum ether=0-70%) to give the target compound (170mg). LCMS(ESI)[M+H-Boc]⁺=360.0.

### Step 6: Synthesis of tert-butyl (R)-3-((S)-3-(5-(aminomethyl)benzo[b]thiophen-3-yl)-1-(tert-butoxy)-1-oxopropan-2-yl)pyrrolidine-1-carboxylate

*Tert-*butyl (R)-3-((S)-1-(tert-butoxy)-3-(5-formylbenzo[b]thiophen-3-yl)-1-oxopropan-2-yl)pyrrolidine-1-carboxylate (60mg, 0.13mmol, 1eq) was added to ammonia methanol solution (2mL, 7 M) with an appropriate amount of 4A molecular sieve, and the mixture was stirred at room temperature for 40 min. sodium cyanoborohydride (56.5mg, 0.91mmol, 7eq) was added. The reaction was carried out under microwave irradiation at 70°C for 1h. LCMS showed the reaction of starting material was completed. The reaction mixture was concentrated to give a crude product, which was purified by column chromatography (methanol/dichloromethane=0-10%) to give the target compound (35mg). LCMS(ESI)[M+H]⁺=461.0.

### Step 7: Synthesis of di-tert-butyl 3,3'-((2S,2'S)-((azanediylbis(methylene))bis(benzo[b]thiophene-5,3-diyl))bis(3-(tert-butoxy)-3-oxopropane-1,2-diyl))(3R,3'R)-bis(pyrrolidine-1-carboxylate)

*Tert-*butyl (R)-3-((S)-3-(5-(aminomethyl)benzo[b]thiophen-3-yl)-1-(tert-butoxy)-1-oxopropan-2-yl)pyrrolidine-1-carboxylate (30mg, 0.07mmol, 1eq) and *tert-*butyl (R)-3-((S)-1-(tert-butoxy)-3-(5-formylbenzo[b]thiophen-3-yl)-1-oxopropan-2-yl)pyrrolidine-1-carboxylate (48.26mg, 0.11mmol, 1.5eq) were dissolved in tetrahydrofuran (3mL), and a drop of acetic acid was added dropwise. The reaction mixture was stirred at room temperature for 2h. sodium cyanoborohydride (8.66mg, 0.14mmol, 2eq) was added, and the mixture was stirred for 12h; LCMS showed the reaction of starting material was completed. The reaction mixture was concentrated to give a crude product, which was purified by column chromatography (methanol/dichloromethane=0-10%) to give the target compound (40mg). LCMS(ESI)[M+H]⁺=904.1.

### Step 8: Synthesis of tri-tert-butyl 3,3',3"-((2S,2'S,2"S)-((nitrilotris(methylene))tris(benzo[b]thiophene-5,3-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((azanediylbis(methylene))bis(benzo[b]thiophene-5,3-diyl))bis(3-(tert-butoxy)-3-oxopropane-1,2-diyl))(3R,3'R)-bis(pyrrolidine-1-carboxylate) (40mg, 0.04mmol, 1eq) and *tert-*butyl (R)-3-((S)-1-(tert-butoxy)-3-(5-formylbenzo[b]thiophen-3-yl)-1-oxopropan-2-yl)pyrrolidine-1-carboxylate (30.5mg, 0.07mmol, 1.5eq) were dissolved in tetrahydrofuran (4mL), a drop of acetic acid was added dropwise, and the reaction mixture was stirred at room temperature for 2h. sodium cyanoborohydride (4.1mg, 0.07mmol, 1.5eq) was added, and the mixture was stirred for 12h; LCMS showed the reaction of starting material was completed. The reaction mixture was concentrated to remove the solvent and give a crude product, which was purified by column chromatography (methanol/dichloromethane=0-10%) to give the target compound (20mg). LCMS(ESI)[M+H]⁺=1347.1.

### Step 9: Synthesis of (2S,2'S,2"S)-3,3',3"-((nitrilotris(methylene))tris(benzo[b]thiophene-5,3-diyl))tris(2-((R)-pyrrolidin-3-yl)propanoic acid)

Tri-*tert*-butyl 3,3',3"-((2S,2'S,2"S)-((nitrilotris(methylene))tris(benzo[b]thiophene-5,3-diyl))tris(3-(tert-butoxy)-3-oxopropane-1,2-diyl))(3R,3'R,3"R)-tris(pyrrolidine-1-carboxylate) (20mg, 0.01mmol) was dissolved in dichloromethane (5mL), and trifluoroacetic acid (11.4mg, 0.1mmol, 10eq) was added dropwise. After the addition, the reaction mixture was stirred at room temperature for 12h. LCMS showed the reaction of starting material was completed. The reaction mixture was concentrated to remove the solvent, and the residue was dissolved in acetonitrile, filtered, and separated and purified by Prep-HPLC (C₁₈, 0.1%FA in water, MeCN) to give the target compound (1.64mg). LCMS(ESI)[M+H]⁺=879.0; ¹HNMR(400MHz, D₂O) δ 8.16-7.57 (m, 3H), 7.53-6.56 (m, 9H), 3.41-2.70 (m, 15H), 2.56-1.95 (m, 12H), 1.93-1.33 (m, 9H).

### Example 45 (Compound 130)

### Preparation of (2S,2'S)-3,3'-((((2-(3-((S)-2-carboxy-2-((R)-pyrrolidin-3-yl)ethyl)phenyl)-2-oxoethyl)azanediyl)bis(methylene))bis(3,1-phenylene))bis(2-((R)-pyrrolidin-3-yl)propanoic acid)

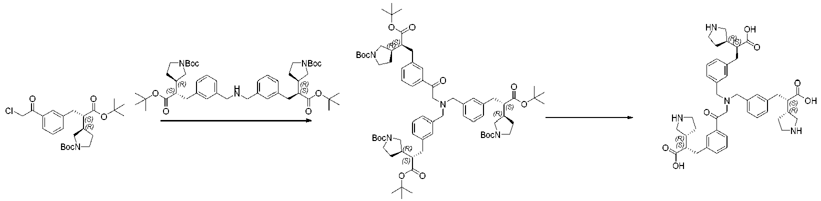

### Step 1: Synthesis of di-tert-butyl 3,3'-((2S,2'S)-((((2-(3-((S)-3-(tert-butoxy)-2-((R)-1-(tert-butoxycarbonyl)pyrrolidin-3-yl)-3-oxopropyl)phenyl)-2-oxoethyl)azanediyl)bis(methylene))bis(3,1-phenylene))bis(3-(tert-butoxy)-3-oxopropane-1,2-diyl))(3R,3'R)-bis(pyrrolidine-1-carboxylate)

*Tert-*butyl (R)-3-((S)-1-(tert-butoxy)-3-(3-(2-chloroacetyl)phenyl)-1-oxopropan-2-yl)pyrrolidine-1-carboxylate(120mg, 0.27mmol, 1.0eq) was dissolved in DMF (5mL), followed by adding di-*tert-*butyl 3,3'-((2S,2'S)-((azanediylbis(methylene))bis(3,1-phenylene))bis(3-(tert-butoxy)-3-oxopropane-1,2-diyl))(3R,3'R)-bis(pyrrolidine-1-carboxylate) (252.35mg, 0.32mmol, 1.2eq) and potassium carbonate (110mg, 0.8mmol, 3.0eq). The mixture was stirred at 105°C for 24h. LCMS showed that the reaction was completed. The reaction mixture was directly concentrated, separated and purified by rapid chromatography (Silica gel, petroleum ether:ethyl acetate=1:8) to give the target compound (100mg). LCMS(ESI)[M+H]⁺=1207.2.

### Step 2: Synthesis of (2S,2'S)-3,3'-((((2-(3-((S)-2-carboxy-2-((R)-pyrrolidin-3-yl)ethyl)phenyl)-2-oxoethyl)azanediyl)bis(methylene))bis(3,1-phenylene))bis(2-((R)-pyrrolidin-3-yl)propanoic acid)

Di-*tert*-butyl 3,3'-((2S,2'S)-((((2-(3-((S)-3-(tert-butoxy)-2-((R)-1-(tert-butoxycarbonyl)pyrrolidin-3-yl)-3-oxopropyl)phenyl)-2-oxoethyl)azanediyl)bis(methylene))bis(3,1-phenylene))bis(3-(tert-butoxy)-3-oxopropane-1,2-diyl))(3R,3'R)-bis(pyrrolidine-1-carboxylate) (100mg, 0.08mmol) was dissolved in hydrochloric acid/1,4-dioxane (2mL), and the mixture was stirred at room temperature for 36h. LCMS showed the reaction was completed. The reaction mixture was concentrated directly and separated and purified by preparative chromatography to give the target compound (45.78mg).LCMS(ESI)[M+H]⁺=739.1; ¹HNMR(400MHz, Deuterium oxide) δ 7.52-7.38 (m, 3H), 7.31 (t, J=7.7Hz, 1H), 7.21 (t, J=7.4Hz, 2H), 7.14-7.05 (m, 5H), 3.83 (d, J=5.3Hz, 2H), 3.70 (d, J=5.0Hz, 3H), 3.55-3.02 (m, 10H), 3.01-2.62 (m, 8H), 2.61-2.52 (m, 2H), 2.45-2.27 (m, 6H), 2.03 (dt, J=6.5, 3.6Hz, 3H), 1.71-1.58 (m, 3H).

### Example 46 (Compound 131)

### Preparation of (S)-3-(3-(3-(3-((S)-2-carboxy-2-((R)-pyrrolidin-3-yl)ethyl)benzyl)-3-(2-(3-((S)-2-carboxy-2-((R)-pyrrolidin-3-yl)ethyl)phenoxy)ethyl)ureido)phenyl)-2-((R)-pyrrolidin-3-yl)propanoic acid

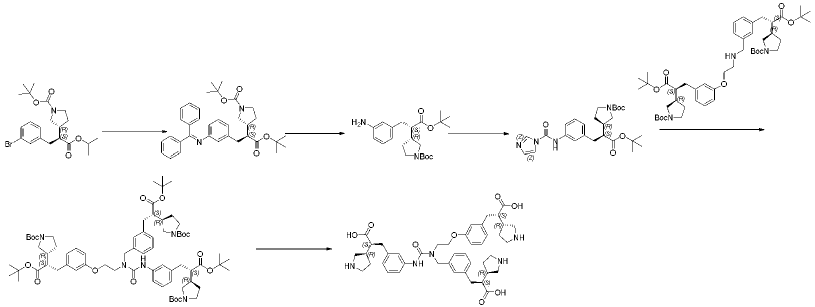

### Step 1: Synthesis of tert-butyl (R)-3-((S)-1-(tert-butoxy)-3-(3-((diphenylmethylene)amino)phenyl)-1-oxopropan-2-yl)pyrrolidine-1-carboxylate

*Tert-*butyl (R)-3-((S)-3-(3-bromophenyl)-1-isopropoxy-1-oxopropan-2-yl)pyrrolidine-1-carboxylate(150mg, 0.34mmol, 1eq), sodium tert-butoxide (50mg, 0.51mmol, 1.5eq), 1,1'-binaphthalene-2,2'-bis(diphenylphosphine) (15mg, 0.02mmol, 0.07eq) and tris(dibenzylideneacetone)dipalladium(0) (9mg, 0.01mmol, 0.03eq) were added in 1,4-dioxane (2mL), followed by adding benzophenone imine (75mg, 0.41mmol, 1.2eq). The reaction mixture was stirred at 90°C for 4h under nitrogen. LCMS showed the reaction of starting material was completed. The reaction mixture was cooled to room temperature and extracted with ethyl acetate and water. The organic phase was dried over anhydrous sodium sulfate, filtered and concentrated to give a crude product, which was purified by column chromatography (Silica gel, petroleum ether:ethyl acetate=10:1) to give the target compound (162mg). LCMS(ESI)[M+H]⁺=555.50.

### Step 2: Synthesis of tert-butyl (R)-3-((S)-3-(3-aminophenyl)-1-(tert-butoxy)-1-oxopropan-2-yl)pyrrolidine-1-carboxylate

The product from the first step (400mg, 0.72mmol, 1eq) was dissolved in THF (10mL), and citric acid (276mg, 1.44mmol, 2eq) was added. The mixture was stirred overnight at room temperature, and LCMS showed the reaction was completed. The reaction mixture was concentrated and purified by column chromatography (Silica gel, petroleum ether:ethyl acetate=10:1) to give the target product (250mg). LCMS(ESI)[M+H-Boc]⁺=291.0.

### Step 3: Synthesis of tert-butyl (R)-3-((S)-3-(3-(1H-imidazole-1-carboxamido)phenyl)-1-(tert-butoxy)-1-oxopropan-2-yl)pyrrolidine-1-carboxylate

The product from the second step (15mg, 0.04mmol, 1eq) and N,N'-carbonyldiimidazole (6.85mg, 0.044mmol, 1.1eq) were dissolved in DCM (0.5mL), and then triethylamine (7.77mg, 0.08mmol, 2eq) was added. The mixture was stirred at room temperature for 2h, and LCMS showed the reaction of starting material was completed. The solvent was removed to dryness, and the crude product was directly used in the next step.

### Step 4: Synthesis of tert-butyl (R)-3-((S)-1-(tert-butoxy)-3-(3-((1-(2-(3-((S)-3-(tert-butoxy)-2-((R)-1-(tert-butoxycarbonyl)pyrrolidin-3-yl)-3-oxopropyl)phenoxy)ethyl)-3-(3-((S)-3-(tert-butoxy)-2-((R)-1-(tert-butoxycarbonyl)pyrrolidin-3-yl)-3-oxopropyl)phenyl)ureido)methyl)phenyl)-1-oxopropan-2-yl)pyrrolidine-1-carboxylate

The product from the third step (45mg, 0.09mmol, 1eq), *tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate(75mg, 0.09mmol, 1eq) was dissolved in THF (1mL), and triethylamine (18mg, 0.18mmol, 2eq) was added. The mixture was stirred overnight at room temperature, and LCMS showed the reaction was completed. Ethyl acetate and water were added, and the mixture was extracted. The organic phase was dried over anhydrous sodium sulfate, filtered, concentrated, and purified by column chromatography (Silica gel, petroleum ether:ethyl acetate=10:1) to give the target product (60mg). LCMS(ESI)[M+H-Boc]⁺=1138.95.

### Step 5: Synthesis of (S)-3-(3-(3-(3-((S)-2-carboxy-2-((R)-pyrrolidin-3-yl)ethyl)benzyl)-3-(2-(3-((S)-2-carboxy-2-((R)-pyrrolidin-3-yl)ethyl)phenoxy)ethyl)ureido)phenyl)-2-((R)-pyrrolidin-3-yl)propanoic acid

The product from the fourth step (90mg, 0.07mmol, 1eq) was dissolved in dioxane (2mL), and hydrochloric acid/1,4-dioxane (2mL, 4.0M) was added in ice bath; the mixture was stirred at room temperature for 24h. LCMS showed the reaction was completed. The solvent was removed to dryness, and the residue was separated and purified by Prep-HPLC (C₁₈, 10mmol/L NH₄HCO₃ in water, MeCN) to give the target compound (10.7mg). LCMS(ESI)[M+H]⁺=770.64; ¹HNMR(400MHz, D₂O) δ 7.40-7.25 (m, 6H), 7.24-7.02 (m, 6H), 4.14 (s, 6H), 3.88-3.78 (m, 6H), 3.62-3.55 (m, 3H), 3.41-3.34 (m, 3H), 3.25-3.17 (m, 3H), 3.06-2.91 (m, 6H), 2.83-2.72 (m, 6H), 2.60-2.51 (m, 3H), 2.13-2.03 (m, 3H), 1.72-1.61 (m, 3H), 0.88 (t, *J*=7.1Hz, 9H).

### Example 47 (Compound 132)

### Preparation of (2S,2'S)-3,3'-(((2-(((3-((S)-2-carboxy-2-((R)-pyrrolidin-3-yl)ethyl)benzyl)oxy)methyl)piperazine-1,4-diyl)bis(methylene))bis(3,1-phenylene))bis(2-((R)-pyrrolidin-3-yl)propanoic acid)

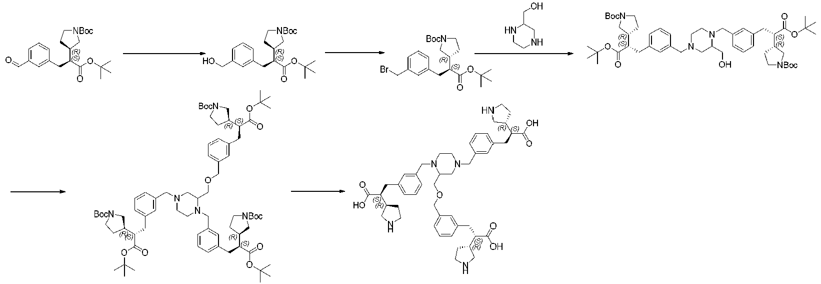

### Step 1: Synthesis of tert-butyl (R)-3-((S)-1-(tert-butoxy)-3-(3-(hydroxymethyl)phenyl)-1-oxopropan-2-yl)pyrrolidine-1-carboxylate

*Tert-*butyl (R)-3-((S)-1-(tert-butoxy)-3-(3-formylphenyl)-1-oxopropan-2-yl)pyrrolidine-1-carboxylate(2g, 4.96mmol, 1eq) was dissolved in methanol (20mL), and sodium borohydride (0.56g, 14.87mmol, 3eq) was added. The reaction mixture was stirred at 0°C for 1h. After the reaction was completed, water (40mL) was added to quench the reaction mixture, followed by extraction with ethyl acetate (30mL×3). The organic phase was dried over anhydrous sodium sulfate, filtered, and concentrated to give a crude product, which was purified by column chromatography (petroleum ether:ethyl acetate=3:1) to give the target compound (1.8g). LCMS(ESI)[M+Na]⁺=428.3; ¹HNMR(400MHz, DMSO-*d*₆) δ 7.21 (t, *J*=7.7Hz, 1H), 7.15-7.10 (m, 2H), 7.03 (d, *J*=7.4Hz, 1H), 5.13 (t, *J*=5.6Hz, 1H), 4.45 (d, *J*=5.4Hz, 2H), 3.57-3.42 (m, 1H), 3.39-3.32 (m, 1H), 3.20-3.08 (m, 1H), 2.98 (t, *J*=9.7Hz, 1H), 2.80-2.68 (m, 2H), 2.48-2.41 (m, 1H), 2.34-2.20 (m, 1H), 1.88-1.78 (m, 1H), 1.64-1.51 (m, 1H), 1.40 (s, 9H), 1.24 (s, 9H).

### Step 2: Synthesis of tert-butyl (R)-3-((S)-3-(3-(bromomethyl)phenyl)-1-(tert-butoxy)-1-oxopropan-2-yl)pyrrolidine-1-carboxylate

The product from first step (2.2g, 5.42mmol, 1eq) was dissolved in dichloromethane (30mL), and triphenylphosphine (2.14g, 8.14mmol, 1.5eq) and carbon tetrabromide (2.7g, 8.14mmol, 1.5eq) were added at 0°C; the reaction mixture was stirred at 0°C for 1h under nitrogen protection. After the reaction was completed, water was added to the reaction mixture, which was extracted with dichloromethane. The organic phase was dried over anhydrous sodium sulfate, filtered and concentrated, and the crude product was purified by column chromatography (petroleum ether:ethyl acetate=5:1) to give the target compound (1.7g). LCMS(ESI)[M+Na]⁺=490.1; ¹HNMR(400MHz, DMSO-*d*₆) δ 7.29-7.24 (m, 3H), 7.17-7.10 (m, 1H), 4.65 (s, 2H), 3.57-3.48 (m, 1H), 3.39-3.32 (m, 2H), 3.19-3.10 (m, 1H), 2.99 (t, *J*=10.0Hz, 1H), 2.78-2.66 (m, 2H), 2.34-2.24 (m, 1H), 1.89-1.79 (m, 1H), 1.63-1.54 (m, 1H), 1.40 (s, 9H), 1.22 (s, 9H).

### Step 3: Synthesis of di-tert-butyl 3,3'-((2S,2'S)-(((2-(hydroxymethyl)piperazine-1,4-diyl)bis(methylene))bis(3,1-phenylene))bis(3-(tert-butoxy)-3-oxopropane-1,2-diyl))(3R,3'R)-bis(pyrrolidine-1-carboxylate)

Piperazin-2-ylmethanol(40mg, 0.34mmol, 1eq) was dissolved in acetonitrile (3mL), and the product from the second step (477.79mg, 1.02mmol, 3eq) and *N,N*-diisopropylethylamine (0.16mL, 1.02mmol, 3eq) were added; the mixture was stirred at 70°C for 18h under nitrogen. The reaction mixture was concentrated to give a crude product, which was separated and purified by rapid chromatography (Silica gel, tetrahydrofuran:petroleum ether=1:1) to give the target compound (120mg). LCMS(ESI)[M+H]⁺=891.4.

### Step 4: Synthesis of di-tert-butyl 3,3'-((2S,2'S)-(((2-(((3-((S)-3-(tert-butoxy)-2-((R)-1-(tert-butoxycarbonyl)pyrrolidin-3-yl)-3-oxopropyl)benzyl)oxy)methyl)piperazine-1,4-diyl)bis(methylene))bis(3,1-phenylene))bis(3-(tert-butoxy)-3-oxopropane-1,2-diyl))(3R,3'R)-bis(pyrrolidine-1-carboxylate)

*Tert-*butyl (R)-3-((S)-3-(3-(bromomethyl)phenyl)-1-(tert-butoxy)-1-oxopropan-2-yl)pyrrolidine-1-carboxylate (63.07mg, 0.13mmol, 1.5eq) was dissolved in dichloromethane (5mL), and silver oxide (16.64mg, 0.07mmol, 0.8eq), tetrabutylammonium iodide (16.58mg, 0.04mmol, 0.5eq) and the product from the third step (80mg, 0.09mmol, 1eq) were added; the mixture was stirred at 25°C for 18h under nitrogen protection. The reaction mixture was filtered, concentrated, and purified by TLC silica gel plate (ethyl acetate:petroleum ether=1:1) to give the target product (35mg). LCMS(ESI)[M+H]⁺=1279.0.

### Step 5: Synthesis of (2S,2'S)-3,3'-(((2-(((3-((S)-2-carboxy-2-((R)-pyrrolidin-3-yl)ethyl)benzyl)oxy)methyl)piperazine-1,4-diyl)bis(methylene))bis(3,1-phenylene))bis(2-((R)-pyrrolidin-3-yl)propanoic acid)

The product from the fourth step (30mg, 0.02mmol, 1eq) was dissolved in dioxane (1mL), and hydrochloric acid/1,4-dioxane (1mL, 4M) was added; the mixture was stirred at 25°C for 3h under nitrogen. The reaction mixture was concentrated and purified by reversed-phase preparation (acetonitrile/ammonium bicarbonate system) to give the target compound (2.12mg). LCMS(ESI)[M+H]⁺=810.4; ¹HNMR(400MHz, D₂O) δ 7.28-7.20 (m, 3H), 7.16-7.09 (m, 7H), 7.07-7.01 (m, 2H), 4.46 (s, 2H), 3.96 (t, *J*=13.0Hz, 1H), 3.79-3.66 (m, 1H), 3.57-3.50 (m, 1H), 3.50-3.43 (m, 2H), 3.42-3.26 (m, 6H), 3.20-3.09 (m, 4H), 2.90-2.80 (m, 3H), 2.80-2.59 (m, 10H), 2.59-2.52 (m, 1H), 2.43-2.31 (m, 6H), 2.19-2.09 (m, 2H), 2.06-1.99 (m, 3H), 1.71-1.58 (m, 3H).

### Example 48 (Compound 133)

### Preparation of (2S,2'S,2"S)-3,3',3"-((nitrilotris(methylene))tris(1-methyl-1H-benzo[d]imidazole-6,2-diyl))tris(2-((R)-pyrrolidin-3-yl)propanoic acid):

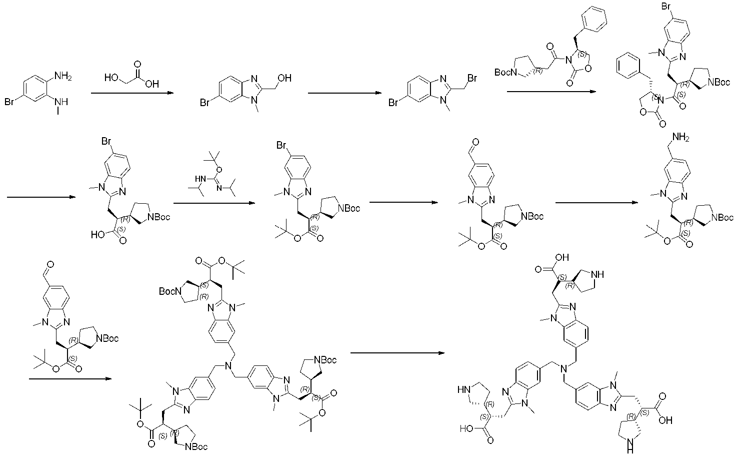

### Step 1: Synthesis of (6-bromo-1-methyl-1H-benzo[d]imidazol-2-yl)methanol

5-bromo-N1-methylbenzene-1,2-diamine(25g, 124.34mmol, 1.0eq) was dissolved in water (160mL), hydrochloric acid (110mL, 6 M) and 2-hydroxyacetic acid(47.28g, 621.7mmol, 5.0eq) were added. The reaction mixture was stirred at 100°C for 2h. After the reaction was completed, the mixture was cooled to room temperature and adjusted to pH=9 with ammonia water. The mixture was filtered, and the filter cake was washed with water and dried to give the target compound (22g). LCMS(ESI)[M+H]⁺=241.0; ¹HNMR(400MHz, CDCl₃) δ 7.52 (d, *J*=8.6Hz, 1H), 7.44 (d, *J*=1.7Hz, 1H), 7.35 (dd, *J*=8.5, 1.8Hz, 1H), 4.87 (s, 2H), 3.78 (s, 3H).

### Step 2: Synthesis of 6-bromo-2-(bromomethyl)-1-methyl-1H-benzo[d]imidazole

(6-bromo-1-methyl-1H-benzo[d]imidazol-2-yl)methanol (22g, 91.26mmol, 1.0eq) was dissolved in dichloromethane (500mL), and phosphorus tribromide (247.02g, 912.56mmol, 10.0eq) was slowly added in batches under ice bath; the reaction mixture was reacted at room temperature for 16h. The reaction system was poured into water to quench, and the pH was adjusted to 9 with aqueous sodium carbonate solution. Dichloromethane (1 L) was added for extraction and separation, the organic phase was washed with saturated sodium chloride solution, dried over anhydrous sodium sulfate, filtered, and concentrated to give a crude product, which was purified by silica gel column chromatography (petroleum ether/ethyl acetate=2/1) to give the target compound (22g). LCMS(ESI)[M+H]⁺=302.9; ¹HNMR(400MHz, CDCl₃) δ 7.59 (d, *J*=8.6Hz, 1H), 7.48 (s, 1H), 7.37 (dd, *J*=8.6, 1.1Hz, 1H), 4.65 (s, 2H), 3.79 (s, 3H).

### Step 3: Synthesis of tert-butyl (R)-3-((S)-1-((S)-4-benzyl-2-oxooxazolidin-3-yl)-3-(6-bromo-1-methyl-1H-benzo[d]imidazol-2-yl)-1-oxopropan-2-yl)pyrrolidine- 1-carboxylate

*Tert-*butyl (R)-3-(2-((S)-4-benzyl-2-oxooxazolidin-3-yl)-2-oxoethyl)pyrrolidine-1-carboxylate(5.0g, 12.87mmol, 1eq) was dissolved in tetrahydrofuran (70mL), and lithium bis(trimethylsilyl)amide (18.02mL, 18.02mmol, 1.4eq) was added dropwise at 0°C under nitrogen protection, and the mixture was stirred at 0°C for 1h; 6-bromo-2-(bromomethyl)-1-methyl-1H-benzo[d]imidazole (5.87g, 19.31mmol, 1.5eq) was dissolved in tetrahydrofuran. Under ice bath, the mixture was added dropwise to the reaction system, which was stirred at room temperature for 2h. The reaction mixture was quenched with aqueous ammonium chloride solution (200mL). The mixture was extracted with ethyl acetate, and the organic phase was washed with saturated sodium chloride solution, dried over anhydrous sodium sulfate, filtered, and concentrated to dryness, and the crude product was separated and purified by rapid chromatography (silica gel, petroleum ether/tetrahydrofuran=3/1) to give the target compound (6.4g). LCMS(ESI)[M+H]⁺=611.1; ¹HNMR(400MHz, DMSO-*d*₆) δ 7.75 (d, *J*=1.8Hz, 1H), 7.43 (d, *J*=8.5Hz, 1H), 7.23-7.17 (m, 6H), 4.64 (s, 2H), 4.28 (t, *J*=8.3Hz, 1H), 4.06 (d, *J*=8.7Hz, 1H), 3.74 (s, 3H), 3.51-3.30 (m, 3H), 3.16-3.06 (m, 2H), 3.02 (t, *J*=10.2Hz, 1H), 2.89-2.80 (m, 1H), 2.77-2.67 (m, 1H), 2.56-2.47 (m, 1H), 1.93-1.84 (m, 1H), 1.82-1.65 (m, 1H), 1.36 (s, 9H).

### Step 4: Synthesis of (S)-3-(6-bromo-1-methyl-1H-benzo[d]imidazol-2-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(6-bromo-1-methyl-1H-benzo[d]imidazol-2-yl)-1-oxopropan-2-yl)pyrrolidine-1-carboxylate (6.5g, 10.63mmol, 1.0eq) was dissolved in tetrahydrofuran (80mL), a solution of hydrogen peroxide (13mL) was added under ice bath. Lithium hydroxide (381.85mg, 15.94mmol, 1.5eq) was dissolved in water and then added dropwise to the reaction system under ice bath; the mixture was stirred at room temperature for 3h. LCMS showed the reaction was completed. Sodium sulfite was added to quench the reaction mixture, and aqueous sodium carbonate was added to adjust the pH=10. The mixture was extracted with ethyl acetate. The aqueous phase was retained, and its pH was adjusted to 4 with dilute hydrochloric acid (1M). The mixture was extracted with dichloromethane (100mL×3), and the organic phases were combined, washed with saturated sodium chloride solution, dried over anhydrous sodium sulfate, filtered, and concentrated to dryness to give the target product (3.6g). LCMS(ESI)[M+H]⁺=452.1; ¹HNMR(400MHz, DMSO-*d*₆) δ 12.46 (s, 1H), 7.87 (d, *J*=1.2Hz, 1H), 7.52 (d, *J*=8.5Hz, 1H), 7.33 (dd, *J*=8.5, 1.6Hz, 1H), 3.78 (s, 3H), 3.51-3.35 (m, 2H), 3.25-3.12 (m, 2H), 3.08-2.95 (m, 3H), 2.47-2.36 (m, 1H), 2.00-1.92 (m, 1H), 1.69 (dd, J=21.2, 10.7Hz, 1H), 1.38 (d, J=9.6Hz, 9H).

### Step 5: Synthesis of tert-butyl (R)-3-((S)-3-(6-bromo-1-methyl-1H-benzo[d]imidazol-2-yl)-1-(tert-butoxy)-1-oxopropan-2-yl)pyrrolidine-1-carboxylate

(S)-3-(6-bromo-1-methyl-1H-benzo[d]imidazol-2-yl)-2-((R)-1-(tert-butoxycarbonyl)pyrrolidin-3-yl)propanoic acid (3.2g, 7.07mmol, 1.0eq) was dissolved in 2-methyltetrahydrofuran (20mL), *tert-*butyl N,N'-diisopropylcarbamimidate (4.25g, 21.22mmol, 3.0eq) was added, and the mixture was stirred at 65°C for 16h under nitrogen protection. The mixture was diluted with tetrahydrofuran, filtered, and the filter cake was washed with acetonitrile. The filtrate was concentrated and separated and purified by rapid chromatography (silicon dioxide, petroleum ether/ethyl acetate=3/1) to give the target compound (2.7g). LCMS(ESI)[M+H]⁺=508.2; ¹HNMR(400MHz, DMSO-*d*₆) δ 7.78 (d, *J*=1.8Hz, 1H), 7.46 (d, *J*=8.5Hz, 1H), 7.27 (dd, *J*=8.5, 1.9Hz, 1H), 3.73 (s, 3H), 3.58-3.45 (m, 1H), 3.42-3.34 (m, 1H), 3.25-3.06 (m, 2H), 3.05-2.87 (m, 3H), 2.44-2.32 (m, 1H), 1.96-1.86 (m, 1H), 1.78-1.61 (m, 1H), 1.39 (d, *J*=7.1Hz, 9H), 1.27 (s, 9H). Step 6: Synthesis of *tert-*butyl (R)-3-((S)-1-(tert-butoxy)-3-(6-formyl-1-methyl-1H-benzo[d]imidazol-2-yl)-1-oxopropan-2-yl)pyrrolidine-1-carboxylate

*Tert-*butyl (R)-3-((S)-3-(6-bromo-1-methyl-1H-benzo[d]imidazol-2-yl)-1-(tert-butoxy)-1-oxopropan-2-yl)pyrrolidine-1-carboxylate (400mg, 0.79mmol, 1.0eq) was dissolved in N,N-dimethylformamide (5mL). Sodium carbonate (251.2mg, 2.37mmol, 3.0eq), N-formylsaccharin (417.1mg, 1.98mmol, 2.5eq), 1,4-bis(diphenylphosphino)butane palladium(II) dichloride (95mg, 0.16mmol, 0.2eq) and triethylsilane (183.72mg, 1.58mmol, 2.0eq) were added sequentially; after replacing with nitrogen, the reaction mixture was reacted at 75°C for 16h. The mixture was diluted with water, extracted with ethyl acetate, and the organic phases were combined, washed with saturated sodium chloride aqueous solution, dried over anhydrous sodium sulfate, filtered, and the filtrate was concentrated to dryness. The crude product was separated and purified by rapid chromatography (silicon dioxide, petroleum ether/tetrahydrofuran=1/1) to give the target compound (300mg). LCMS(ESI)[M+H]⁺=458.3; ¹HNMR(400MHz, DMSO-*d*₆) δ 10.05 (s, 1H), 8.13 (d, *J*=0.8Hz, 1H), 7.74 (dd, *J*=8.3, 1.4Hz, 1H), 7.68 (d, *J*=8.3Hz, 1H), 3.85 (s, 3H), 3.60-3.47 (m, 1H), 3.42-3.36 (m, 1H), 3.25-3.12 (m, 2H), 3.11-2.94 (m, 3H), 2.46-2.35 (m, 1H), 1.97-1.88 (m, 1H), 1.80-1.62 (m, 1H), 1.40 (d, J=7.4Hz, 9H), 1.27 (s, 9H).

### Step 7: Synthesis of tert-butyl (R)-3-((S)-3-(6-(aminomethyl)-1-methyl-1H-benzo[d]imidazol-2-yl)-1-(tert-butoxy)-1-oxopropan-2-yl)pyrrolidine-1-carboxylate

*Tert-*butyl (R)-3-((S)-1-(tert-butoxy)-3-(6-formyl-1-methyl-1H-benzo[d]imidazol-2-yl)-1-oxopropan-2-yl)pyrrolidine-1-carboxylate (100mg, 0.22mmol, 1.0eq) was dissolved in methanol (2mL) and ammonia methanol (2mL, 7 M), and a drop of acetic acid was added; the reaction mixture was stirred at room temperature for 2h, sodium cyanoborohydride (68.01mg, 1.1mmol, 5eq) was added, and the mixture was stirred at 80°C for 2h. LCMS showed the reaction was completed and quenched with water. After concentration, the residue was purified by reversed-phase column chromatography (0.1% formic acid in water) to give the target compound (60mg). LCMS(ESI)[M+H]⁺=459.2.

### Step 8: Synthesis of tri-tert-butyl 3,3',3"-((2S,2'S,2"S)-((nitrilotris(methylene))tris(1-methyl-1H-benzo[d]imidazole-6,2-diyl))tris(3-(tert-butoxy)-3-oxopropane-1,2-diyl))(3R,3'R,3"R)-tris(pyrrolidine-1-carboxylate)

*Tert-*butyl (R)-3-((S)-3-(6-(aminomethyl)-1-methyl-1H-benzo[d]imidazol-2-yl)-1-(tert-butoxy)-1-oxopropan-2-yl)pyrrolidine-1-carboxylate (50mg, 0.11mmol, 1.0eq) was dissolved in ethanol (3mL), and *tert-*butyl (R)-3-((S)-1-(tert-butoxy)-3-(6-formyl-1-methyl-1H-benzo[d]imidazol-2-yl)-1-oxopropan-2-yl)pyrrolidine-1-carboxylate (99.78mg, 0.22mmol, 2.0eq) was added. One drop of acetic acid was added dropwise; the reaction mixture was stirred at room temperature for 2h, sodium cyanoborohydride (67.41mg, 1.09mmol, 10eq) was added, and the mixture was stirred at 70°C for 5h, and then stirred at room temperature for 13h. The reaction mixture was quenched with water, and the mixture was concentrated, purified by reverse phase column chromatography (0.1% ammonium bicarbonate in water) to give the target compound (40mg). LCMS(ESI)[M+H]⁺=1341.7.

### Step 9: Synthesis of (2S,2'S,2"S)-3,3',3"-((nitrilotris(methylene))tris(1-methyl-1H-benzo[d]imidazole-6,2-diyl))tris(2-((R)-pyrrolidin-3-yl)propanoic acid)

Tri-*tert*-butyl 3,3',3"-((2S,2'S,2"S)-((nitrilotris(methylene))tris(1-methyl-1H-benzo[d]imidazole-6,2-diyl))tris(3-(tert-butoxy)-3-oxopropane-1,2-diyl))(3R,3'R,3"R)-tris(pyrrolidine-1-carboxylate) (40mg, 0.029mmol, 1.0eq) was dissolved in 1,4-dioxane (2mL), and hydrochloric acid/1,4-dioxane (2mL, 4M) was added; the reaction mixture was stirred at room temperature for 2h. After the reaction was completed, the mixture was concentrated to dryness, dissolved in water, adjusted to pH=7 with sodium hydroxide aqueous solution (1M). After concentration, the mixture was purified by high pressure liquid chromatography (0.1% NH₄HCO₃ / CH₃CN) to give the target compound (23mg). LCMS(ESI)[M+H]⁺=873.4; ¹HNMR(400MHz, D₂O) δ 7.31 (d, *J*=8.1Hz, 3H), 6.77 (d, *J*=11.5Hz, 6H), 3.36 (s, 9H), 3.30-3.10 (m, 12H), 3.08-3.00 (m, 3H), 2.91 (dd, *J*=16.5, 10.4Hz, 3H), 2.75-2.67 (m, 3H), 2.62-2.51 (m, 6H), 2.37-2.25 (m, 3H), 2.04-1.94 (m, 3H), 1.63 (dq, *J*=12.7, 9.7Hz, 3H).

### Example 49 (Compound 134)

### Preparation of (S)-3-(3-((2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethyl)amino)phenyl)-2-((R)-pyrrolidin-3-yl)propanoic acid

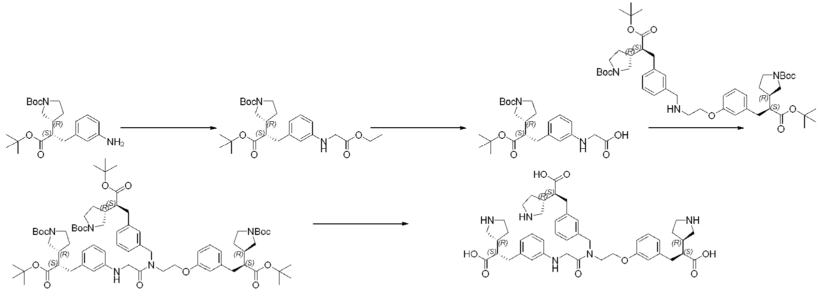

### Step 1: Synthesis of tert-butyl (R)-3-((S)-1-(tert-butoxy)-3-(3-((2-ethoxy-2-oxoethyl)amino)phenyl)-1-oxopropan-2-yl)pyrrolidine-1-carboxylate

*Tert-*butyl (R)-3-((S)-3-(3-aminophenyl)-1-(tert-butoxy)-1-oxopropan-2-yl)pyrrolidine-1-carboxylate(100mg) was dissolved in DMF (0.2mL), ethyl bromoacetate (51mg) and potassium carbonate (70mg) were added; the reaction mixture was stirred at 50°C overnight, and LCMS showed the reaction was completed. Ethyl acetate and water were added, the mixture was extracted, and the organic phase was dried over anhydrous sodium sulfate and purified by column chromatography (Silica gel, petroleum ether:ethyl acetate=10:1) to give the target compound (60mg). LCMS(ESI)[M+H-Boc]⁺=377.3.

### Step 2: Synthesis of (3-((S)-3-(tert-butoxy)-2-((R)-1-(tert-butoxycarbonyl)pyrrolidin-3-yl)-3-oxopropyl)phenyl)glycine

The product from the first step (85mg) was dissolved in tetrahydrofuran (0.3mL)and H₂O (0.3mL), followed by adding lithium hydroxide (13mg). The reaction mixture was stirred overnight at room temperature. LCMS showed the reaction was completed. The solvent was removed by rotary-evaporation to dryness, and the residue was purified by column chromatography (Silica gel, petroleum ether:ethyl acetate=2:1) to give the target compound (60mg). LCMS(ESI)[M+H-Boc]⁺=349.3.

### Step 3: Synthesis of tert-butyl (R)-3-((S)-1-(tert-butoxy)-3-(3-((2-((3-((S)-3-(tert-butoxy)-2-((R)-1-(tert-butoxycarbonyl)pyrrolidin-3-yl)-3-oxopropyl)benzyl)(2-(3-((S)-3-(tert-butoxy)-2-((R)-1-(tert-butoxycarbonyl)pyrrolidin-3-yl)-3-oxopropyl)phenoxy)ethyl)amino)-2-oxoethyl)amino)phenyl)-1-oxopropan-2-yl)pyrrolidine-1-carboxylate

The product from the second step (60mg) and *tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (110mg) were dissolved in N,N-dimethylformamide (0.5mL), O-(7-azabenzotriazol-1-yl)-N,N,N',N'-tetramethyluronium hexafluorophosphate (76mg) and triethylamine (41mg) were added; the reaction mixture was stirred overnight at room temperature, and LCMS showed the reaction was completed. Ethyl acetate and water were added, and the mixture was extracted and separated. The organic phase was dried over anhydrous sodium sulfate, filtered and concentrated to give a crude product, which was purified by column chromatography (Silica gel, petroleum ether:ethyl acetate=5:1) to give the target product (105mg). LCMS(ESI)[M+H-Boc]⁺=1152.97.

### Step 4: Synthesis of (S)-3-(3-((2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethyl)amino)phenyl)-2-((R)-pyrrolidin-3-yl)propanoic acid

The product from the third step (105mg) was dissolved in 1,4-dioxane (3mL), and hydrochloric acid/1,4-dioxane (4.0M, 3mL) was added; the mixture was stirred at room temperature overnight, and LCMS showed the reaction was completed. The solvent was removed by rotary-evaporation to dryness, and the residue was separated and purified by Prep-HPLC (C₁₈, 10mmol/L NH₄HCO₃ in water, MeCN) to give the target compound (6mg). LCMS(ESI)[M+H]⁺=784.62; ¹HNMR(400MHz, D₂O) δ 7.32-6.93 (m, 6H), 6.87-6.36 (m, 6H), 4.68-4.53 (m, 4H), 4.18-4.04 (m, 2H), 3.91-3.74 (m, 2H), 3.41-3.08 (m, 9H), 2.88-2.46 (m, 9H), 2.43-2.25 (m, 6H), 2.13-1.93 (m, 3H), 1.73-1.55 (m, 3H).

### Example 50 (Compound 135)

### Preparation of (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethoxy)phenyl)-2-((R)-pyrrolidin-3-yl)propanoic acid

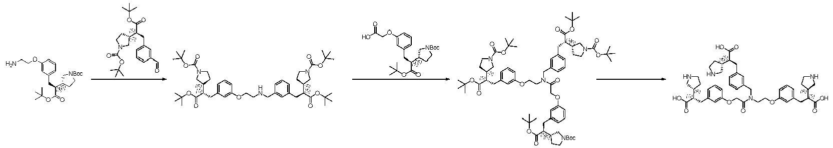

### Step 1: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

*Tert-*butyl (R)-3-((S)-3-(3-(2-aminoethoxy)phenyl)-1-(tert-butoxy)-1-oxopropan-2-yl)pyrrolidine-1-carboxylate (100mg) was dissolved in methanol (3mL). *Tert-*butyl (R)-3-((S)-1-(tert-butoxy)-3-(3-formylphenyl)-1-oxopropan-2-yl)pyrrolidine-1-carboxylate (93mg) and sodium cyanoborohydride (7mg) were added at room temperature. The mixture was stirred at room temperature for 16h. LCMS showed the reaction was completed. The reaction mixture was concentrated directly and purified by rapid chromatography (Silica gel, petroleum ether:ethyl acetate=1:5) to give the target compound (90mg). LCMS(ESI)[M+H]⁺=822.77.

### Step 2: Synthesis of tert-butyl (R)-3-((S)-1-(tert-butoxy)-3-(3-((2-(3-((S)-3-(tert-butoxy)-2-((R)-1-(tert-butoxycarbonyl)pyrrolidin-3-yl)-3-oxopropyl)phenoxy)-N-(2-(3-((S)-3-(tert-butoxy)-2-((R)-1-(tert-butoxycarbonyl)pyrrolidin-3-yl)-3-oxopropyl)phenoxy)ethyl)acetamido)methyl)phenyl)-1-oxopropan-2-yl)pyrrolidine-1-carboxylate

The product from the first step (100mg) was dissolved in DMF (5mL), followed by adding 2-(3-((S)-3-(tert-butoxy)-2-((R)-1-(tert-butoxycarbonyl)pyrrolidin-3-yl)-3-oxopropyl)phenoxy)acetic acid (66mg), O-(7-azabenzotriazol-1-yl)-N,N,N',N'-tetramethyluronium hexafluorophosphate (93mg) and N,N-diisopropylethylamine (47mg). The mixture was stirred at room temperature for 16h. LCMS showed the reaction was completed. The reaction mixture was concentrated directly and purified by rapid chromatography (Silica gel, petroleum ether:ethyl acetate=1:8) to give the target compound (120mg). LCMS(ESI)[M+H]⁺=1253.75.

### Step 3: Synthesis of (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethoxy)phenyl)-2-((R)-pyrrolidin-3-yl)propanoic acid

The product from the second step (120mg) was dissolved in dichloromethane (5mL), and trifluoroacetic acid (2mL) was added; the mixture was stirred at room temperature for 16h. LCMS showed the reaction was completed. The reaction mixture was concentrated directly, separated and purified by preparative chromatography to give the target compound (52.91mg). LCMS(ESI)[M+H]⁺=785.2; ¹HNMR(400MHz, D₂O) δ 7.28-6.93 (m, 6H), 6.85-6.48 (m, 6H), 5.02 (s, 1H), 4.65 (d, *J*=16.5Hz, 2H), 4.51 (d, *J*=15.7Hz, 1H), 4.13-3.96 (m, 2H), 3.71 (q, *J*=9.9, 7.7Hz, 2H), 3.45-3.19 (m, 6H), 3.18-3.05 (m, 3H), 2.83-2.53 (m, 8H), 2.52-2.19 (m, 7H), 2.01 (dtd, *J*=12.1, 6.4, 5.7, 3.0Hz, 3H), 1.72-1.53 (m, 3H).

### Example 51 (Compound 52)

### Preparation of (25,2'S,2"S)-3,3',3"-((nitrilotris(methylene))tris(1H-indazole-3,5-diyl))tris(2-((R)-pyrrolidin-3-yl)propanoic acid)

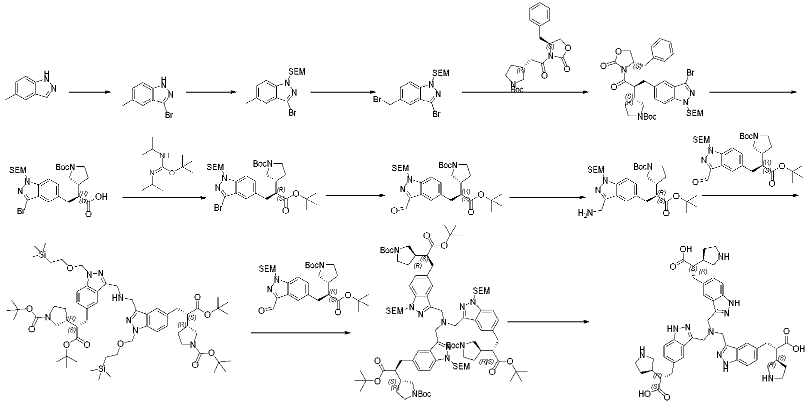

### Step 1: Synthesis of 3-bromo-5-methyl-1H-indazole

5-methyl-1H-indazole (24g, 181.6mmol, 1.0eq) was dissolved in acetonitrile (240mL), and sodium 4,5,6,7-tetrachloro-2',4',5',7'-tetraiodo-3H-spiro[isobenzofuran-1,9'-xanthene]-3',6'-bis(olate) (1.76g, 1.82mmol, 0.01eq) and N-bromosuccinimide (35.55g, 199.76mmol, 1.1eq) were added at room temperature; the reaction mixture was stirred at room temperature for 2h. LCMS showed the reaction of starting material was completed. The reaction mixture was concentrated, and the crude product was purified by column chromatography (silica gel, petroleum ether:ethyl acetate=1:1) to give the target compound (37g). LCMS(ESI)[M+H]⁺=211.0; ¹HNMR(400MHz, DMSO-*d*₆) δ 13.36 (s, 1H), 7.54 (d, *J*=8.6Hz, 1H), 7.41 (s, 1H), 7.38-7.32 (m, 1H), 2.50 (s, 3H). Step 2: Synthesis of 3-bromo-5-methyl-1-((2-(trimethylsilyl)ethoxy)methyl)-1H-indazole 3-bromo-5-methyl-1H-indazole (19g, 90.02mmol, 1.0eq) was dissolved in tetrahydrofuran (200mL), and sodium hydride (4.14g, 180.04mmol, 2.0eq) was added at 0°C. The mixture was stirred for 30min. Then [2-(chloromethoxy)ethyl]trimethylsilane (22.51g, 135.03mmol, 1.5eq) was added at 0°C, and the reaction mixture was stirred at 0°C for 1.5h. LCMS showed the reaction of starting material was completed. Saturated ammonium chloride solution was added, and the mixture was extracted with ethyl acetate. The organic phase was dried over anhydrous sodium sulfate, filtered, and the filtrate was concentrated. The crude product was separated and purified by column chromatography (petroleum ether:ethyl acetate=10:1) to give the target compound (25g). LCMS(ESI)[M+H]⁺=340.9; ¹HNMR(400MHz, CDCl₃-d) δ 7.51 (d, *J*=8.6Hz, 1H), 7.46 (s, 1H), 7.39-7.35 (m, 1H), 5.72 (s, 2H), 3.64-3.59 (m, 2H), 2.55 (s, 3H), 0.94-0.93 (m, 2H), 0.10-0.08 (m, 9H).

Step 3: Synthesis of 3-bromo-5-(bromomethyl)-1-((2-(trimethylsilyl)ethoxy)methyl)-1H-indazole *N*-bromosuccinimide (25.24g, 141.8mmol, 1.1eq) and 2-[2-(1-cyano-1-methylethyl)diazen-1-yl]-2-methylpropanenitrile (2.12g, 12.89mmol, 0.1eq) were sequentially added to a solution of 3-bromo-5-methyl-1-((2-(trimethylsilyl)ethoxy)methyl)-1H-indazole (44g, 128.91mmol, 1.0eq) in carbon tetrachloride (200mL), and the reaction mixture was heated to 80°C under nitrogen protection and stirred for 16h. LCMS showed the reaction was completed. After the reaction mixture was cooled to room temperature, the mixture was filtered to remove the filter residue. The filtrate was concentrated, and the crude product was separated and purified by silica gel column chromatography (petroleum ether/ethyl acetate=200:1-100:1) to give the target compound (14g). LCMS(ESI)[M+H]⁺=418.9; ¹HNMR(400MHz, DMSO-*d*₆) δ 7.77 (d, *J*=8.7Hz, 1H), 7.69 (s, 1H), 7.57 (dd, *J*=8.8, 1.5Hz, 1H), 5.69 (s, 2H), 4.86 (s, 2H), 3.50-3.45 (m, 2H), 0.77-0.72 (m, 2H), - 0.15--0.17 (m, 9H).

### Step 4: tert-butyl (R)-3-((S)-1-((S)-4-benzyl-2-oxooxazolidin-3-yl)-3-(3-bromo-1-((2-(trimethylsilyl)ethoxy)methyl)-1H-indazol-5-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine- 1-carboxylate(5g, 12.87mmol, 1.0eq) was dissolved in anhydrous tetrahydrofuran (70mL), and the reaction mixture was replaced with nitrogen. Under nitrogen protection, the mixture was cooled to -5°C in an ice-salt bath. Lithium bis(trimethylsilyl)amide (19.31mL, 19.31mmol, 1.5eq) was slowly added to the above solution; after the addition was completed, the mixture was stirred at -5°C for 1h. A solution of 3-bromo-5-(bromomethyl)-1-((2-(trimethylsilyl)ethoxy)methyl)-1H-indazole (6.49g, 15.45mmol, 1.2eq) in tetrahydrofuran (10mL) was slowly added dropwise to the reaction mixture. After the addition was completed, the reaction mixture was warmed to room temperature and stirred for 15h. LCMS showed the reaction was completed. The reaction mixture was quenched with saturated ammonium chloride solution, diluted with water, and extracted with ethyl acetate. The combined organic phases were washed with saturated brine, dried over anhydrous sodium sulfate, filtered, and concentrated. The crude product was separated and purified by column chromatography (tetrahydrofuran/petroleum ether=0~25%) to give the target compound (6.2g). LCMS(ESI)[M+Na]⁺=749.0; ¹HNMR(400MHz, DMSO-*d*₆) δ 7.77 (d, *J*=8.7Hz, 1H), 7.53-7.43 (m, 2H), 7.11 (t, *J*=7.4Hz, 1H), 7.02-6.95 (m, 2H), 6.67-6.59 (m, 2H), 5.74-5.67 (m, 2H), 4.69-4.60 (m, 1H), 4.42-4.29 (m, 1H), 4.28-4.18 (m, 1H), 4.03-3.94 (m, 1H), 3.51-3.42 (m, 3H), 3.17-2.91 (m, 5H), 2.74-2.66 (m, 1H), 2.45-2.30 (m, 2H), 1.98-1.87 (m, 1H), 1.72-1.58 (m, 1H), 1.41-1.36 (m, 9H), 0.78-0.70 (m, 2H), -0.14 (s, 9H).

### Step 5: Synthesis of (S)-3-(3-bromo-1-((2-(trimethylsilyl)ethoxy)methyl)-1H-indazol-5-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(3-bromo-1-((2-(trimethylsilyl)ethoxy)methyl)-1H-indazol-5-yl)-1-oxopropan-2-yl)pyrrolidine-1-carboxylate (3g, 4.12mmol, 1.0eq) was dissolved in tetrahydrofuran (50mL), hydrogen peroxide (9.34g, 82.4mmol, 20eq, 30% content) was added, and a solution of lithium hydroxide monohydrate (0.35g, 8.24mmol, 2.0eq) in water (5mL) was added dropwise to the reaction mixture. After the addition was completed, the reaction mixture was stirred at 25°C for 16h. LCMS showed the reaction was completed. The reaction mixture was quenched with saturated sodium sulfate solution (20mL), and the pH was adjusted to around 12 with sodium hydroxide solution. The mixture was washed with ethyl acetate, and the aqueous phase was adjusted to a pH of about 4 with hydrochloric acid (3 M), and extracted with ethyl acetate again. The organic phases were combined, dried over anhydrous sodium sulfate, filtered, and concentrated to give the target compound (3.3g). This intermediate was used directly in the next step without further purification. LCMS(ESI)[M+Na]⁺=590.1.

Step 6: Synthesis of *tert-*butyl (R)-3-((S)-3-(3-bromo-1-((2-(trimethylsilyl)ethoxy)methyl)-1H-indazol-5-yl)-1-(tert-butoxy)-1-oxopropan-2-yl)pyrrolidine-1-carboxylate (S)-3-(3-bromo-1-((2-(trimethylsilyl)ethoxy)methyl)-1H-indazol-5-yl)-2-((R)-1-(tert-butoxycarbonyl)pyrrolidin-3-yl)propanoic acid (3.2g, 5.63mmol, 1.0eq) was dissolved in 2-methyltetrahydrofuran (30mL). *tert*-butyl N,N'-diisopropylcarbamimidate(5.64g, 28.14mmol, 5eq) was added, and the reaction mixture was stirred at 70°C for 3h under nitrogen protection. After cooled to room temperature, the reaction mixture was filtered to remove the filter residue. The filter cake was fully washed with methyl *tert-butyl* ether. The filtrate was concentrated under reduced pressure, and the crude product was purified by column chromatography (tetrahydrofuran/petroleum ether=0%~20%) to give the target compound (2g).LCMS(ESI)[M+Na]⁺=646.1; ¹HNMR(400MHz, DMSO-*d*₆) δ 7.70 (d, *J*=8.7Hz, 1H), 7.42-7.33 (m, 2H), 5.69 (s, 2H), 3.61-3.51 (m, 1H), 3.47 (t, *J*=7.9Hz, 2H), 3.30-3.25 (m, 1H), 3.20-3.10 (m, 1H), 3.06-2.98 (m, 1H), 2.94-2.78 (m, 2H), 2.60-2.52 (m, 1H), 2.36-2.24 (m, 1H), 1.89-1.78 (m, 1H), 1.65-1.52 (m, 1H), 1.39 (s, 9H), 1.16 (s, 9H), 0.79-0.71 (m, 2H), -0.14 (s, 9H).

### Step 7: Synthesis of tert-butyl (R)-3-((S)-1-(tert-butoxy)-3-(3-formyl-1-((2-(trimethylsilyl)ethoxy)methyl)-1H-indazol-5-yl)-1-oxopropan-2-yl)pyrrolidine-1-carboxylate

*Tert-*butyl (R)-3-((S)-3-(3-bromo-1-((2-(trimethylsilyl)ethoxy)methyl)-1H-indazol-5-yl)-1-(tert-butoxy)-1-oxopropan-2-yl)pyrrolidine-1-carboxylate (1g, 1.6mmol, 1.0eq) and sodium carbonate (339.34mg, 3.2mmol, 2.0eq) were dissolved in *N,N*-dimethylformamide (15mL), followed by adding triethylsilane (279.21mg, 2.4mmol, 1.5eq) and [1,1'-bis(diphenylphosphino)ferrocene]palladium(II) dichloride (117.46mg, 0.16mmol, 0.1eq); the reaction mixture was stirred at 80°C for 16h under a carbon monoxide. LCMS showed the reaction was completed. After the reaction mixture was cooled to room temperature, water was added for dilution, and the mixture was extracted with ethyl acetate. The organic phase was washed with water and saturated brine, dried over anhydrous sodium sulfate, filtered and concentrated. The crude product was separated and purified by column chromatography (tetrahydrofuran/petroleum ether=0%~25%) to give the target compound (0.8g). LCMS(ESI)[M+Na]⁺=596.2.

### Step 8: Synthesis of tert-butyl (R)-3-((S)-3-(3-(aminomethyl)-1-((2-(trimethylsilyl)ethoxy)methyl)-1H-indazol-5-yl)- 1 -(tert-butoxy)- 1-oxopropan-2-yl)pyrrolidine-1-carboxylate

*Tert-*butyl (R)-3-((S)-1-(tert-butoxy)-3-(3-formyl-1-((2-(trimethylsilyl)ethoxy)methyl)-1H-indazol-5-yl)-1-oxopropan-2-yl)pyrrolidine-1-carboxylate (200mg, 0.35mmol, 1.0eq) was dissolved in a solution of ammonia methanol (7 M, 5mL), one drop of glacial acetic acid was added dropwise, and the reaction mixture was stirred at 25°C for 15 min. sodium cyanoborohydride (215.51mg, 3.49mmol, 10eq) was added to the reaction mixture, which was heated to 80°C in a sealed tube and stirred for 16h. LCMS showed the reaction was completed. The reaction mixture was cooled to room temperature, concentrated under reduced pressure, and the residue was separated and purified by reversed-phase column (0.1% aqueous formic acid/acetonitrile=62%) to give the target compound (130mg). LCMS(ESI)[M+H]⁺=575.2; ¹HNMR(400MHz, DMSO-*d*₆) δ 8.48 (s, 2H), 7.82-7.75 (m, 2H), 7.45 (d, *J*=8.3Hz, 1H), 5.86-5.78 (m, 2H), 4.55-4.39 (m, 2H), 3.68-3.62 (m, 2H), 3.48-3.43 (m, 2H), 3.30-3.21 (m, 1H), 3.10 (t, *J*=9.9Hz, 1H), 3.00-2.90 (m, 2H), 2.71-2.62 (m, 1H), 2.46-2.35 (m, 1H), 2.01-1.90 (m, 1H), 1.76-1.62 (m, 1H), 1.49 (s, 9H), 1.33-1.25 (m, 9H), 0.94-0.85 (m, 2H), 0.03--0.04 (m, 9H).

### Step 9: Synthesis of di-tert-butyl 3,3'-((2S,2'S)-((azanediylbis(methylene))bis(1-((2-(trimethylsilyl)ethoxy)methyl)-1H-indazole-3,5-diyl))bis(3-(tert-butoxy)-3-oxopropane-1,2-diyl))(3R,3'R)-bis(pyrrolidine-1-carboxylate)

*Tert-*butyl (R)-3-((S)-3-(3-(aminomethyl)-1-((2-(trimethylsilyl)ethoxy)methyl)-1H-indazol-5-yl)-1-(tert-butoxy)-1-oxopropan-2-yl)pyrrolidine-1-carboxylate (80mg, 0.14mmol, 1.0eq) and *tert-*butyl (R)-3-((S)-1-(tert-butoxy)-3-(3-formyl-1-((2-(trimethylsilyl)ethoxy)methyl)-1H-indazol-5-yl)-1-oxopropan-2-yl)pyrrolidine-1-carboxylate(119.78mg, 0.21mmol, 1.5eq) were dissolved in ethanol (4mL), one drop of glacial acetic acid was added, and the reaction mixture was stirred at 25°C for 30min. sodium cyanoborohydride (86.05mg, 1.39mmol, 10eq) was added to the reaction mixture, which was heated to 60°C and stirred for 3.5h. LCMS showed the reaction was completed. After cooled to room temperature, the reaction mixture was concentrated under reduced pressure. The residue was separated and purified by reversed-phase column chromatography (0.1% aqueous trifluoroacetic acid/acetonitrile=72%) to give the target compound (80mg). LCMS(ESI)[M+H]⁺=1132.4; ¹HNMR(400MHz, DMSO-*d*₆) δ 9.97 (s, 1H), 7.84 (d, *J*=8.7Hz, 1H), 7.78 (d, *J*=16.9Hz, 2H), 7.69 (d, *J*=8.6Hz, 1H), 7.49 (d, *J*=7.5Hz, 1H), 7.38 (d, *J*=8.5Hz, 1H), 5.88 (s, 2H), 5.76 (s, 2H), 4.84 (s, 2H), 4.79-4.67 (m, 2H), 3.67-3.55 (m, 6H), 3.47 (t, *J*=9.3Hz, 2H), 3.32-3.23 (m, 2H), 3.16-3.08 (m, 2H), 3.01-2.89 (m, 4H), 2.72-2.64 (m, 2H), 2.47-2.37 (m, 2H), 2.02-1.91 (m, 2H), 1.74-1.65 (m, 2H), 1.51 (s, 18H), 1.29 (d, *J*=10.3Hz, 18H), 0.93-0.86 (m, 4H), 0.02--0.04 (m, 18H).

### Step 10: Synthesis of tri-tert-butyl 3,3',3"-((2S,2'S,2"S)-((nitrilotris(methylene))tris(1-((2-(trimethylsilyl)ethoxy)methyl)-1H-indazole-3,5-diyl))tris(3-(tert-butoxy)-3-oxopropane-1,2-diyl))(3R,3'R,3"R)-tris(pyrrolidine-1-carboxylate)

Di-*tert*-butyl 3,3'-((2S,2'S)-((azanediylbis(methylene))bis(1-((2-(trimethylsilyl)ethoxy)methyl)-1H-indazole-3,5-diyl))bis(3-(tert-butoxy)-3-oxopropane-1,2-diyl))(3R,3'R)-bis(pyrrolidine-1-carboxylate) (50mg) and *tert-*butyl (R)-3-((S)-1-(tert-butoxy)-3-(3-formyl-1-((2-(trimethylsilyl)ethoxy)methyl)-1H-indazol-5-yl)-1-oxopropan-2-yl)pyrrolidine-1-carboxylate(50.66mg) were dissolved in ethanol (2mL), a small drop of glacial acetic acid was added, and the reaction mixture was stirred at 35°C for 6h. sodium cyanoborohydride (27.3mg) was added to the reaction mixture, which was heated to 70°C and stirred for 10h. The reaction mixture was cooled to room temperature, concentrated under reduced pressure. The obtained residue was separated and purified by reversed-phase column chromatography (0.1% formic acid in water/acetonitrile=0~100%) to remove impurities, and the system (0.1% formic acid in water/methanol=100%) was changed to give the target compound (30mg). LCMS(ESI)[M/2+H]⁺=846.0.

### Step 11: Synthesis of (2S,2'S,2"S)-3,3',3"-((nitrilotris(methylene))tris(1H-indazole-3,5-diyl))tris(2-((R)-pyrrolidin-3-yl)propanoic acid)

Tri-*tert*-butyl 3,3',3"-((2S,2'S,2"S)-((nitrilotris(methylene))tris(1-((2-(trimethylsilyl)ethoxy)methyl)-1H-indazole-3,5-diyl))tris(3-(tert-butoxy)-3-oxopropane-1,2-diyl))(3R,3'R,3"R)-tris(pyrrolidine-1-carboxylate) (70mg) was dissolved in hydrochloric acid/1,4-dioxane (4M, 3mL), and the reaction mixture was stirred at room temperature for 4h. LCMS showed the reaction was completed. The reaction mixture was concentrated under reduced pressure, and the residue was separated and purified by preparative chromatography (0.1% ammonium bicarbonate aqueous solution/acetonitrile) to give the target compound (3.7mg). LCMS(ESI)[M+H]⁺=831.1.

### Example 52 (Compound 137)

### Preparation of (25,2'S,2"S)-3,3',3"-((nitrilotris(methylene))tris(thiophene-5,3-diyl))tris(2-((R)-pyrrolidin-3-yl)propanoic acid)

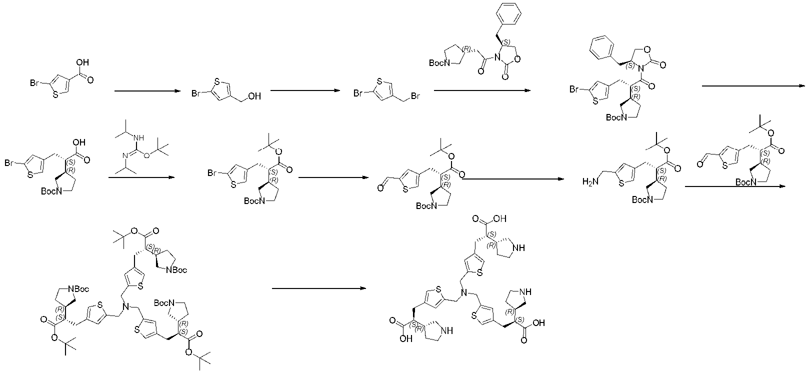

### Step 1: Synthesis of (5-bromothiophen-3-yl)methanol

5-bromothiophene-3-carboxylic acid(12g) was dissolved in tetrahydrofuran (150mL). Borane/tetrahydrofuran (173.87mL) was added dropwise at 0°C, and the reaction mixture was stirred at room temperature for 3h. After the reaction was completed, methanol (30mL) was added to the reaction mixture to quench the reaction, water (300mL) was added for dilution, and the mixture was extracted with ethyl acetate (300mL×3). The organic phase was dried over anhydrous sodium sulfate, filtered, and concentrated to give a crude product, which was purified by column chromatography (petroleum ether/ethyl acetate=5/1) to give the target compound (10.5g). ¹HNMR(400MHz, DMSO-*d₆*) δ 7.32-7.28 (m, 1H), 7.12 (d, *J*=1.6Hz, 1H), 5.18 (t, *J*=5.8Hz, 1H), 4.41-4.38 (m, 2H).

### Step 2: Synthesis of 2-bromo-4-(bromomethyl)thiophene

(5-bromothiophen-3-yl)methanol (21g) was dissolved in dichloromethane (200mL). Phosphorus tribromide (47.91g) was added under ice bath, and the reaction was maintained at room temperature for 2h. LCMS showed the reaction of starting material was completed. The reaction mixture was poured into water to quench and extracted with dichloromethane (300 mL x 3). The organic phases were combined, washed with saturated sodium chloride aqueous solution, dried over anhydrous sodium sulfate, filtered, and concentrated to give a crude product, which was purified by silica gel column chromatography (petroleum ether 100%) to give the target product (22g). ¹HNMR(400MHz, CDCl₃) δ 7.16 (d, *J*=1.5Hz, 1H), 7.07 (d, *J*=1.7Hz, 1H), 4.40 (s, 2H). Step 3: Synthesis of *tert-*butyl (R)-3-((S)-1-((S)-4-benzyl-2-oxooxazolidin-3-yl)-3-(5-bromothiophen-3-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate(5g) was dissolved in tetrahydrofuran (60mL), lithium bis(trimethylsilyl)amide (16.73mL) was added dropwise under nitrogen protection and ice bath, and the mixture was stirred for 1h; 2-bromo-4-(bromomethyl)thiophene (4.94g) was dissolved in tetrahydrofuran. Under ice bath, the mixture was added dropwise to the reaction system, which was stirred at room temperature for 6h. The reaction mixture was quenched with aqueous ammonium chloride solution. The mixture was extracted with ethyl acetate, and the organic phases were combined and washed with saturated sodium chloride solution, dried over anhydrous sodium sulfate, filtered, and concentrated to dryness. The crude product was separated and purified by rapid chromatography (silica gel, petroleum ether/ethyl acetate=3/1) to give the target compound (3.8g).LCMS(ESI)[M+Na]⁺=585.0/587.0; ¹HNMR(400MHz, DMSO-*d₆*) δ 7.28-7.21 (m, 4H), 7.11 (d, *J*=2.3Hz, 1H), 6.95 (d, *J*=6.7Hz, 2H), 4.67 (s, 1H), 4.29 (t, *J*=8.2Hz, 1H), 4.25-4.15 (m, 1H), 4.14-4.07 (m, 1H), 3.50-3.37 (m, 1H), 3.33-3.27 (m, 1H), 3.18-3.02 (m, 1H), 3.00-2.89 (m, 2H), 2.86-2.73 (m, 2H), 2.65 (td, *J*=13.3, 7.6Hz, 1H), 2.45-2.31 (m, 1H), 1.94-1.84 (m, 1H), 1.69-1.52 (m, 1H), 1.39 (s, 9H).

### Step 4: Synthesis of (S)-3-(5-bromothiophen-3-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(5-bromothiophen-3-yl)-1-oxopropan-2-yl)pyrrolidine-1-carboxylate (3.8g) was dissolved in tetrahydrofuran (40mL), hydrogen peroxide solution (7.6mL) was added under ice bath. Dissolved in water, lithium hydroxide (242.26mg) was added dropwise to the mixture under ice bath, and the mixture was stirred at room temperature for 16h. LCMS showed the reaction was completed. The reaction mixture was quenched with sodium sulfite and extracted with dichloromethane. The combined organic phases were washed with saturated sodium chloride aqueous solution, dried over anhydrous sodium sulfate, filtered and concentrated to dryness to give the target product (3.8g). LCMS(ESI)[M+Na]⁺=426.0.

### Step 5: Synthesis of tert-butyl (R)-3-((S)-3-(5-bromothiophen-3-yl)-1-(tert-butoxy)-1-oxopropan-2-yl)pyrrolidine-1-carboxylate

(S)-3-(5-bromothiophen-3-yl)-2-((R)-1-(tert-butoxycarbonyl)pyrrolidin-3-yl)propanoic acid (3.8g, 70% purity) was dissolved in 2-methyltetrahydrofuran (40mL), *tert-*butyl N,N'-diisopropylcarbamimidate (6.59g) was added. The reaction mixture was stirred at 70°C for 16h under nitrogen protection. LCMS showed the reaction of starting material was completed. The mixture was diluted with acetonitrile (50mL), filtered, the filter cake was washed with acetonitrile, and the filtrate was concentrated to give a crude product, which was separated and purified by rapid chromatography (silicon dioxide, petroleum ether/ethyl acetate=5/1) to give the target product (2.5g). LCMS(ESI)[M+H]⁺=460.1; ¹HNMR(400MHz, DMSO-*d₆*) δ 7.24 (s, 1H), 7.13 (d, *J*=3.3Hz, 1H), 3.57-3.48 (m, 1H), 3.43-3.39 (m, 1H), 3.20 (dt, *J*=10.6, 8.3Hz, 1H), 3.00 (t, *J*=10.0Hz, 1H), 2.83-2.69 (m, 2H), 2.55-2.50 (m, 1H), 2.37-2.25 (m, 1H), 1.93-1.85 (m, 1H), 1.69-1.54 (m, 1H), 1.45 (s, 9H), 1.34 (s, 9H).

### Step 6: Synthesis of tert-butyl (R)-3-((S)-1-(tert-butoxy)-3-(5-formylthiophen-3-yl)-1-oxopropan-2-yl)pyrrolidine-1-carboxylate

*Tert*-butyl (R)-3-((S)-3-(5-bromothiophen-3-yl)-1-(tert-butoxy)-1-oxopropan-2-yl)pyrrolidine-1-carboxylate (2.4g) was dissolved in N,N-dimethylformamide (30mL), followed by adding sodium carbonate (1.1g), [1,1'-bis(diphenylphosphino)ferrocene]palladium(II) dichloride (382.45mg) and triethylsilane (1.21g); after replacing with carbon monoxide gas, the reaction mixture was stirred at 80°C for 16h under a carbon monoxide. LCMS showed the reaction was completed. The reaction mixture was quenched with water, extracted with ethyl acetate, and the organic phases were combined and washed with saturated sodium chloride solution, dried over anhydrous sodium sulfate, filtered, and concentrated to dryness. The crude product was separated and purified by rapid chromatography (silica gel, petroleum ether/ethyl acetate=3/1) to give the target compound (1.5g). LCMS(ESI)[M+Na]⁺=432.1; ¹HNMR(400MHz, DMSO-*d₆*) δ 9.89 (d, *J*=1.2Hz, 1H), 7.89 (s, 1H), 7.80 (s, 1H), 3.56-3.44 (m, 1H), 3.39-3.33 (m, 1H), 3.21-3.10 (m, 1H), 2.98 (t, *J*=9.9Hz, 1H), 2.87-2.74 (m, 2H), 2.61-2.54 (m, 1H), 2.34-2.22 (m, 1H), 1.90-1.81 (m, 1H), 1.65-1.52 (m, 1H), 1.40 (s, 9H), 1.26 (s, 9H).

### Step 7: Synthesis of tert-butyl (R)-3-((S)-3-(5-(aminomethyl)thiophen-3-yl)-1-(tert-butoxy)-1-oxopropan-2-yl)pyrrolidine-1-carboxylate

*Tert-*butyl (R)-3-((S)-1-(tert-butoxy)-3-(5-formylthiophen-3-yl)-1-oxopropan-2-yl)pyrrolidine-1-carboxylate (600mg) was dissolved in ammonia methanol (10mL, 7 M), one drop of acetic acid was added, and the mixture was stirred at room temperature for 2h. sodium cyanoborohydride (452.92mg) was added, and the reaction mixture was stirred at 70°C for 4h. LCMS showed the reaction of starting material was completed. The reaction mixture was quenched with water, concentrated, and purified by reversed-phase column chromatography (0.1% ammonia water) to give the target product (240mg). LCMS(ESI)[M+H]⁺=411.2.

### Step 8: Synthesis of tri-tert-butyl 3,3',3"-((2S,2'S,2"S)-((nitrilotris(methylene))tris(thiophene-5,3-diyl))tris(3-(tert-butoxy)-3-oxopropane-1,2-diyl))(3R,3'R,3"R)-tris(pyrrolidine-1-carboxylate)

*Tert-*butyl (R)-3-((S)-3-(5-(aminomethyl)thiophen-3-yl)-1-(tert-butoxy)-1-oxopropan-2-yl)pyrrolidine-1-carboxylate (290mg) was dissolved in ethanol (8mL), *tert-*butyl (R)-3-((S)-1-(tert-butoxy)-3-(5-formylthiophen-3-yl)-1-oxopropan-2-yl)pyrrolidine-1-carboxylate(723.18mg) was added, a drop of acetic acid was added dropwise, and the mixture was stirred at room temperature for 4h. sodium cyanoborohydride (436.73mg) was added and the mixture was stirred at 70°C for 16h. LCMS showed the reaction of starting material was completed. The reaction mixture was quenched with water and concentrated. The residue was purified by reversed-phase column chromatography (0.1% trifluoroacetic acid) to give the target product (400mg). LCMS(ESI)[M+H]⁺=1196.9.

### Step 9: Synthesis of (2S,2'S,2"S)-3,3',3"-((nitrilotris(methylene))tris(thiophene-5,3-diyl))tris(2-((R)-pyrrolidin-3-yl)propanoic acid)

Tri-*tert*-butyl 3,3',3"-((2S,2'S,2"S)-((nitrilotris(methylene))tris(thiophene-5,3-diyl))tris(3-(tert-butoxy)-3-oxopropane-1,2-diyl))(3R,3'R,3"R)-tris(pyrrolidine-1-carboxylate) (400mg) was dissolved in 1,4-dioxane (5mL), hydrochloric acid/1,4-dioxane (5mL, 4M) was added, and the reaction mixture was stirred at room temperature for 16h. After the reaction was completed, the mixture was concentrated to dryness, dissolved in water, adjusted to pH=7 with sodium carbonate aqueous solution (1M). After concentration, the mixture was purified by high pressure liquid chromatography (0.1%NH₄HCO₃) to give the target compound (178.81mg). LCMS(ESI)[M+H]⁺=729.3; ¹HNMR(400MHz, D₂O) δ 6.95 (s, 3H), 6.80 (s, 3H), 3.67 (s, 6H), 3.30 (ddd, *J*=11.2, 7.2, 2.8Hz, 6H), 3.12 (ddd, *J*=11.6, 10.2, 7.3Hz, 3H), 2.79-2.71 (m, 6H), 2.61 (dd, *J*=14.3, 5.1Hz, 3H), 2.42-2.25 (m, 6H), 2.03 (tdd, *J*=10.1, 6.9, 3.4Hz, 3H), 1.65 (dq, *J*=12.9, 9.5Hz, 3H).

### Example 53 (Compound 139)

### Preparation of (S)-3-(3-(2-((3-((S)-2-carboxy-2-((R)-pyrrolidin-3-yl)ethyl)benzyl)((3-((S)-2-carboxy-2-((R)-pyrrolidin-3-yl)ethyl)phenoxy)carbonyl)amino)ethoxy)phenyl)-2-((R)-pyrrolidin-3-yl)propanoic acid

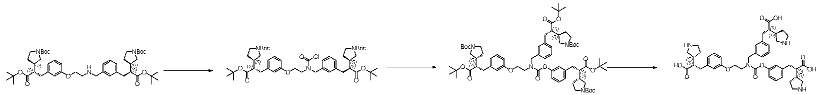

### Step 1: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)methyl)phenyl)-1-oxopropan-2-yl)pyrrolidine-1-carboxylate

*Tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate(98.65mg, 0.12mmol, 1eq) was dissolved in tetrahydrofuran (2mL), and triphosgene (35.61mg, 0.12mmol, 1eq) and *N,N-*diisopropylethylamine (155.09mg, 1.2mmol, 10eq) were added at 0°C; the reaction mixture was stirred at 0°C for 0.5 h under nitrogen protection. LCMS showed the formation of the product, and the reaction mixture was directly used for the next step. LCMS(ESI)[M+H-Boc]⁺=784.4.

### Step 2: Synthesis of tert-butyl (R)-3-((S)-1-(tert-butoxy)-3-(3-((((3-((S)-3-(tert-butoxy)-2-((R)-1-(tert-butoxycarbonyl)pyrrolidin-3-yl)-3-oxopropyl)phenoxy)carbonyl)(2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

The product from the first step (100mg, 0.11mmol, 1eq) was dissolved in tetrahydrofuran (3mL), and *tert-*butyl (R)-3-((S)-1-(tert-butoxy)-3-(3-hydroxyphenyl)-1-oxopropan-2-yl)pyrrolidine-1-carboxylate (44.26mg, 0.11mmol, 1eq) and 4-dimethylaminopyridine (1.38mg, 0.01mmol, 0.1eq) were added; the mixture was stirred at 50°C for 3h under nitrogen protection. The reaction mixture was concentrated and purified by column chromatography (petroleum ether:ethyl acetate=4: 1) to give the target product (100mg). LCMS(ESI)[M+H-Boc]⁺=1139.0.

### Step 3: Synthesis of (S)-3-(3-(2-((3-((S)-2-carboxy-2-((R)-pyrrolidin-3-yl)ethyl)benzyl)((3-((S)-2-carboxy-2-((R)-pyrrolidin-3-yl)ethyl)phenoxy)carbonyl)amino)ethoxy)phenyl)-2-((R)-pyrrolidin-3-yl)propanoic acid

The product from the second step (100mg, 0.08mmol, 1eq) was dissolved in dioxane (2mL), followed by adding hydrochloric acid-1,4-dioxane (4.0M, 2mL). The reaction mixture was stirred at room temperature for 2h under nitrogen protection. After concentration, the reaction mixture was separated and purified by Prep-HPLC (0.1% NH₄HCO₃ in water, MeCN) to give the target compound (35.08mg). LCMS(ESI)[M+H]⁺=771.2; ¹HNMR(400MHz, D₂O) δ 7.33-7.06 (m, 7H), 6.92-6.86 (m, 1H), 6.85-6.76 (m, 2H), 6.72 (d, *J*=12.9Hz, 2H), 4.69-4.66 (m, 1H), 4.56 (s, 1H), 4.17 (d, *J*=4.7Hz, 2H), 3.77 (d, *J*=40.3Hz, 2H), 3.40-3.24 (m, 6H), 3.19-3.08 (m, 3H), 2.85-2.56 (m, 9H), 2.43-2.27 (m, 6H), 2.09-1.96 (m, 3H), 1.72-1.58 (m, 3H).

### Example 54 (Compound 140)

### Preparation of (S)-3-(3-(3-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-3-oxopropyl)phenyl)-2-((R)-pyrrolidin-3-yl)propanoic acid

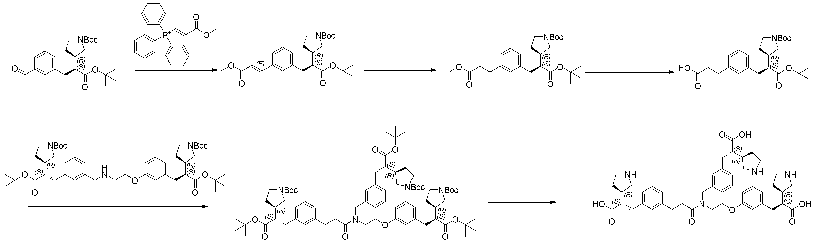

### Step 1: Synthesis of tert-butyl (R)-3-((S)-1-(tert-butoxy)-3-(3-(3-methoxy-3-oxopropyl)phenyl)-1-oxopropan-2-yl)pyrrolidine-1-carboxylate

*Tert-*butyl (R)-3-((S)-1-(tert-butoxy)-3-(3-formylphenyl)-1-oxopropan-2-yl)pyrrolidine-1-carboxylate(600mg) was dissolved in dichloromethane (15mL), and (3-methoxy-3-oxoprop-1-en-1-yl)triphenylphosphonium(994.32mg) was added at 0°C; under nitrogen, the mixture was heated to room temperature and stirred for 16h. The reaction mixture was concentrated and purified by column chromatography (petroleum ether:ethyl acetate=5:1) to give the target product (650mg). LCMS(ESI)[M+H]⁺=460.3.

### Step 2: Synthesis of tert-butyl (R)-3-((S)-1-(tert-butoxy)-3-(3-((E)-3-methoxy-3-oxoprop-1-en-1-yl)phenyl)-1-oxopropan-2-yl)pyrrolidine-1-carboxylate

The product from the first step (650mg) was dissolved in methanol (10mL), followed by adding Pd/C (75.03mg); the reaction mixture was stirred at room temperature for 2h under hydrogen (balloon pressure). The reaction mixture was filtered through diatomaceous earth, and the filtrate was concentrated to give the target product (600mg). LCMS(ESI)[M+H]⁺=462.3; ¹HNMR(400Hz, CDCl₃) δ 7.21-7.15 (m, 1H), 7.05-6.97 (m, 3H), 3.67 (s, 3H), 3.59-3.41 (m, 1H), 3.30-3.19 (m, 1H), 3.08-2.94 (m, 1H), 2.90 (t, *J*=7.8Hz, 2H), 2.85-2.72 (m, 2H), 2.60 (t, *J*=7.9Hz, 2H), 2.53-2.43 (m, 1H), 2.41-2.30 (m, 1H), 1.98-1.90 (m, 1H), 1.71-1.57 (m, 2H), 1.47 (s, 9H), 1.27 (s, 9H).

### Step 3: Synthesis of 3-(3-((S)-3-(tert-butoxy)-2-((R)-1-(tert-butoxycarbonyl)pyrrolidin-3-yl)-3-oxopropyl)phenyl)propanoic acid

The product from the second step (600mg) was dissolved in a mixture of tetrahydrofuran (3mL), water (1.5mL) and methanol (1.5mL), and lithium hydroxide (46.7mg) was added; the reaction mixture was stirred at room temperature for 2h. The pH of the mixture was adjusted to 5 with hydrochloric acid aqueous solution (1.0M). The mixture was separated, the organic phases were combined, dried over anhydrous sodium sulfate, filtered, and the filtrate was concentrated to give the target product (500mg). LCMS(ESI)[M+H]⁺=448.2; ¹HNMR(400MHz, CDCl₃) δ 7.21 (t, *J*=7.8Hz, 1H), 7.09-7.01 (m, 3H), 3.50-3.36 (m, 2H), 3.19 (dd, *J*=17.6, 10.7Hz, 1H), 2.96-2.87 (m, 3H), 2.81-2.67 (m, 2H), 2.67-2.61 (m, 2H), 2.52-2.43 (m, 1H), 2.39-2.27 (m, 1H), 2.02-1.93 (m, 1H), 1.64-1.53 (m, 1H), 1.45 (s, 9H), 1.37 (s, 9H).

### Step 4: Synthesis of tert-butyl (R)-3-((S)-1-(tert-butoxy)-3-(3-(N-(2-(3-((S)-3-(tert-butoxy)-2-((R)-1-(tert-butoxycarbonyl)pyrrolidin-3-yl)-3-oxopropyl)phenoxy)ethyl)-3-(3-((S)-3-(tert-butoxy)-2-((R)-1-(tert-butoxycarbonyl)pyrrolidin-3-yl)-3-oxopropyl)phenyl)propanamido)methyl)phenyl)-1-oxopropan-2-yl)pyrrolidine-1-carboxylate

The product from the third step (70mg) was dissolved in ethyl acetate (4mL), followed by adding *tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (128.58mg), Propylphosphonic anhydride (76.3mg), and *N,N-*diisopropylethylamine (60.64mg); the reaction mixture was stirred at 50°C for 3h under nitrogen protection. LCMS showed the reaction was completed. The reaction mixture was concentrated to give a crude product, which was purified by rapid chromatography (Silica gel, tetrahydrofuran:petroleum ether=1:3) to give the target compound (100mg). LCMS(ESI)[M+H]⁺=1151.0.

### Step 5: Synthesis of (S)-3-(3-(3-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-3-oxopropyl)phenyl)-2-((R)-pyrrolidin-3-yl)propanoic acid

The product from the fourth step (100mg) was dissolved in dioxane (2mL), followed by adding hydrochloric acid-1,4-dioxane (4.0M, 2mL); the mixture was stirred at room temperature for 2h under nitrogen. LCMS showed the reaction was completed. The reaction mixture was concentrated and separated and purified by Prep-HPLC (0.1% NH₄HCO₃ in acetonitrile) to give the target compound (23.8mg). LCMS(ESI)[M+H]⁺=783.3; ¹HNMR(400MHz, D₂O) δ 7.23-6.96 (m, 6H), 6.95-6.76 (m, 4H), 6.70-6.64 (m, 1H), 6.60-6.56 (m, 1H), 4.56-4.44 (m, 2H), 4.00 (t, *J*=5.2Hz, 1H), 3.93-3.84 (m, 1H), 3.67-3.57 (m, 2H), 3.41-3.25 (m, 6H), 3.19-3.08 (m, 3H), 2.90-2.48 (m, 13H), 2.42-2.25 (m, 6H), 2.10-1.94 (m, 3H), 1.72-1.57 (m, 3H).

### Example 55 (Compound 141)

### Preparation of (S)-3-(3-(2-(1,3-bis(3-((S)-2-carboxy-2-((R)-pyrrolidin-3-yl)ethyl)benzyl)ureido)ethoxy)phenyl)-2-((R)-pyrrolidin-3-yl)propanoic acid

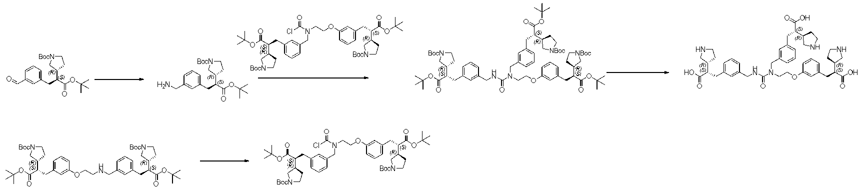

### Step 1: Synthesis of tert-butyl (R)-3-((S)-3-(3-(aminomethyl)phenyl)-1-(tert-butoxy)-1-oxopropan-2-yl)pyrrolidine-1-carboxylate

*Tert*-butyl (R)-3-((S)-1-(tert-butoxy)-3-(3-formylphenyl)-1-oxopropan-2-yl)pyrrolidine-1-carboxylate(1g) was dissolved in ammonia methanol solution (7 M, 10mL), and glacial acetic acid (0.1mL) was added at room temperature. The reaction mixture was stirred at 40°C for 5h; sodium cyanoborohydride (153mg) was added, and the reaction mixture was heated to 70°C and stirred for 3h. LCMS showed the reaction was completed. The reaction mixture was rotary-evaporated to dryness, and the crude product was purified by column chromatography (petroleum ether/ethyl acetate=1/4) to give the target compound (300mg). LCMS(ESI)[M+H]⁺=405.3.

### Step 2: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)methyl)phenyl)-1-oxopropan-2-yl)pyrrolidine-1-carboxylate

*Tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (300mg) was dissolved in tetrahydrofuran (10mL), triethylamine (364.28mg) and triphosgene (106.83mg) were added at 0°C; the mixture was stirred at 0°C for 1h. LCMS showed the reaction was completed. Water was added to the mixture, which was extracted with ethyl acetate, and the organic phase was dried over anhydrous sodium sulfate, filtered, and the filtrate was concentrated to give the target compound (250mg). LCMS(ESI)[M+H-Boc]⁺=784.4.

### Step 3: Synthesis of tert-butyl (R)-3-((S)-3-(3-(2-(1,3-bis(3-((S)-3-(tert-butoxy)-2-((R)-1-(tert-butoxycarbonyl)pyrrolidin-3-yl)-3-oxopropyl)benzyl)ureido)ethoxy)phenyl)-1-(tert-butoxy)-1-oxopropan-2-yl)pyrrolidine-1-carboxylate

The product from the second step (200mg) was dissolved in tetrahydrofuran (5mL), and triethylamine (68.64mg), 4-dimethylaminopyridine (13.81mg) and *tert*-butyl (R)-3-((S)-3-(3-(aminomethyl)phenyl)-1-(tert-butoxy)-1-oxopropan-2-yl)pyrrolidine-1-carboxylate (109.76mg) were added at room temperature; the reaction mixture was stirred at 50°C for 16h. LCMS showed the reaction was completed, and the reaction mixture was rotary-evaporated to dryness to give a crude product, which was purified by column chromatography (PE/EA=3/1) to give the target compound (100mg). LCMS(ESI)[M+H-Boc]⁺=1152.5.

### Step 4: Synthesis of (S)-3-(3-(2-(1,3-bis(3-((S)-2-carboxy-2-((R)-pyrrolidin-3-yl)ethyl)benzyl)ureido)ethoxy)phenyl)-2-((R)-pyrrolidin-3-yl)propanoic acid

*Tert-*butyl (R)-3-((S)-3-(3-(2-(1,3-bis(3-((S)-3-(tert-butoxy)-2-((R)-1-(tert-butoxycarbonyl)pyrrolidin-3-yl)-3-oxopropyl)benzyl)ureido)ethoxy)phenyl)-1-(tert-butoxy)-1-oxopropan-2-yl)pyrrolidine-1-carboxylate (100mg) was dissolved in 1,4-dioxane (2mL), hydrochloric acid/1,4-dioxane (4 M, 2mL) was added at room temperature; the reaction mixture was stirred at 30°C for 3h. LCMS showed the reaction was completed, the reaction mixture was rotary-evaporated to dryness, and the crude product was purified by Pre-HPLC (30 mmol NH₄HCO₃ in H₂O/ACN) to give the target compound (41mg). LCMS(ESI)[M+H]⁺=784.3; ¹HNMR(400MHz, D₂O) δ 7.21-7.12 (m, 3H), 7.07-6.99 (m, 5H), 6.97 (s, 1H), 6.79 (d, *J*=7.5Hz, 1H), 6.61-6.55 (m, 2H), 4.51 (s, 2H), 4.27 (s, 2H), 4.08-4.01 (m, 2H), 3.70-3.62 (m, 2H), 3.39-3.24 (m, 6H), 3.19-3.07 (m, 3H), 2.86-2.77 (m, 1H), 2.74-2.56 (m, 7H), 2.54-2.47 (m, 1H), 2.39-2.25 (m, 6H), 2.07-1.95 (m, 3H), 1.70-1.55 (m, 3H).

### Example 56 (Compound 142)

### Preparation of (S)-3-(3-(2-((2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid

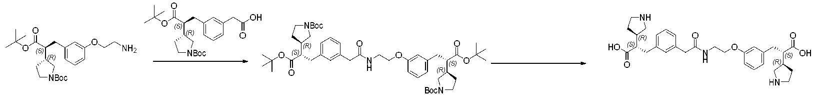

### Step 1: Synthesis of :tert-butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)-2-oxoethyl)phenyl)-1-oxopropan-2-yl)pyrrolidine-1-carboxylate

*Tert*-butyl (R)-3-((S)-3-(3-(2-aminoethoxy)phenyl)-1-(tert-butoxy)-1-oxopropan-2-yl)pyrrolidine-1-carboxylate(220mg) was dissolved in ethyl acetate (3mL). 2-(3-((S)-3-(tert-butoxy)-2-((R)-1-(tert-butoxycarbonyl)pyrrolidin-3-yl)-3-oxopropyl)phenyl)acetic acid(263mg), 1-propylphosphonic acid anhydride (644mg, 50% in ethyl acetate) and *N,N*-diisopropylethylamine (196mg) were added at room temperature. The mixture was stirred at room temperature for 16h. LCMS showed the reaction was completed. The reaction mixture was concentrated directly and separated and purified by rapid chromatography (Silica gel, petroleum ether:EtOAc=1:8) to give the target compound (280mg). LCMS(ESI)[M+H]⁺=850.2.

### Step 2: Synthesis of (S)-3-(3-(2-((2-(3-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid

The product from the first step (280mg) was dissolved in ethyl acetate (5mL), followed by adding hydrogen chloride-ethyl acetate (4.0M, 10mL). The mixture was stirred at room temperature for 24h. LCMS showed the reaction was completed. The reaction mixture was concentrated directly, separated and purified by preparative chromatography to give the target compound (129.85mg). LCMS(ESI)[M+H]⁺=538.1; ¹HNMR(400MHz, D₂O) δ 7.19 (t, *J*=8.1Hz, 2H), 7.07 (dd, *J*=7.7, 1.6Hz, 1H), 7.03 (dd, *J*=4.5, 2.7Hz, 2H), 6.83 (d, *J*=7.8Hz, 1H), 6.75-6.69 (m, 2H), 4.05 (t, *J*=5.2Hz, 2H), 3.49 (d, *J*=7.8Hz, 4H), 3.41 (dd, *J*=11.7, 7.1Hz, 1H), 3.37-3.28 (m, 3H), 3.21-3.11 (m, 2H), 2.87 (dd, *J*=11.7, 9.3Hz, 1H), 2.79-2.57 (m, 5H), 2.43-2.27 (m, 4H), 2.04 (ddt, *J*=17.9, 13.1, 5.7Hz, 2H), 1.65 (ddq, *J*=18.1*,* 13.0, 9.3Hz, 2H).

### Example 57 (Compound 143)

### Preparation of (S)-3-(3-(2-((3-((S)-2-carboxy-2-((R)-pyrrolidin-3-yl)ethyl)benzyl)amino)-2-oxoethyl)phenyl)-2-((R)-pyrrolidin-3-yl)propanoic acid

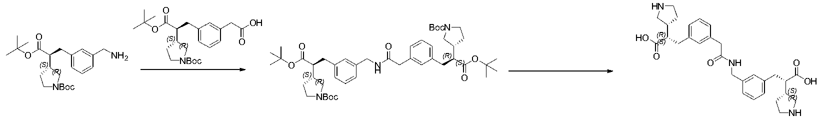

### Step 1: Synthesis of tert-butyl (R)-3-((S)-1-(tert-butoxy)-3-(3-((2-(3-((S)-3-(tert-butoxy)-2-((R)-1-(tert-butoxycarbonyl)pyrrolidin-3-yl)-3-oxopropyl)phenyl)acetamido)methyl)phenyl)-1-oxopropan-2-yl)pyrrolidine-1-carboxylate

*Tert*-butyl (R)-3-((S)-3-(3-(aminomethyl)phenyl)-1-(tert-butoxy)-1-oxopropan-2-yl)pyrrolidine-1-carboxylate (260mg) was dissolved in DMF (5mL), followed by adding 2-(3-((S)-3-(tert-butoxy)-2-((R)-1-(tert-butoxycarbonyl)pyrrolidin-3-yl)-3-oxopropyl)phenyl)acetic acid (307mg), O-(7-azabenzotriazol-1-yl)-N,N,N',N'-tetramethyluronium hexafluorophosphate (489mg) and *N*,*N*-diisopropylethylamine (249mg). The mixture was stirred at room temperature for 16h. LCMS showed the reaction was completed. The reaction mixture was concentrated directly and separated and purified by rapid chromatography (Silica gel, petroleum ether:ethyl acetate=1:8) to give the target compound (300mg). LCMS(ESI)[M+H]⁺=820.2.

### Step 2: Synthesis of (S)-3-(3-(2-((3-((S)-2-carboxy-2-((R)-pyrrolidin-3-yl)ethyl)benzyl)amino)-2-oxoethyl)phenyl)-2-((R)-pyrrolidin-3-yl)propanoic acid

The product from the first step (300mg) was dissolved in ethyl acetate (10mL), followed by adding hydrogen chloride-ethyl acetate (4.0M, 10mL). The mixture was stirred at room temperature for 24h. LCMS showed the reaction was completed. The reaction mixture was concentrated directly, separated and purified by preparative chromatography to give the target compound (208.1mg). LCMS(ESI)[M+H]⁺=508.1;¹HNMR(400MHz, D₂O) δ 7.23 (dt, *J*=14.8, 7.6Hz, 2H), 7.09 (dt, *J*=12.7, 4.7Hz, 4H), 7.04-6.93 (m, 2H), 4.27 (s, 2H), 3.54 (s, 2H), 3.41-3.26 (m, 4H), 3.14 (dddd, *J*=11.7, 9.2, 7.3, 1.8Hz, 2H), 2.87-2.63 (m, 6H), 2.44-2.30 (m, 4H), 2.04 (dq, *J*=10.0, 3.4Hz, 2H), 1.72-1.59 (m, 2H).

### Example 58 (Compound 144)

### Preparation of (2S,2'S,2"S)-3,3',3"-((nitrilotris(methylene))tris(benzo[d]isoxazole-6,3-diyl))tris(2-((R)-pyrrolidin-3-yl)propanoic acid)

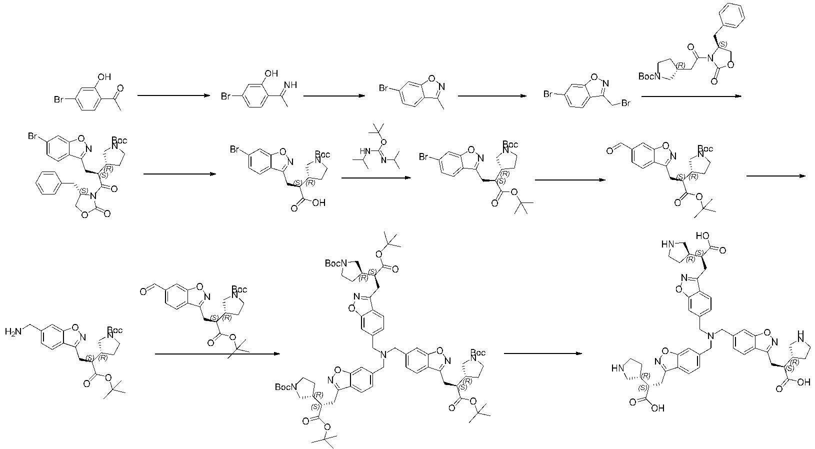

### Step 1: Synthesis of 5-bromo-2-(1-iminoethyl)phenol

1-(4-bromo-2-hydroxyphenyl)ethan-1-one (20g, 93.01mmol, 1.0eq) was dissolved in methanol (100mL), and a solution of ammonia in methanol (26.57mL, 186.01mmol, 2.0eq, 7.0M) was added at room temperature. The reaction was maintained at room temperature for 4h. LCMS showed the reaction was completed. The reaction mixture was concentrated directly to give the target product (19g). LCMS(ESI)[M+H]⁺=214.0/216.0; ¹HNMR(400MHz, DMSO-*d₆*) δ 15.14 (s, 1H), 10.26 (s, 1H), 7.38 (d, J=8.8Hz, 1H), 6.76 (d, J=2.0Hz, 1H), 6.50 (dd, J=8.8, 2.1Hz, 1H), 2.48 (s, 3H).

### Step 2: Synthesis of 6-bromo-3-methylbenzo[d]isoxazole

5-bromo-2-(1-iminoethyl)phenol (19g, 88.76mmol, 1.0eq) was dissolved in tetrahydrofuran (200mL), potassium carbonate (24.54g, 177.52mmol, 2.0eq) and N-chlorosuccinimide (17.78g, 133.14mmol, 1.5eq) were added sequentially; the reaction was maintained at room temperature for 16h and LCMS showed the reaction was completed. The reaction mixture was diluted with water (300mL), extracted with ethyl acetate (200 mL x 3), the organic phases were combined, washed with saturated aqueous sodium chloride solution, dried over anhydrous sodium sulfate, filtered, and the filtrate was concentrated to give a crude product, which was separated and purified by rapid chromatography (silica, petroleum ether/ethyl acetate=15/1) to give the target compound (16g). LCMS(ESI)[M+H]⁺=211.9/213.9; ¹HNMR(400MHz, DMSO-*d₆*) δ 8.06 (d, *J*=1.2Hz, 1H), 7.83 (d, *J*=8.4Hz, 1H), 7.57 (dd, *J*=8.4, 1.5Hz, 1H), 2.58 (s, 3H).

### Step 3: Synthesis of 6-bromo-3-(bromomethyl)benzo[d]isoxazole

6-bromo-3-methylbenzo[d]isoxazole (15.6g, 73.57mmol, 1.0eq) was dissolved in carbon tetrachloride (200mL). N-bromosuccinimide (14.4g, 80.93mmol, 1.1eq) and benzoyl peroxide (1.78g, 7.36mmol, 0.1eq) were added at room temperature. The reaction mixture was stirred at 80°C for 16h. The mixture was diluted with water (300mL), extracted with dichloromethane (200mL×3). The organic phases were combined, washed with saturated sodium chloride aqueous solution, dried over anhydrous sodium sulfate, filtered, and concentrated to dryness. The crude product was separated and purified by rapid chromatography (silicon dioxide, petroleum ether/ethyl acetate=15/1) to give the target product (16g). ¹HNMR(400MHz, CDCl₃) δ 7.80 (d, *J*=1.2Hz, 1H), 7.70 (d, *J*=8.4Hz, 1H), 7.51 (dd, *J*=8.4, 1.4Hz, 1H), 4.71 (s, 2H).

### Step 4: Synthesis of tert-butyl (R)-3-((S)-1-((S)-4-benzyl-2-oxooxazolidin-3-yl)-3-(6-bromobenzo[d]isoxazol-3-yl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate (6g, 15.45mmol, 1eq) was dissolved in tetrahydrofuran (70mL), lithium bis(trimethylsilyl)amide (20.08mL, 20.08mmol, 1.3eq, 1.0M) was added dropwise under nitrogen protection and ice bath; the mixture was stirred for 1h under ice bath. 6-bromo-3-(bromomethyl)benzo[d]isoxazole (8g, 19.25mmol, 1.25eq) was dissolved in tetrahydrofuran, and the mixture was added dropwise to the reaction system, then the reaction mixture was stirred for 16h at room temperature. LCMS showed the reaction was completed and then the reaction was quenched with aqueous ammonium chloride solution. The mixture was extracted with ethyl acetate. The organic phases were combined and washed with saturated sodium chloride solution, dried over anhydrous sodium sulfate, filtered and concentrated to give a crude product, which was separated and purified by rapid chromatography (silica gel, petroleum ether/tetrahydrofuran=3/1) to give the target product (6.0g). LCMS(ESI)[M+Na]⁺=620.1/622.1; ¹HNMR(400MHz, DMSO-*d₆*) δ 8.12 (d, *J*=1.2Hz, 1H), 7.91 (dd, *J*=8.3, 4.3Hz, 1H), 7.60 (d, *J*=8.4Hz, 1H), 7.28-7.17 (m, 5H), 4.68-4.63 (m, 1H), 4.61-4.52 (m, 1H), 4.12 (d, *J*=8.9Hz, 1H), 3.54-3.44 (m, 2H), 3.38 (d, *J*=9.3Hz, 1H), 3.22-3.09 (m, 2H), 3.08-2.97 (m, 2H), 2.86-2.78 (m, 1H), 2.69-2.61 (m, 1H), 2.06-1.88 (m, 2H), 1.74-1.66 (m, 1H), 1.39 (s, 9H).

### Step 5: Synthesis of (S)-3-(6-bromobenzo[d]isoxazol-3-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(6-bromobenzo[d]isoxazol-3-yl)-1-oxopropan-2-yl)pyrrolidine-1-carboxylate (8.7g, 14.54mmol, 1.0eq) was dissolved in tetrahydrofuran (100mL), hydrogen peroxide solution (18mL) was added under ice bath; an aqueous solution (10mL) of lithium hydroxide (522.24mg, 21.81mmol, 1.5eq) was added dropwise to the above solution under ice bath, and the mixture was stirred at room temperature for 16h. LCMS showed the reaction was completed, and the reaction was quenched with sodium sulfate, and aqueous sodium carbonate was added to adjust the pH=10. The mixture was extracted with ethyl acetate. The aqueous phase was retained, and its pH was adjusted to 4 with dilute hydrochloric acid (1M). The mixture was extracted with dichloromethane, and the organic phases were combined, washed with saturated sodium chloride solution, dried over anhydrous sodium sulfate, filtered, and concentrated to give the target product (5.5g). LCMS(ESI)[M+Na]⁺=461.1/463.1; ¹HNMR(400MHz, DMSO-*d₆*) δ 12.46 (s, 1H), 8.09 (d, *J*=1.2Hz, 1H), 7.88 (dd, *J*=8.1, 5.9Hz, 1H), 7.59 (dd, *J*=8.4, 1.3Hz, 1H), 3.63-3.48 (m, 1H), 3.44-3.38 (m, 1H), 3.29-3.23 (m, 1H), 3.17 (dd, *J*=12.7, 7.4Hz, 2H), 3.05 (t, *J*=9.9Hz, 1H), 2.94-2.83 (m, 1H), 2.46-2.30 (m, 1H), 1.97-1.89 (m, 1H), 1.67 (dq, *J*=21.7, 10.7Hz, 1H), 1.40 (s, 9H).

### Step 6: Synthesis of tert-butyl (R)-3-((S)-3-(6-bromobenzo[d]isoxazol-3-yl)-1-(tert-butoxy)-1-oxopropan-2-yl)pyrrolidine-1-carboxylate

(S)-3-(6-bromobenzo[d]isoxazol-3-yl)-2-((R)-1-(tert-butoxycarbonyl)pyrrolidin-3-yl)propanoic acid (5.5g, 12.52mmol, 1.0eq) was dissolved in 2-methyltetrahydrofuran (60mL), *tert-*butyl N,N'-diisopropylcarbamimidate (7.52g, 37.56mmol, 3.0eq) was added. The reaction mixture was stirred at 70°C for 16h under nitrogen protection. LCMS showed the reaction was completed. The mixture was diluted with tetrahydrofuran (50mL), filtered, and the filter cake was washed with acetonitrile. The filtrate was concentrated and separated and purified by rapid chromatography (silicon dioxide, petroleum ether/ethyl acetate=3/1) to give the target compound (3.5g). LCMS(ESI)[M+Na]⁺=517.1/519.1; ¹HNMR(400MHz, DMSO-*d₆*) δ 8.09 (d, *J*=1.3Hz, 1H), 7.91 (dd, *J*=8.2, 4.1Hz, 1H), 7.59 (dd, *J*=8.4, 1.5Hz, 1H), 3.57 (dt, *J*=14.2*,* 10.1Hz, 1H), 3.42-3.35 (m, 1H), 3.25-3.12 (m, 3H), 3.08 (t, *J*=10.0Hz, 1H), 2.91-2.81 (m, 1H), 2.46-2.31 (m, 1H), 1.96-1.86 (m, 1H), 1.74-1.60 (m, 1H), 1.40 (s, 9H), 1.19 (s, 9H).

### Step 7: Synthesis of tert-butyl (R)-3-((S)-1-(tert-butoxy)-3-(6-formylbenzo[d]isoxazol-3-yl)-1-oxopropan-2-yl)pyrrolidine-1-carboxylate

*Tert-*butyl (R)-3-((S)-3-(6-bromobenzo[d]isoxazol-3-yl)-1-(tert-butoxy)-1-oxopropan-2-yl)pyrrolidine-1-carboxylate (2.0g, 4.04mmol, 1.0eq) was dissolved in N,N-dimethylformamide (30mL), followed by adding sodium carbonate (855.78mg, 8.07mmol, 2.0eq), [1,1'-bis(diphenylphosphino)ferrocene]palladium(II) dichloride (296.21mg, 0.4mmol, 0.1eq), and triethylsilane (938.86mg, 8.07mmol, 2.0eq); the reaction mixture was replaced with carbon monoxide gas and the reaction was maintained at 80°C for 2h under a carbon monoxide. LCMS showed the reaction was completed. The reaction was quenched with water. The mixture was extracted with ethyl acetate. The organic phases were combined and washed with saturated sodium chloride solution, dried over anhydrous sodium sulfate, filtered and concentrated to give a crude product, which was separated and purified by rapid chromatography (silica gel, petroleum ether/ethyl acetate=5/1) to give the target compound (900mg). LCMS(ESI)[M+Na]⁺=467.1.

### Step 8: Synthesis of tert-butyl (R)-3-((S)-3-(6-(aminomethyl)benzo[d]isoxazol-3-yl)-1-(tert-butoxy)-1-oxopropan-2-yl)pyrrolidine-1-carboxylate

*Tert*-butyl (R)-3-((S)-1-(tert-butoxy)-3-(6-formylbenzo[d]isoxazol-3-yl)-1-oxopropan-2-yl)pyrrolidine-1-carboxylate (400mg, 0.9mmol, 1.0eq) was dissolved in ammonia methanol (10mL, 7 M), one drop of acetic acid was added, and the mixture was stirred at room temperature for 4h; sodium cyanoborohydride (278.19mg, 4.5mmol, 5eq) was added, and the reaction mixture was stirred at 70°C for 2h. LCMS showed the reaction was completed. The reaction mixture was quenched with water, concentrated, and purified by reversed-phase column chromatography (0.1% ammonia water) to give the target compound (200mg). LCMS(ESI)[M+H]⁺=446.2.

### Step 9: Synthesis of tri-tert-butyl 3,3',3"-((2S,2'S,2"S)-((nitrilotris(methylene))tris(benzo[d]isoxazole-6,3-diyl))tris(3-(tert-butoxy)-3-oxopropane-1,2-diyl))(3R,3'R,3"R)-tris(pyrrolidine-1-carboxylate)

*Tert-*butyl (R)-3-((S)-3-(6-(aminomethyl)benzo[d]isoxazol-3-yl)-1-(tert-butoxy)-1-oxopropan-2-yl)pyrrolidine-1-carboxylate (200mg, 0.45mmol, 1.0eq) was dissolved in ethanol (10mL), *tert-*butyl (R)-3-((S)-1-(tert-butoxy)-3-(6-formylbenzo[d]isoxazol-3-yl)-1-oxopropan-2-yl)pyrrolidine-1-carboxylate (399.08mg, 0.9mmol, 2.0eq) was added. One drop of acetic acid was added dropwise, and the mixture was stirred at room temperature for 4h. sodium cyanoborohydride (138.77mg, 2.24mmol, 5eq) was added, and the reaction mixture was stirred at room temperature for 16h. LCMS showed the reaction of starting material was completed. The reaction mixture was quenched with water and concentrated. The residue was purified by reversed-phase column chromatography (C₁₈, 0.1% trifluoroacetic acid in water) to give the target compound (100mg). LCMS(ESI)[M+Na]⁺=1324.4.

### Step 10: Synthesis of (25,2'S,2"S)-3,3',3"-((nitrilotris(methylene))tris(benzo[d]isoxazole-6,3-diyl))tris(2-((R)-pyrrolidin-3-yl)propanoic acid)

Tri-*tert*-butyl 3,3',3"-((2S,2'S,2"S)-((nitrilotris(methylene))tris(benzo[d]isoxazole-6,3-diyl))tris(3-(tert-butoxy)-3-oxopropane-1,2-diyl))(3R,3'R,3"R)-tris(pyrrolidine-1-carboxylate) (100mg, 0.08mmol, 1eq) was dissolved in 1,4-dioxane (2mL), hydrochloric acid/1,4-dioxane (2mL, 4M) was added, and the reaction mixture was stirred at room temperature for 16h. After the reaction was completed, the mixture was concentrated to dryness, dissolved in water, adjusted to pH=7 with sodium carbonate aqueous solution (1M). After concentration, the mixture was purified by high pressure liquid chromatography (0.1%NH₄HCO₃) to give the target compound (17.64g). LCMS(ESI)[M+H]⁺=834.5; ¹HNMR(400MHz, D₂O) δ 7.38 (d, *J*=7.7Hz, 3H), 7.11 (d, *J*=24.3Hz, 3H), 6.99 (t, *J*=8.3Hz, 3H), 3.31 (s, 6H), 3.25-2.98 (m, 12H), 2.84 (t, *J*=10.6Hz, 3H), 2.76 (d, *J*=10.4Hz, 3H), 2.61 (dd, *J*=13.6, 7.9Hz, 3H), 2.44-2.31 (m, 3H), 2.00 (s, 3H), 1.74-1.62 (m, 3H).

### Example 59 (Compound 145)

### Preparation of (S)-3-(3-(2-((3-((S)-2-carboxy-2-((R)-pyrrolidin-3-yl)ethyl)benzyl)(((3-((S)-2-carboxy-2-((R)-pyrrolidin-3-yl)ethyl)benzyl)oxy)carbonyl)amino)ethoxy)phenyl)-2-((R)-pyrrolidin-3-yl)propanoic acid

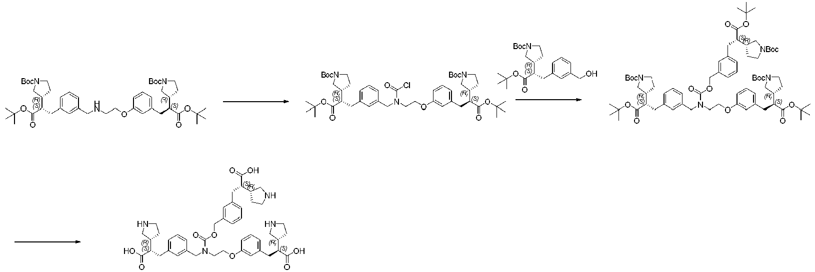

### Step 1: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)methyl)phenyl)-1-oxopropan-2-yl)pyrrolidine-1-carboxylate

*Tert*-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (300mg, 0.36mmol, 1eq) was dissolved in tetrahydrofuran (10mL), triethylamine (364.28mg, 3.6mmol, 10eq) and triphosgene (106.83mg, 0.36mmol, 1eq) were added at 0°C; the mixture was stirred at 0°C for 1h. LCMS showed the reaction was completed. Water was added to the mixture, which was extracted with ethyl acetate, and the organic phase was dried over anhydrous sodium sulfate, filtered, and the filtrate was concentrated to give the target compound (250mg). LCMS(ESI)[M+Na]⁺=906.3.

### Step 2: Synthesis of tert-butyl (R)-3-((S)-1-(tert-butoxy)-3-(3-(((((3-((S)-3-(tert-butoxy)-2-((R)-1-(tert-butoxycarbonyl)pyrrolidin-3-yl)-3-oxopropyl)benzyl)oxy)carbonyl)(2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

*Tert-*butyl (R)-3-((S)-1-(tert-butoxy)-3-(3-(hydroxymethyl)phenyl)-1-oxopropan-2-yl)pyrrolidine-1-carboxylate (111.93mg, 0.28mmol, 1.2eq) was dissolved in tetrahydrofuran (5mL), sodium hydride (6.35mg, 0.28mmol, 1.2eq) was added at 0°C, and the mixture was stirred at 0°C for 0.5h. *tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)methyl)phenyl)-1-oxopropan-2-yl)pyrrolidine-1-carboxylate (200mg, 0.23mmol, 1eq) was added, and the mixture was stirred at 0°C for 2h. LCMS showed the reaction was completed. Water was added, and the mixture was extracted with ethyl acetate. The organic phase was dried over anhydrous sodium sulfate, filtered, and the filtrate was removed to dryness to give a crude product, which was purified by column chromatography (petroleum ether:ethyl acetate=3:1) to give the target compound (100mg). LCMS(ESI)[M+H-Boc]⁺=1153.6.

### Step 3: Synthesis of (S)-3-(3-(2-((3-((S)-2-carboxy-2-((R)-pyrrolidin-3-yl)ethyl)benzyl)(((3-((S)-2-carboxy-2-((R)-pyrrolidin-3-yl)ethyl)benzyl)oxy)carbonyl)amino)ethoxy)phenyl)-2-((R)-pyrrolidin-3-yl)propanoic acid

*Tert*-butyl (R)-3-((S)-1-(tert-butoxy)-3-(3-(((((3-((S)-3-(tert-butoxy)-2-((R)-1-(tert-butoxycarbonyl)pyrrolidin-3-yl)-3-oxopropyl)benzyl)oxy)carbonyl)(2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (100mg, 0.08mmol, 1eq) was dissolved in 1,4-dioxane (2mL), hydrochloric acid/1,4-dioxane (4 M, 2mL) solution was added at room temperature, and the mixture was stirred at room temperature for 3h. LCMS showed the reaction was completed, the reaction mixture was rotary-evaporated to dryness, and the crude product was purified by Pre-HPLC (30 mmol NH₄HCO₃ in H₂O / ACN) to give the target compound (33mg). LCMS(ESI)[M+H]⁺=785.6; ¹HNMR(400MHz, D₂O) δ 7.30-7.21 (m, 1H), 7.20-7.11 (m, 4H), 7.06-6.90 (m, 4H), 6.84-6.75 (m, 1H), 6.66-6.60 (m, 1H), 6.58-6.51 (m, 1H), 5.11-5.02 (m, 2H), 4.58-4.39 (m, 2H), 4.14-3.94 (m, 2H), 3.65 (s, 2H), 3.42-3.24 (m, 6H), 3.20-3.07 (m, 3H), 2.84-2.47 (m, 9H), 2.42-2.24 (m, 6H), 2.10-1.95 (m, 3H), 1.74-1.56 (m, 3H).

### Example 60 (Compound 146)

### Preparation of (S)-3-(3-(2-((3-((S)-2-carboxy-2-((R)-pyrrolidin-3-yl)ethyl)benzyl)(2-((3-((S)-2-carboxy-2-((R)-pyrrolidin-3-yl)ethyl)phenyl)amino)ethyl)amino)-2-oxoethyl)phenyl)-2-((R)-pyrrolidin-3-yl)propanoic acid

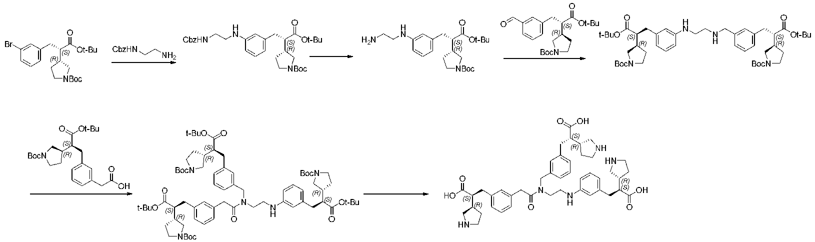

### Step 1: Synthesis of tert-butyl (R)-3-((S)-3-(3-((2-(((benzyloxy)carbonyl)amino)ethyl)amino)phenyl)-1-(tert-butoxy)-1-oxopropan-2-yl)pyrrolidine-1-carboxylate:

*Tert-*butyl (R)-3-((S)-3-(3-bromophenyl)-1-(tert-butoxy)-1-oxopropan-2-yl)pyrrolidine-1-carboxylate(500mg) was dissolved in 1,4-dioxane (10mL), benzyl (2-aminoethyl)carbamate (427.44mg), potassium carbonate (456.24mg) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (99.86mg) were added at room temperature; the mixture was stirred at 100°C for 16h. LCMS showed the reaction was completed. The reaction mixture was rotary-evaporated to dryness, and the crude product was purified by column chromatography (petroleum ether:ethyl acetate=4:1) to give the target compound (200mg). LCMS(ESI)[M+H]⁺=568.2; ¹HNMR(400MHz, CDCl₃) δ 7.37-7.31 (m, 5H), 7.08-7.02 (m, 1H), 6.55-6.49 (m, 1H), 6.45 (d, *J*=8.2Hz, 1H), 6.42-6.37 (m, 1H), 5.11 (s, 2H), 3.60-3.37 (m, 4H), 3.32-3.20 (m, 3H), 3.03-2.91 (m, 1H), 2.82-2.63 (m, 2H), 2.51-2.41 (m, 1H), 2.39-2.27 (m, 1H), 1.97-1.89 (m, 1H), 1.70-1.64 (m, 1H), 1.46 (s, 9H), 1.30 (s, 9H).

### Step 2: Synthesis of tert-butyl (R)-3-((S)-3-(3-((2-aminoethyl)amino)phenyl)-1-(tert-butoxy)-1-oxopropan-2-yl)pyrrolidine-1-carboxylate:

The product from step 1 (150mg) was dissolved in methanol (5mL), Pd/C (30mg) was added at room temperature. Replaced with hydrogen gas three times, the mixture was stirred at room temperature for 16h under hydrogen. LCMS showed the reaction was completed. The reaction mixture was filtered, and the filtrate was concentrated to give the target compound (110mg). LCMS(ESI)[M+H]⁺=434.3.

### Step 3: Synthesis of tert-butyl (R)-3-((S)-1-(tert-butoxy)-3-(3-(((2-((3-((S)-3-(tert-butoxy)-2-((R)-1-(tert-butoxycarbonyl)pyrrolidin-3-yl)-3-oxopropyl)phenyl)amino)ethyl)amino)methyl)phenyl)-1-oxopropan-2-yl)pyrrolidine-1-carboxylate:

The product from step 2 (110mg) and *tert-*butyl (R)-3-((S)-1-(tert-butoxy)-3-(3-formylphenyl)-1-oxopropan-2-yl)pyrrolidine-1-carboxylate(122.85mg) was dissolved in ethanol (5mL), and titanium tetraisopropanolate (144.21mg) was added at room temperature; and the reaction mixture was stirred at room temperature for 1h. Sodium borohydride (14.4mg) was added, and the reaction mixture was stirred the at room temperature for 2h. LCMS showed the reaction was completed. Saturated ammonium chloride solution was added, and the mixture was extracted with ethyl acetate. The organic phase was dried over anhydrous sodium sulfate, filtered, and the filtrate was rotary-evaporated to dryness to give a crude product, which was purified by column chromatography (petroleum ether:ethyl acetate=3:1) to give the target compound (75mg). LCMS(ESI)[M+H]⁺=822.0.

### Step 4: Synthesis of tert-butyl (R)-3-((S)-1-(tert-butoxy)-3-(3-((2-(3-((S)-3-(tert-butoxy)-2-((R)-1-(tert-butoxycarbonyl)pyrrolidin-3-yl)-3-oxopropyl)phenyl)-N-(2-((3-((S)-3-(tert-butoxy)-2-((R)-1-(tert-butoxycarbonyl)pyrrolidin-3-yl)-3-oxopropyl)phenyl)amino)ethyl)acetamido)methyl)phenyl)-1-oxopropan-2-yl)pyrrolidine-1-carboxylate:

The product from step 3 (25mg) and 2-(3-((S)-3-(tert-butoxy)-2-((R)-1-(tert- butoxycarbonyl)pyrrolidin-3-yl)-3-oxopropyl)phenyl)acetic acid (14.52mg) were dissolved in ethyl acetate (3mL). 1-propanephosphonic acid anhydride (14.53mg) and *N,N-*diisopropylethylamine (7.87mg) were added at room temperature. The reaction mixture was stirred at 50°C for 16h. LCMS showed the reaction was completed. The reaction mixture was rotary-evaporated to dryness to give the crude product, which was purified by column chromatography (petroleum ether:ethyl acetate=3:1) to give the target compound (20mg). LCMS(ESI)[M+H-Boc]⁺=1136.6.

Step 5: The product from step 4 (20mg) was dissolved in 1,4-dioxane (1mL), hydrochloric acid/1,4-dioxane (4 M, 1mL) was added at room temperature, and the mixture was stirred at room temperature for 3h. LCMS showed the reaction was completed, the reaction mixture was evaporated to dryness, and the crude product was purified by Pre-HPLC (30 mmol NH₄HCO₃ in H₂O/ACN) to give the target compound (2.07mg). LCMS(ESI)[M+H]⁺=768.8; ¹HNMR(400MHz, D₂O) δ 7.25-7.18 (m, 2H), 7.15-7.05 (m, 3H), 7.01-6.74 (m, 4H), 6.64-6.59 (m, 1H), 6.49 (dd, *J*=20.0, 7.4Hz, 2H), 4.62-4.51 (m, 2H), 3.70-3.58 (m, 2H), 3.53-3.41 (m, 2H), 3.37-3.19 (m, 8H), 3.18-3.05 (m, 3H), 2.83-2.65 (m, 6H), 2.64-2.52 (m, 3H), 2.45-2.25 (m, 6H), 2.09-1.94 (m, 3H), 1.73-1.55 (m, 3H).

### Example 61 (Compound 147)

### Preparation of (S)-3-(3-(3-((3-((S)-2-carboxy-2-((R)-pyrrolidin-3-yl)ethyl)benzyl)(2-((3-((S)-2-carboxy-2-((R)-pyrrolidin-3-yl)ethyl)phenyl)amino)ethyl)amino)-3-oxopropyl)phenyl)-2-((R)-pyrrolidin-3-yl)propanoic acid

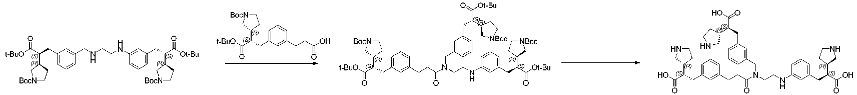

### Step 1: Synthesis of tert-butyl (R)-3-((S)-1-(tert-butoxy)-3-(3-((2-(N-(3-((S)-3-(tert-butoxy)-2-((R)-1-(tert-butoxycarbonyl)pyrrolidin-3-yl)-3-oxopropyl)benzyl)-3-(3-((S)-3-(tert-butoxy)-2-((R)-1-(tert-butoxycarbonyl)pyrrolidin-3-yl)-3-oxopropyl)phenyl)propanamido)ethyl)amino)phenyl)-1-oxopropan-2-yl)pyrrolidine-1-carboxylate:

*Tert-*butyl (R)-3-((S)-1-(tert-butoxy)-3-(3-(((2-((3-((S)-3-(tert-butoxy)-2-((R)-1-(tert-butoxycarbonyl)pyrrolidin-3-yl)-3-oxopropyl)phenyl)amino)ethyl)amino)methyl)phenyl)-1-oxopropan-2-yl)pyrrolidine-1-carboxylate (50mg, 0.06mmol, 1eq) and 3-(3-((S)-3-(tert-butoxy)-2-((R)-1-(tert-butoxycarbonyl)pyrrolidin-3-yl)-3-oxopropyl)phenyl)propanoic acid (29.98mg, 0.07mmol, 1.1eq) were dissolved in ethyl acetate (3mL). 1-propanephosphonic acid anhydride (29.06mg, 0.09mmol, 1.5eq) and N,N-diisopropylethylamine (23.61mg, 0.18mmol, 3eq) were added at room temperature; the reaction mixture was stirred at 50°C for 16h. LCMS showed the reaction was completed. The reaction mixture was concentrated to dryness to give the crude product, which was purified by column chromatography (petroleum ether:ethyl acetate=3:1) to give the target compound (50mg). LCMS(ESI)[M+H-Boc]⁺=1150.6.

Step 2: The product from step 1 (50mg, 0.04mmol, 1eq) was dissolved in 1,4-dioxane (1mL), and hydrochloric acid/1,4-dioxane (4 M, 1mL) solution was added at room temperature. The mixture was stirred at room temperature for 3h. LCMS showed the reaction was completed, the reaction mixture was rotary-evaporated to dryness, and the crude product was purified by Pre-HPLC (30 mmol NH₄HCO₃ in H₂O/ACN) to give the target compound (8.8mg). LCMS(ESI)[M+H]⁺=782.6. ¹HNMR(400MHz, D₂O) δ 7.22-6.99 (m, 5H), 6.98-6.69 (m, 4H), 6.63-6.58 (m, 1H), 6.52-6.43 (m, 2H), 4.50-4.44 (m, 1H), 4.40-4.31 (m, 1H), 3.44-3.22 (m, 8H), 3.18-3.05 (m, 5H), 2.83-2.65 (m, 8H), 2.66-2.49 (m, 5H), 2.42-2.25 (m, 6H), 2.11-1.95 (m, 3H), 1.73-1.57 (m, 3H).

### Example 62 (Compound 148)

### Preparation of (2S,2'S)-3,3'-(((((3-(3-((S)-2-carboxy-2-((R)-pyrrolidin-3-yl)ethyl)phenyl)propanoyl)azanediyl)bis(ethane-2,1-diyl))bis(oxy))bis(3,1-phenylene))bis(2-((R)-pyrrolidin-3-yl)propanoic acid)

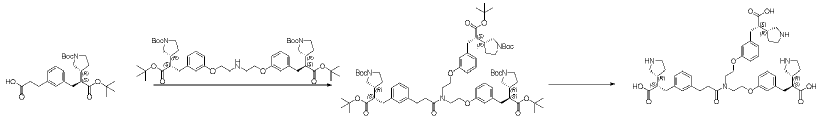

### Step 1: Synthesis of di-tert-butyl 3,3'-((2S,2'S)-(((((3-(3-((S)-3-(tert-butoxy)-2-((R)-1-(tert-butoxycarbonyl)pyrrolidin-3-yl)-3-oxopropyl)phenyl)propanoyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate

Di-*tert*-butyl 3,3'-((2 S,2'S)-(((azanediylbis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)(100mg, 0.12mmol, 1eq) was dissolved in ethyl acetate (4mL), and 3-(3-((S)-3-(tert-butoxy)-2-((R)-1-(tert-butoxycarbonyl)pyrrolidin-3-yl)-3-oxopropyl)phenyl)propanoic acid(53.71mg, 0.12mmol, 1eq), 1-propylphosphonic acid anhydride (57.27mg, 0.18mmol, 1.5eq) and *N*,*N*-diisopropylethylamine (46.53mg, 0.36mmol, 3eq) were added; the reaction was maintained at 50°C for 18h under nitrogen protection. LCMS showed the reaction was completed. The reaction mixture was concentrated to give a crude product, which was separated and purified by rapid chromatography (Silica gel, tetrahydrofuran:petroleum ether=1:3) to give the target compound (70mg). LCMS(ESI)[M+H-Boc]⁺=1181.6.

### Step 2: Synthesis of (2S,2'S)-3,3'-(((((3-(3-((S)-2-carboxy-2-((R)-pyrrolidin-3-yl)ethyl)phenyl)propanoyl)azanediyl)bis(ethane-2,1-diyl))bis(oxy))bis(3,1-phenylene))bis(2-((R)-pyrrolidin-3-yl)propanoic acid):

Di-*tert*-butyl 3,3'-((2S,2'S)-(((((3-(3-((S)-3-(tert-butoxy)-2-((R)-1-(tert-butoxycarbonyl)pyrrolidin-3-yl)-3-oxopropyl)phenyl)propanoyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate) (70mg, 0.05mmol, 1eq) was dissolved in dioxane (2mL), hydrochloric acid/1,4-dioxane (4 M, 2mL) was added; the reaction mixture was stirred at room temperature for 2h under nitrogen. The reaction mixture was concentrated to give a crude product, which was purified by Prep-HPLC (0.1% NH₄HCO₃ in water, MeCN) to give the target compound (12mg). LCMS(ESI)[M+H]⁺=813.5;¹HNMR(400MHz, D₂O) δ 7.17 (t, *J*=7.6Hz, 2H), 7.04-6.98 (m, 1H), 6.97-6.91 (m, 2H), 6.88 (d, *J*=7.6Hz, 1H), 6.80 (d, *J*=7.5Hz, 2H), 6.71-6.56 (m, 4H), 4.07-3.99 (m, 2H), 3.98-3.88 (m, 2H), 3.71-3.58 (m, 4H), 3.38-3.09 (m, 9H), 2.94-2.47 (m, 13H), 2.41-2.27 (m, 6H), 2.07-1.94 (m, 3H), 1.71-1.54 (m, 3H).

### Example 63 (Compound 149)

### Preparation of (2S,2'S,2"S)-3,3',3''-(((benzene-1,3,5-triyltris(methylene))tris(oxy))tris(benzene-3,1-diyl))tris(2-((R)-pyrrolidin-3-yl)propanoic acid):

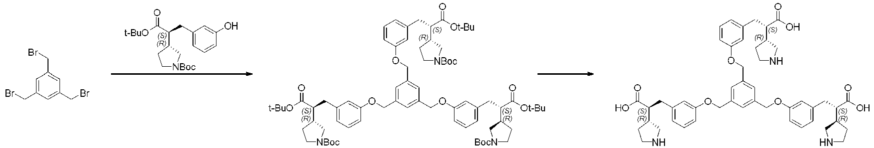

### Step 1: Synthesis of tri-tert-butyl 3,3',3"-((2S,2'S,2"S)-(((benzene-1,3,5-triyltris(methylene))tris(oxy))tris(benzene-3,1-diyl))tris(3-(tert-butoxy)-3-oxopropane-1,2-diyl))(3R,3'R,3"R)-tris(pyrrolidine-1-carboxylate):

Under nitrogen protection, 1,3,5-tris(bromomethyl)benzene (60mg, 0.17mmol, 1eq) was dissolved in acetonitrile (5mL), *tert-*butyl (R)-3-((S)-1-(tert-butoxy)-3-(3-hydroxyphenyl)-1-oxopropan-2-yl)pyrrolidine-1-carboxylate(329mg, 0.84mmol, 5eq) and potassium carbonate (139.42mg, 1.01mmol, 6eq) were added, and the reaction mixture was stirred at 70°C for 18hours. The reaction mixture was concentrated under reduced pressure and purified by rapid chromatography (silica gel, tetrahydrofuran:petroleum ether=1:5) to give the target compound (160mg). LCMS(ESI)[M+H-Boc]⁺=1188.7.

Step 2: The product from step 1 (170mg, 0.13mmol, 1eq) was dissolved in 1,4-dioxane (3mL), hydrochloric acid/1,4-dioxane (3mL, 4.0M) was added, and the reaction mixture was stirred at room temperature for 2h. The reaction mixture was concentrated, adjusted to pH=7 with sodium hydroxide (1.0M), and purified by Prep-HPLC (C₁₈, 0.1% NH₄HCO₃ in water, MeCN) to give the target compound (42.21mg). LCMS(ESI)[M+H]⁺=820.5; ¹HNMR(400MHz, D₂O) δ 7.00 (t, *J*=7.7Hz, 3H), 6.90 (s, 3H), 6.73 (d, *J*=7.6Hz, 3H), 6.61 (s, 3H), 6.42 (d, *J*=7.8Hz, 3H), 4.39 (s, 6H), 3.27-3.17 (m, 3H), 3.06-2.95 (m, 6H), 2.69-2.58 (m, 3H), 2.52 (t, *J*=10.3Hz, 3H), 2.40-2.15 (m, 9H), 1.96-1.87 (m, 3H), 1.64-1.51 (m, 3H).

### Example 64 (Compound 150)

### Preparation of (2S,2'S)-3,3'-((((R)-2-(((3-((S)-2-carboxy-2-((R)-pyrrolidin-3-yl)ethyl)benzyl)oxy)methyl)piperazine-1,4-diyl)bis(methylene))bis(3,1-phenylene))bis(2-((R)-pyrrolidin-3-yl)propanoic acid)

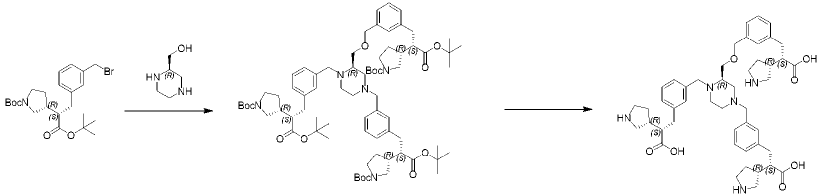

### Step 1: Synthesis of di-tert-butyl 3,3'-((2S,2'S)-((((R)-2-(((3-((S)-3-(tert-butoxy)-2-((R)-1-(tert-butoxycarbonyl)pyrrolidin-3-yl)-3-oxopropyl)benzyl)oxy)methyl)piperazine-1,4-diyl)bis(methylene))bis(3,1-phenylene))bis(3-(tert-butoxy)-3-oxopropane-1,2-diyl))(3R,3'R)-bis(pyrrolidine-1-carboxylate)

Under nitrogen protection, (R)-piperazin-2-ylmethanol(80mg, 0.69mmol, 1eq) was dissolved in acetonitrile (6mL), *tert*-butyl (R)-3-((S)-3-(3-(bromomethyl)phenyl)-1-(tert-butoxy)-1-oxopropan-2-yl)pyrrolidine-1-carboxylate(967.81mg, 2.07mmol, 3eq) and *N,N-*diisopropylethylamine (0.36mL, 2.07mmol, 3eq) were added. The mixture was stirred at 70°C for 18hours. The reaction mixture was concentrated to give a crude product, which was purified by reversed-phase column chromatography (C₁₈, 0.1% trifluoroacetic acid in acetonitrile) to give the target compound (500mg). LCMS(ESI)[M+H]⁺=1278.6; ¹HNMR(400MHz, DMSO-*d₆*) δ 7.50-7.34 (m, 6H), 7.31-7.23 (m, 3H), 7.18-7.07 (m, 3H), 5.10-4.79 (m, 3H), 4.44-4.31 (m, 6H), 3.88-3.61 (m, 6H), 3.52-3.30 (m, 9H), 3.23-3.00 (m, 6H), 2.98-2.88 (m, 3H), 2.81-2.65 (m, 6H), 2.34-2.21 (m, 3H), 1.88-1.78 (m, 3H), 1.62-1.53 (m, 3H), 1.38 (s, 27H), 1.19 (d, *J*=8.0Hz, 27H).

Step 2: The product from step 1 (300mg, 0.23mmol, 1eq) was dissolved in 1,4-dioxane (5mL), followed by adding hydrochloric acid/1,4-dioxane (5mL, 4.0M). The reaction mixture was stirred at room temperature for 3h. Upon completion, the reaction mixture was concentrated and the pH was adjusted to 7 with sodium hydroxide (1.0M) aqueous solution. The mixture was separated and purified by prep-HPLC (C₁₈, 0.1%NH₄HCO₃ in H₂O/ACN) to give the target compound (57.44mg). LCMS(ESI)[M+H]⁺=810.5; ¹HNMR(400MHz, D₂O) δ 7.41-7.25 (m, 7H), 7.16-7.09 (m, 5H), 4.74-4.72 (m, 2H), 4.30 (q, *J*=13.1Hz, 2H), 4.05 (d, *J*=12.9Hz, 1H), 3.89-3.79 (m, 2H), 3.53-3.41 (m, 4H), 3.38-3.29 (m, 5H), 3.24-3.07 (m, 6H), 3.00-2.89 (m, 4H), 2.83-2.67 (m, 7H), 2.44-2.31 (m, 6H), 2.08-1.97 (m, 3H), 1.74-1.58 (m, 3H).

### Example 65 (Compound 151)

### Preparation of (2S,2'S)-3,3'-((((S)-2-(((3-((S)-2-carboxy-2-((R)-pyrrolidin-3-yl)ethyl)benzyl)oxy)methyl)piperazine-1,4-diyl)bis(methylene))bis(3,1-phenylene))bis(2-((R)-pyrrolidin-3-yl)propanoic acid)

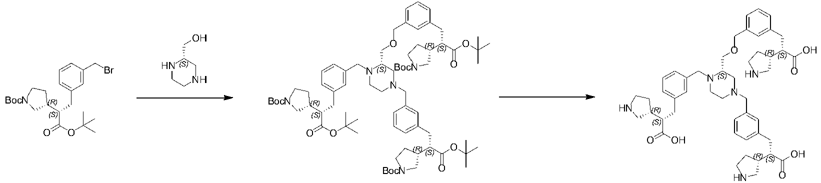

Compound 151 (43.77mg) was prepared using a procedure similar to that described for Example 64, except using (S)-piperazin-2-ylmethanol(30mg, 0.16mmol, 1eq) as the starting material. LCMS(ESI)[M+H]⁺=810.6; ¹HNMR(400MHz, D₂O) δ 7.42-7.22 (m, 7H), 7.19-7.09 (m, 5H), 4.78-4.72 (m, 2H), 4.37-4.24 (m, 2H), 4.08-3.99 (m, 1H), 3.86-3.74 (m, 2H), 3.55-3.40 (m, 4H), 3.39-3.28 (m, 5H), 3.28-3.08 (m, 6H), 3.02-2.69 (m, 11H), 2.48-2.31 (m, 6H), 2.12-1.96 (m, 3H), 1.75-1.56 (m, 3H).

### Example 66 (Compound 152)

### Preparation of (S)-3-(3-(2-((3-((S)-2-carboxy-2-((R)-pyrrolidin-3-yl)ethyl)-5-fluorobenzyl)(2-(3-((S)-2-carboxy-2-((R)-pyrrolidin-3-yl)ethyl)-5-fluorophenoxy)ethyl)amino)-2-oxoethyl)-5-fluorophenyl)-2-((R)-pyrrolidin-3-yl)propanoic acid

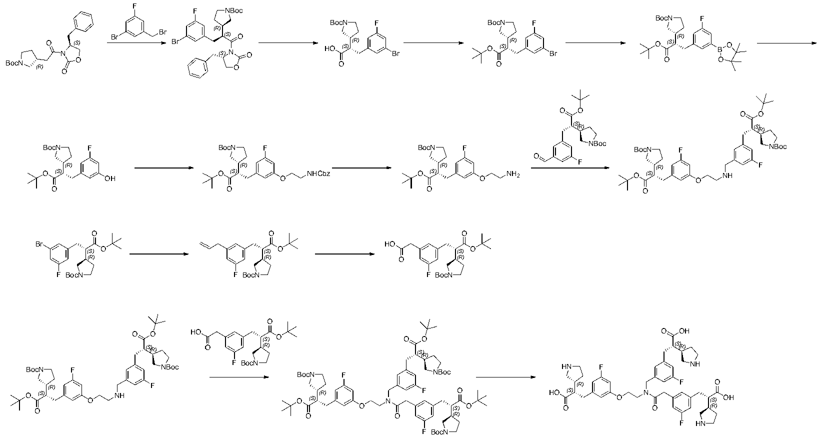

### Step 1: Synthesis of tert-butyl (R)-3-((S)-1-((S)-4-benzyl-2-oxooxazolidin-3-yl)-3-(3-bromo-5-fluorophenyl)-1-oxopropan-2-yl)pyrrolidine-1-carboxylate

Under nitrogen protection, a solution of lithium bis(trimethylsilyl)amide (30.89mL, 1.0 M in THF) was added dropwise to a solution of *tert-*butyl (R)-3-(2-((S)-4-benzyl-2-oxooxazolidin-3-yl)-2-oxoethyl)pyrrolidine-1-carboxylate(10g) in tetrahydrofuran. The reaction mixture was stirred at 0°C for 30min, then a solution of 1-bromo-3-(bromomethyl)-5-fluorobenzene (7.59g) in tetrahydrofuran was added, and the reaction temperature was slowly raised to room temperature and stirred for 16h. LCMS showed the reaction was completed. Saturated ammonium chloride solution was added to the mixture, which was extracted with ethyl acetate. The organic phase was washed with saturated NaCl solution, dried over anhydrous sodium sulfate, filtered and concentrated to give the target compound (15g). LCMS(ESI)[M+H-Boc]⁺=475.22.

### Step 2: Synthesis of (S)-3-(3-bromo-5-fluorophenyl)-2-((R)-1-(tert-butoxycarbonyl)pyrrolidin-3-yl)propanoic acid

The crude product from Step 1 (15g) was dissolved in tetrahydrofuran (300mL), and lithium hydroxide (0.94g) and hydrogen peroxide (1.33g) were added. The reaction mixture was stirred at room temperature for 16h. LCMS showed the consumption of starting material was completed. The solvent was removed by rotary-evaporation to dryness, water and ethyl acetate were added, and the mixture was extracted. The organic phase was dried over anhydrous sodium sulfate, filtered, and concentrated to give a crude product, which was purified by column chromatography (petroleum ether:ethyl acetate=2:1) to give the product (9g). LCMS(ESI)[M+H-Boc]⁺=316.10. Step 3: Synthesis of *tert*-butyl (R)-3-((S)-3-(3-bromo-5-fluorophenyl)-1-(tert-butoxy)-1-oxopropan-2-yl)pyrrolidine-1-carboxylate

Under nitrogen protection, the product from step 2 (17.8g) was dissolved in 2-methyltetrahydrofuran (150mL), and *tert*-butyl N,N'-diisopropylcarbamimidate (44g) was added. The reaction mixture was heated to 65°C and stirred for 16h. LCMS showed the reaction was completed. The insoluble materials were filtered off, and the filter cake was washed with methyl *tert*-butyl ether. The filtrate was concentrated, and separated and purified by rapid column chromatography (Silica gel, petroleum ether:ethyl acetate=10:1) to give the product (11g). LCMS(ESI)[M+H-2 *tert*-butyl]⁺=360.13.

### Step 4: Synthesis of tert-butyl (R)-3-((S)-1-(tert-butoxy)-3-(3-fluoro-5-(4,4,5,5-tetramethyl-1,3,2-dioxaborolan-2-yl)phenyl)-1-oxopropan-2-yl)pyrrolidine-1-carboxylate

The product from step 3 (8g), bis(pinacolato)diboron (6.48g), [1,1'-bis(diphenylphosphino)ferrocene]palladium(II) dichloride (1.28g) and potassium acetate (3.36g) were added to 1,4-dioxane (40mL) and H₂O (10mL). The mixture was stirred at 90°C under nitrogen for 4h. LCMS showed the reaction was completed. After cooling to room temperature, water and ethyl acetate were added, and the mixture was extracted. The organic phase was concentrated to give the crude product (8g), which was used directly in the next step. LCMS(ESI)[M+H- Boc]⁺=420.43.

### Step 5: Synthesis of tert-butyl (R)-3-((S)-1-(tert-butoxy)-3-(3-fluoro-5-hydroxyphenyl)-1-oxopropan-2-yl)pyrrolidine-1-carboxylate

The product from step 4 (8g) was dissolved in tetrahydrofuran (20mL), and hydrogen peroxide (2.62g) was added. The mixture was stirred at room temperature for 16h. LCMS showed the reaction was completed. An appropriate amount of saturated sodium thiosulfate solution was added, and the mixture was extracted with ethyl acetate. The solvent was removed under reduced pressure, and the residue was purified by column chromatography (silica gel, petroleum ether:ethyl acetate=10:1) to give the product (6.1g). LCMS(ESI)[M+H-2×tBu]⁺=298.18.

### Step 6: Synthesis of tert-butyl (R)-3-((S)-3-(3-(2-(((benzyloxy)carbonyl)amino)ethoxy)-5-fluorophenyl)-1-(tert-butoxy)-1-oxopropan-2-yl)pyrrolidine-1-carboxylate

The product from step 5 (6.1g) was dissolved in N,N-dimethylformamide (50mL), followed by adding potassium carbonate (6.1g) and benzyl (2-bromoethyl)carbamate (7.6g). The reaction mixture was stirred at 90°C overnight. Upon completion of the reaction as monitored by LCMS, the mixture was cooled to room temperature. Ethyl acetate and water were added, and the mixture was extracted. The organic phase was rotary-evaporated under reduced pressure, and the residue was purified by column chromatography (petroleum ether:ethyl acetate=4: 1) to give the target compound (6.8g). LCMS(ESI)[M+H-Boc]⁺=487.37.

### Step 7: Synthesis of tert-butyl (R)-3-((S)-3-(3-(2-aminoethoxy)-5-fluorophenyl)-1-(tert-butoxy)-1-oxopropan-2-yl)pyrrolidine-1-carboxylate

The product from step 6 (6.8g) was dissolved in tetrahydrofuran (100mL), and Pd/C (340mg, 10%) was added. The mixture was stirred at room temperature under a hydrogen atmosphere for 16h. LCMS showed the reaction was completed. After filtration, the filtrate was rotary-evaporated to dryness to give a crude product (4.25g), which was used directly in the next step. LCMS(ESI)[M+H]⁺=453.41.

### Step 8: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)-5-fluorophenoxy)ethyl)amino)methyl)-5-fluorophenyl)-1-oxopropan-2-yl)pyrrolidine-1-carboxylate

The product from step 7 (2.2g) was dissolved in tetrahydrofuran (25mL), and *tert*-butyl (R)-3-((S)-1-(tert-butoxy)-3-(3-fluoro-5-formylphenyl)-1-oxopropan-2-yl)pyrrolidine-1-carboxylate(2.05g) was added. The resulting mixture was stirred at room temperature for 1h. Then, sodium cyanoborohydride (0.6g) was added and the mixture was stirred at room temperature for 36hours. LCMS showed the reaction was completed. The reaction was quenched with saturated ammonium chloride solution, and the mixture was extracted with ethyl acetate. The organic phase was dried over anhydrous sodium sulfate, filtered, and concentrated to give a crude product, which was then purified by rapid chromatography (silica gel, petroleum ether:ethyl acetate=1:5) to give the target compound (1.2g). LCMS(ESI)[M+H]⁺=858.78.

### Step 9: Synthesis of tert-butyl (R)-3-((S)-3-(3-allyl-5-fluorophenyl)-1-(tert-butoxy)-1-oxopropan-2-yl)pyrrolidine-1-carboxylate

Under nitrogen protection, *tert-*butyl (R)-3-((S)-3-(3-bromo-5-fluorophenyl)-1-(tert-butoxy)-1-oxopropan-2-yl)pyrrolidine-1-carboxylate(3g), [1,1'-bis(diphenylphosphino)ferrocene]palladium(II) dichloride (0.47g) and potassium phosphate (4.04g) were dissolved in 1,4-dioxane (7mL) and water (30mL), and 2-allyl-4,4,5,5-tetramethyl-1,3,2-dioxaborolane (2.13g) was added. The reaction mixture was stirred at 90°C overnight, and LCMS showed the reaction was completed. After cooling to room temperature, ethyl acetate and water were added, and the mixture was extracted. The organic phase was dried over anhydrous sodium sulfate, filtered, and concentrated to give a crude product, which was purified by column chromatography (silica gel, petroleum ether:ethyl acetate=5:1) to give the target compound (1.5g). LCMS(ESI)[M+H-2×t-butyl]⁺=322.22.

### Step 10: Synthesis of 2-(3-((S)-3-(tert-butoxy)-2-((R)-1-(tert-butoxycarbonyl)pyrrolidin-3-yl)-3-oxopropyl)-5-fluorophenyl)acetic acid

The product from step 9 (1.5g) was dissolved in acetonitrile (15mL) and water (15mL), followed by adding sodium iopanoate (2.96g) and ruthenium trichloride (60mg); the reaction mixture was stirred at room temperature for 16h, and LCMS showed the reaction was completed. Ethyl acetate and water were added to the reaction mixture for extraction, and the organic phase was dried over anhydrous sodium sulfate, filtered, and concentrated to give a crude product, which was purified by column chromatography (silica gel, petroleum ether:ethyl acetate=5:1) to give the product (800mg). LCMS(ESI)[M+H-2×*tert*-butyl]⁺=340.18.

### Step 11: Synthesis of tert-butyl (R)-3-((S)-1-(tert-butoxy)-3-(3-((N-(2-(3-((S)-3-(tert-butoxy)-2-((R)-1-(tert-butoxycarbonyl)pyrrolidin-3-yl)-3-oxopropyl)-5-fluorophenoxy)ethyl)-2-(3-((S)-3-(tert-butoxy)-2-((R)-1-(tert-butoxycarbonyl)pyrrolidin-3-yl)-3-oxopropyl)-5-fluorophenyl)acetamido)methyl)-5-fluorophenyl)-1-oxopropan-2-yl)pyrrolidine-1-carboxylate

The product from step 8 (700mg) was dissolved in N,N-dimethylformamide (3mL), followed by adding the product from step 10 (368mg), O-(7-azabenzotriazol-1-yl)-N,N,N',N'-tetramethyluronium hexafluorophosphate (465mg) and *N,N*-diisopropylethylamine (316mg). The reaction mixture was stirred at room temperature for 16h. LCMS showed the reaction was completed. Ethyl acetate and water were added, and the mixture was extracted. The organic phase was dried over anhydrous sodium sulfate, filtered, and concentrated to give a crude product, which was purified by rapid chromatography (petroleum ether:ethyl acetate=1:8) to give the target compound (800mg).

Step 12: The product from the Step 11 (1.3g) was dissolved in 1,4-dioxane (10mL), hydrogen chloride/1,4-dioxane (10mL, 4.0M) was added, and the mixture was stirred at room temperature for 16h. LCMS showed the reaction was completed. The solvent was removed by rotary-evaporation under reduced pressure, and the residue was purified by Prep-HPLC (C₁₈, 10mmol/L NH₄HCO₃ in water, MeCN) to give the product (240mg). LCMS(ESI)[M+H]⁺=823.62; ¹HNMR(400MHz, D₂O) δ 6.97-6.60 (m, 6H), 6.59-6.36 (m, 2H), 6.35-6.25 (m, 1H), 4.65-4.48 (m, 2H), 4.10-3.64 (m, 6H), 3.46-3.26 (m, 6H), 3.21-3.08 (m, 3H), 2.89-2.21 (m, 15H), 2.10-1.94 (m, 3H), 1.74-1.55 (m, 3H).

### Example 67 (Compound 153)

### Preparation of (S)-3-(5-(2-(2-(5-((S)-2-carboxy-2-((R)-pyrrolidin-3-yl)ethyl)pyridin-3-yl)-N-((5-((S)-2-carboxy-2-((R)-pyrrolidin-3-yl)ethyl)pyridin-3-yl)methyl)acetamido)ethoxy)pyridin-3-yl)-2-((R)-pyrrolidin-3-yl)propanoic acid

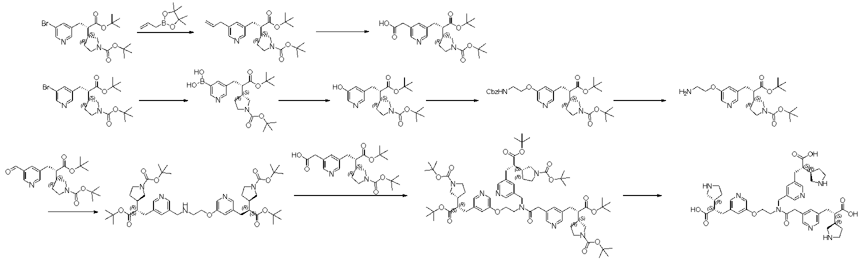

### Step 1: Synthesis of tert-butyl (R)-3-((S)-3-(5-allylpyridin-3-yl)-1-(tert-butoxy)-1-oxopropan-2-yl)pyrrolidine-1-carboxylate

Potassium phosphate (1006.84mg) and [1,1'-bis(diphenylphosphino)ferrocene]palladium(II) dichloride (154.57mg) were added to *tert-*butyl (R)-3-((S)-3-(5-bromopyridin-3-yl)-1-(tert-butoxy)-1-oxopropan-2-yl)pyrrolidine-1-carboxylate(1.08g) and 2-allyl-4,4,5,5-tetramethyl-1,3,2-dioxaborolane (797.05mg) in a mixture of dioxane (15mL) and water (3mL) under nitrogen under nitrogen. The mixture was heated to 90°C and stirred for 2h. LCMS showed the reaction was completed. After cooled to room temperature, the mixture was dilute with water (30mL), and extract with ethyl acetate. The organic phase was washed with saturated brine, dried over anhydrous sodium sulfate, filtered and concentrated to give a crude product, which was separated and purified by column chromatography (silica gel, tetrahydrofuran:petroleum ether=1:3) to give the target compound (900mg). LCMS(ESI)[M+H]⁺=417.3.

### Step 2: 2-(5-((S)-3-(tert-butoxy)-2-((R)-1-(tert-butoxycarbonyl)pyrrolidin-3-yl)-3-oxopropyl)pyridin-3-yl)acetic acid

Under ice bath, sodium periodate (2.16g) and ruthenium(III) chloride hydrate (56.81mg) was added to a solution of the product from step 1 (1.05g) in acetonitrile (15mL) and water (15mL). The reaction mixture was stirred at 0°C for 2h. After completion of the reaction, the reaction mixture was diluted with diatomaceous earth, filtered through celite, and the filtrate was concentrated under reduced pressure. The residue was diluted with water, extracted with ethyl acetate, and the combined organic phases were washed with saturated brine, dried over anhydrous sodium sulfate, filtered, and concentrated. The residue was purified by preparative separation (C₁₈, 0.03% trifluoroacetic acid in water, acetonitrile) to give the target compound (140mg). LCMS(ESI)[M+H]⁺=435.2. ¹HNMR(400MHz, DMSO-*d₆*) δ 8.28 (d, *J*=11.4Hz, 2H), 7.51 (s, 1H), 3.59-3.52 (m, 3H), 3.18-3.10 (m, 2H), 3.02 (t, *J*=9.9Hz, 1H), 2.84-2.75 (m, 1H), 2.73-2.66 (m, 1H), 2.57-2.52 (m, 1H), 2.34-2.24 (m, 1H), 1.89-1.79 (m, 1H), 1.65-1.53 (m, 1H), 1.40 (s, 9H), 1.21 (s, 9H).

### Step 3: Synthesis of (5-((S)-3-(tert-butoxy)-2-((R)-1-(tert-butoxycarbonyl)pyrrolidin-3-yl)-3-oxopropyl)pyridin-3-yl)boronic acid

Under nitrogen atmosphere, bis(pinacolato)diboron (2.34g) and [1,1'-bis(diphenylphosphino)ferrocene]palladium(II) dichloride (902.27mg) were added to a solution of *tert-*butyl (R)-3-((S)-3-(5-bromopyridin-3-yl)-1-(tert-butoxy)-1-oxopropan-2-yl)pyrrolidine-1-carboxylate(2.8g) and potassium acetate (1.81g) in 1,4-dioxane (50mL). The reaction mixture was heated to 90°C and stirred for 16h. LCMS showed the reaction was completed. After the reaction mixture was cooled to room temperature, it was filtered through diatomaceous earth to remove the filter residue, and the filtrate was concentrated under reduced pressure to give the target compound (5g, crude product). This intermediate was used directly in the next step without further purification. LCMS(ESI)[M+H]⁺=421.2.

### Step 4: Synthesis of tert-butyl (R)-3-((S)-1-(tert-butoxy)-3-(5-hydroxypyridin-3-yl)-1-oxopropan-2-yl)pyrrolidine-1-carboxylate

Under ice bath, hydrogen peroxide (6.74g) was added slowly to a solution of the product from step 3 (5g) in methanol (40mL). The reaction mixture was warmed to room temperature and stirred for 16h. LCMS showed the reaction was completed. The reaction mixture was quenched with saturated aqueous sodium thiosulfate (8mL), concentrated under reduced pressure, and the residue was diluted with water and extracted with ethyl acetate. The combined organic phases were washed with saturated brine, dried over anhydrous sodium sulfate, filtered, and concentrated. The residue was purified by column chromatography (silica gel, tetrahydrofuran/petroleum ether=1:1) to give the target compound (1.6g). LCMS(ESI)[M+H]⁺=393.2.

### Step 5: Synthesis of tert-butyl (R)-3-((S)-3-(5-(2-(((benzyloxy)carbonyl)amino)ethoxy)pyridin-3-yl)-1-(tert-butoxy)-1-oxopropan-2-yl)pyrrolidine-1-carboxylate

Potassium carbonate (1.48g) was added to a solution of the product from step 4 (1.4g) and benzyl (2-bromoethyl)carbamate (1.84g) in *N,N-*dimethylformamide (15mL). The reaction mixture was heated to 80°C and stirred for 4h. LCMS showed the reaction was completed. The reaction solution is cooled to room temperature, diluted with water, extracted with ethyl acetate, the organic phases are combined, washed with saturated brine, dried over anhydrous sodium sulfate, filtered and concentrated, and the resulting residue is separated and purified by column chromatography (silica gel, tetrahydrofuran/petroleum ether=1:3) to obtain the target compound (1.3g). LCMS(ESI)[M+H]⁺=570.4.

### Step 6: Synthesis of (3R)-3-[(2S)-3-[5-(2-aminoethoxy)pyridine-3-yl]-1-(tert-butoxy)-1-oxopropan-2-yl]pyrrolidin-1-carboxylic acid tert-butyl ester:

Pd/C (650.82mg, 10% w/t) was added to a solution of the product from step 5 (1.3g) in tetrahydrofuran (20mL). The reaction mixture was purged with hydrogen and stirred at room temperature for 16h. LCMS showed the reaction was completed. The reaction solution was filtered through diatomaceous earth, and the filtrate was concentrated under reduced pressure. The residue was purified by reversed-phase chromatography (C₁₈, 0.1% ammonia water in water/acetonitrile) to give the target compound (590mg). LCMS(ESI)[M+H]⁺=436.3; ¹HNMR(400MHz, DMSO-*d₆*) δ 8.12 (d, *J*=2.7Hz, 1H), 7.98 (d, *J*=1.2Hz, 1H), 7.25 (s, 1H), 4.00-3.92 (m, 2H), 3.56-3.47 (m, 1H), 3.40-3.37 (m, 1H), 3.17-3.09 (m, 1H), 3.02 (t, *J*=10.0Hz, 1H), 2.87 (t, *J*=5.8Hz, 2H), 2.78-2.66 (m, 2H), 2.61-2.53 (m, 1H), 2.35-2.22 (m, 1H), 1.90-1.80 (m, 1H), 1.63-1.53 (m, 1H), 1.40 (s, 9H), 1.22 (s, 9H).

### Step 7: Synthesis of tert-butyl (R)-3-((S)-1-(tert-butoxy)-3-(5-(((2-((5-((S)-3-(tert-butoxy)-2-((R)-1-(tert-butoxycarbonyl)pyrrolidin-3-yl)-3-oxopropyl)pyridin-3-yl)oxy)ethyl)amino)methyl)pyridin-3-yl)-1-oxopropan-2-yl)pyrrolidine-1-carboxylate

Titanium tetraisopropanolate (421.59mg) was added to a solution of *tert-*butyl (R)-3-((S)-1-(tert-butoxy)-3-(5-formylpyridin-3-yl)-1-oxopropan-2-yl)pyrrolidine-1-carboxylate(300mg) and the product from step 6 (355.34mg) in ethanol (12mL). The reaction mixture was stirred at room temperature for 14hours, then sodium borohydride (84.17mg) was added to the reaction system, and the reaction mixture was stirred at room temperature for another 2 hours. The reaction mixture was quenched with saturated ammonium chloride, then filtered through diatomaceous earth, and the filtrate was dried over anhydrous sodium sulfate, filtered and concentrated. The residue was purified by column chromatography (silica gel, petroleum ether:tetrahydrofuran=1:1) to give the target compound (550mg). LCMS(ESI)[M+H]⁺=824.6.

### Step 8: Synthesis of tert-butyl (R)-3-((S)-1-(tert-butoxy)-3-(5-((2-(5-((S)-3-(tert-butoxy)-2-((R)-1-(tert-butoxycarbonyl)pyrrolidin-3-yl)-3-oxopropyl)pyridin-3-yl)-N-(2-((5-((S)-3-(tert-butoxy)-2-((R)-1-(tert-butoxycarbonyl)pyrrolidin-3-yl)-3-oxopropyl)pyridin-3-yl)oxy)ethyl)acetamido)methyl)pyridin-3-yl)-1-oxopropan-2-yl)pyrrolidine-1-carboxylate

*N,N*-diisopropylethylamine (13.38mg) and O-(7-azabenzotriazol-1-yl)-N,N,N',N'-tetramethyluronium hexafluorophosphate (26.25mg) were added to a solution of the product from step 7 (42.67mg) and 2-(5-((S)-3-(tert-butoxy)-2-((R)-1-(tert-butoxycarbonyl)pyrrolidin-3-yl)-3-oxopropyl)pyridin-3-yl)acetic acid (15mg) in *N,N*-dimethylformamide (2mL), respectively. The reaction mixture was stirred at room temperature for 16h. The reaction mixture was diluted with water, extracted with ethyl acetate, and the organic phase was washed with saturated brine, dried over anhydrous sodium sulfate, filtered, and concentrated. The crude product was purified by reversed-phase separation (C₁₈, 0.1% trifluoroacetic acid in water/acetonitrile) to give the target compound (20mg). LCMS(ESI)[M+H-Boc]⁺=1140.3.

Step 9: The product from step 8 (20mg) was dissolved in a solution of hydrogen chloride/1,4-dioxane (3mL, 4.0M), and the reaction mixture was stirred at room temperature for 6h. Upon completion of the reaction, the reaction mixture was concentrated under reduced pressure, and the pH of the resulting residue was adjusted to 7-8 with sodium hydroxide solution (1.0M). The mixture was separated and purified by reversed-phase preparation (C₁₈, 0.03% ammonium bicarbonate in water/acetonitrile) to give the target compound (2.86mg). LCMS(ESI)[M+H]⁺=772.5; ¹HNMR(400MHz, D₂O) δ 8.24 (d, *J*=9.8Hz, 1H), 8.16-8.06 (m, 2H), 8.01-7.80 (m, 2H), 7.70-7.26 (m, 3H), 7.09-6.86 (m, 1H), 4.63-4.52 (m, 4H), 4.22-4.15 (m, 1H), 4.05-3.98 (m, 3H), 3.84-3.80 (m, 1H), 3.53-3.39 (m, 3H), 3.37-3.30 (m, 3H), 3.22-3.12 (m, 3H), 2.96-2.81 (m, 3H), 2.75-2.66 (m, 3H), 2.63-2.49 (m, 2H), 2.42-2.29 (m, 6H), 2.09-1.98 (m, 3H), 1.74-1.60 (m, 3H).

### Example 68 (Compound 158)

### Preparation of (S)-3-(3-(2-((3-((S)-2-carboxy-2-((R)-pyrrolidin-3-yl)ethyl)benzyl)(2-((3-((S)-2-carboxy-2-((R)-pyrrolidin-3-yl)ethyl)phenyl)thio)ethyl)amino)-2-oxoethyl)phenyl)-2-((R)-pyrrolidin-3-yl)propanoic acid

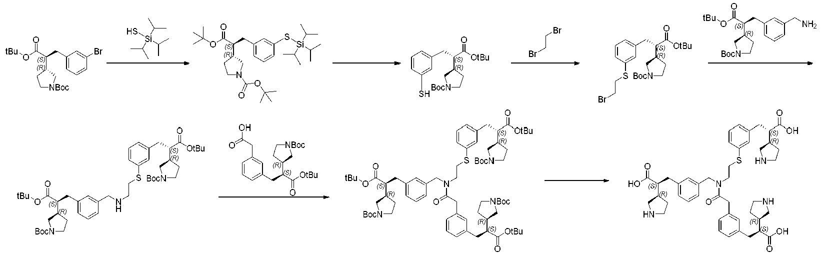

### Step 1: Synthesis of tert-butyl (R)-3-((S)-1-(tert-butoxy)-1-oxo-3-(3-((triisopropylsilyl)thio)phenyl)propan-2-yl)pyrrolidine-1-carboxylate

Under nitrogen protection, *tert-*butyl (R)-3-((S)-3-(3-bromophenyl)-1-(tert-butoxy)-1-oxopropan-2-yl)pyrrolidine-1-carboxylate(4g, 8.8mmol, 1eq) was dissolved in toluene (40mL), triisopropylsilanethiol (1.9mL, 8.8mmol, 1eq), cesium carbonate (5.74g, 17.61mmol, 2eq) and [1,1'-bis(diphenylphosphino)ferrocene]palladium(II) dichloride (0.65g, 0.88mmol, 0.1eq) were added; the reaction mixture was stirred at 120°C for 5hours. The reaction mixture was filtered through diatomaceous earth, the filtrate was concentrated, and separated and purified by rapid chromatography (silica gel, petroleum ether:ethyl acetate=15:1) to give the target compound (3.9g). LCMS(ESI)[M+H-Boc]⁺=464.4; ¹HNMR(400MHz, DMSO-*d₆*) δ 7.38-7.03 (m, 4H), 3.55-3.43 (m, 1H), 3.37-3.33 (m, 1H), 3.18-3.07 (m, 1H), 2.97 (t, *J*=10.0Hz, 1H), 2.80-2.64 (m, 2H), 2.49-2.45 (m, 1H), 2.31-2.19 (m, 1H), 1.87-1.79 (m, 1H), 1.61-1.52 (m, 1H), 1.39 (s, 9H), 1.24-1.18 (m, 9H), 1.01-0.96 (m, 21H).

### Step 2: Synthesis of tert-butyl (R)-3-((S)-1-(tert-butoxy)-3-(3-mercaptophenyl)-1-oxopropan-2-yl)pyrrolidine-1-carboxylate

Under nitrogen protection, the product from step 1 (3.9g, 6.92mmol, 1eq) was dissolved in dioxane (20mL), followed by adding hydrochloric acid (40mL, 1.0M). The reaction mixture was stirred at 30°C for hours. The reaction mixture was quenched with water and extracted with ethyl acetate. The organic phases were combined and dried over anhydrous sodium sulfate, and filtered, and the filtrate was concentrated. The residue was separated and purified by rapid chromatography (silica gel, ethyl acetate:petroleum ether=1:3) to give the target compound (420mg). LCMS(ESI)[M+Na]⁺=430.3.

### Step 3: Synthesis of tert-butyl (R)-3-((S)-3-(3-((2-bromoethyl)thio)phenyl)-1-(tert-butoxy)-1-oxopropan-2-yl)pyrrolidine-1-carboxylate

Under nitrogen protection, the product from step 2 (420mg, 1.03mmol, 1eq) was dissolved in acetonitrile (4mL), 1,2-dibromoethane (967.95mg, 5.15mmol, 5eq) and potassium carbonate (427.28mg, 3.09mmol, 3eq) were added, and the reaction mixture was heated to 70°C and stirred for 16h. Water was added to the reaction mixture, and the mixture was extracted with ethyl acetate. The organic phase was dried over anhydrous sodium sulfate, filtered, and concentrated to give a crude product, which was purified by column chromatography (silica gel, petroleum ether:ethyl acetate=3:1) to give the target compound (300mg). LCMS(ESI)[M+H]⁺=514.2; ¹HNMR(400MHz, DMSO-*d₆*) δ 7.28-7.21 (m, 3H), 7.09-7.04 (m, 1H), 3.60-3.47 (m, 3H), 3.41-3.33 (m, 4H), 3.20-3.09 (m, 1H), 2.99 (t, *J*=10.0Hz, 1H), 2.76-2.64 (m, 2H), 2.32-2.20 (m, 1H), 1.88-1.77 (m, 1H), 1.63-1.53 (m, 1H), 1.40 (s, 9H), 1.22 (s, 9H).

### Step 4: Synthesis of tert-butyl (R)-3-((S)-1-(tert-butoxy)-3-(3-(((2-((3-((S)-3-(tert-butoxy)-2-((R)-1-(tert-butoxycarbonyl)pyrrolidin-3-yl)-3-oxopropyl)phenyl)thio)ethyl)amino)methyl)phenyl)-1-oxopropan-2-yl)pyrrolidine-1-carboxylate

Under nitrogen protection, the product from step 3 (300mg, 0.58mmol, 1eq) was dissolved in acetonitrile (3mL), and *tert-*butyl (R)-3-((S)-3-(3-(aminomethyl)phenyl)-1-(tert-butoxy)-1-oxopropan-2-yl)pyrrolidine-1-carboxylate (235.87mg, 0.58mmol, 1eq) and potassium carbonate (241.76mg, 1.75mmol, 3eq) were added. The reaction mixture was heated to 90°C and stirred for 18hours. After the reaction was completed, the reaction solution was filtered through diatomaceous earth and concentrated to give the crude product, which was purified by column chromatography (silica gel: petroleum ether:ethyl acetate=1:1) to give the target compound (80mg). LCMS(ESI)[M+H]⁺=838.8; ¹HNMR(400MHz, DMSO-*d₆*) δ 7.23-7.10 (m, 6H), 7.05-6.96 (m, 2H), 3.69-3.58 (m, 2H), 3.58-3.40 (m, 3H), 3.40-3.34 (m, 4H), 3.21-3.09 (m, 2H), 3.07-2.87 (m, 4H), 2.75-2.65 (m, 6H), 2.30-2.21 (m, 2H), 1.88-1.78 (m, 2H), 1.62-1.52 (m, 2H), 1.39 (s, 18H), 1.22 (s, 18H).

### Step 5: Synthesis of tert-butyl (R)-3-((S)-1-(tert-butoxy)-3-(3-((2-(3-((S)-3-(tert-butoxy)-2-((R)-1-(tert-butoxycarbonyl)pyrrolidin-3-yl)-3-oxopropyl)phenyl)-N-(2-((3-((S)-3-(tert-butoxy)-2-((R)-1-(tert-butoxycarbonyl)pyrrolidin-3-yl)-3-oxopropyl)phenyl)thio)ethyl)acetamido)methyl)phenyl)-1-oxopropan-2-yl)pyrrolidine-1-carboxylate

The product from step 4 (140mg, 0.17mmol, 1eq) was dissolved in N,N-dimethylformamide (2mL), 2-(3-((S)-3-(tert-butoxy)-2-((R)-1-(tert-butoxycarbonyl)pyrrolidin-3-yl)-3-oxopropyl)phenyl)acetic acid(79.66mg, 0.18mmol, 1.1eq), O-(7-azabenzotriazol-1-yl)-N,N,N',N'-tetramethyluronium hexafluorophosphate (127.02mg, 0.33mmol, 2eq) and *N,N-*diisopropylethylamine (64.76mg, 0.5mmol, 3eq) were added and the reaction solution was stirred at room temperature. After the reaction was completed, the reaction mixture was added with water and extracted with ethyl acetate. The organic phases were combined, washed with saturated saline solution, dried over anhydrous sodium sulfate, filtered and concentrated, and then it was purified by column chromatography (silica gel, petroleum ether:ethyl acetate=3:1) to give the target compound (40mg). LCMS(ESI)[M+H-Boc ]⁺=1153.7.

Step 6: The product from step 5 (35mg, 0.03mmol, 1eq) was dissolved in 1,4-dioxane (1mL), and hydrogen chloride/1,4-dioxane (1mL, 4.0M) was added. The reaction mixture was stirred at room temperature for 3h. After the reaction was completed, the solvent was removed by rotary-evaporation to dryness, and the pH was adjusted to 7 with sodium hydroxide (1.0M). The residue was separated and purified by prep-HPLC (C-18, 0.1%NH₄HCO₃ in H₂O/ACN) to give the target compound (6.48mg). LCMS(ESI)[M+H]⁺=785.5; ¹HNMR(400MHz, D₂O) δ 7.30-7.00 (m, 8H), 7.00-6.71 (m, 4H), 4.48-4.38 (m, 2H), 3.65-3.57 (m, 2H), 3.51-3.41 (m, 2H), 3.36-3.25 (m, 6H), 3.16-2.99 (m, 5H), 2.84-2.55 (m, 9H), 2.42-2.26 (m, 6H), 2.09-1.94 (m, 3H), 1.72-1.54 (m, 3H).

### Example 69 (Compound 159)

### Preparation of (S)-3-(3-(2-((3-((S)-2-carboxy-2-((R)-pyrrolidin-3-yl)ethyl)benzyl)(3-(3-((S)-2-carboxy-2-((R)-pyrrolidin-3-yl)ethyl)phenoxy)propyl)amino)-2-oxoethyl)phenyl)-2-((R)-pyrrolidin-3-yl)propanoic acid

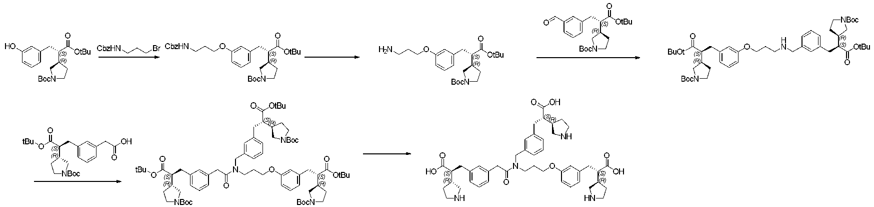

### Step 1: Synthesis of tert-butyl (R)-3-((S)-3-(3-(3-(((benzyloxy)carbonyl)amino)propoxy)phenyl)-1-(tert-butoxy)-1-oxopropan-2-yl)pyrrolidine-1-carboxylate

Under nitrogen protection, *tert-*butyl (R)-3-((S)-1-(tert-butoxy)-3-(3-hydroxyphenyl)-1-oxopropan-2-yl)pyrrolidine-1-carboxylate(500mg, 1.28mmol, 1eq) was dissolved in N,N-dimethylformamide (5mL), benzyl (3-bromopropyl)carbamate (695.12mg, 2.55mmol, 2eq) and potassium carbonate (529.54mg, 3.83mmol, 3eq) were added, and the reaction was stirred at 80°C. After the reaction was completed, the reaction mixture was added with water and extracted with ethyl acetate, the organic phases were combined, washed with saturated brine, dried over sodium sulfate, filtered and concentrated. Then, it was purified by column chromatography (silica gel, petroleum ether:ethyl acetate=5:1) to give the target compound (690mg).LCMS(ESI)[M+H]⁺=583.5; ¹HNMR(400MHz, DMSO-*d₆*) δ 7.39-7.28 (m, 6H), 7.15 (t, *J*=8.1Hz, 1H), 6.77-6.71 (m, 3H), 5.01 (s, 2H), 3.94 (t, *J*=6.3Hz, 2H), 3.54-3.44 (m, 1H), 3.37-3.32 (m, 2H), 3.19-3.09 (m, 3H), 2.97 (t, *J*=10.0Hz, 1H), 2.74-2.65 (m, 2H), 2.32-2.13 (m, 1H), 1.86-1.80 (m, 3H), 1.62-1.53 (m, 1H), 1.39 (s, 9H), 1.23 (s, 9H).

### Step 2: Synthesis of tert-butyl (R)-3-((S)-3-(3-(3-aminopropoxy)phenyl)-1-(tert-butoxy)-1-oxopropan-2-yl)pyrrolidine-1-carboxylate

The product from step 1 (690mg, 1.18mmol, 1eq) was dissolved in tetrahydrofuran (20mL), and Pd/C (126.01mg, 1.18mmol, 1eq) was added. The reaction was stirred at room temperature for 18hours under hydrogen atmosphere. After the reaction was completed, the mixture was filtered and concentrated to give the target compound (450mg). LCMS(ESI)[M+H]⁺=449.3; ¹HNMR(400MHz, DMSO-*d₆*) δ 7.18-7.11 (m, 1H), 6.76-6.70 (m, 3H), 4.02-3.91 (m, 2H), 3.56-3.41 (m, 2H), 3.19-3.07 (m, 2H), 2.97 (t, *J*=10.0Hz, 1H), 2.72-2.64 (m, 3H), 2.64-2.56 (m, 1H), 2.30-2.21 (m, 1H), 1.85-1.73 (m, 3H), 1.63-1.54 (m, 1H), 1.47-1.40 (m, 2H), 1.39 (s, 9H), 1.24 (s, 9H).

### Step 3: Synthesis of tert-butyl (R)-3-((S)-1-(tert-butoxy)-3-(3-(((3-(3-((S)-3-(tert-butoxy)-2-((R)-1-(tert-butoxycarbonyl)pyrrolidin-3-yl)-3-oxopropyl)phenoxy)propyl)amino)methyl)phenyl)-1-oxopropan-2-yl)pyrrolidine-1-carboxylate

*Tert-*butyl (R)-3-((S)-1-(tert-butoxy)-3-(3-formylphenyl)-1-oxopropan-2-yl)pyrrolidine-1-carboxylate(350mg, 0.87mmol, 1eq) was dissolved in ethanol (3mL), and titanium tetraisopropanolate (493.06mg, 1.73mmol, 2eq) and the product from step 2 (428.02mg, 0.95mmol, 1.1eq) were added. The reaction mixture was stirred at room temperature for 18hours. Sodium borohydride (49.22mg, 1.3mmol, 1.5eq) was added to the reaction mixture, and the mixture was stirred at room temperature for 1h. Saturated ammonium chloride aqueous solution (0.2mL) was added to the reaction mixture, and the mixture was filtered. The filtrate was concentrated and purified by column chromatography (silica gel, petroleum ether:tetrahydrofuran=1:1) to give the target compound (240mg). LCMS(ESI)[M+H]⁺=836.6; ¹HNMR(400MHz, DMSO-*d₆*) δ 7.20-7.12 (m, 4H), 7.03-6.99 (m, 1H), 6.75-6.69 (m, 3H), 3.98 (t, *J*=6.4Hz, 2H), 3.64 (s, 2H), 3.54-3.44 (m, 3H), 3.21-3.05 (m, 3H), 3.00-2.93 (m, 2H), 2.72-2.66 (m, 4H), 2.59 (t, *J*=6.6Hz, 2H), 2.47-2.42 (m, 1H), 2.30-2.21 (m, 2H), 1.89-1.78 (m, 5H), 1.61-1.53 (m, 2H), 1.39 (s, 18H), 1.22 (d, *J*=4.8Hz, 18H).

### Step 4: Synthesis of tert-butyl (R)-3-((S)-1-(tert-butoxy)-3-(3-((N-(3-(3-((S)-3-(tert-butoxy)-2-((R)-1-(tert-butoxycarbonyl)pyrrolidin-3-yl)-3-oxopropyl)phenoxy)propyl)-2-(3-((S)-3-(tert-butoxy)-2-((R)-1-(tert-butoxycarbonyl)pyrrolidin-3-yl)-3-oxopropyl)phenyl)acetamido)methyl)phenyl)-1-oxopropan-2-yl)pyrrolidine-1-carboxylate

The product from step 3 (230mg, 0.28mmol, 1eq) was dissolved in N,N-dimethylformamide (2mL), followed by adding 2-(3-((S)-3-(tert-butoxy)-2-((R)-1-(tert-butoxycarbonyl)pyrrolidin-3-yl)-3-oxopropyl)phenyl)acetic acid(133.53mg, 0.31mmol, 1.1eq), O-(7-azabenzotriazol-1-yl)-N,N,N',N'-tetramethyluronium hexafluorophosphate (209.19mg, 0.55mmol, 2eq) and *N,N-*diisopropylethylamine (108.56mg, 0.84mmol, 3eq); the reaction mixture was stirred at room temperature for 16h. After the reaction was completed, the reaction mixture was added with water and extracted with ethyl acetate, the organic phases were combined, washed with saturated brine, dried over anhydrous sodium sulfate, filtered and concentrated, and purified by column chromatography (silica gel, petroleum ether:ethyl acetate=3:1) to give the target compound (180mg). LCMS(ESI)[M+H-Boc]⁺=1151.7.

Step 5: The product from step 4 (170mg, 0.14mmol, 1eq) was dissolved in 1,4-dioxane (3mL), followed by adding hydrogen chloride/1,4-dioxane (3mL, 4.0M). The reaction mixture was stirred at room temperature for 3h. After the reaction was completed, the solvent was removed by rotary-evaporation to dryness, and the pH was adjusted to 7 with sodium hydroxide (1.0M). The residue was separated and purified by prep-HPLC (C-18, 0.1%NH₄HCO₃ in H₂O/ACN) to give the target compound (37.59mg). LCMS(ESI)[M+H]⁺=783.5; ¹HNMR(400MHz, D₂O) δ 7.24-7.14 (m, 3H), 7.10-7.03 (m, 2H), 6.99-6.79 (m, 5H), 6.74-6.63 (m, 2H), 4.57-4.48 (m, 2H), 3.95-3.85 (m, 2H), 3.76-3.65 (m, 2H), 3.52-3.39 (m, 2H), 3.37-3.22 (m, 6H), 3.18-3.07 (m, 3H), 2.83-2.54 (m, 9H), 2.42-2.25 (m, 6H), 2.06-1.82 (m, 5H), 1.71-1.51 (m, 3H).

### Example 70 (Compound 160)

### Preparation of (S)-3-(3-(2-((3-((S)-2-carboxy-2-((R)-pyrrolidin-3-yl)ethyl)benzyl)(2-((3-((S)-2-carboxy-2-((R)-pyrrolidin-3-yl)ethyl)benzyl)oxy)ethyl)amino)-2-oxoethyl)phenyl)-2-((R)-pyrrolidin-3-yl)propanoic acid

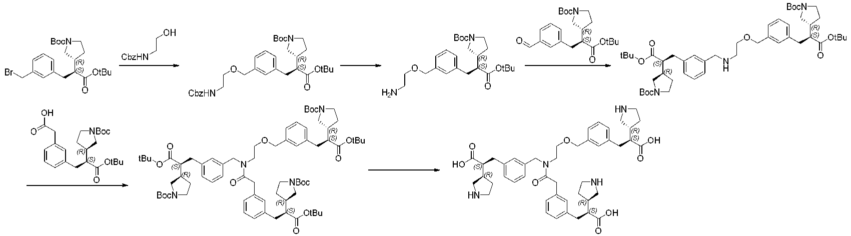

### Step 1: Synthesis of tert-butyl (R)-3-((S)-3-(3-((2-(((benzyloxy)carbonyl)amino)ethoxy)methyl)phenyl)-1-(tert-butoxy)-1-oxopropan-2-yl)pyrrolidine-1-carboxylate

*Tert-*butyl (R)-3-((S)-3-(3-(bromomethyl)phenyl)-1-(tert-butoxy)-1-oxopropan-2-yl)pyrrolidine-1-carboxylate (1g, 2.13mmol, 1eq) was dissolved in tetrahydrofuran (20mL), and benzyl (2-hydroxyethyl)carbamate (833.53mg, 4.27mmol, 2eq), benzyltriethylammonium chloride (48.63mg, 0.21mmol, 0.1eq) and sodium hydroxide aqueous solution (10mL, 50%) were added. The reaction solution was stirred for 18hours at room temperature. After the reaction was completed, the reaction mixture was added with water and extracted with ethyl acetate. The organic phase was dried over anhydrous sodium sulfate, filtered and concentrated, and it was purified by column chromatography (silica gel, petroleum ether:ethyl acetate=3:1) to give the target compound (720mg). LCMS(ESI)[M+H-Boc]⁺=483.4; ¹HNMR(400MHz, DMSO-*d₆*) δ 7.41-7.27 (m, 6H), 7.23 (t, *J*=7.7Hz, 1H), 7.16-7.12 (m, 2H), 7.09 (d, *J*=7.4Hz, 1H), 5.01 (s, 2H), 4.42 (s, 2H), 3.53-3.46 (m, 1H), 3.45-3.36 (m, 4H), 3.21-3.12 (m, 3H), 2.97 (t, *J*=9.9Hz*,* 1H), 2.79-2.68 (m, 2H), 2.31-2.19 (m, 1H), 1.87-1.78 (m, 1H), 1.64-1.53 (m, 1H), 1.39 (s, 9H), 1.21 (s, 9H).

### Step 2: Synthesis of tert-butyl (R)-3-((S)-3-(3-((2-aminoethoxy)methyl)phenyl)-1-(tert-butoxy)-1-oxopropan-2-yl)pyrrolidine-1-carboxylate

The product from step 1 (720mg, 1.24mmol, 1eq) was dissolved in tetrahydrofuran (2mL), and Pd/C (131.49mg, 1.24mmol, 1eq) was added. The reaction was carried out under hydrogen atmosphere at room temperature for 15 minutes. After the reaction was completed, the mixture was filtered and concentrated to give the target compound (550mg). LCMS(ESI)[M+H]⁺=449.7; ¹HNMR(400MHz, DMSO-*d₆*) δ 7.24 (t, *J*=7.8Hz, 1H), 7.18-7.13 (m, 2H), 7.09 (d, *J*=7.6Hz, 1H), 4.46-4.39 (m, 2H), 3.63-3.42 (m, 3H), 3.20-3.08 (m, 2H), 2.98 (t, *J*=9.8Hz, 1H), 2.77-2.63 (m, 4H), 2.30-2.23 (m, 1H), 1.88-1.71 (m, 2H), 1.63-1.54 (m, 1H), 1.39 (s, 9H), 1.22 (s, 9H).

### Step 3: Synthesis of tert-butyl (R)-3-((S)-1-(tert-butoxy)-3-(3-(((2-((3-((S)-3-(tert-butoxy)-2-((R)-1-(tert-butoxycarbonyl)pyrrolidin-3-yl)-3-oxopropyl)benzyl)oxy)ethyl)amino)methyl)phenyl)-1-oxopropan-2-yl)pyrrolidine-1-carboxylate

*Tert-*butyl (R)-3-((S)-1-(tert-butoxy)-3-(3-formylphenyl)-1-oxopropan-2-yl)pyrrolidine-1-carboxylate(370mg, 0.92mmol, 1eq) was dissolved in ethanol (5mL), and titanium tetraisopropanolate (521.23mg, 1.83mmol, 2eq) and the product from step 2 (452.48mg, 1.01mmol, 1.1eq) were added. The reaction mixture was stirred at room temperature for 18hours. Sodium borohydride (52.03mg, 1.38mmol, 1.5eq) was added to the reaction mixture, and the mixture was stirred at room temperature for 1h. After the reaction was completed, saturated ammonium chloride aqueous solution (0.2mL) was added to the reaction mixture, and the mixture was filtered. The filtrate was concentrated and purified by column chromatography (silica gel, petroleum ether:tetrahydrofuran=1:1) to give the target compound (580mg). LCMS(ESI)[M+H]⁺=836.7; ¹HNMR(400MHz, DMSO-*d₆*) δ 7.27-7.06 (m, 7H), 7.02 (d, *J*=7.3Hz, 1H), 4.41 (s, 2H), 3.65 (s, 2H), 3.62-3.58 (m, 1H), 3.56-3.43 (m, 4H), 3.38-3.33 (m, 2H), 3.20-3.09 (m, 2H), 3.01-2.91 (m, 2H), 2.77-2.62 (m, 6H), 2.31-2.19 (m, 2H), 1.85-1.74 (m, 3H), 1.62-1.52 (m, 2H), 1.39 (s, 18H), 1.21 (s, 18H).

### Step 4: Synthesis of tert-butyl (R)-3-((S)-1-(tert-butoxy)-3-(3-((2-(N-(3-((S)-3-(tert-butoxy)-2-((R)-1-(tert-butoxycarbonyl)pyrrolidin-3-yl)-3-oxopropyl)benzyl)-2-(3-((S)-3-(tert-butoxy)-2-((R)-1-(tert-butoxycarbonyl)pyrrolidin-3-yl)-3-oxopropyl)phenyl)acetamido)ethoxy)methyl)phenyl)-1-oxopropan-2-yl)pyrrolidine-1-carboxylate

The product from step 3 (300mg, 0.36mmol, 1eq) was dissolved in N,N-dimethylformamide (3mL), followed by adding 2-(3-((S)-3-(tert-butoxy)-2-((R)-1-(tert-butoxycarbonyl)pyrrolidin-3-yl)-3-oxopropyl)phenyl)acetic acid(171.11mg, 0.39mmol, 1.1eq), O-(7-azabenzotriazol-1-yl)-N,N,N',N'-tetramethyluronium hexafluorophosphate (272.86mg, 0.72mmol, 2eq) and *N,N-*diisopropylethylamine (139.12mg, 1.08mmol, 3eq). The reaction was allowed to proceed at room temperature for 3h. Upon completion, the reaction mixture was quenched with water and extracted with ethyl acetate. The organic phases were combined, washed with saturated brine, dried over anhydrous sodium sulfate, filtered, and concentrated. The residue was purified by column chromatography (silicon gel, petroleum ether:ethyl acetate=3:1) to give the target compound (310mg). LCMS(ESI)[M+Na]⁺=1183.7.

Step 5: The product from step 4 (310mg, 0.25mmol, 1eq) was dissolved in dioxane (3mL), followed by adding hydrogen chloride/1,4-dioxane (3mL, 4.0M). The reaction mixture was stirred at room temperature for 3h. Upon completion, the reaction mixture was concentrated and adjusted to pH=7 with sodium hydroxide (1.0M). The resulting mixture was separated and purified by prep-HPLC (C₁₈, 0.1%NH₄HCO₃ in H₂O/ACN) to give the target compound (63.6mg). LCMS(ESI)[M+H]⁺=783.5; ¹HNMR(400MHz, D₂O) δ 7.30-7.05 (m, 8H), 6.99-6.83 (m, 4H), 4.57-4.36 (m, 4H), 3.81 (s, 1H), 3.68 (s, 1H), 3.63-3.48 (m, 4H), 3.38-3.25 (m, 6H), 3.18-3.08 (m, 3H), 2.82-2.55 (m, 9H), 2.43-2.25 (m, 6H), 2.09-1.94 (m, 3H), 1.73-1.53 (m, 3H).

### Example 71 (Compound 161)

### Preparation of (S)-3-(3-(2-((2-((3-((S)-2-carboxy-2-((R)-pyrrolidin-3-yl)ethyl)benzyl)oxy)ethyl)(3-((S)-2-carboxy-2-((R)-pyrrolidin-3-yl)ethyl)phenethyl)amino)-2-oxoethyl)phenyl)-2-((R)-pyrrolidin-3-yl)propanoic acid

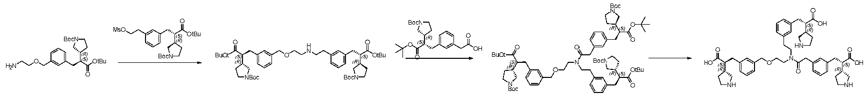

### Step 1: Synthesis of tert-butyl (R)-3-((S)-1-(tert-butoxy)-3-(3-((2-((3-((S)-3-(tert-butoxy)-2-((R)-1-(tert-butoxycarbonyl)pyrrolidin-3-yl)-3-oxopropyl)phenethyl)amino)ethoxy)methyl)phenyl)-1-oxopropan-2-yl)pyrrolidine-1-carboxylate

*Tert-*butyl (R)-3-((S)-3-(3-((2-aminoethoxy)methyl)phenyl)-1-(tert-butoxy)-1-oxopropan-2-yl)pyrrolidine-1-carboxylate(720mg, 1.6mmol, 1eq) and *tert-*butyl (R)-3-((S)-1-(tert-butoxy)-3-(3-(2-((methylsulfonyl)oxy)ethyl)phenyl)-1-oxopropan-2-yl)pyrrolidine-1-carboxylate(796.22mg, 1.6mmol, 1eq) were dissolved in acetonitrile (10mL), potassium carbonate (663.41mg, 4.8mmol, 3eq) was added, and the mixture was stirred at 80°C for 16h. LCMS showed the reaction was completed. The reaction mixture was filtered, the filtrate was concentrated, and the residue was purified by rapid chromatography (silica gel, petroleum ether:tetrahydrofuran=2:1) to give the target compound (680mg). LCMS(ESI)[M+H]⁺=850.9; ¹HNMR (400MHz, CDCl₃) δ 7.26-7.20 (m, 1H), 7.20-7.13 (m, 2H), 7.12-7.05 (m, 2H), 7.05-6.96 (m, 3H), 4.47 (s, 2H), 3.72-3.65 (m, 1H), 3.61-3.49 (m, 4H), 3.48-3.41 (m, 1H), 3.30-3.18 (m, 2H), 3.05-2.92 (m, 2H), 2.89-2.81 (m, 5H), 2.80-2.72 (m, 4H), 2.53-2.43 (m, 2H), 2.42-2.30 (m, 2H), 1.96-1.88 (m, 4H), 1.70-1.60 (m, 1H), 1.47 (s, 18H), 1.28 (s, 9H), 1.27 (s, 9H).

### Step 2: Synthesis of tert-butyl (R)-3-((S)-1-(tert-butoxy)-3-(3-((2-(N-(3-((S)-3-(tert-butoxy)-2-((R)-1-(tert-butoxycarbonyl)pyrrolidin-3-yl)-3-oxopropyl)phenethyl)-2-(3-((S)-3-(tert-butoxy)-2-((R)-1-(tert-butoxycarbonyl)pyrrolidin-3-yl)-3-oxopropyl)phenyl)acetamido)ethoxy)methyl)phenyl)-1-oxopropan-2-yl)pyrrolidine-1-carboxylate

2-(3-((S)-3-(tert-butoxy)-2-((R)-1-(tert-butoxycarbonyl)pyrrolidin-3-yl)-3-oxopropyl)phenyl)acetic acid(51mg, 0.12mmol, 1eq) was dissolved in N,N-dimethylformamide (3mL). Triethylamine (35.71mg, 0.35mmol, 3eq), O-(7-azabenzotriazol-1-yl)-N,N,N',N'-tetramethyluronium hexafluorophosphate (67.09mg, 0.18mmol, 1.5eq) and the product from step 1 (100mg, 0.12mmol, 1eq) were added. The reaction mixture was stirred at room temperature for 3h. LCMS showed the reaction was completed. The mixture was added with water and extracted with ethyl acetate. The organic phases were combined, washed with saturated NaCl, dried over anhydrous sodium sulfate, and filtered. The filtrate was concentrated and purified by rapid chromatography (silicon gel, petroleum ether:tetrahydrofuran=2:1) to give the target compound (130mg). LCMS(ESI)[M+H]⁺=1266.0; ¹HNMR (400MHz, CDCl₃) δ 7.26-7.15 (m, 3H), 7.14-6.97 (m, 8H), 6.95-6.86 (m, 1H), 4.44 (s, 2H), 3.79-3.72 (m, 2H), 3.71-3.64 (m, 2H), 3.62-3.36 (m, 11H), 3.31-3.21 (m, 3H), 3.04-2.91 (m, 3H), 2.90-2.80 (m, 2H), 2.79-2.69 (m, 5H), 2.52-2.42 (m, 3H), 2.41-2.30 (m, 3H), 1.98-1.89 (m, 3H), 1.64-1.57 (m, 2H), 1.47 (s, 27H), 1.26 (s, 27H).

Step 3: The product from step 2 (100mg, 0.08mmol, 1eq) was dissolved in 1,4-dioxane (2mL), hydrochloric acid/1,4-dioxane solution (2mL, 4.0M) was added, and the mixture was stirred at room temperature for 2h. LCMS showed the reaction was completed. The reaction mixture was concentrated, dissolved in water, adjusted to pH=7 with sodium hydroxide (1.0M) aqueous solution, and purified by reversed-phase preparation (C₁₈, 0.1% ammonium bicarbonate aqueous solution, acetonitrile) to give the target product (41.91mg). LCMS(ESI)[M+H]⁺=797.5; ¹HNMR (400MHz, D₂O) δ 7.27-7.17 (m, 2H), 7.16-7.05 (m, 5H), 7.05-7.00 (m, 1H), 7.00-6.78 (m, 3H), 6.75-6.70 (m, 1H), 4.41 (d, *J*=10.9Hz, 2H), 3.64 (s, 2H), 3.59-3.41 (m, 4H), 3.39-3.21 (m, 7H), 3.21-3.19 (m, 1H), 3.17-3.04 (m, 3H), 2.83-2.65 (m, 8H), 2.64-2.52 (m, 3H), 2.42-2.25 (m, 6H), 2.08-1.95 (m, 3H), 1.71-1.55 (m, 3H).

### Example 72 (Compound 162)

### Preparation of (S)-3-(3-(2-((3-((S)-2-carboxy-2-((R)-pyrrolidin-3-yl)ethyl)phenethyl)((3-((S)-2-carboxy-2-((R)-pyrrolidin-3-yl)ethyl)phenoxy)carbonyl)amino)ethoxy)phenyl)-2-((R)-pyrrolidin-3-yl)propanoic acid

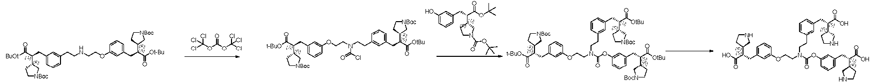

### Step 1: tert-butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)ethyl)phenyl)-1-oxopropan-2-yl)pyrrolidine-1-carboxylate

Under nitrogen protection, *tert*-butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)ethyl)phenyl)-1-oxopropan-2-yl)pyrrolidine-1-carboxylate(200mg, 0.24mmol, 1eq) was dissolved in tetrahydrofuran (5mL). At 0°C, triphosgene(70.98mg, 0.24mmol, 1eq) and triethylamine (242.05mg, 2.39mmol, 10eq) were added and stirred for 0.5h. LCMS showed the reaction was completed successfully. The reaction solution was directly used for the next step. LCMS(ESI)[M+H-Boc]⁺=798.6.

### Step 2: Synthesis of tert-butyl (R)-3-((S)-1-(tert-butoxy)-3-(3-(((3-((S)-3-(tert-butoxy)-2-((R)-1-(tert-butoxycarbonyl)pyrrolidin-3-yl)-3-oxopropyl)phenethyl)(2-(3-((S)-3-(tert-butoxy)-2-((R)-1-(tert-butoxycarbonyl)pyrrolidin-3-yl)-3-oxopropyl)phenoxy)ethyl)carbamoyl)oxy)phenyl)-1-oxopropan-2-yl)pyrrolidine-1-carboxylate

Under nitrogen protection, the product from step 1 (200mg, 0.22mmol, 1eq) was dissolved in tetrahydrofuran (3mL), and *tert-*butyl (R)-3-((S)-1-(tert-butoxy)-3-(3-hydroxyphenyl)-1-oxopropan-2-yl)pyrrolidine-1-carboxylate(87.14mg, 0.22mmol, 1eq) and N,N-dimethylpyridine-4-amine (2.27mg, 0.02mmol, 0.1eq) were added. The mixture was stirred at 50°C for 18hours. LCMS showed the reaction was completed. The reaction mixture was concentrated and separated and purified by rapid chromatography (silica gel, petroleum ether:tetrahydrofuran=5:1) to give the target compound (220mg). LCMS(ESI)[M+H-Boc]⁺=1153.7.

### Step 3: Synthesis of (S)-3-(3-(2-((3-((S)-2-carboxy-2-((R)-pyrrolidin-3-yl)ethyl)phenethyl)((3-((S)-2-carboxy-2-((R)-pyrrolidin-3-yl)ethyl)phenoxy)carbonyl)amino)ethoxy)phenyl)-2-((R)-pyrrolidin-3-yl)propanoic acid

Hydrochloric acid/1,4-dioxane (4mL, 4.0M) was added to a solution of the product from step 2 (300mg, 0.24mmol, 1eq) in 1,4-dioxane (4mL) and the mixture was stirred at room temperature for 2h. LCMS showed th reaction was completed. The reaction mixture was concentrated and neutralized with sodium hydroxide (1.0M) solution. The residue was separated and purified by Prep-HPLC (C₁₈, 0.1% NH₄HCO₃ in water, ACN) to give the target compound (86mg). LCMS(ESI)[M+H]=785.5; ¹HNMR(400MHz, D₂O) δ 7.27 (t, *J*=7.9Hz, 1H), 7.22-7.14 (m, 2H), 7.07 (d, *J*=7.6Hz, 1H), 6.83-6.72 (m, 8H), 4.26 (d, *J*=23.6Hz, 4H), 3.79 (d, *J*=50.9Hz, 4H), 3.39-3.25 (m, 6H), 3.19-3.08 (m, 3H), 2.86-2.67 (m, 7H), 2.64-2.54 (m, 2H), 2.36 (d, *J*=5.6Hz, 6H), 2.02 (m, 3H), 1.73-1.58 (m, 3H).

### Example 73 (Compound 163)

### Preparation of (2S,2'S)-3,3'-((((((3-((S)-2-carboxy-2-((R)-pyrrolidin-3-yl)ethyl)phenoxy)carbonyl)azanediyl)bis(ethane-2,1-diyl))bis(oxy))bis(3,1-phenylene))bis(2-((R)-pyrrolidin-3-yl)propanoic acid)

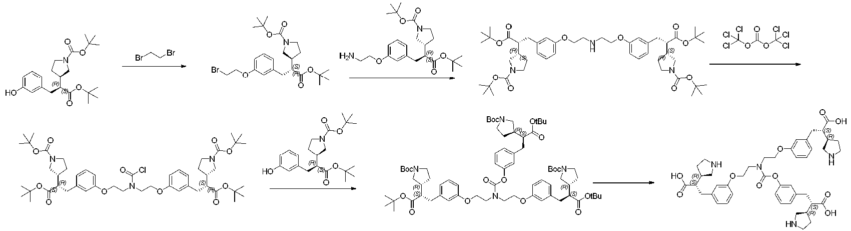

### Step 1: Synthesis of tert-butyl (S)-3-((R)-3-(3-(2-bromoethoxy)phenyl)-1-(tert-butoxy)-1-oxopropan-2-yl)pyrrolidine-1-carboxylate

Under nitrogen protection, *tert-*butyl (R)-3-((S)-1-(tert-butoxy)-3-(3-hydroxyphenyl)-1-oxopropan-2-yl)pyrrolidine-1-carboxylate(1g, 2.55mmol, 1eq) was dissolved in acetonitrile solution (10mL), 1,2-dibromoethane (2.4g, 12.77mmol, 5eq) and potassium carbonate (1.06g, 7.66mmol, 3eq) were added, and the reaction was also stirred at 70°C. LCMS showed the reaction was completed. The reaction mixture was added with water and extracted with ethyl acetate. The organic phases were combined, washed with saturated sodium chloride solution, dried, and concentrated. Then, it was separated and purified by rapid chromatography (silica gel, petroleum ether:tetrahydrofuran=5:1) to give the target compound (520mg). LCMS(ESI)[M+Na]⁺=520.2.

### Step 2: Synthesis of tert-butyl (R)-3-((S)-1-(tert-butoxy)-3-(3-(2-((2-(3-((R)-3-(tert-butoxy)-2-((S)-1-(tert-butoxycarbonyl)pyrrolidin-3-yl)-3-oxopropyl)phenoxy)ethyl)amino)ethoxy)phenyl)-1-oxopropan-2-yl)pyrrolidine-1-carboxylate

Under nitrogen protection, the product from step 1 (520mg, 1.04mmol, 1eq) was dissolved in acetonitrile (5mL), *tert-*butyl (R)-3-((S)-3-(3-(2-aminoethoxy)phenyl)-1-(tert-butoxy)-1-oxopropan-2-yl)pyrrolidine-1-carboxylate(453.36mg, 1.04mmol, 1eq) and potassium carbonate (432.56mg, 3.13mmol, 3eq) were added. The reaction mixture was stirred at 90°C. LCMS showed the reaction was completed. The reaction mixture was added with water and extracted with ethyl acetate. The organic phases were combined, washed with saturated sodium chloride solution, dried over anhydrous sodium sulfate, filtered, concentrated, and the residue was purified by rapid chromatography (silica gel, ethyl acetate:petroleum ether=2:1) to give the target compound (300mg).LCMS(ESI)[M+H]⁺=853.1; ¹HNMR(400MHz, DMSO-*d₆*) δ 11.70-11.68 (m, 2H), 7.18-7.13 (m, 2H), 6.77-6.75 (m, 2H), 6.74-6.72 (m, 2H), 5.37 (d, *J*=3.9Hz, 1H), 4.00 (t, *J*=5.3Hz, 4H), 3.82-3.76 (m, 4H), 3.49 (d, *J*=11.1Hz, 2H), 3.17-3.11 (m, 2H), 2.97-2.91 (m, 4H), 2.71-2.66 (m, 4H), 2.26 (s, 2H), 1.97-1.92 (m, 2H), 1.85-1.80 (m, 4H), 1.39 (s, 18H), 1.23 (s, 18H).

### Step 3: Synthesis of tert-butyl (R)-3-((S)-1-(tert-butoxy)-3-(3-(2-((2-(3-((R)-3-(tert-butoxy)-2-((S)-1-(tert-butoxycarbonyl)pyrrolidin-3-yl)-3-oxopropyl)phenoxy)ethyl)(chlorocarbonyl)amino)ethoxy)phenyl)-1-oxopropan-2-yl)pyrrolidine-1-carboxylate

Under nitrogen protection, the product from step 2 (200mg, 0.24mmol, 1eq) was dissolved in tetrahydrofuran (5mL). Triphosgene(70.98mg, 0.24mmol, 1eq) and triethylamine (242.05mg, 2.39mmol, 10eq) were added at 0°C, and the mixture was stirred at 0°C for 0.5h. LCMS showed the reaction was completed, and the white turbid reaction mixture was directly used for the next step. LCMS(ESI)[M+H]⁺=914.3.

### Step 4: Synthesis of di-tert-butyl 3,3'-((2S,2'S)-((((((3-((S)-3-(tert-butoxy)-2-((R)-1-(tert-butoxycarbonyl)pyrrolidin-3-yl)-3-oxopropyl)phenoxy)carbonyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)

Under nitrogen protection, *tert*-butyl (R)-3-((S)-1-(tert-butoxy)-3-(3-hydroxyphenyl)-1-oxopropan-2-yl)pyrrolidine-1-carboxylate(87.14mg, 0.22mmol, 1eq) and N,N-dimethylpyridine-4-amine (2.27mg, 0.02mmol, 0.1eq) were added to a solution of the product from step 3 (200mg, 0.22mmol, 1eq) in tetrahydrofuran (4mL). The mixture was stirred at 50°C for 18hours. LCMS showed the reaction was completed. The reaction mixture was concentrated and separated and purified by rapid chromatography (silica gel, petroleum ether:tetrahydrofuran=1:4) to give the target compound (100mg). LCMS(ESI)[M+H-Boc]⁺=1169.7.

### Step 5: Synthesis of (2S,2'S)-3,3'-((((((3-((S)-2-carboxy-2-((R)-pyrrolidin-3-yl)ethyl)phenoxy)carbonyl)azanediyl)bis(ethane-2,1-diyl))bis(oxy))bis(3,1-phenylene))bis(2-((R)-pyrrolidin-3-yl)propanoic acid)

Hydrochloric acid/dioxane (4mL, 4.0M) was added to a solution of the product from step 4 (300mg, 0.24mmol, 1eq) in 1,4-dioxane (4mL) and the mixture was stirred at room temperature for 2h. The reaction mixture was concentrated and neutralized with sodium hydroxide solution (1.0M). Then, it was separated and purified by Prep-HPLC (C₁₈, 0.1% NH₄HCO₃ in water, ACN) to give the target compound (13mg). LCMS(ESI)[M+H]⁺=801.5; ¹HNMR(400MHz, D₂O) δ 7.27 (t, *J*=7.9Hz, 1H), 7.22-7.14 (m, 2H), 7.07 (d, *J*=7.6Hz, 1H), 6.83-6.72 (m, 8H), 4.26 (d, *J*=23.6Hz, 4H), 3.79 (d, *J*=50.9Hz, 4H), 3.39-3.25 (m, 6H), 3.19-3.08 (m, 3H), 2.86-2.67 (m, 7H), 2.64-2.54 (m, 2H), 2.36 (d, *J*=5.6Hz, 6H), 2.02 (m, 3H), 1.73-1.58 (m, 3H).

### Example 74 (Compound 164)

### Preparation of (S)-3-(3-(2-((((3-((S)-2-carboxy-2-((R)-pyrrolidin-3-yl)ethyl)benzyl)oxy)carbonyl)(2-((3-((S)-2-carboxy-2-((R)-pyrrolidin-3-yl)ethyl)benzyl)oxy)ethyl)amino)ethyl)phenyl)-2-((R)-pyrrolidin-3-yl)propanoic acid

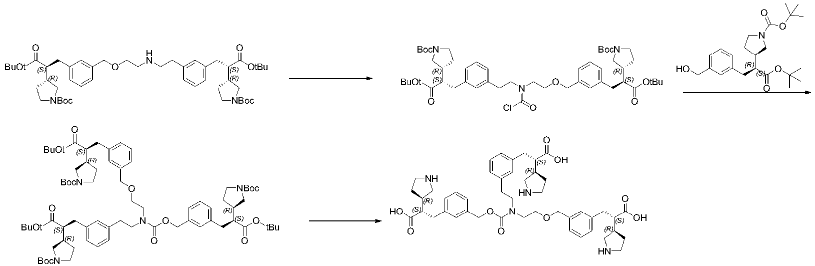

### Step 1: Synthesis of tert-butyl (R)-3-((S)-1-(tert-butoxy)-3-(3-((2-((3-((S)-3-(tert-butoxy)-2-((R)-1-(tert-butoxycarbonyl)pyrrolidin-3-yl)-3-oxopropyl)phenethyl)(chlorocarbonyl)amino)ethoxy)methyl)phenyl)-1-oxopropan-2-yl)pyrrolidine-1-carboxylate

*Tert*-butyl (R)-3-((S)-1-(tert-butoxy)-3-(3-((2-((3-((S)-3-(tert-butoxy)-2-((R)-1-(tert-butoxycarbonyl)pyrrolidin-3-yl)-3-oxopropyl)phenethyl)amino)ethoxy)methyl)phenyl)-1-oxopropan-2-yl)pyrrolidine-1-carboxylate(200mg, 0.24mmol, 1eq) was dissolved in tetrahydrofuran (5mL), triethylamine (242.86mg, 2.4mmol, 10eq) and triphosgene (71.22mg, 0.24mmol, 1eq) were added at 0°C, and the mixture was stirred at 0°C for 1h. LCMS showed the reaction was completed. Water was added to the mixture, which was extracted with ethyl acetate, and the organic phase was dried over anhydrous sodium sulfate, filtered, and concentrated to give the target compound (200mg). LCMS(ESI)[M+H-Boc]⁺=812.5.

### Step 2: Synthesis of tert-butyl (R)-3-((S)-1-(tert-butoxy)-3-(3-((((2-((3-((S)-3-(tert-butoxy)-2-((R)-1-(tert-butoxycarbonyl)pyrrolidin-3-yl)-3-oxopropyl)benzyl)oxy)ethyl)(3-((S)-3-(tert-butoxy)-2-((R)-1-(tert-butoxycarbonyl)pyrrolidin-3-yl)-3-oxopropyl)phenethyl)carbamoyl)oxy)methyl)phenyl)-1-oxopropan-2-yl)pyrrolidine-1-carboxylate

*Tert*-butyl (R)-3-((S)-1-(tert-butoxy)-3-(3-(hydroxymethyl)phenyl)-1-oxopropan-2-yl)pyrrolidine-1-carboxylate(80mg, 0.2mmol, 1eq) was dissolved in tetrahydrofuran (5mL), sodium hydride (9.07mg, 0.39mmol, 2eq) was added at 0°C, and the mixture was stirred at 0°C for 0.5h. The product from step 1 (216.03mg, 0.24mmol, 1.2eq) was added, and the mixture was stirred at 0°C for 2.5h. LCMS showed the reaction was completed. Water was added to the mixture, which was extracted with ethyl acetate. The organic phase was dried over anhydrous sodium sulfate, filtered, and rotary-evaporated to give a crude product, which was purified by column chromatography (silica gel, petroleum ether:tetrahydrofuran=3:1) to give the target compound (100mg). LCMS(ESI)[M+H-Boc]⁺=1181.8; ¹HNMR(400MHz, CDCl₃) δ 7.24-7.19 (m, 3H), 7.17-7.06 (m, 6H), 7.03-6.96 (m, 2H), 6.95-6.87 (m, 1H), 5.30 (s, 2H), 5.08 (s, 2H), 3.75-3.35 (m, 12H), 3.30-3.18 (m, 3H), 3.09-2.92 (m, 3H), 2.88-2.63 (m, 8H), 2.52-2.30 (m, 6H), 1.99-1.87 (m, 3H), 1.69-1.62 (m, 3H), 1.47 (s, 27H), 1.25 (s, 27H).

### Step 3: Synthesis of (S)-3-(3-(2-((((3-((S)-2-carboxy-2-((R)-pyrrolidin-3-yl)ethyl)benzyl)oxy)carbonyl)(2-((3-((S)-2-carboxy-2-((R)-pyrrolidin-3-yl)ethyl)benzyl)oxy)ethyl)amino)ethyl)phenyl)-2-((R)-pyrrolidin-3-yl)propanoic acid

The product from step 2 (100mg, 0.08mmol, 1eq) was dissolved in 1,4-dioxane (2mL), and hydrochloric acid/1,4-dioxane (2mL, 4.0M) was added at room temperature. The mixture was stirred for 3h. LCMS showed the reaction was completed. The reaction mixture was rotary-evaporated to dryness, and the crude product was adjusted to pH=7-8 with sodium hydroxide solution (1.0M). Then it was purified by pre-HPLC (C₁₈, 10 mmol NH₄HCO₃ in H₂O/ACN) to give the target compound (50.65mg). LCMS(ESI)[M+H]⁺=813.6; ¹HNMR(400MHz, D₂O) δ 7.30-7.18 (m, 2H), 7.18-6.91 (m, 9H), 6.89-6.80 (m, 1H), 4.93 (s, 1H), 4.79 (s, 1H), 4.42-4.34 (m, 2H), 3.57-3.50 (m, 1H), 3.48-3.42 (m, 1H), 3.41-3.18 (m, 10H), 3.17-3.06 (m, 3H), 2.82-2.46 (m, 11H), 2.42-2.23 (m, 6H), 2.09-1.92 (m, 3H), 1.72-1.54 (m, 3H).

### Example 75 (Compound 165)

### Preparation of (2S,2'S)-3,3'-((((((3-((S)-2-carboxy-2-((R)-pyrrolidin-3-yl)ethyl)phenyl)carbamoyl)azanediyl)bis(ethane-2,1-diyl))bis(oxy))bis(3,1-phenylene))bis(2-((R)-pyrrolidin-3-yl)propanoic acid)

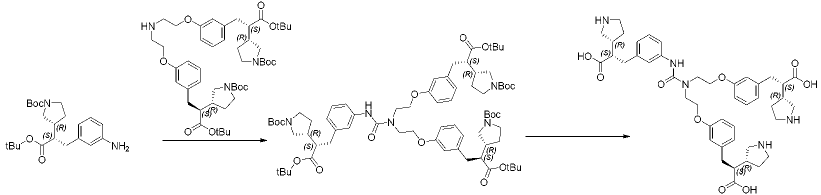

### Step 1: Synthesis of di-tert-butyl 3,3'-((2S,2'S)-((((((3-((S)-3-(tert-butoxy)-2-((R)-1-(tert-butoxycarbonyl)pyrrolidin-3-yl)-3-oxopropyl)phenyl)carbamoyl)azanediyl)bis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)

Under nitrogen protection, *tert-*butyl (R)-3-((S)-3-(3-aminophenyl)-1-(tert-butoxy)-1-oxopropan-2-yl)pyrrolidine-1-carboxylate(100mg, 0.26mmol, 1eq) was dissolved in tetrahydrofuran (3mL). Triethylamine (259.12mg, 2.56mmol, 10eq) and triphosgene (37.99mg, 0.13mmol, 0.5eq) were added at 0°C. The mixture was stirred at 0°C for 1h. Di-*tert*-butyl 3,3'-((2S,2'S)-(((azanediylbis(ethane-2,1-diyl))bis(oxy))bis(3,1-phenylene))bis(3-(tert-butoxy)-3-oxopropane-1,2-diyl))(3R,3'R)-bis(pyrrolidine-1-carboxylate)(261.84mg, 0.31mmol, 1.2eq) was added and the mixture was stirred at room temperature for 2h. LCMS showed the reaction was completed. Water was added to the mixture, which was extracted with ethyl acetate. The organic phase was dried over anhydrous sodium sulfate, filtered, and concentrated to give a crude product, which was purified by column chromatography (C₁₈, 0.03% TFA in H₂O/ACN) to give the target compound (100mg). LCMS(ESI)[M+H-Boc]⁺=1168.7.

### Step 2: Synthesis of (2S,2'S)-3,3'-((((((3-((S)-2-carboxy-2-((R)-pyrrolidin-3-yl)ethyl)phenyl)carbamoyl)azanediyl)bis(ethane-2,1-diyl))bis(oxy))bis(3,1-phenylene))bis(2-((R)-pyrrolidin-3-yl)propanoic acid)

The product from step 1 (100mg, 0.08mmol, 1eq) was dissolved in 1,4-dioxane (2mL), and hydrochloric acid/dioxane (2mL, 4.0M) solution was added at room temperature. The mixture was stirred at room temperature for 3h. LCMS showed the reaction was completed. The solvent was removed by rotary-evaporation to dryness to give a crude product. Water was added, and the crude product was adjusted to pH 7-8 with NaOH solution (1.0M). The crude product was purified by Pre-HPLC (C₁₈, 10 mmol NH₄HCO₃ in H₂O/ACN) to give the target compound (20.57mg). LCMS(ESI)[M+H]⁺=800.5; ¹HNMR(400MHz, D₂O) δ 7.26-7.19 (m, 1H), 7.13 (t, J=7.9Hz, 2H), 7.05-6.98 (m, 2H), 6.93 (d, J=7.5Hz, 1H), 6.81-6.63 (m, 6H), 4.23-4.13 (m, 4H), 3.77-3.62 (m, 4H), 3.44-3.37 (m, 1H), 3.36-3.23 (m, 5H), 3.20-3.07 (m, 3H), 2.91-2.82 (m, 1H), 2.78-2.61 (m, 6H), 2.57-2.48 (m, 2H), 2.45-2.24 (m, 6H), 2.10-1.94 (m, 3H), 1.75-1.54 (m, 3H).

### Example 76 (Compound 166)

### Preparation of (S)-3-(3-(2-(3-(3-((S)-2-carboxy-2-((R)-pyrrolidin-3-yl)ethyl)benzyl)-1-(2-(3-((S)-2-carboxy-2-((R)-pyrrolidin-3-yl)ethyl)phenoxy)ethyl)ureido)ethyl)phenyl)-2-((R)-pyrrolidin-3-yl)propanoic acid

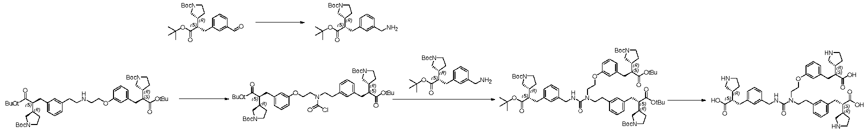

### Step 1: Synthesis of tert-butyl (R)-3-((S)-3-(3-(aminomethyl)phenyl)-1-(tert-butoxy)-1-oxopropan-2-yl)pyrrolidine-1-carboxylate

*Tert-*butyl (R)-3-((S)-1-(tert-butoxy)-3-(3-formylphenyl)-1-oxopropan-2-yl)pyrrolidine-1-carboxylate(1g, 2.48mmol, 1.0eq) was dissolved in ammonia methanol (10mL, 7.0M) solution, and acetic acid (14.88mg, 0.25mmol, 0.1eq) was added. The mixture was stirred at room temperature for 30min, and then sodium cyanoborohydride (1.23g, 19.83mmol, 8.0eq) was added to the reaction mixture. The reaction mixture was heated to 70°C in a sealed tube and stirred for 16h. LCMS showed the reaction was completed. After the reaction mixture was cooled to room temperature and concentrated under reduced pressure to give a crude product, which was separated and purified by reversed-phase column chromatography (C₁₈, 0.1% formic acid in water/acetonitrile) to give the target compound (310mg). LCMS(ESI)[M+H]⁺=405.3; ¹HNMR(400MHz, DMSO-*d₆*) δ 8.35 (s, 1H, HCOOH), 7.29-7.17 (m, 3H), 7.13-7.08 (m, 1H), 3.84 (s, 2H), 3.57-3.47 (m, 1H), 3.39-3.31 (m, 1H), 3.21-3.09 (m, 1H), 3.02-2.93 (m, 1H), 2.77-2.69 (m, 2H), 2.49-2.44 (m, 1H), 2.34-2.23 (m, 1H), 1.89-1.79 (m, 1H), 1.64-1.53 (m, 1H), 1.40 (s, 9H), 1.28-1.20 (m, 9H).

### Step 2: tert-butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)ethyl)phenyl)-1-oxopropan-2-yl)pyrrolidine-1-carboxylate

Under nitrogen atmosphere, triethylamine (363.07mg, 3.59mmol, 10.0eq) and triphosgene (106.48mg, 0.36mmol, 1.0eq) was added to a solution of *tert*-butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)ethyl)phenyl)-1-oxopropan-2-yl)pyrrolidine-1-carboxylate(300mg, 0.36mmol, 1.0eq) in tetrahydrofuran (12mL) successively. The reaction mixture was stirred at 0°C for 30min, LCMS showed the reaction was completed. The crude product was used for the next step without further purification. LCMS(ESI)[M+H-Boc]⁺=798.6. Step 3: Synthesis of *tert-*butyl (R)-3-((S)-1-(tert-butoxy)-3-(3-((3-(3-((S)-3-(tert-butoxy)-2-((R)-1-(tert-butoxycarbonyl)pyrrolidin-3-yl)-3-oxopropyl)phenethyl)-3-(2-(3-((S)-3-(tert-butoxy)-2-((R)-1-(tert-butoxycarbonyl)pyrrolidin-3-yl)-3-oxopropyl)phenoxy)ethyl)ureido)methyl)phenyl)-1-oxopropan-2-yl)pyrrolidine-1-carboxylate

*Tert-*butyl (R)-3-((S)-3-(3-(aminomethyl)phenyl)-1-(tert-butoxy)-1-oxopropan-2-yl)pyrrolidine-1-carboxylate(160.2mg, 0.4mmol, 1.1eq) and N,N-dimethylpyridine-4-amine (4.4mg, 0.04mmol, 0.1eq) were added to the reaction solution from the previous step. The reaction mixture was heated to 50°C and stirred for 16h. After the reaction mixture was cooled to room temperature, it was concentrated under reduced pressure, and the residue was separated and purified by reversed-phase preparation (C₁₈, 0.03% aqueous trifluoroacetic acid/acetonitrile) to give the target compound (95mg). LCMS(ESI)[M+H-Boc]⁺=1166.7.

Step 4: The product from step 3 (85mg, 0.07mmol, 1.0eq) was dissolved in a solution of hydrochloric acid/1,4-dioxane (4mL, 4.0M), and the reaction mixture was stirred at room temperature for 3h. After the reaction was completed, the reaction mixture was concentrated under reduced pressure. The residue was adjusted to pH 7-8 with sodium hydroxide solution (1.0M). The mixture was separated and purified by preparation (C₁₈, 0.03% ammonium bicarbonate in water/acetonitrile) to give the target compound (33.5mg). LCMS(ESI)[M+H]⁺=798.5; ¹HNMR(400MHz, D₂O) δ 7.21 (t, *J*=7.5Hz, 1H), 7.13 (dd, *J*=13.2*,* 7.3Hz, 2H), 7.08-7.02 (m, 2H), 7.00-6.91 (m, 4H), 6.79 (d, *J*=7.3Hz, 1H), 6.60-6.49 (m, 2H), 4.21 (s, 2H), 3.82 (s, 2H), 3.56-3.46 (m, 2H), 3.39-3.25 (m, 8H), 3.16-3.06 (m, 3H), 2.82-2.68 (m, 7H), 2.64-2.56 (m, 3H), 2.51-2.44 (m, 1H), 2.39-2.25 (m, 6H), 2.06-1.95 (m, 3H), 1.70-1.56 (m, 3H).

### Example 77 (Compound 129)

### Preparation of (S)-3-(3-(2-(1,3-bis(3-((S)-2-carboxy-2-((R)-pyrrolidin-3-yl)ethyl)phenethyl)ureido)ethoxy)phenyl)-2-((R)-pyrrolidin-3-yl)propanoic acid

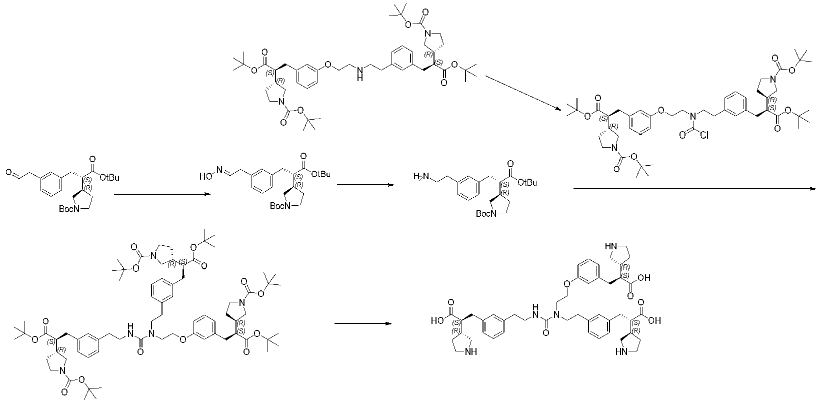

### Step 1: Synthesis of tert-butyl (R)-3-((S)-1-(tert-butoxy)-3-(3-((E)-2-(hydroxyimino)ethyl)phenyl)-1-oxopropan-2-yl)pyrrolidine-1-carboxylate

*Tert-butyl* (R)-3-((S)-1-(tert-butoxy)-1-oxo-3-(3-(2-oxoethyl)phenyl)propan-2-yl)pyrrolidine-1-carboxylate(1.4g, 3.35mmol, 1.0eq) was dissolved in ethanol (15mL), and hydroxylamine hydrochloride (349.5mg, 5.03mmol, 1.5eq) was added at room temperature. The mixture was stirred at room temperature for 4h. LCMS showed the reaction was completed. The mixture was concentrated to give a crude product, which was separated and purified by rapid chromatography (silica gel, petroleum ether:tetrahydrofuran=6:1) to give the target compound (500mg). LCMS(ESI)[M+Na]⁺=455.3.

### Step 2: Synthesis of tert-butyl (R)-3-((S)-3-(3-(2-aminoethyl)phenyl)-1-(tert-butoxy)-1-oxopropan-2-yl)pyrrolidine-1-carboxylate

At room temperature, *tert-*butyl (R)-3-((S)-1-(tert-butoxy)-3-(3-((E)-2-(hydroxyimino)ethyl)phenyl)-1-oxopropan-2-yl)pyrrolidine-1-carboxylate(500mg, 1.18mmol) was dissolved in ethanol (5mL). Pd/C (20mg, 10%) was added, and the reaction mixture was stirred at room temperature for 16h. LCMS showed the reaction was completed. The reaction mixture was filtered and concentrated. The residue was separated and purified by rapid chromatography (silica gel, petroleum ether:tetrahydrofuran=1:1) to give the target compound (300mg). LCMS(ESI)[M+H]⁺=419.4.

### Step 3: Synthesis of tert-butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)ethyl)phenyl)-1-oxopropan-2-yl)pyrrolidine-1-carboxylate

*Tert-*butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)ethyl)phenyl)-1-oxopropan-2-yl)pyrrolidine-1-carboxylate(200mg, 0.24mmol, 1.0eq) was dissolved in tetrahydrofuran (20mL), triethylamine (242.05mg, 2.39mmol, 10eq) and triphosgene (70.98mg, 0.24mmol, 1eq) were added under ice bath, and the reaction mixture was stirred at room temperature for 1h. LCMS showed the reaction was completed. The reaction solution was directly used for the next step. LCMS(ESI)[M+Na]⁺=920.5.

### Step 4: Synthesis of tert-butyl (R)-3-((S)-3-(3-(2-(1,3-bis(3-((S)-3-(tert-butoxy)-2-((R)-1-(tert-butoxycarbonyl)pyrrolidin-3-yl)-3-oxopropyl)phenethyl)ureido)ethoxy)phenyl)-1-(tert-butoxy)-1-oxopropan-2-yl)pyrrolidine-1-carboxylate

*Tert-*butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)ethyl)phenyl)-1-oxopropan-2-yl)pyrrolidine-1-carboxylate(200mg, 0.22mmol, 1.0eq) in tetrahydrofuran (20mL) at room temperature. The reaction mixture was stirred at 50°C for 16h. LCMS showed the reaction was completed. The mixture was concentrated to give a crude product, which was separated and purified by silica gel column chromatography (silica gel, petroleum ether:tetrahydrofuran=2:1) to give the target product (200mg). LCMS(ESI)[M-Boc]⁺=1180.7; ¹HNMR(400MHz, DMSO-*d₆*) δ 7.21-7.13 (m, 3H), 7.08-6.97 (m, 6H), 6.73 (t, *J*=8.7Hz, 3H), 6.40 (t, *J*=5.4Hz, 1H), 3.92 (t, *J*=5.6Hz, 2H), 3.54-3.39 (m, 7H), 3.37-3.34 (m, 3H), 3.28-3.21 (m, 2H), 3.20-3.07 (m, 3H), 2.97 (t, *J*=10.0Hz, 3H), 2.77-2.63 (m, 10H), 2.49-2.42 (m, 3H), 2.26 (s, 3H), 1.89-1.76 (m, 3H), 1.63-1.51 (m, 3H), 1.39 (s, 27H), 1.22 (d, *J*=2.9Hz, 27H).

### Step 5: Synthesis of (S)-3-(3-(2-(1,3-bis(3-((S)-2-carboxy-2-((R)-pyrrolidin-3-yl)ethyl)phenethyl)ureido)ethoxy)phenyl)-2-((R)-pyrrolidin-3-yl)propanoic acid

*Tert-*butyl (R)-3-((S)-3-(3-(2-(1,3-bis(3-((S)-3-(tert-butoxy)-2-((R)-1-(tert-butoxycarbonyl)pyrrolidin-3-yl)-3-oxopropyl)phenethyl)ureido)ethoxy)phenyl)-1-(tert-butoxy)-1-oxopropan-2-yl)pyrrolidine-1-carboxylate(200mg, 0.16mmol, 1.0eq) was dissolved in hydrochloric acid/1,4-dioxane (5mL, 4.0M) and the reaction mixture was stirred at room temperature for 2h. After the reaction is completed, the mixture was concentrated to dryness, added with water to dissolve, and adjusted to pH=7 with sodium hydroxide aqueous solution (1.0M). Then, it was concentrated to give a crude product, which was separated and purified by preparative chromatography (C₁₈, 0.1% NH₄HCO₃ in H₂O/acetonitrile) to give the target compound (50mg). LCMS(ESI)[M+H]⁺=812.6; ¹HNMR(400MHz, D₂O) δ 7.21-7.10 (m, 3H), 7.05-6.77 (m, 7H), 6.65-6.53 (m, 2H), 3.72 (s, 2H), 3.41-3.04 (m, 15H), 2.84-2.46 (m, 13H), 2.44-2.20 (m, 6H), 2.09-1.91 (m, 3H), 1.74-1.53 (m, 3H).

### Example 78 (Compound 138)

### Preparation of (S)-3-(3-(2-((3-((S)-2-carboxy-2-((R)-pyrrolidin-3-yl)ethyl)benzyl)(((3-((S)-2-carboxy-2-((R)-pyrrolidin-3-yl)ethyl)phenyl)thio)carbonyl)amino)ethoxy)phenyl)-2-((R)-pyrrolidin-3-yl)propanoic acid

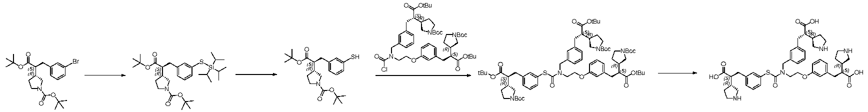

### Step 1 : Synthesis of tert-butyl (R)-3-((S)-1-(tert-butoxy)-1-oxo-3-(3-((triisopropylsilyl)thio)phenyl)propan-2-yl)pyrrolidine-1-carboxylate

Under nitrogen protection, *tert*-butyl (R)-3-((S)-3-(3-bromophenyl)-1-(tert-butoxy)-1-oxopropan-2-yl)pyrrolidine-1-carboxylate(200mg, 0.44mmol, 1eq) was dissolved in toluene (2mL), and [1,1'-bis(diphenylphosphino)ferrocene]palladium(II) dichloride (32.29mg, 0.04mmol, 0.1eq), tris(propan-2-yl)silanethiol (0.08mL, 0.44mmol, 1eq) and cesium carbonate (286.81mg, 0.88mmol, 2eq) were added; the reaction mixture was stirred at 120°C for 5hours. The reaction mixture was filtered through diatomaceous earth, the filtrate was concentrated, and separated and purified by rapid chromatography (silica gel, petroleum ether:ethyl acetate=15:1) to give the target compound (100mg). LCMS(ESI)[M+H-Boc]⁺=464.4; ¹HNMR(400MHz, CDCl₃) δ 7.33-7.28 (m, 2H), 7.13-7.07 (m, 1H), 7.03-6.98 (m, 1H), 3.73-3.41 (m, 2H), 3.29-3.18 (m, 1H), 3.07-2.92 (m, 1H), 2.85-2.66 (m, 2H), 2.46-2.30 (m, 2H), 1.97-1.89 (m, 1H), 1.73-1.63 (m, 1H), 1.47 (s, 9H), 1.29 (d, J=7.2Hz, 9H), 1.26-1.19 (m, 3H), 1.11-1.05 (m, 18H).

### Step 2: Synthesis of tert-butyl (R)-3-((S)-1-(tert-butoxy)-3-(3-mercaptophenyl)-1-oxopropan-2-yl)pyrrolidine-1-carboxylate

The product from the step 1 (800mg, 1.42mmol, 1eq) was dissolved in 1,4-dioxane (6mL), and hydrochloric acid aqueous solution (3mL, 1.0M) was added. The reaction mixture was stirred at room temperature for 2.5h. The reaction mixture was quenched with water and extracted with ethyl acetate. The organic phases were combined, dried over anhydrous sodium sulfate, and filtered. The filtrate was concentrated and separated and purified by rapid chromatography (silica gel, ethyl acetate:petroleum ether=1:3) to give the target compound (200mg). LCMS(ESI)[M+Na]⁺=430.2.

### Step 3: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)(((3-((S)-3-(tert-butoxy)-2-((R)-1-(tert-butoxycarbonyl)pyrrolidin-3-yl)-3-oxopropyl)phenyl)thio)carbonyl)amino)methyl)phenyl)-1-oxopropan-2-yl)pyrrolidine-1-carboxylate

*Tert*-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)(chlorocarbonyl)amino)methyl)phenyl)-1-oxopropan-2-yl)pyrrolidine-1-carboxylate(200mg, 0.22mmol, 1eq) was dissolved in tetrahydrofuran (5mL), *tert-*butyl (R)-3-((S)-1-(tert-butoxy)-3-(3-mercaptophenyl)-1-oxopropan-2-yl)pyrrolidine-1-carboxylate(88.52mg, 0.22mmol, 1eq) and N,N-dimethylpyridine-4-amine (2.76mg, 0.022mmol, 0.1eq) were added under nitrogen protection; the reaction mixture was stirred at room temperature for 18h. The reaction mixture was concentrated and purified by column chromatography (silica gel, petroleum ether:ethyl acetate=4:1) to give the target product (80mg). LCMS(ESI)[M+H-Boc]⁺=1155.6.

### Step 4: Synthesis of (S)-3-(3-(2-((3-((S)-2-carboxy-2-((R)-pyrrolidin-3-yl)ethyl)benzyl)(((3-((S)-2-carboxy-2-((R)-pyrrolidin-3-yl)ethyl)phenyl)thio)carbonyl)amino)ethoxy)phenyl)-2-((R)-pyrrolidin-3-yl)propanoic acid

The product from the step 3 (70mg, 0.06mmol, 1eq) was dissolved in 1,4-dioxane (2mL), hydrochloric acid/1,4-dioxane (2mL, 4.0M) was added, and the reaction mixture was stirred at room temperature for 2h. The reaction mixture was concentrated, and the pH was adjusted to 7 with sodium hydroxide (1.0M) aqueous solution. The mixture was separated and purified by Prep-HPLC (0.1% NH₄HCO₃ in water, H₂O/MeCN) to give the target compound (8.3mg). LCMS(ESI)[M+H]⁺=787.4; ¹HNMR(400MHz, D₂O) δ 7.33-6.98 (m, 9H), 6.84-6.67 (m, 3H), 4.16 (d, *J*=39.3Hz, 2H), 3.78 (d, *J*=44.3Hz, 2H), 3.39-3.25 (m, 6H), 3.17-3.07 (m, 3H), 2.92-2.23 (m, 17H), 2.07-1.97 (m, 3H), 1.70-1.58 (m, 3H).

### Example 79 (Compound 167)

### Preparation of (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)ethyl)phenyl)-2-((R)-pyrrolidin-3-yl)propanoic acid

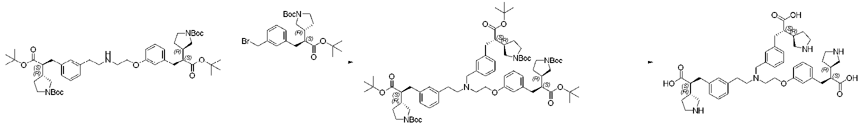

### Step 1: Synthesis of tert-butyl (R)-3-((S)-1-(tert-butoxy)-3-(3-(((3-((S)-3-(tert-butoxy)-2-((R)-1-(tert-butoxycarbonyl)pyrrolidin-3-yl)-3-oxopropyl)phenethyl)(2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

*Tert-*butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)amino)ethyl)phenyl)-1-oxopropan-2-yl)pyrrolidine-1-carboxylate(300mg, 0.36mmol, 1.0eq) was dissolved in acetonitrile (6mL). Potassium carbonate (247.95mg, 1.79mmol, 5eq) and *tert-*butyl (R)-3-((S)-3-(3-(bromomethyl)phenyl)-1-(tert-butoxy)-1-oxopropan-2-yl)pyrrolidine-1-carboxylate(268.91mg, 0.57mmol, 1.6eq) were added at room temperature. The reaction mixture was stirred at 70°C for 16h. The mixture was concentrated to give a crude product, which was separated and purified by reversed-phase column chromatography (C₁₈, water:acetonitrile=1:10) to give the target compound (260mg). LCMS(ESI)[M+H]⁺=1223.6; ¹HNMR(400MHz, DMSO-*d₆*) δ 7.21-7.11 (m, 5H), 7.07-6.95 (m, 4H), 6.72 (t, *J*=6.0Hz, 3H), 3.98 (t, *J*=5.6Hz, 2H), 3.69 (s, 2H), 3.56-3.42 (m, 3H), 3.38-3.33 (m, 3H), 3.19-3.06 (m, 3H), 3.02-2.91 (m, 3H), 2.85 (t, *J*=5.6Hz, 2H), 2.76-2.63 (m, 10H), 2.49-2.40 (m, 3H), 2.26 (s, 3H), 1.87-1.77 (m, 3H), 1.64-1.49 (m, 3H), 1.39 (s, 27H), 1.21 (d, *J*=9.8Hz, 27H).

### Step 2: Synthesis of (S)-3-(3-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)ethyl)phenyl)-2-((R)-pyrrolidin-3-yl)propanoic acid

The product from the step 1 (260mg, 0.21mmol, 1.0eq) was dissolved in hydrochloric acid/1,4-dioxane (6mL, 4.0M) and the reaction mixture was stirred at room temperature for 4h. After the reaction was completed, the mixture was concentrated to dryness, dissolved in water, and the pH was adjusted to 7 with sodium hydroxide aqueous solution (1.0M). The mixture was concentrated to give a crude product, which was separated and purified by reversed-phase column chromatography (C₁₈, 10mmol/L NH₄HCO₃ in water, MeCN) to give the target compound (90mg). LCMS(ESI)[M+H]⁺=755.5; ¹HNMR(400MHz, D₂O) δ 7.22-7.04 (m, 6H), 6.98 (d, *J*=7.7Hz, 1H), 6.95-6.88 (m, 2H), 6.79 (d, *J*=7.6Hz, 1H), 6.70 (s, 1H), 6.66 (d, *J*=8.2Hz, 1H), 3.98 (t, *J*=5.0Hz, 2H), 3.63 (s, 2H), 3.34-3.20 (m, 6H), 3.17-3.04 (m, 3H), 2.87-2.63 (m, 12H), 2.62-2.47 (m, 3H), 2.44-2.24 (m, 6H), 2.06-1.94 (m, 3H), 1.70-1.56 (m, 3H).

### Example 80 (Compound 168)

### Preparation of (S)-3-(4-(2-((4-((S)-2-carboxy-2-((R)-pyrrolidin-3-yl)ethyl)benzyl)(2-(4-((S)-2-carboxy-2-((R)-pyrrolidin-3-yl)ethyl)phenoxy)ethyl)amino)-2-oxoethyl)phenyl)-2-((R)-pyrrolidin-3-yl)propanoic acid

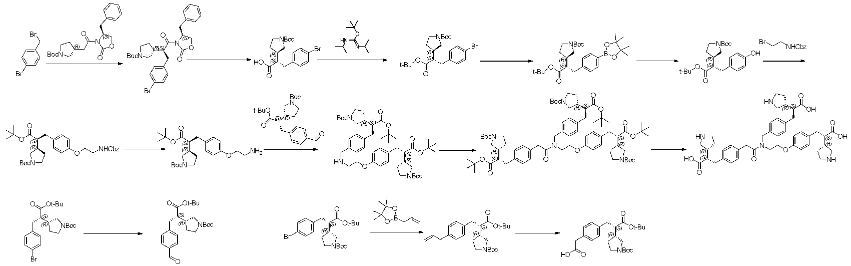

### Step 1: Synthesis of tert-butyl (R)-3-((S)-1-((S)-4-benzyl-2-oxooxazolidin-3-yl)-3-(4-bromophenyl)-1-oxopropan-2-yl)pyrrolidine-1-carboxylate

*Tert-*butyl (R)-3-(2-((S)-4-benzyl-2-oxooxazolidin-3-yl)-2-oxoethyl)pyrrolidine-1-carboxylate(24.87g) was dissolved in anhydrous tetrahydrofuran (200mL). Cooling to 0°C in an ice-water bath, lithium bis(trimethylsilyl)amide (120.03mL, 1.0M) was added to the mixture, which was stirred at 0°C for 30min. Then a solution of 1-bromo-4-(bromomethyl)benzene(20g) in tetrahydrofuran (100mL) was added slowly at this temperature. After the addition was completed, the reaction mixture was slowly warmed to room temperature and stirred at room temperature for 15.5h. LCMS showed the reaction was completed. Saturated ammonium chloride solution was added, and the mixture was extracted with ethyl acetate. The organic phases were combined, dried over anhydrous sodium sulfate, filtered, and concentrated to give the crude product (40g). This crude was used directly in the next step. LCMS(ESI)[M+H-Boc]⁺=459.2.

### Step 2: Synthesis of (S)-3-(4-bromophenyl)-2-((R)-1-(tert-butoxycarbonyl)pyrrolidin-3-yl)propanoic acid

The product from the step 1 (40g) was dissolved in tetrahydrofuran (300mL). Cooling to 0°C in an ice-water bath, hydrogen peroxide (122mL) was added; an aqueous solution (100mL) of lithium hydroxide (2.58g) was added dropwise to the above solution. After the addition was completed, the mixture was slowly warmed to room temperature and stirred for 3h. LCMS showed the reaction was completed. Saturated sodium bisulfite solution (100mL) and an aqueous solution of sodium hydroxide (200mL, 1.0M) were added. The mixture was extracted with methyl *tert-*butyl ether; the aqueous phase was adjusted to a pH of 4-5 with hydrochloric acid (1.0M) and extracted with ethyl acetate; the organic phases were combined, dried over anhydrous sodium sulfate, filtered, and concentrated to give the target compound (18g). LCMS(ESI)[M+H-*tert*-butyl]⁺=342.1.

### Step 3: Synthesis of tert-butyl (R)-3-((S)-3-(4-bromophenyl)-1-(tert-butoxy)-1-oxopropan-2-yl)pyrrolidine-1-carboxylate

(S)-3-(4-bromophenyl)-2-((R)-1-(tert-butoxycarbonyl)pyrrolidin-3-yl)propanoic acid(17g) was dissolved in anhydrous 2-methyltetrahydrofuran (200mL), *tert-*butyl N,N'-diisopropylcarbamimidate (25.65g) was added at room temperature, and the reaction mixture was stirred at 70°C for 3h. LCMS showed the reaction was completed. The reaction mixture was filtered and concentrated to give a crude product, which was purified by column chromatography (silica gel, petroleum ether:tetrahydrofuran=10:1) to give the target compound (7.9g). LCMS(ESI)[M+H]⁺=456.2; ¹HNMR(400MHz, CDCl₃) δ 7.38 (d, *J*=7.5Hz, 2H), 7.09-6.98 (m, 2H), 3.62-3.40 (m, 2H), 3.32-3.20 (m, 1H), 3.07-2.91 (m, 1H), 2.86-2.68 (m, 2H), 2.47-2.40 (m, 1H), 2.39-2.30 (m, 1H), 1.99-1.89 (m, 1H), 1.72-1.64 (m, 1H), 1.47 (s, 9H), 1.29 (s, 9H).

### Step 4: Synthesis of tert-butyl (R)-3-((S)-1-(tert-butoxy)-1-oxo-3-(4-(4,4,5,5-tetramethyl-1,3,2-dioxaborolan-2-yl)phenyl)propan-2-yl)pyrrolidine-1-carboxylate

Under nitrogen protection, *tert-*butyl (R)-3-((S)-3-(4-bromophenyl)-1-(tert-butoxy)-1-oxopropan-2-yl)pyrrolidine-1-carboxylate(3g) was dissolved in 1,4-dioxane (50mL), bis(pinacolato)diboron (3.35g), [1,1'-bis(diphenylphosphino)ferrocene]palladium(II) dichloride (484.26mg) and potassium acetate (1.94g) were added at room temperature; the reaction mixture was stirred at 90°C for 16h. LCMS showed the reaction was completed. Water was added to the mixture, which was extracted with ethyl acetate, and the organic phases were combined, dried over anhydrous sodium sulfate, filtered and concentrated to give the crude product (3g). This crude was used directly in the next step. LCMS(ESI)[M+Na]⁺=524.4.

### Step 5: Synthesis of tert-butyl (R)-3-((S)-1-(tert-butoxy)-3-(4-hydroxyphenyl)-1-oxopropan-2-yl)pyrrolidine-1-carboxylate

*Tert-*butyl (R)-3-((S)-1-(tert-butoxy)-1-oxo-3-(4-(4,4,5,5-tetramethyl-1,3,2-dioxaborolan-2-yl)phenyl)propan-2-yl)pyrrolidine-1-carboxylate(3g) was dissolved in methanol (30mL), and hydrogen peroxide (3mL) was added at room temperature. The mixture was stirred at room temperature for 1h. LCMS showed the reaction was completed. The solvent was concentrated. After the addition of water, the mixture was extracted with ethyl acetate. The organic phase was dried over anhydrous sodium sulfate and concentrated to give a crude product, which was purified by column chromatography (silica gel, petroleum ether:tetrahydrofuran=10:1) to give the target compound (1.5g). LCMS(ESI)[M+H]⁺=392.5; ¹HNMR(400MHz, CDCl₃) δ 6.99 (dd, *J*=20.4, 8.0Hz, 2H), 6.73 (d, *J*=8.0Hz, 2H), 3.69-3.40 (m, 2H), 3.30-3.16 (m, 1H), 3.02-2.92 (m, 1H), 2.82-2.53 (m, 2H), 2.48-2.24 (m, 2H), 1.98-1.88 (m, 1H), 1.66-1.57 (m, 1H), 1.47 (s, 9H), 1.28 (s, 9H).

### Step 6: Synthesis of tert-butyl (R)-3-((S)-3-(4-(2-(((benzyloxy)carbonyl)amino)ethoxy)phenyl)-1-(tert-butoxy)-1-oxopropan-2-yl)pyrrolidine-1-carboxylate

*Tert-*butyl (R)-3-((S)-1-(tert-butoxy)-3-(4-hydroxyphenyl)-1-oxopropan-2-yl)pyrrolidine-1-carboxylate(2g) was dissolved in N,N-dimethylformamide (20mL), potassium carbonate (2.12g) and benzyl (2-bromoethyl)carbamate (2.64g) were added at room temperature, and the mixture was stirred at 80°C for 16h. LCMS showed the reaction was completed. Water was added, and the mixture was extracted with ethyl acetate. The organic phase was washed with saturated saline, dried over anhydrous sodium sulfate, filtered, and the filtrate was rotary-evaporated to dryness to give the crude product, which was purified by reversed-phase column chromatography (C₁₈, 0.03% TFA in H₂O in ACN) to give the target compound (1.6g). LCMS(ESI)[M+H]⁺=569.4.

### Step 7: Synthesis of tert-butyl (R)-3-((S)-3-(4-(2-aminoethoxy)phenyl)-1-(tert-butoxy)-1-oxopropan-2-yl)pyrrolidine-1-carboxylate

*Tert-*butyl (R)-3-((S)-3-(4-(2-(((benzyloxy)carbonyl)amino)ethoxy)phenyl)-1-(tert-butoxy)-1-oxopropan-2-yl)pyrrolidine-1-carboxylate(1.6g) was dissolved in tetrahydrofuran (20mL), Pd/C (320mg, 10% content) was added at room temperature. Replaced with hydrogen gas three times, the mixture was stirred for 2h under hydrogen atmosphere. LCMS showed the reaction was completed. The reaction mixture was filtered, and the filtrate was concentrated to give the target compound (1.1g). LCMS(ESI)[M+H]⁺=435.3; ¹HNMR(400MHz, CDCl₃) δ 7.11-7.02 (m, 2H), 6.81 (d, *J*=8.0Hz, 2H), 3.96 (t, *J=*5.1Hz, 2H), 3.78-3.65 (m, 2H), 3.59-3.50 (m, 1H), 3.29-3.18 (m, 1H), 3.07 (t, *J*=5.1Hz, 2H), 3.03-2.91 (m, 1H), 2.84-2.67 (m, 2H), 2.49-2.39 (m, 1H), 2.36-2.27 (m, 1H), 1.98-1.89 (m, 1H), 1.46 (s, 9H), 1.30 (s, 9H).

### Step 8: Synthesis of tert-butyl (R)-3-((S)-1-(tert-butoxy)-3-(4-formylphenyl)-1-oxopropan-2-yl)pyrrolidine-1-carboxylate

*Tert-*butyl (R)-3-((S)-3-(4-bromophenyl)-1-(tert-butoxy)-1-oxopropan-2-yl)pyrrolidine-1-carboxylate(1.2g) was dissolved in N,N-dimethylformamide (20mL), followed by adding triethylsilane (460.47mg), sodium carbonate (559.63mg) and [1,1'-bis(diphenylphosphino)ferrocene]palladium(II) dichloride (193.7mg). Replaced with carbon monoxide three times, the mixture was heated to 80°C and stirred for 16h under a carbon monoxide atmosphere. LCMS showed the reaction was completed. After cooling to room temperature, water was added, and the mixture was extracted with ethyl acetate. The organic phases were combined, washed with saturated saline, dried over anhydrous sodium sulfate and filtered. The filtrate was rotary-evaporated to dryness to give a crude product, which was purified by column chromatography (silica gel, petroleum ether:tetrahydrofuran=3:1) to give the target compound (610mg). LCMS(ESI)[M+H-Boc-*tert*-butyl]⁺=248.5.

### Step 9: Synthesis of tert-butyl (R)-3-((S)-1-(tert-butoxy)-3-(4-(((2-(4-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

*Tert*-butyl *tert*-butyl (R)-3-((S)-3-(4-(2-aminoethoxy)phenyl)-1-(tert-butoxy)-1-oxopropan-2-yl)pyrrolidine-1-carboxylate(600mg) was dissolved in ethanol (10mL), *tert-*butyl (R)-3-((S)-1-(tert-butoxy)-3-(4-formylphenyl)-1-oxopropan-2-yl)pyrrolidine-1-carboxylate(557.12mg) and titanium tetraisopropanolate (784.83mg) were added, and the mixture was stirred at room temperature for 5h. Sodium borohydride (78.35mg) was added, and the mixture was stirred for another 1 h. LCMS showed the reaction was completed. Saturated ammonium chloride (0.1mL) solution was added. The mixture was filtered, and the filtrate was rotary-evaporated to dryness. The crude product was purified by column chromatography (silica gel, petroleum ether:tetrahydrofuran=3:1) to give the target compound (700mg). LCMS(ESI)[M+H]⁺=822.4.

### Step 10: Synthesis of tert-butyl (R)-3-((S)-3-(4-allylphenyl)-1-(tert-butoxy)-1-oxopropan-2-yl)pyrrolidine-1-carboxylate

*Tert-*butyl (R)-3-((S)-3-(4-bromophenyl)-1-(tert-butoxy)-1-oxopropan-2-yl)pyrrolidine-1-carboxylate(1.5g) was dissolved in 1,4-dioxane (25mL) and water (5mL) under nitrogen protection, 2-allyl-4,4,5,5-tetramethyl-1,3,2-dioxaborolane (831.8mg), potassium phosphate (2.1g) and [1,1'-bis(diphenylphosphino)ferrocene]palladium(II) dichloride (215.08mg) were added. The mixture was heated to 90°C and stirred for 5h. LCMS showed the reaction was completed. After cooling to room temperature, water was added, and the mixture was extracted with ethyl acetate. The organic phases were combined, washed with saturated saline, dried over anhydrous sodium sulfate and filtered. The filtrate was rotary-evaporated to dryness to give a crude product, which was purified by column chromatography (silica gel, petroleum ether:tetrahydrofuran=5:1) to give the target compound (1.2g). LCMS(ESI)[M+H]⁺=416.4.

### Step 11: Synthesis of 2-(4-((S)-3-(tert-butoxy)-2-((R)-1-(tert-butoxycarbonyl)pyrrolidin-3-yl)-3-oxopropyl)phenyl)acetic acid

*Tert-*butyl (R)-3-((S)-3-(4-allylphenyl)-1-(tert-butoxy)-1-oxopropan-2-yl)pyrrolidine-1-carboxylate(1.2g) was dissolved in acetonitrile (10mL) and water (10mL), ruthenium trichloride (49.66mg) and sodium periodate (2.47g) were added, and the mixture was stirred at room temperature for 1h. LCMS showed the reaction was completed. A small amount of sodium sulfite was added, and the reaction mixture was filtered. After the addition of water, the filtrate was extracted with ethyl acetate. The organic phases were combined, washed with saturated saline, dried over anhydrous sodium sulfate, filtered, and the filtrate was rotary-evaporated to dryness. The crude product was purified by reversed-phase column chromatography (C₁₈, H₂O/ACN) to give the target compound (400mg). LCMS(ESI)[M+Na]⁺=456.4.

### Step 12: Synthesis of tert-butyl (R)-3-((S)-1-(tert-butoxy)-3-(4-((N-(2-(4-((S)-3-(tert-butoxy)-2-((R)-1-(tert-butoxycarbonyl)pyrrolidin-3-yl)-3-oxopropyl)phenoxy)ethyl)-2-(4-((S)-3-(tert-butoxy)-2-((R)-1-(tert-butoxycarbonyl)pyrrolidin-3-yl)-3-oxopropyl)phenyl)acetamido)methyl)phenyl)-1-oxopropan-2-yl)pyrrolidine-1-carboxylate

The product from step 9 (150mg) and the product from step 11 (79.11mg) were dissolved in N,N-dimethylformamide (5mL), O-(7-azabenzotriazol-1-yl)-N,N,N',N'-tetramethyluronium hexafluorophosphate (83.25mg) and *N,N*-diisopropylethylamine (70.74mg) were added, and the mixture was stirred at room temperature for 1h. LCMS showed the reaction was completed. Water was added, and the mixture was extracted with ethyl acetate. The organic phases were combined, washed with saturated saline, dried over anhydrous sodium sulfate, and filtered. The filtrate was rotary-evaporated to dryness, and the crude product was purified by column chromatography (C₁₈, water/acetonitrile) to give the target compound (120mg). LCMS(ESI)[M+H-100]⁺=1137.6.

Step 13: The product from step 12 (120mg) was dissolved in 1,4-dioxane (2mL), hydrochloric acid/dioxane (2mL, 4.0M) solution was added at room temperature, and the mixture was stirred for 3h. LCMS showed the reaction was completed. The reaction mixture was rotary-evaporated to dryness to give a crude product, which was adjusted to a pH of 7 with sodium hydroxide solution (1.0M). The mixture was purified by reversed-phase preparative chromatography (C₁₈, 10 mmol NH₄HCO₃ in H₂O/ACN) to give the target compound (47.25mg). LCMS(ESI)[M+H]⁺=769.5; ¹HNMR(400MHz, D₂O) δ 7.17-6.96 (m, 10H), 6.79-6.71 (m, 2H), 4.60-4.51 (m, 2H), 4.11-3.94 (m, 2H), 3.82-3.74 (m, 1H), 3.70-3.56 (m, 3H), 3.42-3.27 (m, 6H), 3.22-3.08 (m, 3H), 2.87-2.59 (m, 9H), 2.45-2.28 (m, 6H), 2.11-1.98 (m, 3H), 1.74-1.56 (m, 3H).

### Example 81 (Compound 176)

### Preparation of (S)-3-(3-(2-((3-((S)-2-carboxy-2-((R)-pyrrolidin-3-yl)ethyl)-5-methoxybenzyl)(2-(3-((S)-2-carboxy-2-((R)-pyrrolidin-3-yl)ethyl)-5-methoxyphenoxy)ethyl)amino)-2-oxoethyl)-5-methoxyphenyl)-2-((R)-pyrrolidin-3-yl)propanoic acid

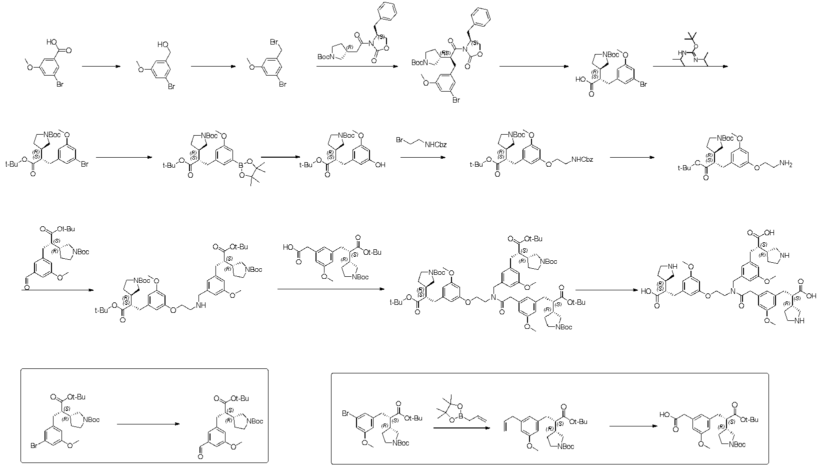

### Step 1: Synthesis of (3-bromo-5-methoxyphenyl)methanol

3-bromo-5-methoxybenzoic acid(20g, 86.57mmol, 1eq) was dissolved in tetrahydrofuran (200mL), and borane/tetrahydrofuran complex (216.41mL, 1.0M, 2.5eq) was added under nitrogen protection and ice bath; the reaction mixture was slowly warmed to room temperature and stirred at room temperature for 18h. The reaction mixture was cooled with an ice-water bath, methanol (20mL) was added, and the reaction mixture was directly concentrated and purified by column chromatography (silica gel, petroleum ether:ethyl acetate=4:1) to give the target compound (16g). ¹HNMR(400MHz, CDCl₃) δ 7.09 (s, 1H), 6.97 (t, J=2.0Hz, 1H), 6.84 (s, 1H), 4.64 (d, J=3.9Hz, 2H), 3.80 (s, 3H).

### Step 2: Synthesis of 1-bromo-3-(bromomethyl)-5-methoxybenzene

(3-bromo-5-methoxyphenyl)methanol(16g, 73.71mmol, 1eq) was dissolved in dichloromethane (160mL). Phosphorus tribromide (3.46mL, 36.86mmol, 0.5eq) was added under nitrogen protection and ice bath. The reaction mixture was slowly warmed to room temperature and stirred at room temperature for 2h. The reaction mixture was cooled with an ice bath, and saturated sodium bicarbonate (100mL) was added. The mixture was extracted with dichloromethane, and the organic phase was dried over anhydrous sodium sulfate, filtered, concentrated, and purified by column chromatography (silica gel, petroleum ether:ethyl acetate=4:1) to give the target compound (13g). ¹HNMR(400MHz, CDCl₃) δ 7.12 (d, J=1.4Hz, 1H), 6.98 (t, J=2.0Hz, 1H), 6.87-6.84 (m, 1H), 4.38 (s, 2H), 3.80 (s, 3H).

### Step 3: Synthesis of tert-butyl (R)-3-((S)-1-((S)-4-benzyl-2-oxooxazolidin-3-yl)-3-(3-bromo-5-methoxyphenyl)-1-oxopropan-2-yl)pyrrolidine-1-carboxylate

A solution of lithium bis(trimethylsilyl)amide (49.43mL, 49.43mmol, 1.0M in THF, 1.2eq) was added dropwise to a solution of *tert-*butyl (R)-3-(2-((S)-4-benzyl-2-oxooxazolidin-3-yl)-2-oxoethyl)pyrrolidine-1-carboxylate (16g, 41.19mmol, 1eq) in tetrahydrofuran (160mL) under ice bath and nitrogen protection. The reaction mixture was stirred at 0°C for 30 min, and a solution of 1-bromo-3-(bromomethyl)-5-methoxybenzene (13.83g, 49.43mmol, 1.2eq) in tetrahydrofuran (50mL) was added slowly. The reaction temperature was slowly raised to room temperature, and the mixture was stirred for 18h. The reaction mixture was cooled with an ice-water bath, and saturated aqueous ammonium chloride solution and water were added. The mixture was extracted with ethyl acetate, and the organic phase was washed with saturated sodium chloride aqueous solution, dried over anhydrous sodium sulfate, filtered, and concentrated to give the target compound (24g, crude). LCMS(ESI)[M+H-Boc]⁺=487.1

### Step 4: Synthesis of (S)-3-(3-bromo-5-methoxyphenyl)-2-((R)-1-(tert-butoxycarbonyl)pyrrolidin-3-yl)propanoic acid

A solution of hydrogen peroxide (69.63mL, 61.28mmol, 1.5eq) was added in one portion to a solution of *tert-*butyl (R)-3-((S)-1-((S)-4-benzyl-2-oxooxazolidin-3-yl)-3-(3-bromo-5-methoxyphenyl)-1-oxopropan-2-yl)pyrrolidine-1-carboxylate (24g, 40.85mmol, 1eq) in tetrahydrofuran (200mL). The reaction mixture was cooled with an ice-water bath, and a solution of lithium hydroxide monohydrate (2.57mg, 61.28mmol, 1.5eq) in water (30mL) was added. The reaction temperature was raised to room temperature and stirred for 2.5h. LCMS showed the reaction was completed. The reaction mixture was cooled to 0°C, and an aqueous solution of sodium bisulfite was added for extraction and separation, and the organic phase was adjusted to pH>12 with an aqueous solution of sodium hydroxide (1.0M); water was added, and the mixture was extracted with methyl *tert*-butyl ether. The aqueous phase was adjusted to pH=3 with hydrochloric acid (1.0M) and extracted with methyl *tert-*butyl ether. The organic phase was washed with saturated sodium chloride, dried over anhydrous sodium sulfate, filtered, and concentrated to give the target compound (15g). LCMS(ESI)[M-H]⁻=426.1.

### Step 5: Synthesis of tert-butyl (R)-3-((S)-3-(3-bromo-5-methoxyphenyl)-1-(tert-butoxy)-1-oxopropan-2-yl)pyrrolidine-1-carboxylate

Under nitrogen protection, *tert-*butyl N,N'-diisopropylcarbamimidate (131.91mL, 140.08mmol, 4eq) was added dropwise to a solution of (S)-3-(3-bromo-5-methoxyphenyl)-2-((R)-1-(tert-butoxycarbonyl)pyrrolidin-3-yl)propanoic acid (15g, 35.02mmol, 1eq) in 2-methyltetrahydrofuran (100mL), and the mixture was stirred at 70°C for 4h. The reaction mixture was filtered, washed with methyl *tert-*butyl ether, and the filtrate was concentrated. The residue was purified by rapid chromatography (silica gel: petroleum ether:tetrahydrofuran=5:1) to give the target compound (8.0g). LCMS(ESI)[M+ Na]⁺=506.2.

### Step 6: Synthesis of tert-butyl (R)-3-((S)-1-(tert-butoxy)-3-(3-methoxy-5-(4,4,5,5-tetramethyl-1,3,2-dioxaborolan-2-yl)phenyl)-1-oxopropan-2-yl)pyrrolidine-1-carboxylate

*Tert-*butyl (R)-3-((S)-3-(3-bromo-5-methoxyphenyl)-1-(tert-butoxy)-1-oxopropan-2-yl)pyrrolidine-1-carboxylate (2.9g, 6.0mmol, 1eq) was dissolved in 1,4-dioxane solution (30mL), bis(pinacolato)diboron (3.04g, 11.97mmol, 2eq), potassium acetate (1.76g, 17.96mmol, 3eq) and [1,1'-bis(diphenylphosphino)ferrocene]palladium(II) dichloride (439.24mg, 0.6mmol, 0.1eq) were added; the reaction mixture was heated to 90°C under nitrogen protection and stirred at this temperature for 16h. LCMS showed the reaction was completed. Water was added, and the reaction mixture was extracted with ethyl acetate, the organic phases were combined, washed with saturated sodium chloride solution, filtered, dried over anhydrous sodium sulfate, concentrated, and separated and purified by rapid chromatography (silica gel, petroleum ether:tetrahydrofuran=10:1) to give the target compound (2.9g). LCMS(ESI)[M+Na]⁺=554.1; ¹HNMR(400MHz, DMSO-*d₆*) δ 7.11 (s, 1H), 6.98 (s, 1H), 6.88 (s, 1H), 3.73 (s, 3H), 3.58-3.48 (m, 1H), 3.38-3.32 (m, 2H), 3.18-3.11 (m, 1H), 3.00 (t, *J*=10.0Hz, 1H), 2.76-2.67 (m, 2H), 2.31-2.23 (m, 1H), 1.87-1.78 (m, 1H), 1.63-1.53 (m, 1H), 1.40 (s, 9H), 1.28 (d, *J*=3.6Hz, 12H), 1.25 (s, 9H).

### Step 7: Synthesis of tert-butyl (R)-3-((S)-1-(tert-butoxy)-3-(3-hydroxy-5-methoxyphenyl)-1-oxopropan-2-yl)pyrrolidine-1-carboxylate

*Tert-*butyl (R)-3-((S)-1-(tert-butoxy)-3-(3-methoxy-5-(4,4,5,5-tetramethyl-1,3,2-dioxaborolan-2-yl)phenyl)-1-oxopropan-2-yl)pyrrolidine-1-carboxylate (3.5g, 6.59mmol, 1eq) was dissolved in methanol (30mL) solution, hydrogen peroxide (7.47g, 65.85mmol, 10eq, 30% content) was added and the mixture was stirred at 25°C for 1h. LCMS showed the reaction was completed. The reaction mixture was added with water and extracted with ethyl acetate. The organic phases were combined, washed with saturated sodium chloride solution, dried over anhydrous sodium sulfate, filtered, and concentrated to give the crude product, which was separated and purified by rapid chromatography (silica gel, petroleum ether:tetrahydrofuran=5:1) to give the target compound (2.0g). LCMS(ESI)[M+Na]⁺=444.2; ¹HNMR(400MHz, DMSO-*d₆*) δ 10.21 (s, 1H), 6.27-6.08 (m, 3H), 3.67 (d, *J*=9.9Hz, 3H), 3.54-3.46 (m, 2H), 3.18-3.11 (m, 1H), 3.00-2.93 (m, 1H), 2.60 (d, *J*=8.4Hz, 2H), 2.46-2.39 (m, 1H), 2.29-2.20 (m, 1H), 1.87-1.80 (m, 1H), 1.61-1.53 (m, 1H), 1.40 (s, 9H), 1.28 (s, 9H).

### Step 8: Synthesis of tert-butyl (R)-3-((S)-3-(3-(2-(((benzyloxy)carbonyl)amino)ethoxy)-5-methoxyphenyl)-1-(tert-butoxy)-1-oxopropan-2-yl)pyrrolidine-1-carboxylate

*Tert*-butyl (R)-3-((S)-1-(tert-butoxy)-3-(3-hydroxy-5-methoxyphenyl)-1-oxopropan-2-yl)pyrrolidine-1-carboxylate (1g, 2.37mmol, 1eq) was dissolved in N,N-dimethylformamide (10mL) solution, benzyl (2-bromoethyl)carbamate (1.22g, 4.74mmol, 2eq) and potassium carbonate (983.63mg, 7.12mmol, 3eq) were added. The reaction mixture was stirred at 80°C for 18h under nitrogen protection. LCMS showed the reaction was completed. The reaction mixture was added with water and extracted with ethyl acetate. The organic phases were combined, washed with saturated sodium chloride solution, dried over anhydrous sodium sulfate, filtered, and concentrated to give the crude product, which was separated and purified by reversed-phase column chromatography (C₁₈, 0.1% formyl, acetonitrile:water=3:1) to give the target compound (380mg). LCMS(ESI)[M+Na]⁺=621.5; ¹HNMR(400MHz, DMSO-*d₆*) δ 7.35 (d, *J*=2.9Hz, 5H), 6.38-6.31 (m, 3H), 5.03 (s, 2H), 3.94 (t, *J*=5.7Hz, 2H), 3.70 (s, 3H), 3.51-3.42 (m, 3H), 3.16 (d, *J*=6.3Hz, 1H), 2.98 (t, *J*=10.0Hz, 1H), 2.66 (d, *J*=8.1Hz, 2H), 2.30-2.21 (m, 1H), 1.87-1.80 (m, 1H), 1.61-1.54 (m, 1H), 1.40 (s, 9H), 1.26 (s, 9H).

### Step 9: Synthesis of tert-butyl (R)-3-((S)-3-(3-(2-aminoethoxy)-5-methoxyphenyl)-1-(tert-butoxy)-1-oxopropan-2-yl)pyrrolidine-1-carboxylate

*Tert*-butyl (R)-3-((S)-3-(3-(2-(((benzyloxy)carbonyl)amino)ethoxy)-5-methoxyphenyl)-1-(tert-butoxy)-1-oxopropan-2-yl)pyrrolidine-1-carboxylate (760mg, 1.27mmol, 1eq) in tetrahydrofuran (10mL) was added with Pd/C (13.52mg, 0.127mmol, 0.1eq, 10%) and the mixture was stirred at room temperature for 18h under a hydrogen atmosphere. LCMS showed the reaction was completed. The mixture was filtered and the filtrate was concentrated to give the target compound (560mg). LCMS(ESI)[M+H]⁺=465.4; ¹HNMR(400MHz, DMSO-*d₆*) δ 6.40-6.30 (m, 3H), 3.92 (t, *J*=5.4Hz, 2H), 3.70 (s, 3H), 3.59 (d, *J*=6.3Hz, 1H), 3.52-3.45 (m, 2H), 3.16 (d, *J*=6.8Hz, 1H), 3.00-2.89 (m, 3H), 2.66 (d, *J*=8.0Hz, 2H), 2.28-2.19 (m, 1H), 1.87-1.79 (m, 1H), 1.78-1.74 (m, 1H), 1.40 (s, 9H), 1.27 (s, 9H).

### Step 10: Synthesis of tert-butyl (R)-3-((S)-1-(tert-butoxy)-3-(3-formyl-5-methoxyphenyl)-1-oxopropan-2-yl)pyrrolidine-1-carboxylate

*Tert-*butyl (R)-3-((S)-3-(3-bromo-5-methoxyphenyl)-1-(tert-butoxy)-1-oxopropan-2-yl)pyrrolidine-1-carboxylate (1000mg, 2.1mmol, 1eq) was dissolved in N,N-dimethylformamide (50mL), sodium carbonate (437.6mg, 4.1mmol, 2eq), triethylsilane (0.49mL, 3.1mmol, 1.5eq) and [1,1'-bis(diphenylphosphino)ferrocene]palladium(II) dichloride (151.5mg, 0.2mmol, 0.1eq) were added; the reaction mixture was stirred at 80°C for 18h under a carbon monoxide atmosphere. The reaction mixture was added with water and extracted with ethyl acetate. The organic phases were combined, washed with saturated saline, dried over anhydrous sodium sulfate, filtered, and concentrated to give a crude product, which was purified by column chromatography (silica gel, petroleum ether:ethyl acetate=5:1) to give the target compound (700mg). LCMS(ESI)[M+H-Boc]⁺=334.3; ¹HNMR(400MHz, CDCl₃) δ 9.93 (s, 1H), 7.31-7.27 (m, 1H), 7.26-7.22 (m, 1H), 6.99 (s, 1H), 3.85 (s, 3H), 3.75-3.44 (m, 2H), 3.30-3.21 (m, 1H), 3.10-2.79 (m, 3H), 2.54-2.35 (m, 2H), 1.99-1.91 (m, 1H), 1.75-1.65 (m, 1H), 1.47 (s, 9H), 1.29 (s, 9H).

### Step 11: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)-5-methoxyphenoxy)ethyl)amino)methyl)-5-methoxyphenyl)-1-oxopropan-2-yl)pyrrolidine-1-carboxylate

*Tert-*butyl (R)-3-((S)-1-(tert-butoxy)-3-(3-formyl-5-methoxyphenyl)-1-oxopropan-2-yl)pyrrolidine-1-carboxylate (420mg, 0.97mmol, 1eq) was dissolved in ethanol (5mL) solution, *tert-*butyl (R)-3-((S)-3-(3-(2-aminoethoxy)-5-methoxyphenyl)-1-(tert-butoxy)-1-oxopropan-2-yl)pyrrolidine-1-carboxylate(495.09mg, 1.07mmol, 1.1eq) and titanium tetraisopropanolate (0.55mL, 1.94mmol, 2eq) were added. The reaction mixture was stirred at room temperature for 17h. The reaction mixture was cooled in an ice bath, and sodium borohydride (54.85mg, 1.45mmol, 1.5eq) was added. The mixture was stirred at room temperature for 1h. LCMS showed the reaction was completed. Ammonium chloride was added to quench the reaction. The mixture was filtered through celite, concentrated, and separated and purified by rapid chromatography (silica gel, petroleum ether:tetrahydrofuran=3:2) to give the target compound (300mg). LCMS(ESI)[M+H]⁺=882.5; ¹HNMR(400MHz, DMSO-*d₆*) δ 6.74 (d, *J*=14.8Hz, 2H), 6.61 (s, 1H), 6.37-6.29 (m, 3H), 4.02-3.92 (m, 2H), 3.75-3.66 (m, 8H), 3.62-3.58 (m, 6H), 3.52-3.44 (m, 2H), 3.17-3.08 (m, 2H), 2.96 (t, *J*=10.0Hz, 2H), 2.80 (t, *J*=5.7Hz, 2H), 2.68-2.65 (m, 2H), 2.30-2.22 (m, 2H), 1.86-1.79 (m, 2H), 1.61-1.52 (m, 2H), 1.39 (s, 18H), 1.25 (d, *J*=5.2Hz, 18H).

### Step 12: Synthesis of tert-butyl (R)-3-((S)-3-(3-allyl-5-methoxyphenyl)-1-(tert-butoxy)-1-oxopropan-2-yl)pyrrolidine-1-carboxylate

Under nitrogen protection, *tert-*butyl (R)-3-((S)-3-(3-bromo-5-methoxyphenyl)-1-(tert-butoxy)-1-oxopropan-2-yl)pyrrolidine-1-carboxylate (1.5g, 3.1mmol, 1eq) was dissolved in tetrahydrofuran (20mL) and water (5mL), followed by adding 2-allyl-4,4,5,5-tetramethyl-1,3,2-dioxaborolane (1040.67mg, 6.19mmol, 2eq), potassium phosphate (1971.87mg, 9.3mmol, 3eq) and [1,1'-bis(diphenylphosphino)ferrocene]palladium(II) dichloride (227.2mg, 0.31mmol, 0.1eq); the reaction mixture was stirred at 80°C for 2h. The mixture was diluted with water, extracted with ethyl acetate, and the organic phases were combined, washed with saturated sodium chloride aqueous solution, dried over anhydrous sodium sulfate, filtered, and concentrated to give a crude product, which was separated and purified by rapid chromatography (silica gel, petroleum ether:tetrahydrofuran=10:1) to give the target compound (1.2g). LCMS(ESI)[M+Na]⁺=468.4.

### Step 13: Synthesis of 2-(3-((S)-3-(tert-butoxy)-2-((R)-1-(tert-butoxycarbonyl)pyrrolidin-3-yl)-3-oxopropyl)-5-methoxyphenyl)acetic acid

*Tert-*butyl (R)-3-((S)-3-(3-allyl-5-methoxyphenyl)-1-(tert-butoxy)-1-oxopropan-2-yl)pyrrolidine-1-carboxylate (1g) was dissolved in acetonitrile (10mL) and water (10mL), sodium periodate (3.36g) and ruthenium trichloride(46.55mg) were added at room temperature; and the mixture was stirred for 3h at room temperature. The mixture was diluted with acetonitrile, filtered, and the filter cake was washed with acetonitrile. The filtrate was concentrated, diluted with water and extracted with ethyl acetate. The organic phases were combined, washed with saturated sodium chloride solution, dried over anhydrous sodium sulfate, filtered and concentrated to give the crude product, which was separated and purified by reversed-phase rapid chromatography (C₁₈, 0.1% TFA, acetonitrile:water=1:1) to give the target compound (350mg). LCMS(ESI)[M+H]⁺=464.3.

### Step 14: Synthesis of tert-butyl (R)-3-((S)-1-(tert-butoxy)-3-(3-((N-(2-(3-((S)-3-(tert-butoxy)-2-((R)-1-(tert-butoxycarbonyl)pyrrolidin-3-yl)-3-oxopropyl)-5-methoxyphenoxy)ethyl)-2-(3-((S)-3-(tert-butoxy)-2-((R)-1-(tert-butoxycarbonyl)pyrrolidin-3-yl)-3-oxopropyl)-5-methoxyphenyl)acetamido)methyl)-5-methoxyphenyl)-1-oxopropan-2-yl)pyrrolidine-1-carboxylate

2-(3-((S)-3-(tert-butoxy)-2-((R)-1-(tert-butoxycarbonyl)pyrrolidin-3-yl)-3-oxopropyl)-5-methoxyphenyl)acetic acid (173.42mg, 0.37mmol, 1.1eq) was dissolved in N,N-dimethylformamide (5mL), and O-(7-azabenzotriazol-1-yl)-N,N,N',N'-tetramethyluronium hexafluorophosphate (193.97mg, 0.51mmol, 1.5eq), triethylamine (103.24mg, 1.02mmol, 3eq) and *tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)-5-methoxyphenoxy)ethyl)amino)methyl)-5-methoxyphenyl)-1-oxopropan-2-yl)pyrrolidine-1-carboxylate (300mg, 0.34mmol, 1eq) were added; the reaction mixture was stirred at room temperature for 16h. LCMS showed the reaction was completed. The reaction mixture was added with water and extracted with ethyl acetate. The organic phases were combined, washed with saturated saline, dried over anhydrous sodium sulfate, filtered, and the filtrate was concentrated. The residue was separated and purified by rapid chromatography (silica gel, petroleum ether:tetrahydrofuran=2:1) to give the target compound (230mg). ¹HNMR (400MHz, CDCl₃) δ 6.71-6.45 (m, 6H), 6.37-6.22 (m, 3H), 4.67-4.56 (m, 2H), 3.97-3.85 (m, 2H), 3.80-3.71 (m, 9H), 3.69-3.64 (m, 4H), 3.62-3.43 (m, 6H), 3.31-3.20 (m, 3H), 3.06-2.89 (m, 3H), 2.87-2.67 (m, 6H), 2.52-2.41 (m, 3H), 2.39-2.28 (m, 3H), 1.97-1.89 (m, 3H), 1.71-1.63 (m, 3H), 1.46 (s, 27H), 1.31-1.26 (m, 27H).

Step 15: The product from step 14 (200mg, 0.15mmol, 1eq) was dissolved in dioxane (2mL), hydrochloric acid/dioxane (2mL, 4.0M) was added, and the reaction mixture was stirred at room temperature for 2h. LCMS showed the reaction was completed. The reaction mixture was concentrated, dissolved in water, neutralized with sodium hydroxide (1.0M) aqueous solution, and purified by reversed-phase preparative chromatography (C₁₈, 0.1% ammonium bicarbonate aqueous solution, acetonitrile) to give the target compound (61.33mg). LCMS(ESI)[M+H]⁺=858.8; ¹HNMR(400MHz, D₂O) δ 6.72-6.67 (m, 1H), 6.65-6.53 (m, 3H), 6.52-6.39 (m, 2H), 6.39-5.93 (m, 3H), 4.64-4.48 (m, 2H), 4.13-3.94 (m, 1H), 3.87-3.81 (m, 2H), 3.77 (s, 1H), 3.72-3.59 (m, 8H), 3.57-3.50 (m, 3H), 3.39-3.20 (m, 6H), 3.19-3.04 (m, 3H), 2.87-2.44 (m, 9H), 2.41-2.23 (m, 6H), 2.09-1.93 (m, 3H), 1.72-1.57 (m, 3H).

### Example 82 (Compound 177)

### Preparation of (S)-3-(3-(2-((2-(3-((S)-2-carboxy-2-((R)-pyrrolidin-3-yl)ethyl)phenoxy)ethyl)((4-((S)-2-carboxy-2-((R)-pyrrolidin-3-yl)ethyl)thiophen-2-yl)methyl)amino)-2-oxoethyl)phenyl)-2-((R)-pyrrolidin-3-yl)propanoic acid

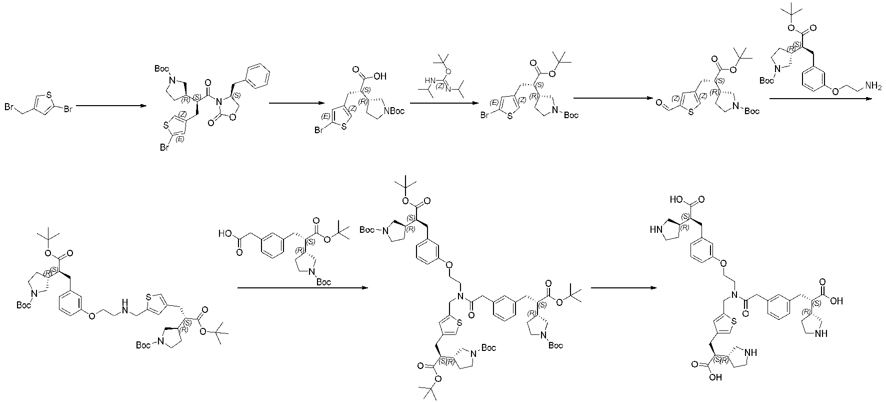

### Step 1: Synthesis of tert-butyl (R)-3-((S)-1-((S)-4-benzyl-2-oxooxazolidin-3-yl)-3-(5-bromothiophen-3-yl)-1-oxopropan-2-yl)pyrrolidine-1-carboxylate

Under nitrogen protection, *tert-butyl* (R)-3-(2-((S)-4-benzyl-2-oxooxazolidin-3-yl)-2-oxoethyl)pyrrolidine-1-carboxylate (10g, 25.74mmol, 1.0eq) was dissolved in tetrahydrofuran (150mL), lithium bis(trimethylsilyl)amide (33.47mL, 33.47mmol, 1.3eq) was added dropwise under ice bath, and the mixture was stirred for 1h; 2-bromo-4-(bromomethyl)thiophene (9.88g, 38.61mmol, 1.5eq) was dissolved in tetrahydrofuran and added dropwise to the above reaction mixture, which was warmed to room temperature and stirred for another 15h. The reaction mixture was quenched with saturated ammonium chloride aqueous solution, diluted with water, extracted with ethyl acetate, and the organic phases were combined, washed with saturated sodium chloride aqueous solution, dried over anhydrous sodium sulfate, filtered and concentrated to give the target compound (20.3g, crude). The crude product was used directly in the next step without further purification. LCMS(ESI)[M+Na]⁺=585.1.

### Step 2: Synthesis of (S)-3-(5-bromothiophen-3-yl)-2-((R)-1-(tert-butoxycarbonyl)pyrrolidin-3-yl)propanoic acid

Under ice bath, *tert-*butyl (R)-3-((S)-1-((S)-4-benzyl-2-oxooxazolidin-3-yl)-3-(5-bromothiophen-3-yl)-1-oxopropan-2-yl)pyrrolidine-1-carboxylate (19.8g, 35.14mmol, 1.0eq) was dissolved in tetrahydrofuran (150mL), followed by adding hydrogen peroxide (39.83g, 351.38mmol, 10eq, 30% content) and a solution of lithium hydroxide monohydrate (2.21g, 52.71mmol, 1.5eq) in water (15mL); the above reaction mixture was stirred at room temperature for 2h. LCMS showed the reaction was completed. The reaction mixture was quenched with saturated sodium sulfite solution, diluted with water, washed with methyl *tert-*butyl ether, and the aqueous phase was adjusted to a pH of about 4 with hydrochloric acid (1.0M), extracted with ethyl acetate. The organic phases were combined, washed with saturated saline, dried over anhydrous sodium sulfate, filtered, and concentrated to give the target compound (7.2g). LCMS(ESI)[M+Na]⁺=426.0.

### Step 3: Synthesis of tert-butyl (R)-3-((S)-3-(5-bromothiophen-3-yl)-1-(tert-butoxy)-1-oxopropan-2-yl)pyrrolidine-1-carboxylate

(S)-3-(5-bromothiophen-3-yl)-2-((R)-1-(tert-butoxycarbonyl)pyrrolidin-3-yl)propanoic acid (7.4g, 18.3mmol, 1.0eq) in 2-methyltetrahydrofuran (100mL) solution was added with *tert-*butyl N,N'-diisopropylcarbamimidate (18.33g, 91.51mmol, 5.0eq); the reaction mixture was heated to 65°C under nitrogen protection and stirred for 16h. The reaction mixture was cooled to room temperature, filtered, and the filter cake was washed with methyl *tert*-butyl ether. The filtrate was concentrated under reduced pressure to give a crude product, which was separated and purified by column chromatography (tetrahydrofuran/petroleum ether=1:5) to give the target compound (4.3g). LCMS(ESI)[M+H]⁺=460.1. ¹HNMR(400MHz, DMSO-*d₆*) δ 7.18 (s, 1H), 7.08 (d, J=3.5Hz, 1H), 3.52-3.41 (m, 1H), 3.38-3.32 (m, 1H), 3.18-3.09 (m, 1H), 2.94 (t, *J*=10.0Hz, 1H), 2.76-2.62 (m, 2H), 2.49-2.45 (m, 1H), 2.28-2.17 (m, 1H), 1.88-1.77 (m, 1H), 1.61-1.51 (m, 1H), 1.40-1.38 (m, 9H), 1.28 (s, 9H).

### Step 4: Synthesis of tert-butyl (R)-3-((S)-1-(tert-butoxy)-3-(5-formylthiophen-3-yl)-1-oxopropan-2-yl)pyrrolidine-1-carboxylate

*Tert-*butyl (R)-3-((S)-3-(5-bromothiophen-3-yl)-1-(tert-butoxy)-1-oxopropan-2-yl)pyrrolidine-1-carboxylate (1g, 2.17mmol, 1.0eq) was dissolved in *N,N*-dimethylformamide (12mL), followed by adding sodium carbonate (460.4mg, 4.34mmol, 2.0eq), [1,1'-bis(diphenylphosphino)ferrocene]palladium(II) dichloride (159.36mg, 0.22mmol, 0.1eq) and triethylsilane (505.09mg, 4.34mmol, 2.0eq); after replacing with carbon monoxide, the reaction mixture was stirred at 80°C for 16h. LCMS showed the reaction was completed. The reaction mixture was added with aqueous solution, extracted with ethyl acetate. The organic phases were combined, washed with saturated saline solution, dried over anhydrous sodium sulfate, filtered and concentrated to give a crude product, which was separated and purified by column chromatography (tetrahydrofuran/petroleum ether=1:5) to give the target compound (800mg). LCMS(ESI)[M+Na]⁺=432.2. ¹HNMR(400MHz, DMSO-*d₆*) δ 9.89 (d, *J*=1.2Hz, 1H), 7.89 (s, 1H), 7.80 (s, 1H), 3.55-3.44 (m, 1H), 3.39-3.33 (m, 1H), 3.21-3.09 (m, 1H), 2.98 (t, *J*=9.9Hz, 1H), 2.84-2.74 (m, 2H), 2.62-2.54 (m, 1H), 2.35-2.21 (m, 1H), 1.90-1.80 (m, 1H), 1.65-1.52 (m, 1H), 1.40 (s, 9H), 1.26 (s, 9H).

### Step 5: Synthesis of tert-butyl (R)-3-((S)-1-(tert-butoxy)-3-(3-(2-(((4-((S)-3-(tert-butoxy)-2-((R)-1-(tert-butoxycarbonyl)pyrrolidin-3-yl)-3-oxopropyl)thiophen-2-yl)methyl)amino)ethoxy)phenyl)-1-oxopropan-2-yl)pyrrolidine-1-carboxylate

*Tert-*butyl (R)-3-((S)-1-(tert-butoxy)-3-(5-formylthiophen-3-yl)-1-oxopropan-2-yl)pyrrolidine-1-carboxylate (400mg, 0.98mmol, 1.0eq) and *tert-*butyl (R)-3-((S)-3-(3-(2-aminoethoxy)phenyl)-1-(tert-butoxy)-1-oxopropan-2-yl)pyrrolidine-1-carboxylate(466.89mg, 1.07mmol, 1.1eq) in ethanol (20mL) solution was added with titanium tetraisopropanolate (555.2mg, 1.95mmol, 2.0eq); the reaction mixture was stirred at room temperature for 14h, sodium borohydride (110.85mg, 2.93mmol, 3.0eq) was added to the reaction system, and the reaction mixture was stirred at room temperature for another 2h. The reaction mixture was added with saturated ammonium chloride (0.5mL), filtered through celite, and the filtrate was dried over anhydrous sodium sulfate, filtered and concentrated to give a crude product, which was purified by column chromatography (petroleum ether:tetrahydrofuran=1:3) to give the target compound (800mg). LCMS(ESI)[M+H]⁺=828.6.

Step 6: Synthesis of *tert-*butyl (R)-3-((S)-1-(tert-butoxy)-3-(3-(2-((2-(3-((S)-3-(tert-butoxy)-2-((R)-1-(tert-butoxycarbonyl)pyrrolidin-3-yl)-3-oxopropyl)phenoxy)ethyl)((4-((S)-3-(tert-butoxy)-2-((R)-1-(tert-butoxycarbonyl)pyrrolidin-3-yl)-3-oxopropyl)thiophen-2-yl)methyl)amino)-2-oxoethyl)phenyl)-1-oxopropan-2-yl)pyrrolidine-1-carboxylate 2-(3-((S)-3-(tert-butoxy)-2-((R)-1-(tert-butoxycarbonyl)pyrrolidin-3-yl)-3-oxopropyl)phenyl)acetic acid(190mg, 0.44mmol, 1.0eq) and *tert-*butyl (R)-3-((S)-1-(tert-butoxy)-3-(3-(2-(((4-((S)-3-(tert-butoxy)-2-((R)-1-(tert-butoxycarbonyl)pyrrolidin-3-yl)-3-oxopropyl)thiophen-2-yl)methyl)amino)ethoxy)phenyl)-1-oxopropan-2-yl)pyrrolidine-1-carboxylate (399.21mg, 0.48mmol, 1.1eq) in *N,N-*dimethylformamide (15mL) solution was added with *N,N*-diisopropylethylamine (169.92mg, 1.31mmol, 3.0eq) and O-(7-azabenzotriazol-1-yl)-N,N,N',N'-tetramethyluronium hexafluorophosphate (249.96mg, 0.66mmol, 1.5eq) at room temperature; the reaction mixture was stirred at room temperature for 16h. The reaction mixture was added with water, extracted with ethyl acetate, and the organic phases were combined, washed with water and saturated saline respectively, dried over anhydrous sodium sulfate, filtered and concentrated to give a crude product, which was separated and purified by reversed-phase column chromatography (C₁₈, 0.1% trifluoroacetic acid, water/acetonitrile=3:1) to give the target compound (400mg). LCMS(ESI)[M+H-Boc]⁺=1243.7.

Step 7: The product from step 6 (400mg, 0.32mmol, 1.0eq) was dissolved in hydrochloric acid/1,4-dioxane (15mL, 4.0M) solution, and the reaction mixture was stirred at room temperature for 4h. LCMS showed the reaction was completed. The reaction mixture was concentrated under reduced pressure to give the crude product which was adjusted to a pH of 7-8 with sodium hydroxide (1.0M) solution; and the mixture was separated and purified by reversed-phase preparative chromatography (C₁₈, 0.03% ammonium bicarbonate, water/acetonitrile=8:1) to give the target compound (151.43mg). LCMS(ESI)[M+H]⁺=775.4; ¹HNMR(400MHz, D₂O) δ 7.24-7.14 (m, 2H), 7.09-7.04 (m, 1H), 7.01-6.86 (m, 3H), 6.81 (d, *J*=7.4Hz, 1H), 6.76-6.62 (m, 3H), 4.12-4.02 (m, 2H), 3.82-3.69 (m, 4H), 3.41-3.07 (m, 10H), 2.88-2.45 (m, 10H), 2.42-2.26 (m, 6H), 2.07-1.97 (m, 3H), 1.71-1.57 (m, 3H).

### Example 83 (Compound 178)

### Preparation of ((S)-3-(5-(2-((3-((S)-2-carboxy-2-((R)-pyrrolidin-3-yl)ethyl)benzyl)(2-(3-((S)-2-carboxy-2-((R)-pyrrolidin-3-yl)ethyl)phenoxy)ethyl)amino)-2-oxoethyl)thiophen-3-yl)-2-((R)-pyrrolidin-3-yl)propanoic acid)

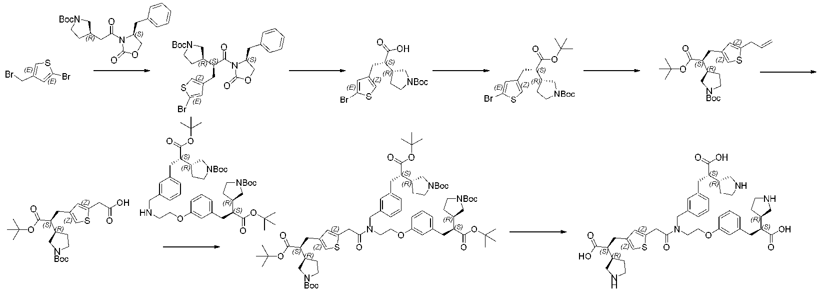

### Step 1: tert-butyl (R)-3-((S)-1-((S)-4-benzyl-2-oxooxazolidin-3-yl)-3-(5-bromothiophen-3-yl)-1-oxopropan-2-yl)pyrrolidine-1-carboxylate

Under nitrogen protection and ice bath, a solution of lithium bis(trimethylsilyl)amide (11.72mL, 1.0 M in THF) was added dropwise to a solution of *tert-butyl* (R)-3-(2-((S)-4-benzyl-2-oxooxazolidin-3-yl)-2-oxoethyl)pyrrolidine-1-carboxylate (4.56g) in tetrahydrofuran. The reaction mixture was stirred at 0°C for 30 min, and a solution of 2-bromo-4-(bromomethyl)thiophene (3g) in tetrahydrofuran was added. The reaction mixture was slowly warmed to room temperature and stirred for 16h. LCMS showed the reaction was completed. A saturated solution of ammonium chloride was added, and the mixture was extracted with ethyl acetate. The organic phase was washed with saturated sodium chloride aqueous solution, dried over anhydrous sodium sulfate, filtered, and concentrated to give the target compound (5.0g). LCMS(ESI)[M+H-*tert*-butyl]⁺=507.17.

### Step 2: Synthesis of (S)-3-(5-bromothiophen-3-yl)-2-((R)-1-(tert-butoxycarbonyl)pyrrolidin-3-yl)propanoic acid

*Tert-*butyl (R)-3-((S)-1-((S)-4-benzyl-2-oxooxazolidin-3-yl)-3-(5-bromothiophen-3-yl)-1-oxopropan-2-yl)pyrrolidine-1-carboxylate (5g) was dissolved in tetrahydrofuran (50mL), lithium hydroxide (0.318g) and hydrogen peroxide (0.452g) were added. The mixture was stirred at room temperature for 16h. LCMS showed the consumption of starting material was completed. The solvent was removed by rotary-evaporation to dryness and added with water. The mixture was extracted with ethyl acetate, and the organic phase was dried over anhydrous sodium sulfate, filtered, and concentrated to give a crude product, which was purified by column chromatography (petroleum ether:ethyl acetate=2:1) to give the target compound (3.5g). LCMS(ESI)[M+H-Boc]⁺=304.04.

### Step 3: Synthesis of tert-butyl (R)-3-((S)-3-(5-bromothiophen-3-yl)-1-(tert-butoxy)-1-oxopropan-2-yl)pyrrolidine-1-carboxylate

(S)-3-(5-bromothiophen-3-yl)-2-((R)-1-(tert-butoxycarbonyl)pyrrolidin-3-yl)propanoic acid (3.5g) was dissolved in tetrahydrofuran (25mL), and *tert-*butyl N,N'-diisopropylcarbamimidate (8.66g) was added. The mixture was heated to 65°C under nitrogen protection and stirred for 16h. LCMS showed the reaction was completed. The insoluble mateirals were filtered off, and the filter cake was washed with methyl *tert-*butyl ether. The filtrate was concentrated to give a crude product, which was separated and purified by rapid chromatography (silica gel, petroleum ether:ethyl acetate=10:1) to give the target compound (2.50g). LCMS(ESI)[M+H-2×*tert*-butyl]⁺=348.03. Step 4: Synthesis of *tert-*butyl (R)-3-((S)-3-(5-allylthiophen-3-yl)-1-(tert-butoxy)-1-oxopropan-2-yl)pyrrolidine-1-carboxylate

Under nitrogen protection, *tert-*butyl (R)-3-((S)-3-(5-bromothiophen-3-yl)-1-(tert-butoxy)-1-oxopropan-2-yl)pyrrolidine-1-carboxylate (2g), [1,1'-bis(diphenylphosphino)ferrocene]palladium(II) dichloride (310mg) and potassium phosphate (2690mg) were dissolved in water (2mL) and dioxane (10mL), and 2-allyl-4,4,5,5-tetramethyl-1,3,2-dioxaborolane (1420mg) was added. The mixture was stirred at 90°C for 16h under nitrogen protection, and LCMS showed the reaction was completed. The reaction mixture was cooled to room temperature, and ethyl acetate and water were added, and the mixture was extracted. The organic phase was dried over anhydrous sodium sulfate and concentrated to give a crude product, which was purified by column chromatography (silica gel, petroleum ether:ethyl acetate=5:1) to give the target compound (750mg). LCMS(ESI)[M+H-2×*tert*-butyl]⁺=310.19.

### Step 5: Synthesis of 2-(4-((S)-3-(tert-butoxy)-2-((R)-1-(tert-butoxycarbonyl)pyrrolidin-3-yl)-3-oxopropyl)thiophen-2-yl)acetic acid

*Tert-*butyl (R)-3-((S)-3-(5-allylthiophen-3-yl)-1-(tert-butoxy)-1-oxopropan-2-yl)pyrrolidine-1-carboxylate(500mg) was dissolved in acetonitrile (2mL) and water (2mL), sodium periodate (1016mg) and ruthenium trichloride (16mg) were added, and the mixture was stirred at room temperature overnight; LCMS showed the reaction was completed. Ethyl acetate and water were added, and the mixture was extracted, and the organic phase was dried over anhydrous sodium sulfate, concentrated to give a crude product, which was purified by column chromatography (silica gel, petroleum ether:ethyl acetate=5:1) to give the target compound (250mg). LCMS(ESI)[M+H-Boc]⁺=340.25.

### Step 6: Synthesis of tert-butyl (R)-3-((S)-1-(tert-butoxy)-3-(3-((N-(2-(3-((S)-3-(tert-butoxy)-2-((R)-1-(tert-butoxycarbonyl)pyrrolidin-3-yl)-3-oxopropyl)phenoxy)ethyl)-2-(4-((S)-3-(tert-butoxy)-2-((R)-1-(tert-butoxycarbonyl)pyrrolidin-3-yl)-3-oxopropyl)thiophen-2-yl)acetamido)methyl)phenyl)-1-oxopropan-2-yl)pyrrolidine-1-carboxylate

*Tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (460mg) was dissolved in N,N-dimethylformamide (5mL), followed by adding 2-(4-((S)-3-(tert-butoxy)-2-((R)-1-(tert-butoxycarbonyl)pyrrolidin-3-yl)-3-oxopropyl)thiophen-2-yl)acetic acid (250mg), O-(7-azabenzotriazol-1-yl)-N,N,N',N'-tetramethyluronium hexafluorophosphate (320mg) and *N*,*N*-diisopropylethylamine (215mg). The mixture was stirred at room temperature for 16h, and LCMS showed the reaction was completed. Ethyl acetate and water were added, and the mixture was extracted. The organic phase was dried over anhydrous sodium sulfate and concentrated to give a crude product, which was separated and purified by rapid chromatography (silica gel, petroleum ether:ethyl acetate=1:8) to give the target compound (150mg). LCMS(ESI)[M+H-Boc]⁺=1143.91.

Step 7: The product from step 6 (150mg) was dissolved in 1,4-dioxane (2mL), hydrochloric acid/1,4-dioxane (1mL, 4.0M) was added, and the mixture was stirred at room temperature overnight. LCMS showed the reaction was completed. The solvent was removed by rotary-evaporation to dryness, and the residue was separated and purified by Prep-HPLC (C₁₈, 10mmol/L NH₄HCO₃ in water, MeCN) to give the target compound (23mg). LCMS(ESI)[M+H]⁺=775.56; ¹HNMR(400MHz, D₂O) δ 7.32-6.51 (m, 10H), 4.12-3.99 (m, 3H), 3.87-3.66 (m, 3H), 3.52-3.03 (m, 10H), 2.92-2.22 (m, 16H), 2.10-1.94 (m, 3H), 1.72-1.56 (m, 3H).

### Example 84 (Compound 180)

### Preparation of (S)-3-(5-(2-((2-(3-((S)-2-carboxy-2-((R)-pyrrolidin-3-yl)ethyl)phenoxy)ethyl)((4-((S)-2-carboxy-2-((R)-pyrrolidin-3-yl)ethyl)thiophen-2-yl)methyl)amino)-2-oxoethyl)thiophen-3-yl)-2-((R)-pyrrolidin-3-yl)propanoic acid

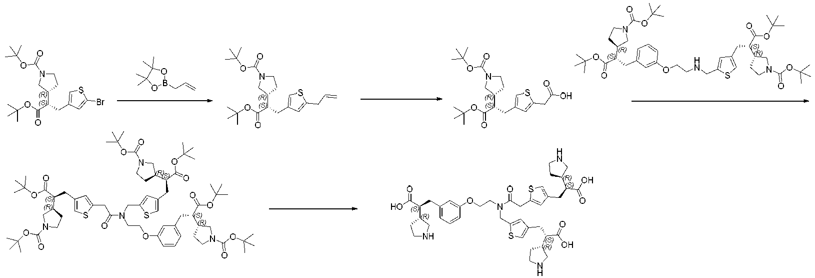

### Step 1: Synthesis of tert-butyl (R)-3-((S)-3-(5-allylthiophen-3-yl)-1-(tert-butoxy)-1-oxopropan-2-yl)pyrrolidine-1-carboxylate

Under nitrogen protection, *tert-*butyl (R)-3-((S)-3-(5-bromothiophen-3-yl)-1-(tert-butoxy)-1-oxopropan-2-yl)pyrrolidine-1-carboxylate(2.0g, 4.34mmol, 1.0eq) was dissolved in 1,4-dioxane (30mL) and water (6mL), followed by adding potassium phosphate (2.77g, 13.03mmol, 3.0eq), [1,1'-bis(diphenylphosphino)ferrocene]palladium(II) dichloride (318.71mg, 0.43mmol, 0.1eq) and 2-allyl-4,4,5,5-tetramethyl-1,3,2-dioxaborolane(1.46g, 8.69mmol, 2.0eq), and the reaction mixture was stirred at 90°C for 16h. Water was added, and the mixture was extracted with ethyl acetate. The organic phases were combined, washed with saturated sodium chloride aqueous solution, dried over anhydrous sodium sulfate, filtered and concentrated to give a crude product, which was separated and purified by rapid chromatography (silica gel, petroleum ether:ethyl acetate=9:1) to give the target compound (1.6g). LCMS(ESI)[M+Na]⁺=444.4; ¹HNMR(400MHz, DMSO-*d*₆) δ 6.93 (s, 1H), 6.69 (s, 1H), 6.01-5.85 (m, 1H), 5.09 (dd, *J*=23.0, 13.5Hz, 2H), 3.55-3.42 (m, 3H), 3.39-3.34 (m, 1H), 3.19-3.07 (m, 1H), 2.94 (t, *J*=10.0Hz, 1H), 2.76-2.58 (m, 2H), 2.48-2.40 (m, 1H), 2.27-2.15 (m, 1H), 1.88-1.77 (m, 1H), 1.64-1.52 (m, 1H), 1.39 (t, *J*=2.8Hz, 9H), 1.28 (s, 9H).

### Step 2: Synthesis of 2-(4-((S)-3-(tert-butoxy)-2-((R)-1-(tert-butoxycarbonyl)pyrrolidin-3-yl)-3-oxopropyl)thiophen-2-yl)acetic acid

At room temperature, the product from step 1 (1.6g, 3.8mmol, 1.0eq) was dissolved in acetonitrile (20mL) and water (20mL), ruthenium trichloride (39.36mg, 0.19mmol, 0.05eq) and sodium periodate (4.06g, 18.98mmol, 5.0eq) were added; the mixture was stirred at room temperature for 2h. LCMS showed there was no remaining starting materials. Water was added, and the mixture was extracted with ethyl acetate. The organic phases were combined and washed with sodium thiosulfate solution and saturated sodium chloride aqueous solution in sequence. The organic phase was dried over anhydrous sodium sulfate, filtered, and concentrated to give a crude product, which was separated and purified by reversed-phase column chromatography (C₁₈, 10 mmol NH₄HCO₃, water/acetonitrile=1:3) to give the target compound (100mg). LCMS(ESI)[M+Na]⁺=462.3.

### Step 3: Synthesis of tert-butyl (R)-3-((S)-1-(tert-butoxy)-3-(3-(2-(2-(4-((S)-3-(tert-butoxy)-2-((R)-1-(tert-butoxycarbonyl)pyrrolidin-3-yl)-3-oxopropyl)thiophen-2-yl)-N-((4-((S)-3-(tert-butoxy)-2-((R)-1-(tert-butoxycarbonyl)pyrrolidin-3-yl)-3-oxopropyl)thiophen-2-yl)methyl)acetamido)ethoxy)phenyl)-1-oxopropan-2-yl)pyrrolidine-1-carboxylate

The product from step 2 (80mg, 0.18mmol, 1.0eq) was dissolved in N,N-dimethylformamide (5mL), followed by adding triethylamine (92.08mg, 0.91mmol, 5eq), O-(7-azabenzotriazol-1-yl)-N,N,N',N'-tetramethyluronium hexafluorophosphate (138.4mg, 0.36mmol, 2eq) and *tert-*butyl (R)-3-((S)-1-(tert-butoxy)-3-(3-(2-(((4-((S)-3-(tert-butoxy)-2-((R)-1-(tert-butoxycarbonyl)pyrrolidin-3-yl)-3-oxopropyl)thiophen-2-yl)methyl)amino)ethoxy)phenyl)-1-oxopropan-2-yl)pyrrolidine-1-carboxylate(150.71mg, 0.18mmol, 1.0eq) at room temperature; the reaction mixture was stirred at room temperature for 16h. Water was added, and the mixture was extracted with ethyl acetate. The organic phases were combined, washed with saturated sodium chloride aqueous solution, dried over anhydrous sodium sulfate, filtered, and concentrated to give a crude product, which was separated and purified by reversed-phase column chromatography (C₁₈, 10 mM NH₄HCO₃, water/acetonitrile=1:8) to give the target compound (80mg). LCMS(ESI)[M+H]⁺=1249.4; ¹HNMR(400MHz, DMSO-*d*₆) δ 7.17 (dt, *J=*11.5, 8.2Hz, 1H), 7.09-6.95 (m, 2H), 6.84 (s, 1H), 6.79-6.69 (m, 4H), 4.75 (d, *J*=57.4Hz, 2H), 4.14-3.90 (m, 4H), 3.71-3.58 (m, 2H), 3.56-3.42 (m, 3H), 3.36 (s, 3H), 3.20-3.08 (m, 3H), 3.03-2.82 (m, 3H), 2.74-2.55 (m, 6H), 2.48-2.37 (m, 3H), 2.24 (s, 3H), 1.83 (s, 3H), 1.64-1.51 (m, 3H), 1.39 (s, 27H), 1.29-1.22 (m, 27H).

Step 4: The product from step 3 (80mg, 0.06mmol, 1.0eq) was dissolved in hydrochloric acid/1,4-dioxane (3mL, 4.0M). The reaction mixture was stirred at room temperature for 16h. The reaction mixture was concentrated to dryness, dissolved in water, adjusted to pH=7 with sodium hydroxide aqueous solution (1.0M). The crude product was separated and purified by preparative column chromatography (C₁₈, 0.1% NH₄HCO₃, water/acetonitrile=10:1) to give the target compound (25mg). LCMS(ESI)[M+H]⁺=781.3; ¹HNMR(400MHz, D₂O) δ 7.21-7.13 (m, 1H), 6.92 (d, *J*=5.4Hz, 1H), 6.87 (d, *J=*11.8Hz, 1H), 6.80 (d, *J*=7.6Hz, 1H), 6.75-6.63 (m, 4H), 4.64 (s, 2H), 4.06 (dt, *J=*16.4, 4.9Hz, 2H), 3.92 (d, *J*=24.2Hz, 2H), 3.77-3.66 (m, 2H), 3.39-3.23 (m, 6H), 3.20-3.07 (m, 3H), 2.87-2.48 (m, 9H), 2.43-2.22 (m, 6H), 2.08-1.96 (m, 3H), 1.71-1.57 (m, 3H).

### Example 85 (Compound 190)

### Preparation of (S)-3-(3-(2-((3-((S)-2-carboxy-2-((R)-pyrrolidin-3-yl)ethyl-1,1-d₂)benzyl)(2-(3-((S)-2-carboxy-2-((R)-pyrrolidin-3-yl)ethyl-1,1-d₂)phenoxy)ethyl)amino)-2-oxoethyl)phenyl)-2-((R)-pyrrolidin-3-yl)propanoic-3,3-d2 acid

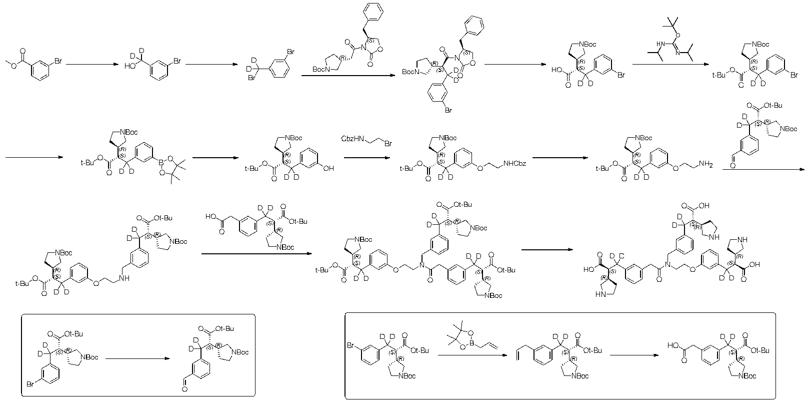

### Step 1: Synthesis of (3-bromophenyl)methan-d2-ol

At 0°C, a solution of methyl 3-bromobenzoate (20g, 93.01mmol, 1eq) in tetrahydrofuran (100mL) was added dropwise to the solution of lithium aluminium deuteride (2.34g, 55.80mmol, 0.6eq) in tetrahydrofuran (100mL). After stirring at 0°C for 1.5 h, the reaction was quenched with sodium sulfate decahydrate, filtered through celite, concentrated and purified by column chromatography (silica gel, petroleum ether:ethyl acetate=5:1) to give the target compound (14.7g). LCMS(ESI)[M+H-H₂O]⁺=171.0; ¹HNMR(400MHz, DMSO-*d₆*) δ 7.53-7.48 (m, 1H), 7.44-7.40 (m, 1H), 7.32-7.28 (m, 2H), 5.27 (s, 1H).

### Step 2: Synthesis of 1-bromo-3-(bromomethyl-d₂)benzene

(3-bromophenyl)methan-d2-ol (14.7g, 77.76mmol, 1eq) was dissolved in diethyl ether (150mL), phosphorus tribromide (5.12mL, 54.43mmol, 0.7eq) was added at 0°C; the reaction mixture was stirred at 40°C for 2h. Upon completion of the reaction, the reaction mixture was slowly poured into water and extracted with ethyl acetate. The organic phases were combined, dried over anhydrous sodium sulfate, filtered, and concentrated to give a crude product, which was purified by column chromatography (silica gel, petroleum ether:ethyl acetate=10:1) to give the target compound (17g). LCMS(ESI)[M +Na]⁺=273; ¹HNMR(400MHz, DMSO-*d₆*) δ 7.68 (t, *J*=1.8Hz, 1H), 7.53-7.50 (m, 1H), 7.47-7.44 (m, 1H), 7.33 (t, *J*=7.8Hz, 1H).

### Step 3: Synthesis of tert-butyl (R)-3-((S)-1-((S)-4-benzyl-2-oxooxazolidin-3-yl)-3-(3-bromophenyl)-1-oxopropan-2-yl-3,3-d₂)pyrrolidine-1-carboxylate

Under nitrogen protection and ice bath, a solution of lithium bis(trimethylsilyl)amide (58.69mL, 58.69mmol, 1.0 M in THF, 1.2eq) was added dropwise to the solution of *tert-*butyl (R)-3-(2-((S)-4-benzyl-2-oxooxazolidin-3-yl)-2-oxoethyl)pyrrolidine-1-carboxylate(19g, 48.91mmol, 1eq) in tetrahydrofuran (150mL). The mixture was stirred at 0°C for 30 min, then a solution of 1-bromo-3-(bromomethyl-*d₂*)benzene (12.32g, 48.97mmol, 1eq) in tetrahydrofuran (50mL) was added slowly. The reaction temperature was slowly raised to room temperature and the mixture was stirred for 18h. The reaction mixture was cooled with an ice-water bath, and the saturated ammonium chloride aqueous solution was added; then, water was added, and the mixture was extracted with ethyl acetate. The organic phases were combined, washed with saturated sodium chloride aqueous solution, dried over anhydrous sodium sulfate, filtered, and concentrated to give the target compound (25g, crude). LCMS(ESI)[M+H-*tert*-butyl]⁺=503.

### Step 4: Synthesis of (S)-3-(3-bromophenyl)-2-((R)-1-(tert-butoxycarbonyl)pyrrolidin-3-yl)propanoic-3,3-d2 acid

*Tert-*butyl (R)-3-((S)-1-((S)-4-benzyl-2-oxooxazolidin-3-yl)-3-(3-bromophenyl)-1-oxopropan-2-yl-3,3-*d₂*)pyrrolidine-1-carboxylate (25g, 44.68mmol, 1eq) was dissolved in tetrahydrofuran (300mL), and hydrogen peroxide (60mL) and an aqueous solution of lithium hydroxide (1.61g, 67.03mmol, 1.5eq) were added at 0°C; the reaction mixture was stirred at room temperature for 3h. LCMS showed the reaction was completed. The reaction was quenched with sodium sulfite, and the mixture was diluted with water, adjusted to pH=13 with sodium hydroxide (1.0M), and extracted with ethyl acetate; the aqueous phase was adjusted to pH=5 with hydrochloric acid (1.0M), extracted with dichloromethane. The organic phases were combined, dried over anhydrous sodium sulfate, filtered, and concentrated to give the target compound (8g). LCMS(ESI)[M+H-Boc]⁺=300.

### Step 5: Synthesis of tert-butyl (R)-3-((S)-3-(3-bromophenyl)-1-(tert-butoxy)-1-oxopropan-2-yl-3,3-d₂)pyrrolidine-1-carboxylate

(S)-3-(3-bromophenyl)-2-((R)-1-(tert-butoxycarbonyl)pyrrolidin-3-yl)propanoic-3,3-d2 acid (8g, 19.99mmol, 1eq) was dissolved in methyltetrahydrofuran (100mL), *tert-*butyl N,N'-diisopropylcarbamimidate (12.01mL, 59.96mmol, 3eq) was added; under nitrogen protection, the reaction mixture was heated to 65°C and stirred for 16h. The LCMS showed the reaction was completed. The reaction mixture was filtered, concentrated and purified by column chromatography (silica gel, petroleum ether:ethyl acetate=4: 1) to give the target compound (3g). LCMS(ESI)[M+H]⁺=456.3; ¹HNMR(400MHz, DMSO-*d₆*) δ 7.43-7.36 (m, 2H), 7.26-7.19 (m, 2H), 3.55-3.45 (m, 1H), 3.35-3.34 (m, 1H), 3.18-3.13 (m, 1H), 2.99 (t, *J*=10.1Hz, 1H), 2.34-2.24 (m, 2H), 1.86-1.80 (m, 1H), 1.61-1.53 (m, 1H), 1.40 (s, 9H), 1.23 (s, 9H).

### Step 6: Synthesis of tert-butyl (R)-3-((S)-1-(tert-butoxy)-1-oxo-3-(3-(4,4,5,5-tetramethyl-1,3,2-dioxaborolan-2-yl)phenyl)propan-2-yl-3,3-d₂)pyrrolidine-1-carboxylate

*Tert-*butyl (R)-3-((S)-3-(3-bromophenyl)-1-(tert-butoxy)-1-oxopropan-2-yl-3,3-*d₂*)pyrrolidine-1-carboxylate (3g, 6.57mmol, 1eq) was dissolved in 1,4-dioxane (40mL), potassium acetate (1.94g, 19.72mmol, 3eq), [1,1'-bis(diphenylphosphino)ferrocene]palladium(II) dichloride (0.48g, 0.66mmol, 0.1eq) and bis(pinacolato)diboron (3.34g, 13.15mmol, 2eq) was added, under nitrogen protection, the reaction mixture was heated to 90°C, and stirred for 18h. LCMS showed the reaction was completed. Water was added to the reaction mixture, which was extracted with ethyl acetate, and the organic phases were combined, dried over anhydrous sodium sulfate, filtered and concentrated to give the target compound (3g, crude), which was used directly in the next step. LCMS(ESI)[M+H-Boc]⁺=404.3.

### Step 7: Synthesis of tert-butyl (R)-3-((S)-1-(tert-butoxy)-3-(3-hydroxyphenyl)-1-oxopropan-2-yl-3,3-d₂)pyrrolidine-1-carboxylate

*Tert-*butyl (R)-3-((S)-1-(tert-butoxy)-1-oxo-3-(3-(4,4,5,5-tetramethyl-1,3,2-dioxaborolan-2-yl)phenyl)propan-2-yl-3,3-*d₂*)pyrrolidine-1-carboxylate (3g, 5.96mmol, 1eq) was dissolved in methanol (40mL). Hydrogen peroxide (2.03mL, 59.59mmol, 10eq) was added at 0°C, and the mixture was stirred at room temperature for 1h. LCMS showed the reaction was completed. The reaction mixture was concentrated, added with water, extracted with ethyl acetate. The organic phases were combined, dried over anhydrous sodium sulfate, filtered, and concentrated to give a crude product, which was purified by column chromatography (silica gel, petroleum ether:ethyl acetate=5:1) to give the target compound (2.2g). LCMS(ESI)[M+H]⁺=394.3.

### Step 8: Synthesis of tert-butyl (R)-3-((S)-3-(3-(2-(((benzyloxy)carbonyl)amino)ethoxy)phenyl)-1-(tert-butoxy)-1-oxopropan-2-yl-3,3-d₂)pyrrolidine-1-carboxylate

*Tert-*butyl (R)-3-((S)-1-(tert-butoxy)-3-(3-hydroxyphenyl)-1-oxopropan-2-yl-3,3-*d₂*)pyrrolidine-1-carboxylate (2g, 5.08mmol, 1eq) was dissolved in N,N-dimethylformamide (20mL), benzyl (2-bromoethyl)carbamate (2.62g, 10.16mmol, 2eq) and potassium carbonate (2.11g, 15.25mmol, 3eq) were added, under nitrogen protection, the reaction mixture was heated to 80°C and stirred for 18h. LCMS showed the reaction was completed. The reaction mixture was added with water and extracted with ethyl acetate. The organic phases were combined, washed with saturated saline solution, dried over anhydrous sodium sulfate, filtered and concentrated to give the crude product, which was purified by column chromatography (silica gel, petroleum ether:ethyl acetate=5:1) to give the target compound (1.3g). LCMS(ESI)[M+H]⁺=571.5.

### Step 9: Synthesis of tert-butyl (R)-3-((S)-3-(3-(2-aminoethoxy)phenyl)-1-(tert-butoxy)-1-oxopropan-2-yl-3,3-d₂)pyrrolidine-1-carboxylate

*Tert-*butyl (R)-3-((S)-3-(3-(2-(((benzyloxy)carbonyl)amino)ethoxy)phenyl)-1-(tert-butoxy)-1-oxopropan-2-yl-3,3-*d₂*)pyrrolidine-1-carboxylate (1.3g, 2.28mmol, 1eq) was dissolved in tetrahydrofuran (20mL), Pd/C (242.41mg, 2.28mmol, 1eq) was added, and the reaction was maintained at room temperature for 18h under hydrogen atmosphere (balloon pressure). LCMS showed the reaction was completed. The mixture was filtered and concentrated to give the target compound (0.9g). LCMS(ESI)[M+H]⁺=437.4.

### Step 10: Synthesis of tert-butyl (R)-3-((S)-1-(tert-butoxy)-3-(3-formylphenyl)-1-oxopropan-2-yl-3,3-d₂)pyrrolidine-1-carboxylate

*Tert-*butyl (R)-3-((S)-3-(3-bromophenyl)-1-(tert-butoxy)-1-oxopropan-2-yl-3,3-*d₂*)pyrrolidine-1-carboxylate (1g, 2.19mmol, 1eq) was dissolved in N,N-dimethylformamide (10mL), sodium carbonate (464.24mg, 4.38mmol, 2eq), triethylsilane (0.53mL, 3.29mmol, 1.5eq) and [1,1'-bis(diphenylphosphino)ferrocene]palladium(II) dichloride (160.68mg, 0.22mmol, 0.1eq) were added, and the reaction mixture was heated to 80°C under carbon monoxide atmosphere (balloon pressure) and stirred for 18h. LCMS showed the reaction was completed. The reaction mixture was added with water and extracted with ethyl acetate. The organic phases were combined, washed with saturated saline solution, dried over anhydrous sodium sulfate, filtered and concentrated to give the crude product, which was purified by column chromatography (silica gel, petroleum ether:ethyl acetate=5:1) to give the target compound (400mg). LCMS(ESI)[M+H]⁺=406.4; ¹HNMR(400MHz, DMSO-*d₆*) δ 9.99 (s, 1H), 7.78-7.71 (m, 2H), 7.57-7.49 (m, 2H), 3.59-3.47 (m, 1H), 3.39-3.34 (m, 1H), 3.20-3.10 (m, 1H), 3.01 (t, *J*=10.0Hz, 1H), 2.58-2.52 (m, 1H), 2.35-2.25 (m, 1H), 1.89-1.79 (m, 1H), 1.65-1.53 (m, 1H), 1.40 (s, 9H), 1.19 (s, 9H).

### Step 11: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl-1,1-d₂)phenoxy)ethyl)amino)methyl)phenyl)-1-oxopropan-2-yl-3,3-d₂)pyrrolidine-1-carboxylate

*Tert-*butyl (R)-3-((S)-1-(tert-butoxy)-3-(3-formylphenyl)-1-oxopropan-2-yl-3,3-*d₂*)pyrrolidine-1-carboxylate (400mg, 0.99mmol, 1eq) was dissolved in ethanol (7mL), followed by adding titanium tetraisopropanolate (560.7mg, 1.97mmol, 2eq) and *tert-*butyl (R)-3-((S)-3-(3-(2-aminoethoxy)phenyl)-1-(tert-butoxy)-1-oxopropan-2-yl-3,3-*d₂*)pyrrolidine-1-carboxylate (473.7mg, 1.09mmol, 1.1eq). The reaction mixture was stirred at room temperature for 18h. Sodium borohydride (55.97mg, 1.48mmol, 1.5eq) was added. The mixture was stirred for another 1 h. LCMS showed the reaction was completed. The reaction mixture was quenched with saturated aqueous ammonium chloride solution (0.4mL), filtered, and the filtrate was concentrated to give a crude product, which was purified by column chromatography (silica gel, petroleum ether:tetrahydrofuran=1:1) to give the target compound (650mg). LCMS(ESI)[M+H]⁺=826.4; ¹HNMR(400MHz, DMSO-*d₆*) δ 7.51-7.08 (m, 6H), 6.79-6.76 (m, 2H), 4.37-4.20 (m, 2H), 4.14-4.06 (m, 2H), 3.98-3.92 (m, 1H), 3.60-3.42 (m, 5H), 3.39-3.36 (m, 1H), 3.19-2.94 (m, 6H), 2.33-2.23 (m, 2H), 1.88-1.79 (m, 2H), 1.63-1.52 (m, 2H), 1.39 (s, 18H), 1.23 (d, *J*=1.4Hz, 18H).

### Step 12: Synthesis of tert-butyl (R)-3-((S)-3-(3-allylphenyl)-1-(tert-butoxy)-1-oxopropan-2-yl-3,3-d₂)pyrrolidine-1-carboxylate

Under nitrogen protection, *tert-*butyl (R)-3-((S)-3-(3-bromophenyl)-1-(tert-butoxy)-1-oxopropan-2-yl-3,3-*d₂*)pyrrolidine-1-carboxylate(1.5g, 3.29mmol, 1eq) was dissolved in tetrahydrofuran (20mL) and water (5mL), potassium phosphate (2.09g, 9.86mmol, 3eq), 2-allyl-4,4,5,5-tetramethyl-1,3,2-dioxaborolane (1.38mg, 8.22mmol, 2.5eq) and [1,1'-bis(diphenylphosphino)ferrocene]palladium(II) dichloride (241.14mg, 0.33mmol, 0.1eq) were added, and the reaction mixture was stirred at 90°C for 18h. LCMS showed the reaction was completed. The reaction mixture was added with water and extracted with ethyl acetate. The organic phases were combined, dried over anhydrous sodium sulfate, filtered and concentrated to give the crude product, which was purified by column chromatography (silica gel, petroleum ether:tetrahydrofuran=10:1) to give the target compound (1.3g). LCMS(ESI)[M+H]⁺=418.4; ¹HNMR(400MHz, DMSO-*d₆*) δ 7.19 (t, *J*=7.5Hz, 1H), 7.06-6.94 (m, 3H), 6.01-5.83 (m, 1H), 5.16-4.93 (m, 2H), 3.56-3.45 (m, 1H), 3.31-3.27 (m, 2H), 3.18-3.11 (m, 1H), 2.97 (t, *J*=10.0Hz, 1H), 2.47-2.41 (m, 1H), 2.37-2.21 (m, 2H), 1.86-1.78 (m, 1H), 1.68-1.54 (m, 1H), 1.40 (s, 9H), 1.22 (s, 9H).

### Step 13: Synthesis of 2-(3-((S)-3-(tert-butoxy)-2-((R)-1-(tert-butoxycarbonyl)pyrrolidin-3-yl)-3-oxopropyl-1,1-d₂)phenyl)acetic acid

*Tert-*butyl (R)-3-((S)-3-(3-allylphenyl)-1-(tert-butoxy)-1-oxopropan-2-yl-3,3-*d₂*)pyrrolidine-1-carboxylate (1.3g, 3.11mmol, 1eq) was dissolved in acetonitrile (10mL) and water (10mL), ruthenium trichloride hydrate (70.18mg, 0.31mmol, 0.1eq) and sodium periodate (3.33g, 15.57mmol, 5eq) were added, and the reaction mixture was stirred at room temperature for 1h. LCMS showed the reaction was completed. The reaction mixture was added with acetonitrile, filtered, and the filtrate was concentrated. After concentration, water was added, and the mixture was extracted with ethyl acetate. The organic phase was dried over anhydrous sodium sulfate, filtered, and concentrated to give the crude product, which was purified by reversed-phase column chromatography (C₁₈, water/acetonitrile=1:1) to give the target compound (800mg). LCMS(ESI)[M+H]⁺=436.3.

### Step 14: Synthesis of tert-butyl (R)-3-((S)-1-(tert-butoxy)-3-(3-((N-(2-(3-((S)-3-(tert-butoxy)-2-((R)-1-(tert-butoxycarbonyl)pyrrolidin-3-yl)-3-oxopropyl-1,1-d₂)phenoxy)ethyl)-2-(3-((S)-3-(tert-butoxy)-2-((R)-1-(tert-butoxycarbonyl)pyrrolidin-3-yl)-3-oxopropyl-1,1-d₂)phenyl)acetamido)methyl)phenyl)-1-oxopropan-2-yl-3,3-d₂)pyrrolidine-1-carboxylate

*Tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl-1,1-*d₂*)phenoxy)ethyl)amino)methyl)phenyl)-1-oxopropan-2-yl-3,3-*d₂*)pyrrolidine-1-carboxylate(550mg, 0.67mmol, 1eq) was dissolved in N,N-dimethylformamide (5mL), and 2-(3-((S)-3-(tert-butoxy)-2-((R)-1-(tert-butoxycarbonyl)pyrrolidin-3-yl)-3-oxopropyl-1,1-*d₂*)phenyl)acetic acid (318mg, 0.73mmol, 1.1eq), O-(7-azabenzotriazol-1-yl)-N,N,N',N'-tetramethyluronium hexafluorophosphate (506.29mg, 1.33mmol, 2eq) and *N*,*N*-diisopropylethylamine (0.33mL, 2mmol, 3eq) were added. The reaction was maintained at room temperature for 3h. LCMS showed the reaction was completed. The reaction mixture was added with water and extracted with ethyl acetate. The organic phases were combined, washed with saturated saline solution, dried over anhydrous sodium sulfate, filtered and concentrated to give the crude product, which was purified by column chromatography (C₁₈, water:acetonitrile=1:10) to give the target compound (460mg). LCMS(ESI)[M+H-Boc]⁺=1143.1; ¹HNMR(400MHz, DMSO-*d₆*) δ 7.32-6.89 (m, 9H), 6.79-6.70 (m, 3H), 4.79-4.39 (m, 2H), 4.11-3.94 (m, 2H), 3.84 (s, 1H), 3.74-3.42 (m, 6H), 3.39-3.33 (m, 3H), 3.21-3.08 (m, 3H), 3.01-2.86 (m, 3H), 2.48-2.34 (m, 3H), 2.33-2.19 (m, 3H), 1.91-1.75 (m, 3H), 1.62-1.50 (m, 3H), 1.39 (s, 27H), 1.26-1.16 (m, 27H).

Step 15: The product from step 14 (450mg, 0.36mmol, 1eq) was dissolved in dioxane (5mL), hydrochloric acid/dioxane (5mL, 4.0M) was added, and the reaction mixture was stirred at room temperature for 3h. LCMS showed the reaction was completed. The reaction mixture was concentrated, added with water (20mL), and adjusted to pH=7 with aqueous sodium hydroxide solution (1.0M). The mixture was separated and purified by prep-HPLC (C₁₈, 0.1% NH₄HCO₃ in H₂O/ACN) to give the target compound (118.9mg). LCMS(ESI)[M+H]⁺=775.4; ¹HNMR(400MHz, D₂O) δ 7.25-6.79 (m, 10H), 6.68-6.57 (m, 2H), 4.62-4.48 (m, 2H), 4.15-3.99 (m, 1H), 3.97-3.89 (m, 1H), 3.86-3.77 (m, 1H), 3.76-3.52 (m, 3H), 3.37-3.19 (m, 6H), 3.18-3.05 (m, 3H), 2.83-2.62 (m, 3H), 2.42-2.23 (m, 6H), 2.10-1.93 (m, 3H), 1.72-1.52 (m, 3H).

### Example 86 (Compound 193)

### Preparation of (S)-3-(3-(2-((2-(3-((S)-2-carboxy-2-((R)-pyrrolidin-3-yl)ethyl)phenoxy)ethyl)((3-((S)-2-carboxy-2-((R)-pyrrolidin-3-yl)ethyl)phenyl)methyl-d₂)amino)-2-oxoethyl)phenyl)-2-((R)-pyrrolidin-3-yl)propanoic acid

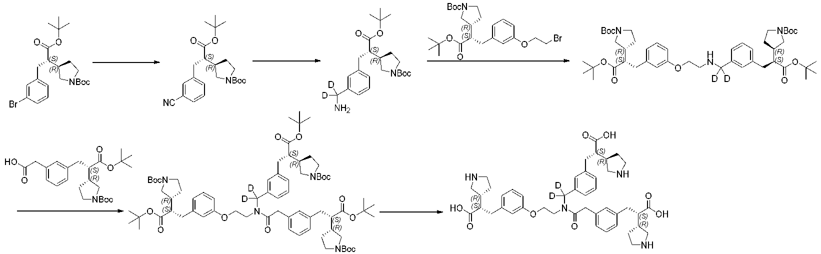

### Step 1: Synthesis of tert-butyl (R)-3-((S)-1-(tert-butoxy)-3-(3-cyanophenyl)-1-oxopropan-2-yl)pyrrolidine-1-carboxylate

*Tert-*butyl (R)-3-((S)-3-(3-bromophenyl)-1-(tert-butoxy)-1-oxopropan-2-yl)pyrrolidine-1-carboxylate (500mg, 1.1mmol, 1.0eq) in N,N-dimethylformamide (7.0mL) solution was added with zinc cyanide (193.79mg, 1.65mmol, 1.5eq) and tetrakis(triphenylphosphine)palladium (127.15mg, 0.11mmol, 0.1eq). After replacing with nitrogen, the reaction mixture was heated to 130°C and stirred for 2h. After the reaction mixture was cooled to room temperature, water was added, and the mixture was extracted with ethyl acetate. The organic phases were combined, washed with water and saturated saline solution in sequence, dried over anhydrous sodium sulfate, filtered and concentrated. The crude product was separated and purified by silica gel column chromatography (silica gel, petroleum ether:ethyl acetate=3:1) to give the target compound (320mg). LCMS(ESI)[M+Na+acetonitrile]⁺=464.3; ¹HNMR(400MHz, DMSO-*d*₆) δ 7.70-7.66 (m, 2H), 7.58-7.54 (m, 1H), 7.49 (t, *J=*7.9Hz, 1H), 3.56-3.45 (m, 1H), 3.40-3.35 (m, 1H), 3.20-3.09 (m, 1H), 3.05-2.96 (m, 1H), 2.89-2.80 (m, 1H), 2.80-2.71 (m, 1H), 2.62-2.53 (m, 1H), 2.35-2.22 (m, 1H), 1.89-1.79 (m, 1H), 1.64-1.51 (m, 1H), 1.42-1.39 (m, 9H), 1.23-1.19 (m, 9H).

### Step 2: Synthesis of tert-butyl (R)-3-((S)-3-(3-(aminomethyl-d₂)phenyl)-1-(tert-butoxy)-1-oxopropan-2-yl)pyrrolidine-1-carboxylate

*Tert-*butyl (R)-3-((S)-1-(tert-butoxy)-3-(3-cyanophenyl)-1-oxopropan-2-yl)pyrrolidine-1-carboxylate (200mg, 0.5mmol, 1.0eq) was placed in a three-necked flask. The reaction apparatus was replaced with nitrogen and added with samarium(II) iodide (29.96mL, 3mmol, 6.0eq), triethylamine (1819.1mg, 17.98mmol, 36eq) and deuterium oxide (1.3mL), sequentially; the reaction mixture was stirred at room temperature for 1.5h. LCMS showed the reaction was completed.The reaction mixture was added with sodium hydroxide solution (10.0mL, 1.0M), diluted with water, extracted with dichloromethane, and the organic phases were combined, washed with saturated saline solution, dried over anhydrous sodium sulfate, filtered, and concentrated to give a crude product, which was separated and purified by reversed-phase column chromatography (C₁₈, 0.1% trifluoroacetic acid, water:acetonitrile=1:1) to give the target compound (180mg). LCMS(ESI)[M+H]⁺=407.2; ¹HNMR(400MHz, DMSO-*d*₆) δ 7.21-7.11 (m, 3H), 7.00 (d, *J*=7.1Hz, 1H), 3.54-3.46 (m, 1H), 3.20-3.11 (m, 1H), 2.97 (t, *J*=9.6Hz, 1H), 2.75-2.68 (m, 2H), 2.47-2.41 (m, 1H), 2.33-2.22 (m, 1H), 1.86-1.76 (m, 2H), 1.62-1.54 (m, 1H), 1.40 (s, 9H), 1.24 (s, 9H).

### Step 3: Synthesis of tert-butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl-d₂)phenyl)-1-oxopropan-2-yl)pyrrolidine-1-carboxylate

*Tert-*butyl (R)-3-((S)-3-(3-(aminomethyl-*d₂*)phenyl)-1-(tert-butoxy)-1-oxopropan-2-yl)pyrrolidine-1-carboxylate (260mg, 0.64mmol, 1.0eq) in acetonitrile (20mL) solution was added with *tert-*butyl (R)-3-((S)-3-(3-(2-bromoethoxy)phenyl)-1-(tert-butoxy)-1-oxopropan-2-yl)pyrrolidine-1-carboxylate (382.52mg, 0.77mmol, 1.2eq) and potassium carbonate (176.77mg, 1.28mmol, 2.0eq). The reaction mixture was heated to 80°C and stirred for 72h. The reaction mixture was cooled to room temperature, filtered, and the filter cake was washed thoroughly with tetrahydrofuran. The filtrate was concentrated to give a crude product, which was separated and purified by column chromatography (silica gel, tetrahydrofuran:petroleum ether=1:2) to give the target compound (260mg). LCMS(ESI)[M+H]⁺=825.1.

### Step 4: Synthesis of tert-butyl (R)-3-((S)-1-(tert-butoxy)-3-(3-(N-(2-(3-((S)-3-(tert-butoxy)-2-((R)-1-(tert-butoxycarbonyl)pyrrolidin-3-yl)-3-oxopropyl)phenoxy)ethyl)-2-(3-((S)-3-(tert-butoxy)-2-((R)-1-(tert-butoxycarbonyl)pyrrolidin-3-yl)-3-oxopropyl)phenyl)acetamido)methyl-d₂)phenyl)-1-oxopropan-2-yl)pyrrolidine-1-carboxylate

2-(3-((S)-3-(tert-butoxy)-2-((R)-1-(tert-butoxycarbonyl)pyrrolidin-3-yl)-3-oxopropyl)phenyl)acetic acid(164.14mg, 0.38mmol, 1.2eq) in N,N-dimethylformamide (10mL) solution was added with *N*,*N*-diisopropylethylamine (122.33mg, 0.95mmol, 3.0eq), O-(7-azabenzotriazol-1-yl)-N,N,N',N'-tetramethyluronium hexafluorophosphate (215.93mg, 0.57mmol, 1.8eq) and *tert-*butyl (R)-3-((S)-1-(tert-butoxy)-3-(3-(((2-(3-((S)-3-(tert-butoxy)-2-((R)-1-(tert-butoxycarbonyl)pyrrolidin-3-yl)-3-oxopropyl)phenoxy)ethyl)amino)methyl-*d₂*)phenyl)-1-oxopropan-2-yl)pyrrolidine-1-carboxylate(260mg, 0.32mmol, 1.0eq). The reaction mixture was stirred at room temperature for 16h. LCMS showed the reaction was completed. The reaction mixture was added with water and extracted with ethyl acetate. The organic phases were combined and washed with water and saturated saline solution, dried over anhydrous sodium sulfate, filtered and concentrated to give a crude product, which was separated and purified by reversed-phase column chromatography (C₁₈, 0.1% trifluoroacetic acid, water:acetonitrile=1:10) to give the target compound (300mg). LCMS(ESI)[M+H-100]⁺=1039.

Step 5: The product from step 4 (250mg, 0.2mmol, 1.0eq) was dissolved in a solution of hydrochloric acid/1,4-dioxane (8mL, 4.0M), and the reaction mixture was stirred at room temperature for 3h. After the reaction was completed, the reaction mixture was concentrated under reduced pressure, and the residue was adjusted to a pH of 7-8 with sodium hydroxide solution (1.0M); the mixture was separated and purified by reversed-phase preparative chromatography (C₁₈, 0.03% ammonium bicarbonate, water:acetonitrile=8: 1) to give the target compound (105.61mg). LCMS(ESI)[M+H]⁺=771.4; ¹HNMR(400MHz, D₂O) δ 7.28-7.13 (m, 3H), 7.12-6.80 (m, 7H), 6.70-6.60 (m, 2H), 4.66-4.53 (m, 1H), 4.09 (t, *J*=5.1Hz, 1H), 3.98 (t, *J*=4.8Hz, 1H), 3.86 (s, 1H), 3.77-3.72 (m, 1H), 3.71-3.63 (m, 2H), 3.40-3.22 (m, 6H), 3.19-3.07 (m, 3H), 2.87-2.48 (m, 9H), 2.42-2.25 (m, 6H), 2.09-1.96 (m, 3H), 1.72-1.57 (m, 3H).

### Example 87 (Compound 209)

### Preparation of (R)-3-(3-(2-((3-((R)-2-carboxy-2-((R)-pyrrolidin-3-yl)ethyl)benzyl)(2-(3-((R)-2-carboxy-2-((R)-pyrrolidin-3-yl)ethyl)phenoxy)ethyl)amino)-2-oxoethyl)phenyl)-2-((R)-pyrrolidin-3-yl)propanoic acid

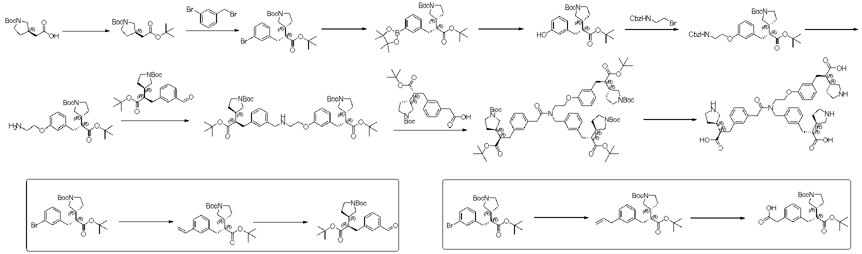

### Step 1: Synthesis of tert-butyl (R)-3-(2-(tert-butoxy)-2-oxoethyl)pyrrolidine-1-carboxylate

Under nitrogen protection, (R)-2-(1-(tert-butoxycarbonyl)pyrrolidin-3-yl)acetic acid (51g, 222.45mmol, 1.0eq) was dissolved in 2-methyltetrahydrofuran (500mL), followed by adding *tert-*butyl N,N'-diisopropylcarbamimidate (133.68g, 667.34mmol, 3.0eq), and the reaction mixture was stirred at 65°C for 16h. LCMS showed the reaction was completed. The reaction mixture was directly concentrated and separated and purified by rapid chromatography (silica gel, petroleum ether:ethyl acetate=2:1) to give the target compound (41.0g). LCMS(ESI)[M+H]⁺=286.0.

### Step 2: Synthesis of tert-butyl (R)-3-((R)-3-(3-bromophenyl)-1-(tert-butoxy)-1-oxopropan-2-yl)pyrrolidine-1-carboxylate

Under nitrogen protection and ice bath, a solution of lithium bis(trimethylsilyl)amide (158.03mL, 1.0 M in THF, 1.1eq) was added dropwise to a solution of *tert-*butyl (R)-3-(2-(tert-butoxy)-2-oxoethyl)pyrrolidine-1-carboxylate (41g, 143.67mmol, 1.0eq) in tetrahydrofuran (450mL). The reaction mixture was stirred at 0°C for 30min; then a solution of 1-bromo-3-(bromomethyl)benzene (37.702g, 150.85mmol, 1.05eq) in tetrahydrofuran (50mL) was slowly added. The reaction temperature was slowly raised to room temperature and the mixture was stirred for 24h. The reaction mixture was cooled with an ice-water bath, and a saturated aqueous solution of ammonium chloride and water were added. The mixture was extracted with ethyl acetate. The organic phase was washed with saturated sodium chloride aqueous solution, dried over anhydrous sodium sulfate, filtered, and concentrated to give a racemate (17.0g). LCMS(ESI)[M+H-2×*tert-*butyl]⁺=341.8. 10 g of the racemate was separated by chiral column to give 6.2 g of the target compound.

### Step 3: Synthesis of tert-butyl (R)-3-((R)-1-(tert-butoxy)-1-oxo-3-(3-(4,4,5,5-tetramethyl-1,3,2-dioxaborolan-2-yl)phenyl)propan-2-yl)pyrrolidine-1-carboxylate

Under nitrogen protection, *tert-*butyl (R)-3-((R)-3-(3-bromophenyl)-1-(tert-butoxy)-1-oxopropan-2-yl)pyrrolidine-1-carboxylate(2.0g, 4.4mmol, 1.0eq) was dissolved in 1,4-dioxane (20mL), followed by adding bis(pinacolato)diboron (2236mg, 8.8mmol, 2.0eq), [1,1'-bis(diphenylphosphino)ferrocene]palladium(II) dichloride (323mg, 0.44mmol, 0.1eq) and potassium acetate (1296mg, 13.2mmol, 3eq), and the reaction mixture was stirred at 80°C for 16h. LCMS showed the reaction was completed. The reaction mixture was concentrated directly and separated and purified by rapid chromatography (silica gel, petroleum ether:ethyl acetate=2:1) to give the target compound (1.5g). LCMS(ESI)[M+H]⁺=416.

### Step 4: Synthesis of tert-butyl (R)-3-((R)-1-(tert-butoxy)-3-(3-hydroxyphenyl)-1-oxopropan-2-yl)pyrrolidine-1-carboxylate

*Tert-*butyl (R)-3-((R)-1-(tert-butoxy)-1-oxo-3-(3-(4,4,5,5-tetramethyl-1,3,2-dioxaborolan-2-yl)phenyl)propan-2-yl)pyrrolidine-1-carboxylate (1.5g, 2.99mmol, 1.0eq) was dissolved in tetrahydrofuran (20mL), followed by adding hydrogen peroxide (3mL). The mixture was stirred at room temperature for 16h. LCMS showed the reaction was completed. The reaction mixture was directly concentrated, separated and purified by rapid chromatography (silica gel, petroleum ether:ethyl acetate=1:1) to give the target compound (800mg). LCMS(ESI)[M+H-2×*tert-*butyl]⁺=392.0.

### Step 5: Synthesis of tert-butyl (R)-3-((R)-3-(3-(2-(((benzyloxy)carbonyl)amino)ethoxy)phenyl)-1-(tert-butoxy)-1-oxopropan-2-yl)pyrrolidine-1-carboxylate

Under nitrogen protection, *tert-*butyl (R)-3-((R)-1-(tert-butoxy)-3-(3-hydroxyphenyl)-1-oxopropan-2-yl)pyrrolidine-1-carboxylate(800mg, 2.04mmol, 1.0eq) was dissolved in N,N-dimethylformamide (10mL) under nitrogen protection, followed by adding benzyl (2-bromoethyl)carbamate (1055mg, 4.09mmol, 2.0eq) and potassium carbonate (847mg, 6.13mmol, 3.0eq), and the reaction mixture was stirred at 80°C for 16h. LCMS showed the reaction was completed. The reaction mixture was added with water and extracted with ethyl acetate. The organic phase was washed with saturated saline, dried over anhydrous sodium sulfate, filtered and concentrated to give the crude product, which was separated and purified by rapid chromatography (silica gel, petroleum ether:ethyl acetate=2:1) to give the target compound (700mg). LCMS(ESI)[M+H]⁺=569.1.

### Step 6: Synthesis of tert-butyl (R)-3-((R)-3-(3-(2-aminoethoxy)phenyl)-1-(tert-butoxy)-1-oxopropan-2-yl)pyrrolidine-1-carboxylate

*Tert-*butyl (R)-3-((R)-3-(3-(2-(((benzyloxy)carbonyl)amino)ethoxy)phenyl)-1-(tert-butoxy)-1-oxopropan-2-yl)pyrrolidine-1-carboxylate (700mg, 1.23mmol, 1.0eq) was dissolved in methanol (10mL), and Pd/C (150mg) was added. The mixture was stirred at room temperature for 16h under a hydrogen atmosphere. LCMS showed that the reaction was completed. The reaction mixture was directly concentrated, separated and purified by rapid chromatography (silica gel, petroleum ether:ethyl acetate=2:1) to give the target compound (500mg). LCMS(ESI)[M+H]⁺=435.1.

### Step 7: Synthesis of tert-butyl (R)-3-((R)-1-(tert-butoxy)-1-oxo-3-(3-vinylphenyl)propan-2-yl)pyrrolidine-1-carboxylate

Under nitrogen protection, *tert-*butyl (R)-3-((R)-3-(3-bromophenyl)-1-(tert-butoxy)-1-oxopropan-2-yl)pyrrolidine-1-carboxylate (2000mg, 4.4mmol, 1.0eq) was dissolved in 1,4-dioxane (25mL) and water (5mL), followed by adding potassium vinyltrifluoroborate (1179mg, 8.8mmol, 2.0eq), [1,1'-bis(diphenylphosphino)ferrocene]palladium(II) dichloride (323mg, 0.44mmol, 0.1eq) and cesium carbonate (4302mg, 13.2mmol, 3.0eq); the mixture was stirred at 90°C for 16h. LCMS showed that the reaction was completed. The reaction mixture was directly concentrated, separated and purified by rapid chromatography (silica gel, petroleum ether:ethyl acetate=2:1) to give the target compound (1500mg). LCMS [M+H]⁺=402.1.

### Step 8: Synthesis of tert-butyl (R)-3-((R)-1-(tert-butoxy)-3-(3-formylphenyl)-1-oxopropan-2-yl)pyrrolidine-1-carboxylate

*Tert-*butyl (R)-3-((R)-1-(tert-butoxy)-1-oxo-3-(3-vinylphenyl)propan-2-yl)pyrrolidine-1-carboxylate (1500mg, 3.74mmol, 1.0eq) was dissolved in 1,4-dioxane/water (3:1, 20mL), followed by adding sodium periodate (3196g, 14.94mmol, 4eq) and potassium osmate dihydrate (131mg, 0.37mmol, 0.1eq), and the mixture was stirred at room temperature for 24h. LCMS showed that the reaction was completed. The reaction mixture was directly concentrated, separated and purified by rapid chromatography (silica gel column, petroleum ether:ethyl acetate=1:1) to give the target compound (800mg). LCMS(ESI)[M+Na]⁺=426.0.

### Step 9: Synthesis of tert-butyl (R)-3-((R)-1-(tert-butoxy)-3-(3-(((2-(3-((R)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

*Tert-*butyl (R)-3-((R)-3-(3-(2-aminoethoxy)phenyl)-1-(tert-butoxy)-1-oxopropan-2-yl)pyrrolidine-1-carboxylate (320mg, 0.74mmol, 1.0eq) was dissolved in methanol (10mL), followed by adding *tert-*butyl (R)-3-((R)-1-(tert-butoxy)-3-(3-formylphenyl)-1-oxopropan-2-yl)pyrrolidine-1-carboxylate (297mg, 0.74mmol, 1.0eq) and sodium cyanoborohydride (46mg, 0.74mmol, 1.0eq). The mixture was stirred at room temperature for 24h. LCMS showed the reaction was completed. The reaction mixture was concentrated directly and separated and purified by rapid chromatography (silica gel, petroleum ether:ethyl acetate=1:5) to give the target compound (260mg). LCMS(ESI)[M+H]⁺=822.2.

### Step 10: Synthesis of tert-butyl (R)-3-((R)-3-(3-allylphenyl)-1-(tert-butoxy)-1-oxopropan-2-yl)pyrrolidine-1-carboxylate

Under nitrogen protection, *tert-*butyl (R)-3-((R)-3-(3-bromophenyl)-1-(tert-butoxy)-1-oxopropan-2-yl)pyrrolidine-1-carboxylate(2000mg, 4.4mmol, 1.0eq) was dissolved in 1,4-dioxane (25mL) and water (5mL), 2-allyl-4,4,5,5-tetramethyl-1,3,2-dioxaborolane (1.109g, 6.6mmol, 1.5eq), [1,1'-bis(diphenylphosphino)ferrocene]palladium(II) dichloride dichloromethane complex (359mg, 0.44mmol, 0.1eq) and potassium phosphate (2.803g, 13.2mmol, 3eq) were added, and the mixture was stirred at 80°C for 16h. LCMS showed that the reaction was completed. The reaction mixture was directly concentrated, separated and purified by rapid chromatography (silica gel, petroleum ether:ethyl acetate=2:1) to give the target compound (1500mg). LCMS [M+H]⁺=416.1.

### Step 11: Synthesis of 2-(3-((R)-3-(tert-butoxy)-2-((R)-1-(tert-butoxycarbonyl)pyrrolidin-3-yl)-3-oxopropyl)phenyl)acetic acid

*Tert-*butyl (R)-3-((R)-3-(3-allylphenyl)-1-(tert-butoxy)-1-oxopropan-2-yl)pyrrolidine-1-carboxylate(1500mg, 3.61mmol, 1.0eq) was dissolved in acetonitrile/water (1:1, 40mL), followed by adding sodium periodate (3.860g, 18.05mmol, 5eq) and ruthenium trichloride (75mg, 0.36mmol, 0.1eq), and the mixture was stirred at room temperature for 24h. LCMS showed the reaction was completed. The reaction mixture was concentrated directly and separated and purified by rapid chromatography (silica gel, petroleum ether:ethyl acetate=1:1) to give the target compound (750mg). LCMS(ESI)[M+H]⁺=434.1.

### Step 12: Synthesis of tert-butyl (R)-3-((R)-1-(tert-butoxy)-3-(3-((N-(2-(3-((R)-3-(tert-butoxy)-2-((R)-1-(tert-butoxycarbonyl)pyrrolidin-3-yl)-3-oxopropyl)phenoxy)ethyl)-2-(3-((R)-3-(tert-butoxy)-2-((R)-1-(tert-butoxycarbonyl)pyrrolidin-3-yl)-3-oxopropyl)phenyl)acetamido)methyl)phenyl)-1-oxopropan-2-yl)pyrrolidine-1-carboxylate

Under nitrogen protection, *tert-*butyl (R)-3-((R)-1-(tert-butoxy)-3-(3-(((2-(3-((R)-3-(tert-butoxy)-2-((R)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (240mg, 0.29mmol, 1.0eq) was dissolved in DMF (10mL), followed by adding 2-(3-((R)-3-(tert-butoxy)-2-((R)-1-(tert-butoxycarbonyl)pyrrolidin-3-yl)-3-oxopropyl)phenyl)acetic acid (139mg, 0.32mmol, 1.1eq), O-(7-azabenzotriazol-1-yl)-N,N,N',N'-tetramethyluronium hexafluorophosphate (222mg, 0.58mmol, 2.0eq) and *N*,*N*-diisopropylethylamine (113mg, 0.88 mmol, 3.0eq). The mixture was stirred at room temperature for 20h. LCMS showed the reaction was completed. The reaction mixture was concentrated directly and separated and purified by rapid chromatography (silica gel, petroleum ether:ethyl acetate=1:8) to give the target compound (300mg). LCMS(ESI)[M+H]⁺=1237.3.

Step 13: The product from step 12 (300mg, 0.24mmol, 1.0eq) was dissolved in 1,4-dioxane (8mL), hydrogen chloride/dioxane (8mL, 4.0M) was added and the mixture was stirred at room temperature for 20h. LCMS showed the reaction was completed. The reaction mixture was concentrated directly, separated and purified by preparative chromatography to give the target compound (152.67mg). LCMS(ESI)[M+H]⁺=769.1; ¹HNMR(400MHz, D₂O) δ 7.27-6.78 (m, 10H), 6.71-6.55 (m, 2H), 4.65-4.58 (m, 2H), 4.09 (t, *J*=5.2Hz, 1H), 3.98 (t, *J=5.1Hz,* 1H), 3.87 (s, 1H), 3.77 (d, *J*=5.1Hz, 1H), 3.69 (d, *J*=6.9Hz, 2H), 3.27 (dt, *J*=17.5, 11.4Hz, 6H), 3.12 (dt, *J*=17.4, 7.2Hz, 3H), 2.93-2.79 (m, 3H), 2.78-2.52 (m, 6H), 2.45-2.23 (m, 6H), 2.20-2.00 (m, 3H), 1.54 (ddt, *J*=41.8, 19.7, 9.5Hz, 3H).

### Example 88 (Compound 210)

### Preparation of (S)-3-(3-(2-((3-((S)-2-carboxy-2-((S)-pyrrolidin-3-yl)ethyl)benzyl)(2-(3-((S)-2-carboxy-2-((S)-pyrrolidin-3-yl)ethyl)phenoxy)ethyl)amino)-2-oxoethyl)phenyl)-2-((S)-pyrrolidin-3-yl)propanoic acid

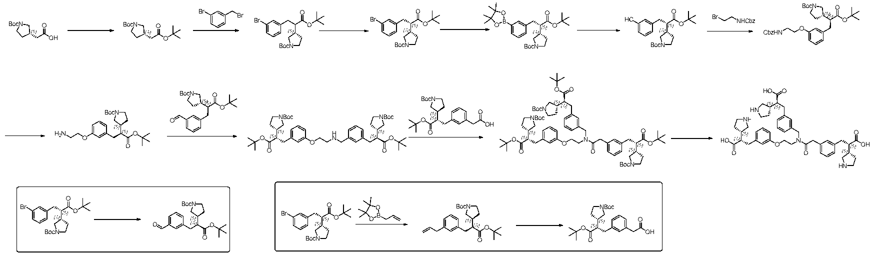

### Step 1: Synthesis of tert-butyl (S)-3-(2-(tert-butoxy)-2-oxoethyl)pyrrolidine-1-carboxylate

Under nitrogen protection, a solution of (S)-2-(1-(tert-butoxycarbonyl)pyrrolidin-3-yl)acetic acid (10g) in 2-methyltetrahydrofuran (100mL) was added with *tert-*butyl N,N'-diisopropylcarbamimidate (43.69g), and the reaction mixture was heated to 70°C and stirred for 16h. After cooled to room temperature, the reaction mixture was filtered to remove the filter residue. The filter cake was fully washed with methyl *tert-*butyl ether. The filtrate was concentrated under reduced pressure, and the crude product was purified by column chromatography (silica gel, tetrahydrofuran:petroleum ether=1:5) to give the target compound (11g); ¹HNMR(400MHz, DMSO-*d₆*) δ 3.54-3.45 (m, 1H), 3.38-3.33 (m, 1H), 3.27-3.16 (m, 1H), 2.94-2.84 (m, 1H), 2.49-2.39 (m, 1H), 2.38-2.32 (m, 2H), 2.07-1.96 (m, 1H), 1.63-1.51 (m, 1H), 1.50-1.40 (m, 18H).

### Step 2: Synthesis of tert-butyl (3S)-3-(3-(3-bromophenyl)-1-(tert-butoxy)-1-oxopropan-2-yl)pyrrolidine-1-carboxylate

Under nitrogen protection, *tert-*butyl (S)-3-(2-(tert-butoxy)-2-oxoethyl)pyrrolidine-1-carboxylate (20g) was dissolved in anhydrous tetrahydrofuran (280mL), and after the mixture was cooled to - 5°C in an ice-salt bath, lithium bis(trimethylsilyl)amide (84.1mL) was slowly added to the reaction system, the mixture was stirred at -5°C for 1h. Then a solution of 1-bromo-3-(bromomethyl)benzene (19.27g) in tetrahydrofuran (20mL) was slowly added dropwise to the reaction mixture which was heated to room temperature and stirred for 15h. LCMS showed the reaction was completed. The reaction mixture was quenched with saturated ammonium chloride solution, diluted with water, and extracted with ethyl acetate. The organic phase was washed with saturated saline, dried over anhydrous sodium sulfate, filtered, and concentrated. The crude product was separated and purified by reversed-phase column (C₁₈, 0.1% trifluoroacetic acid, water:acetonitrile=1:4) to give the target compound (10g). LCMS(ESI)[M+Na]⁺=476; ¹HNMR(400MHz, DMSO-d6) δ 7.44-7.38 (m, 2H), 7.28-7.19 (m, 2H), 3.66-3.56 (m, 1H), 3.42-3.29 (m, 2H), 3.23-3.11 (m, 1H), 3.05-2.86 (m, 2H), 2.72-2.63 (m, 1H), 2.35-2.21 (m, 1H), 2.14-2.01 (m, 1H), 1.76-1.60 (m, 1H), 1.43-1.36 (m, 9H), 1.24 (s, 9H).

### Step 3: Synthesis of tert-butyl (S)-3-((S)-3-(3-bromophenyl)-1-(tert-butoxy)-1-oxopropan-2-yl)pyrrolidine-1-carboxylate

*Tert-*butyl (3S)-3-(3-(3-bromophenyl)-1-(tert-butoxy)-1-oxopropan-2-yl)pyrrolidine-1-carboxylate(10g) was separated by chiral column to give the target compound (3.2g).

### Step 4: Synthesis of tert-butyl (S)-3-((S)-1-(tert-butoxy)-1-oxo-3-(3-(4,4,5,5-tetramethyl-1,3,2-dioxaborolan-2-yl)phenyl)propan-2-yl)pyrrolidine-1-carboxylate

Under nitrogen protection, *tert-*butyl (S)-3-((S)-3-(3-bromophenyl)-1-(tert-butoxy)-1-oxopropan-2-yl)pyrrolidine-1-carboxylate (1.3g) was dissolved in 1,4-dioxane solution (15mL), bis(pinacolato)diboron (1.45g), potassium acetate (842.04mg) and [1,1'-bis(diphenylphosphino)ferrocene]palladium(II) dichloride (212.78mg) were added, and the reaction mixture was stirred at 90°C for 18h. LCMS showed the reaction was completed. The mixture was filtered and concentrated to give the crude product, which was separated and purified by rapid chromatography (silica gel, petroleum ether:tetrahydrofuran=10:1) to give the target compound (1.3g). LCMS(ESI)[M+Na]⁺=524.8; ¹HNMR(400MHz, DMSO-*d₆*) δ 7.51 (d, *J*=10.8Hz, 2H), 7.34-7.25 (m, 2H), 3.60-3.48 (m, 1H), 3.39-3.35 (m, 1H), 3.16 (d, *J*=9.1Hz, 1H), 3.04-2.97 (m, 1H), 2.74 (d, *J*=10.2Hz, 2H), 2.49-2.43 (m, 1H), 2.31 (d, *J*=19.7Hz, 1H), 1.84 (d, *J*=5.3Hz, 1H), 1.59 (d, *J*=11.2Hz, 1H), 1.41 (s, 9H), 1.29 (d, *J*=3.5Hz, 12H), 1.23 (s, 9H).

### Step 5: Synthesis of tert-butyl (S)-3-((S)-1-(tert-butoxy)-3-(3-hydroxyphenyl)-1-oxopropan-2-yl)pyrrolidine-1-carboxylate

*Tert-*butyl (S)-3-((S)-1-(tert-butoxy)-1-oxo-3-(3-(4,4,5,5-tetramethyl-1,3,2-dioxaborolan-2-yl)phenyl)propan-2-yl)pyrrolidine-1-carboxylate (1.3g) was dissolved in methanol (10mL), hydrogen peroxide (0.88mL) was added, and the mixture was stirred at room temperature for 3h. LCMS showed the reaction was completed. The reaction mixture was added with water and extracted with ethyl acetate. The organic phases were combined, washed with saturated sodium chloride solution, dried over anhydrous sodium sulfate, concentrated, and separated and purified by rapid chromatography (petroleum ether:tetrahydrofuran=5:1) to give the target compound (950mg). LCMS(ESI)[M+Na+acetonitrile]⁺=455.3; ¹HNMR(400MHz, DMSO-*d₆*) δ 9.22 (s, 1H), 7.15-6.85 (m, 1H), 6.65-6.49 (m, 3H), 3.56-3.42 (m, 1H), 3.37 (s, 1H), 3.22-3.08 (m, 1H), 3.01-2.90 (m, 1H), 2.64 (d, *J*=9.4Hz, 2H), 2.44 (d, *J*=7.3Hz, 1H), 2.27 (s, 1H), 1.83 (d, *J*=5.6Hz, 1H), 1.66-1.51 (m, 1H), 1.40 (s, 9H), 1.24 (s, 9H).

### Step 6: Synthesis of tert-butyl (S)-3-((S)-3-(3-(2-(((benzyloxy)carbonyl)amino)ethoxy)phenyl)-1-(tert-butoxy)-1-oxopropan-2-yl)pyrrolidine-1-carboxylate

*Tert-*butyl (S)-3-((S)-1-(tert-butoxy)-3-(3-hydroxyphenyl)-1-oxopropan-2-yl)pyrrolidine-1-carboxylate (950mg) was dissolved in N,N-dimethylformamide (10mL), benzyl (2-bromoethyl)carbamate (1.25g) and potassium carbonate (1.01g) were added, and the mixture was stirred at 80°C for 18h. LCMS showed the reaction was completed. The reaction mixture was added with water and extracted with ethyl acetate. The organic phases were combined, washed with saturated sodium chloride solution, dried over anhydrous sodium sulfate, filtered and concentrated to give the crude product, which was separated and purified by reversed-phase chromatography (C₁₈, 0.1% trifluoroacetic acid, acetonitrile:water=4:1) to give the target compound (690mg). LCMS(ESI)[M+Na]⁺=591.9; ¹HNMR(400MHz, DMSO-*d₆*) δ 7.54-7.41 (m, 1H), 7.39-7.26 (m, 5H), 7.16 (s, 1H), 6.84-6.71 (m, 3H), 3.95 (t, *J*=5.8Hz, 2H), 3.56-3.42 (m, 3H), 3.36 (d, *J*=6.0Hz, 4H), 3.17-3.11 (m, 1H), 2.98 (t, *J*=10.0Hz, 1H), 2.69 (d, *J*=9.1Hz, 2H), 2.35-2.20 (m, 1H), 1.84 (d, *J*=5.5Hz, 1H), 1.63-1.51 (m, 1H), 1.40 (s, 9H), 1.23 (s, 9H).

### Step 7: Synthesis of tert-butyl (S)-3-((S)-3-(3-(2-aminoethoxy)phenyl)-1-(tert-butoxy)-1-oxopropan-2-yl)pyrrolidine-1-carboxylate

A solution of *tert-*butyl (S)-3-((S)-3-(3-(2-(((benzyloxy)carbonyl)amino)ethoxy)phenyl)-1-(tert-butoxy)-1-oxopropan-2-yl)pyrrolidine-1-carboxylate (680mg) in methanol (10mL) was added with Pd/C (12.72mg, 10%). The mixture was stirred at room temperature for 18h under a hydrogen atmosphere. LCMS showed the reaction was completed. The reaction mixture was filtered and concentrated to give the target compound (360mg). LCMS(ESI)[M+H]⁺=435.6; ¹HNMR(400MHz, DMSO-*d₆*) δ 7.22-7.10 (m, 1H), 6.82-6.69 (m, 3H), 3.92 (t, *J*=5.7Hz, 2H), 3.54-3.42 (m, 1H), 3.38 (s, 3H), 3.19-3.08 (m, 1H), 2.98 (t, *J*=10.0Hz*,* 1H), 2.91 (s, 1H), 2.70 (d, *J*=9.1Hz, 2H), 2.32-2.20 (m, 1H), 1.84 (d, *J*=8.9Hz, 1H), 1.65-1.51 (m, 1H), 1.40 (s, 9H), 1.25 (s, 9H).

### Step 8: Synthesis of tert-butyl (S)-3-((S)-1-(tert-butoxy)-3-(3-formylphenyl)-1-oxopropan-2-yl)pyrrolidine-1-carboxylate

*Tert-*butyl (S)-3-((S)-3-(3-bromophenyl)-1-(tert-butoxy)-1-oxopropan-2-yl)pyrrolidine-1-carboxylate (400mg) was dissolved in N,N-dimethylformamide (5mL), sodium carbonate (186.54mg), triethylsilane (0.15mL) and [1,1'-bis(diphenylphosphino)ferrocene]palladium(II) dichloride (64.57mg), and the reaction mixture was stirred at 80°C for 18h under a carbon monoxide atmosphere. After the reaction was completed, the reaction mixture was added with water and extracted with ethyl acetate, the organic phases were combined, washed with saturated saline, dried over anhydrous sodium sulfate, filtered, concentrated and purified by column chromatography (silica gel, petroleum ether:ethyl acetate=5:1) to give the target compound (277mg). LCMS(ESI)[M+Na+acetonitrile]⁺=467.3; ¹HNMR(400MHz, DMSO-*d₆*) δ 10.00 (s, 1H), 7.79-7.73 (m, 2H), 7.58-7.50 (m, 2H), 3.57-3.50 (m, 1H), 3.39-3.37 (m, 1H), 3.20-3.11 (m, 1H), 3.02 (t, *J*=10.0Hz, 1H), 2.92-2.77 (m, 2H), 2.60-2.55 (m, 1H), 2.34-2.24 (m, 1H), 1.90-1.80 (m, 1H), 1.65-1.54 (m, 1H), 1.41 (s, 9H), 1.20 (s, 9H).

### Step 9: Synthesis of tert-butyl (S)-3-((S)-1-(tert-butoxy)-3-(3-(((2-(3-((S)-3-(tert-butoxy)-2-((S)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

*Tert-*butyl (S)-3-((S)-1-(tert-butoxy)-3-(3-formylphenyl)-1-oxopropan-2-yl)pyrrolidine-1-carboxylate (200mg) was dissolved in ethanol (5mL), *tert-*butyl (S)-3-((S)-3-(3-(2-aminoethoxy)phenyl)-1-(tert-butoxy)-1-oxopropan-2-yl)pyrrolidine-1-carboxylate (236.93mg) and titanium tetraisopropanolate (0.29mL) were added, and the reaction mixture was stirred at room temperature for 17h. Sodium borohydride (28.13mg) was added under ice bath, and the mixture was stirred at room temperature for 1h. LCMS showed the reaction was completed. The reaction was quenched with saturated ammonium chloride solution, and the mixture was filtered through celite, concentrated and separated and purified by rapid chromatography (silica gel, petroleum ether:tetrahydrofuran=3:2) to give the target compound (120mg). LCMS(ESI)[M+H]⁺=822.6.

### Step 10: Synthesis of tert-butyl (S)-3-((S)-3-(3-allylphenyl)-1-(tert-butoxy)-1-oxopropan-2-yl)pyrrolidine-1-carboxylate

Under nitrogen protection, *tert-*butyl (S)-3-((S)-3-(3-bromophenyl)-1-(tert-butoxy)-1-oxopropan-2-yl)pyrrolidine-1-carboxylate (600mg) was dissolved in 1,4-dioxane (10mL) and water (1mL), potassium phosphate (840.86mg), [1,1'-bis(diphenylphosphino)ferrocene]palladium(II) dichloride (96.88mg) and 2-allyl-4,4,5,5-tetramethyl-1,3,2-dioxaborolane (443.77mg) were added; the reaction was maintained at 90°C for 16h. LCMS showed the reaction of starting material was completed. The mixture was diluted with water, extracted with ethyl acetate, and the organic phases were combined, washed with saturated sodium chloride aqueous solution, dried over anhydrous sodium sulfate, filtered and concentrated to dryness. The crude product was separated and purified by rapid chromatography (silica gel, petroleum ether:tetrahydrofuran=10:1) to give the target compound (300mg). LCMS(ESI)[M+Na]⁺=438.3; ¹HNMR(400MHz, DMSO-*d*₆) δ 13.84-13.76 (m, 1H), 7.19 (t, *J*=7.5Hz, 1H), 7.01 (d, *J*=7.8Hz, 3H), 5.98-5.86 (m,1H), 5.11-4.98 (m, 2H), 3.57-3.45 (m, 1H), 3.38-3.33 (m, 2H), 3.30 (s, 1H), 3.22-3.08 (m, 1H), 2.97 (t, *J*=10.0Hz, 1H), 2.77-2.64 (m, 2H), 2.48-2.42 (m, 1H), 2.34-2.21 (m, 1H), 1.89-1.77 (m, 1H), 1.65-1.50 (m, 1H), 1.40 (s, 9H), 1.22 (s, 9H).

### Step 11: Synthesis of 2-(3-((S)-3-(tert-butoxy)-2-((S)-1-(tert-butoxycarbonyl)pyrrolidin-3-yl)-3-oxopropyl)phenyl)acetic acid

At room temperature, *tert-*butyl (S)-3-((S)-3-(3-allylphenyl)-1-(tert-butoxy)-1-oxopropan-2-yl)pyrrolidine-1-carboxylate (300mg) was dissolved in acetonitrile (5mL) and water (5mL), sodium periodate (772.04mg) and ruthenium trichloride hydrate (14.97mg) were added. The mixture was stirred at room temperature for 1h. The mixture was diluted with acetonitrile, filtered, and the filter cake was washed with acetonitrile. The filtrate was concentrated, diluted with water, and extracted with ethyl acetate. The organic phases were combined, washed with saturated sodium chloride aqueous solution, dried over anhydrous sodium sulfate, filtered and concentrated. The crude product was separated and purified by rapid chromatography (silica gel, petroleum ether:tetrahydrofuran=5:1) to give the target compound (120mg). LCMS(ESI)[M+Na]⁺=456.3; ¹HNMR(400MHz, DMSO-*d*₆) δ 12.25 (s, 1H), 7.21 (t, *J*=7.8Hz, 1H), 7.08 (dd, *J*=12.2, 7.4Hz, 3H), 3.56-3.48 (m, 4H), 3.21-3.09 (m, 1H), 2.99 (t, *J*=10.0Hz, 1H), 2.79-2.66 (m, 2H), 2.50-2.42 (m, 1H), 2.35-2.24 (m, 1H), 1.90-1.80 (m, 1H), 1.65-1.53 (m, 1H), 1.40 (s, 9H), 1.23 (s, 9H).

### Step 12: Synthesis of tert-butyl (S)-3-((S)-1-(tert-butoxy)-3-(3-((N-(2-(3-((S)-3-(tert-butoxy)-2-((S)-1-(tert-butoxycarbonyl)pyrrolidin-3-yl)-3-oxopropyl)phenoxy)ethyl)-2-(3-((S)-3-(tert-butoxy)-2-((S)-1-(tert-butoxycarbonyl)pyrrolidin-3-yl)-3-oxopropyl)phenyl)acetamido)methyl)phenyl)-1-oxopropan-2-yl)pyrrolidine-1-carboxylate

*Tert-*butyl (S)-3-((S)-1-(tert-butoxy)-3-(3-(((2-(3-((S)-3-(tert-butoxy)-2-((S)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate(120mg) was dissolved in N,N-dimethylformamide (3mL) solution, 2-(3-((S)-3-(tert-butoxy)-2-((S)-1-(tert-butoxycarbonyl)pyrrolidin-3-yl)-3-oxopropyl)phenyl)acetic acid (71.53mg), *N*,*N*-diisopropylethylamine (58.16mg) and O-(7-azabenzotriazol-1-yl)-N,N,N',N'-tetramethyluronium hexafluorophosphate (114.07mg) were added, and the mixture was stirred at room temperature for 3h. LCMS showed the reaction was completed. The reaction mixture was added with water and extracted with ethyl acetate. The organic phases were combined, washed with saturated sodium chloride solution, dried over anhydrous sodium sulfate, filtered, concentrated and separated and purified by rapid chromatography (C₁₈, water:acetonitrile=1:10) to give the target compound (130mg). LCMS(ESI)[M+H-Boc]⁺=1137.7; ¹HNMR(400MHz, DMSO-*d₆*) δ 7.27-6.95 (m, 9H), 6.74 (d, *J*=7.3Hz, 3H), 4.78-4.45 (m, 2H), 4.03 (s, 2H), 3.84 (s, 1H), 3.62 (d, *J*=28.9Hz, 3H), 3.48 (d, *J*=8.7Hz, 6H), 3.27-3.04 (m, 4H), 3.00-2.87 (m, 3H), 2.69 (d, *J*=10.1Hz, 7H), 2.33-2.20 (m, 3H), 2.04-1.97 (m, 1H), 1.90-1.75 (m, 3H), 1.58 (d, *J*=8.9Hz, 3H), 1.39 (s, 27H), 1.25-1.19 (m, 27H). Step 13: A solution of the product from step 12 (120mg) in 1,4-dioxane (2mL, 4.0M) was added with hydrochloric acid/dioxane solution (2mL, 4.0M), and the mixture was stirred at room temperature for 2h. LCMS showed the reaction was completed. The reaction mixture was concentrated, neutralized with sodium hydroxide solution (1.0M) and separated and purified by Prep-HPLC (C₁₈, 0.1% NH₄HCO₃ in water, acetonitrile) to give the target compound (32.6mg). LCMS(ESI)[M+H]⁺=769.6; ¹HNMR(400MHz, D₂O) δ 7.28-7.15 (m, 3H), 7.10-6.81 (m, 7H), 6.70-6.60 (m, 2H), 4.61 (s, 2H), 4.12 (s, 1H), 4.02 (s, 1H), 3.90 (s, 1H), 3.80 (s, 1H), 3.71 (s, 2H), 3.38-3.24 (m, 6H), 3.18-3.08 (m, 3H), 2.89-2.81 (m, 1H), 2.78-2.49 (m, 8H), 2.41-2.27 (m, 6H), 2.08-1.98 (m, 3H), 1.70-1.57 (m, 3H).

### Example 89 (Compound 211)

### Preparation of (R)-3-(3-(2-((3-((R)-2-carboxy-2-((S)-pyrrolidin-3-yl)ethyl)benzyl)(2-(3-((R)-2-carboxy-2-((S)-pyrrolidin-3-yl)ethyl)phenoxy)ethyl)amino)-2-oxoethyl)phenyl)-2-((S)-pyrrolidin-3-yl)propanoic acid

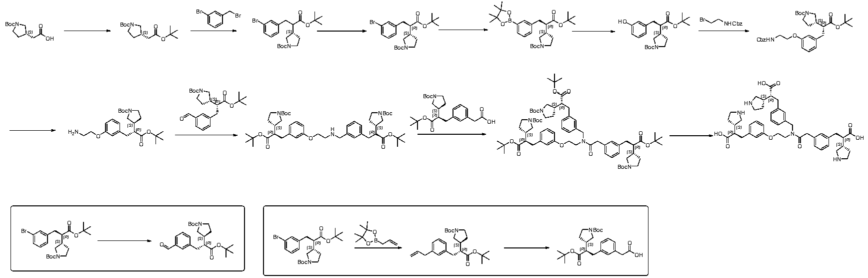

### Step 1: Synthesis of tert-butyl (S)-3-(2-(tert-butoxy)-2-oxoethyl)pyrrolidine-1-carboxylate

Under nitrogen protection, a solution of (S)-2-(1-(tert-butoxycarbonyl)pyrrolidin-3-yl)acetic acid (10g, 43.62mmol, 1eq) in 2-methyltetrahydrofuran (100mL) was added with *tert-*butyl N,N'-diisopropylcarbamimidate (43.69g, 218.08mmol, 5eq), and the reaction mixture was heated to 70°C and stirred for 16h. After cooled to room temperature, the reaction mixture was filtered to remove the filter residue. The filter cake was fully washed with methyl *tert-*butyl ether. The filtrate was concentrated under reduced pressure, and the crude product was purified by column chromatography (silica gel, tetrahydrofuran:petroleum ether=1:5) to give the target compound (11g); ¹HNMR(400MHz, DMSO-*d₆*) δ 3.54-3.45 (m, 1H), 3.38-3.33 (m, 1H), 3.27-3.16 (m, 1H), 2.94-2.84 (m, 1H), 2.49-2.39 (m, 1H), 2.38-2.32 (m, 2H), 2.07-1.96 (m, 1H), 1.63-1.51 (m, 1H), 1.50-1.40 (m, 18H).

### Step 2: Synthesis of tert-butyl (3S)-3-(3-(3-bromophenyl)-1-(tert-butoxy)-1-oxopropan-2-yl)pyrrolidine-1-carboxylate

Under nitrogen protection, *tert-*butyl (S)-3-(2-(tert-butoxy)-2-oxoethyl)pyrrolidine-1-carboxylate (20g, 70.08mmol, 1eq) was dissolved in ultra-dry tetrahydrofuran (280mL), and after the mixture was cooled to -5°C in an ice-salt bath, lithium bis(trimethylsilyl)amide (84.1mmol, 1.2eq) was slowly added to the reaction system, the mixture was stirred at -5°C for 1h. Then a solution of 1-bromo-3-(bromomethyl)benzene (19.27g, 77.09mmol, 1.1eq) in tetrahydrofuran (20mL) was slowly added dropwise to the reaction mixture which was heated to room temperature and stirred for 15h. LCMS showed the reaction was completed. The reaction mixture was quenched with saturated ammonium chloride solution, diluted with water, and extracted with ethyl acetate. The organic phase was washed with saturated saline, dried over anhydrous sodium sulfate, filtered, and concentrated. The crude product was separated and purified by reversed-phase column chromatography (C₁₈, 0.1% trifluoroacetic acid, water:acetonitrile=1:4) to give the target compound (10g). LCMS(ESI)[M+Na]⁺=476; ¹HNMR(400MHz, DMSO-d6) δ 7.44-7.38 (m, 2H), 7.28-7.19 (m, 2H), 3.66-3.56 (m, 1H), 3.42-3.29 (m, 2H), 3.23-3.11 (m, 1H), 3.05-2.86 (m, 2H), 2.72-2.63 (m, 1H), 2.35-2.21 (m, 1H), 2.14-2.01 (m, 1H), 1.76-1.60 (m, 1H), 1.43-1.36 (m, 9H), 1.24 (s, 9H).

### Step 3: Synthesis of tert-butyl (S)-3-((R)-3-(3-bromophenyl)-1-(tert-butoxy)-1-oxopropan-2-yl)pyrrolidine-1-carboxylate

*Tert-*butyl (3S)-3-(3-(3-bromophenyl)-1-(tert-butoxy)-1-oxopropan-2-yl)pyrrolidine-1-carboxylate (10g) was separated by chiral column to give the target compound (4.0g).

### Step 4: Synthesis of tert-butyl (S)-3-((R)-1-(tert-butoxy)-1-oxo-3-(3-(4,4,5,5-tetramethyl-1,3,2-dioxaborolan-2-yl)phenyl)propan-2-yl)pyrrolidine-1-carboxylate

Under nitrogen protection, *tert-*butyl (S)-3-((R)-3-(3-bromophenyl)-1-(tert-butoxy)-1-oxopropan-2-yl)pyrrolidine-1-carboxylate (1.5g, 3.3mmol, 1eq) was dissolved in 1,4-dioxane (15mL), and bis(pinacolato)diboron (1.68g, 6.6mmol, 2eq), potassium acetate (971.59mg, 9.9mmol, 3eq) and [1,1'-bis(diphenylphosphino)ferrocene]palladium(II) dichloride (242.13mg, 0.33mmol, 0.1eq) were added; the reaction mixture was stirred at 90°C for 18h. LCMS showed the reaction was completed. The mixture was filtered, concentrated, and separated and purified by rapid chromatography (silica gel, petroleum ether:tetrahydrofuran=10:1) to give the target compound (1.4g). LCMS(ESI)[M+Na]⁺=524.3; ¹HNMR(400MHz, DMSO-*d₆*) δ 7.61-7.45 (m, 2H), 7.39-7.24 (m, 2H), 3.39 (d, *J*=8.3Hz, 1H), 3.33-3.30 (m, 1H), 3.24-3.11 (m, 1H), 3.05-2.85 (m, 2H), 2.76-2.64 (m, 1H), 2.49-2.41 (m, 1H), 2.36-2.22 (m, 1H), 2.15-2.02 (m, 1H), 1.68 (d, *J*=6.0Hz, 1H), 1.39 (s, 9H), 1.29 (s, 12H), 1.24 (s, 9H).

### Step 5: Synthesis of tert-butyl (S)-3-((R)-1-(tert-butoxy)-3-(3-hydroxyphenyl)-1-oxopropan-2-yl)pyrrolidine-1-carboxylate

*Tert-*butyl (S)-3-((R)-1-(tert-butoxy)-1-oxo-3-(3-(4,4,5,5-tetramethyl-1,3,2-dioxaborolan-2-yl)phenyl)propan-2-yl)pyrrolidine-1-carboxylate (1.4g, 2.79mmol, 1eq) was dissolved in methanol (10mL), hydrogen peroxide (0.95mL, 27.92mmol, 10eq) was added, and the mixture was stirred at room temperature for 3h. LCMS showed the reaction was completed. The reaction mixture was added with water and extracted with ethyl acetate. The organic phases were combined, washed with saturated sodium chloride solution, dried over anhydrous sodium sulfate, filtered, concentrated and separated and purified by rapid chromatography (silica gel, petroleum ether:tetrahydrofuran=5:1) to give the target compound (1g). LCMS(ESI)[M+Na]⁺=414.1; ¹HNMR(400MHz, DMSO-*d₆*) δ 9.22 (s, 1H), 7.20-6.90 (m, 1H), 6.66-6.50 (m, 3H), 3.40-3.34 (m, 1H), 3.32-3.29 (m, 1H), 3.22-3.09 (m, 1H), 3.03-2.88 (m, 1H), 2.83-2.71 (m, 1H), 2.65-2.55 (m, 1H), 2.46-2.36 (m, 1H), 2.30-2.18 (m, 1H), 2.06 (d, *J*=10.3Hz, 1H), 1.70-1.54 (m, 1H), 1.38 (s, 9H), 1.25 (s, 9H).

### Step 6: Synthesis of tert-butyl (S)-3-((R)-3-(3-(2-(((benzyloxy)carbonyl)amino)ethoxy)phenyl)-1-(tert-butoxy)-1-oxopropan-2-yl)pyrrolidine-1-carboxylate

*Tert-*butyl (S)-3-((R)-1-(tert-butoxy)-3-(3-hydroxyphenyl)-1-oxopropan-2-yl)pyrrolidine-1-carboxylate (1g, 2.55mmol, 1eq) was dissolved in N,N-dimethylformamide (10mL), benzyl (2-bromoethyl)carbamate (1.32g, 5.11mmol, 2eq) and potassium carbonate (1.06g, 7.66mmol, 3eq) were added, and the reaction mixture was stirred at 80°C for 18h. LCMS showed the reaction was completed. The reaction mixture was added with water and extracted with ethyl acetate. The organic phases were combined, washed with saturated sodium chloride solution, dried over anhydrous sodium sulfate, filtered and concentrated, and the crude product was separated and purified by reversed-phase chromatography (C₁₈, 0.1% trifluoroacetic acid, acetonitrile:water=4:1) to give the target compound (700mg). LCMS(ESI)[M+H]⁺=569.4; ¹HNMR(400MHz, DMSO-*d₆*) δ 7.47 (s, 1H), 7.40-7.31 (m, 5H), 7.19-7.14 (m, 1H), 6.77-6.73 (m, 3H), 5.03 (s, 2H), 4.28 (s, 2H), 3.95 (t, *J*=5.8Hz, 2H), 3.41-3.37 (m, 1H), 3.33-3.28 (m, 1H), 3.21-3.12 (m, 1H), 3.01-2.91 (m, 1H), 2.85-2.78 (m, 1H), 2.69-2.61 (m, 1H), 2.29-2.19 (m, 1H), 2.11-1.97 (m, 2H), 1.70-1.60 (m, 1H), 1.38 (s, 9H), 1.23 (s, 9H).

### Step 7: Synthesis of tert-butyl (S)-3-((R)-3-(3-(2-aminoethoxy)phenyl)-1-(tert-butoxy)-1-oxopropan-2-yl)pyrrolidine-1-carboxylate

A solution of *tert-*butyl (S)-3-((R)-3-(3-(2-(((benzyloxy)carbonyl)amino)ethoxy)phenyl)-1-(tert-butoxy)-1-oxopropan-2-yl)pyrrolidine-1-carboxylate (700mg, 1.23mmol, 1eq) in methanol (10mL) was added with Pd/C (13.1mg, 0.12mmol, 0.1eq, 10%). The mixture was stirred at room temperature for 18h under a hydrogen atmosphere. LCMS showed the reaction was completed. The reaction mixture was filtered and concentrated to give the target compound (470mg). LCMS(ESI)[M+H]⁺=435.6; ¹HNMR(400MHz, DMSO-*d₆*) δ 7.24-7.14 (m, 1H), 6.88-6.66 (m, 3H), 4.09-3.99 (m, 3H), 3.40-3.32 (m, 2H), 3.17 (t, *J*=8.9Hz, 1H), 3.11-3.01 (m, 2H), 3.00-2.92 (m, 1H), 2.88-2.80 (m, 1H), 2.71-2.62 (m, 1H), 2.31-2.19 (m, 1H), 2.06 (d, *J*=7.8Hz, 1H), 1.74-1.58 (m, 1H), 1.38 (s, 9H), 1.25 (s, 9H).

### Step 8: Synthesis of tert-butyl (S)-3-((R)-1-(tert-butoxy)-3-(3-formylphenyl)-1-oxopropan-2-yl)pyrrolidine-1-carboxylate

*Tert-*butyl (S)-3-((R)-3-(3-bromophenyl)-1-(tert-butoxy)-1-oxopropan-2-yl)pyrrolidine-1-carboxylate (500mg, 1.1mmol, 1eq) was dissolved in N,N-dimethylformamide (6mL), sodium carbonate (233.25mg, 2.2mmol, 2eq), triethylsilane (0.27mL, 1.65mmol, 1.5eq) and [1,1'-bis(diphenylphosphino)ferrocene]palladium(II) dichloride (80.74mg, 0.11mmol, 0.1eq) were added; and the reaction mixture was stirred at 80°C for 18h under a carbon monoxide atmosphere. After the reaction was completed, the reaction mixture was added with water and extracted with ethyl acetate. The organic phases were combined, washed with saturated saline, dried over anhydrous sodium sulfate, filtered and concentrated to give a crude product, which was purified by column chromatography (silica gel, petroleum ether:ethyl acetate=5:1) to give the target compound (380mg). LCMS(ESI)[M+Na]⁺=426.2; ¹HNMR(400MHz, DMSO-*d₆*) δ 9.99 (s, 1H), 7.79-7.72 (m, 2H), 7.58-7.49 (m, 2H), 3.43-3.34 (m, 2H), 3.23-3.13 (m, 1H), 3.03-2.91 (m, 2H), 2.82-2.74 (m, 1H), 2.58-2.52 (m, 1H), 2.34-2.25 (m, 1H), 2.15-2.04 (m, 1H), 1.74-1.63 (m, 1H), 1.39 (d, *J*=3.2Hz, 9H), 1.20 (s, 9H).

### Step 9: Synthesis of tert-butyl (S)-3-((R)-1-(tert-butoxy)-3-(3-(((2-(3-((R)-3-(tert-butoxy)-2-((S)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate

*Tert-*butyl (S)-3-((R)-1-(tert-butoxy)-3-(3-formylphenyl)-1-oxopropan-2-yl)pyrrolidine-1-carboxylate (274mg, 0.68mmol, 1eq) was dissolved in ethanol (6mL), followed by adding *tert-*butyl (S)-3-((R)-3-(3-(2-aminoethoxy)phenyl)-1-(tert-butoxy)-1-oxopropan-2-yl)pyrrolidine-1-carboxylate (324.6mg, 0.75mmol, 1.1eq) and titanium tetraisopropanolate (0.4mL, 1.36mmol, 2eq). The reaction mixture was stirred at room temperature for 17h. Sodium borohydride (38.53mg, 1.02mmol, 1.5eq) was added under ice bath, and the mixture was stirred at room temperature for 1h. LCMS showed the reaction was completed. The reaction was quenched with saturated ammonium chloride solution, and the mixture was filtered through celite, concentrated and separated and purified by rapid chromatography (silica gel, petroleum ether:tetrahydrofuran=3:2) to give the target compound (290mg). LCMS(ESI)[M+H]⁺=822.6.

### Step 10: Synthesis of tert-butyl (S)-3-((R)-3-(3-allylphenyl)-1-(tert-butoxy)-1-oxopropan-2-yl)pyrrolidine-1-carboxylate

Under nitrogen protection, *tert-*butyl (S)-3-((R)-3-(3-bromophenyl)-1-(tert-butoxy)-1-oxopropan-2-yl)pyrrolidine-1-carboxylate (1g, 2.2mmol, 1eq) was dissolved in tetrahydrofuran (16mL) and water (4mL), potassium phosphate (1.4g, 6.6mmol, 3eq), 2-allyl-4,4,5,5-tetramethyl-1,3,2-dioxaborolane (0.92g, 5.5mmol, 2.5eq) and [1,1'-bis(diphenylphosphino)ferrocene]palladium(II) dichloride (161.42mg, 0.22mmol, 0.1eq) were added, and the reaction mixture was stirred at 90°C for 3h. After the reaction was completed, the reaction mixture was added with water, extracted with ethyl acetate, dried over anhydrous sodium sulfate, filtered and concentrated to give a crude product, which was purified by column chromatography (silica gel, petroleum ether:tetrahydrofuran=10:1) to give the target compound (700mg). LCMS(ESI)[M+H]⁺=416; ¹HNMR(400MHz, DMSO-*d₆*) δ 7.19 (t, *J*=7.5Hz, 1H), 7.07-6.94 (m, 3H), 6.00-5.85 (m, 1H), 5.11-4.96 (m, 2H), 3.42-3.33 (m, 2H), 3.32-3.29 (m, 2H), 3.22-3.10 (m, 1H), 3.01-2.89 (m, 1H), 2.88-2.81 (m, 1H), 2.70-2.62 (m, 1H), 2.48-2.41 (m, 1H), 2.31-2.19 (m, 1H), 2.13-2.00 (m, 1H), 1.72-1.58 (m, 1H), 1.38 (d, *J*=2.4Hz, 9H), 1.23 (s, 9H).

### Step 11: Synthesis of 2-(3-((R)-3-(tert-butoxy)-2-((S)-1-(tert-butoxycarbonyl)pyrrolidin-3-yl)-3-oxopropyl)phenyl)acetic acid

*Tert-*butyl (S)-3-((R)-3-(3-allylphenyl)-1-(tert-butoxy)-1-oxopropan-2-yl)pyrrolidine-1-carboxylate (700mg, 1.68mmol, 1eq) was dissolved in acetonitrile (15mL) and water (15mL), ruthenium trichloride hydrate (37.97mg, 0.17mmol, 0.1eq) and sodium periodate (2161.7mg, 10.11mmol, 6eq) were added, and the reaction was maintained at room temperature for 1h. After the reaction was completed, the reaction mixture was added with acetonitrile, filtered, and the filtrate was concentrated, added with water, extracted with ethyl acetate, dried over anhydrous sodium sulfate, filtered and concentrated to give a crude product, which was purified by column chromatography (silica gel, petroleum ether:tetrahydrofuran=5:1) to give the target compound (354mg). LCMS(ESI)[M+Na]⁺=456.2; ¹HNMR(400MHz, DMSO-*d₆*) δ 12.25 (s, 1H), 7.24-7.17 (m, 1H), 7.12-7.03 (m, 3H), 3.49 (s, 2H), 3.42-3.36 (m, 2H), 3.20-3.13 (m, 1H), 3.01-2.92 (m, 1H), 2.88-2.81 (m, 1H), 2.71-2.64 (m, 1H), 2.46-2.41 (m, 1H), 2.30-2.21 (m, 1H), 2.12-2.03 (m, 1H), 1.70-1.59 (m, 1H), 1.38 (s, 9H), 1.23 (s, 9H).

### Step 12: Synthesis of tert-butyl (S)-3-((R)-1-(tert-butoxy)-3-(3-((N-(2-(3-((R)-3-(tert-butoxy)-2-((S)-1-(tert-butoxycarbonyl)pyrrolidin-3-yl)-3-oxopropyl)phenoxy)ethyl)-2-(3-((R)-3-(tert-butoxy)-2-((S)-1-(tert-butoxycarbonyl)pyrrolidin-3-yl)-3-oxopropyl)phenyl)acetamido)methyl)phenyl)-1-oxopropan-2-yl)pyrrolidine-1-carboxylate

*Tert-*butyl (S)-3-((R)-1-(tert-butoxy)-3-(3-(((2-(3-((R)-3-(tert-butoxy)-2-((S)-1-(tert-butoxycarbonyl)pyrrolidin-3-yl)-3-oxopropyl)phenoxy)ethyl)amino)methyl)phenyl)-1-oxopropan-2-yl)pyrrolidine-1-carboxylate (235mg, 0.29mmol, 1eq) was dissolved in N,N-dimethylformamide (4mL), followed by adding 2-(3-((R)-3-(tert-butoxy)-2-((S)-1-(tert-butoxycarbonyl)pyrrolidin-3-yl)-3-oxopropyl)phenyl)acetic acid (136.33mg, 0.31mmol, 1.1eq), *N*,*N*-diisopropylethylamine (110.83mg, 0.86mmol, 3eq) and O-(7-azabenzotriazol-1-yl)-N,N,N',N'-tetramethyluronium hexafluorophosphate (217.39mg, 0.57mmol, 2eq). The mixture was stirred at room temperature for 3h. LCMS showed the reaction was completed. The reaction mixture was added with water and extracted with ethyl acetate. The organic phases were combined, washed with saturated sodium chloride solution, dried, concentrated and separated and purified by reversed-phase chromatography (C₁₈, water:acetonitrile=1:10) to give the target compound (300mg). LCMS(ESI)[M+H-Boc]⁺=1137.7; ¹HNMR(400MHz, DMSO-*d₆*) δ 7.25-6.97 (m, 9H), 6.79-6.71 (m, 3H), 4.66-4.50 (m, 2H), 4.04 (s, 2H), 3.84 (s, 1H), 3.67-3.53 (m, 3H), 3.15 (s, 4H), 3.01-2.89 (m, 3H), 2.86-2.76 (m, 3H), 2.74-2.55 (m, 4H), 2.50-2.37 (m, 6H), 2.31-2.20 (m, 3H), 2.10-1.94 (m, 4H), 1.66-1.54 (m, 3H), 1.38 (s, 27H), 1.25-1.19 (m, 27H).

Step 13: A solution of the product from step 12 (250mg, 0.2mmol, 1eq) in 1,4-dioxane (3mL) was added with hydrochloric acid/dioxane solution (3mL, 4.0M) and the mixture was stirred at room temperature for 2h. LCMS showed the reaction was completed. The reaction mixture was concentrated, neutralized with sodium hydroxide (1.0M) solution and separated and purified by Prep-HPLC (C₁₈, 0.1% NH₄HCO₃ in water, acetonitrile) to give the target compound (83.41mg). LCMS(ESI)[M+H]⁺=769.5; ¹HNMR(400MHz, D₂O) δ 7.26-7.14 (m, 3H), 7.10-6.81 (m, 7H), 6.70-6.58 (m, 2H), 4.63 (s, 2H), 4.10 (d, *J*=5.3Hz, 1H), 4.00 (s, 1H), 3.89 (s, 1H), 3.79 (s, 1H), 3.70 (d, *J*=7.2Hz, 2H), 3.35-3.25 (m, 6H), 3.17-3.07 (m, 3H), 2.93-2.83 (m, 3H), 2.74 (t, *J*=8.4Hz, 3H), 2.66-2.55 (m, 3H), 2.39-2.26 (m, 6H), 2.11 (d, *J*=21.7Hz, 3H), 1.64-1.46 (m, 3H).

### Synthesis of compounds 1-4, 7, 9, 10, 13-46, 48, 50-61, 63, 64, 66, 68-70, 72, 73-79, 82-88, 92, 114-116, 118-120, 124, 125, 154-157, 169-175, 179, 181-189, 191-192, 194-208, 212-228

Compounds 29-31 were prepared according to the preparation method of Example 1.

Compounds 1-4, 7, 9, 10, 13-46, 48, 50-61, 63, 64, 66, 68-70, 72, 73-79, 82-88, 92, 114-116, 118-120, 124, 125, 172 were prepared according to the preparation method of Examples 1-8.

Specifically, for example
Compounds 1, 7, 9, 10, 14-26, 32-46, 48, 50-61, 118-120, and 124 were prepared according to the preparation method of Examples 2 and 4;
Compounds 13, 79, 83-84, and 114-116 were prepared according to the preparation method of Example 3;
Compounds 2-4 were prepared according to the preparation method of Example 5;
Compounds 27, 28, 63, 64, 66, 68-70, 72, and 73 were prepared according to the preparation method of Example 6;
Compounds 170, 171, 174, 175, 204-208 were prepared according to the preparation method of Compounds 152 and 176.
Compounds 154 and 155 were prepared according to the preparation method of Compound 150.
Compounds 156 and 157 were prepared according to the preparation method of Compound 129.
Compounds 169, 191, 192, 194-196 were prepared according to the preparation method of Compound 190 and 193.
Compounds 179, 181-189 were prepared according to the preparation method of Compound 177, 178, and 180.
Compounds 197-203 were prepared according to the preparation method of Compounds 74, 96, and 138.
Compounds 173, 212-221 were prepared according to the preparation method of Compound 96.
Compound 222 was prepared according to the preparation method of Compound 160.
Compounds 224 and 225 were prepared according to the preparation method of Compounds 96 and 146.
Compound 226 was prepared according to the preparation method of Compounds 146 and 147;
Compounds 223 and 227 were prepared according to the preparation method of Compound 158;
Compound 228 was prepared according to the preparation method of Compound 159.

| Comp. No. | Chemical Structure | LCMS (ESI) [M+H]⁺ | Comp. No. | Chemical Structure | LCMS (ESI) [M+H]⁺ |
|---|---|---|---|---|---|
| 1 | 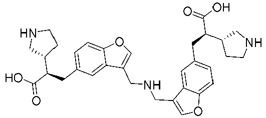 | 560.27 | 2 | 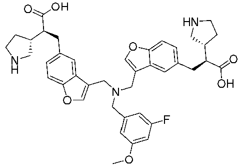 | 698.32 |
| 3 | 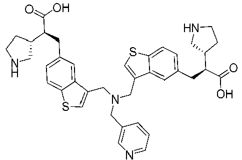 | 683.26 | 4 | 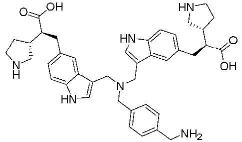 | 677.37 |
| 7 | 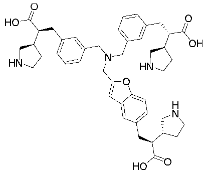 | 751.40 | 9 | 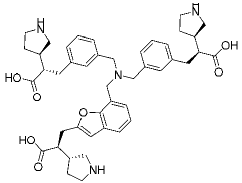 | 751.40 |
| 10 | 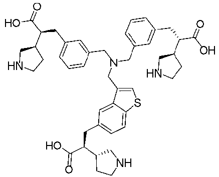 | 767.38 | 13 | 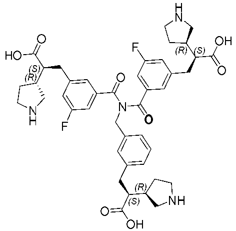 | 775.34 |
| 14 | 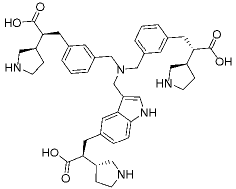 | 750.42 | 15 | 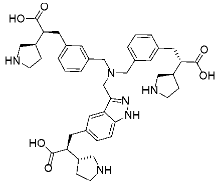 | 751.41 |
| 16 | 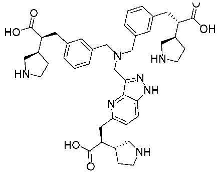 | 752.41 | 17 | 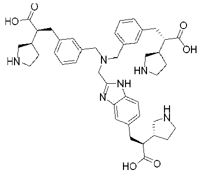 | 751.41 |
| 18 | 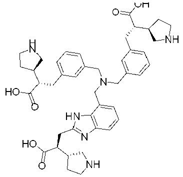 | 751.41 | 19 | 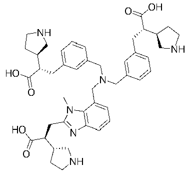 | 765.43 |
| 20 | 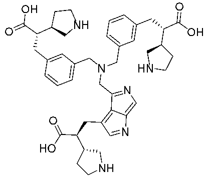 | 737.39 | 21 | 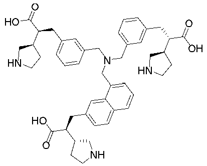 | 761.42 |
| 22 | 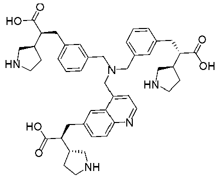 | 762.42 | 23 | 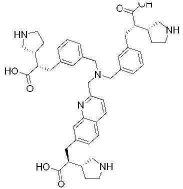 | 762.42 |
| 24 | 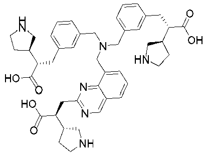 | 763.42 | 25 | 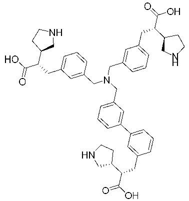 | 787.44 |
| 26 | 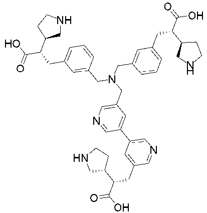 | 789.73 | 27 | 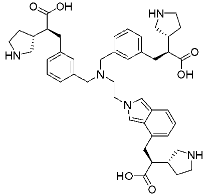 | 764.43 |
| 28 | 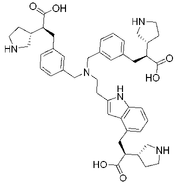 | 764.43 | 29 | 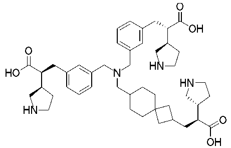 | 757.48 |
| 30 | 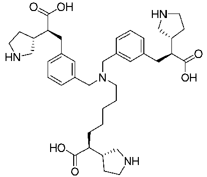 | 677.42 | 31 | 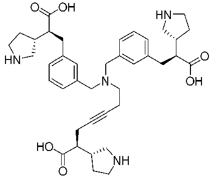 | 673.39 |
| 32 | 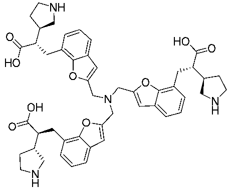 | 831.39 | 33 | 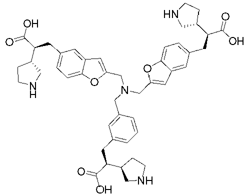 | 791.39 |
| 34 | 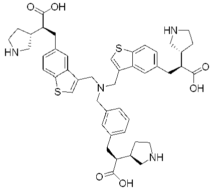 | 823.35 | 35 | 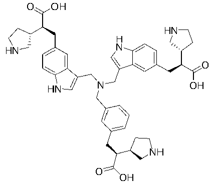 | 789.43 |
| 36 | 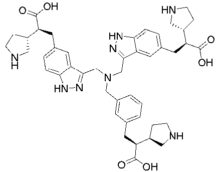 | 791.42 | 37 | 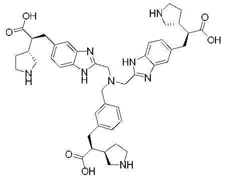 | 791.42 |
| 38 | 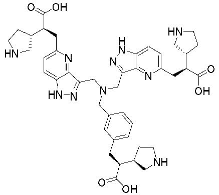 | 793.41 | 39 | 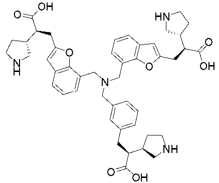 | 791.39 |
| 40 | 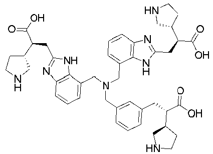 | 791.42 | 41 | 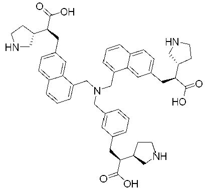 | 811.44 |
| 42 | 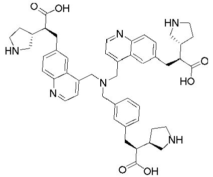 | 813.43 | 43 | 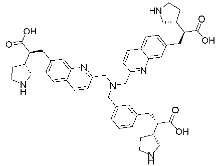 | 813.43 |
| 44 | 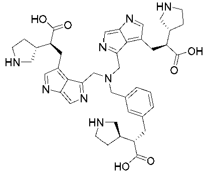 | 763.39 | 45 | 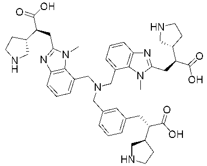 | 819.45 |
| 46 | 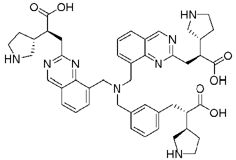 | 815.42 | 48 | 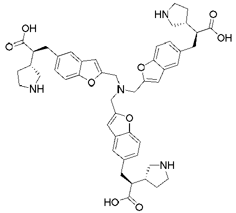 | 831.39 |
| 50 | 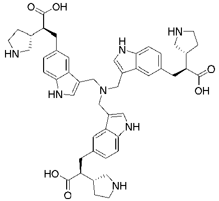 | 828.44 | 51 | 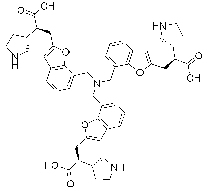 | 831.39 |
| 136 | 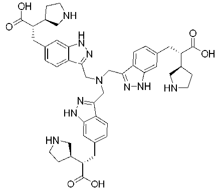 | 831.42 | 53 | 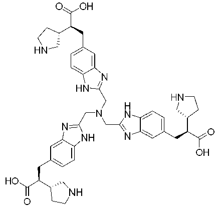 | 831.42 |
| 54 | 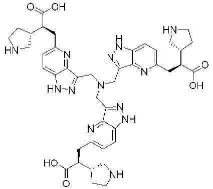 | 834.41 | 55 | 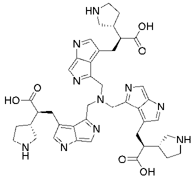 | 789.38 |
| 56 | 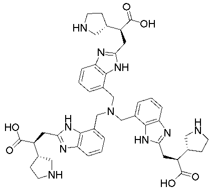 | 831.42 | 57 | 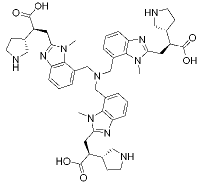 | 873.47 |
| 58 | 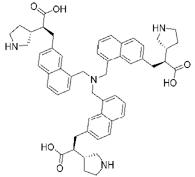 | 861.45 | 59 | 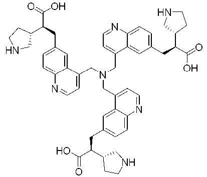 | 864.44 |
| 60 | 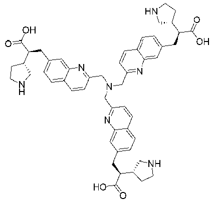 | 864.44 | 61 | 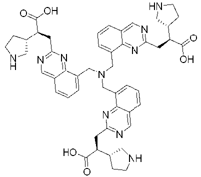 | 867.44 |
| 63 | 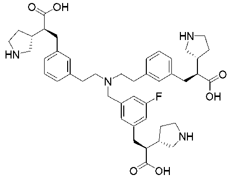 | 757.43 | 64 | 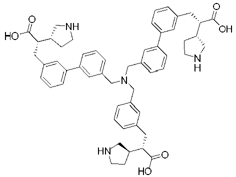 | 862.47 |
| 66 | 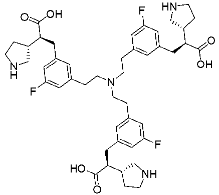 | 807.42 | 68 | 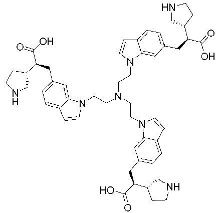 | 870.48 |
| 69 | 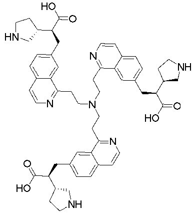 | 906.48 | 70 | 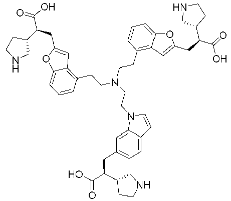 | 872.45 |
| 72 | 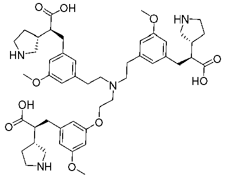 | 859.48 | 73 | 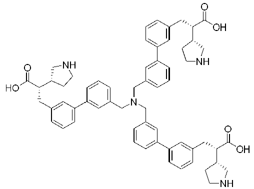 | 939.50 |
| 74 | 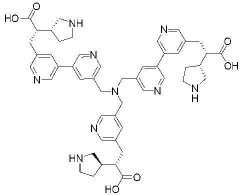 | 867.45 | 75 | 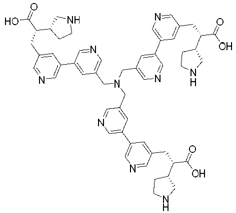 | 944.47 |
| 76 | 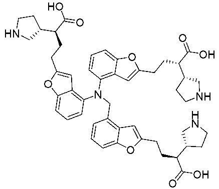 | 845.40 | 77 | 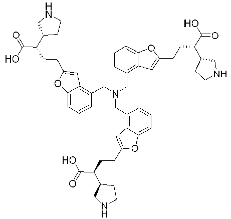 | 873.44 |
| 78 | 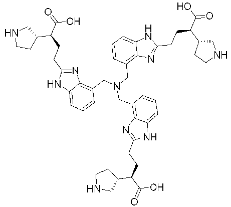 | 873.47 | 79 | 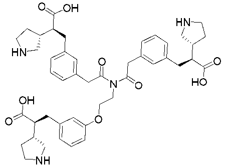 | 797.40 |
| 82 | 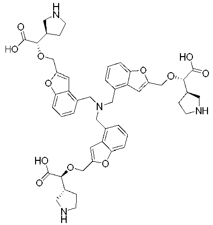 | 879.37 | 83 | 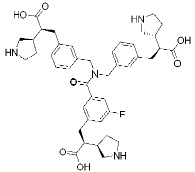 | 743.37 |
| 84 | 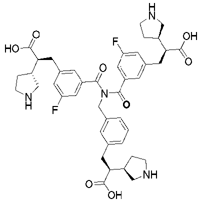 | 775.34 | 85 | 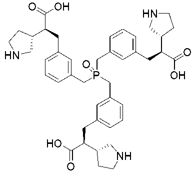 | 744.37 |
| 86 | 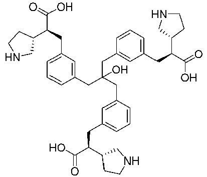 | 726.40 | 87 | 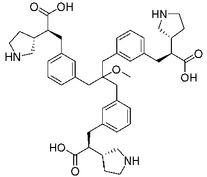 | 740.42 |
| 88 | 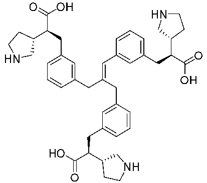 | 708.39 | 92 | 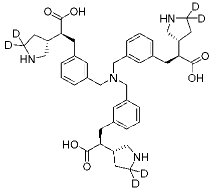 | 717.44 |
| 114 | 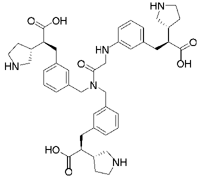 | 754.40 | 115 | 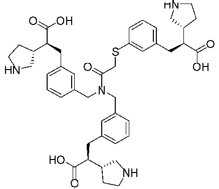 | 771.37 |
| 116 | 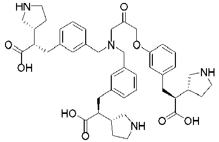 | 769.40 | 118 | 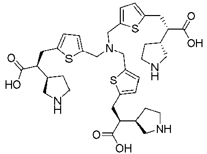 | 729.40 |
| 119 | 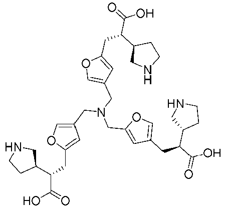 | 681.40 | 120 | 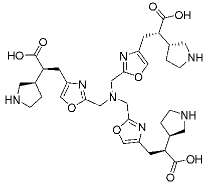 | 684.33 |
| 124 | 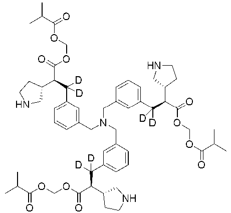 | 1017.50 | 125 | 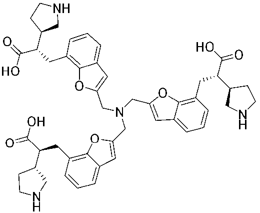 | 831.39 |
| 154 | 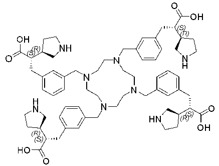 | 1097.80 | 155 | 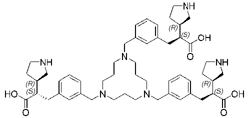 | 865.47 |
| 156 | 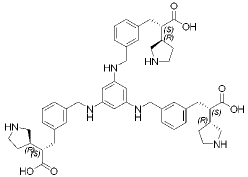 | 817.58 | 157 | 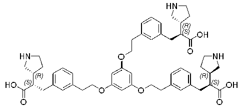 | 862.64 |
| 169 | 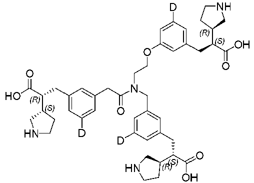 | 772.64 | 170 | 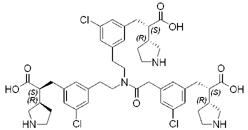 | 869.51 |
| 171 | 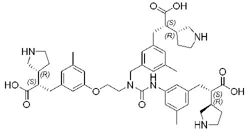 | 812.67 | 172 | 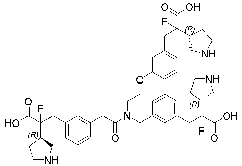 | 823.56 |
| 173 | 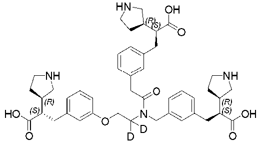 | 771.64 | 174 | 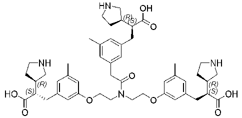 | 841.60 |
| 175 | 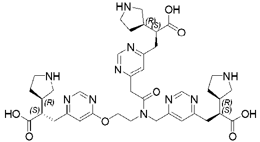 | 775.52 | 179 | 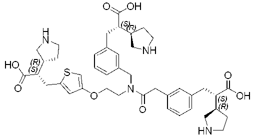 | 775.56 |
| 181 | 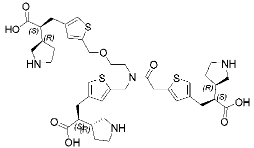 | 801.43 | 182 | 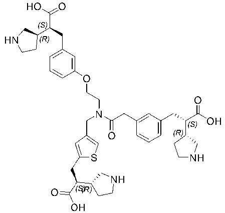 | 775.64 |
| 183 | 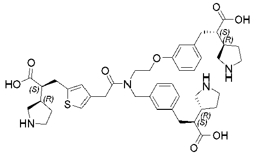 | 775.37 | 184 | 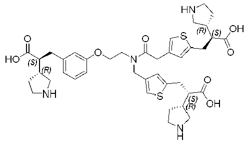 | 781.50 |
| 185 | 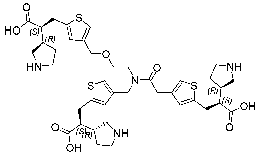 | 801.41 | 186 | 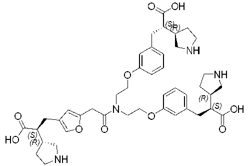 | 789.57 |
| 187 | 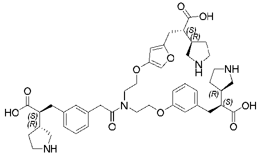 | 789.63 | 188 | 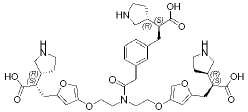 | 779.53 |
| 189 | 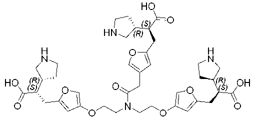 | 769.51 | 191 | 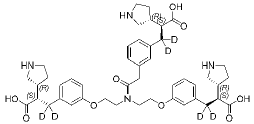 | 805.68 |
| 192 | 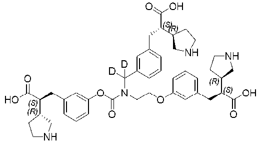 | 773.52 | 194 | 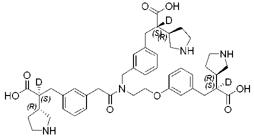 | 772.61 |
| 195 | 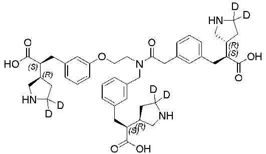 | 775.56 | 196 | 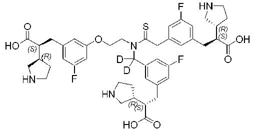 | 841.60 |
| 197 | 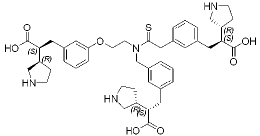 | 785.40 | 198 | 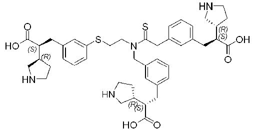 | 801.51 |
| 199 | 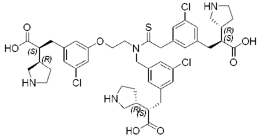 | 887.42 | 200 | 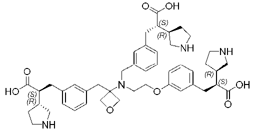 | 797.40 |
| 201 | 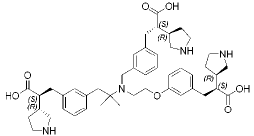 | 783.60 | 202 | 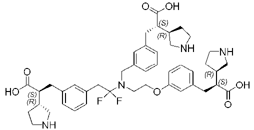 | 791.60 |
| 203 | 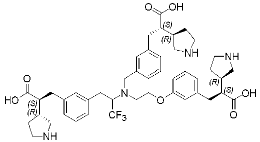 | 823.40 | 204 | 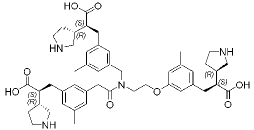 | 811.60 |
| 205 | 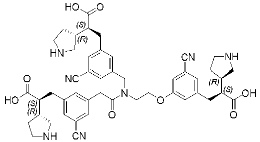 | 844.50 | 206 | 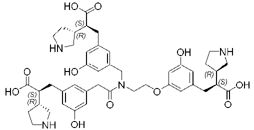 | 817.50 |
| 207 | 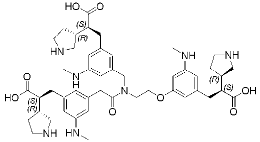 | 856.70 | 208 | 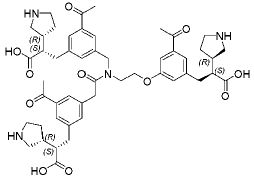 | 895.50 |
| 212 | 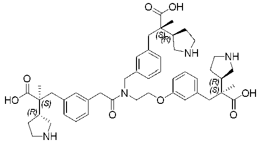 | 811.52 | 213 | 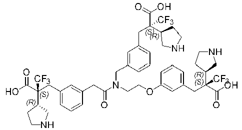 | 973.60 |
| 214 | 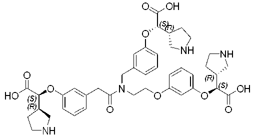 | 775.48 | 215 | 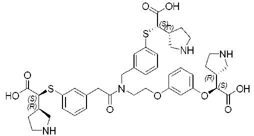 | 807.38 |
| 216 | 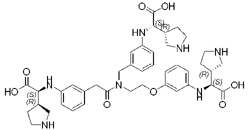 | 772.80 | 217 | 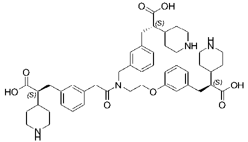 | 811.46 |
| 218 | 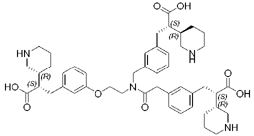 | 811.46 | 219 | 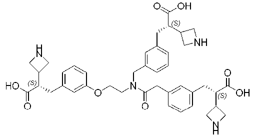 | 727.52 |
| 220 | 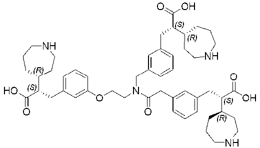 | 853.47 | 221 | 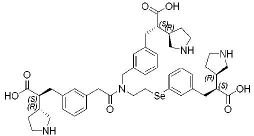 | 833.28 |
| 222 | 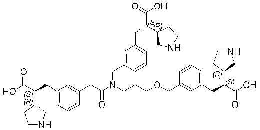 | 797.50 | 223 | 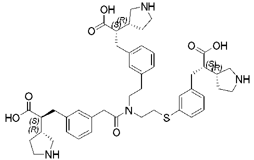 | 799.62 |
| 224 | 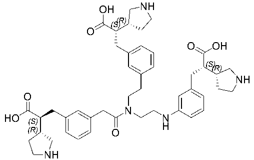 | 782.68 | 225 | 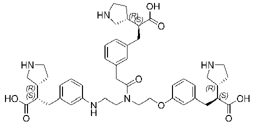 | 798.67 |
| 226 | 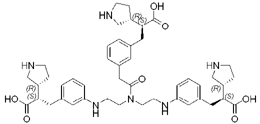 | 797.60 | 227 | 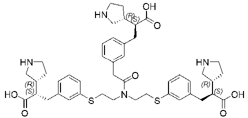 | 831.50 |
| 228 | 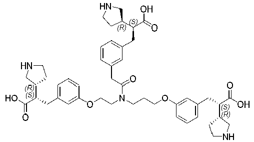 | 813.54 | | | |

The following are the effect tests and data of the compound of the invention.

### Biological Test Evaluation

### Test Example 1 and Methods of ELISA Assay on Screening Lp(a) Assembly Inhibition

1. Reagents, Consumables, Instruments

| **Reagents** | **Manufacturer** | **Catalog No.** |
|---|---|---|
| HepG2 cells overexpressing ApoA protein | \ | \ |
| DMEM medium | Gibco | 11995-065 |
| DPBS buffer | Invitrogen | 14190-250 |
| Trypan Blue | Invitrogen | T10282 |
| FBS | Gibco | 10091148 |
| DMSO | Solarbio | D8371 |
| Penicillin-streptomycin | Solarbio | P1400 |
| Puromycin | Gibco | A1113802 |
| DuoSet ELISA kit | R&D | DY008 |
| EACA | Sigma | A2504 |
| Tween-20 | Solarbio | T8220 |
| PBS buffer | Solarbio | P1020 |

| **Consumables** | **Manufacturer** | **Catalog No.** |
|---|---|---|
| Culture flask (T75) | Corning | 430641 |
| 96-well plate | Corning | 3599 |
| Centrifuge tubes (15mL, 50mL) | BD | 352096, 352070 |
| Reagent reservoir | JET BIOFIL | LTT001050 |

| **Instruments** | **Manufacturer** | **Catalog No.** |
|---|---|---|
| Biosafety cabinet | Thermo Scientific | 1300 Series A2 |
| Centrifuge | Eppendorf | 5804R |
| Carbon dioxide incubator | Thermo Scientific | 371 |
| Cell counter | Invitrogen | C10281 |
| Dispenser | BIOHIT | Easypet |
| Microscope | Olympus | CKX41 |
| Microplate reader | Perkin Elmer | Envision 2104 |

2. Cell Culture Media Preparation
2.1. Complete Cell Culture Media

| | |
|---|---|
| DMEM medium | 90% |
| FBS | 10% |
| Penicillin-streptomycin | 1% |
| Puromycin | 2ug/ml |

2.2. Test Culture Media

| | |
|---|---|
| DMEM medium | 90% |
| FBS | 10% |
| Penicillin-streptomycin | 1% |
| HEPES buffer | 20mM |

3. Test Procedure
3.1 Cell Culture and Passage
   1) Preheated the culture media, DPBS, and pancreatin in a 37°C water bath in advance.
   2) Digested cells with pancreatin, transferred the cell suspension to a 15 mL centrifuge tube, and centrifuged at 1000 rpm for 5 min.
   3) Resuspended the cells with culture media and transferred the cell suspension to a new T75 cell culture flask.
   4) Cultured at 37°C in a 5% CO₂ incubator.
3.2. Cell Seeding and Drug Administration
   1) Seeded HepG2 cells overexpressing of ApoA protein into a 96-well plate at 100 µL per well and incubated overnight for attachment.
   2) Replaced with fresh culture media the next day, then added diluted compounds, and cultured the cell plate in a 5% CO₂, 37°C incubator for 24h.
3.3. Preparation of Cell Culture Supernatant
   1) Added 10 µL of 1.5 M EACA to each well.
   2) Centrifuged at 1000 rpm for 1 min, and transferred 100 µL of cell culture supernatant to a pre-coated ELISA plate.
3.4. ELISA Test
   1) Added 100 µL of capture antibody to each well of a pre-coated ELISA plate and incubated overnight at 25°C.
   2) Added 300 µL of washing solution to each well and washed 5 times for 1min each time.
   3) Added 100 µL of blocking solution to each well and incubated at 25°C for 2h.
   4) Added 300 µL of washing solution to each well and washed 5 times for 1min each time.
   5) Added 100 µL of sample to each well and incubated at 25°C for 1h.
   6) Added 300 µL of washing solution to each well and washed 5 times for 1min each time.
   7) Added 100 µL of detection antibody to each well and incubated at 25°C for 1h.
   8) Added 300 µL of washing solution to each well and washed 5 times for 1min each time.
   9) Added 100 µL substrate solution (Color Reagent A: Color Reagent B, 1:1) to each well and incubated for 20min at 25°C in the dark.
   10) Added 50 mL of stop solution to each well, gently blowed and sucked several times with the pipette tip, and read within 5 min.

4. IC50 Data Analysis
H=Ave (DMSO or H2O)
L=Ave (1uM Reference)
SD (H)=STDEV (DMSO or H2O)
SD (L)=STDEV (1uM Reference) $CV % \left(DMSO\right) = 100 * \left(SD_H/Ave_H\right)$ $CV % \left(1uM Reference\right) = 100 * SD_L / Ave_L$ $Z ′ = 1 - 3 * \left(SD_H+SD_L\right) / \left(Ave_H-Ave_L\right)$ $Inhibition % = \left(Ave_H-Sample\right) / \left(Ave_H-Ave_L\right)$ Nonlinear Fitting Regression Equation for the Compound IC50 $Y = Bottom + \left(Top-Bottom\right) / \left(1+{10}^{∧}\left(\left(LogIC50-X\right)*HillSlope\right)\right)$
X: cpd concentration
Y: inhibition%
Top and Bottom: Plateaus in same units as Y
logIC50: same log units as X
HillSlope: Slope factor or Hill slope

5. Test Results

The results showed that the compounds of the invention had strong inhibitory effects on Lp(a) assembly, and exemplary compounds were shown in Table 1.

**Table 1**

| **Compound No.** | **Lp(a)/IC₅₀(nM)** | **Compound No.** | **Lp(a)/IC₅₀(nM)** |
|---|---|---|---|
| LY3473329 | 1.05 | 121 | 1.53 |
| 5 | 4.12 | 122 | 1.87 |
| 6 | 1.13 | 126 | 1.26 |
| 11 | 0.89 | 127 | 0.90 |
| 12 | 1.17 | 128 | 1.34 |
| 47 | 1.03 | 130 | 1.45 |
| 49 | 3.22 | 131 | 1.19 |
| 65 | 0.87 | 132 | 0.88 |
| 67 | 8.55 | 133 | 0.94 |
| 71 | 0.54 | 134 | 0.85 |
| 80 | 2.31 | 135 | 0.71 |
| 81 | 0.85 | 136 | 2.16 |
| 90 | 0.83 | 137 | 1.08 |
| 91 | 1.03 | 138 | 2.00 |
| 93 | 0.81 | 139 | 0.66 |
| 94 | 0.65 | 140 | 0.79 |
| 95 | 0.68 | 141 | 1.02 |
| 96 | 0.58 | 142 | 3.19 |
| 97 | 1.46 | 143 | 3.81 |
| 98 | 0.85 | 144 | 1.18 |
| 99 | 0.96 | 145 | 1.72 |
| 100 | 1.12 | 146 | 0.93 |
| 101 | 2.71 | 147 | 0.86 |
| 102 | 0.74 | 148 | 1.50 |
| 103 | 2.11 | 149 | 2.53 |
| 104 | 1.04 | 152 | 1.26 |
| 105 | 0.84 | 153 | 2.91 |
| 106 | 1.84 | 158 | 1.65 |
| 107 | 1.66 | 159 | 1.68 |
| 108 | 1.16 | 160 | 1.37 |
| 111 | 1.02 | 161 | 1.89 |
| 112 | 3.40 | 176 | 1.81 |
| 113 | 1.17 | 177 | 1.26 |
| 117 | 2.58 | 210 | 1.52 |

### Test Example 2 and Pharmacokinetic Test in C57BL/6J Female Mice

C57BL/6J female mice were used as test animals, and the drug concentration in plasma of C57BL/6J female mice at different time points after intravenous and gavage administration was determined to study the pharmacokinetic characteristics of the compound in C57BL/6J female mice.

### Test animals: C57BL/6J female mice

Drug formulation: a certain amount of compound was weighed and normal saline was added to prepare clear, colorless solutions of 0.4 mg/mL (IV) and 1 mg/mL (PO).

Test procedure: C57BL/6J female mice were fasted overnight before gavage administration, followed by non-fasted intravenous administration. The intravenous dose was 2 mg/kg, and the gavage dose was 10 mg/kg. Approximately 0.03 mL of blood was collected from the orbital venous plexus at 0.083 (IV), 0.25, 0.5, 1, 2, 4, 8 and 24 h after administration, respectively, placed in a centrifuge tube containing EDTA-K2, centrifuged at 3000 rpm/min at 2-8°C for 10min to obtain plasma, and stored at -80°C.

Determination of the content of the compound to be tested in plasma of C57BL/6J female mice at different blood collection time points: 10 µL of plasma of C57BL/6J female mice collected at each time point after administration of LY3473329 and the compound of this application was added with 200 µL of 500 mM trichloroacetic acid aqueous solution to precipitate protein, which contained 100 ng/mL internal standard, vortexed for 1 min, centrifuged at 14000 rpm for 7min for samples treated with test tubes, and centrifuged at 4000 rpm for 10min for samples treated with 96-well plates, and 190 µL of the supernatant was transferred to a 96-well plate, and 8 µL of the supernatant was injected for LC-MS/MS analysis. WO2023078333A1 Example 6:12 µL of plasma of C57BL/6J female mice collected at each time point after administration was added with 240 µL of methanol to precipitate protein, which contained 10 ng/mL internal standard, vortexed for 1 min, centrifuged at 14000 rpm for 7min for samples treated with test tubes, and centrifuged at 4000 rpm for 10min for samples treated with 96-well plates. 220 µL of the supernatant was transferred to a 96-well plate, and 10 µL of the supernatant was taken for LC-MS/MS analysis.

### Pharmacokinetic Parameter Results

After plasma concentrations were determined by LC/MS/MS analysis method, pharmacokinetic parameters (e.g. Cₘₐₓ, AUC, T_{1/2}, etc.) were calculated using WinNonlin software and noncompartmental analysis.

The results showed that some compounds of the present invention exhibited favorable pharmacokinetic properties, with better plasma exposure after intravenous and gavage administration and a long half-life, indicating excellent oral administration performance; the pharmacokinetic properties of the preferred compounds, such as plasma exposure and half-life, were superior to those of the control compound LY3473329 and the compound of Example 6 in WO2023078333A1. The pharmacokinetic parameters for intravenous and gavage administration of an exemplary compound to C57BL/6J female mice are shown in Tables 2 and 3.

**Table 2: Pharmacokinetic parameters for gavage administration to C57BL/6J female mice**

| Compound No. | mg/kg | Cₘₐₓ (ng/ml) | AUC (ng/mL*h) | T_{1/2} (h) | F% |
|---|---|---|---|---|---|
| LY3473329 | 10 | 629 | 4462 | 7.4 | 10.1 |
| 96 | 10 | 919 | 12377 | 11.4 | 13.9 |
| 131 | 10 | 893 | 14278 | 16.0 | 13.2 |
| 159 | 10 | 874 | 21050 | 28.9 | 13.5 |
| 160 | 10 | 816 | 23547 | 28.8 | 15.9 |
| Example 6 of WO2023078333A1 | 10 | 511 | 5840 | 9.9 | 13.2 |

**Table 3: Pharmacokinetic parameters for intravenous administration to C57BL/6J female mice**

| Compound No. | mg/kg | AUC (ng/mL*h) | T _{1/2} (h) |
|---|---|---|---|
| LY3473329 | 2 | 8820 | 9.8 |
| 96 | 2 | 22925 | 23.3 |
| 131 | 2 | 31337 | 33.7 |
| 159 | 2 | 33427 | 35.5 |
| 160 | 2 | 19182 | 12.1 |
| Example 6 of WO2023078333A1 | 2 | 7673 | 7.7 |

### Test Example 3 and Humanized Mouse Lp(a) Inhibition Test

The *in vivo* pharmacodynamics of the compounds in reducing Lp(a) was evaluated in a humanized Lp(a) transgenic mouse model.Lp(a) mice were obtained by crossing mice expressing humanized apoB-100 with mice expressing humanized apo(a) containing 16 Kringle repeats.Female mice aged 8-9 weeks were selected for the test.Housing conditions: standard light cycle (12 h light/12 h dark), temperature 20-25°C, humidity 40-70%, free access to water and normal diet.

One day before the test, mice were divided into vehicle control group and test article group (n=5/group) according to body weight and baseline plasma Lp(a) concentration. Mice were orally administered with vehicle (normal saline, 10 mL/kg) or test compound (prepared with normal saline to 3 or 0.5 mg/mL, each mouse received 10 mL/kg), once daily (9:00 am) for 3 consecutive days, and blood was collected to determine the change of serum Lp(a). Eight hours after the last dose, 10 µL of blood was collected from the tail vein and transferred to a 1.5 mL centrifuge tube containing 90 µL of normal saline, mixed well, and left to stand on ice for 30min.The above samples were centrifuged at 4°C, 3000 rpm for 10 min, and the supernatant was collected and placed on ice.The Lp(a) concentration in the supernatant was measured by double-antibody sandwich ELISA using an anti-apo(a) capture antibody and an HRP-conjugated anti-ApoB detection antibody, with TMB for color development. The reaction was stopped with 1 N sulfuric acid, and the OD value was read at 450 nm on a Tecan microplate reader.Inhibition rate was calculated for each dosing group. Inhibition rate (%)=(vehicle control group Lp(a) concentration or self pre-dose Lp(a) concentration post-dose Lp(a) concentration)/vehicle control group Lp(a) concentration or self pre-dose Lp(a) concentration * 100.

The results showed that the compounds of the present invention significantly reduced serum Lp(a) levels in mice.

Some compounds of the present invention were significantly more effective in reducing serum Lp(a) levels in mice than the control compound LY3473329 and/or the compound of Example 6 in WO2023078333A1.

The inhibition rates of exemplary compounds against Lp(a) *in vivo* were shown in Table 4.

**Table 4 Single-dose in Vivo Pharmacodynamic Study in Mice**

| Compound No. | Dose (mg/kg) | Lp(a) Inhibition Rate |
|---|---|---|
| 95 | 30 | 95% |
| 96 | 30 | 96% |
| 96 | 5 | 91% |
| 100 | 5 | 86% |
| 111 | 5 | 92% |
| 113 | 5 | 79% |
| 131 | 5 | 81% |
| 138 | 5 | 88% |
| 152 | 5 | 90% |
| 159 | 5 | 84% |
| 160 | 5 | 83% |
| 161 | 5 | 83% |
| 162 | 5 | 79% |
| 163 | 5 | 81% |
| 176 | 5 | 78% |
| 177 | 5 | 90% |
| 178 | 5 | 84% |
| 180 | 5 | 80% |
| 190 | 5 | 83% |
| 193 | 5 | 88% |
| Example 6 of WO2023078333A1 | 5 | 75% |
| LY3473329 | 5 | 56% |

LY3473329 is the compound of Example 1 in CN114008021A, the preparation method of which is shown in Example 1 of patent CN114008021A, and the preparation method of Example 6 compound in WO2023078333A1 is shown in Example 6 of patent WO2023078333A1.

### Test Example 4 and Safety Assessment Test for Selective Effects on 44 Targets

The activity of 44 early safety targets closely related to the heart, central nervous system, gastrointestinal tract, and immune system was tested. The off-target effects of the test compound on 44 targets (including 24 G protein-coupled receptors (GPCRs), 8 ion channel targets, 3 transporters, 2 nuclear receptors, and 7 enzymes) were examined *in vitro* at the cellular or molecular level using radioisotope binding methods, calcium flow assays, and enzymatic fluorescence or chemiluminescence techniques.

The results showed that at a single concentration of 10 µM (the test concentration of Eta agonism was 12 µM), some compounds of the present invention had no significant binding, inhibition or agonistic effect on 44 targets (inhibition rate/agonism rate< 50%).

### Test Example 5 and Safety Assessment Test in Rats

The safety of the compounds of the present invention was evaluated by oral gavage administration to SD rats as the test animals.

Test method: 50 SD rats (half males and half females) were randomly divided into vehicle control group and test article group according to their body weight. The vehicle saline or the compound of the present invention or LY3473329 (300 mg/kg, 1000 mg/kg) was administered orally once daily for 14 consecutive days.

Results: no changes related to the compound(s) of the invention or LY3473329 were observed in clinical observation, body weight and weight gain, food consumption, hematology, blood biochemistry, urine, organ weight and coefficients, and histopathology in all dose groups. The no observed adverse effect level (NOAEL) of the compound(s) of the present invention was 1000 mg/kg, suggesting that the compound(s) of the present invention has excellent safety.

### Test example 6 and The affinity between compound and human plasminogen (PLG) detected by SPR test

1.Reagent

| Reagent | Manufacturer |
|---|---|
| HBS-EP buffer | Cytiva |
| DMSO solution | Sigma |
| Amine Coupling Kit | Cytiva |
| Plasminogen | MCE |
| CM5 CHIP | Cytiva |

2.Test Procedure
2.1 The Running buffer preparation
   The Running buffer was prepared from Storage buffer to a final concentration of 10mM Hepes, 150mM NaCl, 3mM EDTA, 0.05% Surfactant P20, pH 7.4 (HBS-EP), 3% DMSO, and filtered by 0.22µm filtration membrane.
2.2. Sample Preparation
   2.2.1. Stock solution: Dilute compounds into 10mM with DMSO.
   2.2.2. Testing solution: The stock solution was gradiently diluted to 8 concentrations with DMSO-free running buffer.
2.3. Human Plasminogen Protein Immobilization
   2.3.1. Balance Series S Chip C5 chip to room temperature.
   2.3.2. Immobilize the human PLG protein on Fc2 via amine-coupling method.
   2.3.3. EDC+NHS solution flowed through Fcl and Fc2 at a flow rate of 10 µL/min, with 1 injections of 420 seconds each, to activate the chip surface.
   2.3.4. Dilute PLG protein to 40µg/mL, in running buffer, following injected PLG over Fc2 at a flow rate of 5uL/min, injection time was 720 s, and the final protein capture level was 15000RU/channel.
   2.3.5. Injected Ethanolamine-HCL solution over Fcl and Fc2 for 420s at a flow rate of 10uL/min to block sensor chip.
2.4. Setting of Sample Injection Procedure
   2.4.1. The 8 concentrations of compounds testing solution were injected from low to high concentration, flowing through Fcl to Fc2. The association phase lasted for 90 seconds, followed by a dissociation phase of 150 seconds, with a flow rate of 30 µL/min.

3. Data Analysis
3.1. Subtracted experimental channel signal value (Fc2) from the reference channel (Fc1) and buffer blank control to obtain the final association and dissociation sensorgram. The affinity data was derived from fitting the data using a Bivalent Fit model.
3.2. QC verification criteria for experiment results: at least 5 concentration points meets concentration-dependence, The Chi² value is less than or equal to 1/10 Rmax value.
3.3. Exported the real-time response value (RU) of association and dissociation from the Biacore-S200 instrument.
3.4. Exported the constants Ka. Kd and KD from the Biacore-S200 instrument. KD=Kd/Ka 4. Results

Preferred compound(s) of this invention exhibited a low affinity for PLG, suggesting good specificity.

## Claims

1. A compound represented by the following formula (I) or a pharmaceutically acceptable salt thereof, having the following structure: 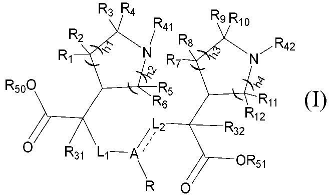
wherein - - - is a single bond or a double bond;
A is CR_{X}, P, P(O), N, C₆₋₁₂ aryl, 5-14 membered heteroaryl, C₃₋₈ carbocyclyl, or 5-18 membered heterocyclyl; the aryl, heteroaryl, carbocyclyl, and heterocyclyl are optionally substituted by one or more substituents independently selected from deuterium, halogen, -CN, C₁₋₃alkyl, and C₁₋₃alkoxy;
when - - - is a single bond, R_{X} is H, -OH, or C₁₋₃alkoxy;
when - - - is a double bond, R_{X} does not exist;
L₁ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 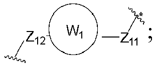 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₁ and A;
L₂ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 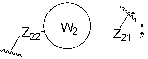 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀ aryl, -C₁₋₃alkylene-5-12 membered heteroaryl, C₁₋₆alkyl, and 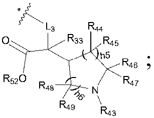 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkoxy, and C₁₋₃aminoalkyl; the C₁₋₆alkyl is optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-C₆₋₁₀aryl-C₁₋₃alkylene-, -C₁₋₃alkylene-5-12 membered heteroaryl-5-12 membered heteroaryl-C₁₋₃alkylene-, and 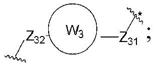 the alkylene, alkenylene, and alkynylene are optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkyl, and C₁₋₃alkoxy; * is the connecting end of L₃ and A;
each of the rings W₁, W₂, and W₃ is independently selected from C₆₋₁₂ aryl, 5-12 membered heteroaryl, C₆₋₁₄cycloalkyl, and 5-12 membered heterocyclyl; the aryl, heteroaryl, cycloalkyl, and heterocyclyl are optionally substituted by one or more substituents independently selected from deuterium, halogen, -CN, -OH, -NH₂, -CHO, C₁₋₃alkyl, C₁₋₃alkoxy, C₁₋₃haloalkyl, C₁₋₃haloalkoxy, -N(C₁₋₃alkyl)₂, -NH(C₁₋₃alkyl), and -C(O)C₁₋₃alkyl;
each of Z₁₁, Z₁₂, Z₂₁, Z₂₂, Z₃₁, and Z₃₂ is independently selected from a bond, -O-, -S-, -NH-, -Se-, -C₁₋₄alkylene-, -C₁₋₈oxaalkylene-, -C₁₋₄thiaalkylene-, -C₁₋₄azaalkylene-, and -C₁₋₄selenaalkylene-; the alkylene, oxaalkylene, thiaalkylene, azaalkylene, and selenaalkylene are optionally substituted by one or more substituents independently selected from Z₄₁; Z₄₁ is independently selected from deuterium, halogen, oxo, thio, -CN, -OH, -NH₂, C₁₋₃alkyl, C₁₋₃haloalkyl, C₁₋₃alkoxy, and C₁₋₃haloalkoxy; or any two Z₄₁ together with the atom to which they are attached form C₃₋₆cycloalkyl, 3-6 membered heterocyclyl, phenyl, and 5-6 membered heteroaryl;
each of R₃₁, R₃₂, and R₃₃ is independently selected from hydrogen, deuterium, halogen, C₁₋₆alkyl, C₁₋₆haloalkyl, C₁₋₆deuteroalkyl, C₁₋₆alkoxy, C₁₋₆haloalkoxy, and C₁₋₆deuteroalkoxy;
each of h1, h2, h3, h4, h5, and h6 is independently 0, 1, or 2;
each of R₁, R₂, R₃, R₄, R₅, R₆, R₇, R₈, R₉, R₁₀, R₁₁, R₁₂, R₄₁, R₄₂, R₄₃, R₄₄, R₄₅, R₄₆, R₄₇, R₄₈, and R₄₉ is independently selected from H, deuterium, -CN, -OH, -NH₂, halogen, C₁₋₆alkyl, C₁₋₆haloalkyl, C₁₋₆alkoxy, C₁₋₆haloalkoxy, -N(C₁₋₃alkyl)₂, and -NH(C₁₋₃alkyl);
each of R₅₀, R₅₁, R₅₂ is independently selected from H, deuterium, C₁₋₆alkyl, C₂₋₆alkenyl, C₁₋₆alkoxy, -C₁₋₃alkylene-O-C₁₋₆alkyl, and -C₁₋₃alkylene-O-C(O)-C₁₋₆alkyl; the alkyl, alkenyl, alkoxy, and alkylene are optionally substituted by one or more substituents independently selected from deuterium, halogen, -OH, -NH₂, -CN, oxo, C₁₋₃alkyl, and C₁₋₃alkoxy;
provided that when A is N, and R₁, R₂, R₃, R₄, R₅, R₆, R₇, R₈, R₉, R₁₀, R₁₁, R₁₂, R₄₁, R₄₂, R₄₃, R₄₄, R₄₅, R₄₆, R₄₇, R₄₈, and R₄₉ are all H, L₁, L₂, and L₃ are not 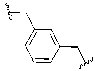 at the same time;
provided that when A is N, R is 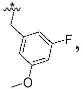 and R₁, R₂, R₃, R₄, R₅, R₆, R₇, R₈, R₉, R₁₀, R₁₁, R₁₂, R₄₁, and R₄₂ are all H, L₁ and L₂ are not 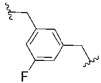 at the same time, * is the connecting end of R and A;
provided that the compound is not 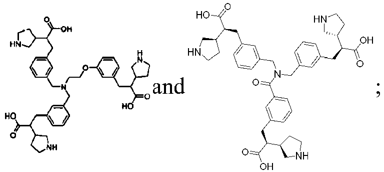
the heteroatom in the heterocyclyl and heteroaryl is independently selected from O, N and S, and the heteroatom number is 1, 2, 3, or 4;
for example, R is a group containing L₃, L₁, L₂ and L₃ are groups containing Z₁₁, Z₂₁ and Z₃₁ respectively, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-.

2. A compound represented by the following formula (I-1-P1), formula (I-1-P2), formula (I-1-P3) or formula (I-1-P4), or a pharmaceutically acceptable salt thereof, 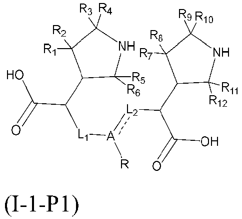
wherein - - - is a single bond or a double bond;
A is CR_{X}, P, P(O) or N;
when - - - is a single bond, R_{X} is H, -OH, or C₁₋₃alkoxy;
when - - - is a double bond, R_{X} does not exist;
L₁ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 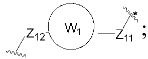 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₁ and A;
L₂ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 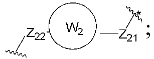 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀ aryl, -C₁₋₃alkylene-5-12 membered heteroaryl, C₁₋₆alkyl, and 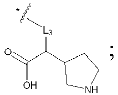 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkoxy, and C₁₋₃aminoalkyl; the C₁₋₆alkyl is optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-C₆₋₁₀aryl-C₁₋₃alkylene-, -C₁₋₃alkylene-5-12 membered heteroaryl-5-12 membered heteroaryl-C₁₋₃alkylene-, and 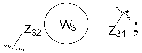 the alkylene, alkenylene, and alkynylene are optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkyl, and C₁₋₃alkoxy; * is the connecting end of L₃ and A;
each of the rings W₁, W₂, and W₃ is independently selected from C₆₋₁₂ aryl, 5-12 membered heteroaryl, C₆₋₁₄cycloalkyl, and 5-12 membered heterocyclyl; the aryl, heteroaryl, cycloalkyl, and heterocyclyl are optionally substituted by one or more substituents independently selected from deuterium, halogen, -CN, C₁₋₃alkyl, and C₁₋₃alkoxy;
each of Z₁₁, Z₁₂, Z₂₁, Z₂₂, Z₃₁, and Z₃₂ is independently selected from a bond, -C₁₋₄alkylene-, and - C₁₋₄oxaalkylene-; the alkylene, and oxaalkylene are optionally substituted by one or more substituents independently selected from deuterium, halogen, oxo, -CN, -OH, -NH₂, C₁₋₃alkyl, and C₁₋₃alkoxy;
each of R₁, R₂, R₃, R₄, R₅, R₆, R₇, R₈, R₉, R₁₀, R₁₁, and R₁₂ is independently selected from H, and deuterium;
provided that when A is N, L₁, L₂, and L₃ are not 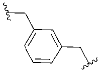 at the same time;
provided that when A is N, R is 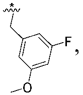 L₁ and L₂ are not 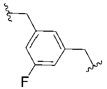 at the same time, * is the connecting end of R and A;
the heteroatom in the heterocyclyl and heteroaryl is independently selected from O, N and S, and the heteroatom number is 1, 2, or 3;
for example, in the compound represented by formula (I-1-P1), R is a group containing L₃, L₁, L₂ and L₃ are groups containing Z₁₁, Z₂₁ and Z₃₁ respectively, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, - CH₂CH₂-, -CD₂CH₂-, -C(O)-, -CH₂C(O)-, -NHC(O)-, -OC(O)-, -CH₂CH₂O-, -OCH₂C(O)-, - CH₂OC(O)-, -CH₂CH₂CH₂O-, and -S-C(O)-;
or 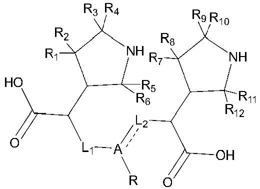 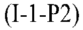
wherein - - - is a single bond or a double bond;
A is CR_{X}, P, P(O) or N;
when - - - is a single bond, R_{X} is H, -OH, or C₁₋₃alkoxy;
when - - - is a double bond, R_{X} does not exist;
L₁ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, 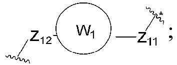 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₁ and A;
L₂ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 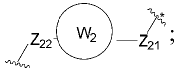 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀ aryl, -C₁₋₃alkylene-5-12 membered heteroaryl, C₁₋₆alkyl, and 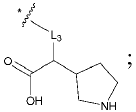 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkoxy, and C₁₋₃aminoalkyl; the C₁₋₆alkyl are optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-C₆₋₁₀aryl-C₁₋₃alkylene-, -C₁₋₃alkylene-5-12 membered heteroaryl-5-12 membered heteroaryl-C₁₋₃alkylene-, and 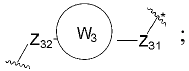 the alkylene, alkenylene, and alkynylene are optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkyl, and C₁₋₃alkoxy; * is the connecting end of L₃ and A;
each of the rings W₁, W₂, and W₃ is independently selected from C₆₋₁₂ aryl, 5-12 membered heteroaryl, C₆₋₁₄cycloalkyl, and 5-12 membered heterocyclyl; the aryl, heteroaryl, cycloalkyl, and heterocyclyl are optionally substituted by one or more substituents independently selected from deuterium, halogen, -CN, C₁₋₃alkyl, and C₁₋₃alkoxy;
each of Z₁₁, Z₁₂, Z₂₁, Z₂₂, Z₃₁, and Z₃₂ is independently selected from a bond, -C₁₋₄alkylene-, -C₁₋₄oxaalkylene-, -C₁₋₄thiaalkylene-, -C₁₋₄azaalkylene-; the alkylene, oxaalkylene, thiaalkylene, and azaalkylene are optionally substituted by one or more substituents independently selected from deuterium, halogen, oxo, -CN, -OH, -NH₂, C₁₋₃alkyl, and C₁₋₃alkoxy;
each of R₁, R₂, R₃, R₄, R₅, R₆, R₇, R₈, R₉, R₁₀, R₁₁, and R₁₂ is independently selected from H, and deuterium;
provided that when A is N, L₁, L₂, and L₃ are not 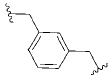 at the same time;
provided that when A is N, R is 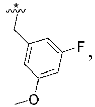 L₁ and L₂ are not 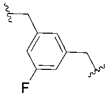 at the same time, * is the connecting end of R and A;
the heteroatom in the heterocyclyl and heteroaryl is independently selected from O, N and S, and the heteroatom number is 1, 2, or 3;
for example, in the compound represented by formula (I-1-P2), R is a group containing L₃, L₁, L₂ and L₃ are groups containing Z₁₁, Z₂₁ and Z₃₁ respectively, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, - CH₂CH₂-, -CD₂CH₂-, -C(O)-, -CH₂C(O)-, -NHC(O)-, -OC(O)-, -CH₂CH₂O-, -OCH₂C(O)-, - CH₂OC(O)-, -CH₂CH₂CH₂O-, and -S-C(O)-;
or 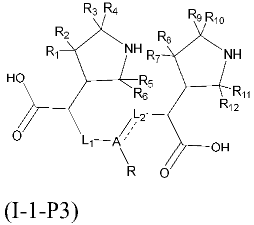
wherein - - - is a single bond or a double bond;
A is CR_{X}, P, P(O), N, C₆₋₁₂ aryl, 5-14 membered heteroaryl, 5-18 membered heterocyclyl; the aryl, heteroaryl, and heterocyclyl are optionally substituted by one or more substituents independently selected from deuterium, halogen, -CN, C₁₋₃alkyl, and C₁₋₃alkoxy;
when - - - is a single bond, R_{X} is H, -OH, or C₁₋₃alkoxy;
when - - - is a double bond, R_{X} does not exist;
L₁ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, 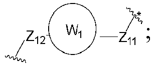 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₁ and A;
L₂ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 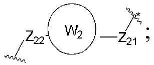 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀ aryl, -C₁₋₃alkylene-5-12 membered heteroaryl, C₁₋₆alkyl, and 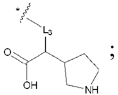 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkoxy, and C₁₋₃aminoalkyl; the C₁₋₆alkyl is optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-C₆₋₁₀aryl-C₁₋₃alkylene-, -C₁₋₃alkylene-5-12 membered heteroaryl-5-12 membered heteroaryl-C₁₋₃alkylene-, and 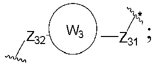 the alkylene, alkenylene, and alkynylene are optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkyl, and C₁₋₃alkoxy; * is the connecting end of L₃ and A;
each of the rings W₁, W₂, and W₃ is independently selected from C₆₋₁₂ aryl, 5-12 membered heteroaryl, C₆₋₁₄cycloalkyl, and 5-12 membered heterocyclyl; the aryl, heteroaryl, cycloalkyl, and heterocyclyl are optionally substituted by one or more substituents independently selected from deuterium, halogen, -CN, C₁₋₃alkyl, and C₁₋₃alkoxy;
each of Z₁₁, Z₁₂, Z₂₁, Z₂₂, Z₃₁, and Z₃₂ is independently selected from a bond, -C₁₋₄alkylene-, -C₁₋₄oxaalkylene-, -C₁₋₄thiaalkylene-, -C₁₋₄azaalkylene-; the alkylene, oxaalkylene, thiaalkylene, and azaalkylene are optionally substituted by one or more substituents independently selected from deuterium, halogen, oxo, -CN, -OH, -NH₂, C₁₋₃alkyl, C₁₋₃haloalkyl, C₁₋₃alkoxy, and C₁₋₃haloalkoxy; each of R₁, R₂, R₃, R₄, R₅, R₆, R₇, R₈, R₉, R₁₀, R₁₁, and R₁₂ is independently selected from H, and deuterium;
provided that when A is N, and R₁, R₂, R₃, R₄, R₅, R₆, R₇, R₈, R₉, R₁₀, R₁₁, and R₁₂ are all H, L₁, L₂, and L₃ are not 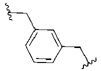 at the same time;
provided that when A is N, R is 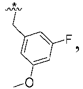 and R₁, R₂, R₃, R₄, R₅, R₆, R₇, R₈, R₉, R₁₀, R₁₁, and R₁₂ are all H, L₁ and L₂ are not 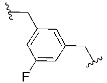 at the same time, * is the connecting end of R and A;
provided that the compound is not 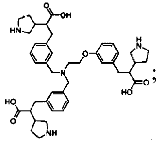
the heteroatom in the heterocyclyl and heteroaryl is independently selected from O, N and S, and the heteroatom number is 1, 2, 3, or 4;
for example, in the compound represented by formula (I-1-P3), R is a group containing L₃, L₁, L₂ and L₃ are groups containing Z₁₁, Z₂₁ and Z₃₁ respectively, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, - CH₂CH₂-, -CD₂CH₂-, -C(O)-, -CH₂C(O)-, -NHC(O)-, -OC(O)-, -CH₂CH₂O-, -OCH₂C(O)-, - CH₂OC(O)-, -CH₂CH₂CH₂O-, and -S-C(O)-;
or 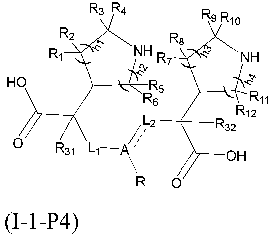
wherein - - - is a single bond or a double bond;
A is CR_{X}, P, P(O), N, C₆₋₁₂ aryl, 5-14 membered heteroaryl, or 5-18 membered heterocyclyl; the aryl, heteroaryl, and heterocyclyl are optionally substituted by one or more substituents independently selected from deuterium, halogen, -CN, C₁₋₃alkyl, and C₁₋₃alkoxy;
when - - - is a single bond, R_{X} is H, -OH, or C₁₋₃alkoxy;
when - - - is a double bond, R_{X} does not exist;
L₁ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 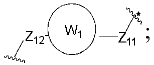 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₁ and A;
L₂ is selected from -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-5-12 membered heteroaryl-C₁₋₃alkylene-, and 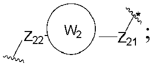 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, and C₁₋₃alkoxy; * is the connecting end of L₂ and A;
R is selected from H, -C₁₋₃alkylene-C₆₋₁₀ aryl, -C₁₋₃alkylene-5-12 membered heteroaryl, C₁₋₆alkyl, and 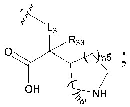 the C₁₋₃alkylene is optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkoxy, and C₁₋₃aminoalkyl; the C₁₋₆alkyl is optionally substituted by one or more substituents independently selected from deuterium, halogen, oxo, carboxyl, -CN, -OH, -NH₂, C₁₋₃alkyl, and C₁₋₃alkoxy; * is the connecting end of R and A;
L₃ is selected from -C₁₋₆alkylene-, -C₂₋₆alkenylene-, -C₂₋₆alkynylene-, -C₁₋₃alkylene-C₆₋₁₀aryl-C₆₋₁₀aryl-C₁₋₃alkylene-, -C₁₋₃alkylene-C₆₋₁₀aryl-5-12 membered heteroaryl-C₁₋₃alkylene-, -C₁₋₃alkylene-5-12 membered heteroaryl-C₆₋₁₀aryl-C₁₋₃alkylene-, -C₁₋₃alkylene-5-12 membered heteroaryl-5-12 membered heteroaryl-C₁₋₃alkylene-, and 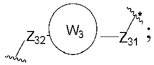 the alkylene, alkenylene, and alkynylene are optionally substituted by one or more substituents independently selected from deuterium, halogen, oxo, -CN, -OH, -NH₂, and C₁₋₃alkyl; the aryl, and heteroaryl are optionally substituted by one or more substituents independently selected from deuterium, halogen, -CN, C₁₋₃alkyl, and C₁₋₃alkoxy; * is the connecting end of L₃ and A;
each of the rings W₁, W₂, and W₃ is independently selected from C₆₋₁₂ aryl, 5-12 membered heteroaryl, C₆₋₁₄cycloalkyl, and 5-12 membered heterocyclyl; the aryl, heteroaryl, cycloalkyl, and heterocyclyl are optionally substituted by one or more substituents independently selected from deuterium, halogen, -CN, -OH, -NH₂, -CHO, C₁₋₃alkyl, C₁₋₃alkoxy, C₁₋₃haloalkyl, C₁₋₃haloalkoxy, -N(C₁₋₃alkyl)₂, -NH(C₁₋₃alkyl), and -C(O)C₁₋₃alkyl;
each of Z₁₁, Z₁₂, Z₂₁, Z₂₂, Z₃₁, and Z₃₂ is independently selected from a bond, -O-, -S-, -NH-, -Se-, -C₁₋₄alkylene-, -C₁₋₈oxaalkylene-, -C₁₋₄thiaalkylene-, -C₁₋₄azaalkylene-, -C₁₋₄selenaalkylene-; the alkylene, oxaalkylene, thiaalkylene, azaalkylene, and selenaalkylene are optionally substituted by one or more substituents independently selected from Z₄₁; Z₄₁ is independently selected from deuterium, halogen, oxo, thio, -CN, -OH, -NH₂, C₁₋₃alkyl, C₁₋₃haloalkyl, C₁₋₃alkoxy, and C₁₋₃haloalkoxy; or any two Z₄₁ together with the atom to which they are attached form C₃₋₆cycloalkyl, 3-6 membered heterocyclyl, phenyl, or 5-6 membered heteroaryl;
each of R₃₁, R₃₂, and R₃₃ is independently selected from hydrogen, deuterium, halogen, C₁₋₆alkyl, C₁₋₆haloalkyl, C₁₋₆deuteroalkyl, C₁₋₆alkoxy, C₁₋₆haloalkoxy, and C₁₋₆deuteroalkoxy;
each of h1, h2, h3, h4, h5, and h6 is independently 0, 1, or 2;
each of R₁, R₂, R₃, R₄, R₅, R₆, R₇, R₈, R₉, R₁₀, R₁₁, and R₁₂ is independently selected from H, and deuterium;
provided that when A is N, and R₁, R₂, R₃, R₄, R₅, R₆, R₇, R₈, R₉, R₁₀, R₁₁, and R₁₂ are all H, L₁, L₂, and L₃ are not at the same time 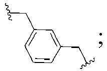
provided that when A is N, R is 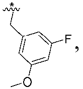 and R₁, R₂, R₃, R₄, R₅, R₆, R₇, R₈, R₉, R₁₀, R₁₁, and R₁₂
are all H, L₁ and L₂ are not at the same time 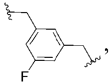 * is the connecting end of R and A;
provided that the compound is not 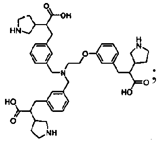
the heteroatom in the heterocyclyl and heteroaryl is independently selected from O, N and S, and the heteroatom number is 1, 2, 3, or 4;
for example, in the compound represented by formula (I-1-P4), R is a group containing L₃, L₁, L₂ and L₃ are groups containing Z₁₁, Z₂₁ and Z₃₁ respectively, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, - CH₂CH₂-, -CD₂CH₂-, -C(O)-, -CH₂C(O)-, -NHC(O)-, -OC(O)-, -CH₂CH₂O-, -OCH₂C(O)-, - CH₂OC(O)-, -CH₂CH₂CH₂O-, and -S-C(O)-.

3. The compound according to any one of claims 1-2, or a pharmaceutically acceptable salt thereof, wherein A is CR_{X}, P, P(O), N, phenyl, 5-6 membered heterocyclyl, or 12 membered heterocyclyl; the heteroatom in the heterocyclyl is N, and the heteroatom number is 1, 2, 3, or 4;
preferably, A is CR_{X}, P, P(O), N, phenyl, piperazinyl, 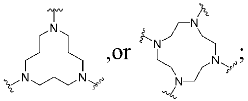 or
A is CR_{X}, P, P(O), N, 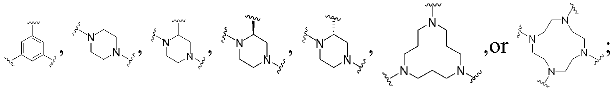 or
A is N, 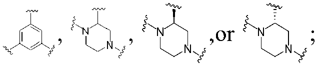 or
A is N; or
A is P(O); or
A is CR_{X}; R_{X} is H, -OH, or methoxy; or
A is selected from C-OH, and C-OCH₃; or
A is 6 membered heterocyclyl; preferably, A is piperazinyl; preferably 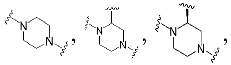 or 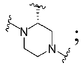 or
A is phenyl; for example

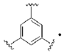

4. The compound according to any one of claims 1-3, or a pharmaceutically acceptable salt thereof, wherein L₁ is selected from -C₁₋₃alkylene-phenyl-phenyl-C₁₋₃alkylene-, -C₁₋₃alkylene-5-6 membered heteroaryl-5-6 membered heteroaryl-C₁₋₃alkylene-, and 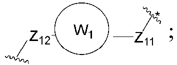 the C₁₋₃alkylene is optionally substituted by one or more substituents independently selected from deuterium, halogen, oxo, -CN, -OH, -NH₂, and C₁₋₃alkyl; the phenyl, and heteroaryl are optionally substituted by one or more substituents independently selected from halogen, -CN, and C₁₋₃alkoxy;
* is the connecting end of L₁ and A;
preferably, L₁ is selected from -methylene-phenyl-phenyl-methylene-, -ethylene-phenyl-phenyl-ethylene-, -methylene-5-6 membered heteroaryl-5-6 membered heteroaryl-methylene-, -ethylene-5-6 membered heteroaryl-5-6 membered heteroaryl-ethylene-, and 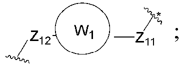 the methylene, and ethylene are optionally substituted by one or more substituents independently selected from deuterium, halogen, oxo, -CN, -OH, -NH₂, and C₁₋₃alkyl; the phenyl, and heteroaryl are optionally substituted by one or more substituents independently selected from halogen, -CN, and C₁₋₃alkoxy; * is the connecting end of L₁ and A; or
L₁ is selected from -methylene-phenyl-phenyl-methylene-, -methylene-6 membered heteroaryl-6 membered heteroaryl-methylene-, and 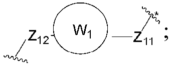 the methylene is optionally substituted by one or more substituents independently selected from deuterium, halogen, oxo, -CN, -OH, - NH₂, and C₁₋₃alkyl; the phenyl, and heteroaryl are optionally substituted by one or more substituents independently selected from deuterium, halogen, -CN, and C₁₋₃alkoxy; * is the connecting end of L₁ and A; or
L₁ is selected from -methylene-phenyl-phenyl-methylene-, -methylene-pyridinyl-pyridinyl-methylene-, and 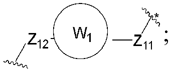 * is the connecting end of L₁ and A; or
L₁ is selected from 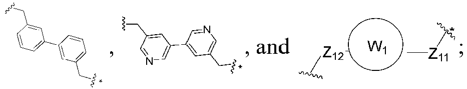 * is the connecting end of L₁ and A; or
L₁ is 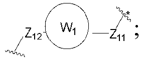 * is the connecting end of L₁ and A; or
L₁ is selected from 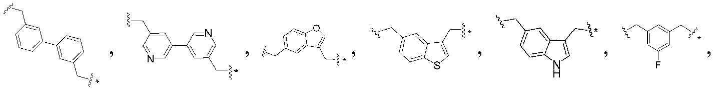 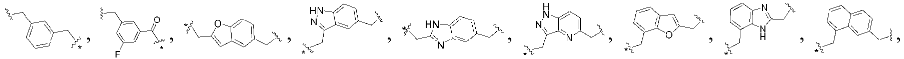 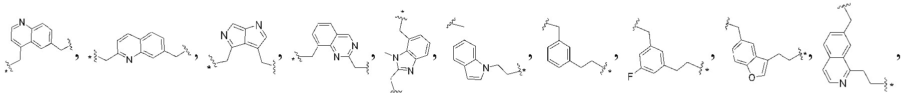 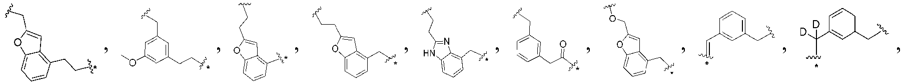 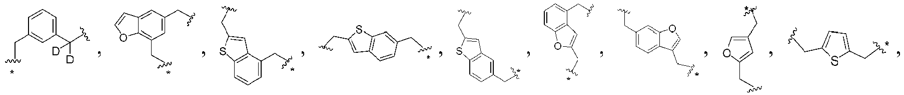 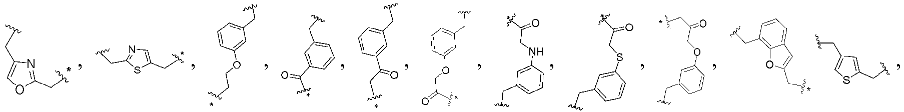 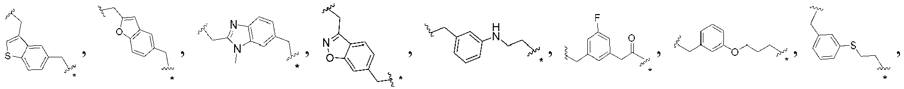 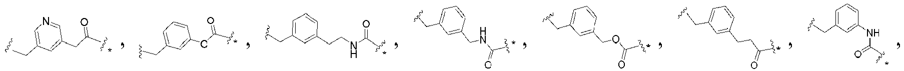 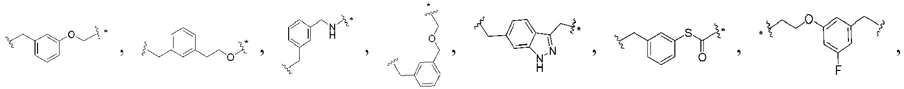 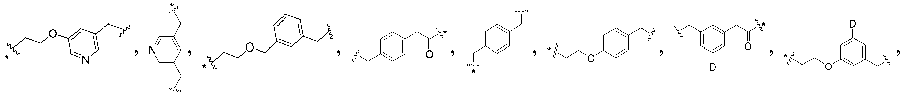 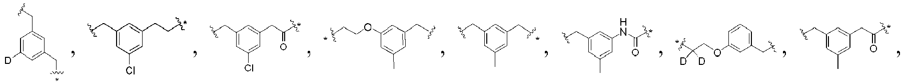 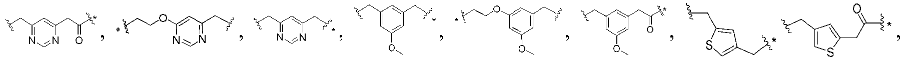 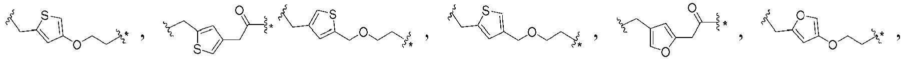 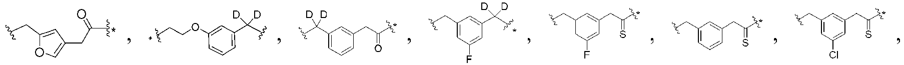 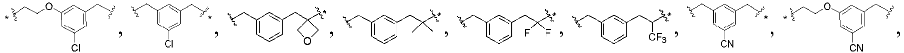 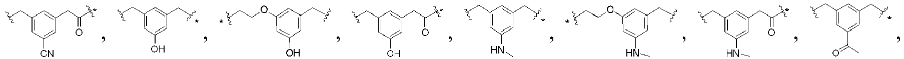 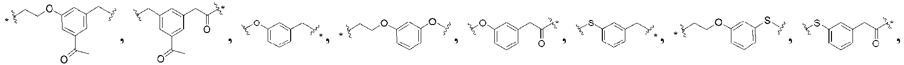 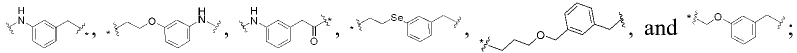 * represents the connecting end with A.

5. The compound according to any one of claims 1-4, or a pharmaceutically acceptable salt thereof, wherein L₂ is selected from -C₁₋₃alkylene-phenyl-phenyl-C₁₋₃alkylene-, -C₁₋₃alkylene-5-6 membered heteroaryl-5-6 membered heteroaryl-C₁₋₃alkylene-, and 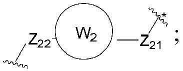 the C₁₋₃alkylene is optionally substituted by one or more substituents independently selected from deuterium, halogen, oxo, -CN, -OH, -NH₂, and C₁₋₃alkyl; the phenyl, and heteroaryl are optionally substituted by one or more substituents independently selected from halogen, -CN, and C₁₋₃alkoxy; * is the connecting end of L₂ and N;
preferably, L₂ is selected from -methylene-phenyl-phenyl-methylene-, -ethylene-phenyl-phenyl-ethylene-, -methylene-5-6 membered heteroaryl-5-6 membered heteroaryl-methylene-, -ethylene-5-6 membered heteroaryl-5-6 membered heteroaryl-ethylene-, and 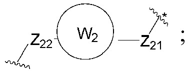 the methylene, and ethylene are optionally substituted by one or more substituents independently selected from deuterium, halogen, oxo, -CN, -OH, -NH₂, and C₁₋₃alkyl; the phenyl, and heteroaryl are optionally substituted by one or more substituents independently selected from halogen, -CN, and C₁₋₃alkoxy; * is the connecting end of L₂ and A; or
L₂ is selected from -methylene-phenyl-phenyl-methylene-, -methylene-6 membered heteroaryl-6 membered heteroaryl-methylene-, and 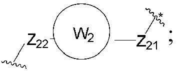 the methylene is optionally substituted by one or more substituents independently selected from deuterium, halogen, oxo, -CN, -OH, - NH₂, and C₁₋₃alkyl; the phenyl, and heteroaryl are optionally substituted by one or more substituents independently selected from deuterium, halogen, -CN, and C₁₋₃alkoxy; * is the connecting end of L₂ and A; or
L₂ is selected from -methylene-phenyl-phenyl-methylene-, -methylene-pyridinyl-pyridinyl-methylene-, and 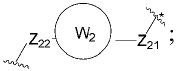 * is the connecting end of L₂ and A; or
L₂ is selected from 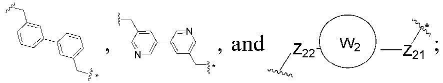 * is the connecting end of L₂ and A; or
L₂ is 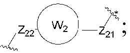 * is the connecting end of L₂ and A; or
L₂ is selected from 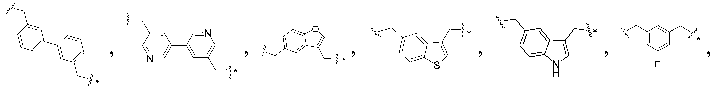 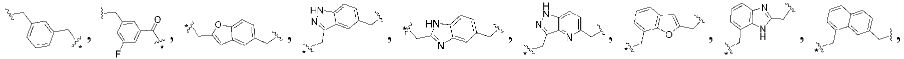 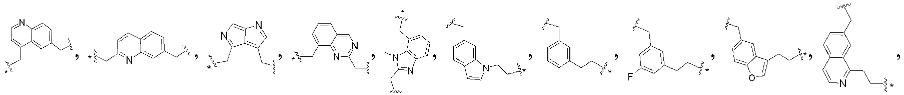 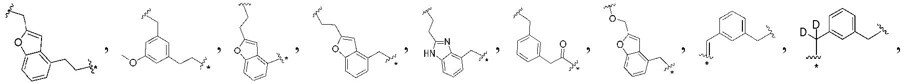 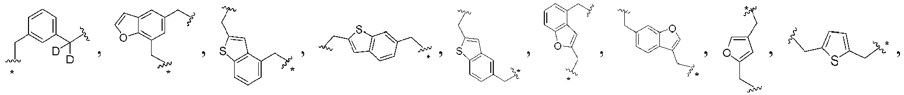 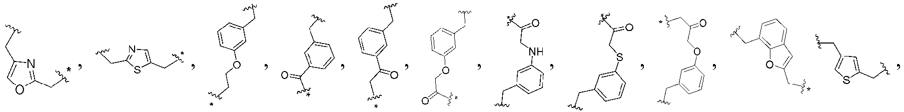 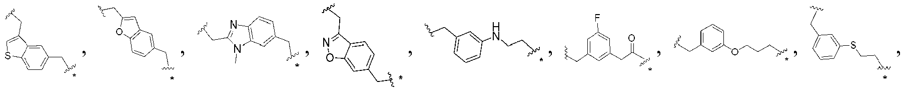 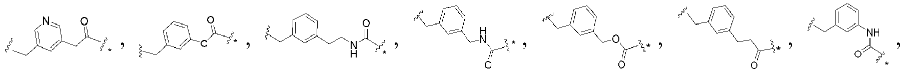 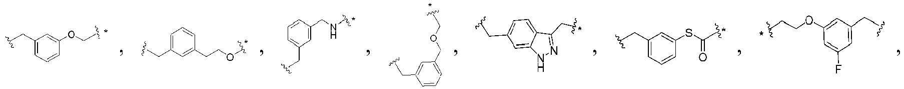 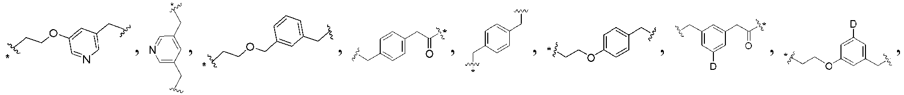 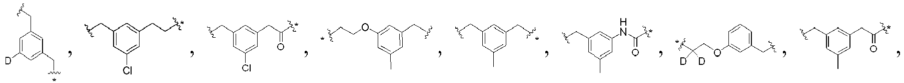 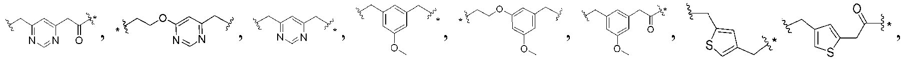 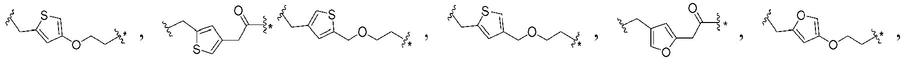 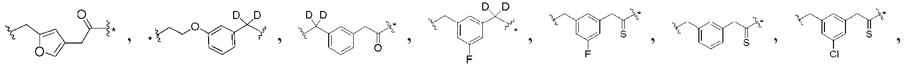 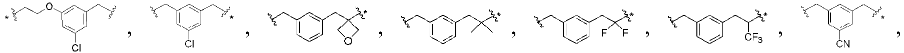 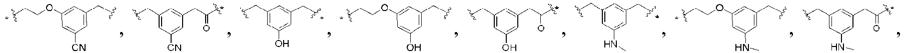 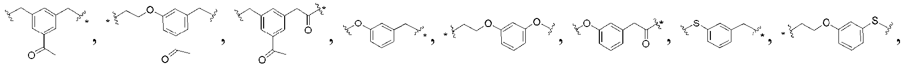 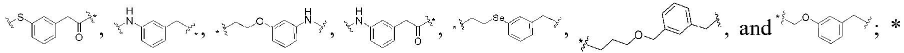 represents the connecting end with A.

6. The compound according to any one of claims 1-5, or a pharmaceutically acceptable salt thereof, wherein each of rings W₁, W₂, and W₃ is independently selected from C₆₋₁₂ aryl, 5-12 membered heteroaryl, and C₆₋₁₄cycloalkyl; the aryl, heteroaryl, and cycloalkyl are optionally substituted by one or more substituents independently selected from deuterium, halogen, -CN, C₁₋₃alkyl, and C₁₋₃alkoxy;
preferably, each of rings W₁, W₂, and W₃ is independently selected from C₆₋₁₀ aryl, 5-10 membered heteroaryl, and C₆₋₁₀cycloalkyl; the aryl, heteroaryl, and cycloalkyl are optionally substituted by one or more substituents independently selected from F, Cl, Br, -CN, methyl, ethyl, methoxy, and ethoxy; or
each of rings W₁, W₂, and W₃ is independently selected from phenyl, naphthyl, 8-10 membered bicyclic heteroaryl, C₈₋₁₀ bicyclic cycloalkyl, and 5-6 membered monocyclic heteroaryl; the phenyl, naphthyl, heteroaryl, and cycloalkyl are optionally substituted by one or more substituents independently selected from halogen, -CN, C₁₋₃alkyl, and C₁₋₃alkoxy; or
each of the rings W₁, W₂, and W₃ is independently selected from phenyl, naphthyl, 5-membered/5-membered fused heteroaryl, 5-membered/6-membered fused heteroaryl, 6-membered/5-membered fused heteroaryl, 6-membered/6-membered fused heteroaryl, 4-membered/6-membered spirocycloalkyl, 6-membered/4-membered spirocycloalkyl, 5-membered/5-membered spirocycloalkyl, 5-membered/6-membered spirocycloalkyl, 6-membered/5-membered spirocycloalkyl, 6-membered/6-membered spirocycloalkyl, and 5-6 membered monocyclic heteroaryl; the phenyl, naphthyl, fused heteroaryl, spirocycloalkyl, and monocyclic heteroaryl are optionally substituted by one or more substituents independently selected from F, Cl, Br, -CN, methyl, ethyl, methoxy, and ethoxy; or
each of the rings W₁, W₂, and W₃ is independently selected from phenyl, 5-6 membered monocyclic heteroaryl; the phenyl, and heteroaryl are optionally substituted by one or more substituents independently selected from deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl; the heteroatom in the heteroaryl is O, N or S, the heteroatom number is 1 or 2; or
each of the rings W₁, W₂, and W₃ is independently selected from the following optionally substituted groups: 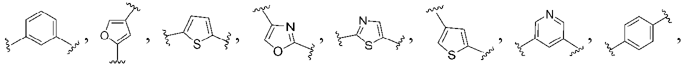 and 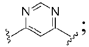 wherein "optionally substituted" refers to unsubstituted or substituted by one or more substituents independently selected from deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl; or
each of the rings W₁, W₂, and W₃ is independently selected from the following optionally substituted groups: 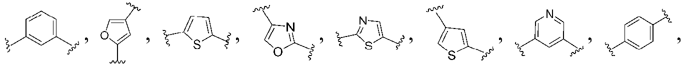 and 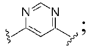 wherein "optionally substituted" refers to unsubstituted or substituted by one or more substituents independently selected from deuterium, F, Cl, Br, -CN, -OH, -NH₂, -CHO, methyl, ethyl, methoxy, ethoxy, -N(CH₃)₂, -NH(CH₃), and -C(O)CH₃; or
each of the rings W₁, W₂, and W₃ is independently selected from phenyl, and thienyl; the phenyl, and thienyl are optionally substituted by one or more substituents independently selected from deuterium, F, Cl, Br, -CN, -OH, -NH₂, -CHO, methyl, ethyl, methoxy, ethoxy, -N(CH₃)₂, - NH(CH₃), and -C(O)CH₃; or
each of the rings W₁, W₂, and W₃ is independently selected from the following optionally substituted groups: 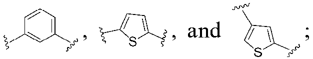 wherein "optionally substituted" refers to unsubstituted or substituted by one or more substituents independently selected from deuterium, F, Cl, Br, -CN, -OH, -NH₂, -CHO, methyl, ethyl, methoxy, ethoxy, -N(CH₃)₂, -NH(CH₃), and-C(O)CH₃; or
each of the rings W₁, W₂, and W₃ is independently selected from phenyl; the phenyl is optionally substituted by one or more substituents independently selected from F, Cl, Br, methyl, ethyl, methoxy, and ethoxy; or
each of W₁, W₂, W₃ is independently selected from

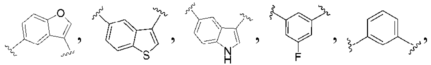

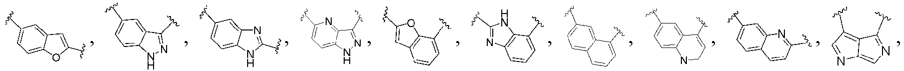

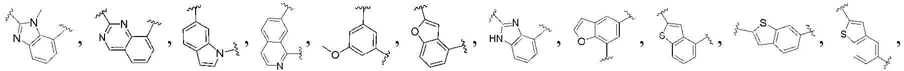

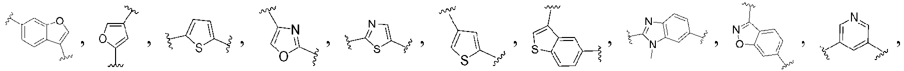

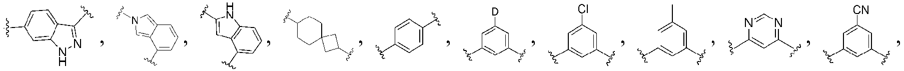

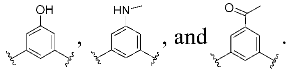

7. The compound according to any one of claims 1-6, or a pharmaceutically acceptable salt thereof, wherein R is selected from H, -C₁₋₃alkylene-C₆₋₁₀ aryl, -C₁₋₃alkylene-5-10 membered heteroaryl, C₁₋₆alkyl, and 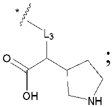 the C₁₋₃alkylene is optionally substituted by one or more substituents independently selected from deuterium, halogen, oxo, -CN, -OH, and -NH₂; the aryl, and heteroaryl are optionally substituted by one or more substituents independently selected from deuterium, halogen, -CN, methoxy, and C₁₋₃aminoalkyl; the C₁₋₆alkyl is optionally substituted by one or more substituents independently selected from deuterium, halogen, oxo, carboxyl, -CN, - OH, -NH₂, C₁₋₃alkyl, and C₁₋₃alkoxy; * is the connecting end of R and A;
preferably, R is selected from H, -methylene-phenyl, -ethylene-phenyl, -methylene-5-6 membered heteroaryl, -ethylene-5-6 membered heteroaryl, C₁₋₆alkyl, and 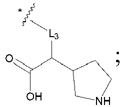 the methylene, and ethylene are optionally substituted by one or more substituents independently selected from halogen, oxo, -CN, -OH, and -NH₂; the phenyl, and heteroaryl are optionally substituted by one or more substituents independently selected from halogen, -CN, methoxy, and 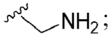 the C₁₋₆alkyl is optionally substituted by one or more substituents independently selected from halogen, oxo, carboxyl, -CN, -OH, and -NH₂; * is the connecting end of R and A; or
R is selected from H, -methylene-phenyl, -ethylene-phenyl, -methylene-pyridinyl, -ethylene-pyridinyl, methyl, ethyl, n-propyl, isopropyl, n-butyl, n-pentyl, n-hexyl, and 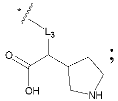 the phenyl, and pyridinyl are optionally substituted by one or more substituents independently selected from halogen, methoxy, and 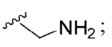 the methyl, ethyl, n-propyl, isopropyl, n-butyl, n-pentyl, and n-hexyl are optionally substituted by one or more substituents independently selected from carboxyl, and -NH₂; * is the connecting end of R and A; or
R is selected from H, -C₁₋₃alkylene-phenyl, -C₁₋₃alkylene-5-6 membered heteroaryl, C₁₋₆alkyl; the C₁₋₃alkylene is optionally substituted by one or more substituents independently selected from halogen, oxo, -CN, -OH, -NH₂, and C₁₋₃alkyl; the phenyl, and heteroaryl are optionally substituted by one or more substituents independently selected from halogen, -CN, C₁₋₃alkoxy, and C₁₋₃aminoalkyl; the C₁₋₆alkyl is optionally substituted by one or more substituents independently selected from halogen, carboxyl, -NH₂, methyl, and methoxy; or
R is selected from H, 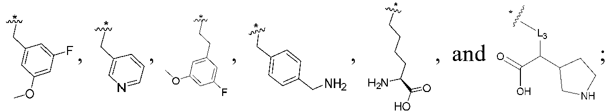 * is the connecting end of R and A; or
R is selected from H, 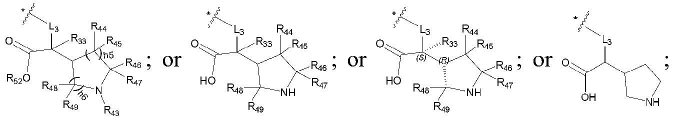 or 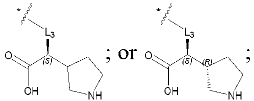 wherein * represents the connecting end with A; or
R is selected from H, 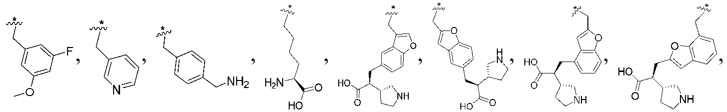 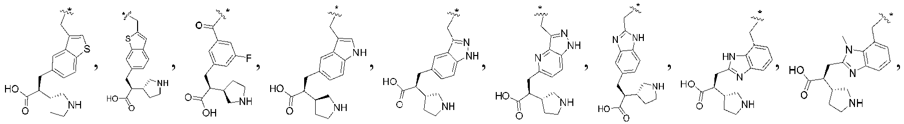 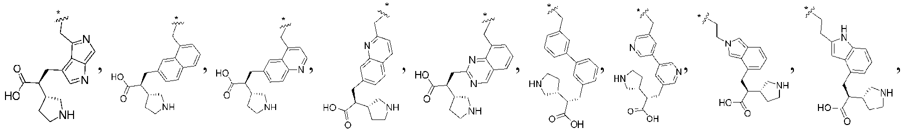 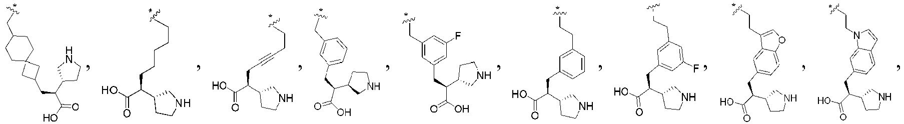 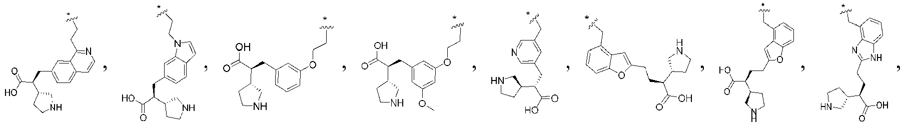 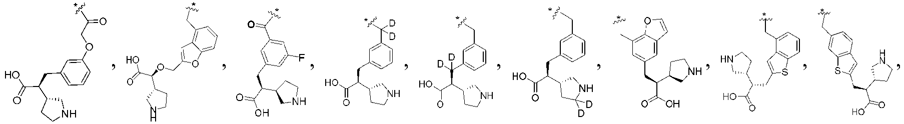 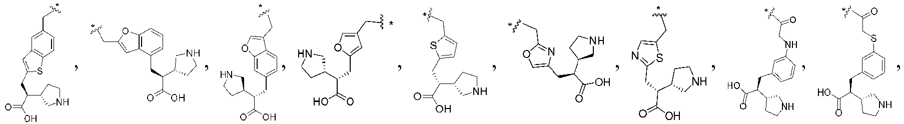 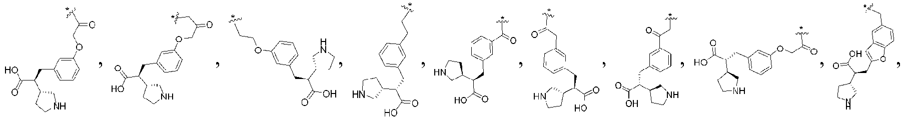 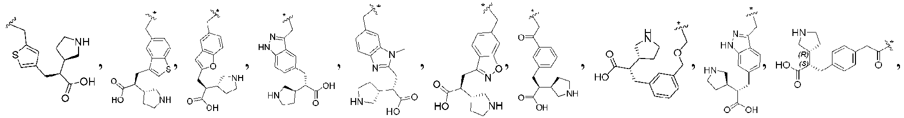 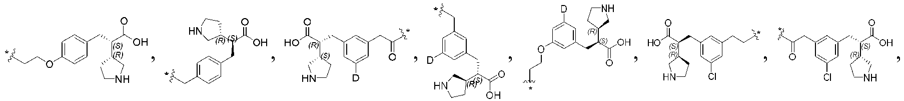 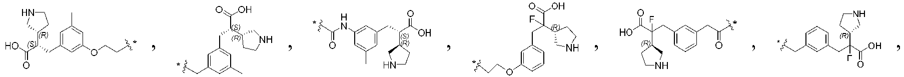 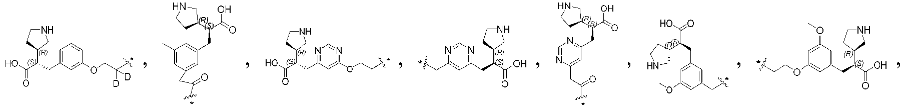 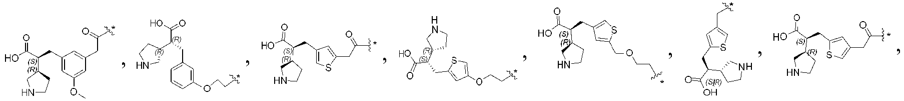 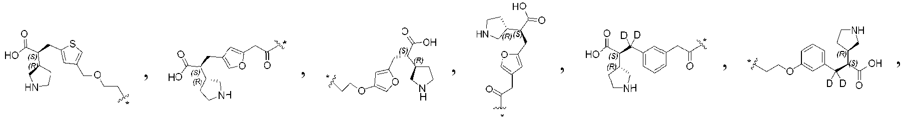 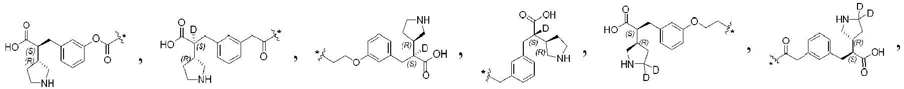 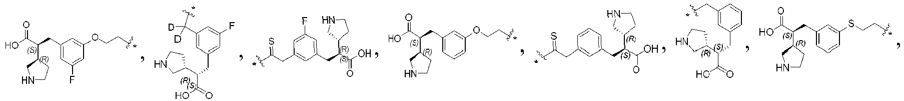 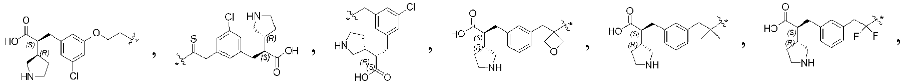 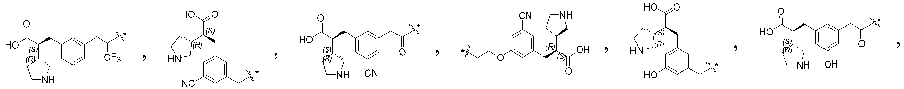 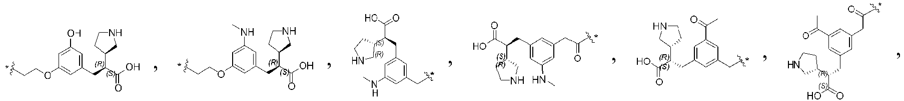 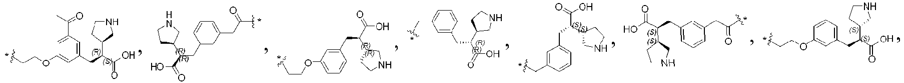 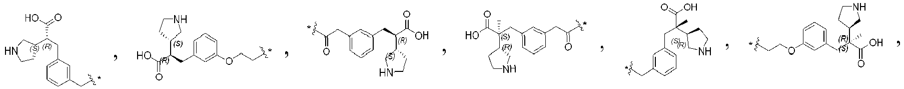 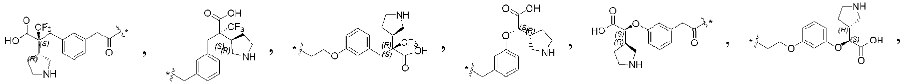 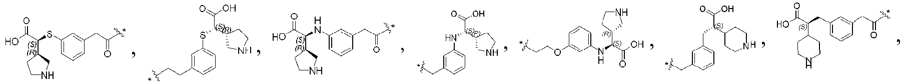 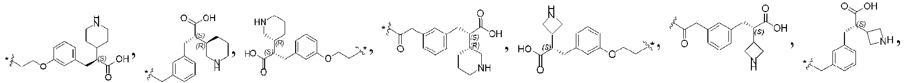 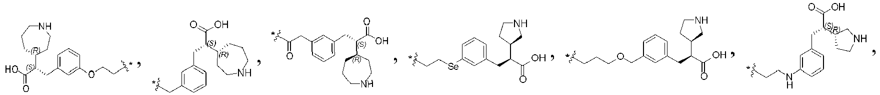 and 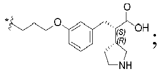 * represents the connecting end with A.

8. The compound according to any one of claims 1-7, or a pharmaceutically acceptable salt thereof, wherein L₃ is selected from -C₁₋₆alkylene-, -C₂₋₆alkynylene-, -methylene-phenyl-phenyl-methylene-, -ethylene-phenyl-phenyl-ethylene-, -methylene-5-6 membered heteroaryl-5-6 membered heteroaryl-methylene-, -ethylene-5-6 membered heteroaryl-5-6 membered heteroaryl-ethylene-, and, 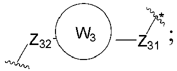 the alkylene methylene, ethylene, and alkynylene are optionally substituted by one or more substituents independently selected from halogen, oxo, -CN, -OH, - NH₂, methyl, and ethyl; the phenyl, and heteroaryl are optionally substituted by one or more substituents independently selected from halogen, -CN, methyl, ethyl, methoxy, and ethoxy; * is the connecting end of L₃ and A;
preferably, L₃ is selected from -C₁₋₆alkylene-, -C₂₋₆alkynylene-, -methylene-phenyl-phenyl-methylene-, -methylene-6 membered heteroaryl-6 membered heteroaryl-methylene-, and, 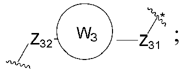 the alkylene, and methylene are optionally substituted by one or more substituents independently selected from F, Cl, Br, oxo, -CN, -OH, and -NH₂; the phenyl, and heteroaryl are optionally substituted by one or more substituents independently selected from F, Cl, Br, -CN, methyl, ethyl, methoxy, and ethoxy; * is the connecting end of L₃ and A; or
L₃ is selected from methylene, ethylene, n-propylene, isopropylene, n-butylene, n-pentylene, n-hexylene, n-propynylene, n-butynylene, n-pentynylene, n-hexynylene, -methylene-phenyl-phenyl-methylene-, -methylene-pyridinyl-pyridinyl-methylene-, and 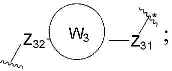 * is the connecting end of L₃ and A; or
L₃ is selected from 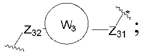 * is the connecting end of L₃ and A; or
L₃ is selected from 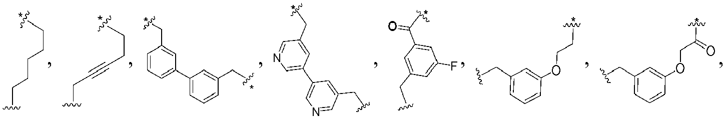 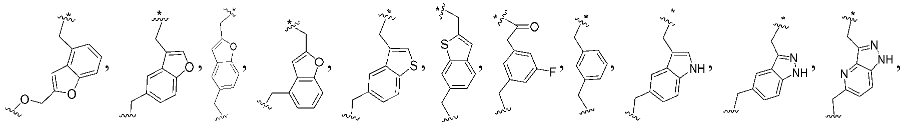 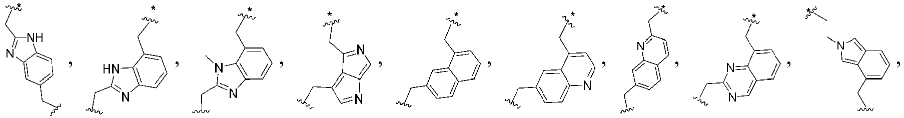 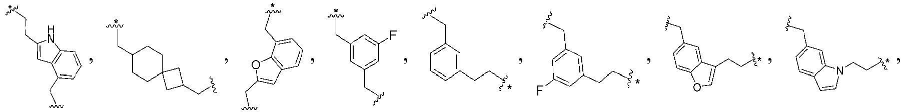 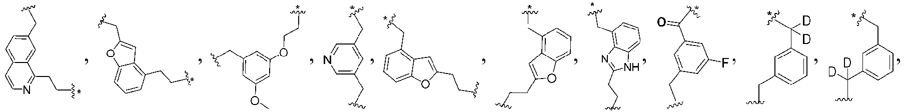 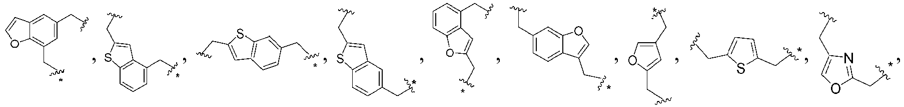 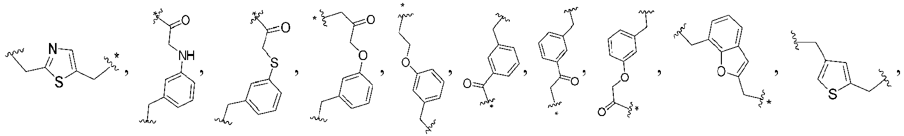 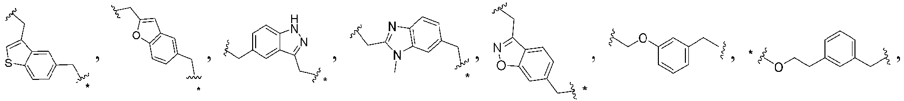 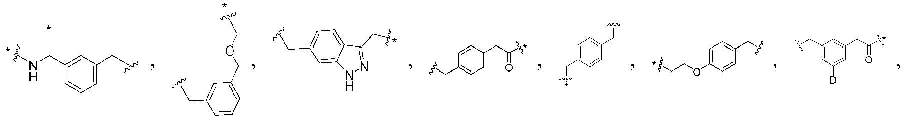 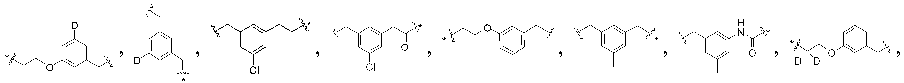 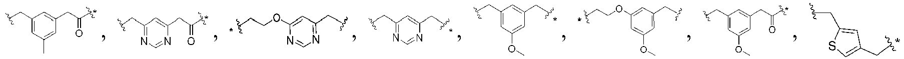 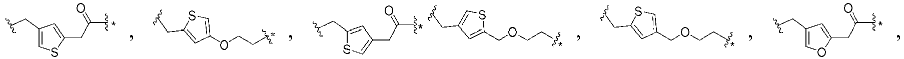 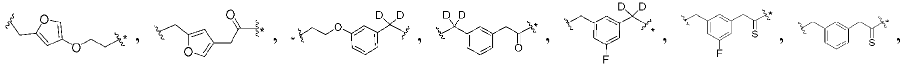 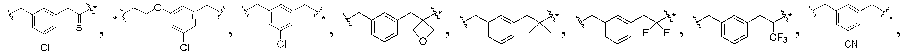 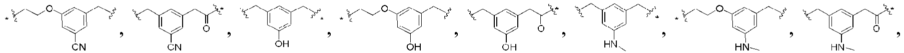 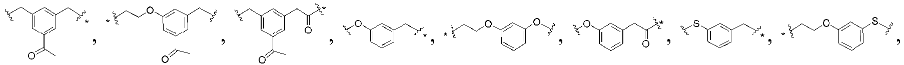 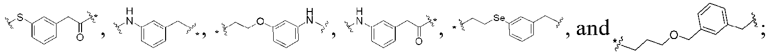 * represents the connecting end with A.

9. The compound according to any one of claims 1-8, or a pharmaceutically acceptable salt thereof, wherein each of Z₁₁, Z₁₂, Z₂₁, Z₂₂, Z₃₁, and Z₃₂ is independently selected from a bond, -O-, -S-, - NH-, -Se-, -C₁₋₄alkylene-, -C₁₋₆oxaalkylene-, -C₁₋₄thiaalkylene-, -C₁₋₄azaalkylene-,and,-C₁₋₄selenaalkylene-; the alkylene, oxaalkylene, thiaalkylene, azaalkylene, and selenaalkylene are optionally substituted by one or more substituents independently selected from Z₄₁; Z₄₁ is independently selected from deuterium, halogen (e.g. F, Cl), oxo, thio, -CN, -OH, -NH₂, C₁₋₃alkyl, C₁₋₃haloalkyl, C₁₋₃alkoxy, and C₁₋₃haloalkoxy; or any two Z₄₁ together with the atom to which they are attached form C₃₋₆cycloalkyl, or 3-6 membered heterocyclyl (e.g. oxetanyl); the heteroatom in the heterocyclyl is O, N or S, the heteroatom number is 1 or 2;
preferably, each of Z₁₁, Z₁₂, Z₂₁, Z₂₂, Z₃₁, and Z₃₂ is independently selected from a bond, -C₁₋₃alkylene-, -C₁₋₃oxaalkylene-, -C₁₋₃thiaalkylene-, and -C₁₋₃azaalkylene-; the alkylene, oxaalkylene, thiaalkylene, and azaalkylene are optionally substituted by one or more substituents independently selected from deuterium, halogen, oxo, -CN, -OH, -NH₂, methyl, and methoxy; preferably, the alkylene, oxaalkylene, thiaalkylene, and azaalkylene are optionally substituted by one or more substituents independently selected from deuterium, and oxo; or
each of Z₁₁, Z₁₂, Z₂₁, Z₂₂, Z₃₁, and Z₃₂ is independently selected from -C₁₋₂alkylene-, -C₁₋₂oxaalkylene-, -C₁₋₂thiaalkylene-, and -C₁₋₂azaalkylene-; the alkylene, oxaalkylene, thiaalkylene, and azaalkylene are optionally substituted by one or more substituents independently selected from deuterium, and oxo; or
each of Z₁₁, Z₁₂, Z₂₁, Z₂₂, Z₃₁, and Z₃₂ is independently selected from a bond, -C₁₋₃alkylene-, and - C₁₋₃oxaalkylene-; the alkylene, and oxaalkylene are optionally substituted by one or more substituents independently selected from deuterium, halogen, oxo, -CN, -OH, -NH₂, methyl, and methoxy; preferably, the alkylene, and oxaalkylene are optionally substituted by one or more substituents independently selected from deuterium, and oxo; or
each of Z₁₁, Z₁₂, Z₂₁, Z₂₂, Z₃₁, and Z₃₂ is independently selected from -C₁₋₂alkylene-, -C₁₋₃oxaalkylene-, and -C₁₋₃azaalkylene-; the alkylene, oxaalkylene, and azaalkylene are optionally substituted by one or more substituents independently selected from and oxo; or
when Z₁₁, Z₂₁, and Z₃₁ are present in the compound at the same time, in a combination of the definitions of Z₁₁, Z₂₁, and Z₃₁, at least one heteroatom or heteroatom group is present, and the heteroatom or heteroatom group is selected from -O-, -S-, -Se-, -NH-, -CO-, and -C(S)-; or
when Z₁₁, Z₂₁, and Z₃₁ are present in the compound at the same time, in a combination of the definitions of Z₁₁, Z₂₁, and Z₃₁, at least one heteroatom or heteroatom group is present, and the heteroatom or heteroatom group is selected from -CO- or -C(S)-; or
when Z₁₁, Z₂₁, and Z₃₁ are present in the compound at the same time, in a combination of the definitions of Z₁₁, Z₂₁, and Z₃₁, at least one group selected from 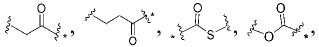 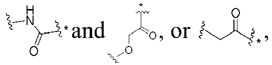 is present; * represents the connecting end with A; or
when Z₁₁, Z₂₁, and Z₃₁ are present in the compound at the same time, in a combination of the definitions of Z₁₁, Z₂₁, and Z₃₁, at least one heteroatom or heteroatom group is present, and the heteroatom or heteroatom group is selected from -O-, -NH-, and -CO-; or
when Z₁₁, Z₂₁, and Z₃₁ are present in the compound at the same time, in a combination of the definitions of Z₁₁, Z₂₁, and Z₃₁, 1-4 heteroatoms or heteroatom groups are present, the heteroatom or heteroatom group is selected from -O-, -S-, -Se-, -NH-, -CO-, and -C(S)-, and the number of heteroatoms or heteroatom groups is specifically 1, 2, 3, or 4; preferably 1-3 heteroatoms or heteroatom groups are present, and the number thereof is specifically 1, 2, or 3; or preferably 2-4 heteroatoms or heteroatom groups are present, and the number thereof is 2, 3, or 4; further preferably 2 or 3 heteroatoms or heteroatom groups are present; the heteroatom or heteroatom group is preferably -O-, -S-, -NH-, or -CO-; the heteroatom or heteroatom group is more preferably -O-, or -CO-; preferably, the heteroatom or heteroatom group at least comprises -CO- or -C(S)-; or
Z₂₁ contains a heteroatom or heteroatom group selected from -O-, -S-, -Se-, and -NH-; and Z₃₁ contains a heteroatom or heteroatom group selected from -CO-, and -C(S)-; and in a combination of the definitions of Z₂₁ and Z₃₁, the total number of the heteroatoms or heteroatom groups is 1-4; or
when Z₁₁, Z₂₁, and Z₃₁ are present in the compound at the same time, in a combination of the definitions of Z₁₁, Z₂₁, and Z₃₁, 1-4, or 1-3, or 2-3 heteroatoms or heteroatom groups are present, and the heteroatom or heteroatom group is selected from -O-, -NH-, and -CO-; preferably, the heteroatom or heteroatom group at least comprises -CO-; or
Z₁₁, Z₂₁ and Z₃₁ all together contain 1-4 heteroatoms or heteroatom groups, and the heteroatom or heteroatom group is selected from -O-, -NH-, and -CO-, specifically the number of heteroatoms or heteroatom groups is 1, 2, 3, or 4; or
Z₁₁, Z₂₁ and Z₃₁ all together contain 1-3 heteroatoms or heteroatom groups, and the heteroatom or heteroatom group is selected from -O-, -NH-, and -CO-, specifically the number of heteroatoms or heteroatom groups is 1, 2, or 3; or
Z₁₁, Z₂₁ and Z₃₁ all together contain 2-3 heteroatoms or heteroatom groups, and the heteroatom or heteroatom group is selected from -O-, -NH-, and -CO-, specifically the number of heteroatoms or heteroatom groups is 2 or 3; preferably, the heteroatom or heteroatom group at least contains -CO-; or
each of Z₁₁, Z₁₂, Z₂₁, Z₂₂, Z₃₁, and Z₃₂ is independently selected from -CD₂-, -C₁₋₄alkylene-, -OC₁₋₃alkylene-, -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₂alkyl-O-C₁₋₃alkylene-, -NH-C₁₋₃alkylene-, -S-C₁₋₃alkylene-, -C₁₋₃alkylene-NH-C(O)-, -C₁₋₃alkylene-O-C(O)-, -NH-C(O)-, -O-C(O)-, -S-C(O)-, -C₁₋₃alkylene-C(S)-, -Se-C₁₋₃alkylene-, 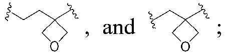 the alkylene, methylene, and ethylene are optionally substituted by one or more substituents independentlyselected from deuterium, F, and Cl; or
each of Z₁₁, Z₁₂, Z₂₁, Z₂₂, Z₃₁, and Z₃₂ is independently selected from -CD₂-, -C₁₋₃alkylene-, -OC₁₋₃alkylene-, -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₃alkylene-C(O)-C₁₋₃alkylene-, -C₁₋₃alkylene-O-C₁₋₃alkylene-, -NH-C₁₋₃alkylene-, -S-C₁₋₃alkylene-, -C₁₋₃alkylene-NH-C(O)-, -C₁₋₃alkylene-O-C(O)-, -NH-C(O)-, -O-C(O)-, and -S-C(O)-; or
each of Z₁₁, Z₁₂, Z₂₁, Z₂₂, Z₃₁, and Z₃₂ is independently selected from a bond, -CD₂-, -C₁₋₃alkylene-, -OC₁₋₃alkylene-, -C(O)-, -C₁₋₃alkylene-C(O)-, -OC₁₋₃alkylene-C(O)-, -NH-C₁₋₃alkylene-C(O)-, -S-C₁₋₃alkylene-C(O)-, and -O-C₁₋₃alkylene-C(O)-C₁₋₃alkylene-; or
each of Z₁₁, Z₁₂, Z₂₁, Z₂₂, Z₃₁, and Z₃₂ is independently selected from -C₁₋₃alkylene-, -OC₁₋₃alkylene-, -C₁₋₃alkylene-C(O)-, -OC₁₋₃alkylene-C(O)-, -NH-C(O)-, and -O-C(O)-; or
each of Z₁₁, Z₁₂, Z₂₁, Z₂₂, Z₃₁, and Z₃₂ is independently selected from -CD₂-, -C₁₋₂alkylene-, -OC₁₋₃alkylene-, -C(O)-, -C₁₋₂alkylene-C(O)-, -OC₁₋₂alkylene-C(O)-, -NH-C₁₋₂alkylene-C(O)-, -S-C₁₋₂alkylene-C(O)-, -O-C₁₋₂alkylene-C(O)-C₁₋₂alkylene-, -C₁₋₂alkylene-O-C₁₋₂alkylene-, -NH-C₁₋₂alkylene-, -S-C₁₋₂alkylene-, -C₁₋₂alkylene-NH-C(O)-, -C₁₋₂alkylene-O-C(O)-, -NH-C(O)-, -O-C(O)-, and -S-C(O)-; or
each of Z₁₁, Z₁₂, Z₂₁, Z₂₂, Z₃₁, and Z₃₂ is independently selected from a bond, -CD₂-, -C₁₋₂alkylene-, -OC₁₋₂alkylene-, -C(O)-, -C₁₋₂alkylene-C(O)-, -OC₁₋₂alkylene-C(O)-, -NH-C₁₋₂alkylene-C(O)-, -S-C₁₋₂alkylene-C(O)-, and -O-C₁₋₂alkylene-C(O)-C₁₋₂alkylene-; or
each of Z₁₁, Z₁₂, Z₂₁, Z₂₂, Z₃₁, and Z₃₂ is independently selected from a bond, methylene, ethylene, -CD₂-, 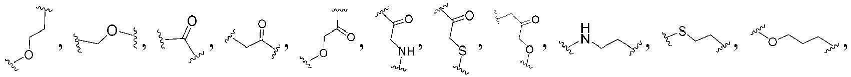 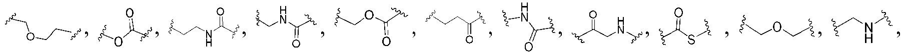 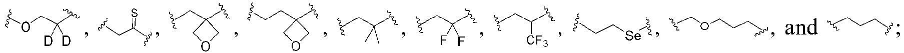 or
each of Z₁₁, Z₁₂, Z₂₁, Z₂₂, Z₃₁, and Z₃₂ is independently selected from methylene, ethylene, 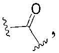 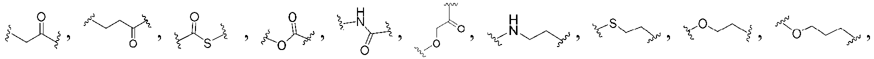 and 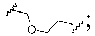 or
each of Z₁₁, Z₁₂, Z₂₁, Z₂₂, Z₃₁, and Z₃₂ is independently selected from methylene, ethylene, -CD₂, 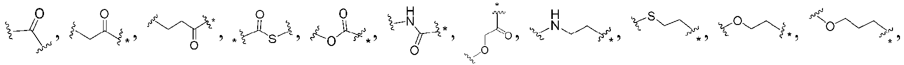 and 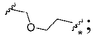 * represents the connecting end with A; or
each of Z₁₂, Z₂₂, and Z₃₂ is independently selected from methylene and -CD₂, and Z₁₁ is selected from methylene, ethylene, -CD₂, 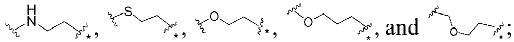 **and** Z₂₁ is selected from methylene, ethylene, -CD₂, 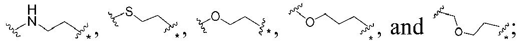 and Z₃₁ is selected from 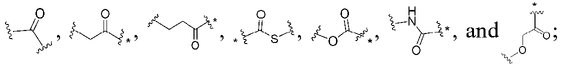 for example Z₃₁ is selected from 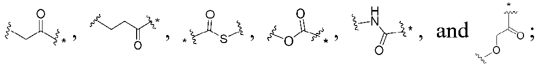 * represents the connecting end with A; or
each of Z₁₂, Z₂₂, and Z₃₂ is independently selected from methylene and -CD₂, and Z₁₁ is selected from methylene, ethylene, -CD₂, and 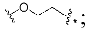 and Z₂₁ is selected from methylene, ethylene, -CD₂, 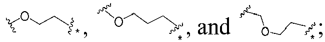 and Z₃₁ is selected from 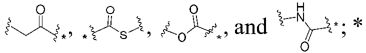 represents the connecting end with A; or
each of Z₁₁, Z₁₂, Z₂₁, Z₂₂, Z₃₁, and Z₃₂ is independently selected from methylene, ethylene, 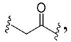 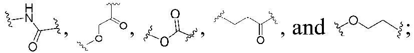 or
each of Z₁₁, Z₁₂, Z₂₁, and Z₂₂ is independently selected from -CD₂ and methylene; or methylene; or
each of Z₁₂, Z₂₂, and Z₃₂ is independently selected from -CD₂, methylene, and ethylene; or
Z₁₂, Z₂₂, Z₃₂ are all -CD₂ or methylene; or
Z₁₁ is selected from -CD₂, -C₁₋₃alkylene-, and -OC₁₋₃alkylene-; or -C₁₋₃alkylene-; or -CD₂, methylene, ethylene, methyleneoxy, and ethyleneoxy; or methylene; or
Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; or -OC₁₋₃alkylene-, -NH-C₁₋₂alkylene-, -S-C₁₋₂alkylene-, and -C₁₋₂alkylene-O-C₁₋₃alkylene-; or
Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-; or -C(O)-, -C₁₋₂alkylene-C(O)-, -OC₁₋₂alkylene-C(O)-, -NH-C(O)-, -O-C(O)-, and -S-C(O)-; or -C₁₋₂alkylene-C(O)-, -OC₁₋₂alkylene-C(O)-, -NH-C(O)-, -O-C(O)-, and -S-C(O)-; or -CH₂C(O)-; or
Z₁₁ is independently selected from -OC₁₋₃alkyl-, -NH-C₁₋₃alkyl-, -S-C₁₋₃alkyl-, -Se-C₁₋₃alkyl-, and -C₁₋₃alkyl-O-C₁₋₃alkyl-; and Z₂₁ is independently selected from -OC₁₋₃alkyl-, -NH-C₁₋₃alkyl-, -S-C₁₋₃alkyl-, -Se-C₁₋₃alkyl-, and -C₁₋₃alkyl-O-C₁₋₃alkyl-; or
Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; and Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkyl-, -C₁₋₃alkylene-NH-C(O)-, -C₁₋₃alkylene-O-C(O)-, -NH-C(O)-, -O-C(O)-, -S-C(O)-, and-C₁₋₃alkylene-C(S)-; or
Z₁₁ is selected from -CD₂, -C₁₋₃alkylene-, and -OC₁₋₃alkylene-; and Z₂₁ is selected from -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; and Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-; or
Z₁₁ is -C₁₋₃alkylene-, -OC₁₋₃alkylene-; and Z₂₁ is selected from -OC₁₋₃alkylene-, -NH-C₁₋₃alkylene-, -S-C₁₋₃alkylene-, -Se-C₁₋₃alkylene-, -C₁₋₂alkylene-O-C₁₋₃alkylene-; and Z₃₁ is selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, - O-C(O)-, -S-C(O)-, -C₁₋₃alkylene-C(S)-; or
Z₁₁ is selected from -CD₂, -C₁₋₃alkylene-, and -OC₁₋₃alkylene-*; and Z₂₁ is selected from -OC₁₋₃alkylene-*, -NH-C₁₋₃alkylene-*, -S-C₁₋₃alkylene-*, -Se-C₁₋₃alkylene-*, and -C₁₋₂alkylene-O-C₁₋₃alkylene-*; and Z₃₁ is selected from -C(O)-, -C₁₋₃alkylene-C(O)-*, -OC₁₋₃alkylene-C(O)-*, -NH-C₁₋₃alkylene-C(O)-*, -S-C₁₋₃alkylene-C(O)-*, -O-C₁₋₂alkylene-C(O)-C₁₋₃alkylene-*, -C₁₋₃alkylene-NH-C(O)-*, -C₁₋₃alkylene-O-C(O)-*, -NH-C(O)-*, -O-C(O)-*, -S-C(O)-*, and -C₁₋₃alkylene-C(S)-*; * represents the connecting end with A; or
Z₁₁ is selected from -C₁₋₃alkylene-, and -OC₁₋₃alkylene-*; and Z₂₁ is selected from -OC₁₋₃alkylene-*, -NH-C₁₋₃alkylene-*, -S-C₁₋₃alkylene-*, -Se-C₁₋₃alkylene-*, and -C₁₋₂alkylene-O-C₁₋₃alkylene-*; and Z₃₁ is selected from -C(O)-, -C₁₋₃alkylene-C(O)-*, -OC₁₋₃alkylene-C(O)-*, -NH-C₁₋₃alkylene-C(O)-*, -S-C₁₋₃alkylene-C(O)-*, -O-C₁₋₂alkylene-C(O)-C₁₋₃alkylene-, -C₁₋₃alkylene-NH-C(O)-*, -C₁₋₃alkylene-O-C(O)-*, -NH-C(O)- *, -O-C(O)-*, -S-C(O)-*, and -C₁₋₃alkylene-C(S)-* ; * represents the connecting end with A; or
Z₁₁ is selected from -CD₂, -C₁₋₂alkylene-, and -OC₁₋₂alkylene-*; and Z₂₁ is selected from -OC₁₋₂alkylene-*, -NH-C₁₋₂alkylene-*, -S-C₁₋₂alkylene-*, -Se-C₁₋₂alkylene-*, and -CH₂-O-C₁₋₂alkylene-*; and Z₃₁ is selected from -C(O)-, -C₁₋₂alkylene-C(O)-*, -OC₁₋₂alkylene-C(O)-*, -NH-C₁₋₂alkylene-C(O)-*, -S-C₁₋₂alkylene-C(O)-*, -C₁₋₂alkylene-NH-C(O)-*, -C₁₋₂alkylene-O-C(O)-*, -NH-C(O)-*, -O-C(O)-*, -S-C(O)-*, and -C₁₋₂alkylene-C(S)-*; for example, Z₃₁ is selected from -C₁₋₂alkylene-C(O)-*, -OC₁₋₂alkylene-C(O)-*, -NH-C₁₋₂alkylene-C(O)-*, -S-C₁₋₂alkylene-C(O)-*, -C₁₋₂alkylene-NH-C(O)-*, -C₁₋₂alkylene-O-C(O)-*, -CH₂NH-C(O)-*, -NH-C(O)-*, -O-C(O)-*,-S-C(O)-*, and -C₁₋₂alkylene-C(S)-*; for example, Z₃₁ is selected from -C₁₋₂alkylene-C(O)-*,-OCH₂-C(O)-*, -CH₂-O-C(O)-*, -NH-C(O)-*, -O-C(O)-*, and -S-C(O)-*; for example, Z₃₁ is selected from -CH₂C(O)-*; * represents the connecting end with A.

10. The compound according to any one of claims 1-9, or a pharmaceutically acceptable salt thereof, wherein
the compound has a structure as represented by formula (I-2): 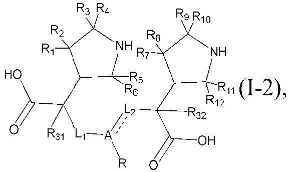
wherein A, - - -, L₁, L₂, R, R₁, R₂, R₃, R₄, R₅, R₆, R₇, R₈, R₉, R₁₀, R₁₁, R₁₂, R₃₁, and R₃₂ are defined as in any of claims 1-9;
for example, in the compound represented by formula (I-2), R is a group containing L₃, L₁, L₂ and L₃ are groups containing Z₁₁, Z₂₁ and Z₃₁ respectively, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, - CH₂CH₂-, -CD₂CH₂-, -C(O)-, -CH₂C(O)-, -NHC(O)-, -OC(O)-, -CH₂CH₂O-, -OCH₂C(O)-, - CH₂OC(O)-, -CH₂CH₂CH₂O-, and -S-C(O)-;
or
the compound has a structure as represented by formula (I-3): 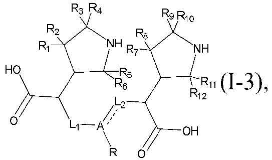
wherein A, - - - , L₁, L₂, R, R₁, R₂, R₃, R₄, R₅, R₆, R₇, R₈, R₉, R₁₀, R₁₁, and R₁₂ are defined as in any of claims 1-9;
for example, in the compound represented by formula (I-3), R is a group containing L₃, L₁, L₂ and L₃ are groups containing Z₁₁, Z₂₁ and Z₃₁ respectively, and only one of Z₁₁, Z₂₁ and Z₃₁ is a group containing a double bond; the double bond is, for example, a double bond between C and O in - C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-,-CH₂CH₂CH₂O-, and -S-C(O)-;
or
the compound has a structure as represented by formula (I'-1), formula (I'-2), or formula (I'-3): 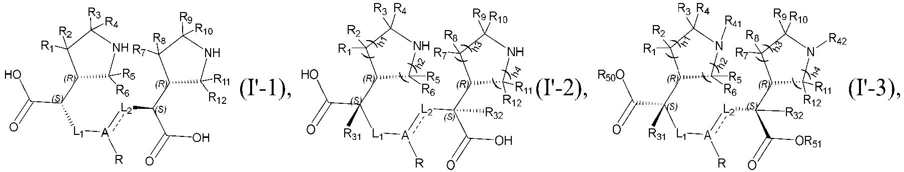
wherein A, L₁, L₂, R, R₁, R₂, R₃, R₄, R₅, R₆, R₇, R₈, R₉, R₁₀, R₁₁, R₁₂, R₃₁, R₃₂, R₄₁, R₄₂, R₅₀, R₅₁, h1, h2, h3, h4 are defined as in any of claims 1-9;
for example, in the compound represented by formula (I'-1), (I'-2), or (I'-3), R is a group containing L₃, one and only one of Z₁₁, Z₂₁ and Z₃₁ is a group containing a double bond; the double bond is, for example, a double bond between C and O in -C(O)-, or a double bond between C and S in - C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-;
or
the compound has a structure as represented by formula (II): 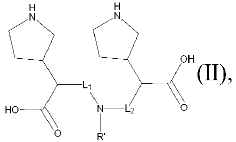
wherein L₁, L₂ are defined as in any of claims 1-9, R' is selected from H, -C₁₋₃alkylene-C₆₋₁₀ aryl, -C₁₋₃alkylene-5-12 membered heteroaryl, and C₁₋₆alkyl; the C₁₋₃alkylene is optionally substituted by one or more substituents independently selected from deuterium, halogen, oxo, -CN, -OH, - NH₂, and C₁₋₃alkyl; the aryl, and heteroaryl are optionally substituted by one or more substituents independently selected from deuterium, halogen, -CN, C₁₋₃alkoxy, and C₁₋₃aminoalkyl; the C₁₋₆alkyl is optionally substituted by one or more substituents independently selected from deuterium, halogen, oxo, carboxyl, -CN, -OH, -NH₂, C₁₋₃alkyl, and C₁₋₃alkoxy;
or
the compound has a structure as represented by formula (II-A), formula (II-B), formula (II-C), formula (II-A'), formula (II-B'), or formula (II-C'): 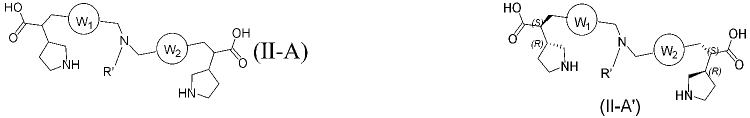 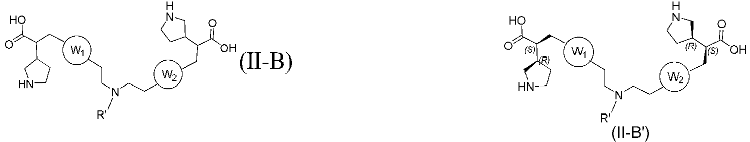 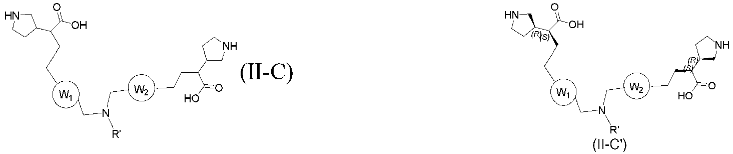
wherein W₁, W₂, R' are defined as in any of claims 1-9;
or
the compound has a structure as represented by formula (III): 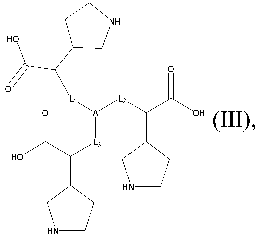
wherein A, L₁, L₂, L₃ are defined as in any of claims 1-9;
for example, in the compound represented by formula (III), L₁, L₂ and L₃ are groups containing Z₁₁, Z₂₁ and Z₃₁ respectively, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-,-CH₂C(O)-, -NHC(O)-, -OC(O)-, -CH₂CH₂O-, -OCH₂C(O)-, -CH₂OC(O)-, -CH₂CH₂CH₂O-, and-S-C(O)-;
or
the compound has a structure as represented by formula (III-1): 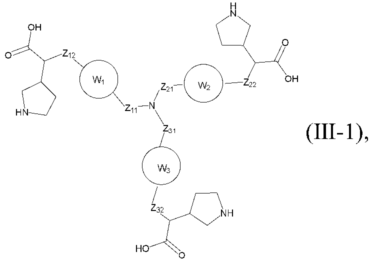
wherein W₁, W₂, W₃, Z₁₁, Z₁₂, Z₂₁, Z₂₂, Z₃₁, Z₃₂ are defined as in any of claims 1-9;
preferably, each of Z₁₂, Z₂₂, and Z₃₂ is independently selected from methylene, and ethylene;
each of Z₁₁, Z₂₁, Z₃₁ is independently selected from a bond, -C₁₋₄alkylene-, -C₁₋₆oxaalkylene- (e.g. -C₁₋₄oxaalkylene-), -C₁₋₄thiaalkylene-, -C₁₋₄azaalkylene-, and -C₁₋₄selenaalkylene-; the alkylene, oxaalkylene, thiaalkylene, azaalkylene, and selenaalkylene are optionally substituted by one or more substituents independently selected from Z₄₁; Z₄₁ is independently selected from deuterium, halogen (e.g. F, Cl), oxo, thio, -CN, -OH, -NH₂, C₁₋₃alkyl, C₁₋₃haloalkyl, C₁₋₃alkoxy, and C₁₋₃haloalkoxy; or any two Z₄₁ together with the atom to which they are attached form C₃₋₆cycloalkyl, or 3-6 membered heterocyclyl (e.g. oxetanyl); the heteroatom in the heterocyclyl is O, N or S, the heteroatom number is 1;
each of the rings W₁, W₂, and W₃ is independently selected from phenyl, and 5-6 membered monocyclic heteroaryl; the phenyl, and heteroaryl are optionally substituted by one or more substituents independently selected from deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl; the heteroatom in the heteroaryl is O, N or S, the heteroatom number is 1 or 2; or
Z₁₂, Z₂₂, Z₃₂ are all methylene; or
when Z₁₁, Z₂₁, and Z₃₁ are present in the compound at the same time, in a combination of the definitions of Z₁₁, Z₂₁, and Z₃₁ 2 or 3 heteroatoms or heteroatom groups are present, and the heteroatom or heteroatom group is selected from -O-, -S-, -NH-, and -CO-;
each of the rings W₁, W₂, and W₃ is independently selected from phenyl, and thienyl; the phenyl, and thienyl are optionally substituted by one or more substituents independently selected from deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl; or
each of the rings W₁, W₂, and W₃ is independently selected from 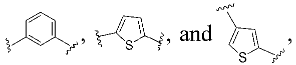 which are optionally substituted by one or more substituents independently selected from deuterium, F, Cl, Br, methyl, ethyl, methoxy, and ethoxy;
for example, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28,
about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-;
or
the compound has a structure as represented by formula (III-2): 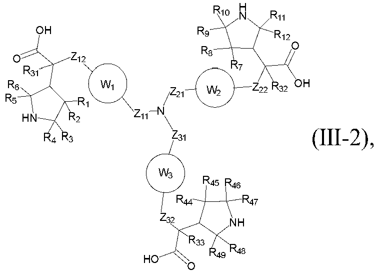
wherein W₁, W₂, W₃, Z₁₁, Z₁₂, Z₂₁, Z₂₂, Z₃₁, Z₃₂, R₁, R₂, R₃, R₄, R₅, R₆, R₇, R₈, R₉, R₁₀, R₁₁, R₁₂, R₄₄, R₄₅, R₄₆, R₄₇, R₄₈, R₄₉, R₃₁, R₃₂, and R₃₃ are defined as in any of claims 1-9;
preferably, the heteroatom or heteroatom group in Z₃₁ comprises -CO- or -C(S)-; or
R₁, R₂, R₃, R₄, R₅, R₆, R₇, R₈, R₉, R₁₀, R₁₁, R₁₂, R₄₄, R₄₅, R₄₆, R₄₇, R₄₈, R₄₉, R₃₁, R₃₂, and R₃₃ are selected from hydrogen or deuterium;
for example, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28,
about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-;
or
the compound has a structure as represented by formula (III-1-1), or formula (III-2-1): 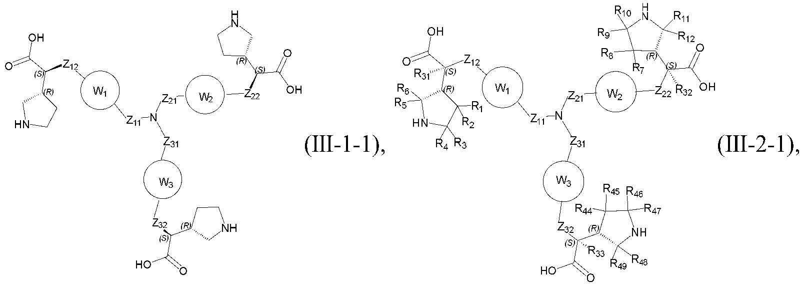
wherein W₁, W₂, W₃, Z₁₁, Z₁₂, Z₂₁, Z₂₂, Z₃₁, Z₃₂, R₁, R₂, R₃, R₄, R₅, R₆, R₇, R₈, R₉, R₁₀, R₁₁, R₁₂, R₄₄, R₄₅, R₄₆, R₄₇, R₄₈, R₄₉, R₃₁, R₃₂, and R₃₃ are defined as in any of claims 1-9;
for example, one and only one of Z₁₁, Z₂₁ and Z₃₁ is a group containing a double bond; the double bond is, for example, a double bond between C and O in -C(O)-, or a double bond between C and S in -C(S)-; further preferably, Z₁₁, Z₂₁ and Z₃₁ are different from each other, or two groups not containing the double bond in Z₁₁, Z₂₁ and Z₃₁ are the same, and the two same groups contain -O-, -NH-, -S-, or -Se-, preferably, -O-; still further preferably, Z₁₁, Z₂₁ and Z₃₁ each independently have a molecular weight of 14.0-107.1, preferably 14.0-60; preferably about 14, about 16, about 28,
about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-;
or
the compound has a structure as represented by formula (III-A), or formula (III-A'): 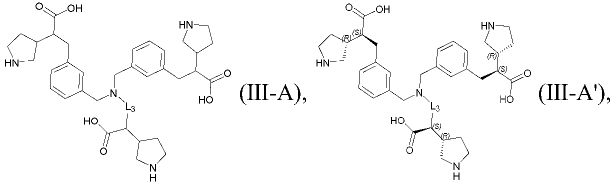
wherein L₃ are defined as in any of claims 1-9;
or
the compound has a structure as represented by formula (III-B), or formula (III-B'-1): 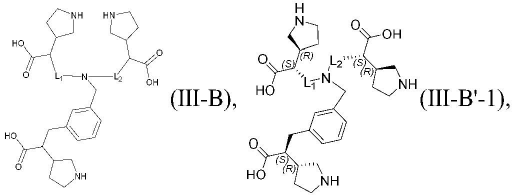
wherein L₁, L₂ are defined as in any of claims 1-9;
preferably, L₁ 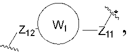 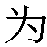 *represents the connecting end with A; or
L₂ is 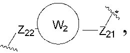 * represents the connecting end with A; or
each of Z₁₁, Z₁₂, Z₂₁, and Z₂₂ is independently selected from -C₁₋₃alkylene-, -C₁₋₃oxaalkylene-, - C₁₋₃thiaalkylene-, and -C₁₋₃azaalkylene-; the alkylene, oxaalkylene, thiaalkylene, and azaalkylene are optionally substituted by one or more substituents independently selected from deuterium, halogen, oxo, -CN, -OH, -NH₂, methyl, and methoxy; or
each of Z₁₁, Z₁₂, Z₂₁, and Z₂₂ is independently selected from -C₁₋₃alkylene-, and -C₁₋₃oxaalkylene-; the alkylene, and oxaalkylene are optionally substituted by one or more substituents independently selected from deuterium, halogen, oxo, -CN, -OH, -NH₂, methyl, and methoxy; or
each of Z₁₁, Z₁₂, Z₂₁, and Z₂₂ is independently selected from -C₁₋₂alkylene-, -C₁₋₂oxaalkylene-, - C₁₋₂thiaalkylene-, and -C₁₋₂azaalkylene-; the alkylene, oxaalkylene, thiaalkylene, and azaalkylene are optionally substituted by one or more substituents independently selected from deuterium, halogen, oxo, -CN, -OH, -NH₂, methyl, and methoxy; or
each of Z₁₁, Z₁₂, Z₂₁, and Z₂₂ is independently selected from methylene, 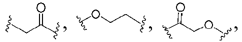 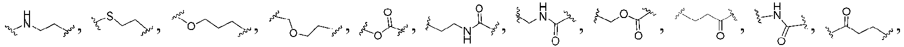 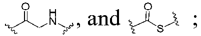
for example, L1 and L2 are groups containing Z₁₁ and Z₂₁ respectively, one and only one of Z₁₁ and Z₂₁ is a group containing a double bond; the double bond is, for example, a double bond between C and O in -C(O)-, or a double bond between C and S in -C(S)-; preferably, the double bond is for example a double bond between C and O in -C(O)-; further preferably, neither Z₁₁ nor Z₂₁ is methylene, or a group not containing the double bond of Z₁₁ and Z₂₁ contains -O-, -NH-, - S-, or -Se-, preferably, -O-; still further preferably, Z₁₁ and Z₂₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, - OC(O)-, -CH₂CH₂O-, -OCH₂C(O)-, -CH₂OC(O)-, -CH₂CH₂CH₂O-, and -S-C(O)-;
or
the compound has a structure as represented by formula (III-C), formula (III-C'), formula (III-D), formula (III-D'), formula (III-E), or formula (III-E'): 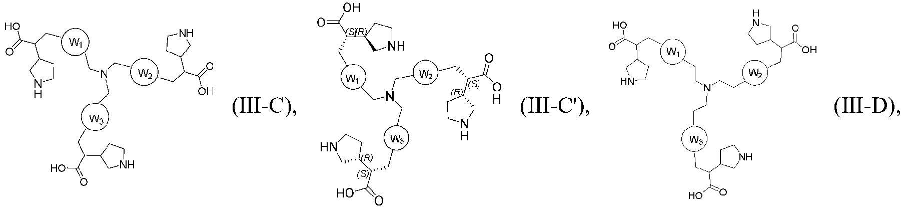 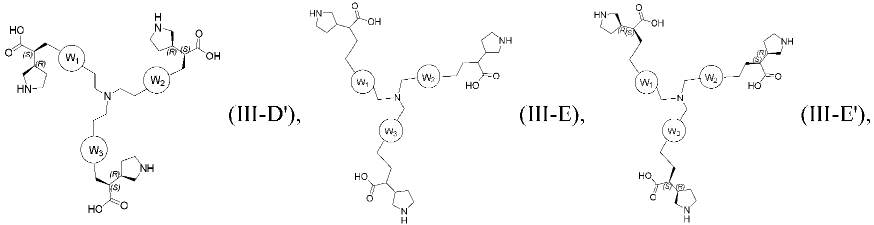
wherein W₁, W₂, W₃ are defined as in any of claims 1-9;
or
the compound has a structure as represented by formula (IV): 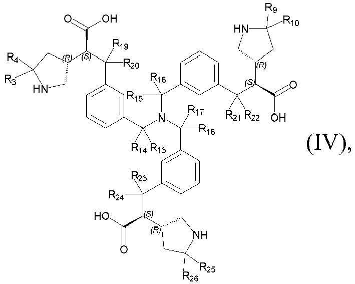
wherein R₃, R₄, R₉, R₁₀, R₁₃, R₁₄, R₁₅, R₁₆, R₁₇, R₁₈, R₁₉, R₂₀, R₂₁, R₂₂, R₂₃, R₂₄, R₂₅, and R₂₆ are each independently selected from H, deuterium, and at least one of R₃, R₄, R₉, R₁₀, and R₁₃-R₂₆ is deuterium;
or
the compound has a structure as represented by formula (A): 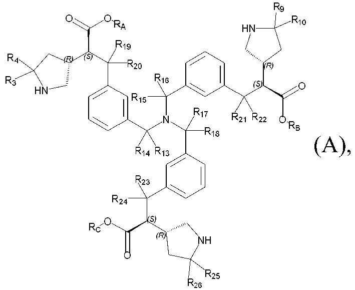
wherein R₃, R₄, R₉, R₁₀, R₁₃, R₁₄, R₁₅, R₁₆, R₁₇, R₁₈, R₁₉, R₂₀, R₂₁, R₂₂, R₂₃, R₂₄, R₂₅, and R₂₆ are each independently selected from H, and deuterium, and at least one of R₃, R₄, R₉, R₁₀, and R₁₃-R₂₆ is deuterium; R_{A}, R_{B}, R_{C} are each independently selected from hydrogen, C₁₋₆alkyl, C₂₋₆alkenyl, C₁₋₆alkoxy, -C₁₋₃alkyleneO-C₁₋₆alkyl, and -C₁₋₃alkylene-O-C(O)-C₁₋₆alkyl, the alkyl, alkenyl, alkoxy, and alkylene are optionally substituted by one or more substituents independently selected from deuterium, halogen, -OH, -NH₂, -CN, oxo, C₁₋₃alkyl, and C₁₋₃alkoxy;
preferably, R_{A}, R_{B}, R_{C} are each independently selected from C₁₋₆alkyl, C₁₋₆alkoxy, -C₁₋₂alkylene-O-C₁₋₄alkyl, and -C₁₋₂alkylene-O-C(O)-C₁₋₄alkyl, the alkyl, C₁₋₆alkoxy, and alkylene are optionally substituted by one or more substituents independently selected from deuterium, F, Cl, Br, -OH, - NH₂, -CN, oxo, methyl, ethyl, n-propyl, isopropyl, methoxy, ethoxy, n-propyloxy, and isopropyloxy; or
R_{A}, R_{B}, R_{C} are each independently selected from C₁₋₃alkyl, -methylene-O-C₁₋₄alkyl, and - methylene-O-C(O)-C₁₋₃alkyl, the methylene and alkyl are optionally substituted by one or more substituents independently selected from methyl, ethyl, n-propyl, and isopropyl; or
R_{A}, R_{B}, R_{C} are each independently selected from methyl, ethyl, and -CH₂-OC(O)-CH(CH₃)₂;
or
the compound has a structure as represented by formula (B), formula (B-1), formula (C), formula (C-1), formula (D), formula (D-1), formula (B-2), formula (B-3), formula (C-2), formula (C-3), formula (D-2), or formula (D-3): 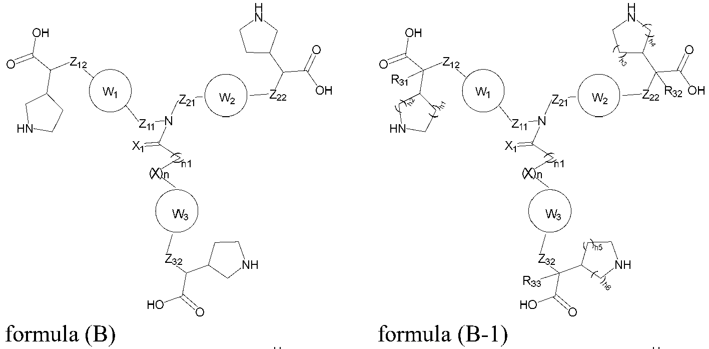 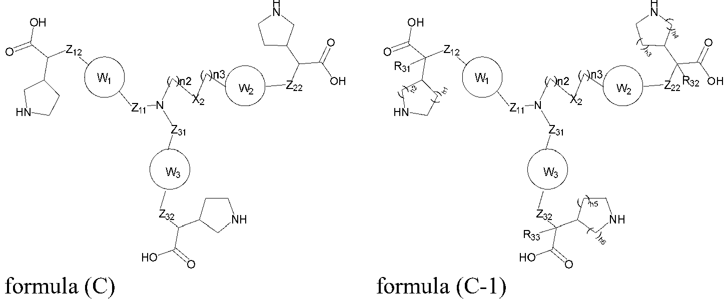 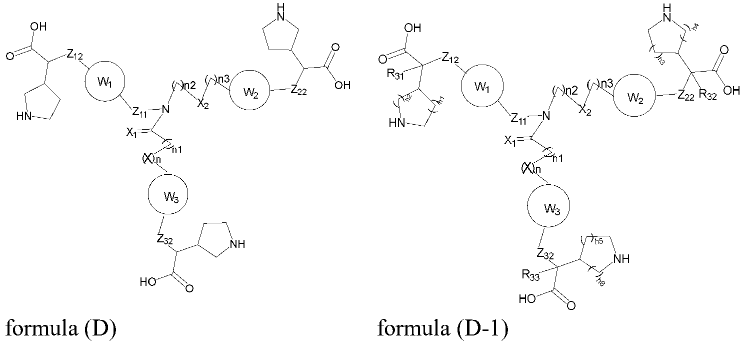 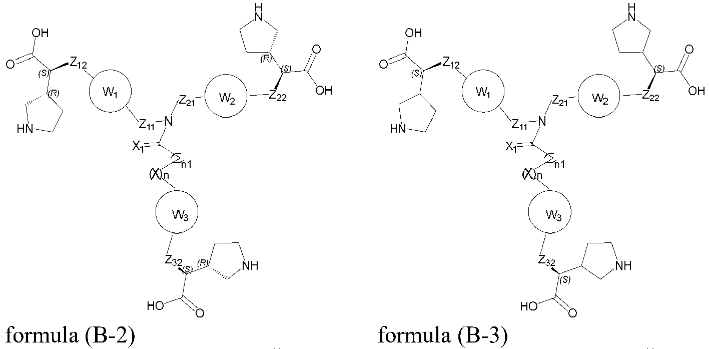 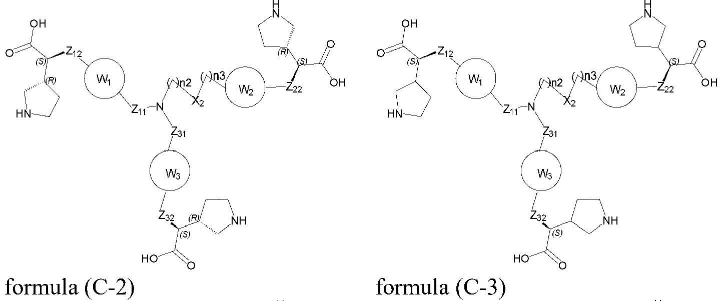 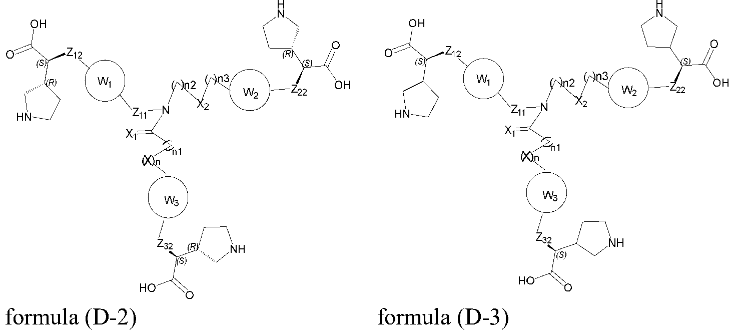
wherein
Z₁₁, Z₂₁, Z₃₁, R₃₁, R₃₂, R₃₃, h1, h2, h3, h4, h5, h6 are defined as in any of claims 1-9,
X is selected from O, CH₂, NH, S, and Se, or X is selected from O, CH₂, NH, and S; or X is CH₂;
X₂ is selected from O, CH₂, NH, S, and Se, or X₂ is selected from O, NH, and S; or X₂ is O;
X₁ is independently selected from O, and S, or X₁ is O;
n, n1, and n3 are each independently 0 or 1,
n2 is independently 0, 1, 2 or 3;
each of Z₁₂, Z₂₂, and Z₃₂ is independently selected from methylene, ethylene, and -CD₂-; or each of Z₁₂, Z₂₂, and Z₃₂ is independently selected from methylene, and -CD₂-; or Z₁₂, Z₂₂, Z₃₂ are all methylene;
each of the rings W₁, W₂, and W₃ is independently selected from phenyl, and 5-6 membered monocyclic heteroaryl; the phenyl, and heteroaryl are optionally substituted by one or more substituents independently selected from deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl; the heteroatom in the heteroaryl is O, N or S, the heteroatom number is 1 or 2;
or
each of the rings W₁, W₂, and W₃ is independently selected from the following optionally substituted groups: 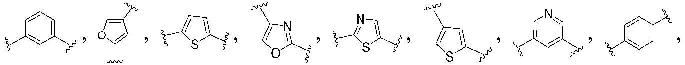 and 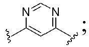 wherein "optionally substituted" refers to unsubstituted or substituted by one or more substituents independently selected from deuterium, halogen, -CN, -OH, -NH₂, -CHO, C₁₋₃alkyl, C₁₋₃alkoxy, -N(C₁₋₃alkyl)₂, -NH(C₁₋₃alkyl), and -C(O)C₁₋₃alkyl;
or
each of the rings W₁, W₂, and W₃ is independently selected from the following optionally substituted groups: 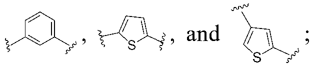 wherein "optionally substituted" refers to unsubstituted or substituted by one or more substituents independently selected from deuterium, F, Cl, Br, methyl, ethyl, methoxy, and ethoxy;
preferably,
the sum of n2 and n3 is not greater than 3 (e.g. 0, 1, 2, 3); for example the sum of n2 and n3 is not greater than 2 (e.g. 0, 1, 2); for example the sum of n2 and n3 is 1 or 2 (e.g. n2 is 2, n3 is 0; or n2 is 1, n3 is 1; or n2 is 0, n3 is 2); or
the sum of n and n1 is 0, 1, or 2; for example the sum of n and n1 is 1 or 2 (i.e. n is 1, n1 is 0; or n is 0, n1 is 1; or n is 1, n1 is 1; or n is 0, n1 is 2; or n is 2, n1 is 0); or
the sum of n and n1 is 1 (i.e. n is 1, n1 is 0; or n is 0, n1 is 1);
for example:
n2 is 0 or 1, n3 is 0, X₂ is CH₂; or
n2 is 2 or 3, n3 is 0, X₂ is O, NH, or S; or
n2 is 1 or 2, n3 is 1, X₂ is O, NH, or S; or
n2 is 2 or 3, n3 is 0, X₂ is O; or
n2 is 2, n3 is 1, X₂ is O; or
n2 is 2, n3 is 0, X₂ is O, NH, or S; or
n2 is 2, n3 is 0, X₂ is O;
and/or for example:
X is selected from O, CH₂, NH, and S, n is 1, n1 is 0; or
X is O or CH₂, n is 1, n1 is 1; or
X₁ is O, X is selected from O, CH₂, NH, and S, n is 1, n1 is 0; or
X₁ is O, X is O or CH₂, n is 1, n1 is 1; or
X₁ is O, X is O or CH₂, n is 1, n1 is 0; or
X₁ is O, X is O or CH₂, n is 1, n1 is 1; or
X₁ is O, X is CH₂, n is 1, n1 is 0;
and/or, preferably:
Z₁₁ is -CD₂-; or
Z₁₁ is independently selected from -C₁₋₃alkylene-, -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; or, Z₁₁ is independently selected from -C₁₋₃alkylene-, -OC₁₋₃alkylene-; or, Z₁₁ is independently selected from methylene, ethylene, methyleneoxy, ethyleneoxy; or, Z₁₁ is independently selected from methylene, and -CD₂-; preferably, Z₁₁ is independently selected from methylene; and/or
Z₂₁ is -CD₂-; or
Z₂₁ is independently selected from -C₁₋₃alkylene-, -OC₁₋₃alkylene-, -NH-C₁₋₃alkylene-, -S-C₁₋₃alkylene-, -Se-C₁₋₃alkylene-, and -C₁₋₂alkylene-O-C₁₋₃alkylene-; or, Z₂₁ is -OC₁₋₃alkylene-, -NH-C₁₋₂alkylene-, -S-C₁₋₂alkylene-, and -C₁₋₂alkylene-O-C₁₋₃alkylene-;
and/or, preferably:
Z₃₁ is independently selected from -C(O)-, -C₁₋₃alkylene-C(O)-, -OC₁₋₃alkylene-C(O)-, -NH-C₁₋₃alkylene-C(O)-, -S-C₁₋₃alkylene-C(O)-, -O-C₁₋₂alkylene-C(O)-C₁₋₃alkylene-, -C₁₋₃alkylene-NH-C(O)-, -C₁₋₃alkylene-O-C(O)-, -NH-C(O)-, -O-C(O)-, -S-C(O)-, and -C₁₋₃alkylene-C(S)-; or, Z₃₁ is -C(O)-, -C₁₋₂alkylene-C(O)-, -OC₁₋₂alkylene-C(O)-, -NH-C(O)-, -O-C(O)-, and -S-C(O)-;
for example, in the above-mentioned formula (B) and formula (B-1),
each of Z₁₂, Z₂₂, and Z₃₂ is independently selected from methylene, and -CD₂-;
each of Z₁₁ and Z₂₁ is independently selected from -CD₂-, -C₁₋₃alkylene- (for example -C₁₋₂alkylene-), -OC₁₋₃alkylene-* (for example -OC₁₋₂alkylene-*), -SC₁₋₃alkylene-*, -NHC₁₋₃alkylene-*, -Se-C₁₋₃alkylene-*, and -CH₂-O-C₁₋₂alkylene-*, * represents the connecting end with N;
X₁ is O or S, X is selected from O, CH₂, NH, and S, n is 1, n1 is 0; or X₁ is O or S, X is O or CH₂, n is 1, n1 is 1; or X₁ is O or S, n is 0, n1 is 0; or
again for example, in the above-mentioned formula (B) and formula (B-1),
each of Z₁₂, Z₂₂, and Z₃₂ is independently selected from methylene, and -CD₂-;
Z₁₁ is independently selected from -CD₂-, -CH₂-, -CH₂CH₂-, -OCH₂-*, and -OCH₂CH₂-*, * represents the connecting end with N;
Z₂₁ is independently selected from -OCH₂CH₂-*, -CH₂OCH₂CH₂-*, and -OCH₂CH₂CH₂-*, * represents the connecting end with N;
X₁ is O, X is selected from O, CH₂, NH, and S, n is 1, n1 is 0; or X₁ is O, X is O or CH₂, n is 1, n1 is 1; or
again for example, in the above-mentioned formula (B) and formula (B-1),
each of Z₁₂, Z₂₂, and Z₃₂ is independently selected from methylene, and -CD₂-;
each of Z₁₁ and Z₂₁ is independently selected from -CD₂-, -C₁₋₃alkylene- (for example -C₁₋₂alkylene-), -OC₁₋₃alkylene-* (for example -OC₁₋₂alkylene-*), -SC₁₋₃alkylene-*, -NHC₁₋₃alkylene-*, -Se-C₁₋₃alkylene-*, and -CH₂-O-C₁₋₂alkylene-*, * represents the connecting end with N,
X₁ is O or S,
X is selected from O, CH₂, NH, and S,
the sum of n and n1 is 0, 1, or 2;
further for example,
Z₂₁ is independently selected from -OCH₂CH₂-*, -CH₂OCH₂CH₂-*, and -OCH₂CH₂CH₂-*, * represents the connecting end with N;
X₁ is O, X is selected from O, CH₂, NH, and S,
the sum of n and n1 is 1, or 2;
further for example, in any of the above-mentioned formula (B), formula (B-1), formula (B-2), and formula (B-3), each of Z₁₁ and Z₂₁ is a group not containing a double bond; the double bond is, for example, a double bond between C and O in -C(O)-, or a double bond between C and S in - C(S)-; further preferably, Z₁₁ and Z₂₁ are identical or different, and at least one of Z₁₁ and Z₂₁ is a group containing -O-, -NH-, -S-, or -Se-, preferably, a group containing -O-, e.g. *-CH₂O-, *-CH₂CH₂O-, * represents the connecting end with N; still further preferably, Z₁₁, Z₂₁ and *-C(X₁)-(CH₂)ₙ₁-(X)ₙ- each independently have a molecular weight of 14.0-107.1, preferably 14.0-60;
preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, - CH₂CH₂CH₂O-, and -S-C(O)-;
for example, in the above-mentioned formula (C) and formula (C-1),
each of Z₁₂, Z₂₂, and Z₃₂ is independently selected from methylene, and -CD₂-; and/or
Z₁₁ is independently selected from -CD₂-, -C₁₋₃alkylene- (for example -C₁₋₂alkylene-), -OC₁₋₃alkylene-* (for example -OC₁₋₂alkylene-*), -SC₁₋₂alkylene-*, -NHC₁₋₂alkylene-*, -Se-C₁₋₂alkylene-*, and -CH₂-O-C₁₋₂alkylene-*, * represents the connecting end with N; and/or
Z₃₁ is independently selected from -C(O)-, -C₁₋₃alkylene-C(O)-*, -OC₁₋₂alkylene-C(O)-*,-NHCH₂-C(O)-*, -SCH₂-C(O)-*, -CH₂-NHC(O)-*, -C₁₋₂alkylene-O-C(O)-*, -NH-C(O)-*, -O-C(O)-*, -S-C(O)-*, and -C₁₋₂alkylene-C(S)-*, * represents the connecting end with N; and/or
n2 is 2 or 3, n3 is 0, X₂ is O, NH, and S; or
n2 is 1 or 2, n3 is 1, X₂ is O, NH, and S; or
n2 is 0 or 1, n3 is 0, X₂ is CH₂;
again for example, in the above-mentioned formula (C) and formula (C-1),
each of Z₁₂, Z₂₂, and Z₃₂ is independently selected from methylene, and -CD₂-; and/or
Z₁₁ is independently selected from -CD₂-, -CH₂-, -CH₂CH₂-, -OCH₂-*, and -OCH₂CH₂-*, * represents the connecting end with N; and/or
Z₃₁ is independently selected from -C(O)-, -CH₂CH₂C(O)-*, -CH₂C(O)-*, -OCH₂C(O)-*,-NHC(O)-*, -OC(O)-*, and -SC(O)-*, * represents the connecting end with N; and/or
n2 is 2 or 3, n3 is 0, X₂ is O; or
n2 is 1 or 2, n3 is 1, X₂ is O; or
n2 is 0 or 1, n3 is 0, X₂ is CH₂;
again for example, in the above-mentioned formula (C) and formula (C-1),
each of Z₁₂, Z₂₂, and Z₃₂ is independently selected from methylene, and -CD₂-; Z₁₁ is independently selected from -CD₂-, -C₁₋₃alkylene- (for example -C₁₋₂alkylene-), -OC₁₋₃alkylene-* (for example - OC₁₋₂alkylene-*), -SC₁₋₂alkylene-*, -NHC₁₋₂alkylene-*, -Se-C₁₋₂alkylene-*, and -CH₂-O-C₁₋₂alkylene-*, and/or
Z₃₁ is independently selected from -C(O)-, -C₁₋₃alkylene-C(O)-*, -OC₁₋₂alkylene-C(O)-*,-NHCH₂-C(O)-*, -SCH₂-C(O)-*, -CH₂-NHC(O)-*, -C₁₋₂alkylene-O-C(O)-*, -NH-C(O)-*, -O-C(O)-*, -S-C(O)-*, and -C₁₋₂alkylene-C(S)-*, * represents the connecting end with N; further for example, Z₃₁ is independently selected from -CH₂CH₂C(O)-*, -CH₂C(O)-*, -OCH₂C(O)-*,-NHC(O)-*, -OC(O)-*, and -SC(O)-*, * represents the connecting end with N; and/or
Z₁₁ is independently selected from -CD₂-, -CH₂-, -CH₂CH₂-, and -OCH₂CH₂-*, * represents the connecting end with N; and/or
X₂ is O, NH, S, or Se, the sum of n2 and n3 is 1, 2, or3; further for example, X₂ is O, the sum of n2 and n3 is 2, or 3;
further for example, in any of the above-mentioned formula (C), formula (C-1), formula (C-2), and formula (C-3), one and only one of Z₁₁ and Z₃₁ is a group containing a double bond; the double bond is, for example, a double bond between C and O in -C(O)-, or a double bond between C and S in -C(S)-; further preferably, the group not containing a double bond of Z₁₁ and Z₃₁ is identical to or different from *-(CH₂)ₙ₂-X₂-(CH₂)ₙ₃- (*represents the connecting end with N); still further preferably, Z₁₁, -(CH₂)ₙ₂-X₂-(CH₂)ₙ₃- and Z₃₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, - OCH₂C(O)-, -CH₂OC(O)-, -CH₂CH₂CH₂O-, and -S-C(O)-;
for example, in the above-mentioned formula (D), and formula (D-1),
each of Z₁₂, Z₂₂, and Z₃₂ is independently selected from methylene, and -CD₂-; and/or
Z₁₁ is independently selected from -CD₂-, -C₁₋₂alkylene-, -OC₁₋₂alkylene-*, -SC₁₋₂alkylene-*, - NHC₁₋₂alkylene-*, -Se-C₁₋₂alkylene-*, and -CH₂-O-C₁₋₂alkylene-*, * represents the connecting end with N; and/or
n2 is 2 or 3, n3 is 0, X₂ is O, NH, or S; or
n2 is 1 or 2, n3 is 1, X₂ is O, NH, or S; or
n2 is 0 or 1, n3 is 0, X₂ is CH₂; and/or
X₁ is O or S, X is selected from O, CH₂, NH, and S, n is 1, n1 is 0; or
X₁ is O or S, X is O or CH₂, n is 1, n1 is 1;
alternatively for example, in the above-mentioned formula (D) and formula (D-1),
each of Z₁₂, Z₂₂, and Z₃₂ is independently selected from methylene, and -CD₂-; and/or
Z₁₁ is independently selected from -CD₂-, -CH₂-, -CH₂CH₂-, -OCH₂-*, and -OCH₂CH₂-*, * represents the connecting end with N; and/or
n2 is 2 or 3, n3 is 0, X₂ is O; or
n2 is 1 or 2, n3 is 1, X₂ is O; or
n2 is 0 or 1, n3 is 0, X₂ is CH₂; and/or
X₁ is O, X is selected from O, CH₂, NH, and S, n is 1, n1 is 0; or
X₁ is O, X is O or CH₂, n is 1, n1 is 1;
alternatively for example, in the above-mentioned formula (D) and formula (D-1),
each of Z₁₂, Z₂₂, and Z₃₂ is independently selected from methylene, and -CD₂-; and/or
Z₁₁ is independently selected from -CD₂-, -CH₂-, -CH₂CH₂-, and -OCH₂CH₂-*, * represents the connecting end with N; and/or
n2 is 2 or 3, n3 is 0, X₂ is O; or
n2 is 2, n3 is 1, X₂ is O; and/or
X₁ is O, X is selected from O, CH₂, NH, and S, n is 1, n1 is 0;
alternatively for example, in the above-mentioned formula (D) and formula (D-1),
each of Z₁₂, Z₂₂, and Z₃₂ is independently selected from methylene, and -CD₂-;
Z₁₁ is independently selected from -CD₂-, -C₁₋₂alkylene-, -OC₁₋₂alkylene-*, -SC₁₋₂alkylene-*, - NHC₁₋₂alkylene-*, -Se-C₁₋₂alkylene-*, and -CH₂-O-C₁₋₂alkylene-*, * represents the connecting end with N; or, Z₁₁ is independently selected from -CD₂-, -CH₂-, -CH₂CH₂-, and -OCH₂CH₂-*, * represents the connecting end with N; and/or
X₂ is O, NH, S, or Se, the sum of n2 and n3 is 1, 2, or 3;or, X₂ is O, the sum of n2 and n3 is 2, or 3; and/or
X₁ is O or S, X is selected from O, CH₂, NH, and S, the sum of n and n1 is 0, 1, or 2;or, X₁ is O,
X is selected from O, CH₂, NH, and S, the sum of n and n1 is 1, or 2; and/or
further for example, in any of the above-mentioned formula (D), formula (D-1), formula (D-2), and formula (D-3), Z₁₁ is a group not containing a double bond; the double bond is, for example, a double bond between C and O in -C(O)-, or a double bond between C and S in -C(S)-; further preferably, Z₁₁ is identical to or different from *-(CH₂)ₙ₂-X₂-(CH₂)ₙ₃- (* represents the connecting end with N); still further preferably, Z₁₁, -C(X₁)-(CH₂)ₙ₁-(X)ₙ₋ and -(CH₂)ₙ₂-X₂-(CH₂)ₙ₃- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, - CH₂C(O)-, -NHC(O)-, -OC(O)-, -CH₂CH₂O-, -OCH₂C(O)-, -CH₂OC(O)-, -CH₂CH₂CH₂O-, and - S-C(O)-;
or
the compound has a structure as represented by formula (E), formula (F), formula (E-1), formula (E-2), formula (E-3), formula (E-4), formula (F-1), or formula (F-2): 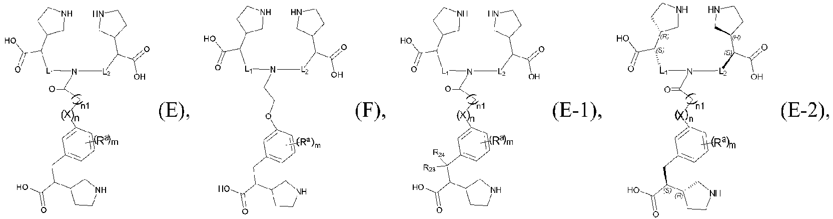 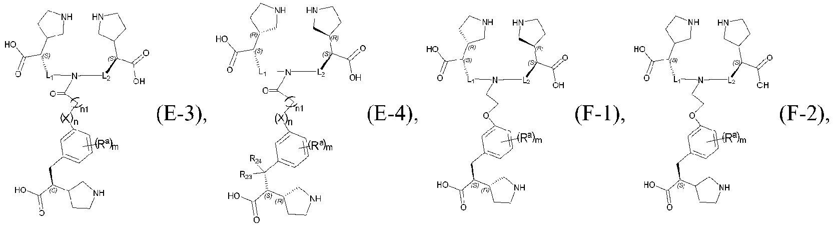
wherein
L₁, L₂ are defined as in any of claims 1-9, X is O, CH₂, or NH, R^{a} is deuterium, halogen, -CN, - OH, -NH₂, -CHO, C₁₋₃alkyl, C₁₋₃alkoxy, -N(C₁₋₃alkyl)₂, -NH(C₁₋₃alkyl), or -C(O)C₁₋₃alkyl, n1 is 0 or 1, m is 0 or 1, n is 0 or 1; R₂₃ and R₂₄ are each independently selected from H, and deuterium;
for example,
L₁ is 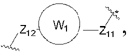 L₂ is 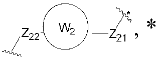 represents the connecting end with N; wherein
each of rings W₁ and W₂ is independently selected from 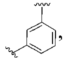 which is optionally substituted by one or more substituents independently selected from halogen, and C₁₋₃alkoxy (preferably F, methoxy);
each of Z₁₁, Z₁₂, Z₂₁, and Z₂₂ is independently selected from -C₁₋₂alkylene-, -OC₁₋₃alkylene-, -NH-C₁₋₂alkylene-, -S-C₁₋₂alkylene-, -C₁₋₂alkylene-O-C₁₋₂alkylene-, -C₁₋₂alkylene-C(O)-, -OC₁₋₂alkylene-C(O)-, -C₁₋₂alkylene-NH-C(O)-, -C₁₋₂alkylene-O-C(O)-, -NH-C(O)-, -NH-C₁₋₂alkylene-C(O)-, -O-C(O)-, and -S-C(O)-; or
each of Z₁₁, Z₁₂, Z₂₁, and Z₂₂ is independently selected from methylene, ethylene, 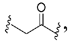 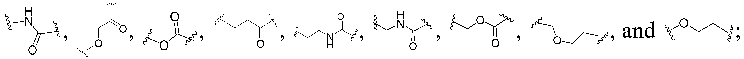
or, for example
each of L1 and L2 is independently selected from 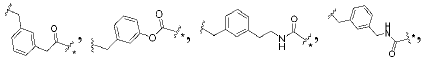 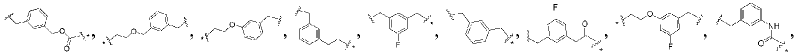 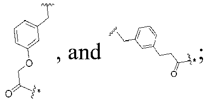 * represents the connecting end withN;
X is selected from O, CH₂, NH, and S, for example X is O, CH₂, or NH, e.g. X is CH₂; and/or
R^{a} is deuterium, halogen, -CN, -OH, -NH₂, -CHO, C₁₋₃alkyl, C₁₋₃alkoxy, -N(C₁₋₃alkyl)₂, -NH(C₁₋₃alkyl), or -C(O)C₁₋₃alkyl, for example, R^{a} is F, Cl, methyl, ethyl, methoxy, or ethoxy; for example, R^{a} is F; and/or
n1 is 0 or 1, and/or
m is 0 or 1, for example m is 1, and/or
n is 0 or 1; for example, the sum of n and n1 is 0, 1, or 2; for example, the sum of n and n1 is 1 or 2 (i.e. n is 1, n1 is 0; or n is 0, n1 is 1; or n is 1, n1 is 1; or n is 0, n1 is 2; or n is 2, n1 is 0); for example, the sum of n and n1 is 1(i.e. n is 1, n1 is 0; or n is 0, n1 is 1);
preferably,
X is selected from O, CH₂, NH, and S, n is 1, and n1 is 0; or
X is O or CH₂, n is 1, n1 is 1;
alternatively preferably,
R^{a} is F, Cl, methyl, ethyl, methoxy, or ethoxy,
m is 1,
X is selected from O, CH₂, NH, and S,
the sum of n and n1 is 0, 1, or 2;
alternatively preferably,
R^{a} is F or methoxy,
m is 1,
X is selected from O, CH₂, NH, and S,
the sum of n and n1 is 1 or 2;
for example, in any of the above-mentioned formula (E), formula (E-1), formula (E-2), formula (E-3) and formula (E-4), L₁ and L₂ are groups containing Z₁₁ and Z₂₁ respectively, and each of Z₁₁ and Z₂₁ is a group not containing a double bond; the double bond is, for example, a double bond between C and O in -C(O)-, or a double bond between C and S in -C(S)-; further preferably, Z₁₁ and Z₂₁ are identical or different; still further preferably, Z₁₁, Z₂₁ and -C(O)-(CH₂)ₙ₁-(X)ₙ- each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, - CH₂C(O)-, -NHC(O)-, -OC(O)-, -CH₂CH₂O-, -OCH₂C(O)-, -CH₂OC(O)-, -CH₂CH₂CH₂O-, and - S-C(O)-; or
in any of the above-mentioned formula (F), formula (F-1) and formula (F-2), L₁ and L₂ are groups containing Z₁₁ and Z₂₁ respectively, one and only one of Z₁₁ and Z₂₁ is a group containing a double bond; the double bond is, for example, a double bond between C and O in -C(O)-, or a double bond between C and S in -C(S)-; further preferably, the group not containing a double bond of Z₁₁ and Z₂₁ is identical to or different from *-(CH₂)₂-O- (*represents the connecting end with N); still further preferably, Z₁₁ and Z₂₁ each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, -NHC(O)-, -OC(O)-, -CH₂CH₂O-, -OCH₂C(O)-, -CH₂OC(O)-, -CH₂CH₂CH₂O-, and -S-C(O)-;
or, the compound has a structure as represented by formula (G), formula (G-1), formula (G-2), formula (G-3), or formula (G-4): 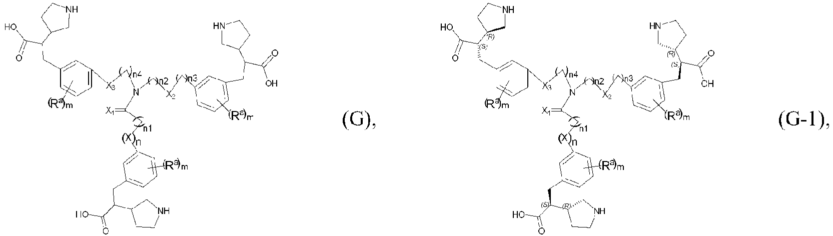 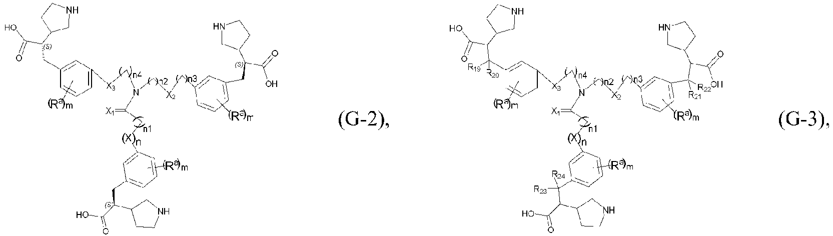 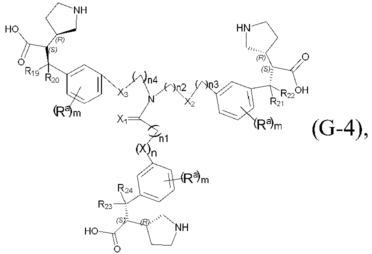
wherein
X, X₁, X₂, n, n1, n2, and n3 are defined as in any of formula (B), formula (C) and formula (D), m is independently 0 or 1, for example m is 1;
n4 is 0, 1, 2 or 3, or , n4 is 0, 1 or 2, or n4 is 0 or 1, or n4 is 0;
X₃ is O, S, CH₂, or NH; or X₃ is O, S, CH₂, NH, or CD₂; or X₃ is O or CH₂; or X₃ is O, CH₂, or CD₂;
R^{a} is deuterium, halogen, -CN, -OH, -NH₂, -CHO, C₁₋₃alkyl, C₁₋₃alkoxy, -N(C₁₋₃alkyl)₂, -NH(C₁₋₃alkyl), or -C(O)C₁₋₃alkyl; for example, R^{a} is F, Cl, methyl, ethyl, methoxy, or ethoxy; for example, R^{a} is F, or methoxy;
R₁₉, R₂₀, R₂₁, R₂₂, R₂₃, and R₂₄ are each independently selected from H and deuterium; preferably,
X₃ is O, and n4 is 2; or
X₃ is CH₂, and n4 is 0 or 1; or
X₃ is CH₂ or CD₂, and n4 is 0 or 1; or
X₃ is CH₂ or CD₂, and n4 is 0; or
alternatively preferably
R^{a} is F, Cl, methyl, ethyl, methoxy, or ethoxy;
m is 1;
X₂ is O, NH, S, or Se,
the sum of n2 and n3 is 1, 2, or 3;
X₁ is O or S,
X is selected from O, CH₂, NH, and S,
the sum of n and n1 is 0, 1, or 2; and
X₃ is O, and n4 is 2; or
X₃ is CH₂ or CD₂, and n4 is 0 or 1;
alternatively preferably
R^{a} is F, Cl, or methoxy;
m is 1;
X₂ is O,
the sum of n2 and n3 is 2 or 3;
X₁ is O,
X is selected from O, CH₂, NH, and S,
the sum of n and n1 is 1, or 2; and
X₃ is O, and n4 is 2; or
X₃ is CH₂, or CD₂, and n4 is 0 or 1;
alternatively preferably
R^{a} is F, Cl, methyl, ethyl, methoxy, or ethoxy;
m is 1;
X₃ is O, and n4 is 2; or
X₃ is CH₂ or CD₂, and n4 is 0 or 1;
n2 is 2 or 3, and n3 is 0, and X₂ is O, NH, or S; or
n2 is 1 or 2, and n3 is 1, and X₂ is O, NH, or S; or
n2 is 0 or 1, and n3 is 0, and X₂ is CH₂; and
X₁ is O or S, X is selected from O, CH₂, NH, and S, n is 1, and n1 is 0; or
X₁ is O or S, X is O or CH₂, n is 1, and n1 is 1;
alternatively preferably
R^{a} is F, Cl, or methoxy;
m is 1;
X₃ is O, and n4 is 2; or
X₃ is CH₂ or CD₂, and n4 is 0 or 1;
n2 is 2 or 3, n3 is 0, and X₂ is O; or
n2 is 1 or 2, n3 is 1, and X₂ is O; or
n2 is 0 or 1, n3 is 0, and X₂ is CH₂; and
X₁ is O, X is selected from O, CH₂, NH, and S, n is 1, and n1 is 0; or
X₁ is O, X is O or CH₂, n is 1, and n1 is 1;
in any of the above-mentioned formula (G), formula (G-1), formula (G-2), formula (G-3), and
formula (G-4), *-(CH₂)ₙ₄-X₃-, *-(CH₂)ₙ₂-X₂-(CH₂)ₙ₃-, and *-C(X₁)-(CH₂)ₙ₁-(X)ₙ- (* represents the connecting end with N) are identical to or different from each other, and they each independently have a molecular weight of 14.0-107.1, preferably 14.0-60; preferably about 14, about 16, about 28, about 30, about 42, about 43, about 44, about 46, about 58, or about 60; still more preferably, Z₁₁, Z₂₁ and Z₃₁ are selected from -CH₂-, -CD₂-, -CH₂CH₂-, -CD₂CH₂-, -C(O)-, -CH₂C(O)-, - NHC(O)-, -OC(O)-, -CH₂CH₂O-, -OCH₂C(O)-, -CH₂OC(O)-, -CH₂CH₂CH₂O-, and -S-C(O)-;
or, the compound has a structure as represented by formula (H), formula (H-1), formula (H-2), formula (H-3), or formula (H-4): 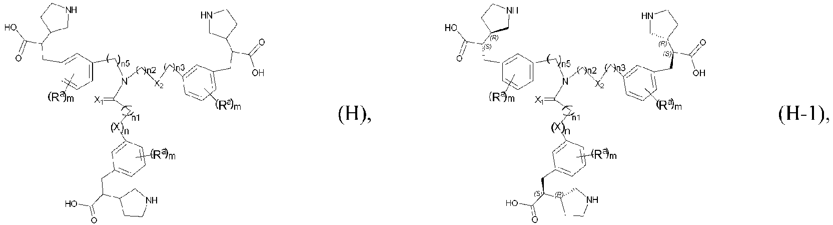 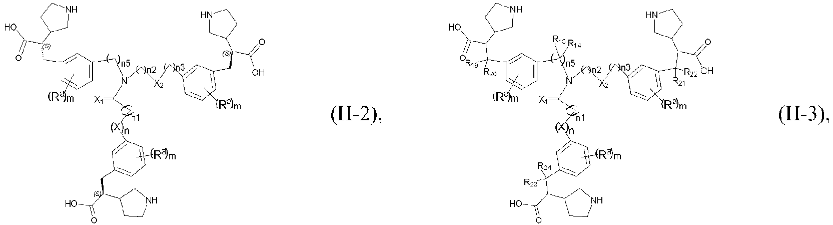 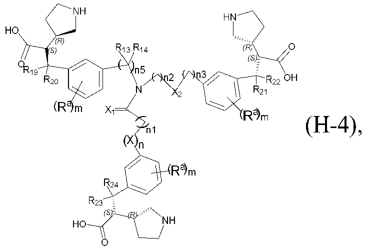
wherein
X, X₁, X₂, n, n1, n2, and n3 are defined as in formula (B), formula (C), formula (D) or formula (G),
m is independently 0 or 1, or m is 1;
n5 is 1, 2, 3 or 4, or n5 is 1, 2, or 3, or n5 is 1 or 2, or n5 is 1;
R^{a} is deuterium, halogen, -CN, -OH, -NH₂, -CHO, C₁₋₃alkyl, C₁₋₃alkoxy, -N(C₁₋₃alkyl)₂, -NH(C₁₋₃alkyl), or -C(O)C₁₋₃alkyl; or R^{a} is F, Cl, methyl, ethyl, methoxy, or ethoxy; or R^{a} is F, or methoxy; each of R₁₃, R₁₄, R₁₉, R₂₀, R₂₁, R₂₂, R₂₃, and R₂₄ is independently selected from H and deuterium.

11. A compound, or a pharmaceutically acceptable salt thereof, wherein the compound is selected from:
| Compoun d No. | Structure | Compoun d No. | Structure |
|---|---|---|---|
| 1-a | 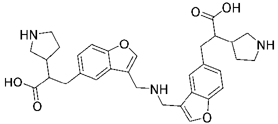 | 2-a | 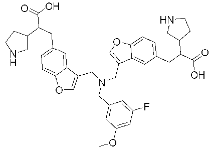 |
| 3-a | 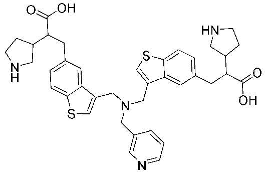 | 4-a | 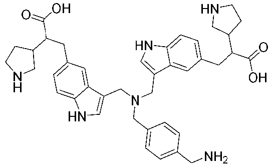 |
| 5-a | 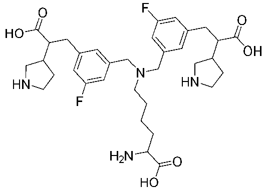 | 6-a | 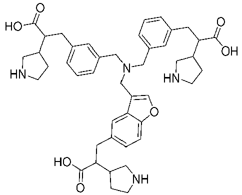 |
| 7-a | 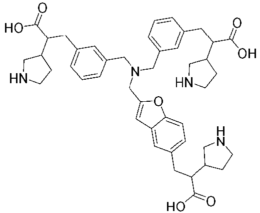 | 8-a | 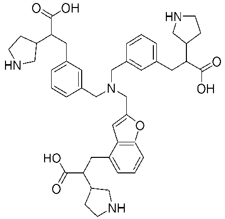 |
| 9-a | 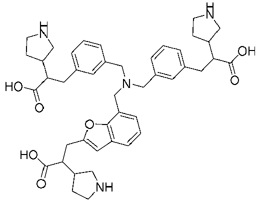 | 10-a | 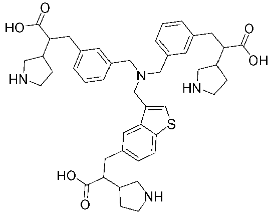 |
| 11-a | 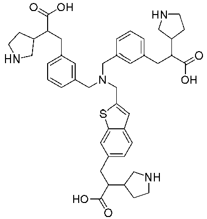 | 12-a | 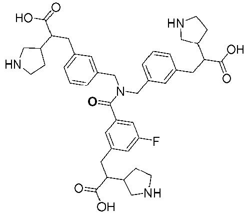 |
| 13-a | 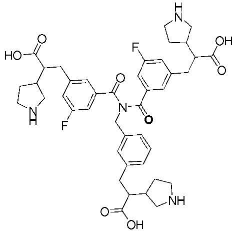 | 14-a | 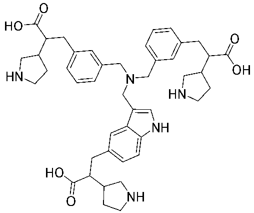 |
| 15-a | 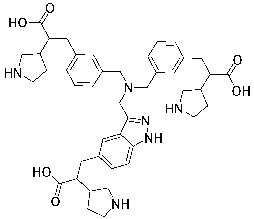 | 16-a | 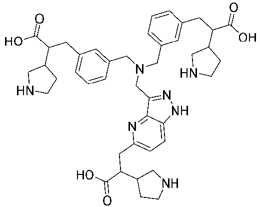 |
| 17-a | 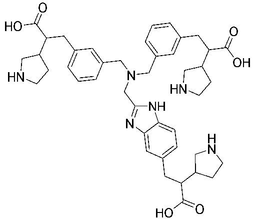 | 18-a | 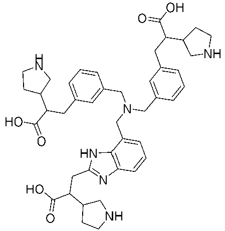 |
| 19-a | 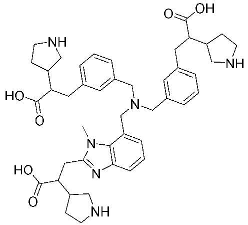 | 20-a | 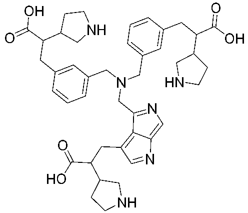 |
| 21-a | 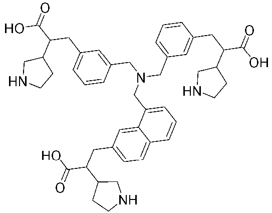 | 22-a | 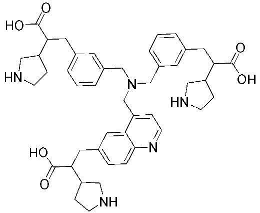 |
| 23-a | 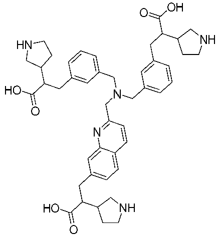 | 24-a | 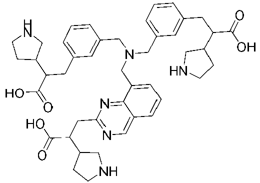 |
| 25-a | 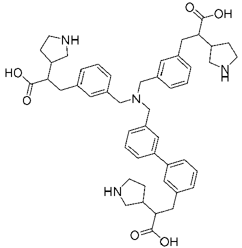 | 26-a | 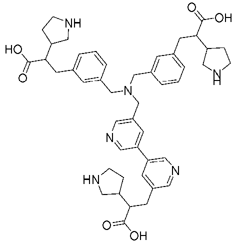 |
| 27-a | 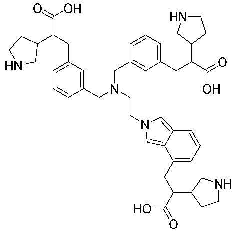 | 28-a | 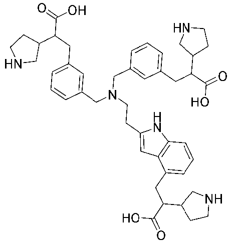 |
| 29-a | 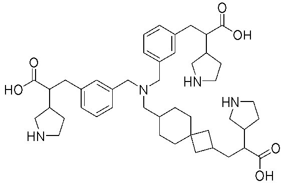 | 30-a | 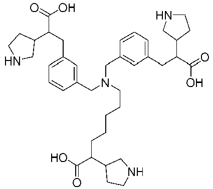 |
| 31-a | 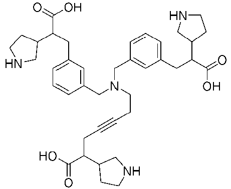 | 32-a | 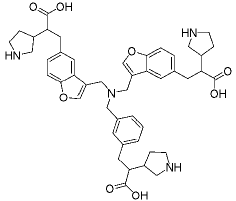 |
| 33-a | 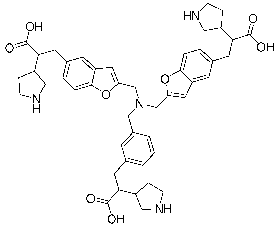 | 34-a | 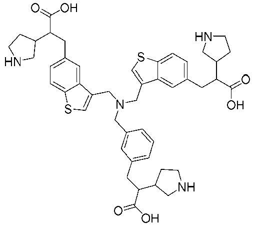 |
| 35-a | 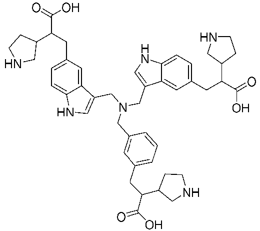 | 36-a | 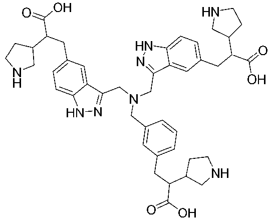 |
| 37-a | 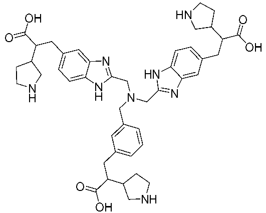 | 38-a | 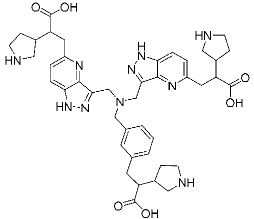 |
| 39-a | 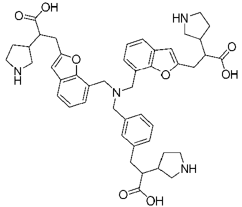 | 40-a | 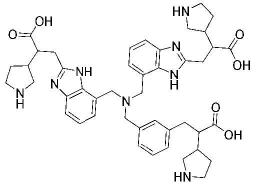 |
| 41-a | 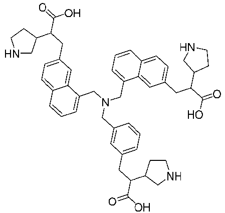 | 42-a | 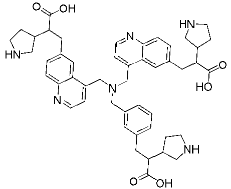 |
| 43-a | 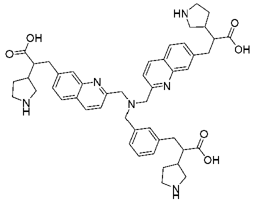 | 44-a | 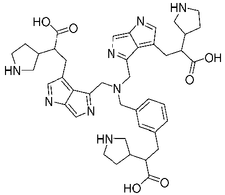 |
| 45-a | 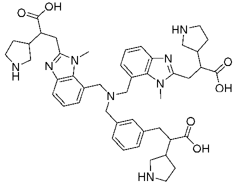 | 46-a | 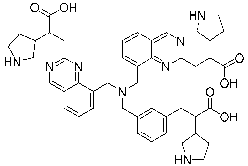 |
| 47-a | 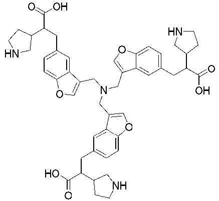 | 48-a | 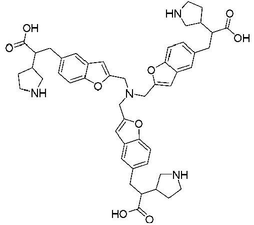 |
| 49-a | 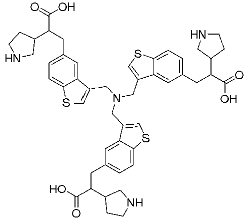 | 50-a | 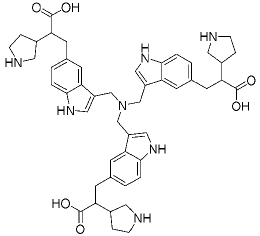 |
| 51-a | 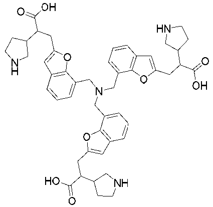 | 52-a | 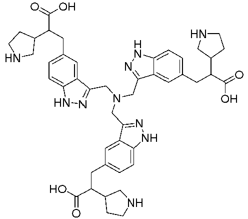 |
| 53-a | 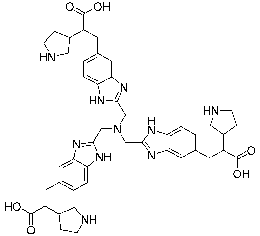 | 54-a | 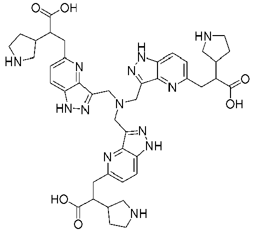 |
| 55-a | 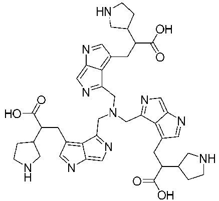 | 56-a | 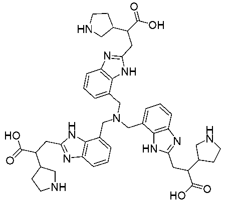 |
| 57-a | 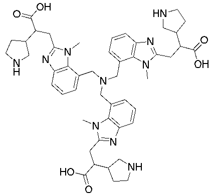 | 58-a | 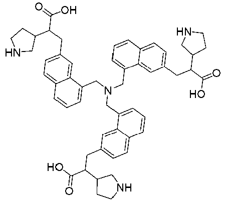 |
| 59-a | 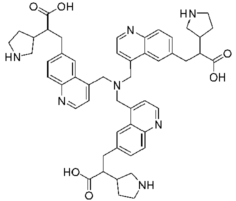 | 60-a | 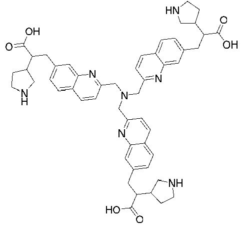 |
| 61-a | 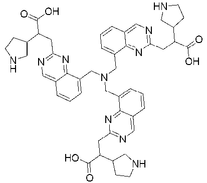 | 62-a | 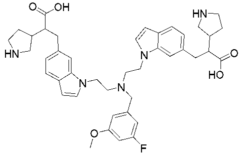 |
| 63-a | 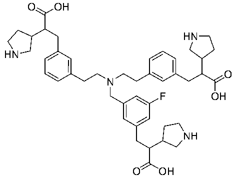 | 64-a | 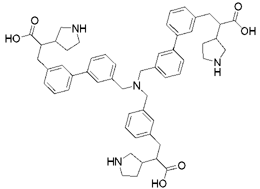 |
| 65-a | 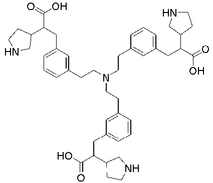 | 66-a | 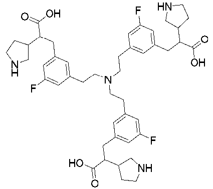 |
| 67-a | 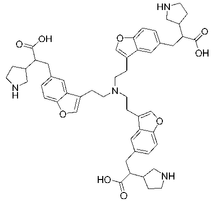 | 68-a | 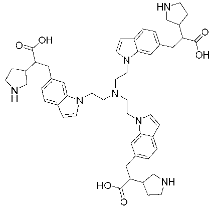 |
| 69-a | 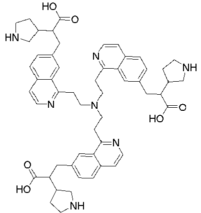 | 70-a | 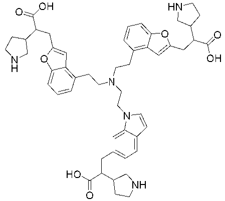 |
| 71-a | 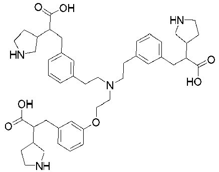 | 72-a | 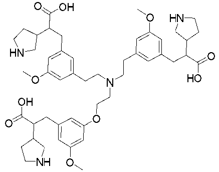 |
| 73-a | 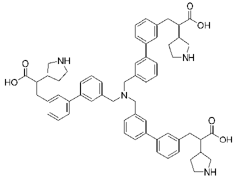 | 74-a | 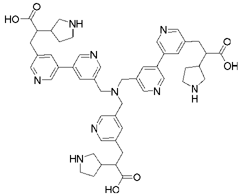 |
| 75-a | 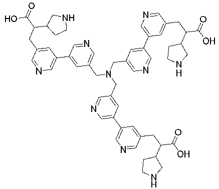 | 76-a | 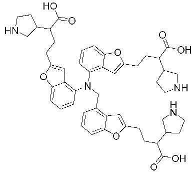 |
| 77-a | 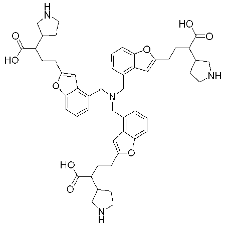 | 78-a | 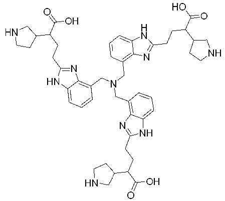 |
| 79-a | 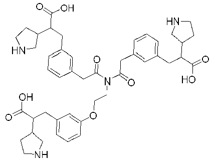 | 80-a | 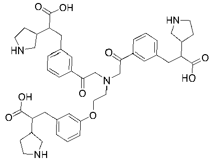 |
| 81-a | 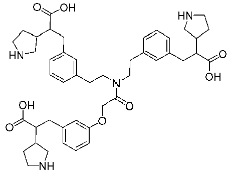 | 82-a | 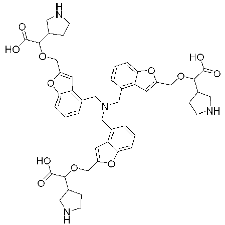 |
| 85-a | 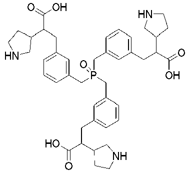 | 86-a | 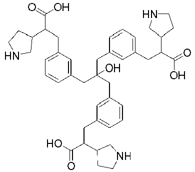 |
| 87-a | 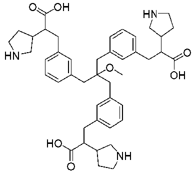 | 88-a | 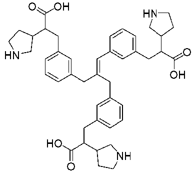 |
| 89-a | 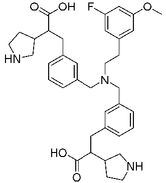 | 90-a | 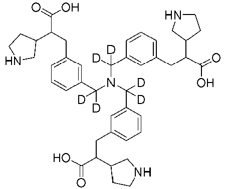 |
| 91-a | 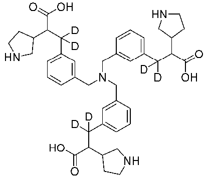 | 92-a | 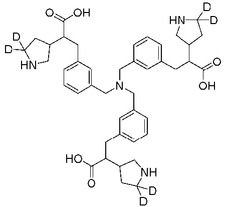 |
| 93-a | 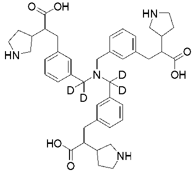 | 94-a | 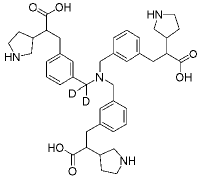 |
| 95-a | 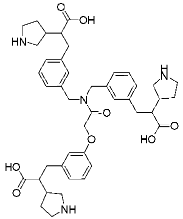 | 96-a | 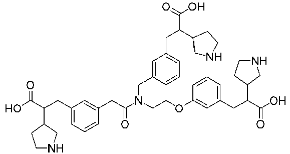 |
| 97-a | 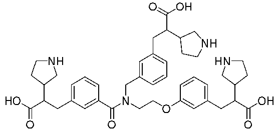 | 98-a | 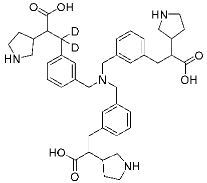 |
| 99-a | 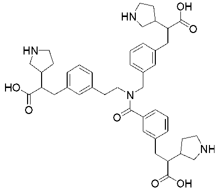 | 100-a | 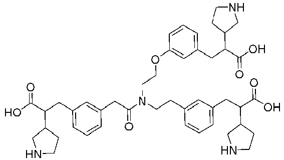 |
| 101-a | 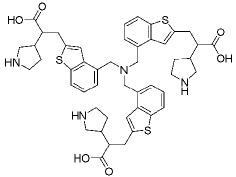 | 102-a | 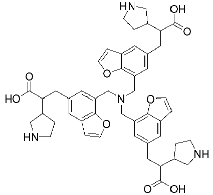 |
| 103-a | 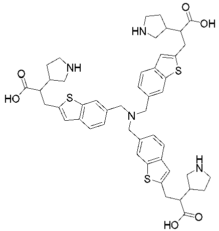 | 104-a | 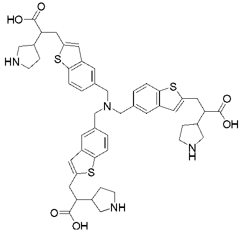 |
| 105-a | 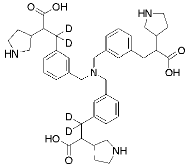 | 106-a | 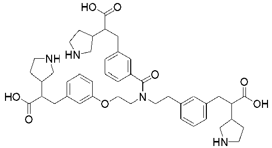 |
| 107-a | 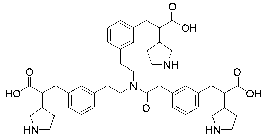 | 108-a | 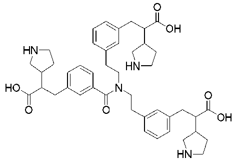 |
| 109-a | 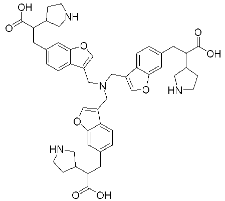 | 110-a | 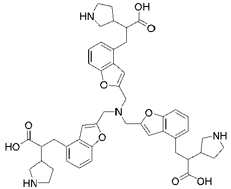 |
| 111-a | 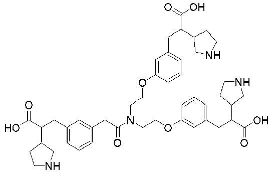 | 112-a | 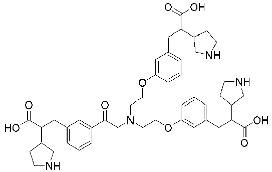 |
| 113-a | 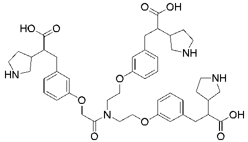 | 114-a | 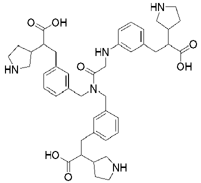 |
| 115-a | 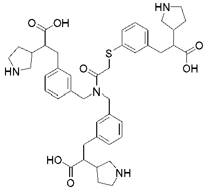 | 116-a | 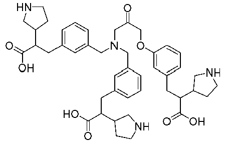 |
| 117-a | 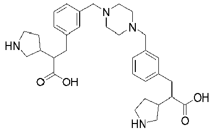 | 118-a | 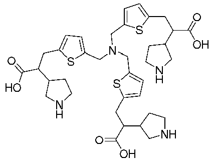 |
| 119-a | 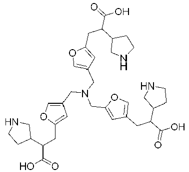 | 120-a | 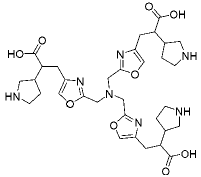 |
| 121-a | 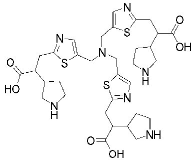 | 122-a | 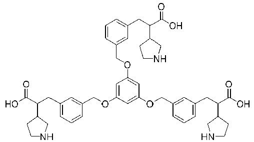 |
| 123-a | 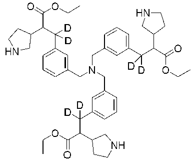 | 124-a | 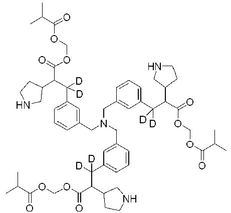 |
| 126-a | 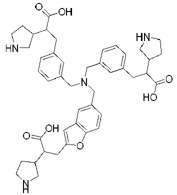 | 127-a | 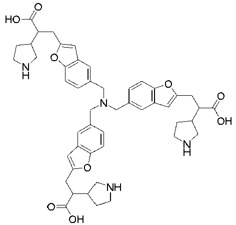 |
| 128-a | 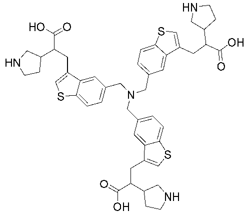 | 129-a | 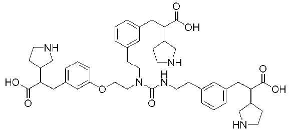 |
| 130-a | 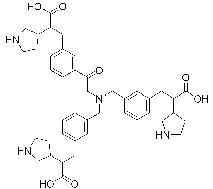 | 131-a | 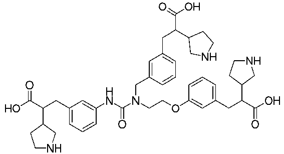 |
| 132-a | 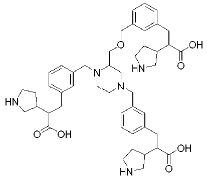 | 133-a | 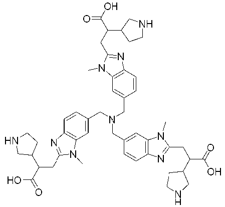 |
| 134-a | 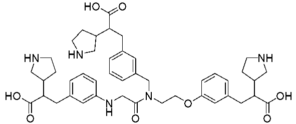 | 135-a | 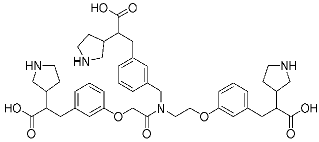 |
| 136-a | 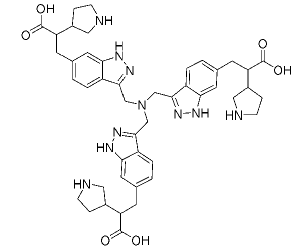 | 137-a | 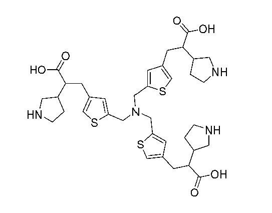 |
| 138-a | 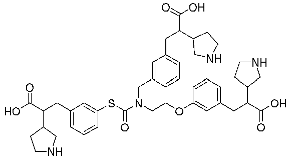 | 139-a | 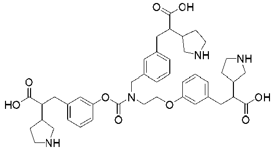 |
| 140-a | 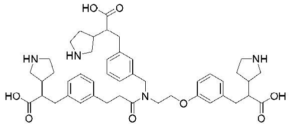 | 141-a | 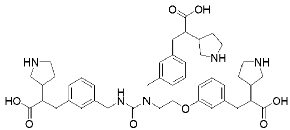 |
| 142-a | 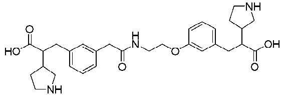 | 143-a | 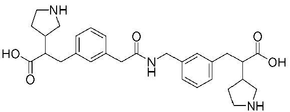 |
| 144-a | 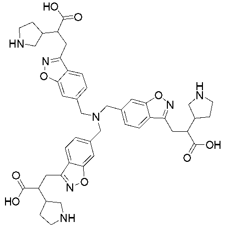 | 145 | 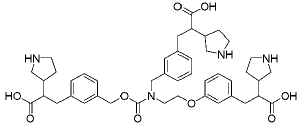 |
| 146-a | 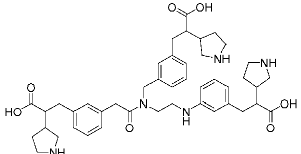 | 147-a | 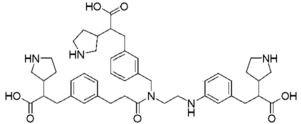 |
| 148-a | 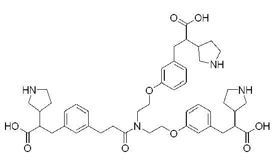 | 149-a | 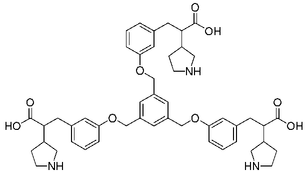 |
| 150-a | 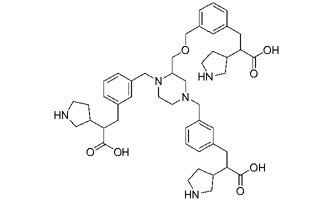 | 152-a | 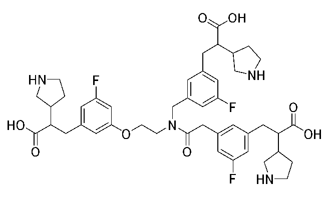 |
| 153-a | 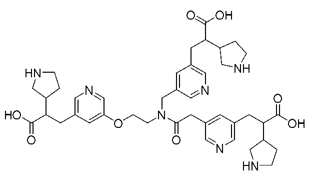 | 154-a | 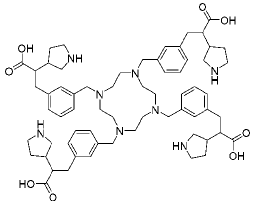 |
| 155-a | 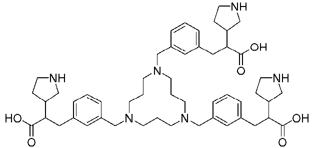 | 156-a | 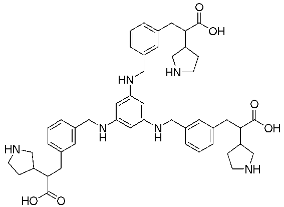 |
| 157-a | 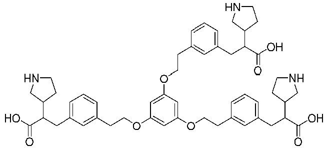 | 158-a | 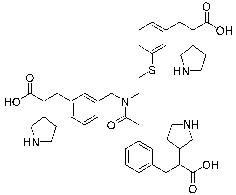 |
| 159-a | 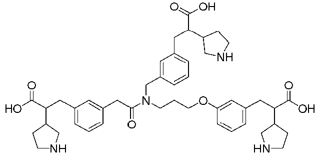 | 160-a | 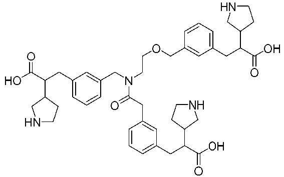 |
| 161-a | 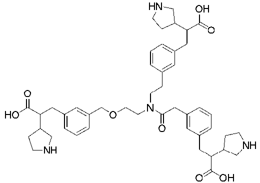 | 162-a | 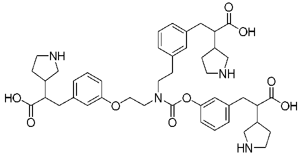 |
| 163-a | 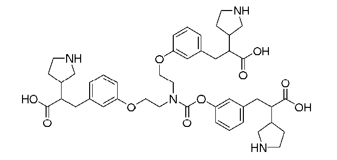 | 164-a | 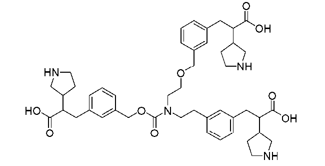 |
| 165-a | 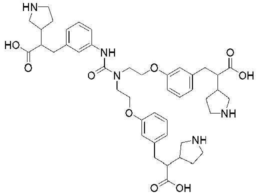 | 166-a | 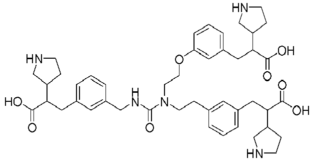 |
| 167-a | 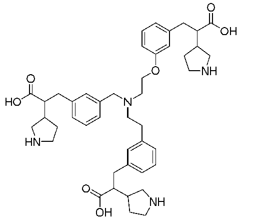 | 168-a | 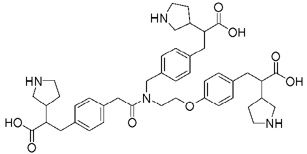 |
| 169-a | 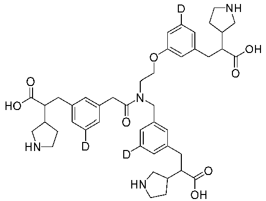 | 170-a | 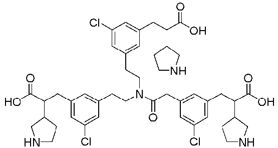 |
| 171-a | 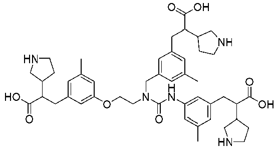 | 172-a | 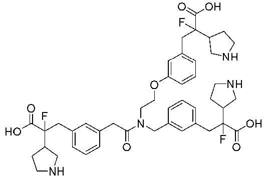 |
| 173-a | 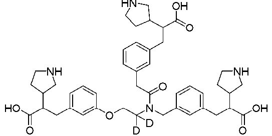 | 174-a | 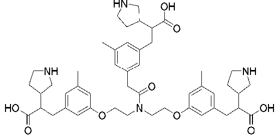 |
| 175-a | 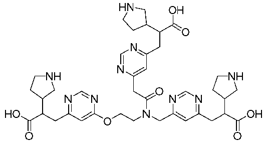 | 176-a | 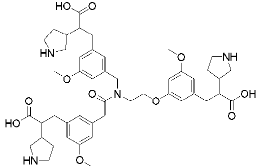 |
| 177-a | 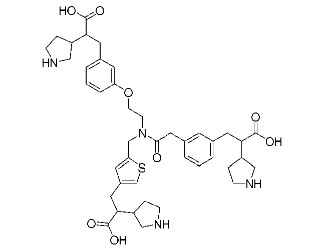 | 178-a | 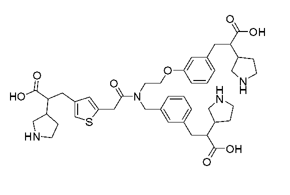 |
| 179-a | 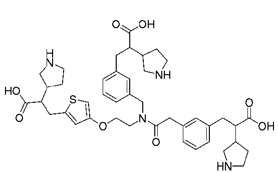 | 180-a | 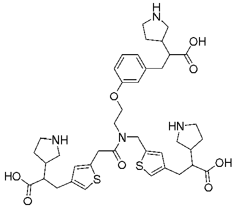 |
| 181-a | 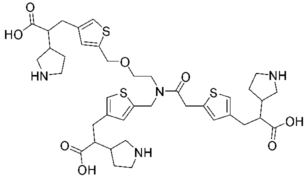 | 182-a | 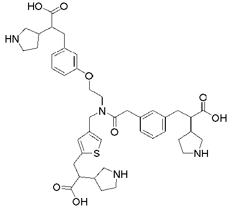 |
| 183-a | 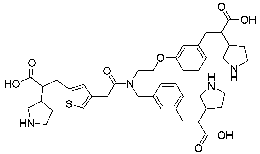 | 184-a | 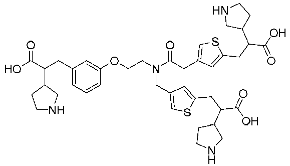 |
| 185-a | 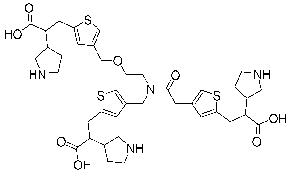 | 186-a | 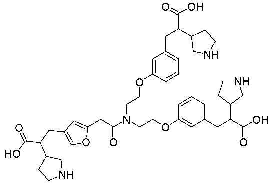 |
| 187-a | 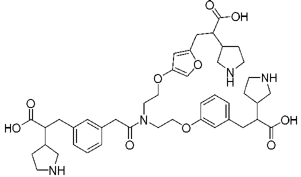 | 188-a | 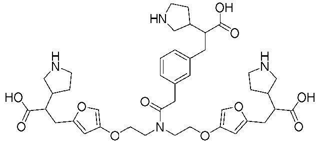 |
| 189-a | 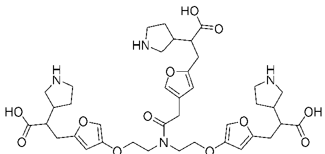 | 190-a | 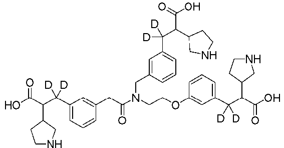 |
| 191-a | 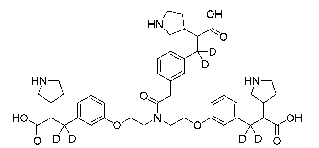 | 192-a | 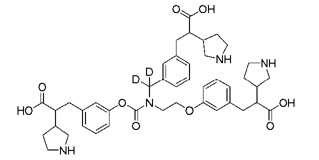 |
| 193-a | 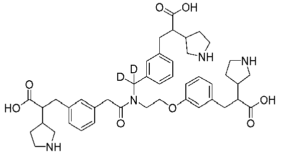 | 194-a | 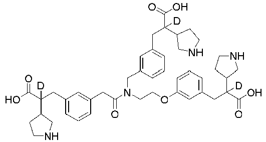 |
| 195-a | 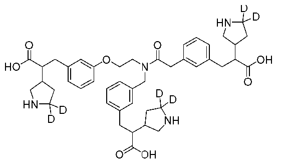 | 196-a | 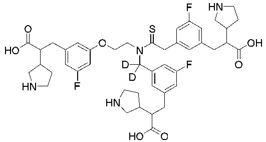 |
| 197-a | 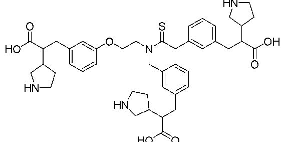 | 198-a | 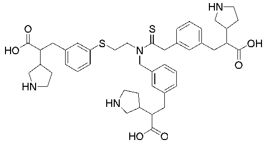 |
| 199-a | 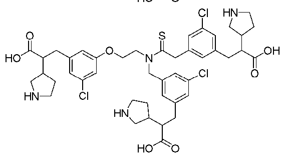 | 200-a | 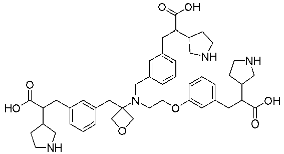 |
| 201-a | 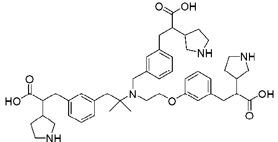 | 202-a | 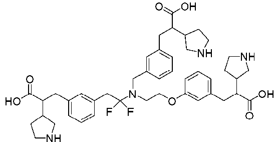 |
| 203-a | 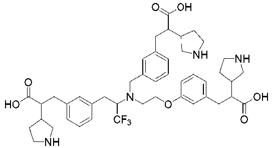 | 204-a | 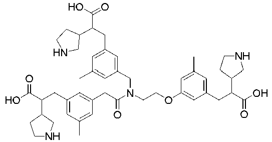 |
| 205-a | 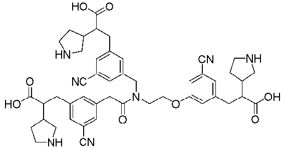 | 206-a | 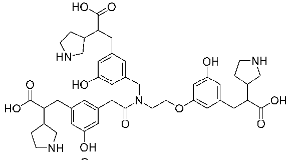 |
| 207-a | 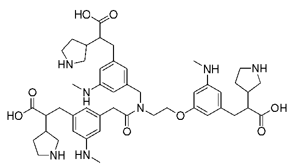 | 208-a | 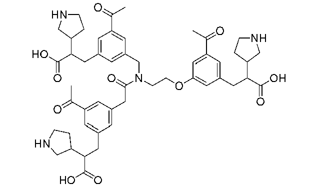 |
| 212-a | 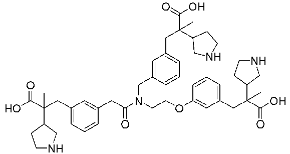 | 213-a | 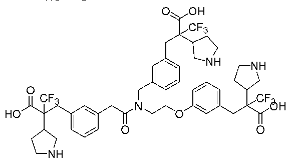 |
| 214-a | 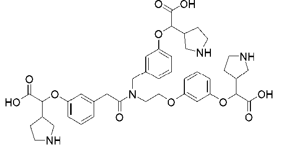 | 215-a | 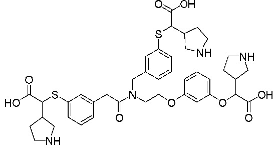 |
| 216-a | 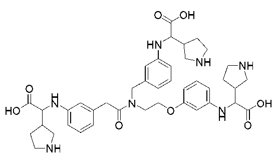 | 217-a | 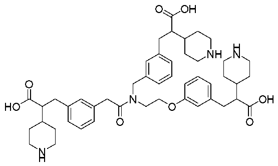 |
| 218-a | 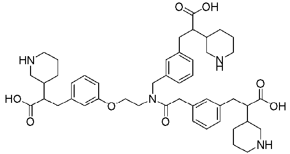 | 219-a | 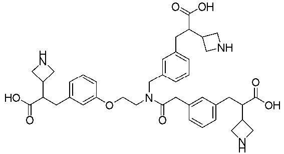 |
| 220-a | 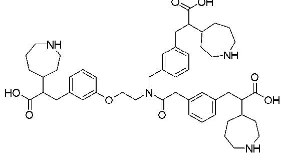 | 221-a | 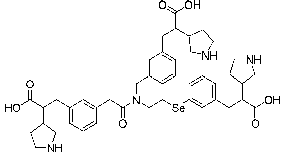 |
| 222-a | 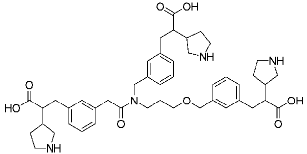 | 223-a | 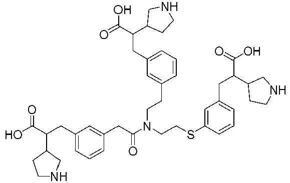 |
| 224-a | 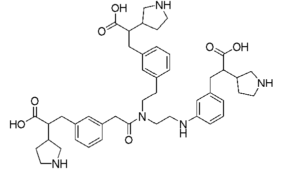 | 225-a | 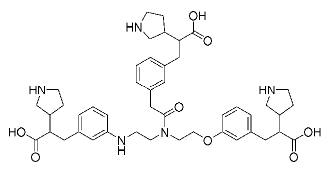 |
| 226-a | 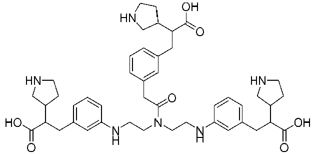 | 227-a | 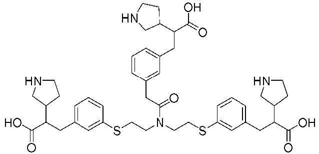 |
| 228-a | 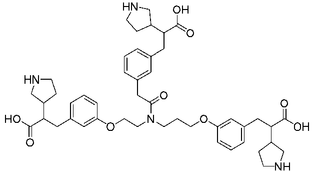 | 96-b | 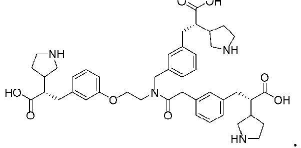 |

12. A compound, or a pharmaceutically acceptable salt thereof, wherein the compound is selected from:
| Compound No. | Structure | Compound No. | Structure |
|---|---|---|---|
| 1 | 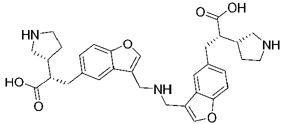 | 2 | 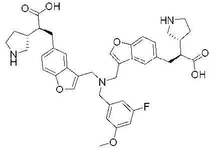 |
| 3 | 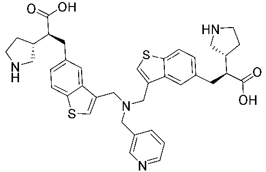 | 4 | 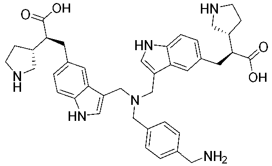 |
| 5 | 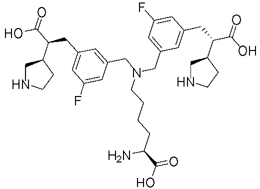 | 6 | 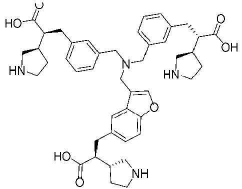 |
| 7 | 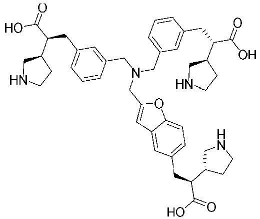 | 8 | 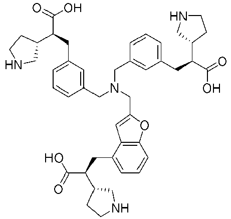 |
| 9 | 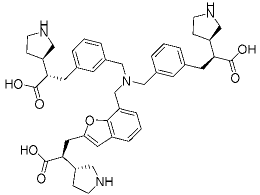 | 10 | 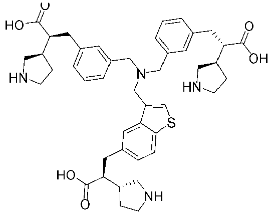 |
| 11 | 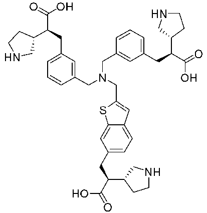 | 12 | 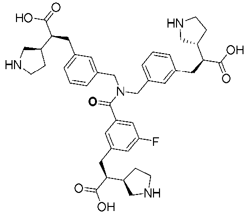 |
| 13 | 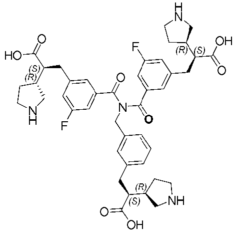 | 14 | 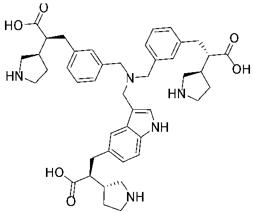 |
| 15 | 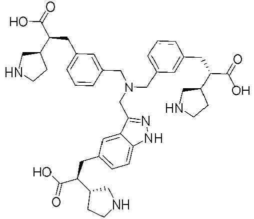 | 16 | 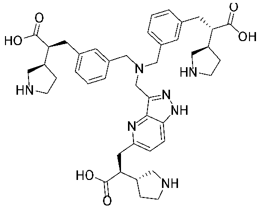 |
| 17 | 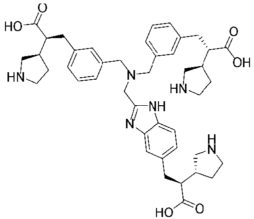 | 18 | 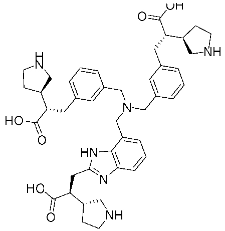 |
| 19 | 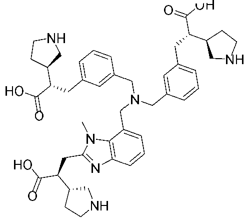 | 20 | 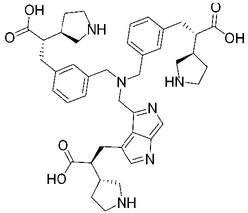 |
| 21 | 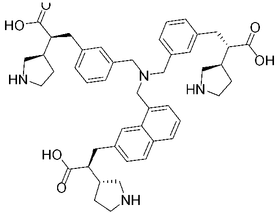 | 22 | 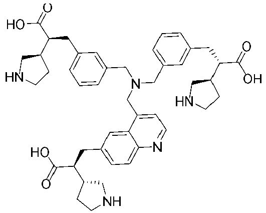 |
| 23 | 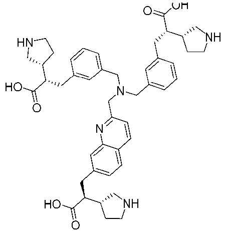 | 24 | 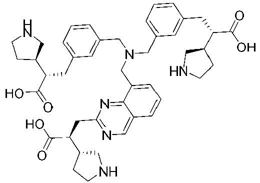 |
| 25 | 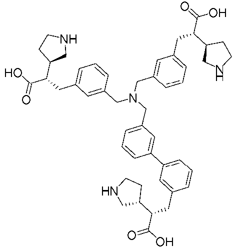 | 26 | 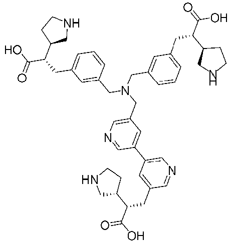 |
| 27 | 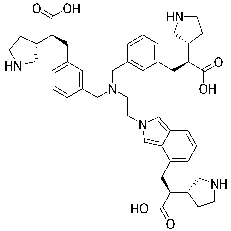 | 28 | 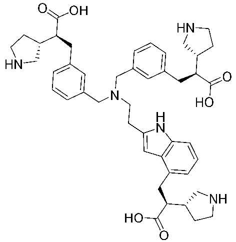 |
| 29 | 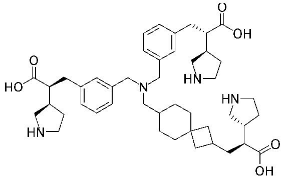 | 30 | 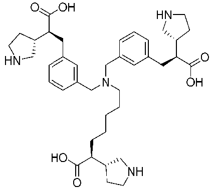 |
| 31 | 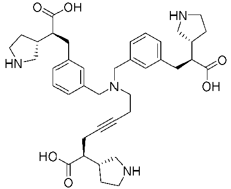 | 32 | 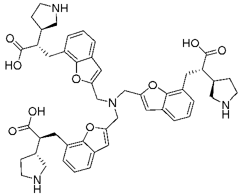 |
| 33 | 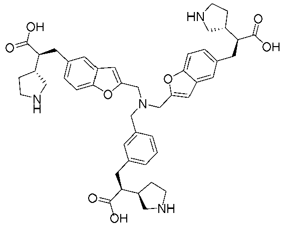 | 34 | 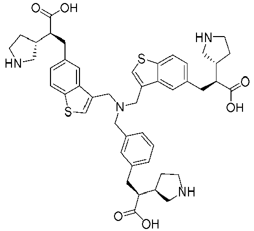 |
| 35 | 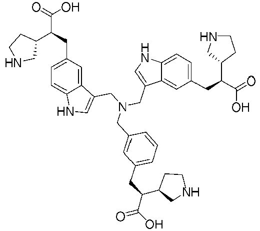 | 36 | 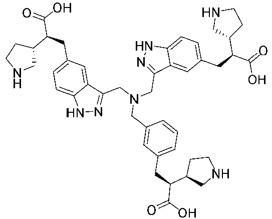 |
| 37 | 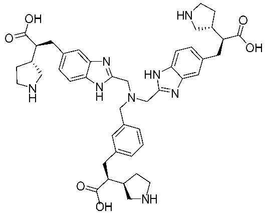 | 38 | 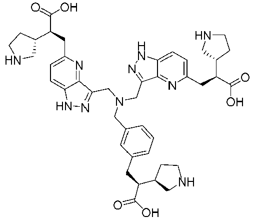 |
| 39 | 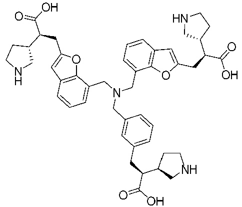 | 40 | 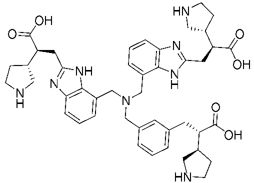 |
| 41 | 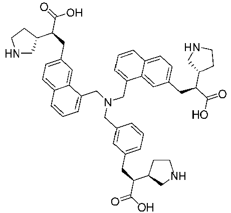 | 42 | 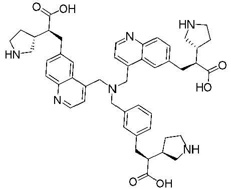 |
| 43 | 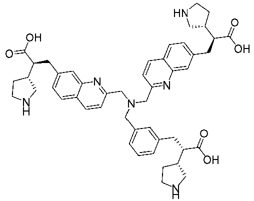 | 44 | 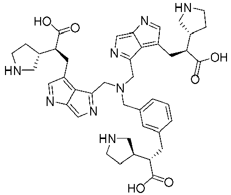 |
| 45 | 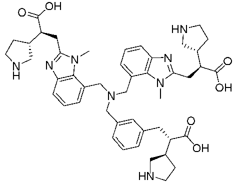 | 46 | 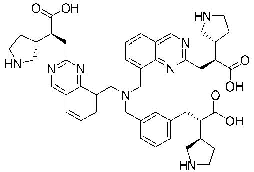 |
| 47 | 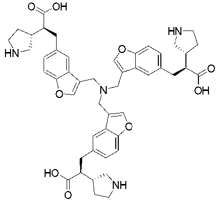 | 48 | 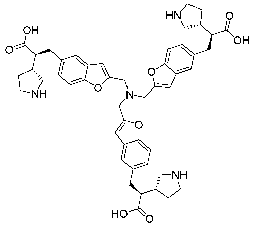 |
| 49 | 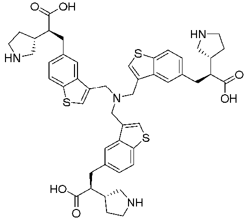 | 50 | 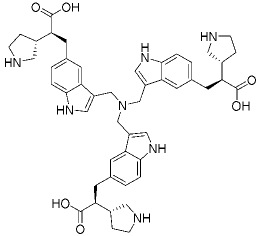 |
| 51 | 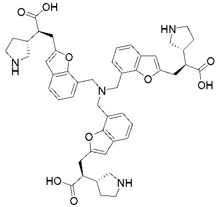 | 52 | 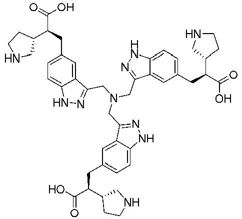 |
| 53 | 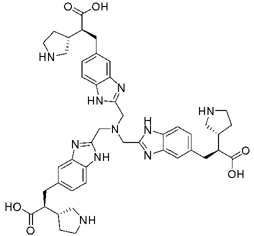 | 54 | 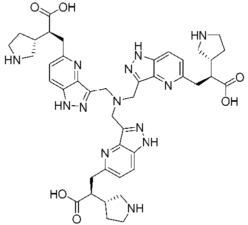 |
| 55 | 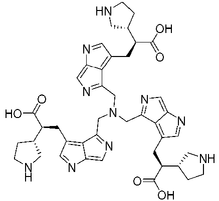 | 56 | 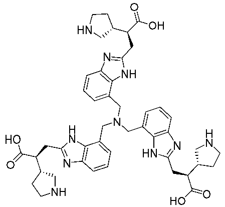 |
| 57 | 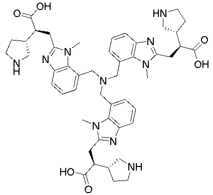 | 58 | 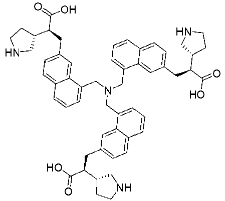 |
| 59 | 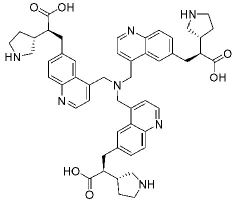 | 60 | 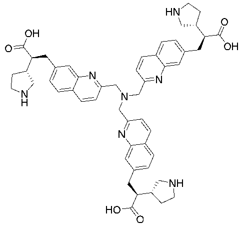 |
| 61 | 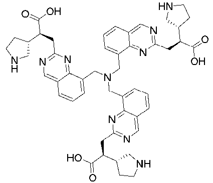 | 62 | 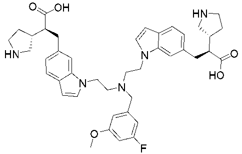 |
| 63 | 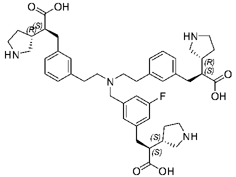 | 64 | 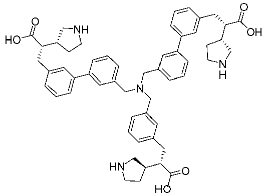 |
| 65 | 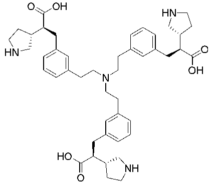 | 66 | 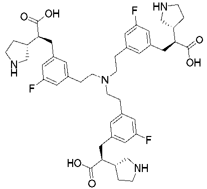 |
| 67 | 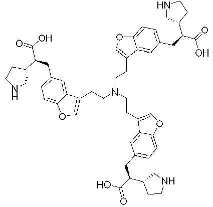 | 68 | 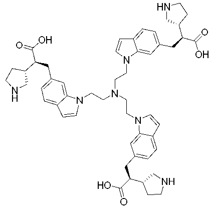 |
| 69 | 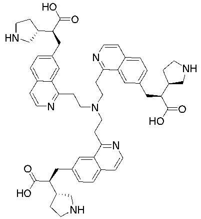 | 70 | 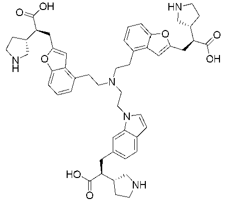 |
| 71 | 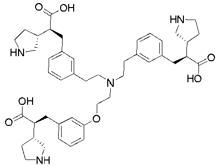 | 72 | 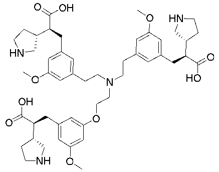 |
| 73 | 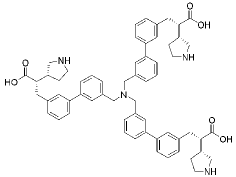 | 74 | 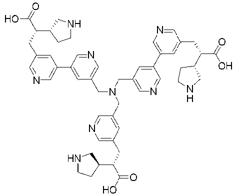 |
| 75 | 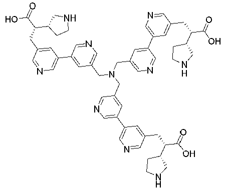 | 76 | 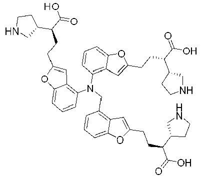 |
| 77 | 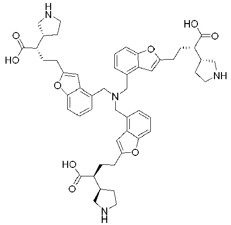 | 78 | 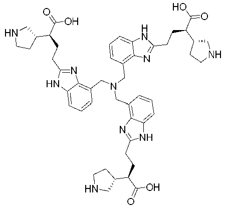 |
| 79 | 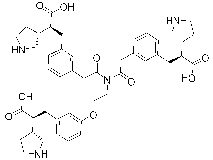 | 80 | 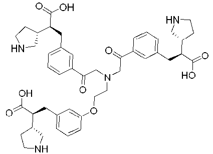 |
| 81 | 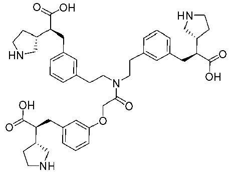 | 82 | 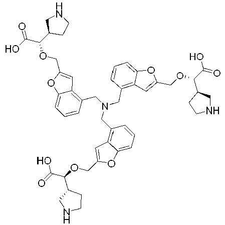 |
| 83 | 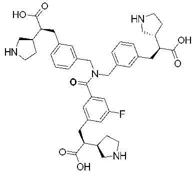 | 84 | 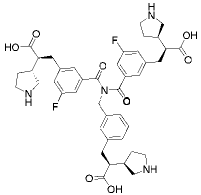 |
| 85 | 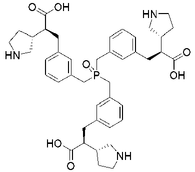 | 86 | 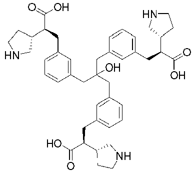 |
| 87 | 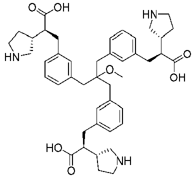 | 88 | 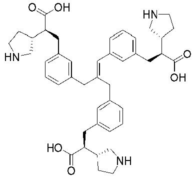 |
| 89 | 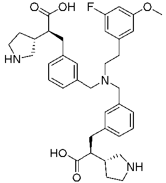 | 90 | 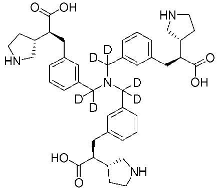 |
| 91 | 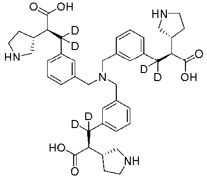 | 92 | 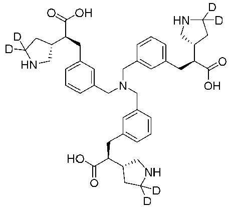 |
| 93 | 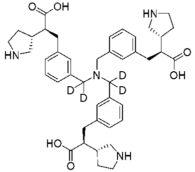 | 94 | 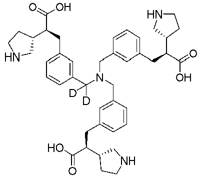 |
| 95 | 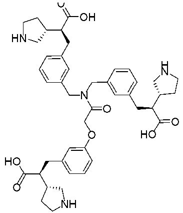 | 96 | 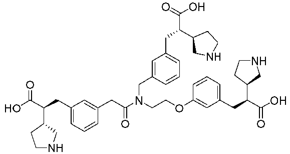 |
| 97 | 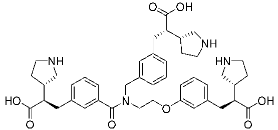 | 98 | 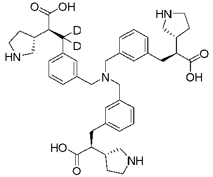 |
| 99 | 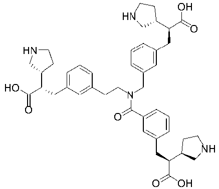 | 100 | 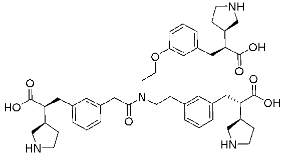 |
| 101 | 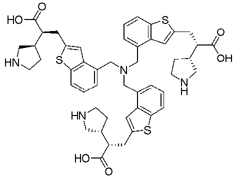 | 102 | 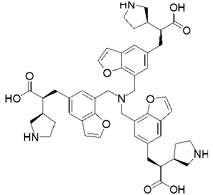 |
| 103 | 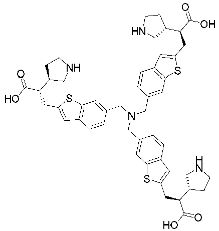 | 104 | 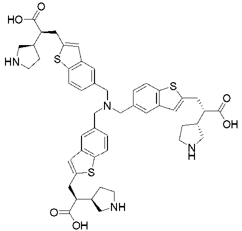 |
| 105 | 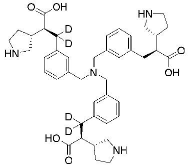 | 106 | 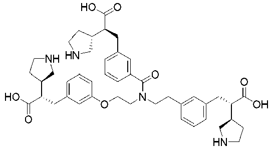 |
| 107 | 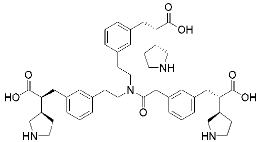 | 108 | 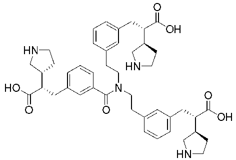 |
| 109 | 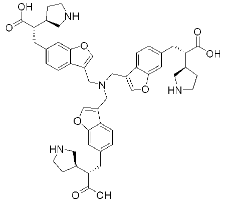 | 110 | 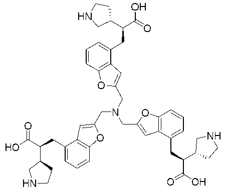 |
| 111 | 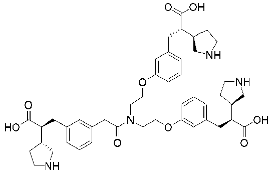 | 112 | 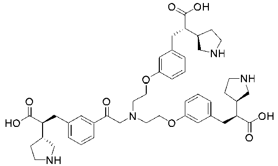 |
| 113 | 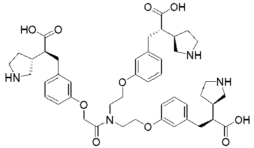 | 114 | 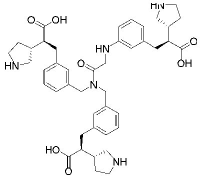 |
| 115 | 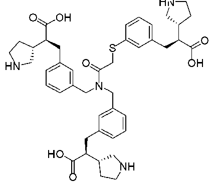 | 116 | 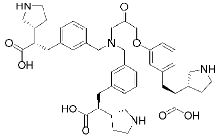 |
| 117 | 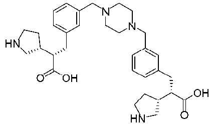 | 118 | 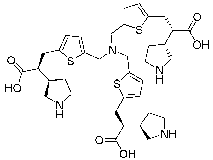 |
| 119 | 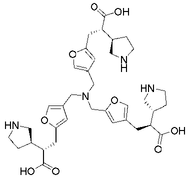 | 120 | 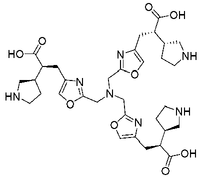 |
| 121 | 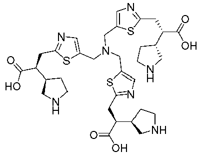 | 122 | 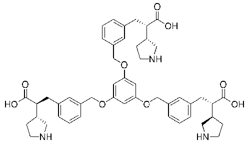 |
| 123 | 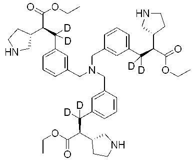 | 124 | 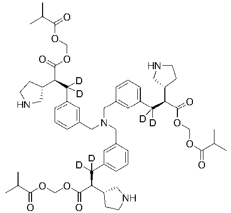 |
| 125 | 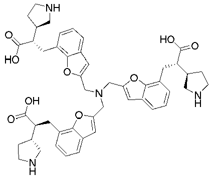 | 126 | 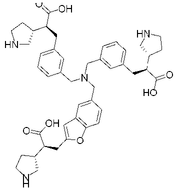 |
| 127 | 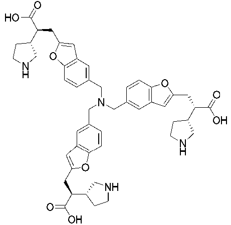 | 128 | 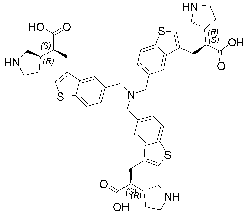 |
| 129 | 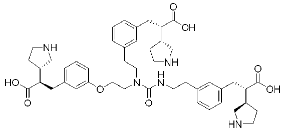 | 130 | 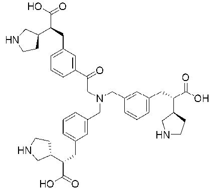 |
| 131 | 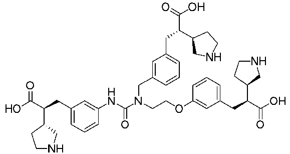 | 132 | 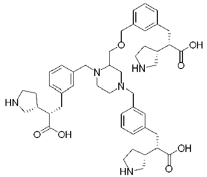 |
| 133 | 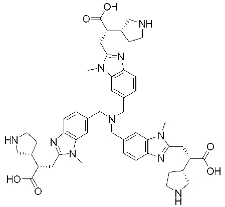 | 134 | 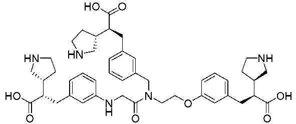 |
| 135 | 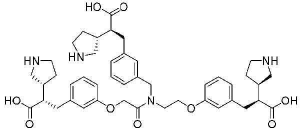 | 136 | 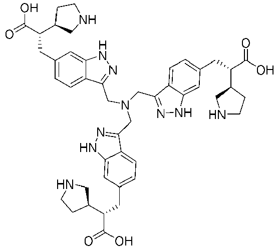 |
| 137 | 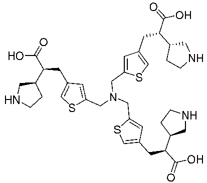 | 138 | 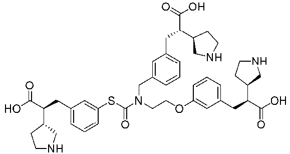 |
| 139 | 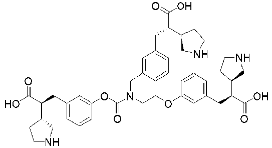 | 140 | 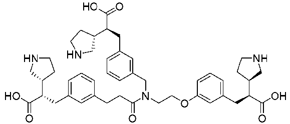 |
| 141 | 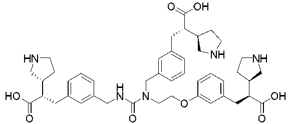 | 142 | 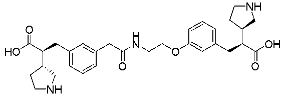 |
| 143 | 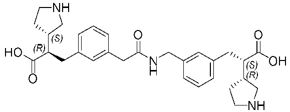 | 144 | 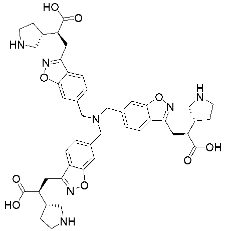 |
| 145 | 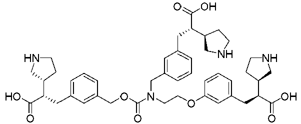 | 146 | 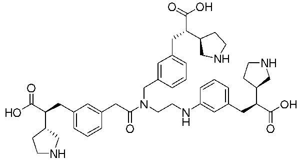 |
| 147 | 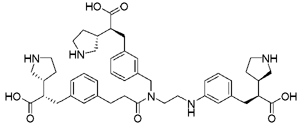 | 148 | 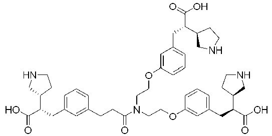 |
| 149 | 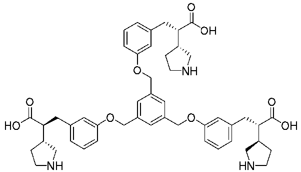 | 150 | 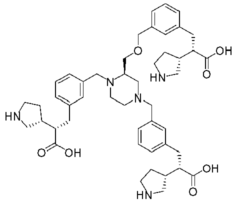 |
| 151 | 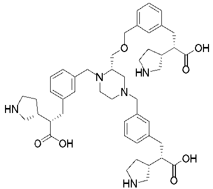 | 152 | 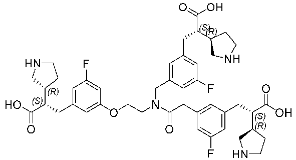 |
| 153 | 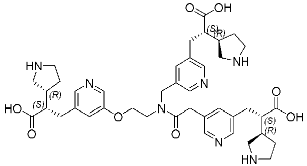 | 154 | 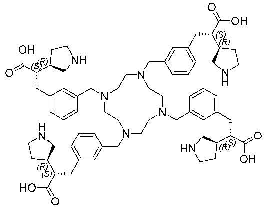 |
| 155 | 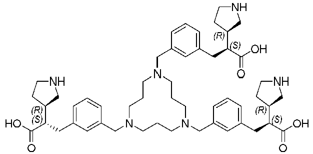 | 156 | 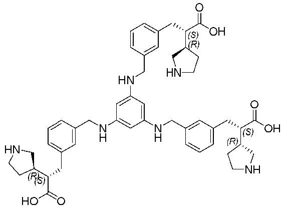 |
| 157 | 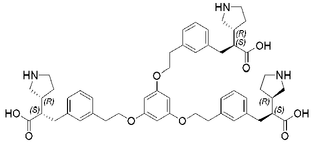 | 158 | 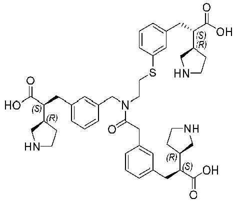 |
| 159 | 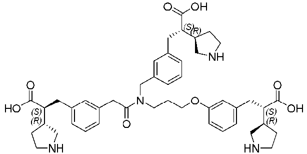 | 160 | 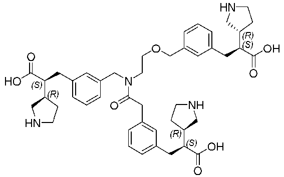 |
| 161 | 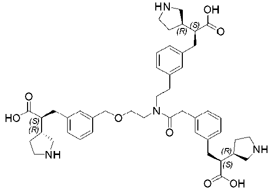 | 162 | 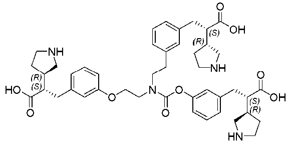 |
| 163 | 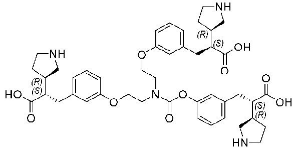 | 164 | 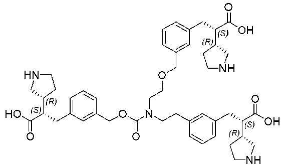 |
| 165 | 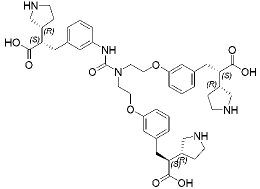 | 166 | 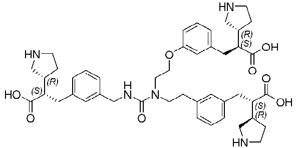 |
| 167 | 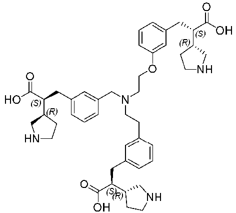 | 168 | 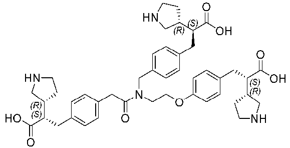 |
| 169 | 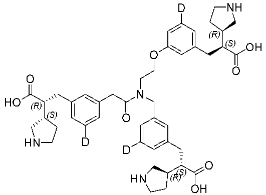 | 170 | 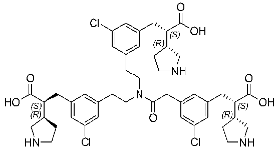 |
| 171 | 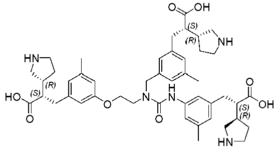 | 172 | 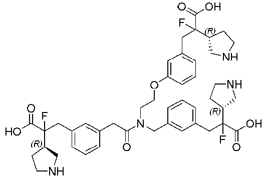 |
| 173 | 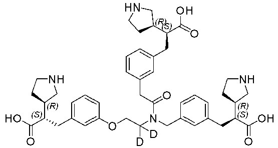 | 174 | 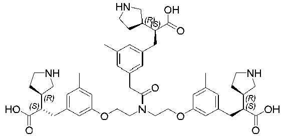 |
| 175 | 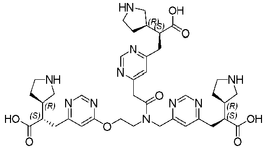 | 176 | 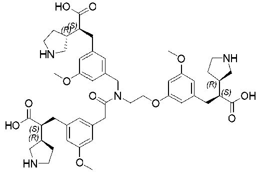 |
| 177 | 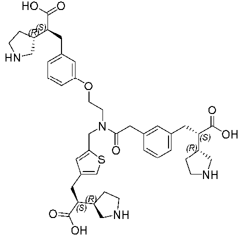 | 178 | 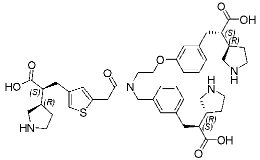 |
| 179 | 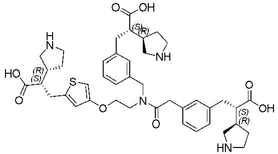 | 180 | 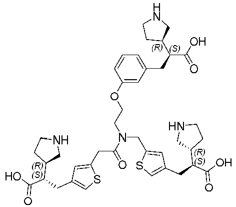 |
| 181 | 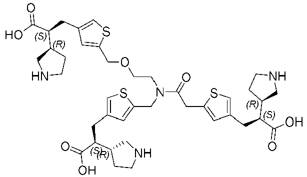 | 182 | 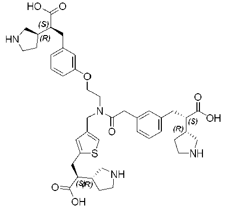 |
| 183 | 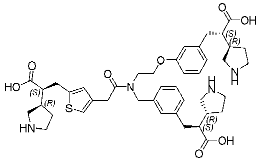 | 184 | 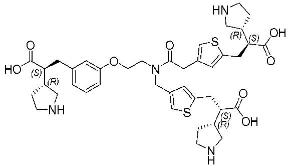 |
| 185 | 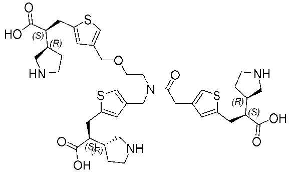 | 186 | 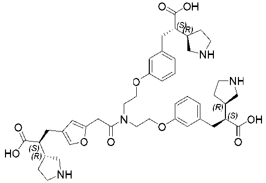 |
| 187 | 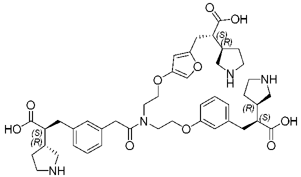 | 188 | 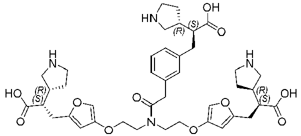 |
| 189 | 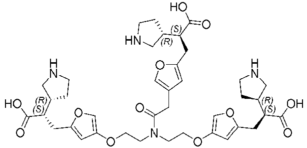 | 190 | 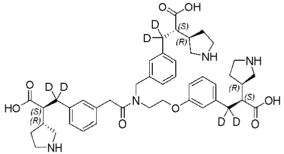 |
| 191 | 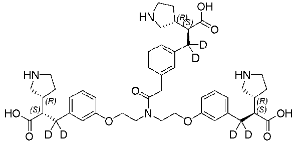 | 192 | 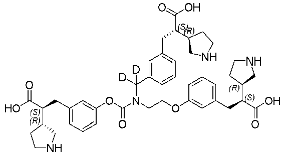 |
| 193 | 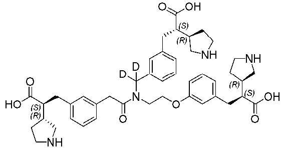 | 194 | 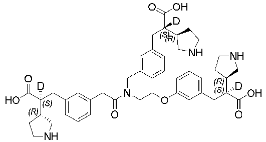 |
| 195 | 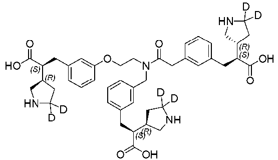 | 196 | 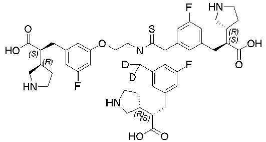 |
| 197 | 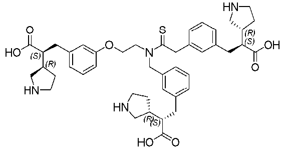 | 198 | 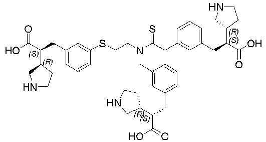 |
| 199 | 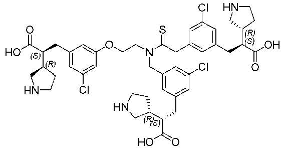 | 200 | 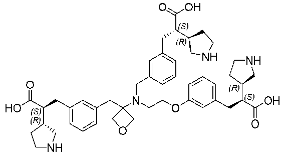 |
| 201 | 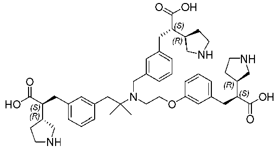 | 202 | 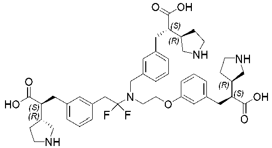 |
| 203 | 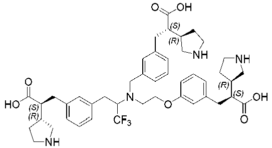 | 204 | 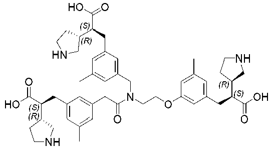 |
| 205 | 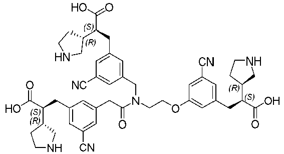 | 206 | 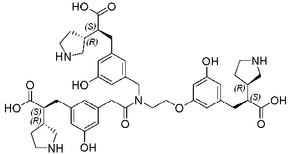 |
| 207 | 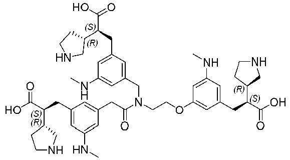 | 208 | 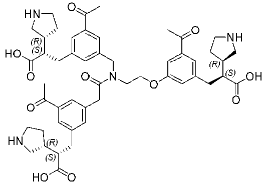 |
| 209 | 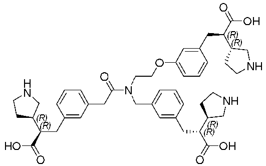 | 210 | 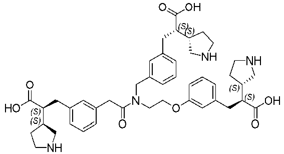 |
| 211 | 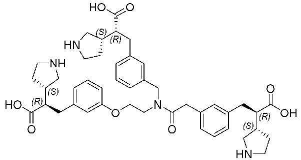 | 212 | 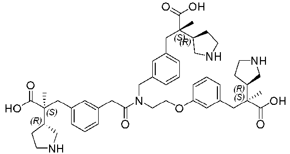 |
| 213 | 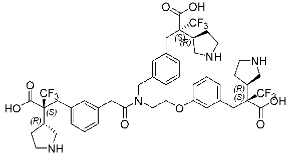 | 214 | 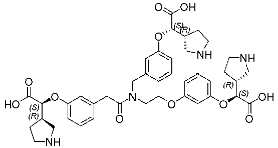 |
| 215 | 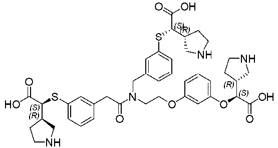 | 216 | 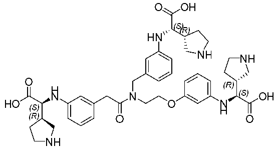 |
| 217 | 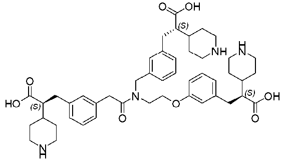 | 218 | 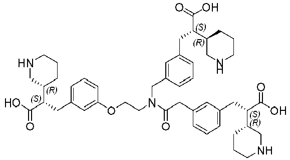 |
| 219 | 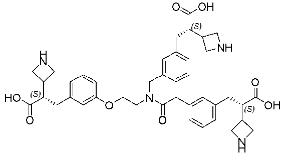 | 220 | 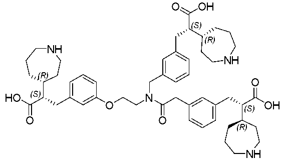 |
| 221 | 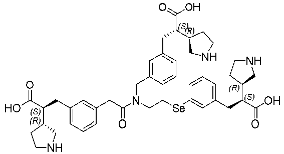 | 222 | 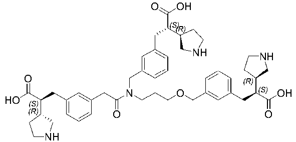 |
| 223 | 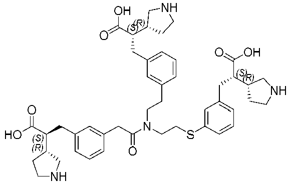 | 224 | 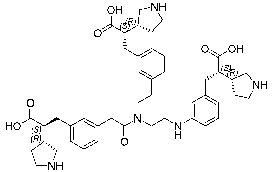 |
| 225 | 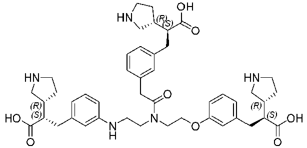 | 226 | 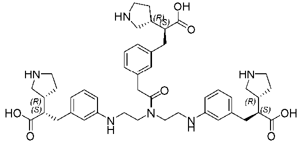 |
| 227 | 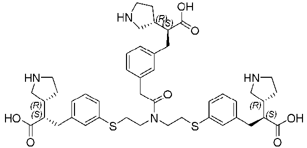 | 228 | 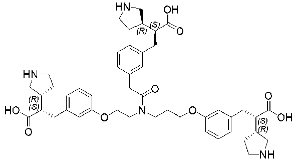 |
alternatively said compound is selected from: 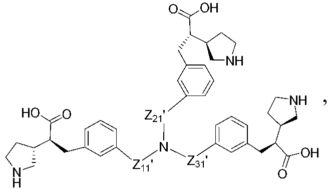 wherein * represents the connecting end with N,
| Compound No. | Z₃₁' | Z₁₁' | Z₂₁' |
|---|---|---|---|
| 229 | C(O) | CH₂ | CH₂CH₂CH₂ |
| 230 | C(O) | CH₂ | *CH₂CH₂S |
| 231 | C(O) | CH₂ | *CH₂CH₂NH |
| 232 | C(O) | CH₂ | *CH₂CH₂CH₂O |
| 233 | C(O) | CH₂ | *CH₂CH₂C(O) |
| 234 | C(O) | CH₂ | *CH₂CH₂C(S) |
| 235 | C(O) | CH₂ | *CH₂CH₂C(O)O |
| 236 | C(O) | CH₂ | *CH₂CH₂C(O)NH |
| 237 | C(O) | CH₂ | *CH₂CH₂C(O)NCH₂ |
| 238 | C(O) | CH₂ | *CH₂CH₂NHC(O) |
| 239 | C(O) | CH₂CH₂ | CH₂CH₂CH₂ |
| 240 | C(O) | CH₂CH₂ | *CH₂CH₂S |
| 241 | C(O) | CH₂CH₂ | *CH₂CH₂CH₂O |
| 242 | C(O) | *CH₂CH₂O | *CH₂CH₂O |
| 243 | C(O) | *CH₂CH₂O | *CH₂CH₂S |
| 244 | C(O) | *CH₂CH₂O | *CH₂CH₂NH |
| 245 | C(O) | *CH₂CH₂O | *CH₂CH₂C(O) |
| 246 | C(O) | *CH₂CH₂O | *CH₂CH₂C(O)NH |
| 247 | *C(O)CH₂ | CH₂ | CH₂ |
| 248 | *C(O)CH₂ | CH₂ | CH₂CH₂ |
| 249 | *C(O)CH₂ | CH₂CH₂ | CH₂CH₂CH₂ |
| 250 | *C(O)CH₂ | *CH₂CH₂O | CH₂CH₂CH₂ |
| 251 | *C(O)CH₂ | *CH₂CH₂O | *CH₂CH₂S |
| 252 | *C(O)CH₂CH₂ | *CH₂CH₂O | *CH₂CH₂CH₂S |
| 253 | *C(O)CH₂CH₂ | *CH₂CH₂O | *CH₂CH₂C(O) |
| 254 | *C(O)CH₂CH₂ | *CH₂CH₂O | CH₂CH₂ |
| 255 | *C(O)CH₂CH₂ | CH₂ | *CH₂CH₂CH₂O |
| 256 | *C(O)CH₂CH₂ | CH₂ | *CH₂CH₂C(O) |
| 257 | *C(O)CH₂CH₂ | CH₂ | *CH₂CH₂C(O)O |
| 258 | *C(O)CH₂CH₂ | CH₂ | *CH₂CH₂C(O)NH |
| 259 | *CH₂C(O) | CH₂ | CH₂CH₂ |
| 260 | *CH₂C(O) | CH₂ | *CH₂CH₂O |
| 261 | *CH₂C(O) | CH₂ | *CH₂CH₂NH |
| 262 | *CH₂CH₂C(O) | CH₂ | CH₂ |
| 263 | *CH₂CH₂C(O) | CH₂ | CH₂CH₂ |
| 264 | *CH₂CH₂C(O) | CH₂ | *CH₂CH₂O |
| 265 | *CH₂CH₂C(O) | CH₂ | *CH₂CH₂S |
| 266 | *CH₂CH₂O | *CH₂CH₂O | *CH₂CH₂O |
| 267 | *CH₂CH₂O | *CH₂CH₂O | *CH₂CH₂C(O) |
| 268 | *CH₂CH₂O | *CH₂CH₂O | *CH₂CH₂C(O)NH |
| 269 | *CH₂CH₂NH | CH₂ | *CH₂CH₂C(O)NCH₂ |
| 270 | *CH₂CH₂NH | CH₂ | *CH₂CH₂NHC(O) |
| 271 | *CH₂CH₂NH | *CH₂CH₂NH | *CH₂CH₂O |
| 272 | *CH₂CH₂NH | *CH₂CH₂NCH₂ | *CH₂CH₂C(O) |
| 273 | *CH₂CH₂NH | *CH₂CH₂O | *CH₂CH₂C(O)NH |
| 274 | *C(O)OCH₂ | CH₂ | *CH₂CH₂NH |
| 275 | *C(O)OCH₂ | CH₂ | *CH₂CH₂S |
| 276 | *C(O)OCH₂ | CH₂CH₂ | *CH₂CH₂O |
| 277 | *C(O)OCH₂ | CH₂CH₂ | *CH₂CH₂S |
| 278 | *C(O)OCH₂ | *CH₂CH₂NH | *CH₂CH₂O |
| 279 | *C(O)OCH₂ | *CH₂CH₂NH | *CH₂CH₂CH₂O |
| 280 | *C(O)OCH₂ | CH₂ | CH₂ |
| 281 | *C(O)OCH₂ | CH₂CH₂ | CH₂ |
| 282 | *C(O)NHCH₂ | CH₂ | *CH₂CH₂NH |
| 283 | *C(O)NHCH₂ | CH₂CH₂ | *CH₂CH₂NH |
| 284 | *C(O)NHCH₂ | *CH₂CH₂NH | *CH₂CH₂O |
| 285 | *C(O)NH | CH₂ | *CH₂CH₂S |
| 286 | *C(O)NH | CH₂CH₂ | *CH₂CH₂NH |
| 287 | *C(O)NH | CH₂CH₂ | *CH₂CH₂O |
| 288 | *C(O)NH | CH₂CH₂ | *CH₂CH₂S |
| 289 | *C(O)NH | *CH₂CH₂NH | *CH₂CH₂O |
| 290 | *C(O)O | CH₂ | *CH₂CH₂S |
| 291 | *C(O)O | CH₂CH₂ | *CH₂CH₂S |
| 292 | *C(O)CH₂O | CH₂ | *CH₂CH₂NH |
| 293 | *C(O)CH₂O | CH₂CH₂ | *CH₂CH₂C(O)NH |
| 294 | *C(O)CH₂O | CH₂CH₂ | *CH₂CH₂O |
| 295 | *C(O)CH₂O | CH₂CH₂ | *CH₂CH₂NH |
| 296 | *C(O)CH₂NH | *CH₂CH₂O | *CH₂CH₂S |
| 297 | *C(O)CH₂NH | *CH₂CH₂O | *CH₂CH₂O |
| 298 | *C(O)CH₂NH | CH₂CH₂ | *CH₂CH₂NH |
| 299 | CH₂ | *CH₂CH₂O | *CH₂CH₂O. |

13. A compound as represented by formula (D-c), formula (D-f), formula (D-g), or formula (D-h), or a pharmaceutically acceptable salt thereof: 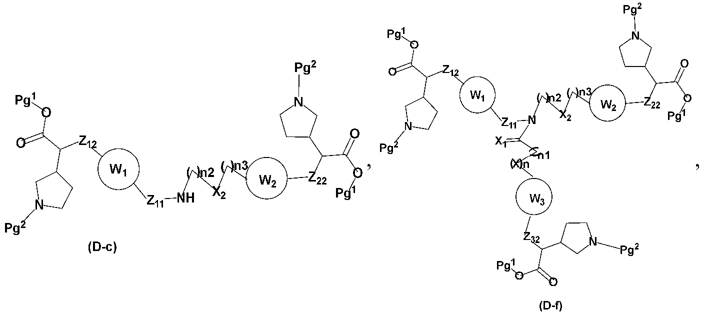 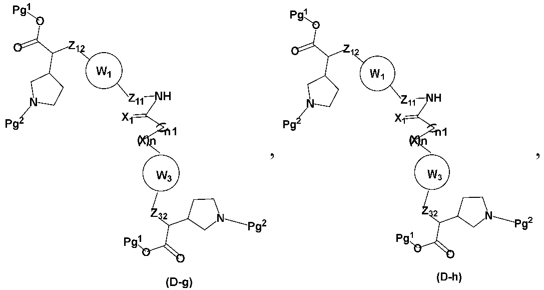
wherein Pg¹ and Pg² are protecting groups respectively; Z₁₁, Z₁₂, Z₂₂, Z₃₂, X, X₁, X₂, n, n1, n2, n3, and rings W₁, W₂, and W₃ are as defined in any one of claims 1 to 12;
for example, Pg¹ is independently selected from tert-butyl, methyl, and benzyl, Pg² is independently selected from tert-butyloxycarbonyl (Boc), 9-fluorenylmethoxycarbonyl (FMOC), and benzyloxycarbonyl (Cbz); or
Pg² is Boc.

14. A pharmaceutical composition comprising the compound according to any one of claims 1 to 12, or a pharmaceutically acceptable salt thereof; optionally the pharmaceutical composition further comprises a pharmaceutically acceptable excipient thereof.

15. Use of the compound according to any one of claims 1 to 12 or a pharmaceutically acceptable salt thereof, or the pharmaceutical composition according to claim 14 in the preparation of a medicament for preventing and/or treating diseases or conditions associated with elevated blood plasma Lp(a) levels; preferably, the disease or condition associated with elevated blood plasma Lp(a) levels is a cardiovascular disease (CVD); preferably, the cardiovascular disease (CVD) including, but not limited to atherosclerotic cardiovascular disease (ASCVD), coronary artery stenosis, aortic valve stenosis, heart failure, and atrial fibrillation; said ASCVD includes peripheral vascular disease, coronary heart disease, and ischemic stroke.

16. A method of preventing and/or treating diseases or conditions associated with elevated blood plasma Lp(a) levels, which method comprises administrating a therapeutically effective amount of the compound according to any one of claims 1 to 12 or a pharmaceutically acceptable salt thereof, or the pharmaceutical composition according to claim 14 to a patient;
preferably, the disease or condition associated with elevated blood plasma Lp(a) levels is a cardiovascular disease (CVD); preferably, the cardiovascular disease (CVD) including, but not limited to atherosclerotic cardiovascular disease (ASCVD), coronary artery stenosis, aortic valve stenosis, heart failure, and atrial fibrillation; said ASCVD includes peripheral vascular disease, coronary heart disease, and ischemic stroke.

17. The compound according to any one of claims 1 to 12 or a pharmaceutically acceptable salt thereof, or the pharmaceutical composition according to claim 14 for use in medicament.

18. The compound according to any one of claims 1 to 12 or a pharmaceutically acceptable salt thereof, or the pharmaceutical composition according to claim 14 for use in preventing and/or treating diseases or conditions associated with elevated blood plasma Lp(a) levels,
preferably, the disease or condition associated with elevated blood plasma Lp(a) levels is a cardiovascular disease (CVD); preferably, the cardiovascular disease (CVD) including, but not limited to atherosclerotic cardiovascular disease (ASCVD), coronary artery stenosis, aortic valve stenosis, heart failure, and atrial fibrillation; said ASCVD includes peripheral vascular disease, coronary heart disease, and ischemic stroke.

19. The compound according to any one of claims 1 to 12 or a pharmaceutically acceptable salt thereof, or the pharmaceutical composition according to claim 14 for use in preventing and/or treating cardiovascular diseases in combination with another compound;
preferably, the another compound is for preventing and/or treating cardiovascular diseases;
preferably, the cardiovascular diseases are diseases or conditions associated with elevated blood plasma Lp(a) levels;
preferably, the disease or condition associated with elevated blood plasma Lp(a) levels is including, but not limited to atherosclerotic cardiovascular disease (ASCVD), coronary artery stenosis, aortic valve stenosis, heart failure, and atrial fibrillation; said ASCVD includes peripheral vascular disease, coronary heart disease, and ischemic stroke.
